# EUROPEAN PATENT SPECIFICATION

(11) **EP 3 234 145 B1**
(45) Date of publication and mention of the grant of the patent: **05.06.2019**
(21) Application number: 15823866.7
(22) Date of filing: 14.12.2015
(51) Int. Cl.: C12N 15/62, A61K 48/00, C12N 5/10, C12N 5/0783

(54) **METHODS FOR CONTROLLED ACTIVATION OR ELIMINATION OF THERAPEUTIC CELLS**
VERFAHREN ZUR KONTROLLIERTEN AKTIVIERUNG ODER ELIMINIERUNG VON THERAPEUTISCHEN ZELLEN
PROCÉDÉ POUR L'ACTIVATION OU L'ELIMINATION DE CELLULES THÉRAPEUTIQUES

(30) Priority: 15.12.2014 US 201462092149 P; 16.04.2015 US 201562148386 P
(43) Date of publication of application: 25.10.2017
(73) Proprietor: Bellicum Pharmaceuticals, Inc., Houston, TX 77030 (US)
(72) Inventor: SPENCER, David, M., Houston, TX 77025 (US); BAYLE, Joseph, Henri, Houston, TX 77005 (US); FOSTER, Aaron, Edward, Houston, TX 77030 (US); SLAWIN, Kevin, M., Houston, TX 77030 (US); MOSELEY, Annemarie, Austin, TX 78733 (US); COLLINSON-PAUTZ, Matthew, R., Houston, TX 77098 (US); DUONG, MyLinh, Sugarland, TX 77478 (US)
(74) Representative: Vossius & Partner Patentanwälte Rechtsanwälte mbB
(86) International application number: PCT/US2015/065646
(87) International publication number: WO 2016/100241

(56) References cited:
- WO-A1-2015/123527
- WO-A1-2016/036746
- US-A1- 2014 087 468
- US-A1- 2014 286 987
- PRIYADHARSHINI NARAYANAN ET AL: "A composite MyD88/CD40 switch synergistically activates mouse and human dendritic cells for enhanced antitumor efficacy", JOURNAL OF CLINICAL INVESTIGATION, vol. 121, no. 4, 1 April 2011 (2011-04-01) , pages 1524-1534, XP055032964, ISSN: 0021-9738, DOI: 10.1172/JCI44327
- TESSA GARGETT ET AL: "The inducible caspase-9 suicide gene system as a safety switch to limit on-target,off-tumor toxicities of chimeric antigen receptor T cells", FRONTIERS IN PHARMACOLOGY, vol. 123, no. 132, 28 October 2014 (2014-10-28), page 3895, XP055267474, DOI: 10.1182/blood-2014-01-551671
- LIHUA E. BUDDE ET AL: "Combining a CD20 Chimeric Antigen Receptor and an Inducible Caspase 9 Suicide Switch to Improve the Efficacy and Safety of T Cell Adoptive Immunotherapy for Lymphoma", PLOS ONE, vol. 8, no. 12, 17 December 2013 (2013-12-17), page e82742, XP055213511, DOI: 10.1371/journal.pone.0082742
- KARIN C STRAATHOF ET AL: "A inducible caspase 9 safety switch for T-cell therapy", BLOOD, AMERICAN SOCIETY OF HEMATOLOGY, US, vol. 105, no. 11, 1 June 2005 (2005-06-01) , pages 4247-4254, XP008126232, ISSN: 0006-4971, DOI: 10.1182/BLOOD-2004-11-4564
- NARAYANAN PRIYADHARSHINI ET AL: "The iCD40.MyD88 combo-vector: A new platform for enhanced DC tumor immunotherapy", AMERICAN ASSOCIATION FOR CANCER RESEARCH. PROCEEDINGS OF THE ANNUAL MEETING, AMERICAN ASSOCIATION FOR CANCER RESEARCH, US, vol. 51, 1 April 2010 (2010-04-01), XP009179655, ISSN: 0197-016X
- Aaron Foster ET AL: "Inducible MyD88/CD40 Allows AP1903--Dependent Costimulation to Control Proliferation and Survival of Chimeric Antigen Receptor--Modified T Cells", Poster, 7 December 2014 (2014-12-07), XP055268436, Retrieved from the Internet: URL:http://www.bellicum.com/wp-content/upl oads/2014/02/Foster-ASH-2014.pdf [retrieved on 2016-04-25]
- SIOK-KEEN TEY: "Adoptive T-cell therapy: adverse events and safety switches", CLINICAL & TRANSLATIONAL IMMUNOLOGY, vol. 3, no. 6, 20 June 2014 (2014-06-20), page e17, XP055268360, DOI: 10.1038/cti.2014.11
- ANTONIO DI STASI ET AL: "Inducible Apoptosis as a Safety Switch for Adoptive Cell Therapy", NEW ENGLAND JOURNAL OF MEDICINE, vol. 365, no. 18, 3 November 2011 (2011-11-03), pages 1673-1683, XP055181696, ISSN: 0028-4793, DOI: 10.1056/NEJMoa1106152
- DEBOKI CHAKRAVARTI ET AL: "Synthetic biology in cell-based cancer immunotherapy", TRENDS IN BIOTECHNOLOGY., vol. 33, no. 8, 1 August 2015 (2015-08-01) , pages 449-461, XP055268332, GB ISSN: 0167-7799, DOI: 10.1016/j.tibtech.2015.05.001

## Description

### Field

The present invention is defined by the claims. The technology described herein relates in part to methods for controlling the activity or elimination of therapeutic cells using multimerization of proteins to manipulate individual protein-protein interactions in therapeutic cells, for example, by activating or eliminating cells used to promote engraftment, to treat diseases or condition, or to control or modulate the activity of therapeutic cells that express chimeric antigen receptors or recombinant T cell receptors.

### Background

There is an increasing use of cellular therapy in which modified or unmodified cells, such as T cells, are administered to a patient. In some examples, cells are genetically engineered to express a heterologous gene, these modified cells are then administered to patients. Heterologous genes may be used to express chimeric antigen receptors (CARs), which are artificial receptors designed to convey antigen specificity to T cells without the requirement for MHC antigen presentation. They include an antigen-specific component, a transmembrane component, and an intracellular component selected to activate the T cell and provide specific immunity. CAR-expressing T cells may be used in various therapies, including cancer therapies. These treatments are used, for example, to target tumors for elimination, and to treat cancer and blood disorders, but these therapies may have negative side effects.

In some instances of therapeutic cell-induced adverse events, there is a need for rapid and near complete elimination of the therapeutic cells. Overzealous on-target effects, such as those directed against large tumor masses, can lead to cytokine storms, associated with tumor lysis syndrome (TLS), cytokine release syndrome (CRS) or macrophage activation syndrome (MAS). As a result, there is great interest in the development of a stable, reliable "suicide gene" that can eliminate transferred T cells or stem cells in the event that they trigger serious adverse events (SAEs), or become obsolete following treatment. Yet in some instances, the need for therapy may remain, and there may be a way to reduce the negative effects, while maintaining a sufficient level of therapy.

In some instances, there is a need to increase the activity of the therapeutic cell. For example, costimulating polypeptides may be used to enhance the activation of T cells, and of CAR-expressing T cells against target antigens, which would increase the potency of adoptive immunotherapy.

Thus, there is a need for controlled activation or elimination of therapeutic cells, to rapidly enhance the activity of or to remove the possible negative effects of donor cells used in cellular therapy, while retaining part or all of the beneficial effects of the therapy.

### Summary

Autologous T cells expressing chimeric antigen receptors (CARs) directed toward tumor-associated antigens (TAAs) have had a transformational effect in initial clinical trials on the treatment of certain types of leukemias ("liquid tumors") and lymphomas with objective response (OR) rates approaching 90%. Despite their great clinical promise and the predictable accompanying enthusiasm, this success is tempered by the observed high level of on-target, off-tumor adverse events, typical of a cytokine release syndrome (CRS). To maintain the benefit of these revolutionary treatments while minimizing the risk, a tunable safety switch has been developed, in order to control the activity level of CAR-expressing T cells. An inducible costimulatory chimeric polypeptide allows for a sustained, modulated control of a chimeric antigen receptor (CAR) that is co-expressed in the cell. The ligand inducer activates the CAR-expressing cell by multimerizing the inducible chimeric signaling molecules, which, in turn, induces NF-κB and other intracellular signaling pathways, leading to the activation of the target cells, for example, a T cell, a tumor-infiltrating lymphocyte (TIL), a natural killer (NK) cell, or a natural killer T (NK-T) cell. In the absence of the ligand inducer, the T cell is quiescent, or has a basal level of activity.
At the second level of control, a "dimmer" switch may allow for continued cell therapy, while reducing or eliminating significant side effects by eliminating the therapeutic cells from the subject, as needed. This dimmer switch is dependent on a second ligand inducer. In some examples, where there is a need to rapidly eliminate the therapeutic cells, an appropriate dose of the second ligand inducer is administered in order to eliminate over 90% or 95% of the therapeutic cells from the patient. This second level of control may be "tunable," that is, the level of removal of the therapeutic cells may be controlled so that it results in partial removal of the therapeutic cells. This second level of control may include, for example, a chimeric pro-apoptotic polypeptide.

In some examples, the chimeric apoptotic polypeptide comprises a binding site for rapamycin, or a rapamycin analog (rapalog); also present in the therapeutic cell is an inducible chimeric polypeptide that, upon induction by a ligand inducer, activates the therapeutic cell; in some examples, the inducible chimeric polypeptide provides costimulatory activity to the therapeutic cell. The CAR may be present on a separate polypeptide expressed in the cell. In other examples, the CAR may be present as part of the same polypeptide as the inducible chimeric polypeptide. Using this controllable first level, the need for continued therapy, or the need to stimulate therapy, may be balanced with the need to eliminate or reduce the level of negative side effects.

A rapamycin analog, or "rapalog", may be administered to the patient, which then binds to both the caspase polypeptide and the chimeric antigen receptor, thus recruiting the caspase polypeptide to the location of the CAR, and aggregating the caspase polypeptide. Upon aggregation, the caspase polypeptide induces apoptosis. The amount of rapamycin or rapamycin analog administered to the patient may vary; if the removal of a lower level of cells by apoptosis is desired in order to reduce side effects and continue CAR therapy, a lower level of rapamycin or rapalog may be administered to the patient.

At the second level of therapeutic cell elimination, selective apoptosis may be induced in cells that express a chimeric Caspase-9 polypeptide fused to a dimeric ligand binding polypeptide, such as, for example, the AP1903-binding polypeptide FKBP12v36, by administering rimiducid (AP1903). In some examples, the Caspase-9 polypeptide includes amino acid substitutions that result in a lower level of basal apoptotic activity as part of the inducible chimeric polypeptide, than the wild type Caspase-9 polypeptide.

Thus, described herein is a nucleic acid comprising a promoter, operably linked to a) a first polynucleotide encoding a first chimeric polypeptide, wherein the first chimeric polypeptide comprises (i) a first multimerizing region or a second multimerizing region; (ii) a MyD88 polypeptide region or a truncated MyD88 polypeptide region lacking the TIR domain; and (iii) a CD40 cytoplasmic polypeptide region lacking the CD40 extracellular domain; and
b) a second polynucleotide encoding a second chimeric polypeptide, wherein the second chimeric polypeptide comprises (i) a pro-apoptotic polypeptide region and (ii) the first multimerizing region or the second multimerizing region, wherein: the second multimerizing region has a different amino acid sequence than the first multimerizing region; the first chimeric polypeptide comprises the first multimerizing region and the second chimeric polypeptide comprises the second multimerizing region, or the first chimeric polypeptide comprises the second multimerizing region and the second chimeric polypeptide comprises the first multimerizing region; the first multimerizing region and the second multimerizing region bind to a first ligand; the first multimerizing region binds to a second ligand; and the second ligand does not significantly bind to the second multimerizing region. Also provided herein are nucleic acids that comprise a promoter, operably linked to a) a first polynucleotide encoding a first chimeric polypeptide, wherein the first chimeric polypeptide comprises (i) a first multimerizing region or a second multimerizing region; and (ii) a MyD88 polypeptide region or a truncated MyD88 polypeptide region lacking the TIR domain; and b) a second polynucleotide encoding a second chimeric polypeptide, wherein the second chimeric polypeptide comprises (i) a pro-apoptotic polypeptide region and (ii) the first multimerizing region or the second multimerizing region, wherein the second multimerizing region has a different amino acid sequence than the first multimerizing region; the first chimeric polypeptide comprises the first multimerizing region and the second chimeric polypeptide comprises the second multimerizing region, or the first chimeric polypeptide comprises the second multimerizing region and the second chimeric polypeptide comprises the first multimerizing region; the first multimerizing region and the second multimerizing region bind to a first ligand; the first multimerizing region binds to a second ligand; and the second ligand does not significantly bind to the second multimerizing region.

The first ligand may comprise a first portion, the first multimerizing region binds to the first portion, and the second multimerizing region does not significantly bind to the first portion. The second ligand may not be capable of binding to the second multimerizing region. The first multimerizing region may be a FKBP12 or FKBP12 variant region and the second multimerizing region is a FKBP-12-Rapamycin Binding (FRB) or FRB variant region. The first ligand may be rapamycin or a rapalog, and the second ligand may be selected from the group consisting of AP1903, AP20187, and AP1510.

Also described herein are nucleic acids comprising a promoter, operably linked to a) a first polynucleotide encoding a first chimeric polypeptide, wherein the first chimeric polypeptide comprises (i) a FKBP12 or FKBP12 variant region; (ii) a MyD88 polypeptide region or a truncated MyD88 polypeptide region lacking the TIR domain; and (iii) a CD40 cytoplasmic polypeptide region lacking the CD40 extracellular domain; and b) a second polynucleotide encoding a second chimeric polypeptide, wherein the second chimeric polypeptide comprises a Caspase-9 region and a FRB or FRB variant region. Also described herein is a nucleic acid comprising a promoter, operably linked to a) a first polynucleotide encoding a first chimeric polypeptide, wherein the first chimeric polypeptide comprises (i) at least two FKBP12 or FKBP12 variant regions; (ii) a MyD88 polypeptide region or a truncated MyD88 polypeptide region lacking the TIR domain; and (iii) a CD40 cytoplasmic polypeptide region lacking the CD40 extracellular domain; and b) a second polynucleotide encoding a second chimeric polypeptide, wherein the second chimeric polypeptide comprises a Caspase-9 region and a FRB or FRB variant region.

Also a modified cell is dewcribed that is transduced or transfected with a nucleic acid of the present application. Also described are modified cells, comprising a) a first polynucleotide encoding a first chimeric polypeptide, wherein the first chimeric polypeptide comprises (i) a first multimerizing region or a second multimerizing region; (ii) a MyD88 polypeptide region or a truncated MyD88 polypeptide region lacking the TIR domain; and (iii) a CD40 cytoplasmic polypeptide region lacking the CD40 extracellular domain; and b) a second polynucleotide encoding a second chimeric polypeptide, wherein the second chimeric polypeptide comprises (i) a pro-apoptotic polypeptide region and (ii) the first multimerizing region or the second multimerizing region, wherein: the second multimerizing region has a different amino acid sequence than the first multimerizing region; the first chimeric polypeptide comprises the first multimerizing region and the second chimeric polypeptide comprises the second multimerizing region, or the first chimeric polypeptide comprises the second multimerizing region and the second chimeric polypeptide comprises the first multimerizing region; the first multimerizing region and the second multimerizing region bind to a first ligand; the first multimerizing region binds to a second ligand; and the second ligand does not significantly bind to the second multimerizing region. The second ligand may not be capable of binding to the second multimerizing region.

Also described herein are methods of controlling survival of transplanted modified cells in a subject, comprising a) transplanting modified cells of the present application into the subject; and b) after (a), administering to the subject the first ligand in an amount effective to kill less than 30%, or at least 30, 40, 50, 60, 70, 80, 90, or 95% of the modified cells that express the second chimeric polypeptide wherein the first chimeric polypeptide comprises the first multimerizing region and the second chimeric polypeptide comprises the second multimerizing region.

Also described herein are methods for treating a subject having a disease or condition associated with an elevated expression of a target antigen expressed by a target cell, comprising (a) transplanting an effective amount of modified cells into the subject; wherein the modified cells comprise a modified cell of the present application, wherein the modified cell comprises a chimeric antigen receptor comprising an antigen recognition moiety that binds to the target antigen, and (b) after a), administering an effective amount of the second ligand to reduce the number or concentration of target antigen or target cells in the subject wherein the first chimeric polypeptide comprises the first multimerizing region and the second chimeric polypeptide comprises the second multimerizing region. The methods may further comprise after b), administering the first ligand in an amount effective to kill less than 30%, or at least 30, 40, 50, 60, 70, 80, 90, or 95% of the modified cells that express the second chimeric polypeptide.

The first multimerizing region may comprise two FKBP12v36 regions, and the second multimerizing region comprises an FRB or FRB variant region. The pro-apoptotic polypeptide may be a caspase polypeptide.

Also described herein are nucleic acids that comprise a promoter, operably linked to a first polynucleotide and a second polynucleotide, wherein a) the first polynucleotide encodes a first chimeric apoptotic polypeptide comprising a first multimerizing region and a pro-apoptotic polypeptide region; and b) the second polynucleotide encodes a second chimeric apoptotic polypeptide comprising a second multimerizing region and a pro-apoptotic polypeptide region, wherein the second multimerizing region has a different amino acid sequence than the first multimerizing region; wherein the first and second multimerizing regions bind to a first ligand and the pro-apoptotic polypeptide regions are together capable of multimerizing following binding to the first ligand and inducing apoptosis in a cell. The first multimerizing region may bind to a second ligand that does not significantly bind to the second multimerizing region. The second ligand may not be capable of binding to the second multimerizing region. The proapoptotic polypeptide may be a caspase polypeptide.

Also described herein are nucleic acids that comprise a promoter operably linked to a polynucleotide coding for a polypeptide comprising a FRB or FRB variant region and a caspase polypeptide region. Also described are nucleic acids that comprise a promoter, operably linked to a first polynucleotide and a second polynucleotide, wherein a) the first polynucleotide encodes a first chimeric caspase polypeptide comprising a FRB or FRB variant region and a caspase polypeptide region; and b) the second polynucleotide encodes a second chimeric caspase polypeptide comprising an FKBP12 or FKBP12 variant region and a caspase polypeptide region.

Also described herein are modified cells are that are transfected or transduced with a nucleic acid of the present application. Also described herein are modified cells, that comprise a) a first polynucleotide encoding a first chimeric apoptotic polypeptide comprising a first multimerizing region and a pro-apoptotic polypeptide region and a second multimerizing region; and b) a second polynucleotide encoding a second chimeric apoptotic polypeptide comprising a second multimerizing region and a pro-apoptotic polypeptide region, wherein the second multimerizing region has a different amino acid sequence than the first multimerizing region; wherein the first and second multimerizing regions bind to a first ligand and the pro-apoptotic polypeptide regions are together capable of multimerizing following binding to the first ligand and inducing apoptosis in the cell. The first multimerizing region may bind to a second ligand that does not significantly bind to the second multimerizing region.

Also describd herein is a nucleic acid comprising a promoter, operably linked to a polynucleotide encoding a first polypeptide, wherein the first polypeptide comprises a scaffold region comprising at least two first multimerizing regions or at least two second multimerizing regions, wherein each of the first multimerizing regions is different than each of the second multimerizing regions. The nucleic acid may further comprises a second polynucleotide encoding a second chimeric polypeptide, wherein the second chimeric polypeptide comprises a pro-apoptotic polypeptide region and the first multimerizing region or the second multimerizing region, wherein the second multimerizing region has a different amino acid sequence than the first multimerizing region; wherein: the first multimerizing region and the second multimerizing region bind to a first ligand; and the first polypeptide comprises the first multimerizing region and the second chimeric polypeptide comprises the second multimerizing region, or the first polypeptide comprises the second multimerizing region and the second chimeric polypeptide comprises the first multimerizing region. The first ligand may comprise a first portion, the first multimerizing region binds to the first portion, and the second multimerizing region does not significantly bind to the first portion. The second multimerizing region may bind to a second ligand, and the first multimerizing region may not significantly bind to the first multimerizing region.

Also describd herein are modified cells that comprise a polynucleotide encoding a first polypeptide, wherein the first polypeptide comprises a scaffold region comprising at least two first multimerizing regions or at least two second multimerizing regions, wherein each of the first multimerizing regions is different than each of the second multimerizing regions. The modified cell further may comprise a second polynucleotide encoding a second chimeric polypeptide, wherein the second chimeric polypeptide comprises a pro-apoptotic polypeptide region and the first multimerizing region or the second multimerizing region, wherein the second multimerizing region has a different amino acid sequence than the first multimerizing region; wherein: the first multimerizing region and the second multimerizing region bind to a first ligand; and the first polypeptide comprises the first multimerizing region and the second chimeric polypeptide comprises the second multimerizing region, or the first polypeptide comprises the second multimerizing region and the second chimeric polypeptide comprises the first multimerizing region.

Also described herein are modified cells that comprise a first polynucleotide encoding a scaffold polypeptide, wherein the scaffold polypeptide comprises at least two FRB or FRB variant regions, and a second polynucleotide encoding a chimeric polypeptide comprising an FKBP12 or FKBP12 variant region and a Caspase-9 polypeptide. Also describd herein are modified cells that comprise a first polynucleotide encoding a scaffold polypeptide, wherein the scaffold polypeptide comprises at least two FKBP12 or FKBP12 variant regions, and a second polynucleotide encoding a chimeric polypeptide comprising a FRB or FRB variant and a Caspase-9 polypeptide. The modified cells may further comprise a chimeric antigen receptor, or a chimeric T cell receptor.

Also describd herein are methods of controlling survival of transplanted modified cells in a subject, comprising: a) transplanting modified cells of the present application into the subject; and b) after (a), administering to the subject rapamycin or a rapalog, in an amount effective to kill less than 30%, or at least 30, 40, 50, 60, 70, 80, 90, or 95% of the modified cells that express the second chimeric polypeptide comprising the pro-apoptotic polypeptide region. The second multimerizing region may be a FKBP12 or FKBP12 variant region, further comprising administering a ligand that binds to the FKBP12 or FKBP12 variant region on the second chimeric polypeptide comprising the pro-apoptotic polypeptide region in an amount effective to kill at least 90% of the modified cells that express the second chimeric polypeptide. Also describd herein methods for treating a subject having a disease or condition associated with an elevated expression of a target antigen expressed by a target cell, comprising (a) administering to the subject an effective amount of a modified cell of the present application, wherein the modified cell further comprises a polynucleotide coding for a chimeric antigen receptor or a T cell receptor that bind to the target antigen; and (b) after a), administering an effective amount of a ligand, rapamycin, or a rapalog.

Also describd herein is a nucleic acid comprising a promoter, operably linked to
a) a first polynucleotide encoding a first chimeric polypeptide, wherein the first chimeric polypeptide comprises (i) two FKBP12v36 regions; (ii) a truncated MyD88 polypeptide region lacking the TIR domain; and (iii) a CD40 cytoplasmic polypeptide region lacking the CD40 extracellular domain;
b) a second polynucleotide encoding a second chimeric polypeptide, wherein the second chimeric polypeptide comprises a Caspase-9 region and a FRB_{L}; and
c) a third polynucleotide encoding a chimeric antigen receptor comprising a transmembrane region, a T cell activation molecule, and an antigen recognition moiety selected from the group consisting of Her2/Neu, PSCA, and CD19. Also describd herein is a modified cell, comprising a) a first polynucleotide encoding a first chimeric polypeptide, wherein the first chimeric polypeptide comprises (i) two FKBP12v36 regions; (ii) a truncated MyD88 polypeptide region lacking the TIR domain; and (iii) a CD40 cytoplasmic polypeptide region lacking the CD40 extracellular domain; b) a second polynucleotide encoding a second chimeric polypeptide, wherein the second chimeric polypeptide comprises a Caspase-9 region and a FRB_{L}; and c) a third polynucleotide encoding a chimeric antigen receptor comprising a transmembrane region, a T cell activation molecule, and a Her2/Neu antigen recognition moiety.

Also describd herein is a nucleic acid comprising a promoter, operably linked to
a) a first polynucleotide encoding a first chimeric polypeptide, wherein the first chimeric polypeptide comprises (i) two FKBP12v36 regions; (ii) a truncated MyD88 polypeptide region lacking the TIR domain; and (iii) a CD40 cytoplasmic polypeptide region lacking the CD40 extracellular domain;
b) a second polynucleotide encoding a second chimeric polypeptide, wherein the second chimeric polypeptide comprises a Caspase-9 region and a FRB_{L}; and
c) a third polynucleotide encoding a chimeric T cell receptor. Also described herein is a modified cell, comprising a) a first polynucleotide encoding a first chimeric polypeptide, wherein the first chimeric polypeptide comprises (i) two FKBP12v36 regions; (ii) a truncated MyD88 polypeptide region lacking the TIR domain; and (iii) a CD40 cytoplasmic polypeptide region lacking the CD40 extracellular domain; b) a second polynucleotide encoding a second chimeric polypeptide, wherein the second chimeric polypeptide comprises a Caspase-9 region and a FRB_{L}; and c) a third polynucleotide encoding a chimeric T cell receptor.

Also describd herein is a nucleic acid comprising a promoter operably linked to a first polynucleotide encoding a first chimeric polypeptide, wherein the first chimeric polypeptide comprises (i) a first ligand binding region; (ii) a MyD88 polypeptide region or a truncated MyD88 polypeptide region lacking the TIR domain; and (iii) a CD40 cytoplasmic polypeptide region lacking the CD40 extracellular domain; and a second polynucleotide encoding a second chimeric polypeptide, wherein the second chimeric polypeptide comprises a pro-apoptotic polypeptide region and a second ligand binding region, wherein the second ligand binding region has a different amino acid sequence than the first ligand binding region; wherein the first and second ligand binding regions are capable of binding to a first multimeric ligand; the first ligand binding region is capable of binding to a second ligand; and the second ligand does not significantly bind to the second ligand binding region. Also describd herein is a nucleic acid comprising a promoter, operably linked to a first polynucleotide encoding a first chimeric polypeptide, wherein the first chimeric polypeptide comprises (i) a first ligand binding region; and (ii) a MyD88 polypeptide region or a truncated MyD88 polypeptide region lacking the TIR domain; and a second polynucleotide encoding a second chimeric polypeptide, wherein the second chimeric polypeptide comprises a pro-apoptotic polypeptide region and a second ligand binding region, wherein the second ligand binding region has a different amino acid sequence than the first ligand binding region; wherein the first and second ligand binding regions are capable of binding to a first ligand; the first ligand binding region is capable of binding to a second ligand; and the second ligand does not significantly bind to the second ligand binding region. The nucleic acid may further comprise a polynucleotide encoding a linker polypeptide between the first and second polynucleotides, wherein the linker polypeptide separates the translation products of the first and second polynucleotides during or after translation, for example, the linker polypeptide may be a 2A polypeptide. The second ligand may no not capable of binding to the second ligand binding region.

The nucleic acid may further comprise a polynucleotide encoding a chimeric antigen receptor, a T cell receptor, or a T cell receptor-based chimeric antigen receptor. The chimeric antigen receptor may comprise (i) a transmembrane region, (ii) a T cell activation molecule, and (iii) an antigen recognition moiety.

Also provided are modified cells transfected or transduced with a nucleic acid discussed herein. Also describd herein are modified cells comprising a first polynucleotide encoding a first chimeric polypeptide, wherein the first chimeric polypeptide comprises (i) a first ligand binding region; (ii) a MyD88 polypeptide region or a truncated MyD88 polypeptide region lacking the TIR domain; and (iii) a CD40 cytoplasmic polypeptide region lacking the CD40 extracellular domain; and a second polynucleotide encoding a second chimeric polypeptide, wherein the second chimeric polypeptide comprises a pro-apoptotic polypeptide region and a second ligand binding region, wherein the second ligand binding region has a different amino acid sequence than the first ligand binding region; wherein the first and second ligand binding regions are capable of binding to a first ligand; the first ligand binding region is capable of binding to a second ligand; and the second ligand does not significantly bind to the second ligand binding region. Also describd herein are modified cells comprising a first polynucleotide encoding a first chimeric polypeptide, wherein the first chimeric polypeptide comprises (i) a first ligand binding region; and (ii) a MyD88 polypeptide region or a truncated MyD88 polypeptide region lacking the TIR domain; and a second polynucleotide encoding a second chimeric polypeptide, wherein the second chimeric polypeptide comprises a pro-apoptotic polypeptide region and a second ligand binding region, wherein the second ligand binding region has a different amino acid sequence than the first ligand binding region; wherein the first and second ligand binding regions are capable of binding to a first ligand; the first ligand binding region is capable of binding to a second ligand; and the second ligand does not significantly bind to the second ligand binding region. The second ligand may not be capable of binding to the second ligand binding region. The modified cell modified cell may further comprise a third polynucleotide encoding a marker polypeptide. The first chimeric polypeptide further comprises a marker polypeptide. The second chimeric polypeptide may further comprise a marker polypeptide.

The modified cell may further comprise a polynucleotide encoding a chimeric antigen receptor, a T cell receptor, or a T cell receptor-based chimeric antigen receptor. The chimeric antigen receptor may comprise (i) a transmembrane region, (ii) a T cell activation molecule, and (iii) an antigen recognition moiety.

Also described herein are methods for stimulating an immune response in a subject, comprising: transplanting modified cells discussed herein into the subject; and then administering an effective amount of the second ligand to stimulate a cell mediated immune response. Also described herein are methods of administering a ligand to a human subject who has undergone cell therapy using modified cells discussed herein, comprising administering the second ligand to the human subject. Also described herein are methods for controlling activity of transplanted modified cells in a subject, comprising: transplanting modified cells discussed herein into the subject; and then administering an effective amount of the second ligand to stimulate the activity of the transplanted modified cells. The methods may further comprise then administering to the subject the first ligand in an amount effective to kill less than 30, 40, 50, 60, 70, 80, 90, or 100% of the modified cells that express the second chimeric polypeptide.

Also describd herein are methods for treating a subject having a disease or condition associated with an elevated expression of a target antigen expressed by a target cell, comprising administering to the subject an effective amount of a modified cell of any one of items B1-D14, wherein the cell comprises a chimeric antigen receptor comprising an antigen recognition moiety that binds to the target antigen, and then administering an effective amount of the second ligand to reduce the number or concentration of target antigen or target cells in the subject.

Also describd herein are methods for reducing the size of a tumor in a subject, comprising administering modified cells discussed herein to the subject, wherein the cell comprises a chimeric antigen receptor comprising an antigen recognition moiety that binds to an antigen on the tumor; and then administering an effective amount of the second ligand to reduce the size of the tumor in the subject.

Also describd herein is a modified cell comprising a first polynucleotide encoding a first chimeric polypeptide, wherein the first chimeric polypeptide comprises a membrane-targeting polypeptide region and a first ligand binding region; and a second polynucleotide encoding a second chimeric polypeptide, wherein the second chimeric polypeptide comprises a pro-apoptotic polypeptide region and a second ligand binding region, wherein the second ligand binding region has a different amino acid sequence than the first ligand binding region; wherein the first and second ligand binding regions are capable of binding to a first multimeric ligand.

Also describd herein is a modified cell comprising a first polynucleotide encoding a CAR, wherein the CAR comprises an FKBP12-Rapamycin-Binding domain (FRB); and a second polynucleotide encoding a chimeric caspase polypeptide, wherein the chimeric caspase polypeptide comprises (i) an FKBP multimerizing region and (ii) a caspase polypeptide. Also describd herein is a modified cell, comprising a first polynucleotide encoding a CAR, wherein the CAR comprises (i) a transmembrane region, (ii) a T cell activation molecule, (iii) an antigen recognition moiety, and (iv) an FKBP12-Rapamycin-Binding domain (FRB); and a second polynucleotide encoding a chimeric caspase polypeptide, wherein the chimeric caspase polypeptide comprises (i) an FKBP multimerizing region and (ii) a caspase polypeptide. Also describd herein is a modified cell, comprising a first polynucleotide encoding a CAR, wherein the CAR comprises (i) a transmembrane region, (ii) a MyD88 polypeptide or a truncated MyD88 polypeptide lacking a TIR domain, (iii) a CD40 cytoplasmic polypeptide region lacking a CD40 extracellular domain, (iv) a T cell activation molecule, (v) an antigen recognition moiety, and an FKBP12-Rapamycin-Binding domain (FRB); and a second polynucleotide encoding a chimeric caspase polypeptide, wherein the chimeric caspase polypeptide comprises (i) an FKBP multimerizing region and (ii) a caspase polypeptide.

The polynucleotides encoding the chimeric polypeptides may comprise optimized codons. The cell may be a human cell. The cell described herein may be any type of eukaryotic cell, for example a mammalian cell, for example a horse, dog, cat, cow, or human cell. The cell may be a progenitor cell. The cell may be a hematopoietic progenitor cell. The cell may be selected from the group consisting of mesenchymal stromal cells, embryonic stem cells, and inducible pluripotent stem cells. The cell may be a T cell. The cell may be obtained or prepared from bone marrow. The cell may be obtained or prepared from umbilical cord blood. The cell ma be obtained or prepared from peripheral blood. The cell may be obtained or prepared from peripheral blood mononuclear cells.

The polynucleotide coding for the chimeric polypeptide or modified Caspase-9 polypeptide may be operably linked to a promoter. The promoter may be developmentally regulated and the Caspase-9 polypeptide is expressed in developmentally differentiated cells. The promoter may be tissue-specific and the Caspase-9 polypeptide may be expressed in the specific tissue. The promoter may be activated in activated T cells. The promoter may comprise a 5'LTR sequence. The chimeric protein may further comprise a marker polypeptide, for example, but not limited to, a ΔCD19 polypeptide. The Caspase-9 polypeptide may be a truncated Caspase-9 polypeptide. The Caspase-9 polypeptide may lack the Caspase recruitment domain.

In some aspects of the present application, the cells are transduced or transfected with a viral vector. The viral vector may be, for example, but not limited to, a retroviral vector, such as, for example, but not limited to, a murine leukemia virus vector; an SFG vector; and adenoviral vector, or a lentiviral vector.

The cell may be isolated. The cell may be in a human subject. The cell may be transplanted in a human subject.

Personalized treatment may be provided wherein the stage or level of the disease or condition is determined before administration of the multimeric ligand, before the administration of an additional dose of the multimeric ligand, or in determining method and dosage involved in the administration of the multimeric ligand. These methods may be used in any of the methods of any of the diseases or conditions of the present application. Where these methods of assessing the patient before administering the ligand are discussed in the context of graft versus host disease, it is understood that these methods may be similarly applied to the treatment of other conditions and diseases. Thus, for example, the method may comprise administering therapeutic cells to a patient, and may further comprise identifying a presence or absence of a condition in the patient that requires the removal of transfected or transduced therapeutic cells from the patient; and administering a multimeric ligand that binds to the multimerizing region, maintaining a subsequent dosage of the multimeric ligand, or adjusting a subsequent dosage of the multimeric ligand to the patient based on the presence or absence of the condition identified in the patient. And, for example, the method may further comprise determining whether to administer an additional dose or additional doses of the multimeric ligand to the patient based upon the appearance of graft versus host disease symptoms in the patient. The method may further comprise identifying the presence, absence or stage of graft versus host disease in the patient, and administering a multimeric ligand that binds to the multimerizing region, maintaining a subsequent dosage of the multimeric ligand, or adjusting a subsequent dosage of the multimeric ligand to the patient based on the presence, absence or stage of the graft versus host disease identified in the patient. The method may further comprise identifying the presence, absence or stage of graft versus host disease in the patient, and determining whether a multimeric ligand that binds to the multimerizing region should be administered to the patient, or the dosage of the multimeric ligand subsequently administered to the patient is adjusted based on the presence, absence or stage of the graft versus host disease identified in the patient. The method may further comprise receiving information comprising the presence, absence or stage of graft versus host disease in the patient; and administering a multimeric ligand that binds to the multimerizing region, maintaining a subsequent dosage of the multimeric ligand, or adjusting a subsequent dosage of the multimeric ligand to the patient based on the presence, absence or stage of the graft versus host disease identified in the patient. The method may further comprise identifying the presence, absence or stage of graft versus host disease in the patient, and transmitting the presence, absence or stage of the graft versus host disease to a decision maker who administers a multimeric ligand that binds to the multimerizing region, maintains a subsequent dosage of the multimeric ligand, or adjusts a subsequent dosage of the multimeric ligand administered to the patient based on the presence, absence or stage of the graft versus host disease identified in the subject. The method may further comprise identifying the presence, absence or stage of graft versus host disease in the patient, and transmitting an indication to administer a multimeric ligand that binds to the multimeric binding region, maintain a subsequent dosage of the multimeric ligand or adjust a subsequent dosage of the multimeric ligand administered to the patient based on the presence, absence or stage of the graft versus host disease identified in the subject.

Also provided is a method for administering donor T cells to a human patient, comprising administering a transduced or transfected T cell of the present application to a human patient, wherein the cells are non-allodepleted human donor T cells.

The therapeutic cells may be administered to a subject having a non-malignant disorder, or where the subject has been diagnosed with a non-malignant disorder, such as, for example, a primary immune deficiency disorder (for example, but not limited to, Severe Combined Immune Deficiency (SCID), Combined Immune Deficiency (CID), Congenital T-cell Defect/Deficiency, Common Variable Immune Deficiency (CVID), Chronic Granulomatous Disease, IPEX (Immune deficiency, polyendocrinopathy, enteropathy, X-linked) or IPEX-like, Wiskott-Aldrich Syndrome, CD40 Ligand Deficiency, Leukocyte Adhesion Deficiency, DOCK 8 Deficiency, IL-10 Deficiency/IL-10 Receptor Deficiency, GATA 2 deficiency, X-linked lymphoproliferative disease (XLP), Cartilage Hair Hypoplasia, and the like), Hemophagocytosis Lymphohistiocytosis (HLH) or other hemophagocytic disorders, Inherited Marrow Failure Disorders (such as, for example, but not limited to, Shwachman Diamond Syndrome, Diamond Blackfan Anemia, Dyskeratosis Congenita, Fanconi Anemia, Congenital Neutropenia, and the like), Hemoglobinopathies (such as, for example, but not limited to, Sickle Cell Disease, Thalassemia, and the like), Metabolic Disorders (such as, for example, but not limited to, Mucopolysaccharidosis, Sphingolipidoses, and the like), or an Osteoclast disorder (such as, for example, but not limited to Osteopetrosis).

The therapeutic cells may be, for example, any cell administered to a patient for a desired therapeutic result. The cells may be, for example, T cells, natural killer cells, B cells, macrophages, peripheral blood cells, hematopoietic progenitor cells, bone marrow cells, or tumor cells. The modified Caspase-9 polypeptide can also be used to directly kill tumor cells. In one application, vectors comprising polynucleotides coding for the inducible modified Caspase-9 polypeptide would be injected into a tumor and after 10-24 hours (to permit protein expression), the ligand inducer, such as, for example, AP1903, would be administered to trigger apoptosis, causing the release of tumor antigens to the microenvironment. To further improve the tumor microenvironment to be more immunogenic, the treatment may be combined with one or more adjuvants (e.g., IL-12, TLRs, IDO inhibitors, etc.). The cells may be delivered to treat a solid tumor, such as, for example, delivery of the cells to a tumor bed. A polynucleotide encoding the chimeric Caspase-9 polypeptide may be administered as part of a vaccine, or by direct delivery to a tumor bed, resulting in expression of the chimeric Caspase-9 polypeptide in the tumor cells, followed by apoptosis of tumor cells following administration of the ligand inducer. Thus, also described herein are nucleic acid vaccines, such as DNA vaccines, wherein the vaccine comprises a nucleic acid comprising a polynucleotide that encodes an inducible, or modified inducible Caspase-9 polypeptide of the present application. The vaccine may be administered to a subject, thereby transforming or transducing target cells in vivo. The ligand inducer is then administered following the methods of the present application.

The modified Caspase-9 polypeptide may be a truncated modified Caspase-9 polypeptide. The modified Caspase-9 polypeptide may lack the Caspase recruitment domain. The Caspase-9 polypeptide may comprise the amino acid sequence of SEQ ID NO: 9, or a fragment thereof, or may be encoded by the nucleotide sequence of SEQ ID NO: 8, or a fragment thereof.

The methods may further comprise administering a multimeric ligand that binds to the multimeric ligand binding region. The multimeric ligand binding region may be selected from the group consisting of FKBP, cyclophilin receptor, steroid receptor, tetracycline receptor, heavy chain antibody subunit, light chain antibody subunit, single chain antibodies comprised of heavy and light chain variable regions in tandem separated by a flexible linker domain, and mutated sequences thereof. The multimeric ligand binding region may be an FKBP12 region. The multimeric ligand may be an FK506 dimer or a dimeric FK506-like analog ligand. The multimeric ligand may be AP1903. The number of therapeutic cells may be reduced by from about 60% to 99%, about 70% to 95%, from 80% to 90% or about 90% or more after administration of the multimeric ligand. After administration of the multimeric ligand, donor T cells may survive in the patient that are able to expand and are reactive to viruses and fungi. After administration of the multimeric ligand, donor T cells may survive in the patient that are able to expand and are reactive to tumor cells in the patient.

The suicide gene used in the second level of control may be a caspase polypeptide, for example, Caspase 1, 2, 3, 4, 5, 6, 7, 8, 9, 10, 11, 12, 13, or 14. The caspase polypeptide may be a Caspase-9 polypeptide. The Caspase-9 polypeptide may comprise an amino acid sequence of a catalytically active (not catalytically dead) caspase variant polypeptide provided in Table 5 or 6 herein. The Caspase-9 polypeptide may consist of an amino acid sequence of a catalytically active (not catalytically dead) caspase variant polypeptide provided in Table 5 or 6 herein. A caspase polypeptide may be used that has a lower basal activity in the absence of the ligand inducer. For example, when included as part of a chimeric inducible caspase polypeptide, certain modified Caspase-9 polypeptides may have lower basal activity compared to wild type Caspase-9 in the chimeric construct. For example, the modified Caspase-9 polypeptide may comprise an amino acid sequence having at least 90% sequence identity to SEQ ID NO: 9, and may comprise at least one amino acid substitution.

### Brief Description of the Drawings

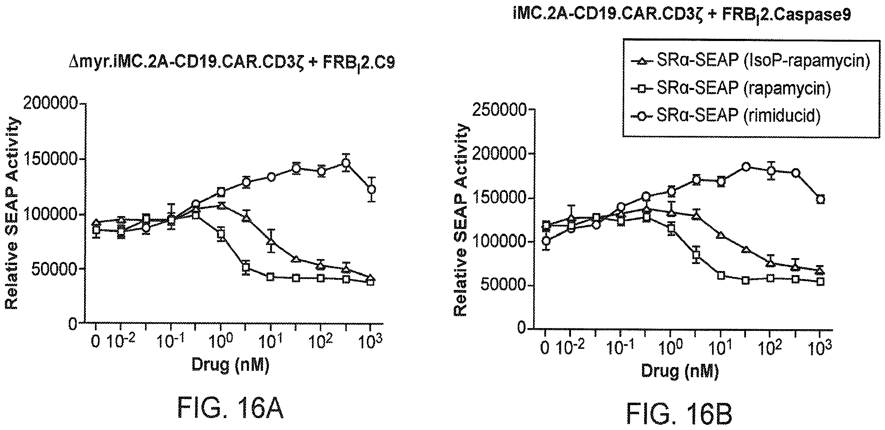

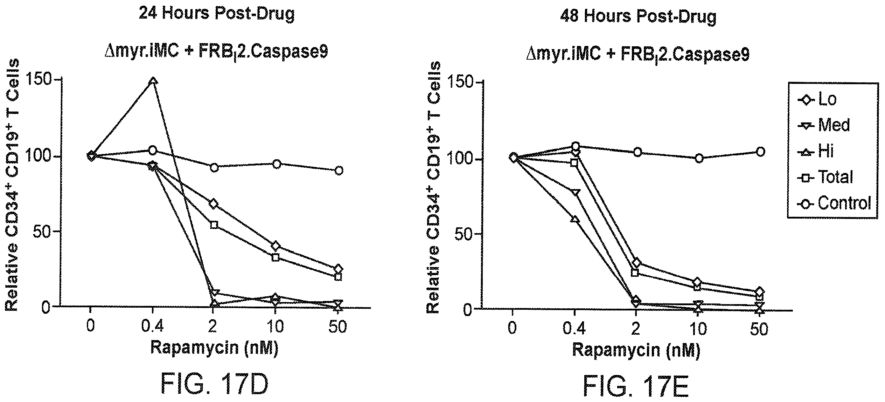

The drawings illustrate the technology described herein and are not limiting. For clarity and ease of illustration, the drawings are not made to scale and, in some instances, various aspects may be shown exaggerated or enlarged to facilitate an understanding of particular aspects.

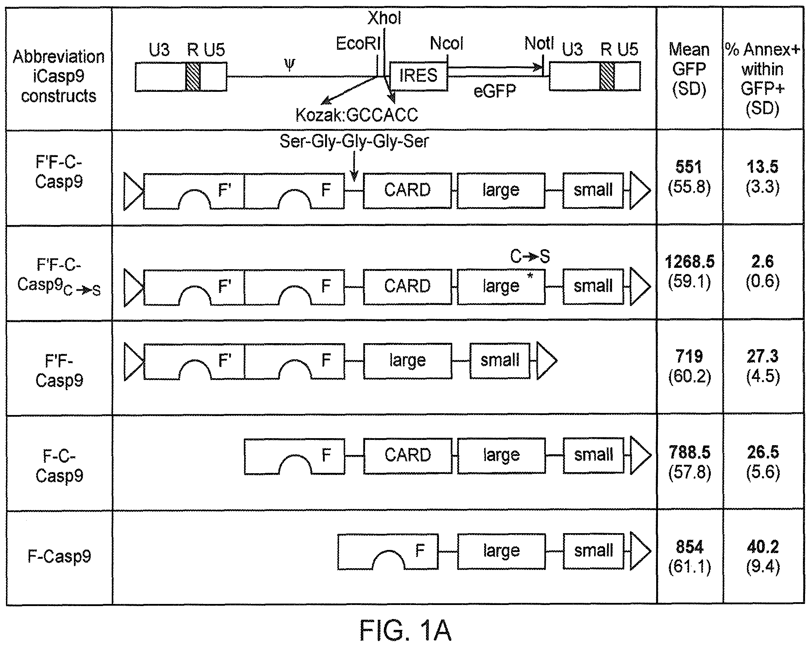

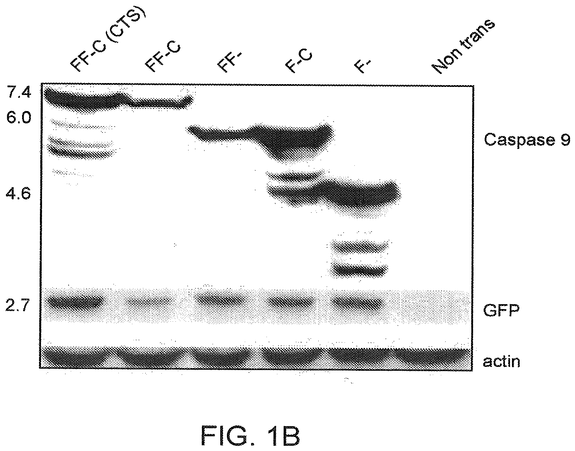

Fig. 1A illustrates various iCasp9 expression vectors as discussed herein. FIG. 1B illustrates a representative western blot of full length and truncated Caspase-9 protein produced by the expression vectors shown in FIG. 1A.

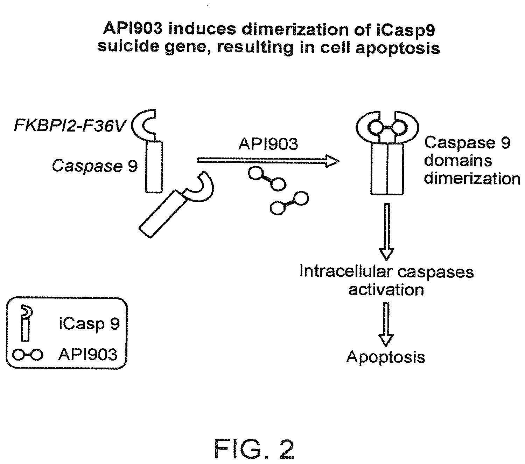

Fig. 2 is a schematic of the interaction of the suicide gene product and the CID to cause apoptosis.

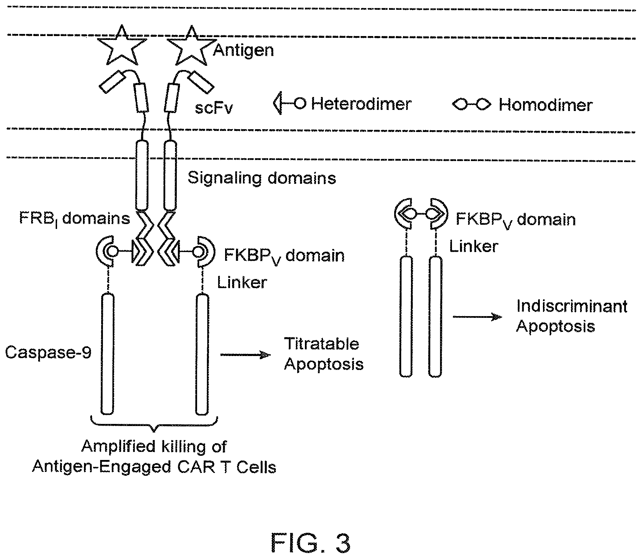

Fig. 3 is a schematic depicting a two-tiered regulation of apoptosis. The left section depicts rapalog-mediated recruitment of an inducible caspase polypeptide to FRBI-modified CAR. The right section depicts a rimiducid (AP1903)-mediated inducible caspase polypeptide.

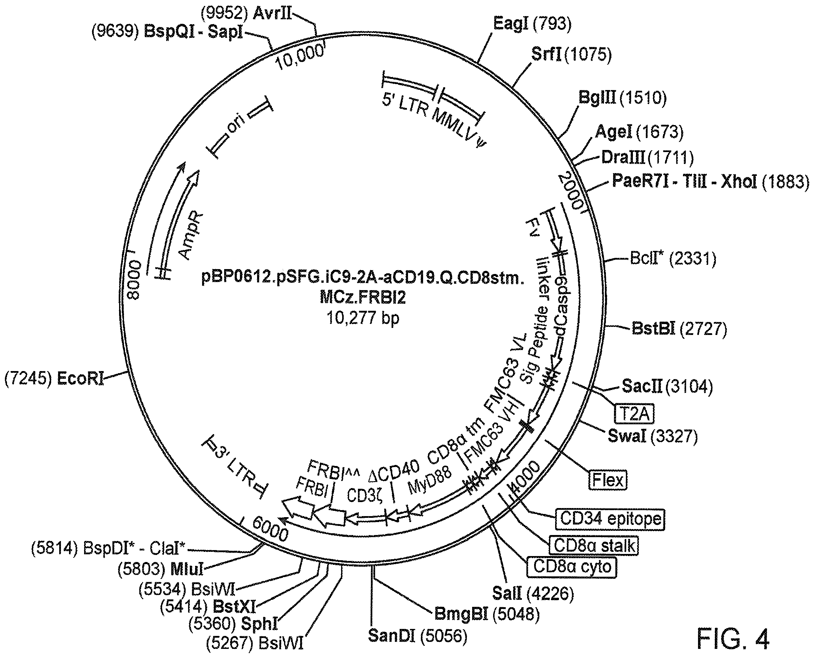

Fig. 4 is a plasmid map of a vector encoding FRB_{L}-modified CD19-MC-CAR and inducible Caspase-9. pSFG-iCasp9-2A-CD19-Q-CD28stm-MCz-FRB_{L}2.

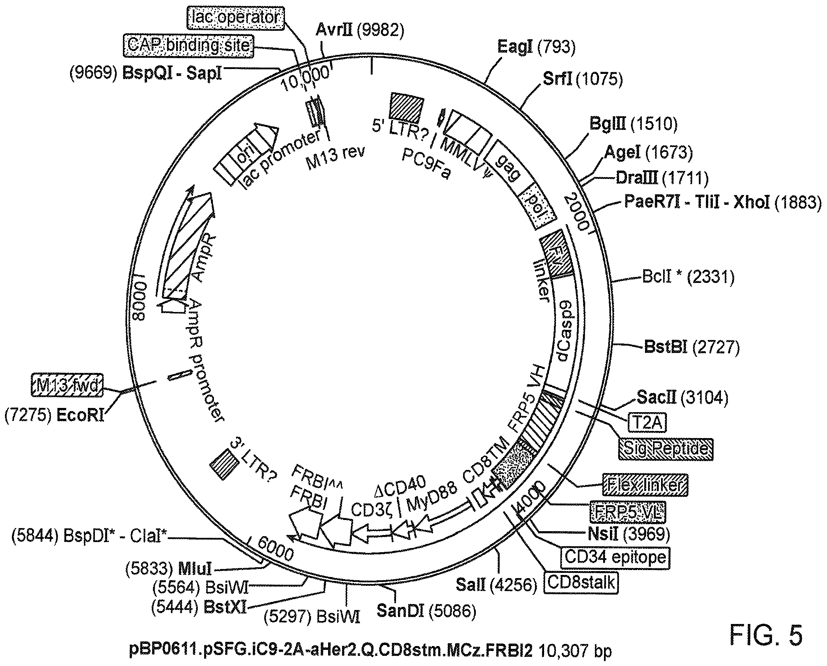

Fig. 5 is a plasmid map of a vector encoding FRB_{L}-modified Her2-MC-CAR and an inducible Caspase-9 polypeptide. pSFG-iCasp9-2A-aHer2-Q_CD28stm-mMCz-FRB_{L}2.

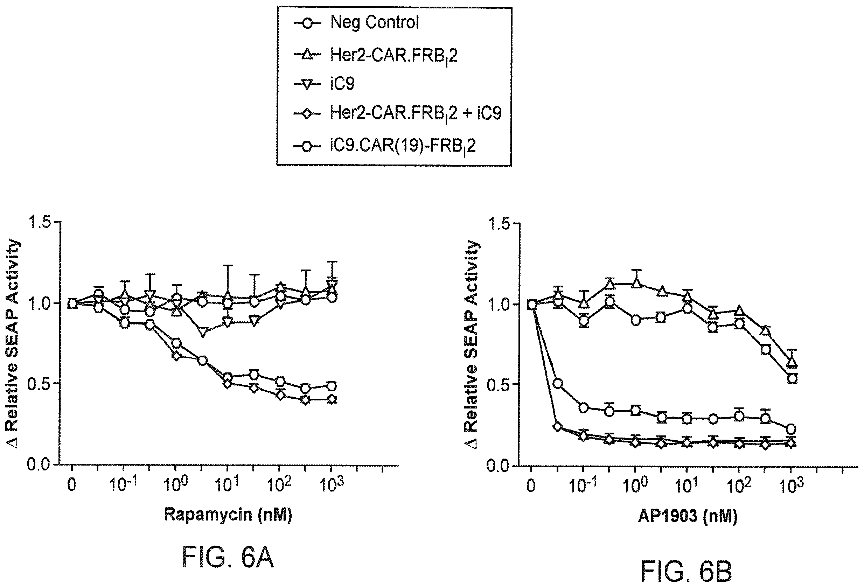

Figs. 6A and 6B provide the results of an assay of two-tiered activation of apoptosis. Fig. 6A shows recruitment of an inducible Caspase-9 polypeptide (iC9) with rapamycin, leading to more gradual apoptosis titration. Fig. 6B shows complete apoptosis using rimiducid (AP1903).

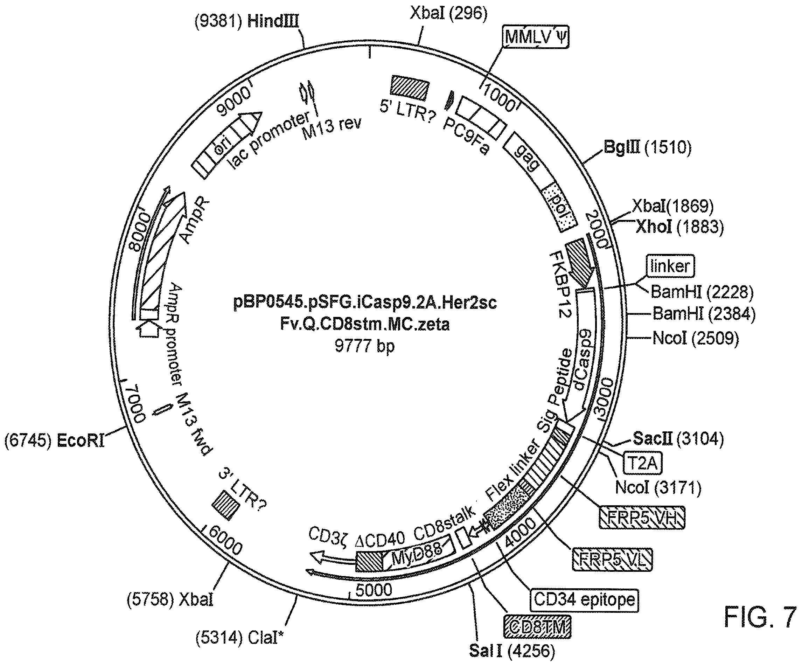

Fig. 7 is a plasmid map of the pBP0545 vector, pBP0545.pSFG.iCasp9.2A.Her2scFv.Q.CD8stm.MC-zeta.

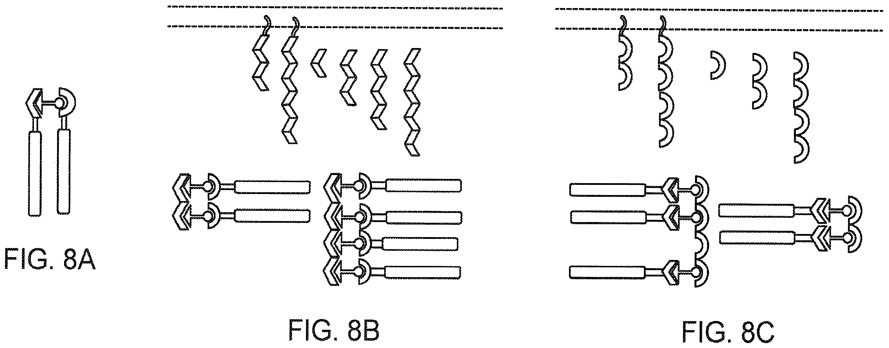

Figs. 8A-8C illustrate that FRB or FKBP12-based scaffolds can multimerize signaling domains. Fig. 8A. Homodimerization of a signaling domain (red stick), like Caspase-9, can be achieved via a heterodimer that binds to the FRB-fused signaling domain on one side and FKBP12-fused domain on the other. Fig. 8B. Dimerization or multimerization of a signaling domain via 2 (left) or more (right) tandem copies of FRB (chevron). The scaffold can contain subcellular targeting sequences to localize proteins to the plasma membrane (as depicted), the nucleus or organelles. Fig. 8C. Similar to Fig. 8B, but domain polarity is reversed.

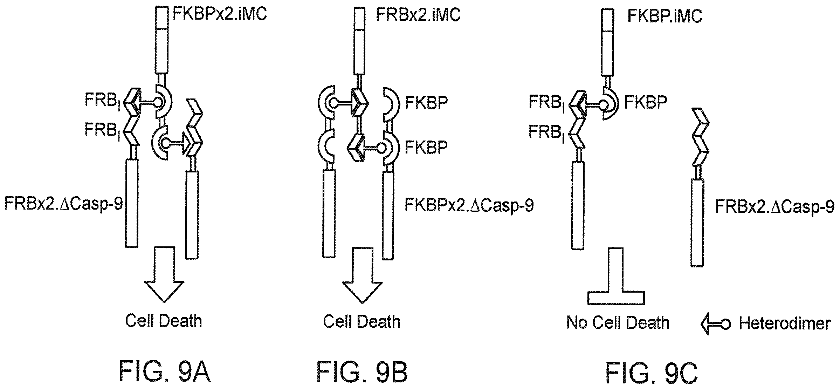

Figs. 9A-9C provide schematics of iMC-mediated scaffolding of FRB_{L}2.Caspase-9. Fig. 9A. In the presence of a heterodimer drug, such as a rapamycin, the FRB_{L}2-linked Caspase-9 binds with and clusters the FKBP-modified MyD88/CD40 (MC) signaling molecule. This clustering effect results in dimerization of FRB_{L}2.Caspase-9 and subsequent induction of cellular death via the apoptotic pathway. Fig. 9B. Similar to panel 9A, however the FKBP and FRB domains have been switched in relation to associated Caspase-9 and MC domains. The clustering effect still occurs in the presence of heterodimer drug. Fig. 9C. Similar to panel 9A; however there is only one FKBP domain attached to MC. Therefore, in the presence of heterodimer, Caspase-9 is no longer capable of being clustered and therefore apoptosis is not induced.

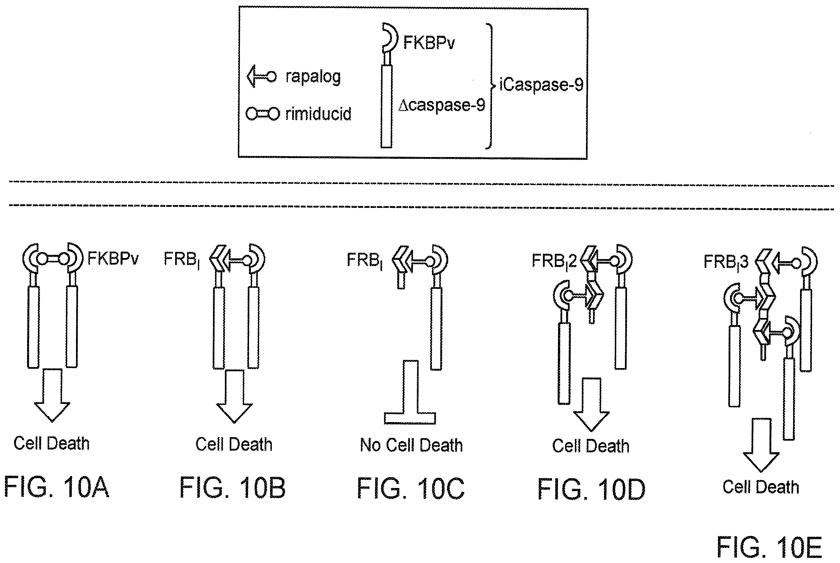

Fig. 10A-10E provide schematics of a rapalog-induced, FRB scaffold-based CaspaCIDe. Fig. 10A: Rimiducid homodimerizes FKBPv-linked Caspase-9, resulting in dimerization and activation of Caspase-9 with subsequent induction of cellular death via the apoptotic pathway. Fig. 10B: Rapalogs heterodimerize FKBPv-linked Caspase-9 with FRB-linked Caspase-9, resulting in dimerization of Caspase-9 and cell death. Fig. 10C, Fig. 10D, Fig. 10E are schematics illustrating that in the presence of a heterodimer drug, such as a rapalog, 2 or more FRB_{L} domains act as a scaffold to recruit binding of FKBPv-linked Caspase-9, leading to dimerization or oligomerization of Caspase-9 and cell death.

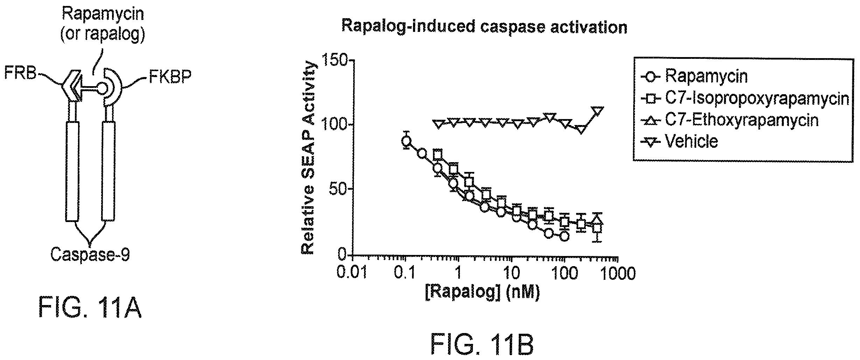

Fig. 11A is a schematic and Figure 11B is a line graph depicting activation of apoptosis by dimerization of a chimeric FRB-Caspase-9 polypeptide and a chimeric FKBP-Caspase-9 polypeptide (FRB_{L}-ΔCaspase-9 and FKBPv-ΔCaspase-9) with rapamycin. Fig. 11A. Schematic representation of dimerization of FRB and FKBP12 with rapamycin to bring together fused Caspase-9 signaling domains and activation of apoptosis. Fig. 11B. Reporter assays were performed in HEK-293T cells transfected with the constitutive SRα-SEAP reporter (pBP046, 1 µg), a fusion of FRB_{L} (L2098) and human ΔCaspase-9 (pBP0463, 2 µg) and a fusion of FKBP12 with ΔCaspase-9 (pBP0044, 2 µg).

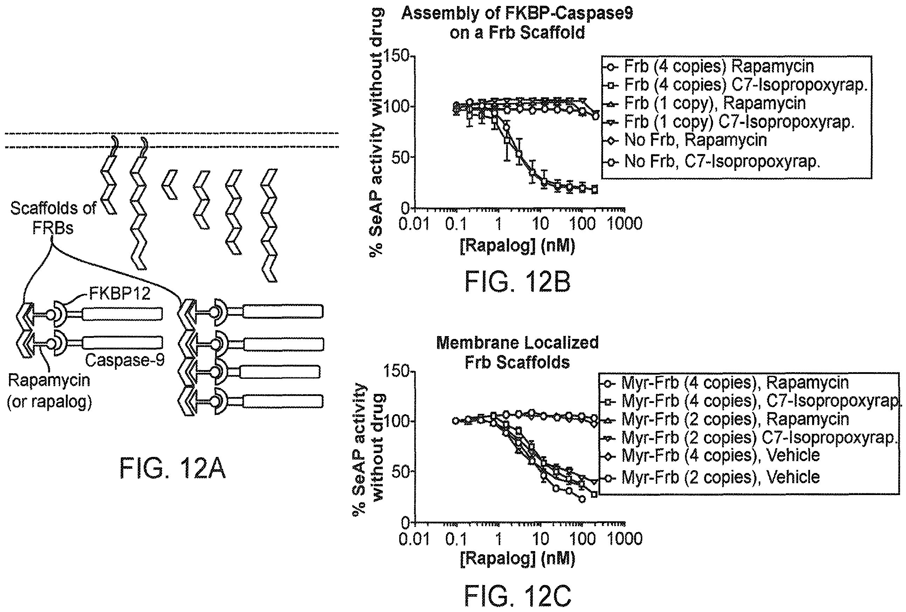

Fig. 12A is a schematic and Figs. 12B and 12C are line graphs depicting assembly of FKBP-Caspase-9 on a FRB-based scaffold. Fig. 12A: Schematic of iterated FRB domains to provide scaffolds for rapamycin (or rapalog)-mediated multimerization of an FKBP12-Caspase-9 fusion protein. Fig. 12B: Cultures of HEK-293 cells were transfected (via Genejuice, Novagen) with the constitutive SRα-SEAP reporter plasmid (pBP0046, 1 µg), a fusion of human FKBP12 with human Caspase-9 (pBP0044, 2 µg) and FRB-encoding expression constructs, containing four copies of FRB_{L} (pBP0725, 2 µg) or control vectors encoding zero or one copy of FRB_{L}. 24 hours post-transfection, cells were distributed into 96-well plates and rapamycin or a derivative rapalog, C7-isopropoxyrapamycin, with specificity for the mutant FRB_{L} (Liberles et al, 1997) were administered in triplicate wells. Placental SEAP reporter activity was determined 24 hours post-drug administration. Fig. 12C: Reporter assays were performed as in (B), but FRB-scaffolds were expressed from constructs encoding iterated FRB_{L} domains with an amino-terminal myristoylation-targeting sequence and two (pBP0465) or four copies (pBP0721) of the FRB_{L} domain.

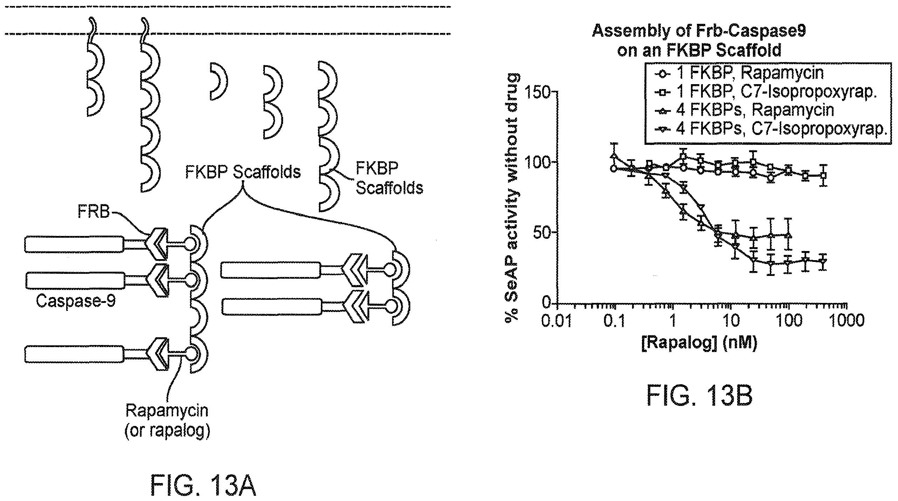

Fig. 13A is a schematic and Fig. 13B is a line graph depicting assembly of FRB-ΔCaspase-9 on an FKBP scaffold. Fig. 13A. Schematic of iterated FKBP12 domains to produce scaffolds for assembly of rapamycin (or rapalog)-mediated multimerization of FRB-ΔCaspase-9 fusion protein, leading to apoptosis. Fig. 13B. Reporter assays were performed as in Fig.s 12B and C with cultures of HEK-293T cells transfected with the constitutive SRα-SEAP reporter (pBP046, 1 µg), a fusion of FRB_{L} (L2098) and CARD domain-deleted human ΔCaspase-9 (pBP0463, 2 µg) and FKBP expression constructs containing four tandem copies of FKBP12 (pBP722, 2 µg) or a control vector with one copy of FKBP (pS-SF1E).

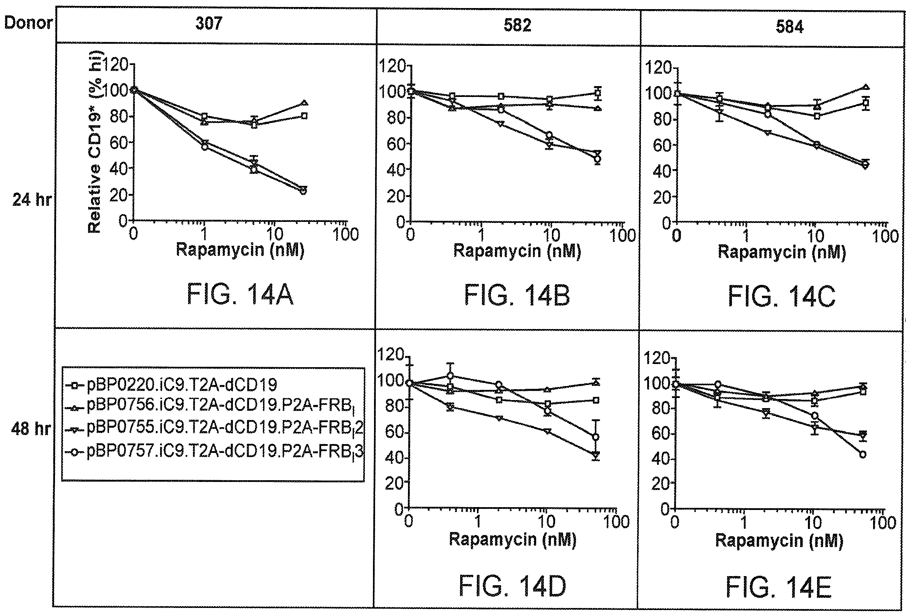

Figs. 14A-14B provide line graphs showing that heterodimerization of FRB_{L} scaffold with iCaspase9 induces cell death. Primary T cells from three different donors (307, 582, 584) were transduced with pBP0220-pSFG-iC9.T2A-ΔCD19, pBP0756-pSFG-iC9.T2A-ΔCD19.P2A-FRB_{L}, pBP0755-pSFG-iC9.T2A-ΔCD19.P2A-FRB_{L}2, or pBP0757-pSFG-iC9.T2A-ΔCD19.P2A-FRB_{L}3, containing CaspaCIDe, CD19 marker, and 0-3 tandem copies of FRB_{L}, respectively. T Cells were plated with varying concentrations of rapamycin and after 24 and 48 hours cell aliquots were harvested, stained with APC-CD19 antibody and analyzed by flow cytometry. Cells were initially gated on live lymphocytes by FSC vs SSC. Lymphocytes were then plotted as a CD19 histogram and subgated for high, medium and low expression within the CD19⁺ gate. Line graphs represent the relative percentage of the total cell population that express high levels of CD19, normalized to the no "0" drug control. All data points were done in duplicates. Fig. 14A : donor 307, 24 hr; Fig. 14B: donor 582, 24 hr; Fig. 14C: donor 584 24 hr; Fig. 14D: donor 582 48 hr; Fig. 14E: donor 584 48 hr.

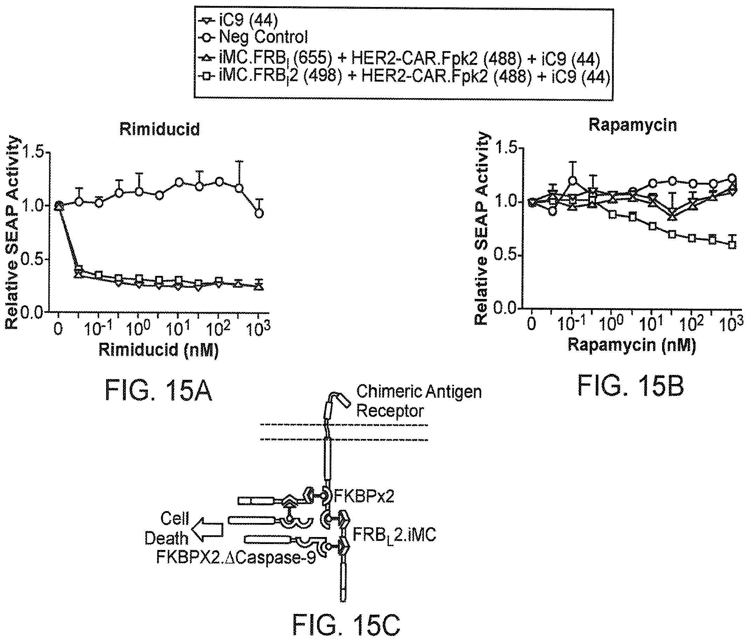

Figs. 15A-15C provide line graphs and a schematic showing that rapamycin induces CaspaCIDe killing in the presence of tandem FRB_{L} domains. HEK-293 cells were transfected with 1 µg of SRα-SEAP constitutive reporter plasmid along with either negative (Neg) control, eGFP (pBP0047), CaspaCIDe (iC9/pBP0044) alone, or CaspaCIDe along with iMC.FRB_{L} (pBP0655) + anti-HER2.CAR.Fpk2 (pBP0488) or iMC.FRB_{L}2 (pBP0498) + anti-HER2.CAR.Fpk2. Cells were then plated with half-log dilutions of rimiducid or rapamycin and assayed for SEAP as previously described. Diminution of SEAP activity correlates with cell elimination. Schematic represents one possible rapamycin-mediated complex of signaling domains, which lead to Caspase-9 clustering and apoptosis. Fig. 15A: rimiducid; Fig. 15B: rapamycin; Fig. 15C: schematic.
Fig.s 16A and 16B are line graphs showing that tandem FKBP scaffold mediates FRB_{L}2.Caspase activation in the presence of rapalogs. Fig. 16A. HEK-293 cells were transfected with 1 µg each of SRα-SEAP reporter plasmid, Δmyr.iMC.2A-anti-CD19.CAR.CD3ζ (pBP0608), and FRB_{L}2.Caspase-9 (pBP0467). After 24 hours, transfected cells were harvested and treated with varying concentrations of either rimiducid, rapamycin, or rapalog, C7-isopropoxy (IsoP)-rapamycin. After ON incubation, cell supernatants were assayed for SEAP activity, as previously described. Fig. 16B. Similar to the experiment described in (Fig. 16A), except that cells were transfected with a membrane-localized (myristoylated) iMC.2A-CD19.CAR.CD3ζ (pBP0609), instead of non-myristoylated Δmyr.iMC.2A-CD19.CAR.CD3ζ (pBP0608).

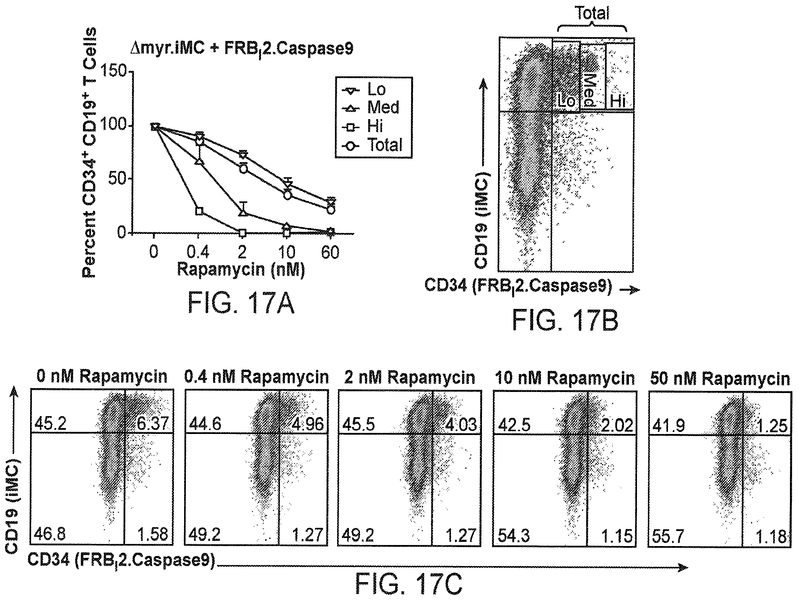

Figs. 17A-17E provides line graphs and the results of FACs analysis showing that the iMC "switch", FKBP2.MyD88.CD40, creates a scaffold for FRB_{L}2.Caspase9 in the presence of rapamycin, inducing cell death. Fig. 17A. Primary T cells (2 donors) were transduced with γ-RV, SFG-ΔMyr.iMC.2A-CD19 (from pBP0606) and SFG-FRB_{L}2.Caspase9.2A-Q.8stm.zeta (from pBP0668). Cells were plated with 5-fold dilutions of rapamycin. After 24 hours, cells were harvested and analyzed by flow cytometry for expression of iMC (anti-CD19-APC), Caspase-9 (anti-CD34-PE), and T cell identity (anti-CD3-PerCPCy5.5). Cells were initially gated for lymphocyte morphology by FSC vs SSC, followed by CD3 expression (-99% of the lymphocytes). CD3⁺ lymphocytes were plotted for CD19 (Δmyr.iMC.2A-CD19) vs CD34 (FRB_{L}2.Caspase9.2A-Q.8stm.zeta) expression.
   To normalize gated populations, percentages of CD34⁺CD19⁺ cells were divided by percent CD19⁺CD34⁻ cells within each sample as an internal control. Those values were then normalized to drug free wells for each transduction which were set at 100%. Similar analysis was applied to the Hi-, Med-, and Lo-expressing cells within the CD34⁺CD19⁺ gate. Fig. 17B. Representative example of how cells were gated for Hi, Med, and Lo expression. Fig. 17C. Representative scatter plots of final CD34 vs CD19 gates. As rapamycin increased, % CD34⁺CD19⁺ cells decreased, indicating elimination of cells. Fig. 17D and Fig. 17E. T cells from a single donor were transduced with ΔMyr.iMC.2A-CD19 (pBP0606) or FRB_{L}2.Caspase9.2A-Q.8stm.zeta (pBP0668). Cells were plated in IL-2-containing media along with varying amounts of rapamycin for 24 or 48 hrs. Cells were then harvested and analyzed, as above.

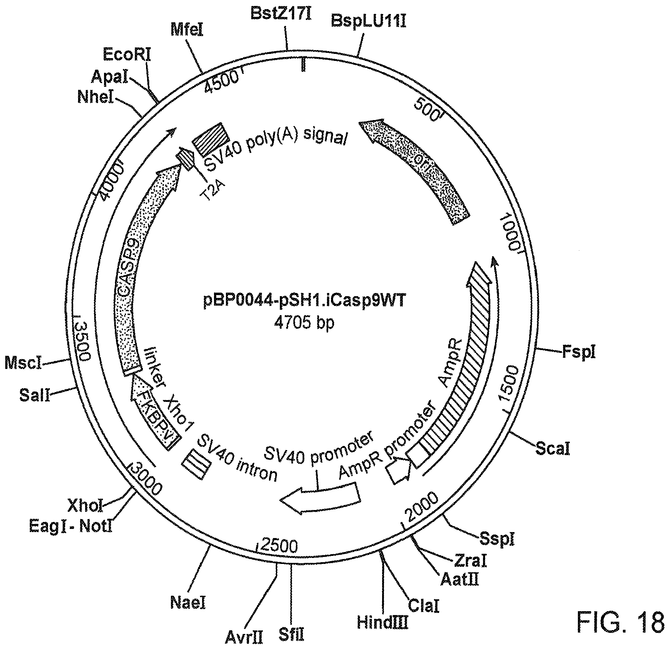

Fig. 18 Plasmid map of pBP0044: pSH1-iCaspase9wt.
Fig. 19 Plasmid map of pBP0463--pSH1-Fpk-Fpk'.LS.Fpk".Fpk"'.LS.HA.

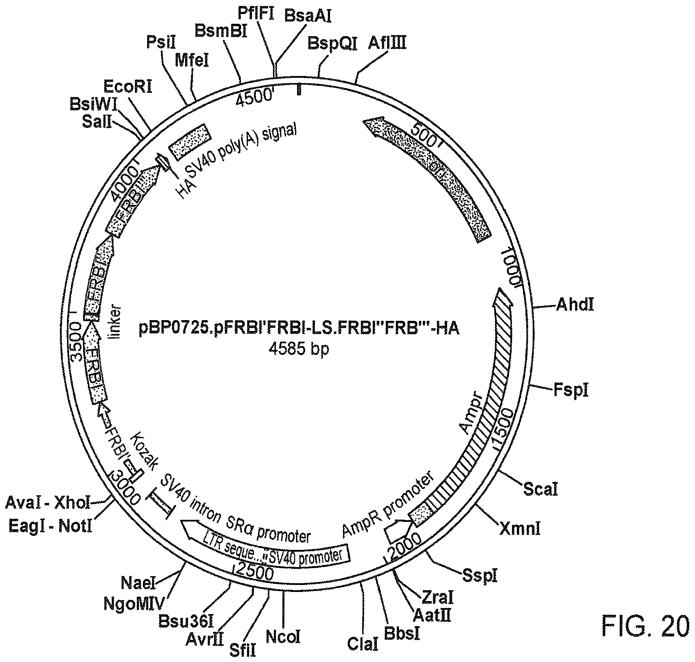

Fig. 20 Plasmid map of pBP0725--pSH1-FRBI.FRBI'.LS.FRBI".FRBI".'

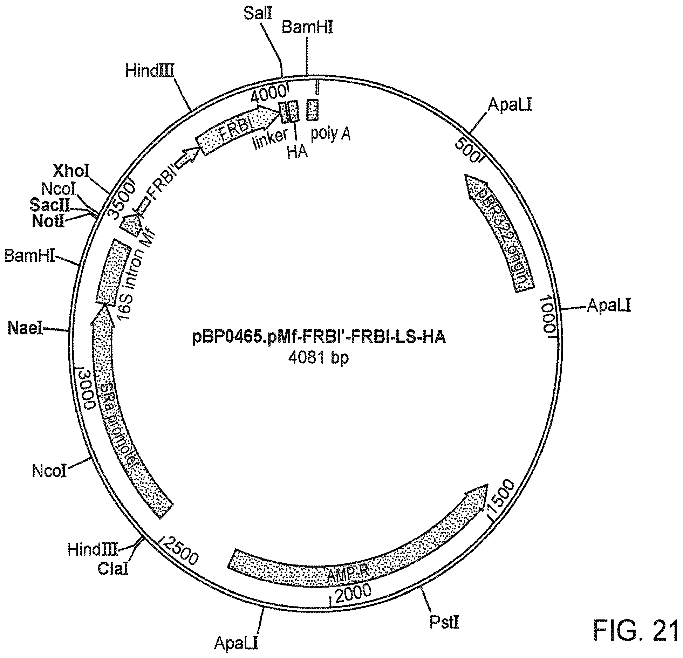

Fig. 21 Plasmid map of pBP0465--pSH1-M-FRBI.FRBI'.LS.HA.

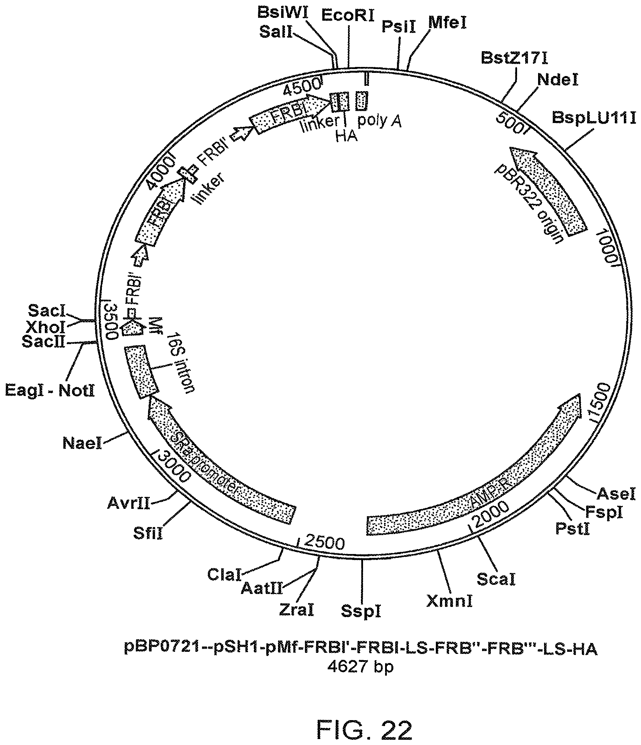

Fig. 22 Plasmid map of pBP0721--pSH1-M-FRBI.FRBI'.LS.FRBI".FRBI'"HA.

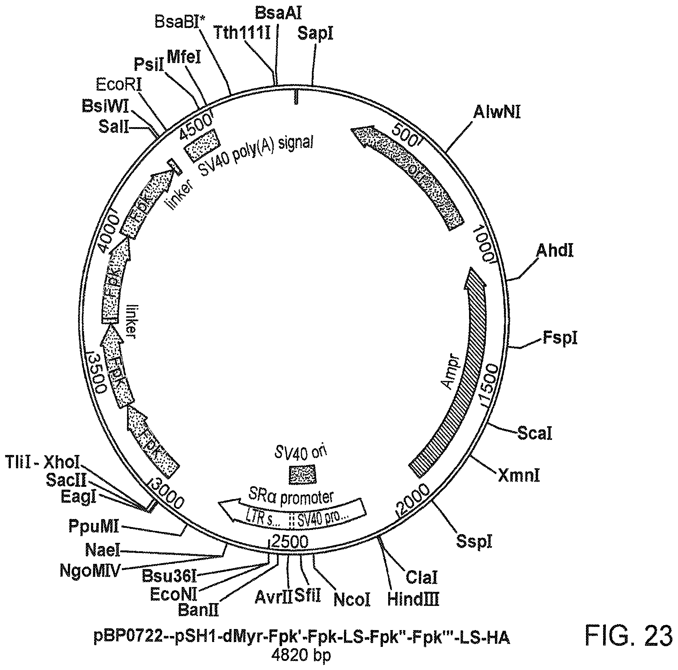

Fig. 23 Plasmid map of pBP0722--pSH1-Fpk-Fpk'.LS.Fpk".Fpk"'.LS.HA.
Fig. 24 Plasmid map of pBP0220--pSFG-iC9.T2A-ΔCD19.

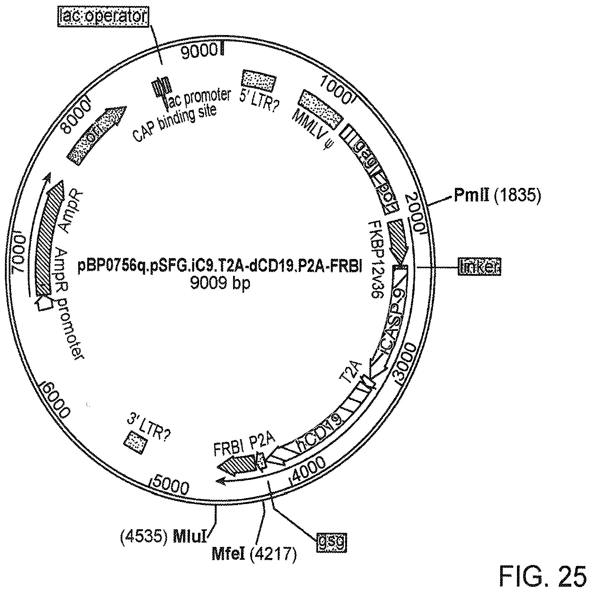

Fig. 25 Plasmid map of pBP0756--pSFG-iC9.T2A-dCD19.P2A-FRBI.

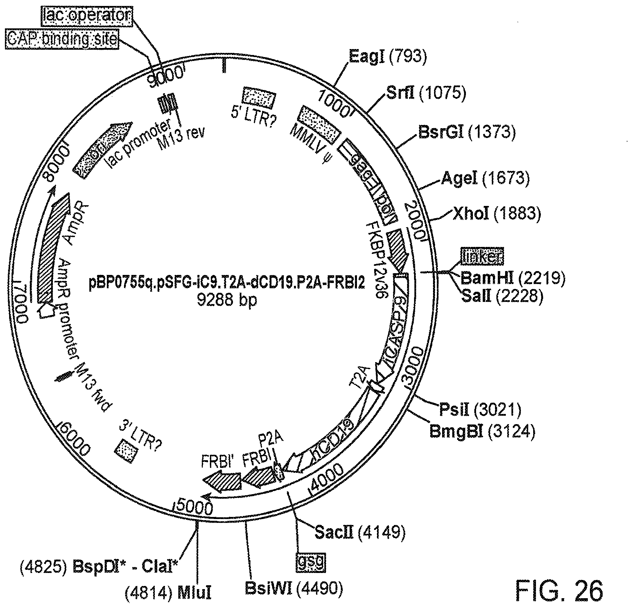

Fig. 26 Plasmid map of pBP0755--pSFG-iC9.T2A-dCD19.P2A-FRBI2.
Fig. 27 Plasmid map of pBP0757--pSFG-iC9.T2A-dCD19.P2A-FRBI3.
Fig. 28 Plasmid map of pBP0655--pSFG-ΔMyr.FRBI.MC.2A-ΔCD19.
Fig. 29 Plasmid map of pBP0498--pSFG-ΔMyr.iMC.FRBI2.P2A-ΔCD19.
Fig. 30 Plasmid map of pBP0488--pSFG-aHER2.Q.8stm.CD3zeta.Fpk2.
Fig. 31 Plasmid map of pBP0467--pSH1-FRBI'.FRBI.LS.ΔCaspase9.
Fig. 32 Plasmid map of pBP0606--pSFG-k-AMyr.iMC.2A-ΔCD19.
Fig. 33 Plasmid map of pBP0607--pSFG-k-iMC.2A-ACD19
Fig. 34 Plasmid map of pBP0668--pSFG-FRBIx2.Caspase9.2A-Q.8stm.CD3zeta.

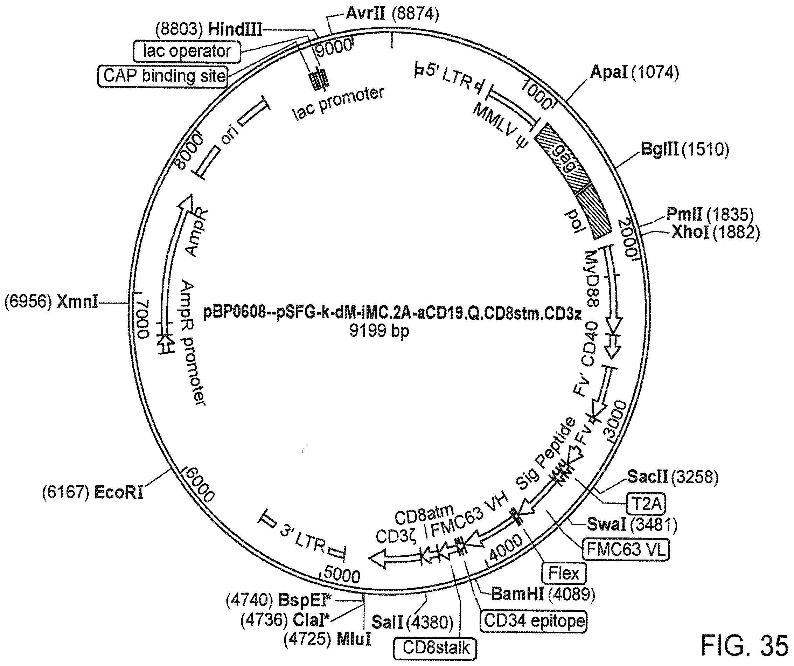

Fig. 35 Plasmid map of pBP0608--pSFG-AMyr.iMC.2A-ΔCD19.Q.8stm.CD3zeta.

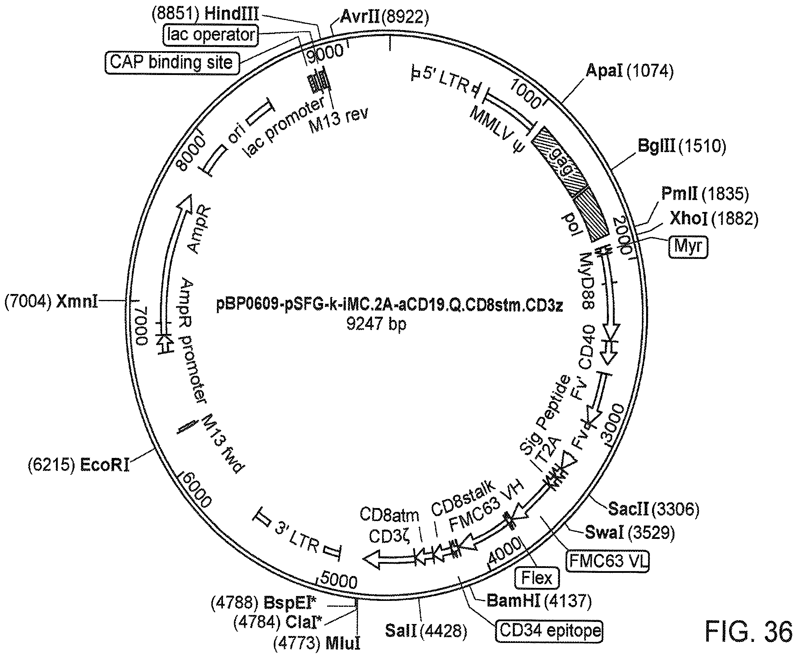

Fig. 36 Plasmid map of pBP0609: pSFG-iMC.2A-ΔCD19.Q.8stm.CD3zeta.
Fig. 37A provides a schematic of rimiducid binding to two copies of a chimeric Caspase-9 polypeptide, each having a FKBP12 multimerizing region. Fig. 37B provides a schematic of rapamycin binding to two chimeric Caspase-9 polypeptides, one of which has a FKBP12 multimerizing region and the other which has a FRB multimerizing region. Fig. 37C provides a graph of assay results using these chimeric polypeptides.
Fig. 38A provides a schematic of rapamycin or rapalog binding to two chimeric Caspase-9 polypeptides, one of which has a FKBP12v36 multimerizing region and the other which has a FRB variant (FRB_{L}) multimerizing region. Fig. 38B provides a graph of assay results using this chimeric polypeptide.
Fig. 39A provides a schematic of rimiducid binding to two chimeric Caspase-9 polypeptides, each of which has a FKBP12v36 multimerizing region, and rapamycin binding to only one chimeric Caspase-9 polypeptide having a FKBP12v36 multimerizing region. Fig. 39B provides a graph of assay results comparing the effects of rimiducid and rapamycin.
Fig. 40A provides a schematic of rimiducid binding to two chimeric Caspase-9 polypeptides, each of which has a FKBP12v36 multimerizing region, and rapamycin binding to only one chimeric Caspase-9 polypeptide having a FKBP12v36 multimerizing region in the presence of a FRB multimerization polypeptide. Fig. 40B provides a graph of assay results using these polypeptides, comparing the effects of rimiducid and rapamycin.
Fig. 41 provides a plasmid map of pBP0463.pFRBI.LS.dCasp9.T2A.
Fig. 42 provides a plasmid map of pBP044-pSH1.iCasp9WT.

### Detailed Description

As a mechanism to translate information from the external environment to the inside of the cell, regulated protein-protein interactions evolved to control most, if not all, signaling pathways. Transduction of signals is governed by enzymatic processes, such as amino acid side chain phosphorylation, acetylation, or proteolytic cleavage that lack intrinsic specificity. Furthermore, many proteins or factors are present at cellular concentrations or at subcellular locations that preclude spontaneous generation of a sufficient substrate/product relationship to activate or propagate signaling. An important component of activated signaling is the recruitment of these components to signaling "nodes" or spatial signaling centers that efficiently transmit (or attenuate) the pathway via appropriate upstream signals.

As a tool to artificially isolate and manipulate individual protein-protein interactions and hence individual signaling proteins, chemically induced dimerization (CID) technology was developed to impose homotypic or heterotypic interactions on target proteins to reproduce natural biological regulation. In its simplest form, a single protein would be modified to contain one or more structurally identical ligand binding domains, which would then be the basis of homodimerization or oligomerization, respectively, in the presence of a cognate homodimeric ligand (Spencer DM et al (93) Science 262, 1019-24). A slightly more complicated version of this concept would involve placing one or more distinct ligand binding domains on two different proteins to enable heterodimerization of these signaling molecules using small molecule, heterodimeric ligands that bind to both distinct domains simultaneously (Ho SN et al (96) Nature 382, 822-6). This drug-mediated dimerization creates a very high local concentration of ligand binding-domain-tagged components sufficient to permit their induced or spontaneous assembly and regulation.

Also described herein are methods to induce multimerization of proteins. In this case, two or more heterodimer ligand binding regions (or "domains") in tandem are used as a "molecular scaffold" to dimerize or oligomerize a second, signaling domain-containing protein that is fused to one or more copies of the second binding site for the heterodimeric ligand. The molecular scaffold can be expressed as an isolated multimer of ligand binding domains (Fig. 8), either localized within the cell or unlocalized (Fig. 8B, 8C), or it can be attached to another protein that provides a structural, signaling, cell marking, or more complex combinatorial function (Fig. 9). By "scaffold" is meant a polypeptide that comprises at least two, for example, two or more, heterodimer ligand binding regions; in certain examples the ligand binding regions are in tandem, that is, each ligand binding region is located directly proximal to the next ligand binding region. In other examples, each ligand binding region may be located close to the next ligand binding region, for example, separated by about 1, 2, 3, 4, 5, 6, 7, 8, 9, 10, 11, 12, 13, 14, 15, 16,17, 18, 19, 20, 25, 30, 35, 40, 45, 50, 55, 60, 65, 70, 75, 80 or more amino acids, but retain the scaffold function of dimerization of an inducible caspase molecule in the presence of a dimerizer. A scaffold may comprise, for example, at least 2, 3, 4, 5, 6, 7, 8, 9, 10, 11, 12,13, 14,15, 16, 17, 18, 19, 20 or more ligand binding regions, and may also be linked to another polypeptide, such as, for example, a marker polypeptide, a costimulating molecule, a chimeric antigen receptor, a T cell receptor, or the like.
The first polypeptide may consist essentially of at least two, three, four, five, six, seven, eight, nine, ten, 11, 12, 13, 14, 15, 16, 17, 18, 19, or 20 units of the first multimerizing region. First polypeptide may consist essentially of the scaffold region. The first polypeptide may consist essentially of a membrane association region or a membrane targeting region. By "consists essentially of" is meant that the scaffold units or the scaffold may be alone, can optionally include linker polypeptides at either terminus of the scaffold, or between the units, and can optionally include small polypeptides such as, for example stem polypeptides as shown in Figs. 10B, 10C, 10D, and 10E.

In one example, a tandem multimer of the ∼ 89 aa FK506-rapamycin binding (FRB) domain derived from the protein kinase mTOR (Chen J et al (95) PNAS, 92, 4947-51) is used to recruit multiple FKBPv36-fused Caspase-9 (CaspaCIDe/iCaspase-9) in the presence of rapamycin or a rapamycin-based analogue ("rapalog") (Liberles SD (97) PNAS 94, 7825-30; Rivera VM (96) Nat Med 2, 1028-1032, Stankunas K (03) Mol Cell 12, 1615-24; Bayle JH (06) Chem & Biol, 13, 99-107) (Figs. 1-3). This recruitment leads to spontaneous caspase dimerization and activation.

In a second example, the tandem FRB domains are fused to a chimeric antigen receptor (CAR) and this provides rapalog-driven CaspaCIDe activation to cells expressing both fusion proteins (Fig. 15, inset).

In a third example, the polarity of the two proteins are reversed so that two or more copies of FKBP12 are used to recruit and multimerize FRB-modified signaling molecules in the presence of rapamycin (Fig. 8C, 9A).

In some examples, a chimeric polypeptide may comprise a single ligand binding region, or a scaffold comprising more than one ligand binding region may be, where the chimeric polypeptide comprises a polypeptide such as, for example, a MyD88 polypeptide, a truncated MyD88 polypeptide, a cytoplasmic CD40 polypeptide, a chimeric MyD88/cytoplasmic CD40 polypeptide or a chimeric truncated MyD88/cytoplasmic CD40 polypeptide:
In a fourth example, unstable FRB variants (e.g., FRBL2098) are used to destabilize the signaling molecule prior to rapalog administration (Stankunas K (03) Mol Cell 12, 1615-24; Stankunas K (07) ChemBioChem 8, 1162-69) (Fig. 9, 10). Following rapalog exposure, the unstable fusion molecule is stabilized leading to aggregation as before, but with lower background signaling.

The use of ligands to direct signaling proteins may be generally applied to activate or attenuate many signaling pathways. Examples are provided herein that demonstrate a utility of the approach by controlling apoptosis or programmed cell death with the "initiating caspase", Caspase-9 as the primary target. Control of apoptosis by dimerization of proapoptotic proteins with widely available rapamycin or more proprietary rapalogs, should permit an experimenter or clinician to tightly and rapidly control the viability of a cell-based implant that displays unwanted effects. Examples of these effects include, but are not limited to, Graft versus Host (GvH) immune responses against off-target tissue or excessive, uncontrolled growth or metastasis of an implant. Rapid induction of apoptosis will severely attenuate the unwanted cell's function and permit the natural clearance of the dead cells by phagocytic cells, such as macrophages, without undue inflammation.
Apoptosis is tightly regulated and naturally uses scaffolds, such as Apaf-1, CRADD/RAIDD, or FADD/Mort1, to oligomerize and activate the caspases that can ultimately kill the cell. Apaf-1 can assemble the apoptotic protease Caspase-9 into a latent complex that then forms an active oligomeric apoptosome upon recruitment of cytochrome C to the scaffold. The key event is oligomerization of the scaffold units causing dimerization and activation of the caspase. Similar adapters, such as CRADD, can oligomerize Caspase-2, leading to apoptosis. The compositions and methods provided herein use, for example, multimeric versions of the ligand binding domains FRB or FKBP to serve as scaffolds that permit the spontaneous dimerization and activation of caspase units present as FRB or FKBP fusions upon recruitment with rapamycin.

Using certain of the methods provided in the examples herein, caspase activation occurs only when rapamycin or rapalogs are present to recruit the FRB or FKBP-fused caspase to the scaffold. In these methods, the FRB or FKBP polypeptides must be present as a multimeric unit not as monomers to drive FKBP- or FRB-caspase dimerization (except when FRB-Caspase-9 is dimerized with FKBP-Caspase-9). The FRB or FKBP-based scaffold can be expressed in a targeted cell as a fusion with other proteins and retains its capacity to serve as a scaffold to assemble and activate proapoptotic molecules. The FRB or FKBP scaffold may be localized within the cytosol as a soluble entity or present in specific subcellular locales, such as the plasma membrane through targeting signals. The components used to activate apoptosis and the downstream components that degrade the cell are shared by all cells and across species. With regard to Caspase-9 activation, these methods can be broadly utilized in cell lines, in normal primary cells, such as, for example, but not limited to, T cells, or in cell implants.

In certain examples of the direct dimerization of FRB-Caspase with FKBP-Caspase with rapamycin to direct apoptosis, it was shown that FKBP-fused Caspases can be dimerized by homodimerizer molecules, such as AP1510, AP20187 or AP1903 (Fig. 6 (right panel), 10A (schematic) (A similar proapototic switch can be directed via heterodimerization of a binary switch using rapamycin or rapalogs by coexpression of a FRB-Caspase-9 fusion protein along with FKBP-Caspase-9, leading to homodimerization of the caspase domains within the chimeric proteins (Fig. 8A (schematic), 10B (schematic), (11).

As used herein, the use of the word "a" or "an" when used in conjunction with the term "comprising" in the claims and/or the specification may mean "one," but it is also consistent with the meaning of "one or more," "at least one," and "one or more than one." Still further, the terms "having", "including", "containing" and "comprising" are interchangeable and one of skill in the art is cognizant that these terms are open ended terms.

The term "allogeneic" as used herein, refers to HLA or MHC loci that are antigenically distinct.

Thus, cells or tissue transferred from the same species can be antigenically distinct. Syngeneic mice can differ at one or more loci (congenics) and allogeneic mice can have the same background.

The term "antigen" as used herein is defined as a molecule that provokes an immune response. This immune response may involve either antibody production, or the activation of specific immunologically-competent cells, or both.

An "antigen recognition moiety" may be any polypeptide or fragment thereof, such as, for example, an antibody fragment variable domain, either naturally-derived, or synthetic, which binds to an antigen. Examples of antigen recognition moieties include, but are not limited to, polypeptides derived from antibodies, such as, for example, single-chain variable fragments (scFv), Fab, Fab', F(ab')2, and Fv fragments; polypeptides derived from T Cell receptors, such as, for example, TCR variable domains; and any ligand or receptor fragment that binds to the extracellular cognate protein .

The term "cancer" as used herein is defined as a hyperproliferation of cells whose unique trait-loss of normal controls-results in unregulated growth, lack of differentiation, local tissue invasion, and metastasis. Examples include but are not limited to, melanoma, non-small cell lung, small-cell lung, lung, hepatocarcinoma, leukemia, retinoblastoma, astrocytoma, glioblastoma, gum, tongue, neuroblastoma, head, neck, breast, pancreatic, prostate, renal, bone, testicular, ovarian, mesothelioma, cervical, gastrointestinal, lymphoma, brain, colon, sarcoma or bladder.

Donor: The term "donor" refers to a mammal, for example, a human, that is not the patient recipient. The donor may, for example, have HLA identity with the recipient, or may have partial or greater HLA disparity with the recipient.

Haploidentical: The term "haploidentical" as used with reference to cells, cell types and/or cell lineages, herein refers to cells sharing a haplotype or cells having substantially the same alleles at a set of closely linked genes on one chromosome. A haploidentical donor does not have complete HLA identity with the recipient, there is a partial HLA disparity.

Blood disease: The terms "blood disease", "blood disease" and/or "diseases of the blood" as used herein, refers to conditions that affect the production of blood and its components, including but not limited to, blood cells, hemoglobin, blood proteins, the mechanism of coagulation, production of blood, production of blood proteins, the like and combinations thereof. Non-limiting examples of blood diseases include anemias, leukemias, lymphomas, hematological neoplasms, albuminemias, haemophilias and the like.

Bone marrow disease: The term "bone marrow disease" as used herein, refers to conditions leading to a decrease in the production of blood cells and blood platelets. In some bone marrow diseases, normal bone marrow architecture can be displaced by infections (e.g., tuberculosis) or malignancies, which in turn can lead to the decrease in production of blood cells and blood platelets. Non-limiting examples of bone marrow diseases include leukemias, bacterial infections (e.g., tuberculosis), radiation sickness or poisoning, apnocytopenia, anemia, multiple myeloma and the like.

T cells and Activated T cells (include that this means CD3+ cells): T cells (also referred to as T lymphocytes) belong to a group of white blood cells referred to as lymphocytes. Lymphocytes generally are involved in cell-mediated immunity. The "T" in "T cells" refers to cells derived from or whose maturation is influenced by the thymus. T cells can be distinguished from other lymphocytes types such as B cells and Natural Killer (NK) cells by the presence of cell surface proteins known as T cell receptors. The term "activated T cells" as used herein, refers to T cells that have been stimulated to produce an immune response (e.g., clonal expansion of activated T cells) by recognition of an antigenic determinant presented in the context of a Class II major histo-compatibility (MHC) marker. T-cells are activated by the presence of an antigenic determinant, cytokines and/or lymphokines and cluster of differentiation cell surface proteins (e.g., CD3, CD4, CD8, the like and combinations thereof). Cells that express a cluster of differential protein often are said to be "positive" for expression of that protein on the surface of T-cells (e.g., cells positive for CD3 or CD 4 expression are referred to as CD3⁺ or CD4⁺). CD3 and CD4 proteins are cell surface receptors or co-receptors that may be directly and/or indirectly involved in signal transduction in T cells.

Peripheral blood: The term "peripheral blood" as used herein, refers to cellular components of blood (e.g., red blood cells, white blood cells and platelets), which are obtained or prepared from the circulating pool of blood and not sequestered within the lymphatic system, spleen, liver or bone marrow.

Umbilical cord blood: Umbilical cord blood is distinct from peripheral blood and blood sequestered within the lymphatic system, spleen, liver or bone marrow. The terms "umbilical cord blood", "umbilical blood" or "cord blood", which can be used interchangeably, refers to blood that remains in the placenta and in the attached umbilical cord after child birth. Cord blood often contains stem cells including hematopoietic cells.

By "cytoplasmic CD40" or "CD40 lacking the CD40 extracellular domain" is meant a CD40 polypeptide that lacks the CD40 extracellular domain. In some examples, the terms also refer to a CD40 polypeptide that lacks both the CD40 extracellular domain and a portion of, or all of, the CD40 transmembrane domain.
By "obtained or prepared" as, for example, in the case of cells, is meant that the cells or cell culture are isolated, purified, or partially purified from the source, where the source may be, for example, umbilical cord blood, bone marrow, or peripheral blood. The terms may also apply to the case where the original source, or a cell culture, hasbeen cultured and the cells have replicated, and where the progeny cells are now derived from the original source.

By "kill" or "killing" as in a percent of cells killed, is meant the death of a cell through apoptosis, as measured using any method known for measuring apoptosis, and, for example, using the assays discussed herein, such as, for example the SEAP assays or T cell assays discussed herein. The term may also refer to cell ablation.

Allodepletion: The term "allodepletion" as used herein, refers to the selective depletion of alloreactive T cells. The term "alloreactive T cells" as used herein, refers to T cells activated to produce an immune response in reaction to exposure to foreign cells, such as, for example, in a transplanted allograft. The selective depletion generally involves targeting various cell surface expressed markers or proteins, (e.g., sometimes cluster of differentiation proteins (CD proteins), CD19, or the like), for removal using immunomagnets, immunotoxins, flow sorting, induction of apoptosis, photodepletion techniques, the like or combinations thereof. In the present methods, the cells may be transduced or transfected with the chimeric protein-encoding vector before or after allodepletion. Also, the cells may be transduced or transfected with the chimeric protein-encoding vector without an allodepletion step, and the non-allodepleted cells may be administered to the patient. Because of the added "safety switch" it is, for example, possible to administer the non-allo-depleted (or only partially allo-depleted) T cells because an adverse event such as, for example, graft versus host disease, may be alleviated upon the administration of the multimeric ligand.

Graft versus host disease: The terms "graft versus host disease" or "GvHD", refer to a complication often associated with allogeneic bone marrow transplantation and sometimes associated with transfusions of un-irradiated blood to immunocompromised patients. Graft versus host disease sometimes can occur when functional immune cells in the transplanted marrow recognize the recipient as "foreign" and mount an immunologic response. GvHD can be divided into an acute form and a chronic form. Acute GVHD (aGVHD) often is observed within the first 100 days following transplant or transfusion and can affect the liver, skin, mucosa, immune system (e.g., the hematopoietic system, bone marrow, thymus, and the like), lungs and gastrointestinal tract. Chronic GVHD (cGVHD) often begins 100 days or later post transplant or transfusion and can attack the same organs as acute GvHD, but also can affect connective tissue and exocrine glands. Acute GvHD of the skin can result in a diffuse maculopapular rash, sometimes in a lacy pattern.

Donor T cell: The term "donor T cell" as used here refers to T cells that often are administered to a recipient to confer anti-viral and/or anti-tumor immunity following allogeneic stem cell transplantation. Donor T cells often are utilized to inhibit marrow graft rejection and increase the success of alloengraftment, however the same donor T cells can cause an alloaggressive response against host antigens, which in turn can result in graft versus host disease (GVHD). Certain activated donor T cells can cause a higher or lower GvHD response than other activated T cells. Donor T cells may also be reactive against recipient tumor cells, causing a beneficial graft vs. tumor effect.

Mesenchymal stromal cell: The terms "mesenchymal stromal cell" or "bone marrow derived mesenchymal stromal cell" as used herein, refer to multipotent stem cells that can differentiate ex vivo, in vitro and in vivo into adipocytes, osteoblasts and chondroblasts, and may be further defined as a fraction of mononuclear bone marrow cells that adhere to plastic culture dishes in standard culture conditions, are negative for hematopoietic lineage markers and are positive for CD73, CD90 and CD105.

Embryonic stem cell: The term "embryonic stem cell" as used herein, refers to pluripotent stem cells derived from the inner cell mass of the blastocyst, an early-stage embryo of between 50 to 150 cells. Embryonic stem cells are characterized by their ability to renew themselves indefinitely and by their ability to differentiate into derivatives of all three primary germ layers, ectoderm, endoderm and mesoderm. Pluripotent is distinguished from mutipotent in that pluripotent cells can generate all cell types, while multipotent cells (e.g., adult stem cells) can only produce a limited number of cell types.

Inducible pluripotent stem cell: The terms "inducible pluripotent stem cell" or "induced pluripotent stem cell" as used herein refers to adult, or differentiated cells, that are "reprogrammed" or induced by genetic (e.g., expression of genes that in turn activates pluripotency), biological (e.g., treatment viruses or retroviruses) and/or chemical (e.g., small molecules, peptides and the like) manipulation to generate cells that are capable of differentiating into many if not all cell types, like embryonic stem cells. Inducible pluripotent stem cells are distinguished from embryonic stem cells in that they achieve an intermediate or terminally differentiated state (e.g., skin cells, bone cells, fibroblasts, and the like) and then are induced to dedifferentiate, thereby regaining some or all of the ability to generate multipotent or pluripotent cells. CD34⁺ cell: The term "CD34⁺ cell" as used herein refers to a cell expressing the CD34 protein on its cell surface. "CD34" as used herein refers to a cell surface glycoprotein (e.g., sialomucin protein) that often acts as a cell-cell adhesion factor and is involved in T cell entrance into lymph nodes, and is a member of the "cluster of differentiation" gene family. CD34 also may mediate the attachment of stem cells to bone marrow, extracellular matrix or directly to stromal cells. CD34⁺ cells often are found in the umbilical cord and bone marrow as hematopoietic cells, a subset of mesenchymal stem cells, endothelial progenitor cells, endothelial cells of blood vessels but not lymphatics (except pleural lymphatics), mast cells, a sub-population of dendritic cells (which are factor Xllla negative) in the interstitium and around the adnexa of dermis of skin, as well as cells in certain soft tissue tumors (e.g., alveolar soft part sarcoma, pre-B acute lymphoblastic leukemia (Pre-B-ALL), acute myelogenous leukemia (AML), AML-M7, dermatofibrosarcoma protuberans, gastrointestinal stromal tumors, giant cell fibroblastoma, granulocytic sarcoma, Kaposi's sarcoma, liposarcoma, malignant fibrous histiocytoma, malignant peripheral nerve sheath tumors, mengingeal hemangiopericytomas, meningiomas, neurofibromas, schwannomas, and papillary thyroid carcinoma).

Gene expression vector: The terms "gene expression vector", "nucleic acid expression vector", or "expression vector" as used herein, which can be used interchangeably throughout the document, generally refers to a nucleic acid molecule (e.g., a plasmid, phage, autonomously replicating sequence (ARS), artificial chromosome, yeast artificial chromosome (e.g., YAC)) that can be replicated in a host cell and be utilized to introduce a gene or genes into a host cell. The genes introduced on the expression vector can be endogenous genes (e.g., a gene normally found in the host cell or organism) or heterologous genes (e.g., genes not normally found in the genome or on extra-chromosomal nucleic acids of the host cell or organism). The genes introduced into a cell by an expression vector can be native genes or genes that have been modified or engineered. The gene expression vector also can be engineered to contain 5' and 3' untranslated regulatory sequences that sometimes can function as enhancer sequences, promoter regions and/or terminator sequences that can facilitate or enhance efficient transcription of the gene or genes carried on the expression vector. A gene expression vector sometimes also is engineered for replication and/or expression functionality (e.g., transcription and translation) in a particular cell type, cell location, or tissue type. Expression vectors sometimes include a selectable marker for maintenance of the vector in the host or recipient cell.

Developmentally regulated promoter: The term "developmentally regulated promoter" as used herein refers to a promoter that acts as the initial binding site for RNA polymerase to transcribe a gene which is expressed under certain conditions that are controlled, initiated by or influenced by a developmental program or pathway. Developmentally regulated promoters often have additional control regions at or near the promoter region for binding activators or repressors of transcription that can influence transcription of a gene that is part of a development program or pathway. Developmentally regulated promoters sometimes are involved in transcribing genes whose gene products influence the developmental differentiation of cells.

Developmentally differentiated cells: The term "developmentally differentiated cells", as used herein refers to cells that have undergone a process, often involving expression of specific developmentally regulated genes, by which the cell evolves from a less specialized form to a more specialized form in order to perform a specific function. Non-limiting examples of developmentally differentiated cells are liver cells, lung cells, skin cells, nerve cells, blood cells, and the like. Changes in developmental differentiation generally involve changes in gene expression (e.g., changes in patterns of gene expression), genetic re-organization (e.g., remodeling or chromatin to hide or expose genes that will be silenced or expressed, respectively), and occasionally involve changes in DNA sequences (e.g., immune diversity differentiation). Cellular differentiation during development can be understood as the result of a gene regulatory network. A regulatory gene and its cis-regulatory modules are nodes in a gene regulatory network that receive input (e.g., protein expressed upstream in a development pathway or program) and create output elsewhere in the network (e.g., the expressed gene product acts on other genes downstream in the developmental pathway or program).

The terms "cell," "cell line," and "cell culture" as used herein may be used interchangeably. All of these terms also include their progeny, which are any and all subsequent generations. It is understood that all progeny may not be identical due to deliberate or inadvertent mutations.

As used here, the term "rapalog" is meant as an analog of the natural antibiotic rapamycin. Certain rapalogs as described herein have properties such as stability in serum, a poor affinity to wildtype FRB (and hence the parent protein, mTOR, leading to reduction or elimination of immunosuppressive properties), and a relatively high affinity to a mutant FRB domain. For commercial purposes, the rapalogs may have useful scaling and production properties. Examples of rapalogs include, but are not limited to, S-o,p-dimethoxyphenyl (DMOP)-rapamycin: EC₅₀ (wt FRB (K2095 T2098 W2101) ∼ 1000 nM), EC₅₀ (FRB-KLW ∼ 5 nM) Luengo JI (95) Chem & Biol 2:471-81; Luengo JI (94) J. Org Chem 59:6512-6513; US Pat 6187757; R-Isopropoxyrapamycin: EC₅₀ (wt FRB (K2095 T2098 W2101) ∼ 300 nM), EC50 (FRB-PLF ∼ 8.5 nM); Liberles S (97) PNAS 94: 7825-30; and S-Butanesulfonamidorap (AP23050): EC₅₀ (wt FRB (K2095 T2098 W2101) ∼ 2.7 nM), EC₅₀ (FRB-KTF ∼ >200 nM) Bayle (06) Chem & Bio. 13: 99-107.

The term "FRB" refers to the FKBP12-Rapamycin-Binding (FRB) domain (residues 2015-2114 encoded within mTOR), and analogs thereof. Also, FRB variants are provided. The properties of an FRB variant are stability (some variants are more labile than others) and ability to bind to various rapalogs. Based on the crystal structure conjugated to rapamcyin, there are 3 key rapamycin-interacting residues that have been most analyzed, K2095, T2098, and W2101. Mutation of all three leads to an unstable protein that can be stabilized in the presence of rapamycin or some rapalogs. This feature can be used to further increase the signal:noise ratio in some applications. Examples of mutants are discussed in Bayle et al (06) Chem & Bio 13: 99-107; Stankunas et al (07) Chembiochem 8:1162-1169; and Liberles S (97) PNAS 94:7825-30). Examples of FRB regions as described herein include, but are not limited to, KLW (with L2098); KTF (with F2101); and KLF (L2098, F2101).

Each ligand can include two or more portions (e.g., defined portions, distinct portions), and sometimes includes two, three, four, five, six, seven, eight, nine, ten, or more portions. The first ligand and second ligand each, independently, can consist of two portions (i.e., dimer), consist of three portions (i.e., trimer) or consist of four portions (i.e., tetramer). The first ligand sometimes includes a first portion and a second portion and the second ligand sometimes includes a third portion and a fourth portion. The first portion and the second portion often are different (i.e., heterogeneous (e.g., heterodimer)), the first portion and the third portion sometimes are different and sometimes are the same, and the third portion and the fourth portion often are the same (i.e., homogeneous (e.g., homodimer)). Portions that are different sometimes have a different function (e.g., bind to the first multimerizing region, bind to the second multimerizing region, do not significantly bind to the first multimerizing region, do not significantly bind to the second multimerizing region (e.g., the first portion binds to the first multimerizing region but does not significantly bind to the second multimerizing region) and sometimes have a different chemical structure. Portions that are different sometimes have a different chemical structure but can bind to the same multimerizing region (e.g., the second portion and the third portion can bind to the second multimerizing region but can have different structures). The first portion sometimes binds to the first multimerizing region and sometimes does not bind significantly to the second multimerizing region. Each portion sometimes is referred to as a "monomer" (e.g., first monomer, second monomer, third monomer and fourth monomer that tracks the first portion, second portion, third portion and fourth portion, respectively). Each portion sometimes is referred to as a "side." Sides of a ligand may sometimes be adjacent to each other, and may sometimes be located at opposing locations on a ligand.

By being "capable of binding", as in the example of a multimeric or heterodimeric ligand binding to a multimerizing region or ligand binding region is meant that the ligand binds to the ligand binding region, for example, a portion, or portions, of the ligand bind to the multimerizing region, and that this binding may be detected by an assay method including, but not limited to, a biological assay, a chemical assay, or physical means of detection such as, for example, x-ray crystallography. In addition, where a ligand is considered to "not significantly bind" is meant that there may be minor detection of binding of a ligand to the ligand binding region, but that this amount of binding, or the stability of binding is not significantly detectable, and, when occurring in the cells as described herein, does not activate the modified cell or cause apoptosis. In certain examples, where the ligand does not "significantly bind," upon administration of the ligand, the amount of cells undergoing apoptosis is less than 10, 5, 4, 3, 2, or 1%.

The multimerizing regions, such as the FRB or FKBP12 multimerizing regions, may be located amino terminal to the pro-apoptotic polypeptide, may be located carboxyl terminal to the pro-apoptotic polypeptide. Additional polypeptides, such as, for example, linker polypeptides, stem polypeptides, spacer polypeptides, or in some examples, marker polypeptides, may be located between the multimerizing region and the pro-apoptotic polypeptide.

By "region"or "domain" is meant a polypeptide, or fragment thereof, that maintains the function of the polypeptide as it relates to the chimeric polypeptides of the present application. That is, for example, an FKBP12 binding domain, FKBP12 domain, FKBP12 region, FKBP12 multimerizing region, and the like, refer to an FKBP12 polypeptide that binds to the CID ligand, such as, for example, rimiducid, or rapamycin, to cause, or allow for, dimerization or multimerization of the chimeric polypeptide. By "region" or "domain" of a pro-apoptotic polypeptide, for example, the Caspase-9 polypeptides or truncated Caspase-9 polypeptides of the present sisclosure, is meant that upon dimerization or multimerization of the Caspase-9 region as part of the chimeric polypeptide, or chimeric pro-apoptotic polypeptde, the dimerized or multimerized chimeric polypeptide can participate in the caspase cascade, allowing for, or causing, apoptosis.

FKBP12 variants may also be used in the FKBP12 or FRB multimerizing regionss. Examples of FKBP12 variants include those from many species, including, for example, yeast. The FKBP12 variant may be FKBP12.6 (calstablin).

Other heterodimers are contemplated in the present application. A calcineurin-A polypeptide, or region may be used in place of the FRB multimerizing region. The first ligand may comprise, for example, cyclosporine.

As used herein, the term "iCaspase-9" molecule, polypeptide, or protein is defined as an inducible Caspase-9. The term "iCaspase-9" embraces iCaspase-9 nucleic acids, iCaspase-9 polypeptides and/or iCaspase-9 expression vectors. The term also encompasses either the natural iCaspase-9 nucleotide or amino acid sequence, or a truncated sequence that is lacking the CARD domain.

As used herein, the term "iCaspase 1 molecule", "iCaspase 3 molecule", or "iCaspase 8 molecule" is defined as an inducible Caspase 1, 3, or 8, respectively. The term iCaspase 1, iCaspase 3, or iCaspase 8, embraces iCaspase 1, 3, or 8 nucleic acids, iCaspase 1, 3, or 8 polypeptides and/or iCaspase 1, 3, or 8 expression vectors, respectively. The term also encompasses either the natural CaspaseiCaspase-1, -3, or -8 nucleotide or amino acid sequence, respectively, or a truncated sequence that is lacking the CARD domain. By "wild type" Caspase-9 in the context of the experimental details provided herein, is meant the Caspase-9 molecule lacking the CARD domain.

Modified Caspase-9 polypeptides comprise at least one amino acid substitution that affects basal activity or IC₅₀, in a chimeric polypeptide comprising the modified Caspase-9 polypeptide. Methods for testing basal activity and IC₅₀ are discussed herein. Non-modified Caspase-9 polypeptides do not comprise this type of amino acid substitution. Both modified and non-modified Caspase-9 polypeptides may be truncated, for example, to remove the CARD domain.

"Function-conservative variants" are proteins or enzymes in which a given amino acid residue has been changed without altering overall conformation and function of the protein or enzyme, including, but not limited to, replacement of an amino acid with one having similar properties, including polar or non-polar character, size, shape and charge. Conservative amino acid substitutions for many of the commonly known non-genetically encoded amino acids are well known in the art. Conservative substitutions for other non-encoded amino acids can be determined based on their physical properties as compared to the properties of the genetically encoded amino acids.

Amino acids other than those indicated as conserved may differ in a protein or enzyme so that the percent protein or amino acid sequence similarity between any two proteins of similar function may vary and can be, for example, at least 70%, at least 80%, at least 90%, and at least 95%, as determined according to an alignment scheme. As referred to herein, "sequence similarity" means the extent to which nucleotide or protein sequences are related. The extent of similarity between two sequences can be based on percent sequence identity and/or conservation. "Sequence identity" herein means the extent to which two nucleotide or amino acid sequences are invariant. "Sequence alignment" means the process of lining up two or more sequences to achieve maximal levels of identity (and, in the case of amino acid sequences, conservation) for the purpose of assessing the degree of similarity. Numerous methods for aligning sequences and assessing similarity/identity are known in the art such as, for example, the Cluster Method, wherein similarity is based on the MEGALIGN algorithm, as well as BLASTN, BLASTP, and FASTA. When using any of these programs, the settings may be selected that result in the highest sequence similarity.

The amino acid residue numbers referred to herein reflect the amino acid position in the non-truncated and non-modified Caspase-9 polypeptide, for example, that of SEQ ID NO: 9. SEQ ID NO: 9 provides an amino acid sequence for the truncated Caspase-9 polypeptide, which does not include the CARD domain. Thus SEQ ID NO: 9 commences at amino acid residue number 135, and ends at amino acid residue number 416, with reference to the full length Caspase-9 amino acid sequence. Those of ordinary skill in the art may align the sequence with other sequences of Caspase-9 polypeptides to, if desired, correlate the amino acid residue number, for example, using the sequence alignment methods discussed herein.

As used herein, the term "cDNA" is intended to refer to DNA prepared using messenger RNA (mRNA) as template. The advantage of using a cDNA, as opposed to genomic DNA or DNA polymerized from a genomic, non- or partially-processed RNA template, is that the cDNA primarily contains coding sequences of the corresponding protein. There are times when the full or partial genomic sequence is used, such as where the non-coding regions are required for optimal expression or where non-coding regions such as introns are to be targeted in an antisense strategy.

As used herein, the term "expression construct" or "transgene" is defined as any type of genetic construct containing a nucleic acid coding for gene products in which part or all of the nucleic acid encoding sequence is capable of being transcribed can be inserted into the vector. The transcript is translated into a protein, but it need not be. Expression may include both transcription of a gene and translation of mRNA into a gene product. Also, expression may only include transcription of the nucleic acid encoding genes of interest. The term "therapeutic construct" may also be used to refer to the expression construct or transgene. The expression construct or transgene may be used, for example, as a therapy to treat hyperproliferative diseases or disorders, such as cancer, thus the expression construct or transgene is a therapeutic construct or a prophylactic construct.

As used herein, the term "expression vector" refers to a vector containing a nucleic acid sequence coding for at least part of a gene product capable of being transcribed. In some cases, RNA molecules are then translated into a protein, polypeptide, or peptide. In other cases, these sequences are not translated, for example, in the production of antisense molecules or ribozymes. Expression vectors can contain a variety of control sequences, which refer to nucleic acid sequences necessary for the transcription and possibly translation of an operatively linked coding sequence in a particular host organism. In addition to control sequences that govern transcription and translation, vectors and expression vectors may contain nucleic acid sequences that serve other functions as well and are discussed infra. As used herein, the term "ex vivo" refers to "outside" the body. The terms "ex vivo" and "in vitro" can be used interchangeably herein.

As used herein, the term "functionally equivalent," as it relates to Caspase-9, or truncated Caspase-9, for example, refers to a Caspase-9 nucleic acid fragment, variant, or analog, refers to a nucleic acid that codes for a Caspase-9 polypeptide, or a Caspase-9 polypeptide, that stimulates an apoptotic response. "Functionally equivalent" refers, for example, to a Caspase-9 polypeptide that is lacking the CARD domain, but is capable of inducing an apoptotic cell response. When the term "functionally equivalent" is applied to other nucleic acids or polypeptides, such as, for example, CD19, the 5'LTR, the multimeric ligand binding region, or CD3, it refers to fragments, variants, and the like that have the same or similar activity as the reference polypeptides of the methods herein.

As used herein, the term "gene" is defined as a functional protein, polypeptide, or peptide-encoding unit. As will be understood, this functional term includes genomic sequences, cDNA sequences, and smaller engineered gene segments that express, or are adapted to express, proteins, polypeptides, domains, peptides, fusion proteins, and mutants.

The term "hyperproliferative disease" is defined as a disease that results from a hyperproliferation of cells. Exemplary hyperproliferative diseases include, but are not limited to cancer or autoimmune diseases. Other hyperproliferative diseases may include vascular occlusion, restenosis, atherosclerosis, or inflammatory bowel disease.

The term "immunogenic composition" or "immunogen" refers to a substance that is capable of provoking an immune response. Examples of immunogens include, e.g., antigens, autoantigens that play a role in induction of autoimmune diseases, and tumor-associated antigens expressed on cancer cells.

The term "immunocompromised" as used herein is defined as a subject that has reduced or weakened immune system. The immunocompromised condition may be due to a defect or dysfunction of the immune system or to other factors that heighten susceptibility to infection and/or disease. Although such a categorization allows a conceptual basis for evaluation, immunocompromised individuals often do not fit completely into one group or the other. More than one defect in the body's defense mechanisms may be affected. For example, individuals with a specific T-lymphocyte defect caused by HIV may also have neutropenia caused by drugs used for antiviral therapy or be immunocompromised because of a breach of the integrity of the skin and mucous membranes. An immunocompromised state can result from indwelling central lines or other types of impairment due to intravenous drug abuse; or be caused by secondary malignancy, malnutrition, or having been infected with other infectious agents such as tuberculosis or sexually transmitted diseases, e.g., syphilis or hepatitis.

As used herein, the term "pharmaceutically or pharmacologically acceptable" refers to molecular entities and compositions that do not produce adverse, allergic, or other untoward reactions when administered to an animal or a human.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the vectors or cells presented herein, its use in therapeutic compositions is contemplated. Supplementary active ingredients also can be incorporated into the compositions.

As used herein, the term "polynucleotide" is defined as a chain of nucleotides. Furthermore, nucleic acids are polymers of nucleotides. Thus, nucleic acids and polynucleotides as used herein are interchangeable. Nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR™, and the like, and by synthetic means. Furthermore, polynucleotides include mutations of the polynucleotides, include but are not limited to, mutation of the nucleotides, or nucleosides by methods well known in the art. A nucleic acid may comprise one or more polynucleotides.

As used herein, the term "polypeptide" is defined as a chain of amino acid residues, usually having a defined sequence. As used herein the term polypeptide is interchangeable with the terms "peptides" and "proteins".

As used herein, the term "promoter" is defined as a DNA sequence recognized by the synthetic machinery of the cell, or introduced synthetic machinery, required to initiate the specific transcription of a gene.

The term "transfection" and "transduction" are interchangeable and refer to the process by which an exogenous DNA sequence is introduced into a eukaryotic host cell. Transfection (or transduction) can be achieved by any one of a number of means including electroporation, microinjection, gene gun delivery, retroviral infection, lipofection, superfection and the like.

As used herein, the term "syngeneic" refers to cells, tissues or animals that have genotypes that are identical or closely related enough to allow tissue transplant, or are immunologically compatible. For example, identical twins or animals of the same inbred strain. Syngeneic and isogeneic can be used interchangeably.

The terms "patient" or "subject" are interchangeable, and, as used herein include, but are not limited to, an organism or animal; a mammal, including, e.g., a human, non-human primate (e.g., monkey), mouse, pig, cow, goat, rabbit, rat, guinea pig, hamster, horse, monkey, sheep, or other non-human mammal; a non-mammal, including, e.g., a non-mammalian vertebrate, such as a bird (e.g., a chicken or duck) or a fish, and a non-mammalian invertebrate.

By "T cell activation molecule" is meant a polypeptide that, when incorporated into a T cell expressing a chimeric antigen receptor, enhances activation of the T cell. Examples include, but are not limited to, ITAM-containing, Signal 1 conferring molecules such as, for example, CD3 ζ polypeptide, and Fc receptor gamma, such as, for example, Fc epsilon receptor gamma (FcεR1γ) subunit (Haynes, N.M., et al. J. Immunol. 166:182-7 (2001)).J. Immunology).

As used herein, the term "under transcriptional control" or "operatively linked" is defined as the promoter is in the correct location and orientation in relation to the nucleic acid to control RNA polymerase initiation and expression of the gene.

As used herein, the terms "treatment", "treat", "treated", or "treating" refer to prophylaxis and/or therapy.

As used herein, the term "vaccine" refers to a formulation that contains a composition presented herein which is in a form that is capable of being administered to an animal. Typically, the vaccine comprises a conventional saline or buffered aqueous solution medium in which the composition is suspended or dissolved. In this form, the composition can be used conveniently to prevent, ameliorate, or otherwise treat a condition. Upon introduction into a subject, the vaccine is able to provoke an immune response including, but not limited to, the production of antibodies, cytokines and/or other cellular responses.

The nucleic acid may be contained within a viral vector. The viral vector may be a retroviral vector. The viral vector may be an adenoviral vector or a lentiviral vector. It is understood that the antigen-presenting cell is contacted with the viral vector ex vivo, and the antigen-presenting cell is contacted with the viral vector in vivo.

### Hematopoietic Stem Cells and Cell Therapy

Hematopoietic stem cells include hematopoietic progenitor cells, immature, multipotent cells that can differentiate into mature blood cell types. These stem cells and progenitor cells may be isolated from bone marrow and umbilical cord blood, and, in some cases, from peripheral blood. Other stem and progenitor cells include, for example, mesenchymal stromal cells, embryonic stem cells, and inducible pluripotent stem cells.

Bone marrow derived mesenchymal stromal cells (MSCs) have been defined as a fraction of mononuclear bone marrow cells that adhere to plastic culture dishes in standard culture conditions, are negative for hematopoietic lineage markers and positive for CD73, CD90 and CD105, and able to differentiate in vitro into adipocytes, osteoblasts, and chondroblasts. While one physiologic role is presumed to be the support of hematopoiesis, several reports have also established that MSCs are able to incorporate and possibly proliferate in areas of active growth, such as cicatricial and neoplastic tissues, and to home to their native microenvironment and replace the function of diseased cells. Their differentiation potential and homing ability make MSCs attractive vehicles for cellular therapy, either in their native form for regenerative applications, or through their genetic modification for delivery of active biological agents to specific microenvironments such as diseased bone marrow or metastatic deposits. In addition, MSCs possess potent intrinsic immunosuppressive activity, and to date have found their most frequent application in the experimental treatment of graft-versus-host disease and autoimmune disorders (Pittenger, M. F., et al. (1999). Science 284: 143-147; Dominici, M., et al. (2006). Cytotherapy 8: 315-317; Prockop, D. J. (1997). Science 276: 71-74; Lee, R. H., et al. (2006). Proc Natl Acad Sci U S A 103: 17438-17443; Studeny, M., et al., (2002). Cancer Res 62: 3603-3608; Studeny, M., et al. (2004). J Natl Cancer Inst 96: 1593-1603; Horwitz, E. M., et al. (1999). Nat Med 5: 309-313; Chamberlain, G., et al., (2007). Stem Cells 25: 2739-2749; Phinney, D. G., and Prockop, D. J. (2007). Stem Cells 25: 2896-2902; Horwitz, E. M., et al. (2002). Proc Natl Acad Sci U S A 99: 8932-8937; Hall, B., et al., (2007). Int J Hematol 86: 8-16; Nauta, A. J., and Fibbe, W. E. (2007). Blood 110: 3499-3506; Le Blanc, K., et al. (2008). Lancet 371: 1579-1586; Tyndall, A., and Uccelli, A. (2009). Bone Marrow Transplant).

MSCs have been infused in hundreds of patients with minimal reported side effects. However, follow-up is limited, long term side effects are unknown, and little is known of the consequences that will be associated with future efforts to induce their in vivo differentiation, for example to cartilage or bone, or to genetically modify them to enhance their functionality. Several animal models have raised safety concerns. For instance, spontaneous osteosarcoma formation in culture has been observed in murine derived MSCs. Furthermore, ectopic ossification and calcification foci have been discussed in mouse and rat models of myocardial infarction after local injection of MSC, and their proarrhythmic potential has also been apparent in co-culture experiments with neonatal rat ventricular myocytes. Moreover, bilateral diffuse pulmonary ossification has been observed after bone marrow transplant in a dog, presumably due to the transplanted stromal components (Horwitz, E. M., et al., (2007). Biol Blood Marrow Transplant 13: 53-57; Tolar, J., et al. (2007). Stem Cells 25: 371-379; Yoon, Y.-S., et al., (2004). Circulation 109: 3154-3157; Breitbach, M., et al. (2007). Blood 110: 1362-1369;Chang, M. G., et al. (2006). Circulation 113: 1832-1841; Sale, G. E., and Storb, R. (1983). Exp Hematol 11: 961-966).

In another example of cell therapy, T cells transduced with a nucleic acid encoding a chimeric antigen receptor have been administered to patients to treat cancer (Zhong, X.-S., (2010) Molecular Therapy 18:413-420). Chimeric antigen receptors (CARs) are artificial receptors designed to convey antigen specificity to T cells without the requirement for MHC antigen presentation. They include an antigen-specific component, a transmembrane component, and an intracellular component selected to activate the T cell.and provide specific immunity. Chimeric antigen receptor-expressing T cells may be used in various therapies, including cancer therapies. Costimulating polypeptides may be used to enhance the activation of CAR-expressing T cells against target antigens, and therefore increase the potency of adoptive immunotherapy.

For example, T cells expressing a chimeric antigen receptor based on the humanized monoclonal antibody Trastuzumab (Herceptin) has been used to treat cancer patients. Adverse events are possible, however, and in at least one reported case, the therapy had fatal consequences to the patient (Morgan, R.A., et al., (2010) Molecular Therapy 18:843-851). Transducing the cells with a chimeric Caspase-9-based safety switch as presented herein, would provide a safety switch that could stop the adverse event from progressing. Therefore, also described herein are nucleic acids, cells, and methods wherein the modified T cell also expresses an inducible Caspase-9 polypeptide. If there is a need, for example, to reduce the number of chimeric antigen receptor modified T cells, an inducible ligand may be administered to the patient, thereby inducing apoptosis of the modified T cells.

The antitumor efficacy from immunotherapy with T cells engineered to express chimeric antigen receptors (CARs) has steadily improved as CAR molecules have incorporated additional signaling domains to increase their potency. T cells transduced with first generation CARs, containing only the CD3ζ intracellular signaling molecule, have demonstrated poor persistence and expansion in vivo following adoptive transfer (Till BG, Jensen MC, Wang J, et al: CD20-specific adoptive immunotherapy for lymphoma using a chimeric antigen receptor with both CD28 and 4-1BB domains: pilot clinical trial results. Blood 119:3940-50, 2012; Pule MA, Savoldo B, Myers GD, et al: Virus-specific T cells engineered to coexpress tumor-specific receptors: persistence and antitumor activity in individuals with neuroblastoma. Nat Med 14:1264-70, 2008; Kershaw MH, Westwood JA, Parker LL, et al: A phase 1 study on adoptive immunotherapy using gene-modified T cells for ovarian cancer. Clin Cancer Res 12:6106-15, 2006), as tumor cells often lack the requisite costimulating molecules necessary for complete T cell activation. Second generation CAR T cells were designed to improve proliferation and survival of the cells. Second generation CAR T cells that incorporate the intracellular costimulating domains from either CD28 or 4-1BB (Carpenito C, Milone MC, Hassan R, et al: Control of large, established tumor xenografts with genetically retargeted human T cells containing CD28 and CD137 domains. Proc Natl Acad Sci U S A 106:3360-5, 2009; Song DG, Ye Q, Poussin M, et al: CD27 costimulation augments the survival and antitumor activity of redirected human T cells in vivo. Blood 119:696-706, 2012), show improved survival and in vivo expansion following adoptive transfer, and more recent clinical trials using anti-CD19 CAR-modified T cells containing these costimulating molecules have shown remarkable efficacy for the treatment of CD19+ leukemia.(Kalos M, Levine BL, Porter DL, et al: T cells with chimeric antigen receptors have potent antitumor effects and can establish memory in patients with advanced leukemia. Sci Transl Med 3:95ra73, 2011; Porter DL, Levine BL, Kalos M, et al: Chimeric antigen receptor-modified T cells in chronic lymphoid leukemia. N Engl J Med 365:725-33, 2011; Brentjens RJ, Davila ML, Riviere I, et al: CD19-targeted T cells rapidly induce molecular remissions in adults with chemotherapy-refractory acute lymphoblastic leukemia. Sci Transl Med 5:177ra38, 2013).

While others have explored additional signaling molecules from tumor necrosis factor (TNF)-family proteins, such as OX40 and 4-1BB, called "third generation" CAR T cells, (Finney HM, Akbar AN, Lawson AD: Activation of resting human primary T cells with chimeric receptors: costimulation from CD28, inducible costimulator, CD134, and CD137 in series with signals from the TCR zeta chain. J Immunol 172:104-13, 2004; Guedan S, Chen X, Madar A, et al: ICOS-based chimeric antigen receptors program bipolar TH17/TH1 cells. Blood, 2014), other molecules which induce T cell signaling distinct from the CD3ζ nuclear factor of activated T cells (NFAT) pathway may provide necessary costimulation for T cell survival and proliferation, and possibly endow CAR T cells with additional, valuable functions, not supplied by more conventional costimulating molecules. Some second and third-generation CAR T cells have been implicated in patient deaths, due to cytokine storm and tumor lysis syndrome caused by highly activated T cells.

By "chimeric antigen receptor" or "CAR" is meant, for example, a chimeric polypeptide which comprises a polypeptide sequence that recognizes a target antigen (an antigen-recognition domain) linked to a transmembrane polypeptide and intracellular domain polypeptide selected to activate the T cell and provide specific immunity. The antigen-recognition domain may be a single-chain variable fragment (ScFv), or may, for example, be derived from other molecules such as, for example, a T cell receptor or Pattern Recognition Receptor. The intracellular domain comprises at least one polypeptide which causes activation of the T cell, such as, for example, but not limited to, CD3 zeta, and, for example, co-stimulatory molecules, for example, but not limited to, CD28, OX40 and 4-1BB. The term "chimeric antigen receptor" may also refer to chimeric receptors that are not derived from antibodies, but are chimeric T cell receptors. These chimeric T cell receptors may comprise a polypeptide sequence that recognizes a target antigen, where the recognition sequence may be, for example, but not limited to, the recognition sequence derived from a T cell receptor or an scFv. The intracellular domain polypeptides are those that act to activate the T cell. Chimeric T cell receptors are discussed in, for example, Gross, G., and Eshar, Z., FASEB Journal 6:3370-3378 (1992), and Zhang, Y., et al., PLOS Pathogens 6:1- 13 (2010).

In one type of chimeric antigen receptor (CAR), the variable heavy (VH) and light (VL) chains for a tumor-specific monoclonal antibody are fused in-frame with the CD3 zeta chain (ζ) from the T cell receptor complex. The VH and VL are generally connected together using a flexible glycine-serine linker, and then attached to the transmembrane domain by a spacer (CH2CH3) to extend the scFv away from the cell surface so that it can interact with tumor antigens. Following transduction, T cells now express the CAR on their surface, and upon contact and ligation with a tumor antigen, signal through the CD3 zeta chain inducing cytotoxicity and cellular activation.

Investigators have noted that activation of T cells through CD3 zeta is sufficient to induce a tumor-specific killing, but is insufficient to induce T cell proliferation and survival. Early clinical trials using T cells modified with first generation CARs expressing only the zeta chain showed that gene-modified T cells exhibited poor survival and proliferation in vivo.

As co-stimulation through the B7 axis is necessary for complete T cell activation, investigators added the co-stimulating polypeptide CD28 signaling domain to the CAR construct. This region generally contains the transmembrane region (in place of the CD3 zeta version) and the YMNM motif for binding PI3K and Lck. In vivo comparisons between T cells expressing CARs with only zeta or CARs with both zeta and CD28 demonstrated that CD28 enhanced expansion in vivo, in part due to increased IL-2 production following activation. The inclusion of CD28 is called a 2nd generation CAR. The most commonly used costimulating molecules include CD28 and 4-1BB, which, following tumor recognition, can initiate a signaling cascade resulting in NF-κB activation, which promotes both T cell proliferation and cell survival.

The use of co-stimulating polypeptides 4-1BB or OX40 in CAR design has further improved T cell survival and efficacy. 4-1BB in particular appears to greatly enhance T cell proliferation and survival. This 3rd generation design (with 3 signaling domains) has been used in PSMA CARs (Zhong XS, et al., Mol Ther. 2010 Feb; 18(2):413-20) and in CD19 CARs, most notably for the treatment of CLL (Milone, M.C., et al., (2009) Mol. Ther. 17:1453-1464; Kalos, M., et al., Sci. Transl. Med. (2011) 3:95ra73; Porter, D., et al., (2011) N. Engl. J. Med. 365: 725-533). These cells showed impressive function in 3 patients, expanding more than a 1000-fold in vivo, and resulted in sustained remission in all three patients.

It is understood that by "derived" is meant that the nucleotide sequence or amino acid sequence may be derived from the sequence of the molecule. The intracellular domain comprises at least one polypeptide which causes activation of the T cell, such as, for example, but not limited to, CD3 zeta, and, for example, co-stimulatory molecules, for example, but not limited to, CD28, OX40 and 4-1BB.

T cell receptors are molecules composed of two different polypeptides that are on the surface of T cells. They recognize antigens bound to major histocompatibility complex molecules; upon recognition with the antigen, the T cell is activated. By "recognize" is meant, for example, that the T cell receptor, or fragment or fragments thereof, such as TCRα polypeptide and TCRβ together, is capable of contacting the antigen and identifying it as a target. TCRs may comprise α and β polypeptides, or chains. The α and β polypeptides include two extracellular domains, the variable and the constant domains. The variable domain of the α and β polypeptides has three complementarity determining regions (CDRs); CDR3 is considered to be the main CDR responsible for recognizing the epitope. The α polypeptide includes the V and J regions, generated by VJ recombination, and the β polypeptide includes the V, D, and J regions, generated by VDJ recombination. The intersection of the VJ regions and VDJ regions corresponds to the CDR3 region. TCRs are often named using the International Immunogenetics (IMGT) TCR nomenclature (IMGT Database, www. IMGT.org; Giudicelli, V., et al., IMGT/LIGM-DB, the IMGT® comprehensive database of immunoglobulin and T cell receptor nucleotide sequences, Nucl. Acids Res., 34, D781-D784 (2006). PMID: 16381979;T cell Receptor Factsbook, LeFranc and LeFranc, Academic Press ISBN 0-12-441352-8).

Chimeric T cell receptors may bind to, for example, antigenic polypeptides such as Bob-1, PRAME, and NY-ESO-1. (U.S. Patent Application No. 14/930,572, filed November 2, 2015, titled "T Cell Receptors Directed Against Bob1 and Uses Thereof," and U.S. Provisional Patent Application No. 62/130,884, filed March 10, 2015, titled "T Cell Receptors Directed Against the Preferentially-Expressed Antigen of Melanoma and Uses Thereof.

In another example of cell therapy, T cells are modified so that they express a non-functional TGF-beta receptor, rendering them resistant to TGF-beta. This allows the modified T cells to avoid the cytotoxicity caused by TGF-beta, and allows the cells to be used in cellular therapy (Bollard, C.J., et al., (2002) Blood 99:3179-3187; Bollard, C.M., et al., (2004) J. Exptl. Med. 200:1623-1633). However, it also could result in a T cell lymphoma, or other adverse effect, as the modified T cells now lack part of the normal cellular control; these therapeutic T cells could themselves become malignant. Transducing these modified T cells with a chimeric Caspase-9-based safety switch as presented herein, would provide a safety switch that could avoid this result.

In other examples, Natural Killer cells are modified to express the membrane-targeting polypeptide. Instead of a chimeric antigen receptor, the heterologous membrane bound polypeptide may be a NKG2D receptor. NKG2D receptors can bind to stress proteins (e.g. MICA/B) on tumor cells and can thereby activate NK cells. The extracellular binding domain can also be fused to signaling domains (Barber, A., et al., Cancer Res 2007;67: 5003-8; Barber A, et al., Exp Hematol. 2008; 36:1318-28; Zhang T., et al., Cancer Res. 2007; 67:11029-36., and this could, in turn, be linked to FRB domains, analogous to FRB-linkered CARs. Moreover, other cell surface receptors, such as VEGF-R could be used as a docking site for FRB domains to enhance tumor-dependent clustering in the presence of hypoxia-triggered VEGF, found at high levels within many tumors.

Cells used in cellular therapy, that express a heterologous gene, such as a modified receptor, or a chimeric receptor, may be transduced with nucleic acid that encodes a chimeric Caspase-9-based safety switch before, after, or at the same time, as the cells are transduced with the heterologous gene.

### Haploidentical stem cell transplantation

While stem cell transplantation has proven an effective means of treating a wide variety of diseases involving hematopoietic stem cells and their progeny, a shortage of histocompatible donors has proved a major impediment to the widest application of the approach. The introduction of large panels of unrelated stem cell donors and or cord blood banks has helped to alleviate the problem, but many patients remain unsuited to either source. Even when a matched donor can be found, the elapsed time between commencing the search and collecting the stem cells usually exceeds three months, a delay that may doom many of the neediest patients. Hence there has been considerable interest in making use of HLA haploidentical family donors. Such donors may be parents, siblings or second-degree relatives. The problem of graft rejection may be overcome by a combination of appropriate conditioning and large doses of stem cells, while graft versus host disease (GvHD) may be prevented by extensive T cell-depletion of the donor graft. The immediate outcomes of such procedures have been gratifying, with engraftment rate > 90% and a severe GvHD rate of < 10% for both adults and children even in the absence of post transplant immunosuppression. Unfortunately the profound immunosuppression of the grafting procedure, coupled with the extensive T cell-depletion and HLA mismatching between donor and recipient lead to an extremely high rate of post-transplant infectious complications, and contributed to high incidence of disease relapse.

Donor T cell infusion is an effective strategy for conferring anti-viral and anti-tumor immunity following allogeneic stem cell transplantation. Simple addback of T cells to the patients after haploidentical transplantation, however, cannot work; the frequency of alloreactive T cells is several orders of magnitude higher than the frequency of, for example, virus specific T lymphocytes. Methods are being developed to accelerate immune reconstitution by administrating donor T cells that have first been depleted of alloreactive ceils. One method of achieving this is stimulating donor T cells with recipient EBV-transformed B lymphoblastoid cell lines (LCLs). Alloreactive T cells upregulate CD25 expression, and are eliminated by a CD25 Mab immunotoxin conjugate, RFT5-SMPT-dgA. This compound consists of a murine IgG1 anti-CD25 (IL-2 receptor alpha chain) conjugated via a hetero-bifunctional crosslinker [N-succinimidyloxycarbonyl-alpha-methyl-d- (2-pyridylthio) toluene] to chemically deglycosylated ricin A chain (dgA).

Treatment with CD25 immunotoxin after LCL stimulation depletes >90% of alloreactive cells. In a phase 1 clinical study, using CD25 immunotoxin to deplete alloreactive lymphocytes immune reconstitution after allodepleted donor T cells were infused at 2 dose levels into recipients of T-cell-depleted haploidentical SCT. Eight patients were treated at 10⁴ cells/kg/dose, and 8 patients received 10⁵ cells/kg/dose. Patients receiving 10⁵ cells/kg/dose showed significantly improved T-cell recovery at 3, 4, and 5 months after SCT compared with those receiving 10⁴ cells/kg/dose (P < .05). Accelerated T-cell recovery occurred as a result of expansion of the effector memory (CD45RA(-)CCR-7(-)) population (P < .05), suggesting that protective T-cell responses are likely to be long lived. T-cell-receptor signal joint excision circles (TRECs) were not detected in reconstituting T cells in dose-level 2 patients, indicating they are likely to be derived from the infused allodepleted cells. Spectratyping of the T cells at 4 months demonstrated a polyclonal Vbeta repertoire. Using tetramer and enzyme-linked immunospot (ELISpot) assays, cytomegalovirus (CMV)- and Epstein-Barr virus (EBV)-specific responses in 4 of 6 evaluable patients at dose level 2 as early as 2 to 4 months after transplantation, whereas such responses were not observed until 6 to 12 months in dose-level 1 patients. The incidence of significant acute (2 of 16) and chronic graft-versus-host disease (GvHD; 2 of 15) was low. These data demonstrate that allodepleted donor T cells can be safely used to improve T-cell recovery after haploidentical SCT. The amount of cells infused was subsequently escalated to 10⁶ cells/kg without evidence of GvHD.

Although this approach reconstituted antiviral immunity, relapse remained a major problem and 6 patients transplanted for high risk leukemia relapsed and died of disease. Higher T cell doses are therefore useful to reconstitute anti-tumor immunity and to provide the hoped-for anti-tumor effect, since the estimated frequency of tumor-reactive precursors is 1 to 2 logs less than frequency of viral-reactive precursors. However, in some patients, these doses of cells will be sufficient to trigger GvHD even after allodepletion (Hurley CK, et al., Biol Blood Marrow Transplant 2003;9:610-615; Dey BR, et al., Br.J Haematol. 2006;135:423-437; Aversa F, et al., N Engl J Med 1998;339:1186-1193; Aversa F, et al., J C lin.On col. 2005;23:3447-3454; Lang P, Mol.Dis. 2004;33:281-287; Kolb HJ, et al., Blood 2004;103:767-776; Gottschalk S, et al., Annu.Rev.Med 2005;56:29-44; Bleakley M, et al., Nat.Rev.Cancer 2004;4:371-380; Andre-Schmutz I, et al., Lancet 2002;360:130-137; Solomon SR, etal., Blood 2005;106:1123-1129; Amrolia PJ, et al., Blood 2006;108:1797-1808; Amrolia PJ, et al., Blood 2003; Ghetie V, et al., J Immunol Methods 1991;142:223-230; Molldrem JJ, et al., Cancer Res 1999;59:2675-2681; Rezvani K, et al., Clin.Cancer Res. 2005;1 1:8799-8807; Rezvani K, et al., Blood 2003;102:2892-2900).

### Graft versus Host Disease (GvHD)

Graft versus Host Disease is a condition that sometimes occurs after the transplantation of donor immunocompetent cells, for example, T cells, into a recipient. The transplanted cells recognize the recipient's cells as foreign, and attack and destroy them. This condition can be a dangerous effect of T cell transplantation, especially when associated with haploidentical stem cell transplantation. Sufficient T cells should be infused to provide the beneficial effects, such as, for example, the reconstitution of an immune system and the graft anti-tumor effect. But, the number of T cells that can be transplanted can be limited by the concern that the transplant will result in severe graft versus host disease.

Graft versus Host Disease may be staged as indicated in the following tables:

**Staging**

| | **Stage 0** | **Stage 1** | **Stage 2** | **Stage 3** | **Stage 4** |
|---|---|---|---|---|---|
| **Skin** | No rash | Rash <25% BSA | 25-50% | >50% Generalized erythroderma | Plus bullae and desquamation |
| **Gut (for pediatric patients)** | <500 mL diarrhea/day | 501-1000 mL/day 5cc/kg-10 cc/kg/day | 1001-1500 mL/day 10cc/kg-15cc/kg/day | >1500 mL/day >15 cc/kg/day | Severe abdominal pain and ileus |
| **UGI** | | Severe nausea/vom iting | | | |
| **Liver** | Bilirubin s 2mg/di | 2.1-3 mg/di | 3.1-6mg/di | 6.1-15 mg/di | >15 mg/di |

Acute GvHD grading may be performed by the consensus conference criteria (Przepiorka D et al., 1994 Consensus Conference on Acute GVHD Grading. Bone Marrow Transplant 1995;15:825-828).

**Grading Index of Acute GvHD**

| | **Skin** | **Liver** | **Gut** | **Upper GI** |
|---|---|---|---|---|
| **0** | None and | None and | None and | None |
| **I** | Stage 1-2 and | None and | None | None |
| **II** | Stage 3 and/or | Stage 1 and/or | Stage 1 and/or | Stage 1 |
| **III** | None-Stage 3 with | Stage 2-3 or | Stage 2-4 | N/A |
| **IV** | Stage 4 or | Stage 4 | N/A | N/A |

### Inducible Caspase-9 as a "Safety Switch" for Cell Therapy and for Genetically Engineered Cell Transplantation

By reducing the effect of graft versus host disease is meant, for example, a decrease in the GvHD symptoms so that the patient may be assigned a lower level stage, or, for example, a reduction of a symptom of graft versus host disease by at least 20%, 30%, 40%, 50%, 60%, 70%, 80%, 90%, 95%, or 99%. A reduction in the effect of graft versus host disease may also be measured by detection of a reduction in activated T cells involved in the GvHD reaction, such as, for example, a reduction of cells that express the marker protein, for example CD19, and express CD3 (CD3⁺ CD19⁺ cells, for example) by at least 30%, 40%, 50%, 60%, 70%, 75%, 80%, 85%, 90%, 95%, or 99%.

Provided herein is an alternative suicide gene strategy that is based on human proapoptotic molecules fused with an FKBP variant that is optimized to bind a chemical inducer of dimerization (CID). Variants may include, for example, an FKBP region that has an amino acid substitution at position 36 selected from the group consisting of valine, leucine, isoleuceine and alanine (Clackson T, et al., Proc Natl Acad Sci USA. 1998, 95:10437-10442). AP1903 is a synthetic molecule that has proven safe in healthy volunteers (luliucci JD, et al., J Clin Pharmacol. 2001, 41:870-879). Administration of this small molecule results in cross-linking and activation of the proapoptotic target molecules. The application of this inducible system in human T lymphocytes has been explored using Fas or the death effector domain (DED) of the Fas-associated death domain-containing protein (FADD) as proapoptotic molecules. Up to 90% of T cells transduced with these inducible death molecules underwent apoptosis after administration of CID (Thomis DC, et al., Blood. 2001, 97:1249-1257; Spencer DM, et al., Curr Biol. 1996, 6: 839-847; Fan L, et al., Hum Gene Ther. 1999, 10: 2273-2285; Berger C, et al., Blood. 2004, 103:1261-1269; Junker K, et al., Gene Ther. 2003, 10:1189- 197). This suicide gene strategy may be used in any appropriate cell used for cell therapy including, for example, hematopoietic stem cells, and other progenitor cells, including, for example, mesenchymal stromal cells, embryonic stem cells, and inducible pluripotent stem cells. AP20187 and AP1950, a synthetic version of AP1903, may also be used as the ligand inducer. (Amara JF (97) PNAS 94:10618-23; Clontech Laboratories-Takara Bio)

Therefore, this safety switch, catalyzed by Caspase-9, may be used where there is a condition in the cell therapy patient that requires the removal of the transfected or transduced therapeutic cells. Conditions where the cells may need to be removed include, for example, GvHD, inappropriate differentiation of the cells into more mature cells of the wrong tissue or cell type, and other toxicities. To activate the Caspase-9 switch in the case of inappropriate differentiation, it is possible to use tissue specific promoters. For example, where a progenitor cell differentiates into bone and fat cells, and the fat cells are not desired, the vector used to transfect or transduce the progenitor cell may have a fat cell specific promoter that is operably linked to the Caspase-9 nucleotide sequence. In this way, should the cells differentiate into fat cells, upon administration of the multimer ligand, apoptosis of the inappropriately differentiated fat cells should result.
The methods may be used, for example, for any disorder that can be alleviated by cell therapy, including cancer, cancer in the blood or bone marrow, other blood or bone marrow borne diseases such as sickle cell anemia and metachromic leukodystrophy, and any disorder that can be alleviated by a stem cell transplantation, for example blood or bone marrow disorders such as sickle cell anemia or metachromal leukodystrophy.

The efficacy of adoptive immunotherapy may be enhanced by rendering the therapeutic T cells resistant to immune evasion strategies employed by tumor cells. In vitro studies have shown that this can be achieved by transduction with a dominant-negative receptor or an immunomodulatory cytokine (Bollard CM, et al., Blood. 2002, 99:3179-3187: Wagner HJ, et al., Cancer Gene Ther. 2004, 11:81-91). Moreover, transfer of antigen-specific T-cell receptors allows for the application of T-cell therapy to a broader range of tumors (Pule M, et al., Cytotherapy. 2003, 5:211-226; Schumacher TN, Nat Rev Immunol. 2002, 2:512-519). A suicide system for engineered human T cells was developed and tested to allow their subsequent use in clinical studies. Caspase-9 has been modified and shown to be stably expressed in human T lymphocytes without compromising their functional and phenotypic characteristics while demonstrating sensitivity to CID, even in T cells that have upregulated antiapoptotic molecules. (Straathof, K.C., et al., 2005, Blood 105:4248-54).

In genetically modified cells used for gene therapy, the gene may be a heterologous polynucleotide sequence derived from a source other than the cell that is used to express the gene. The gene is derived from a prokaryotic or eukaryotic source such as a bacterium, a virus, yeast, a parasite, a plant, or even an animal. The heterologous DNA also is derived from more than one source, i.e., a multigene construct or a fusion protein. The heterologous DNA also may include a regulatory sequence, which is derived from one source and the gene from a different source. Or, the heterologous DNA may include regulatory sequences that are used to change the normal expression of a cellular endogenous gene.

### Other Caspase molecules

Caspase polypeptides other than Caspase-9 that may be encoded by the chimeric polypeptides of the current technology include, for example, Caspase-1, Caspase-3, and Caspase-8. Discussions of these Caspase polypeptides may be found in, for example, MacCorkle, R.A., et al., Proc. Natl. Acad. Sci. U.S.A. (1998) 95:3655-3660; and Fan, L., et al. (1999) Human Gene Therapy 10:2273-2285).

### Engineering Expression Constructs

Expression constructs encode a multimeric ligand binding region and a Caspase-9 polypeptide, or a multimeric ligand binding region and a Caspase-9 polypeptide linked to a marker polypeptide, all operatively linked. In general, the term "operably linked" is meant to indicate that the promoter sequence is functionally linked to a second sequence, wherein, for example, the promoter sequence initiates and mediates transcription of the DNA corresponding to the second sequence. The Caspase-9 polypeptide may be full length or truncated. The marker polypeptide may be linked to the Caspase-9 polypeptide. For example, the marker polypeptide may be linked to the Caspase-9 polypeptide via a polypeptide sequence, such as, for example, a cleavable 2A-like sequence. The marker polypeptide may be, for example, CD19, or may be, for example, a heterologous protein, selected to not affect the activity of the chimeric caspase polypeptide.

The polynucleotide may encode the Caspase-9 polypeptide and a heterologous protein, which may be, for example a marker polypeptide and may be, for example, a chimeric antigen receptor. The heterologous polypeptide, for example, the chimeric antigen receptor, may be linked to the Caspase-9 polypeptide via a polypeptide sequence, such as, for example, a cleavable 2A-like sequence.

In certain examples, a nucleic acid comprising a polynucleotide coding for a chimeric antigen receptor is included in the same vector, such as, for example, a viral or plasmid vector, as a polynucleotide coding for a second polypeptide. This second polypeptide may be, for example, a caspase polypeptide, as discussed herein, or a marker polypeptide. In these examples, the construct may be designed with one promoter operably linked to a nucleic acid comprising a polynucleotide coding for the two polypeptides, linked by a cleavable 2A polypeptide. In this example, the first and second polypeptides are separated during translation, resulting in a chimeric antigen receptor polypeptide, and the second polypeptide. In other examples, the two polypeptides may be expressed separately from the same vector, where each nucleic acid comprising a polynucleotide coding for one of the polypeptides is operably linked to a separate promoter. In yet other examples, one promoter may be operably linked to the two nucleic acids, directing the production of two separate RNA transcripts, and thus two polypeptides. Therefore, the expression constructs discussed herein may comprise at least one, or at least two promoters.

2A-like sequences, or "cleavable" 2A sequences, are derived from, for example, many different viruses, including, for example, from Thosea asigna. These sequences are sometimes also known as "peptide skipping sequences." When this type of sequence is placed within a cistron, between two peptides that are intended to be separated, the ribosome appears to skip a peptide bond, in the case of Thosea asigna sequence, the bond between the Gly and Pro amino acids is omitted. This leaves two polypeptides, in this case the Caspase-9 polypeptide and the marker polypeptide. When this sequence is used, the peptide that is encoded 5' of the 2A sequence may end up with additional amino acids at the carboxy terminus, including the Gly residue and any upstream in the 2A sequence. The peptide that is encoded 3' of the 2A sequence may end up with additional amino acids at the amino terminus, including the Pro residue and any downstream in the 2A sequence. "2A" or "2A-like" sequences are part of a large family of peptides that can cause peptide bond-skipping. Various 2A sequences have been characterized (e.g., F2A, P2A, T2A), and are examples of 2A-like sequences that may be used in the polypeptides of the present application.;

The expression construct may be inserted into a vector, for example a viral vector or plasmid. The steps of the methods provided may be performed using any suitable method; these methods include, without limitation, methods of transducing, transforming, or otherwise providing nucleic acid to the antigen-presenting cell, presented herein. The truncated Caspase-9 polypeptide may be encoded by the nucleotide sequence of SEQ ID NO 8, SEQ ID NO: 23, SEQ ID NO: 25, SEQ ID NO: 27, or a functionally equivalent fragment thereof, with or without DNA linkers, or has the amino acid sequence of SEQ ID NO: 9, SEQ ID NO: 24, SEQ ID NO: 26, or SEQ ID NO: 28 or a functionally equivalent fragment thereof. The CD19 polypeptide may be encoded by the nucleotide sequence of SEQ ID NO 14, or a functionally equivalent fragment thereof, with or without DNA linkers, or has the amino acid sequence of SEQ ID NO: 15, or a functionally equivalent fragment thereof. A functionally equivalent fragment of the Caspase-9 polypeptide has substantially the same ability to induce apoptosis as the polypeptide of SEQ ID NO: 9, with at least 50%, 60%, 70%, 80%, 90%, or 95% of the activity of the polypeptide of SEQ ID NO: 9. A functionally equivalent fragment of the CD19 polypeptide has substantially the same ability as the polypeptide of SEQ ID No: 15, to act as a marker to be used to identify and select transduced or transfected cells, with at least 50%, 60%, 70%, 80%, 90%, or 95% of the marker polypeptide being detected when compared to the polypeptide of SEQ ID NO: 15, using standard detection techniques.

More particularly, more than one ligand binding domain or multimerizing region may be used in the expression construct. Yet further, the expression construct contains a membrane-targeting sequence. Appropriate expression constructs may include a costimulatory polypeptide element on either side of the above FKBP ligand binding elements.

In certain examples, the polynucleotide coding for the inducible caspase polypeptide is included in the same vector, such as, for example, a viral or plasmid vector, as a polynucleotide coding for a chimeric antigen receptor. In these examples, the construct may be designed with one promoter operably linked to a nucleic acid comprising a nucleotide sequence coding for the two polypeptides, linked by a cleavable 2A polypeptide. In this example, the first and second polypeptides are cleaved after expression, resulting in a chimeric antigen receptor polypeptide and an inducible Caspase-9 polypeptide. In other examples, the two polypeptides may be expressed separately from the same vector, where each nucleic acid comprising a nucleotide sequence coding for one of the polypeptides is operably linked to a separate promoter. In yet other examples, one promoter may be operably linked to the two nucleic acids, directing the production of two separate RNA transcripts, and thus two polypeptides. Therefore, the expression constructs discussed herein may comprise at least one, or at least two promoters,

In yet other examples, two polypeptides may be expressed in a cell using two separate vectors. The cells may be co-transfected or co-transformed with the vectors, or the vectors may be introduced to the cells at different times.

### Ligand binding Regions

The ligand binding ("dimerization") domain, or multimerizing region, of the expression construct can be any convenient domain that will allow for induction using a natural or unnatural ligand, for example, an unnatural synthetic ligand. The multimerizing region can be internal or external to the cellular membrane, depending upon the nature of the construct and the choice of ligand. A wide variety of ligand binding proteins, including receptors, are known, including ligand binding proteins associated with the cytoplasmic regions indicated above. As used herein the term "ligand binding domain" can be interchangeable with the term "receptor". Of particular interest are ligand binding proteins for which ligands (for example, small organic ligands) are known or may be readily produced. These ligand binding domains or receptors include the FKBPs and cyclophilin receptors, the steroid receptors, the tetracycline receptor, the other receptors indicated above, and the like, as well as "unnatural" receptors, which can be obtained from antibodies, particularly the heavy or light chain subunit, mutated sequences thereof, random amino acid sequences obtained by stochastic procedures, combinatorial syntheses, and the like. The ligand binding region may be selected from the group consisting of FKBP ligand binding region, cyclophilin receptor ligand binding region, steroid receptor ligand binding region, cyclophilin receptors ligand binding region, and tetracycline receptor ligand binding region. Often, the ligand binding region comprises a F_{v'}Fᵥₗₛ sequence. Sometimes, the F_{v'}Fᵥₗₛ sequence further comprises an additional F_{v'} sequence. Examples include, for example, those discussed in Kopytek, S.J., et al., Chemistry & Biology 7:313-321 (2000) and in Gestwicki, J.E., et al., Combinatorial Chem. & High Throughput Screening 10:667-675 (2007); Clackson T (2006) Chem Biol Drug Des 67:440-2; Clackson, T., in Chemical Biology: From Small Molecules to Systems Biology and Drug Design (Schreiber, s., et al., eds., Wiley, 2007)).

For the most part, the ligand binding domains or receptor domains will be at least about 50 amino acids, and fewer than about 350 amino acids, usually fewer than 200 amino acids, either as the natural domain or truncated active portion thereof. The binding domain may, for example, be small (<25 kDa, to allow efficient transfection in viral vectors), monomeric, nonimmunogenic, have synthetically accessible, cell permeable, nontoxic ligands that can be configured for dimerization.

The receptor domain can be intracellular or extracellular depending upon the design of the expression construct and the availability of an appropriate ligand. For hydrophobic ligands, the binding domain can be on either side of the membrane, but for hydrophilic ligands, particularly protein ligands, the binding domain will usually be external to the cell membrane, unless there is a transport system for internalizing the ligand in a form in which it is available for binding. For an intracellular receptor, the construct can encode a signal peptide and transmembrane domain 5' or 3' of the receptor domain sequence or may have a lipid attachment signal sequence 5' of the receptor domain sequence. Where the receptor domain is between the signal peptide and the transmembrane domain, the receptor domain will be extracellular.

The portion of the expression construct encoding the receptor can be subjected to mutagenesis for a variety of reasons. The mutagenized protein can provide for higher binding affinity, allow for discrimination by the ligand of the naturally occurring receptor and the mutagenized receptor, provide opportunities to design a receptor-ligand pair, or the like. The change in the receptor can involve changes in amino acids known to be at the binding site, random mutagenesis using combinatorial techniques, where the codons for the amino acids associated with the binding site or other amino acids associated with conformational changes can be subject to mutagenesis by changing the codon(s) for the particular amino acid, either with known changes or randomly, expressing the resulting proteins in an appropriate prokaryotic host and then screening the resulting proteins for binding.

Antibodies and antibody subunits, e.g., heavy or light chain, particularly fragments, more particularly all or part of the variable region, or fusions of heavy and light chain to create high-affinity binding, can be used as the binding domain. Antibodies that are contemplated include ones that are an ectopically expressed human product, such as an extracellular domain that would not trigger an immune response and generally not expressed in the periphery (i.e., outside the CNS/brain area). Such examples, include, but are not limited to low affinity nerve growth factor receptor (LNGFR), and embryonic surface proteins (i.e., carcinoembryonic antigen).
Yet further, antibodies can be prepared against haptenic molecules, which are physiologically acceptable, and the individual antibody subunits screened for binding affinity. The cDNA encoding the subunits can be isolated and modified by deletion of the constant region, portions of the Variable region, mutagenesis of the variable region, or the like, to obtain a binding protein domain that has the appropriate affinity for the ligand. In this way, almost any physiologically acceptable haptenic compound can be employed as the ligand or to provide an epitope for the ligand. Instead of antibody units, natural receptors can be employed, where the binding domain is known and there is a useful ligand for binding.

### Oligomerization

The transduced signal will normally result from ligand-mediated oligomerization of the chimeric protein molecules, i.e., as a result of oligomerization following ligand binding, although other binding events, for example allosteric activation, can be employed to initiate a signal. The construct of the chimeric protein will vary as to the order of the various domains and the number of repeats of an individual domain.

For multimerizing the receptor, the ligand for the ligand binding domains/receptor domains of the chimeric surface membrane proteins will usually be multimeric in the sense that it will have at least two binding sites, with each of the binding sites capable of binding to the ligand receptor domain. By "multimeric ligand binding region" is meant a ligand binding region that binds to a multimeric ligand. The term "multimeric ligands" include dimeric ligands. A dimeric ligand will have two binding sites capable of binding to the ligand receptor domain. Desirably, the subject ligands will be a dimer or higher order oligomer, usually not greater than about tetrameric, of small synthetic organic molecules, the individual molecules typically being at least about 150 Da and less than about 5 kDa, usually less than about 3 kDa. A variety of pairs of synthetic ligands and receptors can be employed. For example, aspects involving natural receptors, dimeric FK506 can be used with an FKBP12 receptor, dimerized cyclosporin A can be used with the cyclophilin receptor, dimerized estrogen with an estrogen receptor, dimerized glucocorticoids with a glucocorticoid receptor, dimerized tetracycline with the tetracycline receptor, dimerized vitamin D with the vitamin D receptor, and the like. Alternatively higher orders of the ligands, e.g., trimeric can be used. For aspects involving unnatural receptors, e.g., antibody subunits, modified antibody subunits, single chain antibodies comprised of heavy and light chain variable regions in tandem, separated by a flexible linker domain, or modified receptors, and mutated sequences thereof, and the like, any of a large variety of compounds can be used. A significant characteristic of these ligand units is that each binding site is able to bind the receptor with high affinity and they are able to be dimerized chemically. Also, methods are available to balance the hydrophobicity/hydrophilicity of the ligands so that they are able to dissolve in serum at functional levels, yet diffuse across plasma membranes for most applications. The present methods may utilize the technique of chemically induced dimerization (CID) to produce a conditionally controlled protein or polypeptide. In addition to this technique being inducible, it also is reversible, due to the degradation of the labile dimerizing agent or administration of a monomeric competitive inhibitor.

The CID system uses synthetic bivalent ligands to rapidly crosslink signaling molecules that are fused to ligand binding domains. This system has been used to trigger the oligomerization and activation of cell surface (Spencer, D. M., et al., Science, 1993. 262: p. 1019-1024; Spencer D. M. et al., Curr Biol 1996, 6:839-847; Blau, C. A. et al., Proc Natl Acad.Sci. USA 1997, 94:3076-3081), or cytosolic proteins (Luo, Z. et al., Nature 1996,383:181-185; MacCorkle, R. A. et al., Proc Natl Acad Sci USA 1998, 95:3655-3660), the recruitment of transcription factors to DNA elements to modulate transcription (Ho, S. N. et al., Nature 1996, 382:822-826; Rivera, V. M. et al., Nat.Med. 1996, 2:1028-1032) or the recruitment of signaling molecules to the plasma membrane to stimulate signaling (Spencer D. M. et al., Proc.Natl.Acad.Sci. USA 1995, 92:9805-9809; Holsinger, L. J. et al., Proc.Natl.Acad.Sci. USA 1995, 95:9810-9814).

The CID system is based upon the notion that surface receptor aggregation effectively activates downstream signaling cascades. In the simplest case, the CID system uses a dimeric analog of the lipid permeable immunosuppressant drug, FK506, which loses its normal bioactivity while gaining the ability to crosslink molecules genetically fused to the FK506-binding protein, FKBP12. By fusing one or more FKBPs to Caspase-9, one can stimulate Caspase-9 activity in a dimerizer drug-dependent, but ligand and ectodomain-independent manner. This provides the system with temporal control, reversibility using monomeric drug analogs, and enhanced specificity. The high affinity of third-generation AP20187/AP1903 CIDs for their binding domain, FKBP12, permits specific activation of the recombinant receptor in vivo without the induction of non-specific side effects through endogenous FKBP12. FKBP12 variants having amino acid substitutions and deletions, such as FKBP12v36, that bind to a dimerizer drug, may also be used. FKBP12 variants include, but are not limited to, those having amino acid substitutions at position 36, selected from the group consisting of valine, leucine, isoleuceine, and alanine. In addition, the synthetic ligands are resistant to protease degradation, making them more efficient at activating receptors in vivo than most delivered protein agents.

The ligands used are capable of binding to two or more of the ligand binding domains. The chimeric proteins may be able to bind to more than one ligand when they contain more than one ligand binding domain. The ligand is typically a non-protein or a chemical. Exemplary ligands include, but are not limited to FK506 (e.g., FK1012).

Other ligand binding regions may be, for example, dimeric regions, or modified ligand binding regions with a wobble substitution, such as, for example, FKBP12(V36): The human 12 kDa FK506-binding protein with an F36 to V substitution, the complete mature coding sequence (amino acids 1-107), provides a binding site for synthetic dimerizer drug AP1903 (Jemal, A. et al., CA Cancer J. Clinic. 58, 71-96 (2008); Scher, H.I. and Kelly, W.K., Journal of Clinical Oncology 11, 1566-72 (1993)). Two tandem copies of the protein may also be used in the construct so that higher-order oligomers are induced upon cross-linking by AP1903.

F36V'-FKBP: F36V'-FKBP is a codon-wobbled version of F36V-FKBP. It encodes the identical
polypeptide sequence as F36V-FKPB but has only 62% homology at the nucleotide level.
F36V'-FKBP was designed to reduce recombination in retroviral vectors (Schellhammer, P.F. et al., J. Urol. 157, 1731-5 (1997)). F36V'-FKBP was constructed by a PCR assembly procedure. The transgene contains one copy of F36V'-FKBP linked directly to one copy of F36V-FKBP.

The ligand may be a small molecule. The appropriate ligand for the selected ligand binding region may be selected. Often, the ligand is dimeric, sometimes, the ligand is a dimeric FK506 or a dimeric FK506-like analog. The ligand may be AP1903 (CAS Index Name: 2-Piperidinecarboxylic acid, 1-[(2S)-1-oxo-2-(3,4,5-trimethoxyphenyl)butyl]-, 1,2-ethanediylbis[imino(2-oxo-2,1-ethanediyl)oxy-3,1-phenylene[(1R)-3-(3,4-dimethoxyphenyl)propylidene]] ester, [2S-[1(R*),2R*[S*[S*[1(R*),2R*]]]]]-(9Cl) CAS Registry Number; 195514-63-7; Molecular Formula: C78H98N4O20 Molecular Weight: 1411.65). The ligand may be AP20187. The ligand may be an AP20187 analog, such as, for example, AP1510. Certain analogs may be be appropriate for the FKBP12, and certain analogs appropriate for the wobbled version of FKBP12. One ligand binding region may be included in the chimeric protein. Also, two or more ligand binding regions may be included. Where, for example, the ligand binding region is FKBP12, where two of these regions are included, one may, for example, be the wobbled version.

Other dimerization systems contemplated include the coumermycin/DNA gyrase B system. Coumermycin-induced dimerization activates a modified Raf protein and stimulating the MAP kinase cascade. See Farrar, M. A., et. Al., (1996) Nature 383,178-181. The abscisic acid (ABA) system developed by GR Crabtree and colleagues (Liang FS, et al., Sci Signal. 2011 Mar 15;4(164):rs2), may be used, but like DNA gyrase B, this relies on a foreign protein, which would be immunogenic.

### Membrane-targeting

A membrane-targeting sequence or region provides for transport of the chimeric protein to the cell surface membrane, where the same or other sequences can encode binding of the chimeric protein to the cell surface membrane. Molecules in association with cell membranes contain certain regions that facilitate the membrane association, and such regions can be incorporated into a chimeric protein molecule to generate membrane-targeted molecules. For example, some proteins contain sequences at the N-terminus or C-terminus that are acylated, and these acyl moieties facilitate membrane association. Such sequences are recognized by acyltransferases and often conform to a particular sequence motif. Certain acylation motifs are capable of being modified with a single acyl moiety (often followed by several positively charged residues (e.g. human c-Src: M-G-S-N-K-S-K-P-K-D-A-S-Q-R-R-R) to improve association with anionic lipid head groups) and others are capable of being modified with multiple acyl moieties. For example the N-terminal sequence of the protein tyrosine kinase Src can comprise a single myristoyl moiety. Dual acylation regions are located within the N-terminal regions of certain protein kinases, such as a subset of Src family members (e.g., Yes, Fyn, Lck) and G-protein alpha subunits. Such dual acylation regions often are located within the first eighteen amino acids of such proteins, and conform to the sequence motif Met-Gly-Cys-Xaa-Cys, where the Met is cleaved, the Gly is N-acylated and one of the Cys residues is S-acylated. The Gly often is myristoylated and a Cys can be palmitoylated. Acylation regions conforming to the sequence motif Cys-Ala-Ala-Xaa (so called "CAAX boxes"), which can modified with C15 or C10 isoprenyl moieties, from the C-terminus of G-protein gamma subunits and other proteins (e.g., World Wide Web address ebi.ac.uk/interpro/DisplaylproEntry?ac=IPR001230) also can be utilized. These and other acylation motifs include, for example, those discussed in Gauthier-Campbell et al., Molecular Biology of the Cell 15: 2205-2217 (2004); Glabati et al., Biochem. J. 303: 697-700 (1994) and Zlakine et al., J. Cell Science 110: 673-679 (1997), and can be incorporated in chimeric molecules to induce membrane localization. A native sequence from a protein containing an acylation motif may be incorporated into a chimeric protein. For example, an N-terminal portion of Lck, Fyn or Yes or a G-protein alpha subunit, such as the first twenty-five N-terminal amino acids or fewer from such proteins (e.g., about 5 to about 20 amino acids, about 10 to about 19 amino acids, or about 15 to about 19 amino acids of the native sequence with optional mutations), may be incorporated within the N-terminus of a chimeric protein. A C-terminal sequence of about 25 amino acids or less from a G-protein gamma subunit containing a CAAX box motif sequence (e.g., about 5 to about 20 amino acids, about 10 to about 18 amino acids, or about 15 to about 18 amino acids of the native sequence with optional mutations) may be linked to the C-terminus of a chimeric protein.

An acyl moiety may have a log p value of +1 to +6, and sometimes may have a log p value of +3 to +4.5. Log p values are a measure of hydrophobicity and often are derived from octanol/water partitioning studies, in which molecules with higher hydrophobicity partition into octanol with higher frequency and are characterized as having a higher log p value. Log p values are published for a number of lipophilic molecules and log p values can be calculated using known partitioning processes (e.g., Chemical Reviews, Vol. 71, Issue 6, page 599, where entry 4493 shows lauric acid having a log p value of 4.2). Any acyl moiety can be linked to a peptide composition discussed above and tested for antimicrobial activity using known methods and those discussed hereafter. The acyl moiety sometimes is a C1-C20 alkyl, C2-C20 alkenyl, C2-C20 alkynyl, C3-C6 cycloalkyl, C1-C4 haloalkyl, C4-C12 cyclalkylalkyl, aryl, substituted aryl, or aryl (C1-C4) alkyl, for example. Any acyl-containing moiety sometimes is a fatty acid, and examples of fatty acid moieties are propyl (C3), butyl (C4), pentyl (C5), hexyl (C6), heptyl (C7), octyl (C8), nonyl (C9), decyl (C10), undecyl (C11), lauryl (C12), myristyl (C14), palmityl (C16), stearyl (C18), arachidyl (C20), behenyl (C22) and lignoceryl moieties (C24), and each moiety can contain 0, 1, 2, 3, 4, 5, 6, 7 or 8 unsaturations (i.e., double bonds). An acyl moiety sometimes is a lipid molecule, such as a phosphatidyl lipid (e.g., phosphatidyl serine, phosphatidyl inositol, phosphatidyl ethanolamine, phosphatidyl choline), sphingolipid (e.g., shingomyelin, sphingosine, ceramide, ganglioside, cerebroside), or modified versions thereof. One, two, three, four or five or more acyl moieties may be linked to a membrane association region.

A chimeric protein herein also may include a single-pass or multiple pass transmembrane sequence (e.g., at the N-terminus or C-terminus of the chimeric protein). Single pass transmembrane regions are found in certain CD molecules, tyrosine kinase receptors, serine/threonine kinase receptors, TGFbeta, BMP, activin and phosphatases. Single pass transmembrane regions often include a signal peptide region and a transmembrane region of about 20 to about 25 amino acids, many of which are hydrophobic amino acids and can form an alpha helix. A short track of positively charged amino acids often follows the transmembrane span to anchor the protein in the membrane. Multiple pass proteins include ion pumps, ion channels, and transporters, and include two or more helices that span the membrane multiple times. All or substantially all of a multiple pass protein sometimes is incorporated in a chimeric protein. Sequences for single pass and multiple pass transmembrane regions are known and can be selected for incorporation into a chimeric protein molecule.

Any membrane-targeting sequence can be employed that is functional in the host and may, or may not, be associated with one of the other domains of the chimeric protein. Such sequences may include, but are not limited to myristoylation-targeting sequence, palmitoylation-targeting sequence, prenylation sequences (i.e., farnesylation, geranyl-geranylation, CAAX Box), protein-protein interaction motifs or transmembrane sequences (utilizing signal peptides) from receptors. Examples include those discussed in, for example, ten Klooster JP et al, Biology of the Cell (2007) 99, 1-12, Vincent, S., et al., Nature Biotechnology 21:936-40, 1098 (2003).

Additional protein domains exist that can increase protein retention at various membranes. For example, an ∼ 120 amino acid pleckstrin homology (PH) domain is found in over 200 human proteins that are typically involved in intracellular signaling. PH domains can bind various phosphatidylinositol (PI) lipids within membranes (e.g. PI (3, 4,5)-P3, PI (3,4)-P2, PI (4,5)-P2) and thus play a key role in recruiting proteins to different membrane or cellular compartments. Often the phosphorylation state of PI lipids is regulated, such as by PI-3 kinase or PTEN, and thus, interaction of membranes with PH domains are not as stable as by acyl lipids.

### AP1903 for Injection

AP1903 API is manufactured by Alphora Research Inc. and AP1903 Drug Product for Injection is made by Formatech Inc. It is formulated as a 5 mg/mL solution of AP1903 in a 25% solution of the non-ionic solubilizer Solutol HS 15 (250 mg/mL, BASF). At room temperature, this formulation is a clear, slightly yellow solution. Upon refrigeration, this formulation undergoes a reversible phase transition, resulting in a milky solution. This phase transition is reversed upon re-warming to room temperature. The fill is 2.33 mL in a 3 mL glass vial (-10 mg AP1903 for Injection total per vial).

AP1903 is removed from the refrigerator the night before the patient is dosed and stored at a temperature of approximately 21°C overnight, so that the solution is clear prior to dilution. The solution is prepared within 30 minutes of the start of the infusion in glass or polyethylene bottles or non-DEHP bags and stored at approximately 21°C prior to dosing.

All study medication is maintained at a temperature between 2 degrees C and 8 degrees C, protected from excessive light and heat, and stored in a locked area with restricted access.

Upon determining a need to administer AP1903 and induce the inducible Caspase-9 polypeptide, patients may be, for example, administered a single fixed dose of AP1903 for Injection (0.4 mg/kg) via IV infusion over 2 hours, using a non-DEHP, non-ethylene oxide sterilized infusion set. The dose of AP1903 is calculated individually for all patients, and is not to be recalculated unless body weight fluctuates by ≥10%. The calculated dose is diluted in 100 mL in 0.9% normal saline before infusion.

In a previous Phase 1 study of AP1903, 24 healthy volunteers were treated with single doses of AP1903 for Injection at dose levels of 0.01, 0.05, 0.1, 0.5 and 1.0 mg/kg infused IV over 2 hours. AP1903 plasma levels were directly proportional to dose, with mean Cₘₐₓ values ranging from approximately 10 - 1275 ng/mL over the 0.01 - 1.0 mg/kg dose range. Following the initial infusion period, blood concentrations demonstrated a rapid distribution phase, with plasma levels reduced to approximately 18, 7, and 1% of maximal concentration at 0.5, 2 and 10 hours post-dose, respectively. AP1903 for Injection was shown to be safe and well tolerated at all dose levels and demonstrated a favorable pharmacokinetic profile. luliucci JD, et al., J Clin Pharmacol. 41: 870-9, 2001.

The fixed dose of AP1903 for injection used, for example, may be 0.4 mg/kg intravenously infused over 2 hours. The amount of AP1903 needed in vitro for effective signaling of cells is 10 - 100 nM (1600 Da MW). This equates to 16 - 160 µg/L or -0.016 - 1.6 mg/kg (1.6 -160 µg/kg). Doses up to 1 mg/kg were well-tolerated in the Phase 1 study of AP1903 discussed above. Therefore, 0.4 mg/kg may be a safe and effective dose of AP1903 for this Phase I study in combination with the therapeutic cells.

### Selectable Markers

The expression constructs may contain nucleic acid constructs whose expression is identified in vitro or in vivo by including a marker in the expression construct. Such markers would confer an identifiable change to the cell permitting easy identification of cells containing the expression construct. Usually the inclusion of a drug selection marker aids in cloning and in the selection of transformants. For example, genes that confer resistance to neomycin, puromycin, hygromycin, DHFR, GPT, zeocin and histidinol are useful selectable markers. Alternatively, enzymes such as Herpes Simplex Virus-I thymidine kinase (tk) are employed. Immunologic surface markers containing the extracellular, non-signaling domains or various proteins (e.g. CD34, CD19, LNGFR) also can be employed, permitting a straightforward method for magnetic or fluorescence antibody-mediated sorting. The selectable marker employed is not believed to be important, so long as it is capable of being expressed simultaneously with the nucleic acid encoding a gene product. Further examples of selectable markers include, for example, reporters such as GFP, EGFP, beta-gal or chloramphenicol acetyltransferase (CAT). The marker protein, such as, for example, CD19 may be used for selection of the cells for transfusion, such as, for example, in immunomagnetic selection. As discussed herein, a CD19 marker is distinguished from an anti-CD19 antibody, or, for example, an scFv, TCR, or other antigen recognition moiety that binds to CD19.
A polypeptide may be included in the expression vector to aid in sorting cells. For example, the CD34 minimal epitope may be incorporated into the vector. The expression vectors used to express the chimeric antigen receptors or chimeric stimulating molecules provided herein may further comprise a polynucleotide that encodes the 16 amino acid CD34 minimal epitope. The CD34 minimal epitope may be incorporated at the amino terminal position of the CD8 stalk.

### Transmembrane Regions

A chimeric antigen receptor herein may include a single-pass or multiple pass transmembrane sequence (e.g., at the N-terminus or C-terminus of the chimeric protein). Single pass transmembrane regions are found in certain CD molecules, tyrosine kinase receptors, serine/threonine kinase receptors, TGFβ, BMP, activin and phosphatases. Single pass transmembrane regions often include a signal peptide region and a transmembrane region of about 20 to about 25 amino acids, many of which are hydrophobic amino acids and can form an alpha helix. A short track of positively charged amino acids often follows the transmembrane span to anchor the protein in the membrane. Multiple pass proteins include ion pumps, ion channels, and transporters, and include two or more helices that span the membrane multiple times. All or substantially all of a multiple pass protein sometimes is incorporated in a chimeric protein. Sequences for single pass and multiple pass transmembrane regions are known and can be selected for incorporation into a chimeric protein molecule.

The transmembrane domain may be fused to the extracellular domain of the CAR. The transmembrane domain that naturally may be associated with one of the domains in the CAR is used. Also, a transmembrane domain that is not naturally associated with one of the domains in the CAR may be used. In some instances, the transmembrane domain can be selected or modified by amino acid substitution (e.g., typically charged to a hydrophobic residue) to avoid binding of such domains to the transmembrane domains of the same or different surface membrane proteins to minimize interactions with other members of the receptor complex.

Transmembrane domains may, for example, be derived from the alpha, beta, or zeta chain of the T cell receptor, CD3-ε, CD3 ζ, CD4, CD5, CD8, CD8α, CD9, CD16, CD22, CD28, CD33, CD38, CD64, CD80, CD86, CD134, CD137, or CD154. Or, in some examples, the transmembrane domain may be synthesized *de novo,* comprising mostly hydrophobic residues, such as, for example, leucine and valine. A short polypeptide linker may form the linkage between the transmembrane domain and the intracellular domain of the chimeric antigen receptor. The chimeric antigen receptors may further comprise a stalk, that is, an extracellular region of amino acids between the extracellular domain and the transmembrane domain. For example, the stalk may be a sequence of amino acids naturally associated with the selected transmembrane domain. The chimeric antigen receptor may comprises a CD8 transmembrane domain, the chimeric antigen receptor may comprise a CD8 transmembrane domain, and additional amino acids on the extracellular portion of the transmembrane domain, the chimeric antigen receptor may comprise a CD8 transmembrane domain and a CD8 stalk. The chimeric antigen receptor may further comprise a region of amino acids between the transmembrane domain and the cytoplasmic domain, which are naturally associated with the polypeptide from which the transmembrane domain is derived.

### Control Regions

### Promoters

The particular promoter employed to control the expression of a polynucleotide sequence of interest is not believed to be important, so long as it is capable of directing the expression of the polynucleotide in the targeted cell. Thus, where a human cell is targeted the polynucleotide sequence-coding region may, for example, be placed adjacent to and under the control of a promoter that is capable of being expressed in a human cell. Generally speaking, such a promoter might include either a human or viral promoter.

The human cytomegalovirus (CMV) immediate early gene promoter, the SV40 early promoter, the Rous sarcoma virus long terminal repeat, β-actin, rat insulin promoter and glyceraldehyde-3-phosphate dehydrogenase can be used to obtain high-level expression of the coding sequence of interest. The use of other viral or mammalian cellular or bacterial phage promoters which are well known in the art to achieve expression of a coding sequence of interest is contemplated as well, provided that the levels of expression are sufficient for a given purpose. By employing a promoter with well-known properties, the level and pattern of expression of the protein of interest following transfection or transformation can be optimized.

Selection of a promoter that is regulated in response to specific physiologic or synthetic signals can permit inducible expression of the gene product. For example in the case where expression of a transgene, or transgenes when a multicistronic vector is utilized, is toxic to the cells in which the vector is produced in, it is desirable to prohibit or reduce expression of one or more of the transgenes. Examples of transgenes that are toxic to the producer cell line are pro-apoptotic and cytokine genes. Several inducible promoter systems are available for production of viral vectors where the transgene products are toxic (add in more inducible promoters).

The ecdysone system (Invitrogen, Carlsbad, CA) is one such system. This system is designed to allow regulated expression of a gene of interest in mammalian cells. It consists of a tightly regulated expression mechanism that allows virtually no basal level expression of the transgene, but over 200-fold inducibility. The system is based on the heterodimeric ecdysone receptor of Drosophila, and when ecdysone or an analog such as muristerone A binds to the receptor, the receptor activates a promoter to turn on expression of the downstream transgene high levels of mRNA transcripts are attained. In this system, both monomers of the heterodimeric receptor are constitutively expressed from one vector, whereas the ecdysone-responsive promoter, which drives expression of the gene of interest, is on another plasmid. Engineering of this type of system into the gene transfer vector of interest would therefore be useful. Cotransfection of plasmids containing the gene of interest and the receptor monomers in the producer cell line would then allow for the production of the gene transfer vector without expression of a potentially toxic transgene. At the appropriate time, expression of the transgene could be activated with ecdysone or muristeron A.

Another inducible system that may be useful is the Tet-Off™ or Tet-On™ system (Clontech, Palo Alto, CA) originally developed by Gossen and Bujard (Gossen and Bujard, Proc. Natl. Acad. Sci. USA, 89:5547-5551, 1992; Gossen et al., Science, 268:1766-1769, 1995). This system also allows high levels of gene expression to be regulated in response to tetracycline or tetracycline derivatives such as doxycycline. In the Tet-On™ system, gene expression is turned on in the presence of doxycycline, whereas in the Tet-Off™ system, gene expression is turned on in the absence of doxycycline. These systems are based on two regulatory elements derived from the tetracycline resistance operon of E. coli, he tetracycline operator sequence to which the tetracycline repressor binds, and the tetracycline repressor protein. The gene of interest is cloned into a plasmid behind a promoter that has tetracycline-responsive elements present in it. A second plasmid contains a regulatory element called the tetracycline-controlled transactivator, which is composed, in the Tet-Off™ system, of the VP16 domain from the herpes simplex virus and the wild-type tertracycline repressor. Thus in the absence of doxycycline, transcription is constitutively on. In the Tet-On™ system, the tetracycline repressor is not wild type and in the presence of doxycycline activates transcription. For gene therapy vector production, the Tet-Off™ system may be used so that the producer cells could be grown in the presence of tetracycline or doxycycline and prevent expression of a potentially toxic transgene, but when the vector is introduced to the patient, the gene expression would be constitutively on.

In some circumstances, it is desirable to regulate expression of a transgene in a gene therapy vector. For example, different viral promoters with varying strengths of activity are utilized depending on the level of expression desired. In mammalian cells, the CMV immediate early promoter is often used to provide strong transcriptional activation. The CMV promoter is reviewed in Donnelly, J.J., et al., 1997. Annu. Rev. Immunol. 15:617-48. Modified versions of the CMV promoter that are less potent have also been used when reduced levels of expression of the transgene are desired. When expression of a transgene in hematopoietic cells is desired, retroviral promoters such as the LTRs from MLV or MMTV are often used. Other viral promoters that are used depending on the desired effect include SV40, RSV LTR, HIV-1 and HIV-2 LTR, adenovirus promoters such as from the E1A, E2A, or MLP region, AAV LTR, HSV-TK, and avian sarcoma virus.

In other examples, promoters may be selected that are developmentally regulated and are active in particular differentiated cells. Thus, for example, a promoter may not be active in a pluripotent stem cell, but, for example, where the pluripotent stem cell differentiates into a more mature cell, the promoter may then be activated.

Similarly tissue specific promoters are used to effect transcription in specific tissues or cells so as to reduce potential toxicity or undesirable effects to non-targeted tissues. These promoters may result in reduced expression compared to a stronger promoter such as the CMV promoter, but may also result in more limited expression, and immunogenicity (Bojak, A., et al., 2002. Vaccine. 20:1975-79; Cazeaux., N., et al., 2002. Vaccine 20:3322-31). For example, tissue specific promoters such as the PSA associated promoter or prostate-specific glandular kallikrein, or the muscle creatine kinase gene may be used where appropriate.

Examples of tissue specific or differentiation specific promoters include, but are not limited to, the following: B29 (B cells); CD14 (monocytic cells); CD43 (leukocytes and platelets); CD45 (hematopoietic cells); CD68 (macrophages); desmin (muscle); elastase-1 (pancreatic acinar cells); endoglin (endothelial cells); fibronectin (differentiating cells, healing tissues); and Flt-1 (endothelial cells); GFAP (astrocytes).

In certain indications, it is desirable to activate transcription at specific times after administration of the gene therapy vector. This is done with such promoters as those that are hormone or cytokine regulatable. Cytokine and inflammatory protein responsive promoters that can be used include K and T kininogen (Kageyama et al., (1987) J. Biol. Chem., 262,2345-2351), c-fos, TNF-alpha, C-reactive protein (Arcone, et al., (1988) Nucl. Acids Res., 16(8), 3195-3207), haptoglobin (Oliviero et al., (1987) EMBO J., 6, 1905-1912), serum amyloid A2, C/EBP alpha, IL-1, IL-6 (Poli and Cortese, (1989) Proc. Nat'l Acad. Sci. USA, 86,8202-8206), Complement C3 (Wilson et al., (1990) Mol. Cell. Biol., 6181-6191), IL-8, alpha-1 acid glycoprotein (Prowse and Baumann, (1988) Mol Cell Biol, 8,42-51), alpha-1 antitrypsin, lipoprotein lipase (Zechner et al., Mol. Cell. Biol., 2394-2401, 1988), angiotensinogen (Ron, et al., (1991) Mol. Cell. Biol., 2887-2895), fibrinogen, c-jun (inducible by phorbol esters, TNF-alpha, UV radiation, retinoic acid, and hydrogen peroxide), collagenase (induced by phorbol esters and retinoic acid), metallothionein (heavy metal and glucocorticoid inducible), Stromelysin (inducible by phorbol ester, interleukin-1 and EGF), alpha-2 macroglobulin and alpha-1 anti-chymotrypsin. Other promoters include, for example, SV40, MMTV, Human Immunodeficiency Virus (MV), Moloney virus, ALV, Epstein Barr virus, Rous Sarcoma virus, human actin, myosin, hemoglobin, and creatine.

It is envisioned that any of the above promoters alone or in combination with another can be useful depending on the action desired. Promoters, and other regulatory elements, are selected such that they are functional in the desired cells or tissue. In addition, this list of promoters should not be construed to be exhaustive or limiting; other promoters that are used in conjunction with the promoters and methods disclosed herein.

### Enhancers

Enhancers are genetic elements that increase transcription from a promoter located at a distant position on the same molecule of DNA. Early examples include the enhancers associated with immunoglobulin and T cell receptors that both flank the coding sequence and occur within several introns. Many viral promoters, such as CMV, SV40, and retroviral LTRs are closely associated with enhancer activity and are often treated like single elements. Enhancers are organized much like promoters. That is, they are composed of many individual elements, each of which binds to one or more transcriptional proteins. The basic distinction between enhancers and promoters is operational. An enhancer region as a whole stimulates transcription at a distance and often independent of orientation; this need not be true of a promoter region or its component elements. On the other hand, a promoter has one or more elements that direct initiation of RNA synthesis at a particular site and in a particular orientation, whereas enhancers lack these specificities. Promoters and enhancers are often overlapping and contiguous, often seeming to have a very similar modular organization. A subset of enhancers is locus-control regions (LCRs) that can not only increase transcriptional activity, but (along with insulator elements) can also help to insulate the transcriptional element from adjacent sequences when integrated into the genome.
Any promoter/enhancer combination (as per the Eukaryotic Promoter Data Base EPDB) can be used to drive expression of the gene, although many will restrict expression to a particular tissue type or subset of tissues (reviewed in, for example, Kutzler, M.A., and Weiner, D.B., 2008. Nature Reviews Genetics 9:776-88). Examples include, but are not limited to, enhancers from the human actin, myosin, hemoglobin, muscle creatine kinase, sequences, and from viruses CMV, RSV, and EBV. Appropriate enhancers may be selected for particular applications. Eukaryotic cells can support cytoplasmic transcription from certain bacterial promoters if the appropriate bacterial polymerase is provided, either as part of the delivery complex or as an additional genetic expression construct.

### Polyadenylation Signals

Where a cDNA insert is employed, one will typically desire to include a polyadenylation signal to effect proper polyadenylation of the gene transcript. The nature of the polyadenylation signal is not believed to be crucial to the successful practice of the present methods, and any such sequence is employed such as human or bovine growth hormone and SV40 polyadenylation signals and LTR polyadenylation signals. One non-limiting example is the SV40 polyadenylation signal present in the pCEP3 plasmid (Invitrogen, Carlsbad, California). Also, contemplated as an element of the expression cassette is a terminator. These elements can serve to enhance message levels and to minimize read through from the cassette into other sequences. Termination or poly(A) signal sequences may be, for example, positioned about 11-30 nucleotides downstream from a conserved sequence (AAUAAA) at the 3' end of the mRNA (Montgomery, D.L., et al., 1993. DNA Cell Biol. 12:777-83; Kutzler, M.A., and Weiner, D.B., 2008. Nature Rev. Gen. 9:776-88).

### 4. Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. The initiation codon is placed in-frame with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

The use of internal ribosome entry sites (IRES) elements may be used to create multigene, or polycistronic messages. IRES elements are able to bypass the ribosome-scanning model of 5' methylated cap-dependent translation and begin translation at internal sites (Pelletier and Sonenberg, Nature, 334:320-325, 1988). IRES elements from two members of the picornavirus family (polio and encephalomyocarditis) have been discussed (Pelletier and Sonenberg, 1988), as well an IRES from a mammalian message (Macejak and Sarnow, Nature, 353:90-94, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

### Sequence Optimization

Protein production may also be increased by optimizing the codons in the transgene. Species specific codon changes may be used to increase protein production. Also, codons may be optimized to produce an optimized RNA, which may result in more efficient translation. By optimizing the codons to be incorporated in the RNA, elements such as those that result in a secondary structure that causes instability, secondary mRNA structures that can, for example, inhibit ribosomal binding, or cryptic sequences that can inhibit nuclear export of mRNA can be removed (Kutzler, M.A., and Weiner, D.B., 2008. Nature Rev. Gen. 9:776-88; Yan, J. et al., 2007. Mol. Ther. 15:411-21; Cheung, Y.K., et al., 2004. Vaccine 23:629-38; Narum., D.L., et al., 2001. 69:7250-55; Yadava, A., and Ockenhouse, C.F., 2003. Infect. Immun. 71:4962-69; Smith., J.M., et al., 2004. AIDS Res. Hum. Retroviruses 20:1335-47; Zhou, W., et al., 2002. Vet. Microbiol. 88:127-51; Wu, X., et al., 2004. Biochem. Biophys. Res. Commun. 313:89-96; Zhang, W., et al., 2006. Biochem. Biophys. Res. Commun. 349:69-78; Deml, L.A., et al., 2001. J. Virol. 75:1099-11001; Schneider, R. M., et al., 1997. J. Virol. 71:4892-4903; Wang, S.D., et al., 2006. Vaccine 24:4531-40; zur Megede, J., et al., 2000. J. Virol. 74:2628-2635). For example, the FBP12, the Caspase polypeptide, and the CD19 sequences may be optimized by changes in the codons.

### Leader Sequences

Leader sequences may be added to enhance the stability of mRNA and result in more efficient translation. The leader sequence is usually involved in targeting the mRNA to the endoplasmic reticulum. Examples include the signal sequence for the HIV-1 envelope glycoprotein (Env), which delays its own cleavage, and the IgE gene leader sequence (Kutzler, M.A., and Weiner, D.B., 2008. Nature Rev. Gen. 9:776-88; Li, V., et al., 2000. Virology 272:417-28; Xu, Z.L., et al. 2001. Gene 272:149-56; Malin, A.S., et al., 2000. Microbes Infect. 2:1677-85; Kutzler, M.A., et al., 2005. J. Immunol. 175:112-125; Yang, J.S., et al., 2002. Emerg. Infect. Dis. 8:1379-84; Kumar., S., et al., 2006. DNA Cell Biol. 25:383-92; Wang, S., et al., 2006. Vaccine 24:4531-40). The IgE leader may be used to enhance insertion into the endoplasmic reticulum (Tepler, I, et al. (1989) J. Biol. Chem. 264:5912).

Expression of the transgenes may be optimized and/or controlled by the selection of appropriate methods for optimizing expression. These methods include, for example, optimizing promoters, delivery methods, and gene sequences, (for example, as presented in Laddy, D.J., et al., 2008. PLoS.ONE 3 e2517; Kutzler, M.A., and Weiner, D.B., 2008. Nature Rev. Gen. 9:776-88).

### Nucleic Acids

A "nucleic acid" as used herein generally refers to a molecule (one, two or more strands) of DNA, RNA or a derivative or analog thereof, comprising a nucleobase. A nucleobase includes, for example, a naturally occurring purine or pyrimidine base found in DNA (e.g., an adenine "A," a guanine "G," a thymine "T" or a cytosine "C") or RNA (e.g., an A, a G, an uracil "U" or a C). The term "nucleic acid" encompasses the terms "oligonucleotide" and "polynucleotide," each as a subgenus of the term "nucleic acid." Nucleic acids may be, be at least, be at most, or be about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides, or any range derivable therein, in length.

Nucleic acids herein provided may have regions of identity or complementarity to another nucleic acid. It is contemplated that the region of complementarity or identity can be at least 5 contiguous residues, though it is specifically contemplated that the region is, is at least, is at most, or is about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 contiguous nucleotides.

As used herein, "hybridization", "hybridizes" or "capable of hybridizing" is understood to mean forming a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but preclude hybridization of random sequences. Stringent conditions tolerate little, if any, mismatch between a nucleic acid and a target strand. Such conditions are known, and are often used for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.5 M NaCl at temperatures of about 42 degrees C to about 70 degrees C. It is understood that the temperature and ionic strength of a desired stringency are determined in part by the length of the particular nucleic acid(s), the length and nucleobase content of the target sequence(s), the charge composition of the nucleic acid(s), and the presence or concentration of formamide, tetramethylammonium chloride or other solvent(s) in a hybridization mixture.

It is understood that these ranges, compositions and conditions for hybridization are mentioned by way of non-limiting examples only, and that the desired stringency for a particular hybridization reaction is often determined empirically by comparison to one or more positive or negative controls. Depending on the application envisioned varying conditions of hybridization may be employed to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions," and non-limiting examples of low stringency include hybridization performed at about 0.15 M to about 0.9 M NaCl at a temperature range of about 20 degrees C. to about 50 degrees C. The low or high stringency conditions may be further modified to suit a particular application.

### Nucleic Acid Modification

Any of the modifications discussed below may be applied to a nucleic acid. Examples of modifications include alterations to the RNA or DNA backbone, sugar or base, and various combinations thereof. Any suitable number of backbone linkages, sugars and/or bases in a nucleic acid can be modified (e.g., independently about 5%, 10%, 15%, 20%, 25%, 30%, 35%, 40%, 45%, 50%, 55%, 60%, 65%, 70%, 75%, 80%, 85%, 90%, 95%, up to 100%). An unmodified nucleoside is any one of the bases adenine, cytosine, guanine, thymine, or uracil joined to the 1' carbon of beta-D-ribo-furanose.

A modified base is a nucleotide base other than adenine, guanine, cytosine and uracil at a 1' position. Non-limiting examples of modified bases include inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e. g., 5-methylcytidine), 5-alkyluridines (e. g., ribothymidine), 5-halouridine (e. g., 5-bromouridine) or 6-azapyrimidines or 6-alkylpyrimidines (e. g. 6- methyluridine), propyne, and the like. Other non-limiting examples of modified bases include nitropyrrolyl (e.g., 3-nitropyrrolyl), nitroindolyl (e.g., 4-, 5-, 6-nitroindolyl), hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, difluorotolyl, 4-fluoro-6-methylbenzimidazole, 4-methylbenzimidazole, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl and the like.

For example, a nucleic acid may comprise modified nucleic acid molecules, with phosphate backbone modifications. Non-limiting examples of backbone modifications include phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl modifications. In certain instances, a ribose sugar moiety that naturally occurs in a nucleoside is replaced with a hexose sugar, polycyclic heteroalkyl ring, or cyclohexenyl group. In certain instances, the hexose sugar is an allose, altrose, glucose, mannose, gulose, idose, galactose, talose, or a derivative thereof. The hexose may be a D-hexose, glucose, or mannose. In certain instances, the polycyclic heteroalkyl group may be a bicyclic ring containing one oxygen atom in the ring. In certain instances, the polycyclic heteroalkyl group is a bicyclo[2.2.1]heptane, a bicyclo[3.2.1]octane, or a bicyclo[3.3.1]nonane.

Nitropyrrolyl and nitroindolyl nucleobases are members of a class of compounds known as universal bases. Universal bases are those compounds that can replace any of the four naturally occurring bases without substantially affecting the melting behavior or activity of the oligonucleotide duplex. In contrast to the stabilizing, hydrogen-bonding interactions associated with naturally occurring nucleobases, oligonucleotide duplexes containing 3-nitropyrrolyl nucleobases may be stabilized solely by stacking interactions. The absence of significant hydrogen-bonding interactions with nitropyrrolyl nucleobases obviates the specificity for a specific complementary base. In addition, 4-, 5- and 6-nitroindolyl display very little specificity for the four natural bases. Procedures for the preparation of 1-(2'-O-methyl-.beta.-D-ribofuranosyl)-5-nitroindole are discussed in Gaubert, G.; Wengel, J. Tetrahedron Letters 2004, 45, 5629. Other universal bases include hypoxanthinyl, isoinosinyl, 2-aza-inosinyl, 7-deaza-inosinyl, nitroimidazolyl, nitropyrazolyl, nitrobenzimidazolyl, nitroindazolyl, aminoindolyl, pyrrolopyrimidinyl, and structural derivatives thereof.

Difluorotolyl is a non-natural nucleobase that functions as a universal base. Difluorotolyl is an isostere of the natural nucleobase thymine. But unlike thymine, difluorotolyl shows no appreciable selectivity for any of the natural bases. Other aromatic compounds that function as universal bases are 4-fluoro-6-methylbenzimidazole and 4-methylbenzimidazole. In addition, the relatively hydrophobic isocarbostyrilyl derivatives 3-methyl isocarbostyrilyl, 5-methyl isocarbostyrilyl, and 3-methyl-7-propynyl isocarbostyrilyl are universal bases which cause only slight destabilization of oligonucleotide duplexes compared to the oligonucleotide sequence containing only natural bases. Other non-natural nucleobases include 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenyl, tetracenyl, pentacenyl, and structural derivates thereof. For a more detailed discussion, including synthetic procedures, of difluorotolyl, 4-fluoro-6-methylbenzimidazole, 4-methylbenzimidazole, and other non-natural bases mentioned above, see: Schweitzer et al., J. Org. Chem., 59:7238-7242 (1994);

In addition, chemical substituents, for example cross-linking agents, may be used to add further stability or irreversibility to the reaction. Non-limiting examples of cross-linking agents include, for example, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl) dithio]propioimidate.

A nucleotide analog may also include a "locked" nucleic acid. Certain compositions can be used to essentially "anchor" or "lock" an endogenous nucleic acid into a particular structure. Anchoring sequences serve to prevent disassociation of a nucleic acid complex, and thus not only can prevent copying but may also enable labeling, modification, and/or cloning of the endogeneous sequence. The locked structure may regulate gene expression (i.e. inhibit or enhance transcription or replication), or can be used as a stable structure that can be used to label or otherwise modify the endogenous nucleic acid sequence, or can be used to isolate the endogenous sequence, i.e. for cloning.

Nucleic acid molecules need not be limited to those molecules containing only RNA or DNA, but further encompass chemically-modified nucleotides and non-nucleotides. The percent of non-nucleotides or modified nucleotides may be from 1% to 100% (e.g., about 5, 10, 15, 20, 25, 30, 35, 40, 45, 50, 55, 60, 65, 70, 75, 80, 85, 90 or 95%).

### Nucleic Acid Preparation

A nucleic acid may be provided for use as a control or standard in an assay, or therapeutic, for example. A nucleic acid may be made by any technique known in the art, such as for example, chemical synthesis, enzymatic production or biological production. Nucleic acids may be recovered or isolated from a biological sample. The nucleic acid may be recombinant or it may be natural or endogenous to the cell (produced from the cell's genome). It is contemplated that a biological sample may be treated in a way so as to enhance the recovery of small nucleic acid molecules. Generally, methods may involve lysing cells with a solution having guanidinium and a detergent.

Nucleic acid synthesis may also be performed according to standard methods. Non-limiting examples of a synthetic nucleic acid (e.g., a synthetic oligonucleotide), include a nucleic acid made by in vitro chemical synthesis using phosphotriester, phosphite, or phosphoramidite chemistry and solid phase techniques or via deoxynucleoside H-phosphonate intermediates. Various different mechanisms of oligonucleotide synthesis have been disclosed elsewhere.

Nucleic acids may be isolated using known techniques. Methods for isolating small nucleic acid molecules, and/or isolating RNA molecules can be employed. Chromatography is a process used to separate or isolate nucleic acids from protein or from other nucleic acids. Such methods can involve electrophoresis with a gel matrix, filter columns, alcohol precipitation, and/or other chromatography. If a nucleic acid from cells is to be used or evaluated, methods generally involve lysing the cells with a chaotropic (e.g., guanidinium isothiocyanate) and/or detergent (e.g., N-lauroyl sarcosine) prior to implementing processes for isolating particular populations of RNA.
Methods may involve the use of organic solvents and/or alcohol to isolate nucleic acids. The amount of alcohol added to a cell lysate may achieve an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column is effective for such isolation procedures.

A nucleic acid isolation processes may sometimes include: a) lysing cells in the sample with a lysing solution comprising guanidinium, where a lysate with a concentration of at least about 1 M guanidinium is produced; b) extracting nucleic acid molecules from the lysate with an extraction solution comprising phenol; c) adding to the lysate an alcohol solution to form a lysate/alcohol mixture, wherein the concentration of alcohol in the mixture is between about 35% to about 70%; d) applying the lysate/alcohol mixture to a solid support; e) eluting the nucleic acid molecules from the solid support with an ionic solution; and, f) capturing the nucleic acid molecules. The sample may be dried down and resuspended in a liquid and volume appropriate for subsequent manipulation.

Provided herein are compositions or.kits that comprise nucleic acid comprising the polynucleotides of the present application. Thus, compositions or kits may, for example, comprise both the first and second polynucleotides, encoding the first and second chimeric polypeptides. The nucleic acid may comprise more than one nucleic acid species, that is, for example, the first nucleic acid species comprises the first polynucleotide, and the second nucleic acid species comprises the second polynucleotide. In other examples, the nucleic acid may comprise both the first and second polynucleotides. The kit may, in addition, comprise the first or second ligand, or both.

### Methods of Gene Transfer

In order to mediate the effect of the transgene expression in a cell, it will be necessary to transfer the expression constructs into a cell. Such transfer may employ viral or non-viral methods of gene transfer. This section provides a discussion of methods and compositions of gene transfer.
A transformed cell comprising an expression vector is generated by introducing into the cell the expression vector. Suitable methods for polynucleotide delivery for transformation of an organelle, a cell, a tissue or an organism for use with the current methods include virtually any method by which a polynucleotide (e.g., DNA) can be introduced into an organelle, a cell, a tissue or an organism.

A host cell can, and has been, used as a recipient for vectors. Host cells may be derived from prokaryotes or eukaryotes, depending upon whether the desired result is replication of the vector or expression of part or all of the vector-encoded polynucleotide sequences. Numerous cell lines and cultures are available for use as a host cell, and they can be obtained through the American Type Culture Collection (ATCC), which is an organization that serves as an archive for living cultures and genetic materials.

An appropriate host may be determined. Generally this is based on the vector backbone and the desired result. A plasmid or cosmid, for example, can be introduced into a prokaryote host cell for replication of many vectors. Bacterial cells used as host cells for vector replication and/or expression include DH5alpha, JM109, and KC8, as well as a number of commercially available bacterial hosts such as SURE® Competent Cells and SOLOPACK Gold Cells (STRATAGENE®, La Jolla, CA). Alternatively, bacterial cells such as E. coli LE392 could be used as host cells for phage viruses. Eukaryotic cells that can be used as host cells include, but are not limited to yeast, insects and mammals. Examples of mammalian eukaryotic host cells for replication and/or expression of a vector include, but are not limited to, HeLa, NIH3T3, Jurkat, 293, COS, CHO, Saos, and PC12. Examples of yeast strains include, but are not limited to, YPH499, YPH500 and YPH501.

Nucleic acid vaccines may include, for example, non-viral DNA vectors, "naked" DNA and RNA, and viral vectors. Methods of transforming cells with these vaccines, and for optimizing the expression of genes included in these vaccines are known and are also discussed herein.

### Examples of Methods of Nucleic Acid or Viral Vector Transfer

Any appropriate method may be used to transfect or transform the cells, or to administer the nucleotide sequences or compositions of the present methods. Certain examples are presented herein, and further include methods such as delivery using cationic polymers, lipid like molecules, and certain commercial products such as, for example, IN-VIVO-JET PEI.

### Ex vivo Transformation

Various methods are available for transfecting vascular cells and tissues removed from an organism in an ex vivo setting. For example, canine endothelial cells have been genetically altered by retroviral gene transfer in vitro and transplanted into a canine (Wilson et al., Science, 244:1344-1346, 1989). In another example, Yucatan minipig endothelial cells were transfected by retrovirus in vitro and transplanted into an artery using a double-balloon catheter (Nabel et al., Science, 244(4910):1342-1344, 1989). Thus, it is contemplated that cells or tissues may be removed and transfected ex vivo using the polynucleotides presented herein. In particular aspects, the transplanted cells or tissues may be placed into an organism.

### Injection

An antigen presenting cell or a nucleic acid or viral vector may be delivered to an organelle, a cell, a tissue or an organism via one or more injections (i.e., a needle injection), such as, for example, subcutaneous, intradermal, intramuscular, intravenous, intraprotatic, intratumor, intraperitoneal, etc. Methods of injection include, for example, injection of a composition comprising a saline solution. Further aspects include the introduction of a polynucleotide by direct microinjection. The amount of the expression vector used may vary upon the nature of the antigen as well as the organelle, cell, tissue or organism used.
Intradermal, intranodal, or intralymphatic injections are some of the more commonly used methods of DC administration. Intradermal injection is characterized by a low rate of absorption into the bloodstream but rapid uptake into the lymphatic system. The presence of large numbers of Langerhans dendritic cells in the dermis will transport intact as well as processed antigen to draining lymph nodes. Proper site preparation is necessary to perform this correctly (i.e., hair is clipped in order to observe proper needle placement). Intranodal injection allows for direct delivery of antigen to lymphoid tissues. Intralymphatic injection allows direct administration of DCs.

### Electroporation

A polynucleotide may be introduced into an organelle, a cell, a tissue or an organism via electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent No. 5,384,253).

Transfection of eukaryotic cells using electroporation has been quite successful. Mouse pre-B lymphocytes have been transfected with human kappa-immunoglobulin genes (Potter et al., (1984) Proc. Nat'l Acad. Sci. USA, 81, 7161-7165), and rat hepatocytes have been transfected with the chloramphenicol acetyltransferase gene (Tur-Kaspa et al., (1986) Mol. Cell Biol., 6,716-718) in this manner.

*In vivo* electroporation for vaccines, or eVac, is clinically implemented through a simple injection technique. A DNA vector encoding a polypeptide is injected intradermally in a patient. Then electrodes apply electrical pulses to the intradermal space causing the cells localized there, especially resident dermal dendritic cells, to take up the DNA vector and express the encoded polypeptide. These polypeptide-expressing cells activated by local inflammation can then migrate to lymph-nodes, presenting antigens, for example. A nucleic acid is electroporetically administered when it is administered using electroporation, following, for example, but not limited to, injection of the nucleic acid or any other means of administration where the nucleic acid may be delivered to the cells by electroporation
Methods of electroporation are discussed in, for example, Sardesai, N.Y., and Weiner, D.B., Current Opinion in Immunotherapy 23:421-9 (2011) and Ferraro, B. et al., Human Vaccines 7:120-127 (2011).

### Calcium Phosphate

A polynucleotide may be introduced to the cells using calcium phosphate precipitation. Human KB cells have been transfected with adenovirus 5 DNA (Graham and van der Eb, (1973) Virology, 52,456-467) using this technique. Also in this manner, mouse L(A9), mouse C127, CHO, CV-1, BHK, NIH3T3 and HeLa cells were transfected with a neomycin marker gene (Chen and Okayama, Mol. Cell Biol., 7(8):2745-2752, 1987), and rat hepatocytes were transfected with a variety of marker genes (Rippe et al., Mol. Cell Biol., 10:689-695, 1990).

### DEAE-Dextran

A polynucleotide may be delivered into a cell using DEAE-dextran followed by polyethylene glycol. In this manner, reporter plasmids were introduced into mouse myeloma and erythroleukemia cells (Gopal, T.V., Mol Cell Biol. 1985 May;5(5):1188-90).

### Sonication Loading

Additional aspects include the introduction of a polynucleotide by direct sonic loading. LTK- fibroblasts have been transfected with the thymidine kinase gene by sonication loading (Fechheimer et al., (1987) Proc. Nat'l Acad. Sci. USA, 84,8463-8467).

### Liposome-Mediated Transfection

A polynucleotide may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, (1991) In: Liver Diseases, Targeted Diagnosis and Therapy Using Specific Receptors and Ligands. pp. 87-104). Also contemplated is a polynucleotide complexed with Lipofectamine (Gibco BRL) or Superfect (Qiagen).

### Receptor Mediated Transfection

Still further, a polynucleotide may be delivered to a target cell via receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity.
Certain receptor-mediated gene targeting vehicles comprise a cell receptor-specific ligand and a polynucleotide-binding agent. Others comprise a cell receptor-specific ligand to which the polynucleotide to be delivered has been operatively attached. Several ligands have been used for receptor-mediated gene transfer (Wu and Wu, (1987) J. Biol. Chem., 262, 4429-4432; Wagner et al., Proc. Natl. Acad. Sci. USA, 87(9):3410-3414, 1990; Perales et al., Proc. Natl. Acad. Sci. USA, 91:4086-4090, 1994; Myers, EPO 0273085), which establishes the operability of the technique. Specific delivery in the context of another mammalian cell type has been discussed (Wu and Wu, Adv. Drug Delivery Rev., 12:159-167, 1993). In certain aspects, a ligand is chosen to correspond to a receptor specifically expressed on the target cell population.
A polynucleotide delivery vehicle component of a cell-specific polynucleotide-targeting vehicle may comprise a specific binding ligand in combination with a liposome. The polynucleotide(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a polynucleotide to cells that exhibit upregulation of the EGF receptor.

The polynucleotide delivery vehicle component of a targeted delivery vehicle may be a liposome itself, which may, for example, comprise one or more lipids or glycoproteins that direct cell-specific binding. For example, lactosyl-ceramide, a galactose-terminal asialoganglioside, have been incorporated into liposomes and observed an increase in the uptake of the insulin gene by hepatocytes (Nicolau et al., (1987) Methods Enzymol., 149,157-176). It is contemplated that the tissue-specific transforming constructs may be specifically delivered into a target cell in a similar manner.

### Microprojectile Bombardment

Microprojectile bombardment techniques can be used to introduce a polynucleotide into at least one, organelle, cell, tissue or organism (U.S. Patent No. 5,550,318; U.S. Patent No. 5,538,880; U.S. Patent No. 5,610,042; and PCT Application WO 94/09699). This method depends on the ability to accelerate DNA-coated microprojectiles to a high velocity allowing them to pierce cell membranes and enter cells without killing them (Klein et al., (1987) Nature, 327, 70-73). There are a wide variety of microprojectile bombardment techniques known in the art, many of which are applicable to the present methods.
In this microprojectile bombardment, one or more particles may be coated with at least one polynucleotide and delivered into cells by a propelling force. Several devices for accelerating small particles have been developed. One such device relies on a high voltage discharge to generate an electrical current, which in turn provides the motive force (Yang et al., (1990) Proc. Nat'l Acad. Sci. USA, 87, 9568-9572). The microprojectiles used have consisted of biologically inert substances such as tungsten or gold particles or beads. Exemplary particles include those comprised of tungsten, platinum, and, in certain examples, gold, including, for example, nanoparticles. It is contemplated that in some instances DNA precipitation onto metal particles would not be necessary for DNA delivery to a recipient cell using microprojectile bombardment. However, it is contemplated that particles may contain DNA rather than be coated with DNA. DNA-coated particles may increase the level of DNA delivery via particle bombardment but are not, in and of themselves, necessary.

### Examples of Methods of Viral Vector-Mediated Transfer

Any viral vector suitable for administering nucleotide sequences, or compositions comprising nucleotide sequences, to a cell or to a subject, such that the cell or cells in the subject may express the genes encoded by the nucleotide sequences may be employed in the present methods. A transgene may be incorporated into a viral particle to mediate gene transfer to a cell. Typically, the virus simply will be exposed to the appropriate host cell under physiologic conditions, permitting uptake of the virus. The present methods are advantageously employed using a variety of viral vectors, as discussed below.

### Adenovirus

Adenovirus is particularly suitable for use as a gene transfer vector because of its mid-sized DNA genome, ease of manipulation, high titer, wide target-cell range, and high infectivity. The roughly 36 kb viral genome is bounded by 100-200 base pair (bp) inverted terminal repeats (ITR), in which are contained cis-acting elements necessary for viral DNA replication and packaging. The early (E) and late (L) regions of the genome that contain different transcription units are divided by the onset of viral DNA replication.
The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression, and host cell shut off (Renan, M. J. (1990) Radiother Oncol., 19, 197-218). The products of the late genes (L1, L2, L3, L4 and L5), including the majority of the viral capsid proteins, are expressed only after significant processing of a single primary transcript issued by the major late promoter (MLP). The MLP (located at 16.8 map units) is particularly efficient during the late phase of infection, and all the mRNAs issued from this promoter possess a 5' tripartite leader (TL) sequence, which makes them useful for translation.

In order for adenovirus to be optimized for gene therapy, it is necessary to maximize the carrying capacity so that large segments of DNA can be included. It also is very desirable to reduce the toxicity and immunologic reaction associated with certain adenoviral products. The two goals are, to an extent, coterminous in that elimination of adenoviral genes serves both ends. By practice of the present methods, it is possible to achieve both these goals while retaining the ability to manipulate the therapeutic constructs with relative ease.

The large displacement of DNA is possible because the cis elements required for viral DNA replication all are localized in the inverted terminal repeats (ITR) (100-200 bp) at either end of the linear viral genome. Plasmids containing ITR's can replicate in the presence of a non-defective adenovirus (Hay, R.T., et al., J Mol Biol. 1984 Jun 5; 175(4):493-510). Therefore, inclusion of these elements in an adenoviral vector may permits replication.

In addition, the packaging signal for viral encapsulation is localized between 194-385 bp (0.5-1.1 map units) at the left end of the viral genome (Hearing et al., J. (1987) Virol., 67, 2555-2558). This signal mimics the protein recognition site in bacteriophage lambda DNA where a specific sequence close to the left end, but outside the cohesive end sequence, mediates the binding to proteins that are required for insertion of the DNA into the head structure. E1 substitution vectors of Ad have demonstrated that a 450 bp (0-1.25 map units) fragment at the left end of the viral genome could direct packaging in 293 cells (Levrero et al., Gene, 101:195-202, 1991).

Previously, it has been shown that certain regions of the adenoviral genome can be incorporated into the genome of mammalian cells and the genes encoded thereby expressed. These cell lines are capable of supporting the replication of an adenoviral vector that is deficient in the adenoviral function encoded by the cell line. There also have been reports of complementation of replication deficient adenoviral vectors by "helping" vectors, e.g., wild-type virus or conditionally defective mutants.

Replication-deficient adenoviral vectors can be complemented, in trans, by helper virus. This observation alone does not permit isolation of the replication-deficient vectors, however, since the presence of helper virus, needed to provide replicative functions, would contaminate any preparation. Thus, an additional element was needed that would add specificity to the replication and/or packaging of the replication-deficient vector. That element derives from the packaging function of adenovirus.
It has been shown that a packaging signal for adenovirus exists in the left end of the conventional adenovirus map (Tibbetts et. al. (1977) Cell, 12,243-249). Later studies showed that a mutant with a deletion in the E1A (194-358 bp) region of the genome grew poorly even in a cell line that complemented the early (E1A) function (Hearing and Shenk, (1983) J. Mol. Biol. 167,809-822). When a compensating adenoviral DNA (0-353 bp) was recombined into the right end of the mutant, the virus was packaged normally. Further mutational analysis identified a short, repeated, position-dependent element in the left end of the Ad5 genome. One copy of the repeat was found to be sufficient for efficient packaging if present at either end of the genome, but not when moved toward the interior of the Ad5 DNA molecule (Hearing et al., J. (1987) Virol., 67, 2555-2558).

By using mutated versions of the packaging signal, it is possible to create helper viruses that are packaged with varying efficiencies. Typically, the mutations are point mutations or deletions. When helper viruses with low efficiency packaging are grown in helper cells, the virus is packaged, albeit at reduced rates compared to wild-type virus, thereby permitting propagation of the helper. When these helper viruses are grown in cells along with virus that contains wild-type packaging signals, however, the wild-type packaging signals are recognized preferentially over the mutated versions. Given a limiting amount of packaging factor, the virus containing the wild-type signals is packaged selectively when compared to the helpers. If the preference is great enough, stocks approaching homogeneity may be achieved.

To improve the tropism of ADV constructs for particular tissues or species, the receptor-binding fiber sequences can often be substituted between adenoviral isolates. For example the Coxsackie-adenovirus receptor (CAR) ligand found in adenovirus 5 can be substituted for the CD46-binding fiber sequence from adenovirus 35, making a virus with greatly improved binding affinity for human hematopoietic cells. The resulting "pseudotyped" virus, Ad5f35, has been the basis for several clinically developed viral isolates. Moreover, various biochemical methods exist to modify the fiber to allow re-targeting of the virus to target cells. Methods include use of bifunctional antibodies (with one end binding the CAR ligand and one end binding the target sequence), and metabolic biotinylation of the fiber to permit association with customized avidin-based chimeric ligands. Alternatively, one could attach ligands (e.g. anti-CD205 by heterobifunctional linkers (e.g. PEG-containing), to the adenovirus particle.

### Retrovirus

The retroviruses are a group of single-stranded RNA viruses characterized by an ability to convert their RNA to double-stranded DNA in infected cells by a process of reverse-transcription (Coffin, (1990) In: Virology, ed., New York: Raven Press, pp. 1437-1500). The resulting DNA then stably integrates into cellular chromosomes as a provirus and directs synthesis of viral proteins. The integration results in the retention of the viral gene sequences in the recipient cell and its descendants. The retroviral genome contains three genes - gag, pol and env - that code for capsid proteins, polymerase enzyme, and envelope components, respectively. A sequence found upstream from the gag gene, termed psi, functions as a signal for packaging of the genome into virions. Two long terminal repeat (LTR) sequences are present at the 5' and 3' ends of the viral genome. These contain strong promoter and enhancer sequences and also are required for integration in the host cell genome (Coffin, 1990). Thus, for example, the present technology includes, for example, cells whereby the polynucleotide used to transduce the cell is integrated into the genome of the cell.

In order to construct a retroviral vector, a nucleic acid encoding a promoter is inserted into the viral genome in the place of certain viral sequences to produce a virus that is replication-defective. In order to produce virions, a packaging cell line containing the gag, pol and env genes but without the LTR and psi components is constructed (Mann et al., (1983) Cell, 33,153-159). When a recombinant plasmid containing a human cDNA, together with the retroviral LTR and psi sequences is introduced into this cell line (by calcium phosphate precipitation for example), the psi sequence allows the RNA transcript of the recombinant plasmid to be packaged into viral particles, which are then secreted into the culture media (Nicolas, J.F., and Rubenstein, J.L.R., (1988) In: Vectors: a Survey of Molecular Cloning Vectors and Their Uses, Rodriquez and Denhardt, Eds.). Nicolas and Rubenstein; Temin et al., (1986) In: Gene Transfer, Kucherlapati (ed.), and New York: Plenum Press, pp. 149-188; Mann et al., 1983). The media containing the recombinant retroviruses is collected, optionally concentrated, and used for gene transfer. Retroviral vectors are able to infect a broad variety of cell types. However, integration and stable expression of many types of retroviruses require the division of host cells (Paskind et al., (1975) Virology, 67,242-248).
An approach designed to allow specific targeting of retrovirus vectors recently was developed based on the chemical modification of a retrovirus by the chemical addition of galactose residues to the viral envelope. This modification could permit the specific infection of cells such as hepatocytes via asialoglycoprotein receptors, may be desired.
A different approach to targeting of recombinant retroviruses was designed, which used biotinylated antibodies against a retroviral envelope protein and against a specific cell receptor. The antibodies were coupled via the biotin components by using streptavidin (Roux et al., (1989) Proc. Nat'l Acad. Sci. USA, 86, 9079-9083). Using antibodies against major histocompatibility complex class I and class II antigens, the infection of a variety of human cells that bore those surface antigens was demonstrated with an ecotropic virus in vitro (Roux et al., 1989).

### Adeno-associated Virus

AAV utilizes a linear, single-stranded DNA of about 4700 base pairs. Inverted terminal repeats flank the genome. Two genes are present within the genome, giving rise to a number of distinct gene products. The first, the cap gene, produces three different virion proteins (VP), designated VP-1, VP-2 and VP-3. The second, the rep gene, encodes four non-structural proteins (NS). One or more of these rep gene products is responsible for transactivating AAV transcription.

The three promoters in AAV are designated by their location, in map units, in the genome. These are, from left to right, p5, p19 and p40. Transcription gives rise to six transcripts, two initiated at each of three promoters, with one of each pair being spliced. The splice site, derived from map units 42-46, is the same for each transcript. The four non-structural proteins apparently are derived from the longer of the transcripts, and three virion proteins all arise from the smallest transcript.

AAV is not associated with any pathologic state in humans. Interestingly, for efficient replication, AAV requires "helping" functions from viruses such as herpes simplex virus I and II, cytomegalovirus, pseudorabies virus and, of course, adenovirus. The best characterized of the helpers is adenovirus, and many "early" functions for this virus have been shown to assist with AAV replication. Low-level expression of AAV rep proteins believed to hold AAV structural expression in check, and helper virus infection is thought to remove this block.

The terminal repeats of the AAV vector can be obtained by restriction endonuclease digestion of AAV or a plasmid such as p201, which contains a modified AAV genome (Samulski et al., J. Virol., 61:3096-3101 (1987)), or by other methods, including but not limited to chemical or enzymatic synthesis of the terminal repeats based upon the published sequence of AAV. It can be determined, for example, by deletion analysis, the minimum sequence or part of the AAV ITRs which is required to allow function, i.e., stable and site-specific integration. It can also be determined which minor modifications of the sequence can be tolerated while maintaining the ability of the terminal repeats to direct stable, site-specific integration.

AAV-based vectors have proven to be safe and effective vehicles for gene delivery in vitro, and these vectors are being developed and tested in pre-clinical and clinical stages for a wide range of applications in potential gene therapy, both ex vivo and in vivo (Carter and Flotte, (1995) Ann. N.Y. Acad. Sci., 770; 79-90; Chatteijee, et al., (1995) Ann. N.Y. Acad. Sci., 770,79-90; Ferrari et al., (1996) J. Virol., 70,3227-3234; Fisher et al., (1996) J. Virol., 70,520-532; Flotte et al., Proc. Nat'l Acad. Sci. USA, 90,10613-10617, (1993); Goodman et al. (1994), Blood, 84,1492-1500; Kaplitt et al., (1994) Nat'l Genet., 8,148-153; Kaplitt, M.G., et al., Ann Thorac Surg. 1996 Dec;62(6):1669-76; Kessler et al., (1996) Proc. Nat'l Acad. Sci. USA, 93,14082-14087; Koeberl et al., (1997) Proc. Nat'l Acad. Sci. USA, 94,1426-1431; Mizukami et al., (1996) Virology, 217,124-130).

AAV-mediated efficient gene transfer and expression in the lung has led to clinical trials for the treatment of cystic fibrosis (Carter and Flotte, 1995; Flotte et al., Proc. Nat'l Acad. Sci. USA, 90, 10613-10617, (1993)). Similarly, the prospects for treatment of muscular dystrophy by AAV-mediated gene delivery of the dystrophin gene to skeletal muscle, of Parkinson's disease by tyrosine hydroxylase gene delivery to the brain, of hemophilia B by Factor IX gene delivery to the liver, and potentially of myocardial infarction by vascular endothelial growth factor gene to the heart, appear promising since AAV-mediated transgene expression in these organs has recently been shown to be highly efficient (Fisher et al., (1996) J. Virol., 70,520-532; Flotte et al., 1993; Kaplitt et al., 1994; 1996; Koeberl et al., 1997; McCown et al., (1996) Brain Res.,713,99-107; Ping et al., (1996) Microcirculation, 3,225-228; Xiao et al., (1996) J. Virol., 70,8098-8108).

### Other Viral Vectors

Other viral vectors are employed as expression constructs in the present methods and compositions. Vectors derived from viruses such as vaccinia virus (Ridgeway, (1988) In: Vectors: A survey of molecular cloning vectors and their uses, pp. 467-492; Baichwal and Sugden, (1986) In, Gene Transfer, pp. 117-148; Coupar et al., Gene, 68:1-10, 1988) canary poxvirus, and herpes viruses are employed. These viruses offer several features for use in gene transfer into various mammalian cells.

Once the construct has been delivered into the cell, the nucleic acid encoding the transgene are positioned and expressed at different sites. The nucleic acid encoding the transgene may be stably integrated into the genome of the cell. This integration is in the cognate location and orientation via homologous recombination (gene replacement) or it is integrated in a random, non-specific location (gene augmentation). Yet further, the nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression construct employed.

### Methods for Treating a Disease

The present methods also encompass methods of treatment or prevention of a disease where administration of cells by, for example, infusion, may be beneficial.

Cells, such as, for example, T cells, tumor infiltrating lymphocytes, natural killer cells, natural killer T cells, or progenitor cells, such as, for example, hematopoietic stem cells, mesenchymal stromal cells, stem cells, pluripotent stem cells, and embryonic stem cells may be used for cell therapy. The cells may be from a donor, or may be cells obtained from the patient. The cells may, for example, be used in regeneration, for example, to replace the function of diseased cells. The cells may also be modified to express a heterologous gene so that biological agents may be delivered to specific microenvironments such as, for example, diseased bone marrow or metastatic deposits. Mesenchymal stromal cells have also, for example, been used to provide immunosuppressive activity, and may be used in the treatment of graft versus host disease and autoimmune disorders. The cells provided in the present disclosure contain a safety switch that may be valuable in a situation where following cell therapy, the activity of the therapeutic cells needs to be increased, or decreased. For example, where T cells that express a chimeric antigen receptor are provided to the patient, in some situations there may be an adverse event, such as off-target toxicity. Ceasing the administration of the ligand would return the therapeutic T cells to a non-activated state, remaining at a low, non-toxic, level of expression. Or, for example, the therapeutic cell may work to decrease the tumor cell, or tumor size, and may no longer be needed. In this situation, administration of the ligand may cease, and the therapeutic cells would no longer be activated. If the tumor cells return, or the tumor size increases following the initial therapy, the ligand may be administered again, in order to activate the chimeric antigen receptor-expressing T cells, and retreat the patient.

By "therapeutic cell" is meant a cell used for cell therapy, that is, a cell administered to a subject to treat or prevent a condition or disease. In such cases, where the cells have a negative effect, the present methods may be used to remove the therapeutic cells through selective apoptosis.

In other examples, T cells are used to treat various diseases and conditions, and as a part of stem cell transplantation. An adverse event that may occur after haploidentical T cell transplantation is graft versus host disease (GvHD). The likelihood of GvHD occurring increases with the increased number of T cells that are transplanted. This limits the number of T cells that may be infused. By having the ability to selectively remove the infused T cells in the event of GvHD in the patient, a greater number of T cells may be infused, increasing the number to greater than 10⁶, greater than 10⁷, greater than 10⁸, or greater than 10⁹ cells. The number of T cells/kg body weight that may be administered may be, for example, from about 1 x 10⁴ T cells/kg body weight to about 9 x 10⁷ T cells/kg body weight, for example about 1, 2, 3, 4, 5, 6, 7, 8, or 9 x 10⁴; about 1, 2, 3, 4, 5, 6, 7, 8, or 9 x 10⁵; about 1, 2, 3, 4, 5, 6, 7, 8, or 9 x 10⁶; or about 1, 2, 3, 4, 5, 6, 7, 8, or 9 x 10⁷ T cells/kg body weight. In other examples, therapeutic cells other than T cells may be used. The number of therapeutic cells/kg body weight that may be administered may be, for example, from about 1 x 10⁴ T cells/kg body weight to about 9 x 10⁷ T cells/kg body weight, for example about 1, 2, 3, 4, 5, 6, 7, 8, or 9 x 10⁴; about 1, 2, 3, 4, 5, 6, 7, 8, or 9 x 10⁵; about 1, 2, 3, 4, 5, 6, 7, 8, or 9 x 10⁶; or about 1, 2, 3, 4, 5, 6, 7, 8, or 9 x 10⁷ therapeutic cells/kg body weight.

The term "unit dose" as it pertains to the inoculum refers to physically discrete units suitable as unitary dosages for mammals, each unit containing a predetermined quantity of pharmaceutical composition calculated to produce the desired immunogenic effect in association with the required diluent. The specifications for the unit dose of an inoculum are dictated by and are dependent upon the unique characteristics of the pharmaceutical composition and the particular immunologic effect to be achieved.

An effective amount of the pharmaceutical composition, such as the multimeric ligand presented herein, would be the amount that achieves this selected result of selectively removing the cells that include the Caspase-9 vector, such that over 60%, 70%, 80%, 85%, 90%, 95%, or 97% of the Caspase-9 expressing cells are killed. The term is also synonymous with "sufficient amount."
The effective amount for any particular application can vary depending on such factors as the disease or condition being treated, the particular composition being administered, the size of the subject, and/or the severity of the disease or condition. One can empirically determine the effective amount of a particular composition presented herein without necessitating undue experimentation.

The terms "contacted" and "exposed," when applied to a cell, tissue or organism, are used herein to discuss the process by which the pharmaceutical composition and/or another agent, such as for example a chemotherapeutic or radiotherapeutic agent, are delivered to a target cell, tissue or organism or are placed in direct juxtaposition with the target cell, tissue or organism. To achieve cell killing or stasis, the pharmaceutical composition and/or additional agent(s) are delivered to one or more cells in a combined amount effective to kill the cell(s) or prevent them from dividing. The administration of the pharmaceutical composition may precede, be co-current with and/or follow the other agent(s) by intervals ranging from minutes to weeks. Where the pharmaceutical composition and other agent(s) are applied separately to a cell, tissue or organism, one would generally ensure that a significant period of time did not expire between the times of each delivery, such that the pharmaceutical composition and agent(s) would still be able to exert an advantageously combined effect on the cell, tissue or organism. For example, in such instances, it is contemplated that one may contact the cell, tissue or organism with two, three, four or more modalities substantially simultaneously (i.e., within less than about a minute) with the pharmaceutical composition. In other aspects, one or more agents may be administered within of from substantially simultaneously, about 1 minute, to about 24 hours to about 7 days to about 1 to about 8 weeks or more, and any range derivable therein, prior to and/or after administering the expression vector. Yet further, various combination regimens of the pharmaceutical composition presented herein and one or more agents may be employed.

### Optimized and Personalized Therapeutic Treatment

The induction of apoptosis after administration of the dimer may be optimized by determining the stage of graft versus host disease, or the number of undesired therapeutic cells that remain in the patient.

For example, determining that a patient has GvHD, and the stage of the GvHD, provides an indication to a clinician that it may be necessary to induce Caspase-9 associated apoptosis by administering the multimeric ligand. In another example, determining that a patient has a reduced level of GvHD after treatment with the multimeric ligand may indicate to the clinician that no additional dose of the multimeric ligand is needed. Similarly, after treatment with the multimeric ligand, determining that the patient continues to exhibit GvHD symptoms, or suffers a relapse of GvHD may indicate to the clinician that it may be necessary to administer at least one additional dose of multimeric ligand. The term "dosage" is meant to include both the amount of the dose and the frequency of administration, such as, for example, the timing of the next dose

Following administration of therapeutic cells, for example, therapeutic cells which express a chimeric antigen receptor in addition to the inducible Caspase-9 polypeptide, in the event of a need to reduce the number of modified cells or in vivo modified cells, the multimeric ligand may be administered to the patient. In these instances, the methods comprise determining the presence or absence of a negative symptom or condition, such as Graft vs Host Disease, or off target toxicity, and administering a dose of the multimeric ligand. The methods may further comprise monitoring the symptom or condition and administering an additional dose of the multimeric ligand in the event the symptom or condition persists. This monitoring and treatment schedule may continue while the therapeutic cells that express chimeric antigen receptors or chimeric signaling molecules remain in the patient.

An indication of adjusting or maintaining a subsequent drug dose, such as, for example, a subsequence dose of the multimeric ligand, and/or the subsequent drug dosage, can be provided in any convenient manner. An indication may be provided in tabular form (e.g., in a physical or electronic medium). For example, the graft versus host disease observed symptoms may be provided in a table, and a clinician may compare the symptoms with a list or table of stages of the disease. The clinician then can identify from the table an indication for subsequent drug dose. An indication can be presented (e.g., displayed) by a computer, after the symptoms or the GvHD stage is provided to the computer (e.g., entered into memory on the computer). For example, this information can be provided to a computer (e.g., entered into computer memory by a user or transmitted to a computer via a remote device in a computer network), and software in the computer can generate an indication for adjusting or maintaining a subsequent drug dose, and/or provide the subsequent drug dose amount.

Once a subsequent dose is determined based on the indication, a clinician may administer the subsequent dose or provide instructions to adjust the dose to another person or entity. The term "clinician" as used herein may refer to a decision maker, and a clinician is a medical professional. A decision maker can be a computer or a displayed computer program output, and a health service provider may act on the indication or subsequent drug dose displayed by the computer. A decision maker may administer the subsequent dose directly (e.g., infuse the subsequent dose into the subject) or remotely (e.g., pump parameters may be changed remotely by a decision maker).

In some examples, a dose, or multiple doses of the ligand may be administered before clinical manifestations of GvHD, or other symptoms, such as CRS symptoms, are apparent. In this example, cell therapy is terminated before the appearance of negative symptoms. For example, in case of a hematopoietic cell transplant for the treatment of a genetic disease, the therapy may be terminated after the transplant has made progress toward engraftment, but before clinically observable GvHD, or other negative symptoms, can occur. In other examples, the ligand may be administered to eliminate the modified cells in order to eliminate on target/off-tumor cells, such as, for example, healthy B cells co-expressing the B cell-associated target antigen.

Methods as presented herein include without limitation the delivery of an effective amount of an activated cell, a nucleic acid or an expression construct encoding the same. An "effective amount" of the pharmaceutical composition, generally, is defined as that amount sufficient to detectably and repeatedly to achieve the stated desired result, for example, to ameliorate, reduce, minimize or limit the extent of the disease or its symptoms. Other more rigorous definitions may apply, including elimination, eradication or cure of disease. In some instances there may be a step of monitoring the biomarkers to evaluate the effectiveness of treatment and to control toxicity.

### Dual Control of Therapeutic Cells and Heterdimerizer Control of Apoptosis for Controlled Therapy

Nucleic acids and cells provided herein may be used to achieve dual control of therapeutic cells for controlled therapy. For example, the subject may be diagnosed with a condition, such as a tumor, where there is a need to deliver targeted chimeric antigen receptor therapy. Methods discussed herein provide several examples of ways to control therapy in order to induce activity of the CAR-expressing therapeutic cells, and also to provide a safety switch should there be a need to discontinue therapy completely, or to reduce the number or percent of the therapeutic cells in the subject.

In certain examples, modified T cells are administered to a subject that express the following polypeptides: 1. A chimeric polypeptide (iMyD88/CD40, or "iMC") that comprises two or more FKBP12 ligand binding regions and a costimulatory polypeptide or polypeptides, such as, for example, MyD88 or truncated MyD88 and CD40; 2. A chimeric proapoptotic polypeptide that comprises one or more FRB ligand binding regions and a Caspase-9 polypeptide; 3. A chimeric antigen receptor polypeptide comprising an antigen recognition moiety that binds to a target antigen. In this example, the target antigen is a tumor antigen present on tumor cells in the subject. Following administration, the ligand AP1903 may be administered to the subject, which induces iMC activation of the CAR-T cell. The therapy is monitored, for example, the tumor size or growth may be assessed during the course of therapy. One or more doses of the ligand may be administered during the course of therapy.

Therapy may be modulated by discontinuing administration of AP1903, which may lower the activation level of the CAR-T cell. To discontinue CAR-T cell therapy, the safety switch-chimeric Caspase-9 polypeptide may be activated by administering a rapalog, which binds to the FRB ligand binding region. The amount and dosing schedule of the rapalog may be determined based on the level of CAR-T cell therapy that is needed. As a safety switch, the dose of the rapalog is an amount effective to remove at least 30%, 40%, 50%, 60%, 70%, 80%, 90%, 95%, 97%, 98%, or 99% of the administered modified cells. In other examples, the dose is an amount effective to remove up to 30%; 40%, 50%, 60%, 70%, 80%, 90, 95%, or 100% of the cells that express the chimeric caspase polypeptide, if there is a need to reduce the level of CAR-T cell therapy, but not completely stop the therapy. This may be measured, for example, by obtaining a sample from the subject before inducing the safety switch, before administering the rapamycin or rapalog, and obtaining a sample following administration of the rapamycin or rapalog, at, for example 0.5, 1, 2, 3, 4, 5, 6, 7, 8, 9, 10 hours, or 1, 2, 3, 4, 5 days following administration, and comparing the number or concentration of chimeric caspase-expressing cells between the two samples by, for example, any method available, including, for example, detecting the presence of a marker. This method of determining percent removal of the cells may also be used where the inducing ligand is AP1903 or binds to the FKBP12 or FKBP12 variant multimerizing region.

In some examples, the inducible MyD88/CD40 chimeric polypeptide also comprises the chimeric antigen receptor. In these examples, where the two polypeptides are present on the same molecule, the chimeric polypeptide may comprise one or more ligand binding regions.

Chemical Induction of protein Dimerization (CID) has been effectively applied to make cellular suicide or apoptosis inducible with the small molecule homodimerizing ligand, rimiducid (AP1903). This technology underlies the "safety switch" incorporated as a gene therapy adjunct in cell transplants (1, 2). The central tenet of the technology is that normal cellular regulatory pathways that rely on protein-protein interaction as part of a signaling pathway can be adapted to ligand-dependent, conditional control if a small molecule dimerizing drug is used to control the protein-protein oligomerization event (3-5). Induced dimerization of a fusion protein comprising Caspase-9 and FKBP12 or an FKBP12 variant (i.e., "iCaspase9/iCasp9/iC9/CaspaCIDe") using a homodimerizing ligand, such as rimiducid, AP1510 or AP20187, can rapidly effect cell death. Caspase-9 is an initiating caspase that acts as a "gate-keeper" of the apoptotic process (6). Normally, pro-apoptotic molecules (e.g., cytochrome c) released from the mitochondria of apoptotic cells alter the conformation of Apaf-1, a caspase-9-binding scaffold, leading to its oligomerization and formation of the "apoptosome". This alteration facilitates caspase-9 dimerization and cleavage of its latent form into an active molecule that, in turn, cleaves the "downstream" apoptosis effector, caspase-3, leading to irreversible cell death. Rimiducid binds directly with two FKBP12-V36 moieties and can direct the dimerization of fusion proteins that include FKBP12-V36 (1, 2). iC9 engagement with rimiducid circumvents the need for Apaf1 conversion to the active apoptosome. In this example, the fusion of caspase-9 to protein moieties that engage a heterodimerizing ligand is assayed for its ability to direct its activation and cell death with similar efficacy to rimiducid-mediated iC9 activation.

MyD88 and CD40 were chosen as the basis of the iMC activation switch. MyD88 plays a central signaling role in the detection of pathogens or cell injury by antigen-presenting cells (APCs), like dendritic cells (DCs). Following exposure to pathogen- or necrotic cells-derived "danger" molecules", a subclass of "pattern recognition receptors", called Toll-Like Receptors (TLRs) are activated, leading to the aggregation and activation of adapter molecule, MyD88, via homologous TLR-IL1Ra (TIR) domains on both proteins. MyD88, in turn, activates downstream signaling, via the rest of the protein. This leads to the upregulation of costimulatory proteins, like CD40, and other proteins, like MHC and proteases, needed for antigen processing and presentation. The fusion of signaling domains from MyD88 and CD40 with two Fv domains, provides iMC (also iFvFvMC), which potently activated DCs following exposure to rimiducid (7). It was later found that iMC is a potent costimulatory protein for T cells, as well.

Rapamycin is a natural product macrolide that binds with high affinity (< 1 nM) to FKBP12 and together initiates the high-affinity, inhibitory interaction with the FKBP-Rapamycin-Binding (FRB) domain of mTOR (8). FRB is small (89 amino acids) and can thereby be used as a protein "tag" or "handle" when appended to many proteins (9-11). Coexpression of a FRB-fused protein with a second FKBP12-fused protein renders their approximation rapamycin-inducible (12-16). This and the examples that follow provide experiments and results designed to test whether expression of FRB-bound Caspase-9 with FKBP-bound Caspase-9 (iC9) can also direct apoptosis and serve as the basis for a cell safety switch regulated by the orally available ligand, rapamycin, or derivatives of rapamycin (rapalogs) that do not inhibit mTOR at a low, therapeutic dose but instead bind with selected, Caspase-9-fused mutant FRB domains.

Some of the following references are referred to in this section,.
1. Straathof KC, Pule MA, Yotnda P, Dotti G, Vanin EF, Brenner MK, Heslop HE, Spencer DM, and Rooney CM. An inducible caspase 9 safety switch for T-cell therapy. Blood. 2005;105(11):4247-54.
2. Fan L, Freeman KW, Khan T, Pham E, and Spencer DM. Improved artificial death switches based on caspases and FADD. Hum Gene Ther. 1999;10(14):2273-85.
3. Spencer DM, Wandless TJ, Schreiber SL, and Crabtree GR. Controlling signal transduction with synthetic ligands. Science. 1993;262(5136):1019-24.
4. Acevedo VD, Gangula RD, Freeman KW, Li R, Zhang Y, Wang F, Ayala GE, Peterson LE, Ittmann M, and Spencer DM. Inducible FGFR-1 activation leads to irreversible prostate adenocarcinoma and an epithelial-to-mesenchymal transition. Cancer Cell. 2007;12(6):559-71.
5. Spencer DM, Belshaw PJ, Chen L, Ho SN, Randazzo F, Crabtree GR, and Schreiber SL. Functional analysis of Fas signaling in vivo using synthetic inducers of dimerization. Curr Biol. 1996;6(7):839-47.
6. Strasser A, Cory S, and Adams JM. Deciphering the rules of programmed cell death to improve therapy of cancer and other diseases. EMBO J. 2011;30(18):3667-83.
7. Narayanan P, Lapteva N, Seethammagari M, Levitt JM, Slawin KM, and Spencer DM. A composite MyD88/CD40 switch synergistically activates mouse and human dendritic cells for enhanced antitumor efficacy. J Clin Invest. 2011;121(4):1524-34.
8. Sabatini DM, Erdjument-Bromage H, Lui M, Tempst P, and Snyder SH. RAFT1: a mammalian protein that binds to FKBP12 in a rapamycin-dependent fashion and is homologous to yeast TORs. Cell. 1994;78(1):35-43.
9. Brown EJ, Albers MW, Shin TB, Ichikawa K, Keith CT, Lane WS, and Schreiber SL. A mammalian protein targeted by G1-arresting rapamycin-receptor complex. Nature. 1994;369(6483):756-8.
10. Chen J, Zheng XF, Brown EJ, and Schreiber SL. Identification of an 11-kDa FKBP12-rapamycin-binding domain within the 289-kDa FKBP12-rapamycin-associated protein and characterization of a critical serine residue. Proc Natl Acad Sci USA. 1995;92(11):4947-51.
11. Choi J, Chen J, Schreiber SL, and Clardy J. Structure of the FKBP12-rapamycin complex interacting with the binding domain of human FRAP. Science. 1996;273(5272):239-42.
12. Ho SN, Biggar SR, Spencer DM, Schreiber SL, and Crabtree GR. Dimeric ligands define a role for transcriptional activation domains in reinitiation. Nature. 1996;382(6594):822-6.
13. Klemm JD, Beals CR, and Crabtree GR. Rapid targeting of nuclear proteins to the cytoplasm. Curr Biol. 1997;7(9):638-44.
14. Bayle JH, Grimley JS, Stankunas K, Gestwicki JE, Wandless TJ, and Crabtree GR. Rapamycin analogs with differential binding specificity permit orthogonal control of protein activity. Chem Biol. 2006;13(1):99-107.
15. Stankunas K, Bayle JH, Gestwicki JE, Lin YM, Wandless TJ, and Crabtree GR. Conditional protein alleles using knockin mice and a chemical inducer of dimerization. Mol Cell. 2003;12(6):1615-24.
16. Stankunas K, Bayle JH, Havranek JJ, Wandless TJ, Baker D, Crabtree GR, and Gestwicki JE. Rescue of Degradation-Prone Mutants of the FK506-Rapamycin Binding (FRB) Protein with Chemical Ligands. Chembiochem. 2007.

### Formulations and Routes for Administration to Patients

Where clinical applications are contemplated, it will be necessary to prepare pharmaceutical compositions-expression constructs, expression vectors, fused proteins, transfected or transduced cells, in a form appropriate for the intended application. Generally, this will entail preparing compositions that are essentially free of pyrogens, as well as other impurities that could be harmful to humans or animals.

The multimeric ligand, such as, for example, AP1903, may be delivered, for example at doses of about 0.1 to 10 mg/kg subject weight, of about 0.1 to 5 mg/kg subject weight, of about 0.2 to 4 mg/kg subject weight, of about 0.3 to 3 mg/kg subject weight, of about 0.3 to 2 mg/kg subject weight, or about 0.3 to 1 mg/kg subject weight, for example, about 0.1, 0.2, 0.3, 0.4, 0.5, 0.6, 0.7, 0.8, 0.9, 1.0, 1.5, 2, 2.5, 3, 3.5, 4, 4.5, 5, 6, 7, 8, 9, or 10 mg/kg subject weight. The ligand is provided at 0.4mg/kg per dose, for example at a concentration of 5mg/mL. Vials or other containers may be provided containing the ligand at, for example, a volume per vial of about 0.25 ml to about 10 ml, for example, about 0.25, 0.5, 1, 1.5, 2, 2.5, 3, 3.5, 4, 4.5, 5, 5.5, 6, 6.5, 7, 7.5, 8, 8.5, 9, 9.5, or 10 ml, for example, about 2 ml.
One may generally desire to employ appropriate salts and buffers to render delivery vectors stable and allow for uptake by target cells. Buffers also may be employed when recombinant cells are introduced into a patient. Aqueous compositions comprise an effective amount of the vector to cells, dissolved or dispersed in a pharmaceutically acceptable carrier or aqueous medium. Such compositions also are referred to as inocula. A pharmaceutically acceptable carrier includes any and all solvents, dispersion media, coatings, antibacterial and antifungal agents, isotonic and absorption delaying agents and the like. The use of such media and agents for pharmaceutically active substances is known. Except insofar as any conventional media or agent is incompatible with the vectors or cells, its use in therapeutic compositions is contemplated. Supplementary active ingredients also can be incorporated into the compositions.

The active compositions may include classic pharmaceutical preparations. Administration of these compositions will be via any common route so long as the target tissue is available via that route. This includes, for example, oral, nasal, buccal, rectal, vaginal or topical. Alternatively, administration may be by orthotopic, intradermal, subcutaneous, intramuscular, intraperitoneal or intravenous injection. Such compositions would normally be administered as pharmaceutically acceptable compositions, discussed herein.

The pharmaceutical forms suitable for injectable use include sterile aqueous solutions or dispersions and sterile powders for the extemporaneous preparation of sterile injectable solutions or dispersions. In all cases the form is sterile and is fluid to the extent that easy syringability exists. It is stable under the conditions of manufacture and storage and is preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In certain examples, isotonic agents, for example, sugars or sodium chloride may be included. Prolonged absorption of the injectable compositions can be brought about by the use in the compositions of agents delaying absorption, for example, aluminum monostearate and gelatin.
For oral administration, the compositions may be incorporated with excipients and used in the form of non-ingestible mouthwashes and dentifrices. A mouthwash may be prepared incorporating the active ingredient in the required amount in an appropriate solvent, such as a sodium borate solution (Dobell's Solution). Alternatively, the active ingredient may be incorporated into an antiseptic wash containing sodium borate, glycerin and potassium bicarbonate. The active ingredient also may be dispersed in dentifrices, including, for example: gels, pastes, powders and slurries. The active ingredient may be added in a therapeutically effective amount to a paste dentifrice that may include, for example, water, binders, abrasives, flavoring agents, foaming agents, and humectants.

The compositions may be formulated in a neutral or salt form. Pharmaceutically-acceptable salts include, for example, the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution may be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media can be employed. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations may meet sterility, pyrogenicity, and general safety and purity standards as required by FDA Office of Biologics standards.

### Examples

The examples set forth below illustrate the technology described herein and do not limit the technology.

Mechanisms for selectively ablating the donor cells have been studied as safety switches for cellular therapies, but there have been complications. Some experience with safety-switch genes to date has been in T lymphocytes since immunotherapy with these cells has proved efficacious as treatment for viral infections and malignancies (Walter, E.A., et al., N. Engl. J. Med. 1995, 333:1038-44; Rooney, C.M., et al., Blood. 1998, 92:1549-55; Dudley, M.E., et al., Science 2002, 298:850-54; Marjit, W.A., et al., Proc. Natl. Acad. Sci. USA 2003, 100:2742-47). The herpes simplex virus I-derived thymidine kinase (HSVTK) gene has been used as an in vivo suicide switch in donor T-cell infusions to treat recurrent malignancy and Epstein Barr virus (EBV) lymphoproliferation after hematopoietic stem cell transplantation (Bonini C, etal., Science. 1997, 276:1719-1724; Tiberghien P, etal., Blood. 2001, 97:63-72). However, destruction of T cells causing graft-versus-host disease was incomplete, and the use of gancyclovir (or analogs) as a pro-drug to activate HSV-TK precludes administration of gancyclovir as an antiviral drug for cytomegalovirus infections. This mechanism of action also requires interference with DNA synthesis, relying on cell division, so that cell killing may be protracted over several days and incomplete, producing a lengthy delay in clinical benefit (Ciceri, F., et al., Lancet Oncol. 2009, 262:1019-24). Moreover, HSV-TK-directed immune responses have resulted in elimination of HSV-TK-transduced cells, even in immunosuppressed human immunodeficiency virus and bone marrow transplant patients, compromising the persistence and hence efficacy of the infused T cells. HSV-TK is also virus-derived, and therefore potentially immunogenic (Bonini C, et al., Science. 1997, 276:1719-1724; Riddell SR, et al., Nat Med. 1996, 2:216- 23). The E coli-derived cytosine deaminase gene has also been used clinically (Freytag SO, et al., Cancer Res. 2002, 62:4968-4976), but as a xenoantigen it may be immunogenic and thus incompatible with T-cell-based therapies that require long-term persistence. Transgenic human CD20, which can be activated by a monoclonal chimeric anti-CD20 antibody, has been proposed as a nonimmunogenic safety system (Introna M, et al., Hum Gene Ther. 2000, 11: 611-620).

The following section provides examples of method of providing a safety switch in cells used for cellular therapy, using a Caspase-9 chimeric protein.

### Example 1: Construction and Evaluation of Caspase-9 Suicide Switch Expression Vectors

### Vector construction and confirmation of expression

A safety switch that can be stably and efficiently expressed in human T cells is presented herein. The system includes human gene products with low potential immunogenicity that have been modified to interact with a small molecule dimerizer drug that is capable of causing the selective elimination of transduced T cells expressing the modified gene. Additionally the inducible Caspase-9 maintains function in T cells overexpressing antiapoptotic molecules.

Expression vectors suitable for use as a therapeutic agent were constructed that included a modified human Caspase-9 activity fused to a human FK506 binding protein (FKBP), such as, for example, FKBP12v36. The Caspase-9/FK506 hybrid activity can be dimerized using a small molecule pharmaceutical. Full length, truncated, and modified versions of the Caspase-9 activity were fused to the ligand binding domain, or multimerizing region, and inserted into the retroviral vector MSCV.IRES.GRP, which also allows expression of the fluorescent marker, GFP. FIG. 1A illustrates the full length, truncated and modified Caspase-9 expression vectors constructed and evaluated as a suicide switch for induction of apoptosis.

The full-length inducible Caspase-9 molecule (F'-F-C-Casp9) includes 2, 3, or more FK506 binding proteins (FKBPs-for example, FKBP12v36 variants) linked with a Gly-Ser-Gly-Gly-Gly-Ser linker to the small and large subunit of the Caspase molecule (see FIG. 1A). Full-length inducible Caspase-9 (F'F-C-Casp9.1.GFP) has a full-length Caspase-9, also includes a Caspase recruitment domain (CARD; GenBank NM001 229) linked to 2 12-kDa human FK506 binding proteins (FKBP12; GenBank AH002 818) that contain an F36V mutation (FIG. 1A). The amino acid sequence of one or more of the FKBPs (F') was codon-wobbled (e.g., the 3^{rd} nucleotide of each amino acid codon was altered by a silent mutation that maintained the originally encoded amino acid) to prevent homologous recombination when expressed in a retrovirus. F'F-C-Casp9C3S includes a cysteine to serine mutation at position 287 that disrupts its activation site. In constructs F'F-Casp9, F-C-Casp9, and F'-Casp9, either the Caspase activation domain (CARD), one FKBP, or both, were deleted, respectively. All constructs were cloned into MSCV.IRES.GFP as EcoRI-Xhol fragments.

293T cells were transfected with each of these constructs and 48 hours after transduction expression of the marker gene GFP was analyzed by flow cytometry. In addition, 24 hours after transfection, 293T cells were incubated overnight with 100 nM CID and subsequently stained with the apoptosis marker annexin V. The mean and standard deviation of transgene expression level (mean GFP) and number of apoptotic cells before and after exposure to the chemical inducer of dimerization (CID) (% annexin V within GFP∼ cells) from 4 separate experiments are shown in the second through fifth columns of the table in FIG. 1A. In addition to the level of GFP expression and staining for annexin V, the expressed gene products of the full length, truncated and modified Caspase-9 were also analyzed by western blot to confirm the Caspase-9 genes were being expressed and the expressed product was the expected size. The results of the western blot are presented in FIG. 1B.

Coexpression of the inducible Caspase-9 constructs of the expected size with the marker gene GFP in transfected 293T cells was demonstrated by Western blot using a Caspase-9 antibody specific for amino acid residues 299-318, present both in the full-length and truncated Caspase molecules as well as a GFP-specific antibody. Western blots were performed as presented herein.

Transfected 293T cells were resuspended in lysis buffer (50% Tris/Gly, 10% sodium dodecyl sulfate [SDS], 4% beta-mercaptoethanol, 10% glycerol, 12% water, 4% bromophenol blue at 0.5%) containing aprotinin, leupeptin, and phenylmethylsulfonyl fluoride (Boehringer, Ingelheim, Germany) and incubated for 30 minutes on ice. After a 30-minute centrifugation, supernatant was harvested; mixed 1:2 with Laemmli buffer (Bio-Rad, Hercules, CA), boiled and loaded on a 10% SDS-polyacrylamide gel. The membrane was probed with rabbit anti-Caspase-9 (amino acid residues 299-3 18) immunoglobulin G (IgG; Affinity BioReagents, Golden, CO; 1:500 dilution) and with mouse anti-GFP IgG (Covance, Berkeley, CA; 1:25,000 dilution). Blots were then exposed to appropriate peroxidase-coupled secondary antibodies and protein expression was detected with enhanced chemiluminescence (ECL; Amersham, Arlington Heights, IL). The membrane was then stripped and reprobed with goat polyclonal antiactin (Santa Cruz Biotechnology; 1:500 dilution) to check equality of loading.

Additional smaller size bands, seem in FIG. 1B, likely represent degradation products. Degradation products for the F'F-C-Casp9 and F'F-Casp9 constructs may not be detected due to a lower expression level of these constructs as a result of their basal activity. Equal loading of each sample was confirmed by the substantially equal amounts of actin shown at the bottom of each lane of the western blot, indicating substantially similar amounts of protein were loaded in each lane.

An example of a chimeric polypeptide that may be expressed in the modified cells is provided herein. In this example, a single polypeptide is encoded by the nucleic acid vector. The inducible Caspase-9 polypeptide is separated from the CAR polypeptide during translation, due to skipping of a peptide bond. (Donnelly, ML 2001, J. Gen. Virol. 82:1013-25).

### Evaluation of Caspase-9 suicide switch expression constructs.

### Cell lines

B 95-8 EBV transformed B-cell lines (LCLs), Jurkat, and MT-2 cells (kindly provided by Dr S. Marriott, Baylor College of Medicine, Houston, TX) were cultured in RPMI 1640 (Hyclone, Logan, UT) containing 10% fetal bovine serum (FBS; Hyclone). Polyclonal EBV-specific T-cell lines were cultured in 45% RPMI/45% Clicks (Irvine Scientific, Santa Ana, CA)/10% FBS and generated as previously reported. Briefly, peripheral blood mononuclear cells (2 x 10⁶ per well of a 24-well plate) were stimulated with autologous LCLs irradiated at 4000 rads at a responder-to-stimulator (R/S) ratio of 40:1. After 9 to 12 days, viable cells were restimulated with irradiated LCLs at an R/S ratio of 4:1. Subsequently, cytotoxic T cells (CTLs) were expanded by weekly restimulation with LCLs in the presence of 40 U/mL to 100 U/mL recombinant human interleukin-2 (rhIL-2; Proleukin; Chiron, Emeryville, CA).

### Retrovirus transduction

For the transient production of retrovirus, 293T cells were transfected with iCasp9/iFas constructs, along with plasmids encoding gag-pol and RD 114 envelope using GeneJuice transfection reagent (Novagen, Madison, WI). Virus was harvested 48 to 72 hours after transfection, snap frozen, and stored at ∼80°C until use. A stable FLYRD 18-derived retroviral producer line was generated by multiple transductions with VSV-G pseudotyped transient retroviral supernatant. FLYRD18 cells with highest transgene expression were single-cell sorted, and the clone that produced the highest virus titer was expanded and used to produce virus for lymphocyte transduction. The transgene expression, function, and retroviral titer of this clone was maintained during continuous culture for more than 8 weeks. For transduction of human lymphocytes, a non-tissue-culture-treated 24-well plate (Becton Dickinson, San Jose, CA) was coated with recombinant fibronectin fragment (FN CH-296; Retronectin; Takara Shuzo, Otsu, Japan; 4 µg/mL in PBS, overnight at 4°C) and incubated twice with 0.5 mL retrovirus per well for 30 minutes at 37°C. Subsequently, 3 x105 to 5 x 10⁵ T cells per well were transduced for 48 to 72 hours using 1 mL virus per well in the presence of 100 U/mL IL-2. Transduction efficiency was determined by analysis of expression of the coexpressed marker gene green fluorescent protein (GFP) on a FACScan flow cytometer (Becton Dickinson). For functional studies, transduced CTLs were either non-selected or segregated into populations with low, intermediate, or high GFP expression using a MoFlo cytometer (Dako Cytomation, Ft Collins, CO) as indicated.

### Induction and analysis of apoptosis

CID (AP20187; ARIAD Pharmaceuticals) at indicated concentrations was added to transfected 293T cells or transduced CTLs. Adherent and nonadherent cells were harvested and washed with annexin binding buffer (BD Pharmingen, San Jose, CA). Cells were stained with annexin-V and 7-amino-actinomycin D (7-AAD) for 15 minutes according to the manufacturer's instructions (BD Pharmingen). Within 1 hour after staining, cells were analyzed by flow cytometry using CellQuest software (Becton Dickinson).

### Cytotoxicity assay

The cytotoxic activity of each CTL line was evaluated in a standard 4-hour ⁵¹Cr release assay, as previously presented. Target cells included autologous LCLs, human leukocyte antigen (HLA) class I-mismatched LCLs and the lymphokine-activated killer cell-sensitive T-cell lymphoma line HSB-2. Target cells incubated in complete medium or 1% Triton X-100 (Sigma, St Louis, MO) were used to determine spontaneous and maximum ⁵¹Cr release, respectively. The mean percentage of specific lysis of triplicate wells was calculated as 100 X (experimental release - spontaneous release) / (maximal release - spontaneous release).

### Phenotyping

Cell-surface phenotype was investigated using the following monoclonal antibodies: CD3, CD4, CD8 (Becton Dickinson) and CD56 and TCR-α/β (Immunotech, Miami, FL). ΔNGFR-iFas was detected using anti-NGFR antibody (Chromaprobe, Aptos, CA). Appropriate matched isotype controls (Becton Dickinson) were used in each experiment. Cells were analyzed with a FACSscan flow cytometer (Becton Dickinson).

### Analysis of cytokine production

The concentration of interferon-γ (IFN- γ), IL-2, IL-4, IL-5, IL-10, and tumor necrosis factor- α (TNFα) in CTL culture supernatants was measured using the Human Th1/Th2 cytokine cytometric Bead Array (BD Pharmingen) and the concentration of IL-12 in the culture supernatants was measured by enzyme-linked immunosorbent assay (ELISA; R&D Systems, Minneapolis, MN) according to the instructions of the manufacturer.

### In vivo experiments

Non-obese diabetic severe combined immunodeficient (NOD/SCID) mice, 6 to 8 weeks of age, were irradiated (250 rad) and injected subcutaneously in the right flank with 10 x 10⁶ to 15 x 10⁶ LCLs resuspended in Matrigel (BD Bioscience). Two weeks later mice bearing tumors that were approximately 0.5 cm in diameter were injected into the tail vein with a 1:1 mixture of nontransduced and iCasp9.I.GFPhigh-transduced EBV CTLs (total 15 x 10⁶). At 4 to 6 hours prior and 3 days after CTL infusion, mice were injected intraperitoneally with recombinant hIL-2 (2000 U; Proleukin; Chiron). On day 4, the mice were randomly segregated in 2 groups: 1 group received CID (50 µg AP20187, intraperitoneally) and 1 group received carrier only (16.7% propanediol, 22.5% PEG400, and 1.25% Tween 80, intraperitoneally). On day 7, all mice were killed. Tumors were homogenized and stained with antihuman CD3 (BD Pharmingen). By FACS analysis, the number of GFP+ cells within the gated CD3⁺ population was evaluated. Tumors from a control group of mice that received only nontransduced CTLs (total 15 x 10⁶) were used as a negative control in the analysis of CD3⁺/GFP⁺ cells.

### Optimization of expression and function of inducible Caspase-9

Caspases 3, 7, and 9 were screened for their suitability as inducible safety-switch molecules both in transfected 293T cells and in transduced human T cells. Only inducible Caspase-9 (iCasp9) was expressed at levels sufficient to confer sensitivity to the chosen CID (e.g., chemical inducer of dimerization). An initial screen indicated that the full length iCasp9 could not be maintained stably at high levels in T cells, possibly due to transduced cells being eliminated by the basal activity of the transgene. The CARD domain is involved in physiologic dimerization of Caspase-9 molecules, by a cytochrome C and adenosine triphosphate (ATP)-driven interaction with apoptotic protease-activating factor 1 (Apaf-1). Because of the use of a CID to induce dimerization and activation of the suicide switch, the function of the CARD domain is superfluous in this context and removal of the CARD domain was investigated as a method of reducing basal activity. Given that only dimerization rather than multimerization is required for activation of Caspase-9, a single FKBP12v36 domain also was investigated as a method to effect activation.

The activity of the resultant truncated and/or modified forms of Caspase-9 (e.g., the CARD domain, or one of the 2 FKBP domains, or both, are removed) were compared. A construct with a disrupted activation site, F'F-C-Casp9_{C->S}, provided a nonfunctional control (see FIG. 1A). All constructs were cloned into the retroviral vector MSCV²⁶ in which retroviral long terminal repeats (LTRs) direct transgene expression and enhanced GFP is coexpressed from the same mRNA by use of an internal ribosomal entry site (IRES). In transfected 293T cells, expression of all inducible Caspase-9 constructs at the expected size as well as coexpression of GFP was demonstrated by Western blot (see FIG. 1B). Protein expression (estimated by mean fluorescence of GFP and visualized on Western blot) was highest in the nonfunctional construct F'F-C-Casp9_{C->S} and greatly diminished in the full-length construct F'F-C-Casp9. Removal of the CARD (F'F-Casp9), one FKBP (F-C-Casp9), or both (F-Casp9) resulted in progressively higher expression of both inducible Caspase-9 and GFP, and correspondingly enhanced sensitivity to CID (see FIG. 1A). Based on these results, the F-Casp9 construct (henceforth referred to as iCasp9_{M}) was used for further study in human T lymphocytes.

### Stable expression of iCasp9_{M} in human T lymphocytes

The long-term stability of suicide gene expression is of utmost importance, since suicide genes must be expressed for as long as the genetically engineered cells persist. For T-cell transduction, a FLYRD18-derived retroviral producer clone that produces high-titer RD114-pseudotyped virus was generated to facilitate the transduction of T cells. iCasp9_{M} expression in EBV-specific CTL lines (EBV-CTL) was evaluated since EBV-specific CTL lines have well-characterized function and specificity and are already being used as in vivo therapy for prevention and treatment of EBV-associated malignancies. Consistent transduction efficiencies of EBV-CTLs of more than 70% (mean, 75.3%; range, 71.4%-83.0% in 5 different donors) were obtained after a single transduction with retrovirus. The expression of iCasp9_{M} in EBV-CTLs was stable for at least 4 weeks after transduction without selection or loss of transgene function.

### iCasp9_{M} does not alter transduced T-cell characteristics

To ensure that expression of iCasp9_{M} did not alter T-cell characteristics, the phenotype, antigen-specificity, proliferative potential, and function of nontransduced or nonfunctional iCasp9_{C->S}-transduced EBV-CTLs was compared with that of iCasp9_{M}-transduced EBV-CTLs. In 4 separate donors, transduced and nontransduced CTLs consisted of equal numbers of CD4⁺, CD8⁺, CD56⁺, and TCR α/β + cells. Similarly, production of cytokines including IFN-γ, TNFα, IL-10, IL-4, IL-5, and IL-2 was unaltered by iCasp9_{M} expression. iCasp9_{M}-transduced EBV-CTLs specifically lysed autologous LCLs comparable to nontransduced and control-transduced CTLs. Expression of iCasp9M did not affect the growth characteristics of exponentially growing CTLs, and importantly, dependence on antigen and IL-2 for proliferation was preserved On day 21 after transduction the normal weekly antigenic stimulation with autologous LCLs and IL-2 was continued or discontinued . Discontinuation of antigen stimulation resulted in a steady decline of T cells.

### Elimination of more than 99% of T lymphocytes selected for high transgene expression in vitro

Inducible iCasp9_{M} proficiency in CTLs was tested by monitoring loss of GFP-expressing cells after administration of CID; 91.3% (range, 89.5%-92.6% in 5 different donors) of GFP⁺ cells were eliminated after a single 10-nM dose of CID. Similar results were obtained regardless of exposure time to CID (range, 1 hour-continuous). In all experiments, CTLs that survived CID treatment had low transgene expression with a 70% (range, 55%-82%) reduction in mean fluorescence intensity of GFP after CID. No further elimination of the surviving GFP⁺T cells could be obtained by an antigenic stimulation followed by a second 10-nM dose of CID. Therefore, the non-responding CTLs most likely expressed insufficient iCasp9_{M} for functional activation by CID. To investigate the correlation between low levels of expression and CTL non-response to CID, CTLs were sorted for low, intermediate, and high expression of the linked marker gene GFP and mixed 1:1 with nontransduced CTLs from the same donor to allow for an accurate quantitation of the number of transduced T cells responding to CID-induced apoptosis.

The number of transduced T cells eliminated increased with the level of GFP transgene expression (see FIGS. 4A, 4B and 4C). To determine the correlation between transgene expression and function of iCasp9_{M}, iCasp9_{M} IRES.GFP-transduced EBV-CTL were selected for low (mean 21), intermediate (mean 80) and high (mean 189) GFP expression. Selected T-cells were incubated overnight with 10 nM CID and subsequently stained with annexin V and 7-AAD. Indicated are the percentages of annexin V+/7-AAD- and annexin V+/7-AAD+ T-. Selected T-cells were mixed 1:1 with non-transduced T-cells and incubated with 10 nM CID following antigenic stimulation. Indicated is the percentage of residual GFP-positive T-cells on day 7.

For GFP_{high}-selected cells, 10 nM CID led to deletion of 99.1% (range, 98.7%-99.4%) of transduced cells. On the day of antigen stimulation, F-Casp9_{M}.I.GFP-transduced CTLs were either untreated or treated with 10 nM CID. Seven days later, the response to CID was measured by flow cytometry for GFP. The percentage of transduced T cells was adjusted to 50% to allow for an accurate measurement of residual GFP⁺ cells after CID treatment. The responses to CID in unselected (top row of and GFP_{high}-selected CTLs (bottom row of was compared. The percentage of residual GFP+ cells is indicated.

Rapid induction of apoptosis in the GFP_{high}-selected cells is demonstrated by apoptotic characteristics such as cell shrinkage and fragmentation within 14 hours of CID administration (see .After overnight incubation with 10 nM CID, F-Casp9_{M}.I.GFP_{high}-transduced T cells had apoptotic characteristics such as cell shrinkage and fragmentation by microscopic evaluation. Of the T cells selected for high expression, 64% (range, 59%-69%) had an apoptotic (annexin-V⁺+/7-AAD⁻) and 30% (range, 26%-32%) had a necrotic (annexinV⁺/7-AAD⁺) phenotype. Staining with markers of apoptosis showed that 64% of T cells had an apoptotic phenotype (annexin V⁺, 7-AAD⁻, lower right quadrant) and 32% a necrotic phenotype (annexin V⁺, 7-AAD⁺, upper right quadrant). A representative example of 3 separate experiments is shown.
In contrast, the induction of apoptosis was significantly lower in T cells selected for intermediate or low GFP expression (see FIGS. 4A, 4B and 4C). For clinical applications therefore, versions of the expression constructs with selectable markers that allow selection for high copy number, high levels of expression, or both high copy number and high levels of expression may be desirable. CID-induced apoptosis was inhibited by the panCaspase inhibitor zVAD-fmk (100 µM for 1 hour prior to adding CID. Titration of CID showed that 1 nM CID was sufficient to obtain the maximal deletion effect. A dose-response curve using the indicated amounts of CID (AP20187) shows the sensitivity of F-Casp9_{M}.I.GFP_{high} to CID. Survival of GFP+ cells is measured on day 7 after administration of the indicated amount of CID. The mean and standard deviation for each point are given. Similar results were obtained using another chemical inducer of dimerization (CID), AP1903, which was clinically shown to have substantially no adverse effects when administered to healthy volunteers. The dose response remained unchanged for at least 4 weeks after transduction.

### iCasp9_{M} is functional in malignant cells that express antiapoptotic molecules

Caspase-9 was selected as an inducible proapoptotic molecule for clinical use rather than previously presented iFas and iFADD, because Caspase-9 acts relatively late in apoptosis signaling and therefore is expected to be less susceptible to inhibition by apoptosis inhibitors. Thus, suicide function should be preserved not only in malignant, transformed T-cell lines that express antiapoptotic molecules, but also in subpopulations of normal T cells that express elevated antiapoptotic molecules as part of the process to ensure long-term preservation of memory cells. To further investigate the hypothesis, the function of iCasp9_{M} and iFas was first compared in EBV-CTLs. To eliminate any potential vector based difference, inducible Fas also was expressed in the MSCV.IRES.GFP vector, like iCasp9. For these experiments both ΔNGFR.iFas.I.GFP and iCasp9_{M}.I.GFP-transduced CTLs were sorted for GFP_{high} expression and mixed with nontransduced CTLs at a 1:1 ratio to obtain cell populations that expressed either iFas or iCasp9_{M} at equal proportions and at similar levels The EBV-CTLs were sorted for high GFP expression and mixed 1:1 with nontransduced CTLs as presented. The percentages of ΔNGFR⁺/GFP⁺ and GFP⁺T cells are indicated.

Elimination of GFP⁺ cells after administration of 10 nM CID was more rapid and more efficient in iCasp9_{M} than in iFas-transduced CTLs (99.2% +/- 0.14% of iCasp9_{M}-transduced cells compared with 89.3% +/- 4.9% of iFas-transduced cells at day 7 after CID; P < .05). On the day of LCL stimulation, 10 nM CID was administered, and GFP was measured at the time points indicated to determine the response to CID. Black diamonds represent data for ΔNGFR-iFas.I.GFP; black squares represent data for iCasp9_{M}.I.GFP. Mean and standard deviation of 3 experiments are shown. The function of iCasp9M and iFas was also compared in 2 malignant T-cell lines: Jurkat, an apoptosis-sensitive T-cell leukemia line, and MT-2, an apoptosis-resistant T-cell line, due to c-FLIP and bcl-xL expression. Jurkat cells and MT-2 cells were transduced with iFas and iCasp9_{M} with similar efficiencies (92% vs 84% in Jurkat, 76% vs 70% in MT-2) and were cultured in the presence of 10 nM CID for 8 hours. Annexin-V staining showed that although iFas and iCasp9_{M} induced apoptosis in an equivalent number of Jurkat cells (56.4% +/- 15.6% and 57.2% +/-18.9%, respectively), only activation of iCasp9_{M} resulted in apoptosis of MT-2 cells (19.3% +/- 8.4% and 57.9% +/- 11.9% for iFas and iCasp9_{M}, respectively; see Figure 5C).

The human T-cell lines Jurkat (left) and MT-2 (right) were transduced with ΔNGFR-iFas.I.GFP or iCasp9_{M}.I.GFP. An equal percentage of T cells were transduced with each of the suicide genes: 92% for ΔNGFR-iFas.I.GFP versus 84% for iCasp9_{M}.I.GFP in Jurkat, and 76% for ΔNGFR-iFas.I.GFP versus 70% for iCasp9_{M}.I.GFP in MT-2. T cells were either nontreated or incubated with 10 nM CID. Eight hours after exposure to CID, apoptosis was measured by staining for annexin V and 7-AAD. A representative example of 3 experiments is shown. PE indicates phycoerythrin. These results demonstrate that in T cells overexpressing apoptosis-inhibiting molecules, the function of iFas can be blocked, while iCasp9_{M} can still effectively induce apoptosis.

### iCasp9M-mediated elimination of T cells expressing an immunomodulatory transgene

To determine whether iCasp9M could effectively destroy cells genetically modified to express an active transgene product, the ability of iCasp9_{M} to eliminate EBV-CTLs stably expressing IL-12 was measured. While IL- 12 was undetectable in the supernatant of nontransduced and iCasp9_{M}.IRES.GFP-transduced CTLs, the supernatant of iCasp9_{M}.IRES.IL-12-transduced cells contained 324 pg/mL to 762 pg/mL IL-12. After administration of 10 nM CID, however, the IL-12 in the supernatant fell to undetectable levels (< 7.8 pg/mL). Thus, even without prior sorting for high transgene expressing cells, activation of iCasp9_{M} is sufficient to completely eliminate all T cells producing biologically relevant levels of IL-12.The marker gene GFP in the iCasp9_{M}.I.GFP constructs was replaced by flexi IL-12, encoding the p40 and p35 subunits of human IL-12. iCasp9_{M}.I.GFP- and iCasp9_{M}.I.IL-12-transduced EBV-CTLs were stimulated with LCLs, and then left untreated or exposed to 10 nM CID. Three days after a second antigenic stimulation, the levels of IL-12 in the culture supernatant were measured by IL-12 ELISA (detection limit of this assay is 7.8 pg/mL). The mean and standard deviation of triplicate wells are indicated. Results of 1 of 2 experiments with CTLs from 2 different donors are shown.

### Elimination of more than 99% of T cells selected for high transgene expression in vivo

The function of iCasp9_{M} also was evaluated in transduced EBV-CTLs in vivo. A SCID mouse-human xenograft model was used for adoptive immunotherapy. After intravenous infusion of a 1:1 mixture of nontransduced and iCasp9_{M}.IRES.GFP_{high}-transduced CTLs into SCID mice bearing an autologous LCL xenograft, mice were treated either with a single dose of CID or carrier only. Three days after CID/carrier administration, tumors were analyzed for human CD3⁺/GFP⁺ cells. Detection of the nontransduced component of the infusion product, using human anti-CD3 antibodies, confirmed the success of the tail-vein infusion in mice that received CID.
In mice treated with CID, there was more than a 99% reduction in the number of human CD3+/GFP+ T cells, compared with infused mice treated with carrier alone, demonstrating equally high sensitivity of iCasp9_{M}-transduced T cells in vivo and in vitro.

The function of iCasp9_{M} in vivo, was assayed. NOD/SCID mice were irradiated and injected subcutaneously with 10 x 10⁶ to 15 x 10⁶ LCLs. After 14 days, mice bearing tumors of 0.5cm in diameter received a total of 15 x10⁶ EBV-CTLs (50% of these cells were nontransduced and 50% were transduced with iCasp9_{M}.I.GFP and sorted for high GFP expression). On day 3 after CTL administration, mice received either CID (50 µg AP20187; (black diamonds, n=6) or carrier only (black squares, n=5) and on day 6 the presence of human CD3+/GFP+ T cells in the tumors was analyzed. Human CD3+ T cells isolated from the tumors of a control group of mice that received only nontransduced CTLs (15 x10⁶ CTLs; n= 4) were used as a negative control for the analysis of CD3+/GFP+ T cells within the tumors.

### Discussion

Presented herein are *inter alia* expression vectors expressing suicide genes suitable for eliminating gene-modified T cells in vivo. Suicide gene expression vectors presented herein have certain non-limiting advantageous features including stable coexpression in all cells carrying the modifying gene, expression at levels high enough to elicit cell death, low basal activity, high specific activity, and minimal susceptibility to endogenous antiapoptotic molecules. Presented herein, is *inter alia* an inducible Caspase-9, iCasp9_{M}, which has low basal activity allowing stable expression for more than 4 weeks in human T cells. A single 10-nM dose of a small molecule chemical inducer of dimerization (CID) is sufficient to kill more than 99% of iCasp9_{M}-transduced cells selected for high transgene expression both in vitro and in vivo. Moreover, when coexpressed with Th1 cytokine IL-12, activation of iCasp9_{M} eliminated all detectable IL-12-producing cells, even without selection for high transgene expression. Caspase-9 acts downstream of most antiapoptotic molecules, therefore, a high sensitivity to CID is preserved regardless of the presence of increased levels of antiapoptotic molecules of the bcl-2 family. Thus, iCasp9_{M} also may prove useful for inducing destruction even of transformed T cells and memory T cells that are relatively resistant to apoptosis.

Unlike other Caspase molecules, proteolysis does not appear sufficient for activation of Caspase-9. Crystallographic and functional data indicate that dimerization of inactive Caspase-9 monomers leads to conformational change-induced activation. The concentration of pro-Caspase-9, in a physiologic setting, is in the range of about 20 nM, well below the threshold needed for dimerization.

Without being limited by theory, it is believed the energetic barrier to dimerization can be overcome by homophilic interactions between the CARD domains of Apaf-1 and Caspase-9, driven by cytochrome C and ATP. Overexpression of Caspase-9 joined to 2 FKBPs may allow spontaneous dimerization to occur and can account for the observed toxicity of the initial full length Caspase-9 construct. A decrease in toxicity and an increase in gene expression was observed following removal of one FKBP, most likely due to a reduction in toxicity associated with spontaneous dimerization. While multimerization often is involved in activation of surface death receptors, dimerization of Caspase-9 should be sufficient to mediate activation. Data presented herein indicates that iCasp9 constructs with a single FKBP function as effectively as those with 2 FKBPs. Increased sensitivity to CID by removal of the CARD domain may represent a reduction in the energetic threshold of dimerization upon CID binding.

The persistence and function of virus- or bacteria-derived lethal genes, such as HSV-TK and cytosine deaminase, can be impaired by unwanted immune responses against cells expressing the virus or bacteria derived lethal genes. The FKBPs and proapoptotic molecules that form the components of iCasp9_{M} are human-derived molecules and are therefore less likely to induce an immune response. Although the linker between FKBP and Caspase-9 and the single point mutation in the FKBP domain introduce novel amino acid sequences, the sequences were not immunologically recognized by macaque recipients of iFas-transduced T cells. Additionally, because the components of iCasp9_{M} are human-derived molecules, no memory T cells specific for the junction sequences should be present in a recipient, unlike virus-derived proteins such as HSV-TK, thereby reducing the risk of immune response-mediated elimination of iCasp9_{M}-transduced T cells.
Previous studies using inducible Fas or the death effector domains (DED) of Fas associated death domain proteins (FADD) showed that approximately 10% of transduced cells were unresponsive to activation of the destructive gene. As observed in experiments presented here, a possible explanation for unresponsiveness to CID is low expression of the transgene. The iCasp9_{M}-transduced T cells in our study and iFas-transduced T cells in studies by others that survived after CID administration had low levels of transgene expression. In an attempt to overcome a perceived retroviral "positional effect", increased levels of homogeneous expression of the transgene were achieved by flanking retroviral integrants with the chicken beta-globin chromatin insulator. Addition of the chromatin insulator dramatically increased the homogeneity of expression in transduced 293T cells, but had no significant effect in transduced primary T cell. Selection of T cells with high expression levels minimized variability of response to the dimerizer. Over 99% of transduced T cells sorted for high GFP expression were eliminated after a single 10-nM CID dose. This demonstration supports the hypothesis that cells expressing high levels of suicide gene can be isolated using a selectable marker.

A very small number of resistant residual cells may cause a resurgence of toxicity, a deletion efficiency of up to 2 logs will significantly decrease this possibility. For clinical use, coexpression with a nonimmunogenic selectable marker such as truncated human NGFR, CD20, or CD34 (e.g., instead of GFP) will allow for selection of high transgene-expressing T cells. Coexpression of the suicide switch (e.g., iCASP9_{M}) and a suitable selectable marker (e.g., truncated human NGFR, CD20, CD34, the like and combinations thereof) can be obtained using either an internal ribosome entry site (IRES) or posttranslational modification of a fusion protein containing a self-cleaving sequence (eg, 2A). In contrast, in situations where the sole safety concern is the transgene-mediated toxicity (eg, artificial T-cell receptors, cytokines, the like or combinations thereof), this selection step may be unnecessary, as tight linkage between iCasp9_{M} and transgene expression enables elimination of substantially all cells expressing biologically relevant levels of the therapeutic transgene. This was demonstrated by coexpressing iCasp9_{M} with IL-12. Activation of iCasp9_{M} substantially eliminated any measurable IL- 12 production. The success of transgene expression and subsequent activation of the "suicide switch" may depend on the function and the activity of the transgene.

Another possible explanation for unresponsiveness to CID is that high levels of apoptosis inhibitors may attenuate CID-mediated apoptosis. Examples of apoptosis inhibitors include c-FLIP, bcl-2 family members and inhibitors of apoptosis proteins (IAPs), which normally regulate the balance between apoptosis and survival. For instance, upregulation of c-FLIP and bcl-2 render a subpopulation of T cells, destined to establish the memory pool, resistant to activation-induced cell death in response to cognate target or antigen-presenting cells. In several T-lymphoid tumors, the physiologic balance between apoptosis and survival is disrupted in favor of cell survival. A suicide gene should delete substantially all transduced T cells including memory and malignantly transformed cells. Therefore, the chosen inducible suicide gene should retain a significant portion if not substantially all of its activity in the presence of increased levels of antiapoptotic molecules.

The apical location of iFas (or iFADD) in the apoptosis signaling pathway may leave it especially vulnerable to inhibitors of apoptosis, thus making these molecules less well suited to being the key component of an apoptotic safety switch. Caspase 3 or 7 would seem well suited as terminal effector molecules; however neither could be expressed at functional levels in primary human T cells. Therefore Caspase-9, was chosen as the suicide gene, because Capsase-9 functions late enough in the apoptosis pathway that it bypasses the inhibitory effects of c-FLIP and antiapoptotic bcl-2 family members, and Caspase-9 also could be expressed stably at functional levels. Although X-linked inhibitor of apoptosis (XIAP) could in theory reduce spontaneous Caspase-9 activation, the high affinity of AP20187 (or AP1903) for FKBP_{V36} may displace this noncovalently associated XIAP. In contrast to iFas, iCasp9_{M} remained functional in a transformed T-cell line that overexpresses antiapoptotic molecules, including bcl-xL.

Presented herein is an inducible safety switch, designed specifically for expression from an oncoretroviral vector by human T cells. iCasp9_{M} can be activated by AP1903 (or analogs), a small chemical inducer of dimerization that has proven safe at the required dose for optimum deletional effect, and unlike ganciclovir or rituximab has no other biologic effects in vivo. Therefore, expression of this suicide gene in T cells for adoptive transfer can increase safety and also may broaden the scope of clinical applications.

### Example 2: Using the iCasp9 Suicide Gene to Improve the Safety of Allodepleted T Cells after Haploidentical Stem Cell Transplantation

Presented in this example are expression constructs and methods of using the expression constructs to improve the safety of allodepleted T cells after haploidentical stem cell transplantation. A retroviral vector encoding iCasp9 and a selectable marker (truncated CD19) was generated as a safety switch for donor T cells. Even after allodepletion (using anti-CD25 immunotoxin), donor T cells could be efficiently transduced, expanded, and subsequently enriched by CD19 immunomagnetic selection to >90% purity. The engineered cells retained anti-viral specificity and functionality, and contained a subset with regulatory phenotype and function. Activating iCasp9 with a small-molecule dimerizer rapidly produced >90% apoptosis. Although transgene expression was downregulated in quiescent T cells, iCasp9 remained an efficient suicide gene, as expression was rapidly upregulated in activated (alloreactive) T cells.

### Materials and Methods

### Generation of allodepleted T cells

Allodepleted cells were generated from healthy volunteers as previously presented. Briefly, peripheral blood mononuclear cells (PBMCs) from healthy donors were co-cultured with irradiated recipient Epstein Barr virus (EBV)-transformed lymphoblastoid cell lines (LCL) at responder-to-stimulator ratio of 40:1 in serum-free medium (AIM V; Invitrogen, Carlsbad, CA). After 72 hours, activated T cells that expressed CD25 were depleted from the co-culture by overnight incubation in RFT5-SMPT-dgA immunotoxin. Allodepletion was considered adequate if the residual CD3⁺CD25⁺ population was <1% and residual proliferation by 3H-thymidine incorporation was <10%.

### Plasmid and retrovirus

SFG.iCasp9.2A.CD19 consists of inducible Caspase-9 (iCasp9) linked, via a cleavable 2A-like sequence, to truncated human CD19. iCasp9 consists of a human FK5 06-binding protein (FKBP12; GenBank AH002 818) with an F36V mutation, connected via a Ser-Gly-Gly-Gly-Ser linker to human Caspase-9 (CASP9; GenBank NM 001229). The F36V mutation increases the binding affinity of FKBP12 to the synthetic homodimerizer, AP20187 or AP1903. The Caspase recruitment domain (CARD) has been deleted from the human Caspase-9 sequence because its physiological function has been replaced by FKBP12, and its removal increases transgene expression and function. The 2A-like sequence encodes an 20 amino acid peptide from Thosea asigna insect virus, which mediates >99% cleavage between a glycine and terminal proline residue, resulting in 19 extra amino acids in the C terminus of iCasp9, and one extra proline residue in the N terminus of CD19. CD19 consists of full-length CD19 (GenBank NM 001770) truncated at amino acid 333 (TDPTRRF), which shortens the intracytoplasmic domain from 242 to 19 amino acids, and removes all conserved tyrosine residues that are potential sites for phosphorylation.

A stable PG13 clone producing Gibbon ape leukemia virus (Gal-V) pseudotyped retrovirus was made by transiently transfecting Phoenix Eco cell line (ATCC product #SD3444; ATCC, Manassas, VA)with SFG.iCasp9.2A.CD19. This produced Eco-pseudotyped retrovirus. The PG13 packaging cell line (ATCC) was transduced three times with Eco-pseudotyped retrovirus to generate a producer line that contained multiple SFG.iCasp9.2A.CD19 proviral integrants per cell. Single cell cloning was performed, and the PG13 clone that produced the highest titer was expanded and used for vector production.

### Retroviral transduction

Culture medium for T cell activation and expansion consisted of 45% RPMI 1640 (Hyclone, Logan, UT), 45% Clicks (Irvine Scientific, Santa Ana, CA) and 10% fetal bovine serum (FBS; Hyclone). Allodepleted cells were activated by immobilized anti-CD3 (OKT3; Ortho Biotech, Bridgewater, NJ) for 48 hours before transduction with retroviral vector. Selective allodepletion was performed by co-culturing donor PBMC with recipient EBV-LCL to activate alloreactive cells: activated cells expressed CD25 and were subsequently eliminated by anti-CD25 immunotoxin. The allodepleted cells were activated by OKT3 and transduced with the retroviral vector 48 hours later. Immunomagnetic selection was performed on day 4 of transduction; the positive fraction was expanded for a further 4 days and cryopreserved.

In small-scale experiments, non-tissue culture-treated 24-well plates (Becton Dickinson, San Jose, CA) were coated with OKT3 1 g/ml for 2 to 4 hours at 37°C. Allodepleted cells were added at 1×10⁶ cells per well. At 24 hours, 100U/ml of recombinant human interleukin-2 (IL-2) (Proleukin; Chiron, Emeryville, CA) was added. Retroviral transduction was performed 48 hours after activation. Non-tissue culture-treated 24-well plates were coated with 3.5µg/cm² recombinant fibronectin fragment (CH-296; Retronectin; Takara Mirus Bio, Madison, WI) and the wells loaded twice with retroviral vector-containing supernatant at 0.5ml per well for 30 minutes at 37°C, following which OKT3 -activated cells were plated at 5 ×10⁵ cells per well in fresh retroviral vector-containing supernatant and T cell culture medium at a ratio of 3:1, supplemented with 100U/ml IL-2. Cells were harvested after 2 to 3 days and expanded in the presence of 50U/ml IL-2.

### Scaling-up production of gene-modified allodepleted cells

Scale-up of the transduction process for clinical application used non-tissue culture-treated T75 flasks (Nunc, Rochester, NY), which were coated with 10ml of OKT3 1µg/ml or 10ml of fibronectin 7µg/ml at 4°C overnight. Fluorinated ethylene propylene bags corona-treated for increased cell adherence (2PF-0072AC, American Fluoroseal Corporation, Gaithersburg, MD) were also used. Allodepleted cells were seeded in OKT3 -coated flasks at 1×10⁶ cells/ml. 100U/ml IL-2 was added the next day. For retroviral transduction, retronectin-coated flasks or bags were loaded once with 10ml of retrovirus-containing supernatant for 2 to 3 hours. OKT3-activated T cells were seeded at 1×10⁶ cells/ml in fresh retroviral vector-containing medium and T cell culture medium at a ratio of 3:1, supplemented with 100U/ml IL-2. Cells were harvested the following morning and expanded in tissue-culture treated T75 or T175 flasks in culture medium supplemented with between about 50 to 100U/ml IL-2 at a seeding density of between about 5×10⁵ cells/ ml to 8×10⁵ cells/ ml.

### CD19 immunomagnetic selection

Immunomagnetic selection for CD19 was performed 4 days after transduction. Cells were labeled with paramagnetic microbeads conjugated to monoclonal mouse anti-human CD19 antibodies (Miltenyi Biotech, Auburn, CA) and selected on MS or LS columns in small scale experiments and on a CliniMacs Plus automated selection device in large scale experiments. CD19-selected cells were expanded for a further 4 days and cryopreserved on day 8 post transduction. These cells were referred to as "gene-modified allodepleted cells".

### Immunophenotyping and pentamer analysis

Flow cytometric analysis (FACSCalibur and CellQuest software; Becton Dickinson) was performed using the following antibodies: CD3, CD4, CD8, CD19, CD25, CD27, CD28, CD45RA, CD45RO, CD56 and CD62L. CD19-PE (Clone 4G7; Becton Dickinson) was found to give optimum staining and was used in all subsequent analysis. A Non-transduced control was used to set the negative gate for CD19. An HLA-pentamer, HLA-B8-RAKFKQLL (Proimmune, Springfield, VA) was used to detect T cells recognizing an epitope from EBV lytic antigen (BZLF1). HLA-A2-NLVPMVATV pentamer was used to detect T cells recognizing an epitope from CMV-pp65 antigen.

### Interferon- ELISpot assay for anti-viral response

Interferon- ELISpot for assessment of responses to EBV, CMV and adenovirus antigens was performed using known methods. Gene-modified allodepleted cells cryopreserved at 8 days post transduction were thawed and rested overnight in complete medium without IL-2 prior to use as responder cells. Cryopreserved PBMCs from the same donor were used as comparators. Responder cells were plated in duplicate or triplicate in serial dilutions of 2×10⁵, 1×10⁵, 5×10⁴ and 2.5 ×10⁴ cells per well. Stimulator cells were plated at 1×10⁵ per well. For response to EBV, donor-derived EBV-LCLs irradiated at 40Gy were used as stimulators. For response to adenovirus, donor-derived activated monocytes infected with Ad5f35 adenovirus were used.

Briefly, donor PBMCs were plated in X-Vivo 15 (Cambrex, Walkersville, MD) in 24-well plates overnight, harvested the next morning, infected with Ad5f35 at a multiplicity of infection (MOI) of 200 for 2 hours, washed, irradiated at 30Gy, and used as stimulators. For anti-CMV response, a similar process using Ad5f35 adenovirus encoding the CMV pp65 transgene (Ad5f35-pp65) at an MOI of 5000 was used. Specific spot-forming units (SFU) were calculated by subtracting SFU from responder-alone and stimulator-alone wells from test wells. Response to CMV was the difference in SFU between Ad5f35-pp65 and Ad5f35 wells.

### EBV-specific cytotoxicity

Gene-modified allodepleted cells were stimulated with 40Gy-irradiated donor-derived EBVLCL at a responder: stimulator ratio of 40:1. After 9 days, the cultures were restimulated at a responder: stimulator ratio of 4:1. Restimulation was performed weekly as indicated. After two or three rounds of stimulation, cytotoxicity was measured in a 4-hour 51 Cr-release assay, using donor EBV-LCL as target cells and donor OKT3 blasts as autologous controls. NK activity was inhibited by adding 30-fold excess of cold K562 cells.

### Induction of apoptosis with chemical inducer of dimerization, AP20187

Suicide gene functionality was assessed by adding a small molecule synthetic homodimerizer, AP20187 (Ariad Pharmaceuticals; Cambridge, MA), at 10nM final concentration the day following CD19 immunomagnetic selection. Cells were stained with annexin V and 7-aminoactinomycin (7-AAD)(BD Pharmingen) at 24 hours and analyzed by flow cytometry. Cells negative for both annexin V and 7-AAD were considered viable, cells that were annexin V positive were apoptotic, and cells that were both annexin V and 7-AAD positive were necrotic. The percentage killing induced by dimerization was corrected for baseline viability as follows: Percentage killing = 100% - (%Viability in AP20187-treated cells ÷ %Viability in non-treated cells).

### Assessment of transgene expression following extended culture and reactivation

Cells were maintained in T cell medium containing 50U/ml IL-2 until 22 days after transduction. A portion of cells was reactivated on 24-well plates coated with 1 g/ml OKT3 and 1µg/ml anti-CD28 (Clone CD28.2, BD Pharmingen, San Jose, CA) for 48 to 72 hours. CD19 expression and suicide gene function in both reactivated and non-reactivated cells were measured on day 24 or 25 post transduction.

In some experiments, cells also were cultured for 3 weeks post transduction and stimulated with 30G-irradiated allogeneic PBMC at a responder: stimulator ratio of 1:1. After 4 days of co-culture, a portion of cells was treated with 10nM AP20187. Killing was measured by annexin V/7-AAD staining at 24 hours, and the effect of dimerizer on bystander virus-specific T cells was assessed by pentamer analysis on AP20187-treated and untreated cells.

### Regulatory T cells

CD4, CD25 and Foxp3 expression was analyzed in gene-modified allodepleted cells using flow cytometry. For human Foxp3 staining, the eBioscience (San Diego, CA) staining set was used with an appropriate rat IgG2a isotype control. These cells were co-stained with surface CD25-FITC and CD4-PE. Functional analysis was performed by co-culturing CD4⁺25⁺ cells selected after allodepletion and gene modification with carboxyfluorescein diacetate N-succinimidyl ester (CFSE)-labeled autologous PBMC. CD4⁺25⁺ selection was performed by first depleting CD8⁺ cells using anti-CD 8 microbeads (Miltenyi Biotec, Auburn, CA), followed by positive selection using anti-CD25 microbeads (Miltenyi Biotec, Auburn, CA). CFSE-labeling was performed by incubating autologous PBMC at 2×10⁷/ml in phosphate buffered saline containing 1.5µM CFSE for 10 minutes. The reaction was stopped by adding an equivalent volume of FBS and incubating for 10 minutes at 37°C. Cells were washed twice before use. CFSE-labeled PBMCs were stimulated with OKT3 500ng/ml and 40G-irradiated allogeneic PBMC feeders at a PBMC:allogeneic feeder ratio of 5:1. The cells were then cultured with or without an equal number of autologous CD4⁺25⁺ gene-modified allodepleted cells. After 5 days of culture, cell division was analyzed by flow cytometry; CD19 was used to gate out non-CFSE-labeled CD4⁺CD25⁺ gene-modified T cells.

### Statistical analysis

Paired, 2-tailed Student's t test was used to determine the statistical significance of differences between samples. All data are represented as mean ± 1 standard deviation.

### Results

Selectively allodepleted T cells can be efficiently transduced with iCasp9 and expanded

Selective allodepletion was performed in accordance with clinical protocol procedures. Briefly, 3/6 to 5/6 HLA-mismatched PBMC and lymphoblastoid cell lines (LCL) were co-cultured. RFT5-SMPT-dgA immunotoxin was applied after 72 hours of co-culture and reliably produced allodepleted cells with <10% residual proliferation (mean 4.5 ± 2.8%; range 0.74 to 9.1%; 10 experiments) and containing <1% residual CD3⁺CD25⁺ cells (mean 0.23 ± 0.20%; range 0.06 to 0.73%; 10 experiments), thereby fulfilling the release criteria for selective allodepletion, and serving as starting materials for subsequent manipulation.

Allodepleted cells activated on immobilized OKT3 for 48 hours could be efficiently transduced with Gal-V pseudotyped retrovirus vector encoding SFG.iCasp9.2A.CD19. Transduction efficiency assessed by FACS analysis for CD19 expression 2 to 4 days after transduction was about 53% ± 8%, with comparable results for small-scale (24-well plates) and large-scale (T75 flasks) transduction (about 55 ± 8% versus about 50% ± 10% in 6 and 4 experiments, respectively). Cell numbers contracted in the first 2 days following OKT3 activation such that only about 61% ± 12% (range of about 45% to 80%) of allodepleted cells were recovered on the day of transduction. Thereafter, the cells showed significant expansion, with a mean expansion in the range of about 94 ± 46 -fold (range of about 40 to about153) over the subsequent 8 days, resulting in a net 58 ± 33 -fold expansion. Cell expansion in both small- and large-scale experiments was similar, with net expansion of about 45 ± 29 fold (range of about 25 to about 90) in 5 small-scale experiments and about 79 ± 34 fold (range of about 50 to about 116) in 3 large-scale experiments.

### ΔCD19 enables efficient and selective enrichment of transduced cells on immunomagnetic columns

The efficiency of suicide gene activation sometimes depends on the functionality of the suicide gene itself, and sometimes on the selection system used to enrich for gene-modified cells. The use of CD19 as a selectable marker was investigated to determine if CD19 selection enabled the selection of gene-modified cells with sufficient purity and yield, and whether selection had any deleterious effects on subsequent cell growth. Small-scale selection was performed according to manufacturer's instruction; however, it was determined that large-scale selection was optimum when 10l of CD19 microbeads was used per 1.3 ×10⁷ cells. FACS analysis was performed at 24 hours after immunomagnetic selection to minimize interference from anti-CD19 microbeads. The purity of the cells after immunomagnetic selection was consistently greater than 90%: mean percentage of CD19+ cells was in the range of about 98.3% ± 0.5% (n=5) in small-scale selections and in the range of about 97.4% ± 0.9% (n=3) in large-scale CliniMacs selections

The absolute yield of small- and large-scale selections were about 31% ± 11 % and about 28% ± 6%, respectively; after correction for transduction efficiency. The mean recovery of transduced cells was about 54% ± 14 % in small-scale and about 72% ± 18 % in large-scale selections. The selection process did not have any discernable deleterious effect on subsequent cell expansion. In 4 experiments, the mean cell expansion over 3 days following CD19 immunomagnetic selection was about 3.5 fold for the CD19 positive fraction versus about 4.1 fold for non-selected transduced cells (p=0.34) and about 3.7 fold for non-transduced cells (p=0.75).

### Immunophenotype of gene-modified allodepleted cells

The final cell product (gene-modified allodepleted cells that had been cryopreserved 8 days after transduction) was immunophenotyped and was found to contain both CD4 and CD8 cells, with CD8 cells predominant, at 62% ±11% CD8⁺ versus 23% ± 8% CD4⁺, as shown in the table below. NS= not significant, SD= standard deviation.

**Table 1**

| | Unmanipulated PBMC (mean % ± SD) | Gene-modified allodepleted cells (mean % ± SD) | |
|---|---|---|---|
| T cells: Total CD3⁺ | 82 ± 6 | 95 ± 6 | NS |
| CD3+ 4+ | 54 ± 5 | 23 ± 8 | p < 0.01 |
| CD3+ 8+ | 26 ± 9 | 62 ± 11 | p< 0.001 |
| NK cells: CD3⁻ 56+ | 6 ± 3 | 2 ± 1 | NS |
| Memory phenotype | | | |
| CD45RA⁺ | 66 ± 3 | 10 ± 5 | p<0.001 |
| CD45RO⁺ | 26 ± 2 | 78 ± 7 | p<0.001 |
| CD45RA⁻ CD62L⁺ | 19 ± 1 | 24 ± 7 | NS |
| CD45RA⁻ CD62L⁻ | 9 ± 1 | 64 ± 7 | p<0.001 |
| CD27⁺ CD28⁺ | 67 ± 7 | 19 ± 9 | p<0.001 |
| CD27⁺ CD28⁻ | 7 ± 3 | 9 ± 4 | NS |
| CD27⁻ CD28⁺ | 4 ± 1 | 19 ± 8 | p<0.05 |
| CD27⁻ CD28⁻ | 22 ± 8 | 53 ± 18 | p<0.05 |

The majorities of cells were CD45RO⁺ and had the surface immunophenotype of effector memory T cells. Expression of memory markers, including CD62L, CD27 and CD28, was heterogeneous. Approximately 24% of cells expressed CD62L, a lymph node-homing molecule predominantly expressed on central memory cells.

### Gene-modified allodepleted cells retained antiviral repertoire and functionality

The ability of end-product cells to mediate antiviral immunity was assessed by interferon-ELISpot, cytotoxicity assay, and pentamer analysis. The cryopreserved gene-modified allodepleted cells were used in all analyses, since they were representative of the product currently being evaluated for use in a clinical study. Interferon-y secretion in response to adenovirus, CMV or EBV antigens presented by donor cells was preserved although there was a trend towards reduced anti-EBV response in gene-modified allodepleted cells versus unmanipulated PBMC. The response to viral antigens was assessed by ELISpot in 4 pairs of unmanipulated PBMC and gene-modified allodepleted cells (GMAC). Adenovirus and CMV antigens were presented by donor-derived activated monocytes through infection with Ad5f35 null vector and Ad5f35-pp65 vector, respectively. EBV antigens were presented by donor EBV-LCL. The number of spot-forming units (SFU) was corrected for stimulator- and responder-alone wells. Only three of four donors were evaluable for CMV response, one seronegative donor was excluded.

Cytotoxicity was assessed using donor-derived EBV-LCL as targets. Gene-modified allodepleted cells that had undergone 2 or 3 rounds of stimulation with donor-derived EBV-LCL could efficiently lyse virus-infected autologous target cells Gene-modified allodepleted cells were stimulated with donor EBV-LCL for 2 or 3 cycles. ⁵¹Cr release assay was performed using donor-derived EBV-LCL and donor OKT3 blasts as targets. NK activity was blocked with 30-fold excess cold K562. The left panel shows results from 5 independent experiments using totally or partially mismatched donor-recipient pairs. The right panel shows results from 3 experiments using unrelated HLA haploidentical donor-recipient pairs. Error bars indicate standard deviation.

EBV-LCLs were used as antigen-presenting cells during selective allodepletion, therefore it was possible that EBV-specific T cells could be significantly depleted when the donor and recipient were haploidentical. To investigate this hypothesis, three experiments using unrelated HLA-haploidentical donor-recipient pairs were included, and the results showed that cytotoxicity against donor-derived EBV-LCL was retained. The results were corroborated by pentamer analysis for T cells recognizing HLA-B8-RAKFKQLL, an EBV lytic antigen (BZLF1) epitope, in two informative donors following allodepletion against HLA-B8 negative haploidentical recipients. Unmanipulated PBMC were used as comparators. The RAK-pentamer positive population was retained in gene-modified allodepleted cells and could be expanded following several rounds of in vitro stimulation with donor-derived EBV-LCL. Together, these results indicate that gene-modified allodepleted cells retained significant anti-viral functionality.

### Regulatory T cells in the Gene-modified allodepleted cell population

Flow cytometry and functional analysis were used to determine whether regulatory T cells were retained in our allodepleted, gene modified, T cell product. A Foxp3⁺ CD4⁺25⁺ population was found. Following immunomagnetic separation, the CD4⁺CD25⁺ enriched fraction demonstrated suppressor function when co-cultured with CFSE-labeled autologous PBMC in the presence of OKT3 and allogeneic feeders Donor-derived PBMC was labeled with CFSE and stimulated with OKT3 and allogeneic feeders. CD4⁺CD25⁺ cells were immunomagnetically selected from the gene-modified cell population and added at 1:1 ratio to test wells. Flow cytometry was performed after 5 days. Gene-modified T cells were gated out by CD19 expression. The addition of CD4⁺CD25⁺ gene-modified cells (bottom panel) significantly reduced cell proliferation. Thus, allodepleted T cells may reacquire regulatory phenotype even after exposure to a CD25 depleting immunotoxin.

### Gene-modified allodepleted cells were efficiently and rapidly eliminated by addition of chemical inducer of dimerization

The day following immunomagnetic selection, 10nM of the chemical inducer of dimerization, AP20187, was added to induce apoptosis, which appeared within 24 hours. FACS analysis with annexin V and 7-AAD staining at 24 hours showed that only about 5.5% ± 2.5% of AP20187-treated cells remained viable, whereas about 81.0% ± 9.0 % of untreated cells were viable. Killing efficiency after correction for baseline viability was about 92.9% ± 3.8%. Large-scale CD19 selection produced cells that were killed with similar efficiency as small-scale selection: mean viability with and without AP20187, and percentage killing, in large and small scale were about 3.9%, about 84.0%, about 95.4% (n=3) and about 6.6%, about 79.3%, about 91.4% (n=5) respectively. AP20187 was non-toxic to non-transduced cells: viability with and without AP20187 was about 86% ± 9% and 87% ± 8% respectively (n=6).

### Transgene expression and function decreased with extended culture but were restored upon cell reactivation

To assess the stability of transgene expression and function, cells were maintained in T cell culture medium and low dose IL-2 (50U/ml) until 24 days after transduction. A portion of cells was then reactivated with OKT3/ anti-CD28. CD19 expression was analyzed by flow cytometry 48 to 72 hours later, and suicide gene function was assessed by treatment with 10nM AP20187. The obtained are for cells from day 5 post transduction (ie, 1 day after CD 19 selection) and day 24 post transduction, with or without 48-72 hours of reactivation (5 experiments). In 2 experiments, CD25 selection was performed after OKT3/aCD28 activation to further enrich activated cells. Error bars represent standard deviation. * indicates p<0.05 when compared to cells from day 5 post transduction. By day 24, surface CD19 expression fell from about 98% ± 1% to about 88% ± 4% (p<0.05) with a parallel decrease in mean fluorescence intensity (MFI) from 793 ± 128 to 478 ± 107 (p<0.05) (see FIG. 13B). Similarly, there was a significant reduction in suicide gene function: residual viability was 19.6 ± 5.6% following treatment with AP20187; after correction for baseline viability of 54.8 ± 20.9%, this equated to killing efficiency of only 63.1 ± 6.2%.

To determine whether the decrease in transgene expression with time was due to reduced transcription following T cell quiescence or to elimination of transduced cells, a portion of cells were reactivated on day 22 post transduction with OKT3 and anti-CD28 antibody. At 48 to 72 hours (day 24 or 25 post transduction), OKT3/aCD28-reactivated cells had significantly higher transgene expression than non-reactivated cells. CD19 expression increased from about 88% ± 4% to about 93% ± 4% (p <0.01) and CD19 MFI increased from 478 ± 107 to 643 ± 174 (p<0.01). Additionally, suicide gene function also increased significantly from about a 63.1% ± 6.2% killing efficiency to about a 84.6% ± 8.0% (p<0.01) killing efficiency. Furthermore, killing efficiency was completely restored if the cells were immunomagnetically sorted for the activation marker CD25: killing efficiency of CD25 positive cells was about 93%.2 ± 1.2%, which was the same as killing efficiency on day 5 post transduction (93.1 ± 3.5%). Killing of the CD25 negative fraction was 78.6 ± 9.1%.

An observation of note was that many virus-specific T cells were spared when dimerizer was used to deplete gene-modified cells that have been re-activated with allogeneic PBMC, rather than by non-specific mitogenic stimuli. After 4 days reactivation with allogeneic cells, as shown in FIGS. 14A and 14B, treatment with AP20187 spares (and thereby enriches) viral reactive subpopulations, as measured by the proportion of T cells reactive with HLA pentamers specific for peptides derived from EBV and CMV. Gene-modified allodepleted cells were maintained in culture for 3 weeks post-transduction to allow transgene down-modulation. Cells were stimulated with allogeneic PBMC for 4 days, following which a portion was treated with 10nM AP20187. The frequency of EBV-specific T cells and CMV-specific T cells were quantified by pentamer analysis before allostimulation, after allostimulation, and after treatment of allostimulated cells with dimerizer. The percentage of virus-specific T cells decreased after allostimulation. Following treatment with dimerizer, virus-specific T cells were partially and preferentially retained.

### Discussion

The feasibility of engineering allogeneic T cells with two distinct safety mechanisms, selective allodepletion and suicide gene-modification has been demonstrated herein. In combination, these modifications can enhance and/or enable addback of substantial numbers of T cells with anti-viral and anti-tumor activity, even after haploidentical transplantation. The data presented herein show that the suicide gene, iCasp9, functions efficiently (>90% apoptosis after treatment with dimerizer) and that down-modulation of transgene expression that occurred with time was rapidly reversed upon T cell activation, as would occur when alloreactive T cells encountered their targets. Data presented herein also show that CD19 is a suitable selectable marker that enabled efficient and selective enrichment of transduced cells to >90% purity, Furthermore, the data presented herein indicate that these manipulations had no discernable effects on the immunological competence of the engineered T cells with retention of antiviral activity, and regeneration of a CD4⁺CD25⁺Foxp3⁺ population with Treg activity.

Given that the overall functionality of suicide genes depends on both the suicide gene itself and the marker used to select the transduced cells, translation into clinical use requires optimization of both components, and of the method used to couple expression of the two genes. The two most widely used selectable markers, currently in clinical practice, each have drawbacks. Neomycin phosphotransferase (neo) encodes a potentially immunogenic foreign protein and requires a 7-day culture in selection medium, which not only increases the complexity of the system, but is also potentially damaging to virus-specific T cells. A widely used surface selection marker, LNGFR, has recently had concerns raised, regarding its oncogenic potential and potential correlation with leukemia, in a mouse model, despite its apparent clinical safety. Furthermore, LNGFR selection is not widely available, because it is used almost exclusively in gene therapy. A number of alternative selectable markers have been suggested. CD34 has been well-studied in vitro, but the steps required to optimize a system configured primarily for selection of rare hematopoietic progenitors, and more critically, the potential for altered in vivo T cell homing, make CD34 sub-optimal for use as a selectable marker for a suicide switch expression construct. CD19 was chosen as an alternative selectable marker, since clinical grade CD19 selection is readily available as a method for B-cell depletion of stem cell autografts. The results presented herein demonstrated that CD19 enrichment could be performed with high purity and yield and, furthermore, the selection process had no discernable effect on subsequent cell growth and functionality.

The effectiveness of suicide gene activation in CD19-selected iCasp9 cells compared very favorably to that of neo- or LNGFR-selected cells transduced to express the HSVtk gene. The earlier generations of HSVtk constructs provided 80-90% suppression of ³H-thymidine uptake and showed similar reduction in killing efficiency upon extended in vitro culture, but were nonetheless clinically efficacious. Complete resolution of both acute and chronic GVHD has been reported with as little as 80% in vivo reduction in circulating gene-modified cells. These data support the hypothesis that transgene down-modulation seen in vitro is unlikely to be an issue because activated T cells responsible for GVHD will upregulate suicide gene expression and will therefore be selectively eliminated in vivo. Whether this effect is sufficient to allow retention of virus- and leukemia-specific T cells in vivo will be tested in a clinical setting. By combining in vitro selective allodepletion prior to suicide gene modification, the need to activate the suicide gene mechanism may be significantly reduced, thereby maximizing the benefits of addback T cell based therapies.

The high efficiency of iCasp9-mediated suicide seen in vitro has been replicated in vivo. In a SCID mouse-human xenograft model, more than 99% of iCasp9-modified T cells were eliminated after a single dose of dimerizer. AP1903, which has extremely close functional and chemical equivalence to AP20187, and currently is proposed for use in a clinical application, has been safety tested on healthy human volunteers and shown to be safe. Maximal plasma level of between about 10 ng/ml to about 1275 ng/ml AP1903 (equivalent to between about 7 nM to about 892 nM) was attained over a 0.01 mg/kg to 1.0mg/kg dose range administered as a 2-hour intravenous infusion. There were substantially no significant adverse effects. After allowing for rapid plasma redistribution, the concentration of dimerizer used in vitro remains readily achievable in vivo.

Optimal culture conditions for maintaining the immunological competence of suicide gene-modified T cells must be determined and defined for each combination of safety switch, selectable marker and cell type, since phenotype, repertoire and functionality can all be affected by the stimulation used for polyclonal T cell activation, the method for selection of transduced cells, and duration of culture. The addition of CD28 co-stimulation and the use of cell-sized paramagnetic beads to generate gene modified-cells that more closely resemble unmanipulated PBMC in terms of CD4:CD8 ratio, and expression of memory subset markers including lymph node homing molecules CD62L and CCR7, may improve the in vivo functionality of gene-modified T cells. CD28 co-stimulation also may increase the efficiency of retroviral transduction and expansion. Interestingly however, the addition of CD28 co-stimulation was found to have no impact on transduction of allodepleted cells, and the degree of cell expansion demonstrated was higher when compared to the anti-CD3 alone arm in other studies. Furthermore, iCasp9-modified allodepleted cells retained significant anti-viral functionality, and approximately one fourth retained CD62L expression. Regeneration of CD4⁺CD25⁺Foxp3⁺ regulatory T cells was also seen. The allodepleted cells used as the starting material for T cell activation and transduction may have been less sensitive to the addition of anti-CD28 antibody as co-stimulation. CD25-depleted PBMC / EBV-LCL co-cultures contained T cells and B cells that already express CD86 at significantly higher level than unmanipulated PBMCs and may they provide co-stimulation. Depletion of CD25⁺ regulatory T cells prior to polyclonal T cell activation with anti-CD3 has been reported to enhance the immunological competence of the final T cell product. In order to minimize the effect of in vitro culture and expansion on functional competence, a relatively brief culture period was used in some experiments presented herein, whereby cells were expanded for a total of 8 days post-transduction with CD19-selection being performed on day 4.

Finally, scaled up production was demonstrated such that sufficient cell product can be produced to treat adult patients at doses of up to 10⁷ cells/kg: allodepleted cells can be activated and transduced at 4×10⁷cells per flask, and a minimum of 8-fold return of CD19-selected final cell product can be obtained on day 8 post-transduction, to produce at least 3×10⁸ allodepleted gene-modified cells per original flask. The increased culture volume is readily accommodated in additional flasks or bags.

The allodepletion and iCasp9-modification presented herein may significantly improve the safety of adding back T cells, particularly after haploidentical stem cell allografts. This should in turn enable greater dose-escalation, with a higher chance of producing an anti-leukemia effect.

### Example 3: CASPALLO - Phase 1 Clinical Trial of Allodepleted T Cells Transduced with Inducible Caspase-9 Suicide Gene after Haploidentical Stem Cell Transplantation

This example presents results of a phase 1 clinical trial using the alternative suicide gene strategy illustrated in FIG. 2. Briefly, donor peripheral blood mononuclear cells were co-cultured with recipient irradiated EBV-transformed lymphoblastoid cells (40:1) for 72 hrs, allodepleted with a CD25 immunotoxin and then transduced with a retroviral supernatant carrying the iCasp9 suicide gene and a selection marker (ΔCD19); ΔCD19 allowed enrichment to >90% purity via immunomagnetic selection.

An example of a protocol for generation of a cell therapy product is provided herein.

### Source Material

Up to 240 ml (in 2 collections) of peripheral blood was obtained from the transplant donor according to established protocols. In some cases, dependent on the size of donor and recipient, a leukopheresis was performed to isolate sufficient T cells. 10cc-30cc of blood also was drawn from the recipient and was used to generate the Epstein Barr virus (EBV)-transformed lymphoblastoid cell line used as stimulator cells. In some cases, dependent on the medical history and/or indication of a low B cell count, the LCLs were generated using appropriate 1st degree relative (e.g., parent, sibling, or offspring) peripheral blood mononuclear cells.

### Generation of Allodepleted Cells

Allodepleted cells were generated from the transplant donors as presented herein. Peripheral blood mononuclear cells (PBMCs) from healthy donors were co-cultured with irradiated recipient Epstein Barr virus (EBV)-transformed lymphoblastoid cell lines (LCL) at responder-to-stimulator ratio of 40:1 in serum-free medium (AIM V; Invitrogen, Carlsbad, CA). After 72 hours, activated T cells that express CD25 were depleted from the co-culture by overnight incubation in RFT5-SMPT-dgA immunotoxin. Allodepletion is considered adequate if the residual CD3⁺CD25⁺ population was <1% and residual proliferation by ³H-thymidine incorporation was <10%.

### Retroviral Production

A retroviral producer line clone was generated for the iCasp9-CD19 construct. A master cell-bank of the producer also was generated. Testing of the master-cell bank was performed to exclude generation of replication competent retrovirus and infection by Mycoplasma, HIV, HBV, HCV and the like. The producer line was grown to confluency, supernatant harvested, filtered, aliquoted and rapidly frozen and stored at -80°C. Additional testing was performed on all batches of retroviral supernatant to exclude Replication Competent Retrovirus (RCR) and issued with a certificate of analysis, as per protocol.

### Transduction of Allodepleted Cells

Allodepleted T-lymphocytes were transduced using Fibronectin. Plates or bags were coated with recombinant Fibronectin fragment CH-296 (RetronectinTM, Takara Shuzo, Otsu, Japan). Virus was attached to retronectin by incubating producer supernatant in coated plates or bags. Cells were then transferred to virus coated plates or bags. After transduction allodepleted T cells were expanded, feeding them with IL-2 twice a week to reach the sufficient number of cells as per protocol.

### CD19 Immunomagnetic Selection

Immunomagnetic selection for CD19 was performed 4 days after transduction. Cells are labeled with paramagnetic microbeads conjugated to monoclonal mouse anti-human CD19 antibodies (Miltenyi Biotech, Auburn, CA) and selected on a CliniMacs Plus automated selection device. Depending upon the number of cells required for clinical infusion cells were either cryopreserved after the CliniMacs selection or further expanded with IL-2 and cryopreserved on day 6 or day 8 post transduction.

### Freezing

Aliquots of cells were removed for testing of transduction efficiency, identity, phenotype and microbiological culture as required for final release testing by the FDA. The cells were cryopreserved prior to administration according to protocol.

### Study Drugs

### RFT5-SMPT-dgA

RFT5-SMPT-dgA is a murine IgG1 anti-CD25 (IL-2 receptor alpha chain) conjugated via a hetero--bifunctional crosslinker [N-succinimidyloxycarbonyl-alpha-methyl-d- (2-pyridylthio) toluene] (SMPT) to chemically deglycosylated ricin A chain (dgA). RFT5-SMPT-dgA is formulated as a sterile solution at 0.5 mg/ml.

### Synthetic homodimerizer, AP1903

Mechanism of Action: AP1903-inducible cell death is achieved by expressing a chimeric protein comprising the intracellular portion of the human (Caspase-9 protein) receptor, which signals apoptotic cell death, fused to a drug-binding domain derived from human FK506-binding protein (FKBP). This chimeric protein remains quiescent inside cells until administration of AP1903, which cross-links the FKBP domains, initiating Caspase signaling and apoptosis. Toxicology: AP1903 has been evaluated as an Investigational New Drug (IND) by the FDA and has successfully completed a phase 1 clinical safety study. No significant adverse effects were noted when API 903 was administered over a 0.01 mg/kg to 1.0mglkg dose range.

Pharmacology/Pharmacokinetics: Patients received 0.4 mg/kg of AP1903 as a 2 h infusion - based on published Pk data which show plasma concentrations of 10 ng/mL - I275 ng/mL over the 0.01 mg/kg to 1.0 mg/kg dose range with plasma levels falling to 18% and 7% of maximum at 0.5 and 2 hrs post dose.

Side Effect Profile in Humans: No serious adverse events occurred during the Phase 1 study in volunteers. The incidence of adverse events was very low following each treatment, with all adverse events being mild in severity. Only one adverse event was considered possibly related to AP1903. This was an episode of vasodilatation, presented as "facial flushing" for 1 volunteer at the 1.0 mg/kg AP1903 dosage. This event occurred at 3 minutes after the start of infusion and resolved after 32 minutes duration. All other adverse events reported during the study were considered by the investigator to be unrelated or to have improbable relationship to the study drug. These events included chest pain, flu syndrome, halitosis, headache, injection site pain, vasodilatation, increased cough, rhinitis, rash, gum hemorrhage, and ecchymosis.

Patients developing grade 1 GVHD were treated with 0.4 mg/kg AP1903 as a 2-hour infusion. Protocols for administration of AP1903 to patients grade 1 GVHD were established as follows. Patients developing GvHD after infusion of allodepleted T cells are biopsied to confirm the diagnosis and receive 0.4 mg/kg of AP1903 as a 2 h infusion. Patients with Grade I GVHD received no other therapy initially, however if they showed progression of GvHD conventional GvHD therapy was administered as per institutional guidelines. Patients developing grades 2-4 GVHD were administered standard systemic immunosuppressive therapy per institutional guidelines, in addition to the AP1903 dimerizer drug:

Instructions for preparation and infusion: AP1903 for injection is obtained as a concentrated solution of 2.33 ml in a 3-ml vial, at a concentration of 5 mg/ml, (i.e., 11.66 mg per vial). AP1903 may also be provided, for example, at 8ml per vial, at 5mg/ml. Prior to administration, the calculated dose was diluted to 100 mL in 0.9% normal saline for infusion. AP1903 for injection (0.4 mg/kg) in a volume of 100 ml was administered via IV infusion over 2 hours, using a non-DEHP, non-ethylene oxide sterilized infusion set and infusion pump.

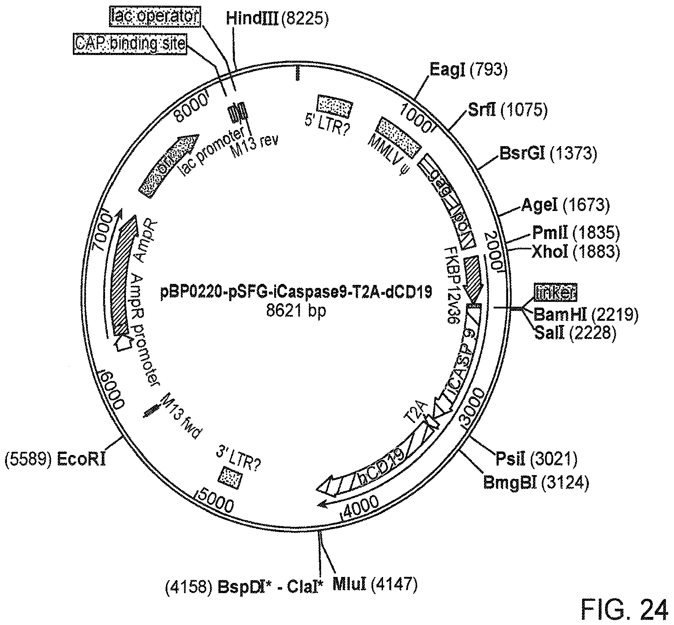

The iCasp9 suicide gene expression construct (e.g., SFG.iCasp9.2A.ΔCD19), shown in FIG. 24 consists of inducible Caspase-9 (iCasp9) linked, via a cleavable 2A-like sequence, to truncated human CD19 (ΔCD19). iCasp9 includes a human FK506-binding protein (FKBP12; GenBank AH002 818) with an F36V mutation, connected via a Ser-Gly-Gly-Gly-Ser-Gly linker to human Caspase-9 (CASP9; GenBank NM 001229). The F36V mutation may increase the binding affinity of FKBP12 to the synthetic homodimerizer, AP20187 or AP1903. The Caspase recruitment domain (CARD) has been deleted from the human Caspase-9 sequence and its physiological function has been replaced by FKBP12. The replacement of CARD with FKBP12 increases transgene expression and function. The 2A-like sequence encodes an 18 amino acid peptide from Thosea Asigna insect virus, which mediates >99% cleavage between a glycine and terminal proline residue, resulting in 17 extra amino acids in the C terminus of iCasp9, and one extra proline residue in the N terminus of CD19. Δ CD19 consists of full length CD19 (GenBank NM 001770) truncated at amino acid 333 (TDPTRRF), which shortens the intracytoplasmic domain from 242 to 19 amino acids, and removes all conserved tyrosine residues that are potential sites for phosphorylation.

### In vivo studies

Three patients received iCasp9+ T cells after haplo-CD34⁺ stem cell transplantation (SCT), at dose levels between about 1x10⁶ to about 3x10⁶ cells/kg.

**Table 2: Characteristics of the patients and clinical outcome.**

| **Patient #** | **Sex (age (yr))** | **Diagnosis** | **Disease status at SCT** | **Days from SCT to T-cell infusion** | **Number of cells infused per kg** | **Acute GvHD** | **Clinical outcome** |
|---|---|---|---|---|---|---|---|
| P1 | M(3) | MDS/AML | CR2 | 63 | 1 x 10⁶ | Grade1/2 (skin, liver) | Alive in CR>12 months No GvHD |
| P2 | F(17) | B-ALL | CR2 | 80 and 112 | (1 x 10⁶)2 | Grade 1 (skin) | Alive in CR>12 months No GvHD |
| P3 | M(8) | T-ALL | PIF/CR1 | 93 | 3 x 10⁶ | None | Alive in CR>12 No GvHD |
| P4 | F(4) | T-ALL | Active disease | 30 | 3 x 10⁶ | Grade 1 (skin) | Alive in CR>12 No GvHD |

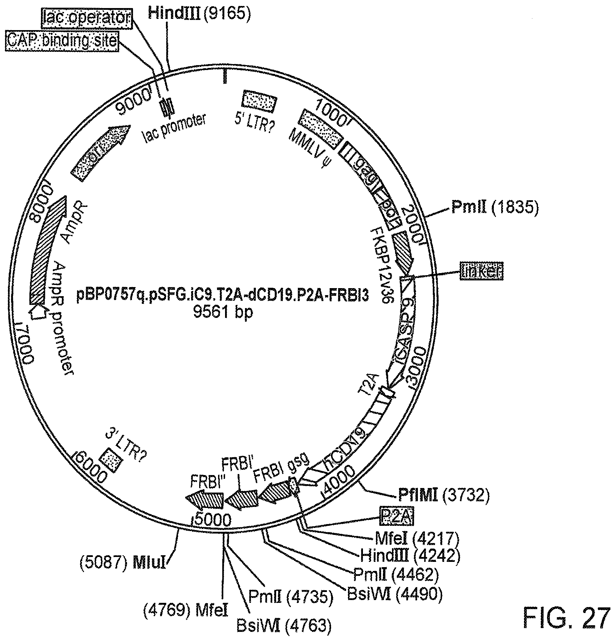

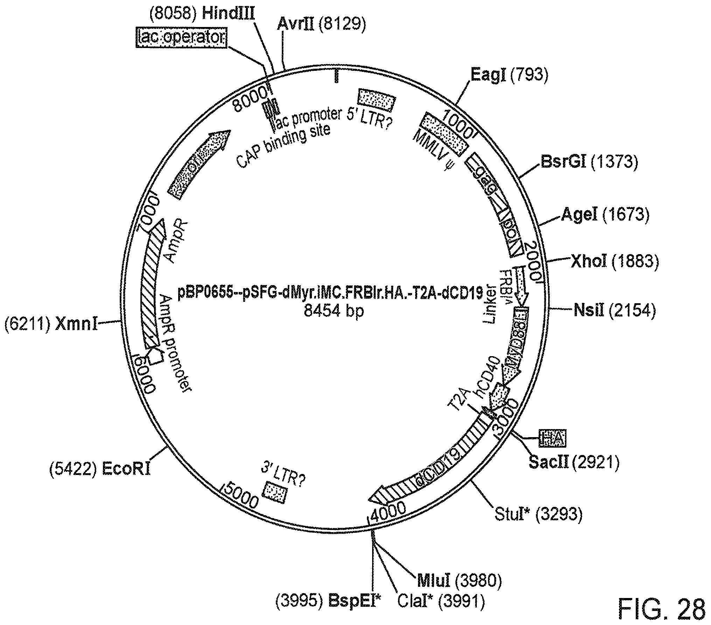

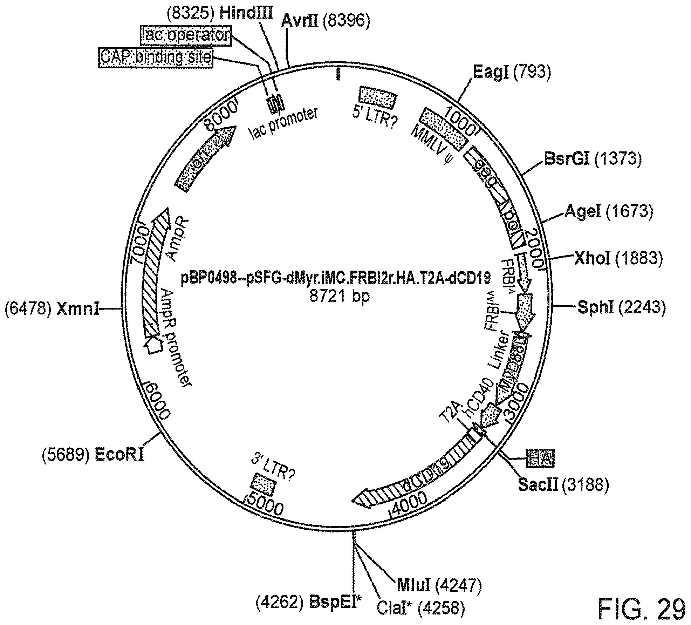

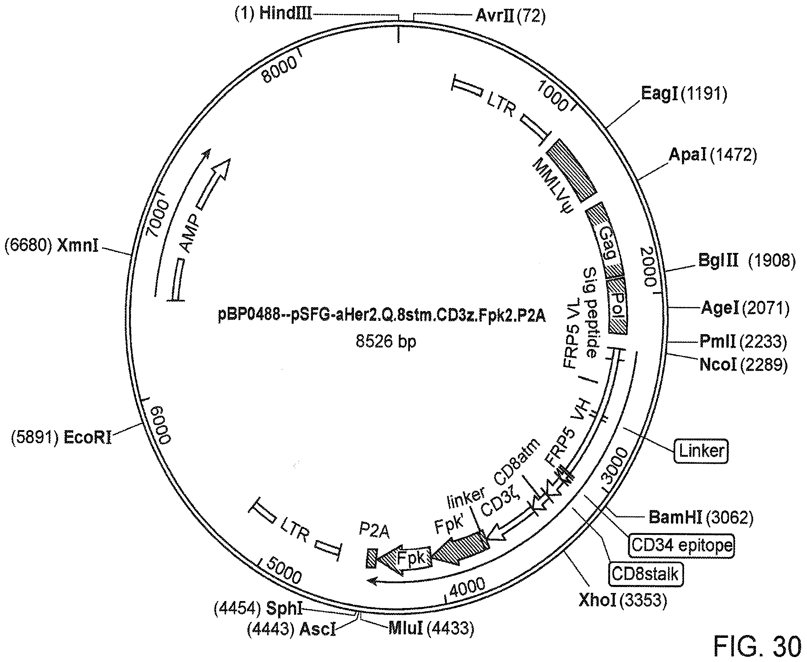

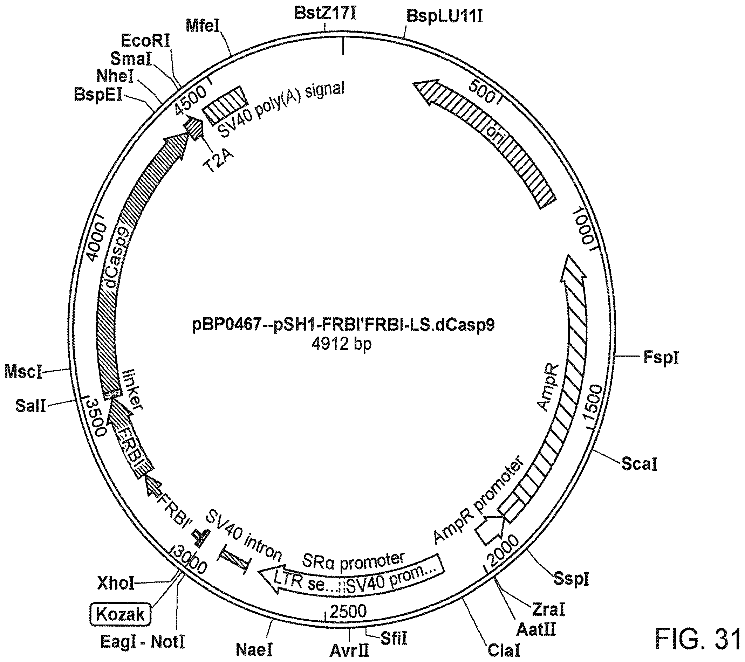

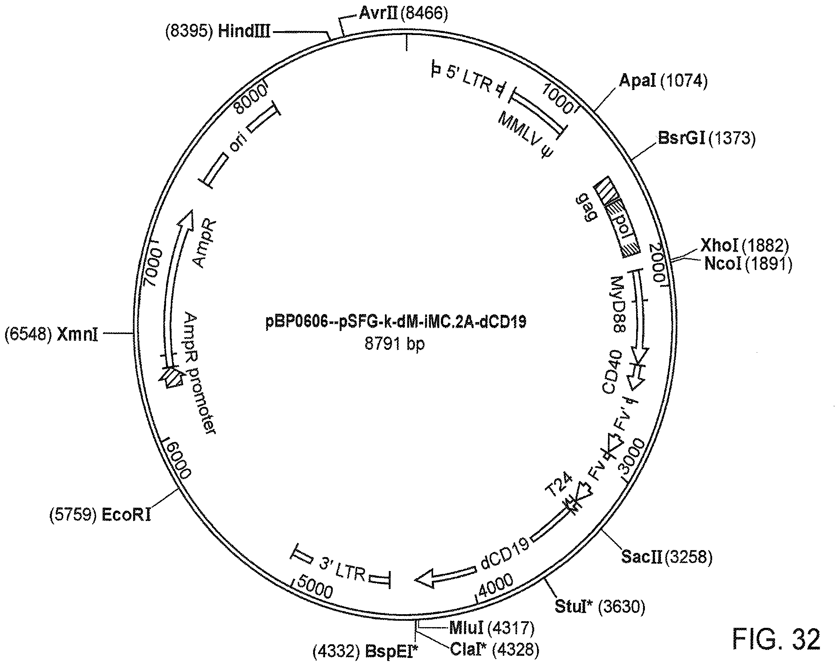

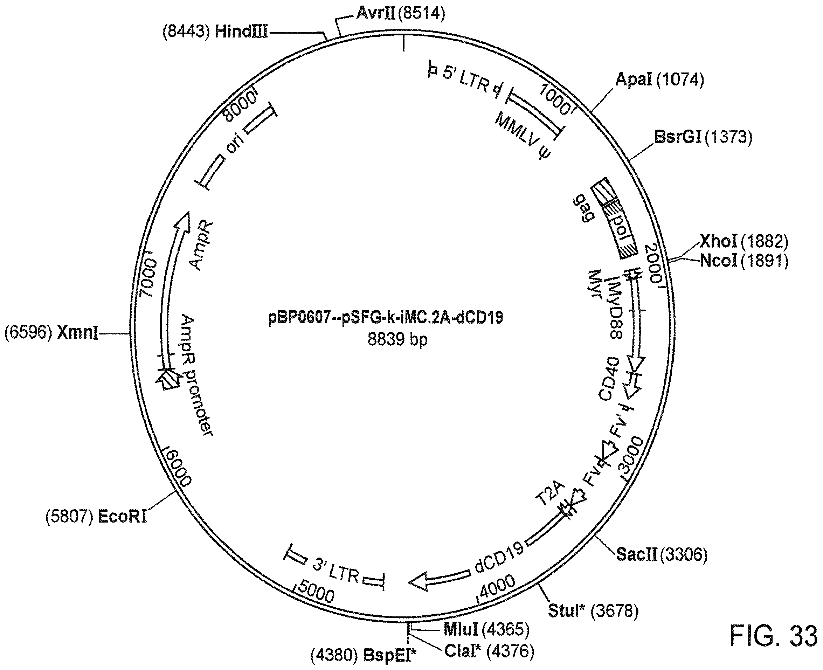

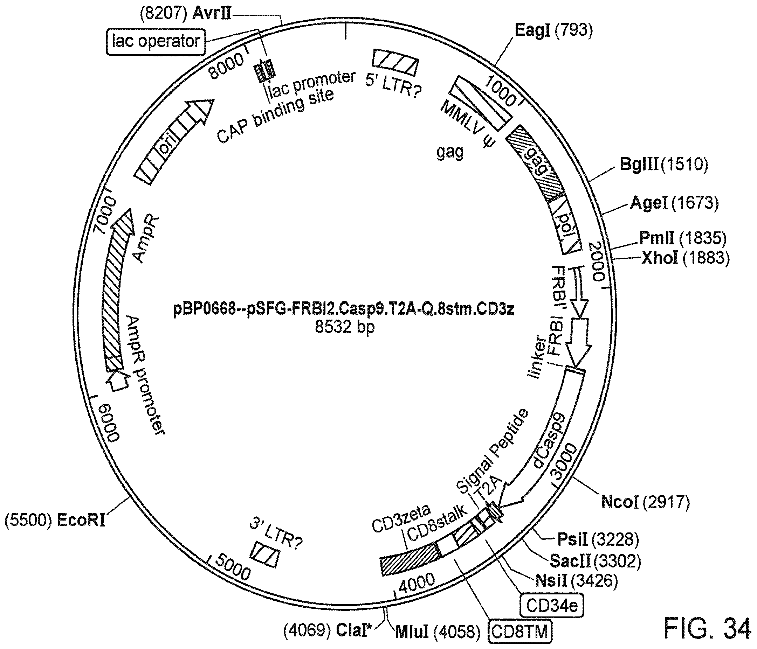

Infused T cells were detected in vivo by flow cytometry (CD3+ΔCD19+) or qPCR as early as day 7 after infusion, with a maximum fold expansion of 170±5 (day 29±9 after infusion), as illustrated in FIGS. 27, 28, and 29. Two patients developed grade I/II aGVHD (see FIGS. 31-32) and AP1903 administration caused >90% ablation of CD3+ΔCD19+ cells, within 30 minutes of infusion (see FIGS. 30, 33, and 34), with a further log reduction within 24 hours, and resolution of skin and liver aGvHD within 24hrs, showing that iCasp9 transgene was functional in vivo. For patient two, the disappearance of skin rash within 24 hours post treatment was observed.

**Table 3: Patients with GvHD (dose level 1)**

| **Patient** | **SCT to GvHD (days)** | **T cells to GvHD (days)** | **GvHD (grade/site)** |
|---|---|---|---|
| 1 | 77 | 14 | 2 (liver, skin) |
| 2 | 124 | 45/13 | 2 (skin) |

Ex vivo experiments confirmed this data. Furthermore, the residual allodepleted T cells were able to expand and were reactive to viruses (CMV) and fungi (Aspergillus fumigatus) (IFN-γ production). These in vivo studies found that a single dose of dimerizer drug can reduce or eliminate the subpopulation of T cells causing GvHD, but can spare virus specific CTLs, which can then re-expand.

### Immune reconstitution

Depending on availability of patient cells and reagents, immune reconstitution studies (Immunophenotyping, T and B cell function) may be obtained at serial intervals after transplant. Several parameters measuring immune reconstitution resulting from iCaspase transduced allodepleted T cells will be analyzed. The analysis includes repeated measurements of total lymphocyte counts, T and CD19 B cell numbers, and FACS analysis of T cell subsets (CD3, CD4, CD8, CD16, CD19, CD27, CD28, CD44, CD62L, CCR7, CD56, CD45RA, CD45RO, alpha/beta and gamma/delta T cell receptors). Depending on the availability of a patients T cells T regulatory cell markers such as CD41CD251FoxP3 also are analyzed. Approximately 10-60 ml of patient blood is taken, when possible, 4 hours after infusion, weekly for 1 month, monthly x 9 months, and then at 1 and 2 years. The amount of blood taken is dependent on the size of the recipient and does not exceed 1-2 cc/kg in total (allowing for blood taken for clinical care and study evaluation) at any one blood draw.

### Persistence and safety of transduced allodepleted T cells

The following analysis was also performed on the peripheral blood samples to monitor function, persistence and safety of transduced T-cells at time-points indicated in the study calendar:
- Phenotype by flow cytometry to detect the presence of transgenic cells.
- RCR testing by PCR.
- Quantitative real-time PCR for detecting retroviral integrants.
- RCR testing by PCR is performed pre study, at 3, 6, and12 months, and
   then yearly for a total of 15 years. Tissue, cell, and serum samples are archived for use in future studies for RCR as required by the FDA.

### Statistical Analysis and Stopping rules.

The MTD is defined to be the dose which causes grade III/IV acute GVHD in at most 25% of eligible cases. The determination is based on a modified continual reassessment method (CRM) using a logistic model with a cohort of size 2. Three dose groups are being evaluated namely, 1x10⁶, 3x10⁶, 1x10⁷ with prior probabilities of toxicity estimated at 10%, 15%, and 30%, respectively. The proposed CRM design employs modifications to the original CRM by accruing more than one subject in each cohort, limiting dose escalation to no more than one dose level, and starting patient enrollment at the lowest dose level shown to be safe for non-transduced cells. Toxicity outcome in the lowest dose cohort is used to update the dose-toxicity curve. The next patient cohort is assigned to the dose level with an associated probability of toxicity closest to the target probability of 25%. This process continues until at least 10 patients have been accrued into this dose-escalation study. Depending on patient availability, at most 18 patients may be enrolled into the Phase 1 trial or until 6 patients have been treated at the current MTD. The final MTD will be the dose with probability closest to the target toxicity rate at these termination points.

Simulations were performed to determine the operating characteristics of the proposed design and compared this with a standard 3+3 dose-escalation design. The proposed design delivers better estimates of the MTD based on a higher probability of declaring the appropriate dose level as the MTD, afforded smaller number of patients accrued at lower and likely ineffective dose levels, and maintained a lower average total number of patients required for the trial. A shallow dose-toxicity curve is expected over the range of doses proposed herein and therefore accelerated dose-escalations can be conducted without comprising patient safety. The simulations performed indicate that the modified CRM design does not incur a larger average number of total toxicities when compared to the standard design (total toxicities equal to 1.9 and 2.1, respectively.).

Grade III/IV GVHD that occurs within 45 days after initial infusion of allodepleted T cells will be factored into the CRM calculations to determine the recommended dose for the subsequent cohort. Real-time monitoring of patient toxicity outcome is performed during the study in order to implement estimation of the dose-toxicity curve and determine dose level for the next patient cohort using one of the pre-specified dose levels.
Treatment limiting toxicities will include:
- grade 4 reactions related to infusion,
- graft failure (defined as a subsequent decline in the ANC to < 500/mm³ for three consecutive measurements on different days, unresponsive to growth factor therapy that persists for at least 14 days.) occurring within 30 days after infusion of TC-T
- grade 4 nonhematologic and noninfectious adverse events, occurring within 30 days after infusion
- grades 3-4 acute GVHD by 45 days after infusion of TC-T
- treatment-related death occurring within 30 days after infusion

GVHD rates are summarized using descriptive statistics along with other measures of safety and toxicity. Likewise, descriptive statistics will be calculated to summarize the clinical and biologic response in patients who receive AP1903 due to great than Grade 1 GVHD.

Several parameters measuring immune reconstitution resulting from iCaspase transduced allodepleted T cells will be analyzed. These include repeated measurements of total lymphocyte counts, T and CD19 B cell numbers, and FACS analysis of T cell subsets (CD3, CD4, CDS, CD16, CD19, CD27, CD44, CD62L, CCR7, CD56, CD45RA, CD45RO, alpha/beta and gamma/delta T cell receptors). If sufficient T cells remain for analysis, T regulatory cell markers such as CD4/CD25/FoxP3 will also be analyzed. Each subject will be measured pre-infusion and at multiple time points post-infusion as presented above.

Descriptive summaries of these parameters in the overall patient group and by dose group as well as by time of measurement will be presented. Growth curves representing measurements over time within a patient will be generated to visualize general patterns of immune reconstitution. The proportion of iCasp9 positive cells will also be summarized at each time point. Pairwise comparisons of changes in these endpoints over time compared to pre-infusion will be implemented using paired t-tests or Wilcoxon signed-ranks test.

Longitudinal analysis of each repeatedly-measured immune reconstitution parameter using the random coefficients model will be performed. Longitudinal analysis allows construction of model patterns of immune reconstitution per patient while allowing for varying intercepts and slopes within a patient. Dose level as an independent variable in the model to account for the different dose levels received by the patients will also be used. Testing whether there is a significant improvement in immune function over time and estimates of the magnitude of these improvements based on estimates of slopes and its standard error will be possible using the model presented herein. Evaluation of any indication of differences in rates of immune reconstitution across different dose levels of CTLs will also be performed. The normal distribution with an identity link will be utilized in these models and implemented using SAS MIXED procedure. The normality assumption of the immune reconstitution parameters will be assessed and transformations (e.g. log, square root) can be performed, if necessary to achieve normality.

A strategy similar to the one presented above can be employed to assess kinetics of T cell survival, expansion and persistence. The ratio of the absolute T cell numbers with the number of marker gene positive cells will be determined and modeled longitudinally over time. A positive estimate of the slope will indicate increasing contribution of T cells for immune recovery. Virus-specific immunity of the iCasp9 T cells will be evaluated by analysis of the number of T cells releasing IFN gamma based on ex-vivo stimulation virus-specific CTLs using longitudinal models. Separate models will be generated for analysis of EBV, CMV and adenovirus evaluations of immunity.

Finally, overall and disease-free survival in the entire patient cohort will be summarized using the Kaplan-Meier product-limit method. The proportion of patients surviving and who are disease-free at 100 days and 1 year post transplant can be estimated from the Kaplan-Meier curves.

In conclusion, addback of iCasp9+ allodepleted T cells after haplo CD34⁺ SCT allows a significant expansion of functional donor lymphocytes in vivo and a rapid clearance of alloreactive T cells with resolution of aGvHD.

### Example 4: In vivo T cell Allodepletion

The protocols provided in Examples 1-3 may also be modified to provide for in vivo T cell allodepletion. To extend the approach to a larger group of subjects who might benefit from immune reconstitution without acute GvHD, the protocol may be simplified, by providing for an in vivo method of T cell depletion. In the pre-treatment allodepletion method, as discussed herein, EBV-transformed lymphoblastoid cell lines are first prepared from the recipient, which then act as alloantigen presenting cells. This procedure can take up to 8 weeks, and may fail in extensively pre-treated subjects with malignancy, particularly if they have received rituximab as a component of their initial therapy. Subsequently, the donor T cells are co-cultured with recipient EBV-LCL, and the alloreactive T cells (which express the activation antigen CD25) are then treated with CD25-ricin conjugated monoclonal antibody. This procedure may take many additional days of laboratory work for each subject.

The process may be simplified by using an in vivo method of allodepletion, building on the observed rapid in vivo depletion of alloreactive T cells by dimerizer drug and the sparing of unstimulated but virus /fungus reactive T cells.

If there is development of Grade I or greater acute GvHD, a single dose of dimerizer drug is administered, for example at a dose of 0.4 mg/kg of AP1903 as a 2 hour intravenous infusion. Up to 3 additional doses of dimerizer drug may be administered at 48 hour intervals if acute GvHD persists. In subjects with Grade II or greater acute GvHD, these additional doses of dimerizer drug may be combined with steroids. For patients with persistent GVHD who cannot receive additional doses of the dimerizer due to a Grade III or IV reaction to the dimerizer, the patient may be treated with steroids alone, after either 0 or 1 doses of the dimerizer.

### Generation of Therapeutic T cells

Up to 240 ml (in 2 collections) of peripheral blood is obtained from the transplant donor according to the procurement consent. If necessary, a leukapheresis is used to obtain sufficient T cells; (either prior to stem cell mobilization or seven days after the last dose of G-CSF). An extra 10-30 mls of blood may also be collected to test for infectious diseases such as hepatitis and HIV.

Peripheral blood mononuclear cells are be activated using anti-human CD3 antibody (e.g. from Orthotech or Miltenyi) on day 0 and expanded in the presence of recombinant human interleukin-2 (rhIL-2) on day 2. CD3 antibody-activated T cells are transduced by the iCaspase-9 retroviral vector on flasks or plates coated with recombinant Fibronectin fragment CH-296 (RetronectinTM, Takara Shuzo, Otsu, Japan). Virus is attached to retronectin by incubating producer supernatant in retronectin coated plates or flasks. Cells are then transferred to virus coated tissue culture devices. After transduction T cells are expanded by feeding them with rhlL-2 twice a week to reach the sufficient number of cells as per protocol.
To ensure that the majority of infused T cells carry the suicide gene, a selectable marker, truncated human CD19 (ΔCD19) and a commercial selection device, may be used to select the transduced cells to >90% purity. Immunomagnetic selection for CD19 may be performed 4 days after transduction. Cells are labeled with paramagnetic microbeads conjugated to monoclonal mouse anti-human CD19 antibodies (Miltenyi Biotech, Auburn, CA) and selected on a CliniMacs Plus automated selection device. Depending upon the number of cells required for clinical infusion cells might either be cryopreserved after the CliniMacs selection or further expanded with IL-2 and cryopreserved as soon as sufficient cells have expanded (up to day 14 from product initiation).

Aliquots of cells may be removed for testing of transduction efficiency, identity, phenotype, autonomous growth and microbiological examination as required for final release testing by the FDA. The cells are be cryopreserved prior to administration.

### Administration of T cells

The transduced T cells are administered to patients from, for example, between 30 and 120 days following stem cell transplantation. The cryopreserved T cells are thawed and infused through a catheter line with normal saline. For children, premedications are dosed by weight. Doses of cells may range from, for example, from about 1 x 10⁴ cells/kg to 1 x 10⁸ cells/kg, for example from about 1 x 10⁵ cells/kg to 1 x 10⁷ cells/kg, from about 1 x 10⁶ cells/kg to 5 x 10⁶ cells/kg, from about 1 x 10⁴ cells/kg to 5 x 10⁶ cells/kg, for example, about 1 x 10⁴, about 1 x 10⁵, about 2 x 10⁵, about 3 x 10⁵, about 5 x 10⁵, 6 x 10⁵, about 7 x 10⁵, about 8 x 10⁵, about 9 x 10⁵, about 1 x 10⁶, about 2 x 10⁶, about 3 x 10⁶, about 4 x 10⁶, or about 5 x 10⁶ cells/kg.

### Treatment of GvHD

Patients who develop grade ≥1 acute GVHD are treated with 0.4mg/kg AP1903 as a 2-hour infusion. AP1903 for injection may be provided, for example, as a concentrated solution of 2.33 ml in a 3 ml vial, at a concentration of 5 mg/ml, (i.e 11.66 mg per vial). AP1903 may also provided in different sized vials, for example, 8 ml at 5mg/ml may be provided. Prior to administration, the calculated dose will be diluted to 100 mL in 0.9% normal saline for infusion. AP1903 for Injection (0.4 mg/kg) in a volume of 100 ml may be administered via IV infusion over 2 hours, using a non-DEHP, non-ethylene oxide sterilized infusion set and an infusion pump.

**Table 4: Sample treatment schedule**

| **Time** | **Donor** | **Recipient** |
|---|---|---|
| Pre-transplant | Obtain up to 240 of blood or unstimulated leukapheresis from bone marrow transplant donor. Prepare T cells and donor LCLs for later immune reconstitution studies. | |
| Day 0 | Anti-CD3 activation of PBMC | |
| Day 2 | IL-2 feed | |
| Day 3 | Transduction | |
| Day 4 | Expansion | |
| Day 6 | CD19 selection. Cryopreservation (*if required dose is met) | |
| Day 8 | Assess transduction efficiency and iCaspase9 transgene functionality by phenotype. Cryopreservation (*if not yet performed) | |
| Day 10 or Day 12 to Day 14 | Cryopreservation (if not yet performed) | |
| From 30 to 120 days post transplant | | Thaw and infuse T cells 30 to 120 days post stem cell infusion. |

Other methods may be followed for clinical therapy and assessment as provided in, for example, Examples 1-3 herein.

### Example 5: Using the iCasp9 Suicide Gene to Improve the Safety of Mesenchymal Stromal Cell Therapies

Mesenchymal stromal cells (MSCs) have been infused into hundreds of patients to date with minimal reported deleterious side effects. The long term side effects are not known due to limited follow-up and a relatively short time since MSCs have been used in treatment of disease. Several animal models have indicated that there exists the potential for side effects, and therefore a system allowing control over the growth and survival of MSCs used therapeutically is desirable. The inducible Caspase-9 suicide switch expression vector construct presented herein was investigated as a method of eliminating MSC's in vivo and in vitro.

### Materials and Methods

### MSC isolation

MSCs were isolated from healthy donors. Briefly, post-infusion discarded healthy donor bone marrow collection bags and filters were washed with RPMI 1640 (HyClone, Logan, UT) and plated on tissue culture flasks in DMEM (Invitrogen, Carlsbad, CA) with 10% fetal bovine serum (FBS), 2 mM alanyl-glutamine (Glutamax, Invitrogen), 100 units/mL penicillin and 100 µg/mL streptomycin (Invitrogen). After 48 hours, the supernatant was discarded and the cells were cultured in complete culture medium (CCM): α-MEM (Invitrogen) with 16.5% FBS, 2 mM alanyl-glutamine, 100 units/mL penicillin and 100 µg/mL streptomycin. Cells were grown to less then 80% confluence and replated at lower densities as appropriate.

### Immunophenotyping

Phycoerythrin (PE), fluorescein isothiocyanate (FITC), peridinin chlorophyll protein (PerCP) or allophycocyanin (APC)-conjugated CD14, CD34, CD45, CD73, CD90, CD105 and CD133 monoclonal antibodies were used to stain MSCs. All antibodies were from Becton Dickinson-Pharmingen (San Diego, CA), except where indicated. Control samples labeled with an appropriate isotype-matched antibody were included in each experiment. Cells were analyzed by fluorescence-activated cell sorting FACScan (Becton Dickinson) equipped with a filter set for 4 fluorescence signals.

### Differentiation studies in vitro

Adipocytic differentiation. MSCs (7.5×10⁴ cells) were plated in wells of 6-well plates in NH AdipoDiff Medium (Miltenyi Biotech, Auburn, CA). Medium was changed every third day for 21 days. Cells were stained with Oil Red O solution (obtained by diluting 0.5% w/v Oil Red O in isopropanol with water at a 3:2 ratio), after fixation with 4% formaldehyde in phosphate buffered saline (PBS).

Osteogenic differentiation. MSCs (4.5×10⁴ cells) were plated in 6-well plates in NH OsteoDiff Medium (Miltenyi Biotech). Medium was changed every third day for 10 days. Cells were stained for alkaline phosphatase activity using Sigma Fast BCIP/NBT substrate (Sigma-Aldrich, St. Louis, MO) as per manufacturer instructions, after fixation with cold methanol.

Chondroblastic differentiation. MSC pellets containing 2.5×10⁵ to 5×10⁵ cells were obtained by centrifugation in 15 mL or 1.5 mL polypropylene conical tubes and cultured in NH ChondroDiff Medium (Miltenyi Biotech). Medium was changed every third day for a total of 24 days. Cell pellets were fixed in 4% formalin in PBS and processed for routine paraffin sectioning. Sections were stained with alcian blue or using indirect immunofluorescence for type II collagen (mouse anti-collagen type II monoclonal antibody MAB8887, Millipore, Billerica, MA) after antigen retrieval with pepsin (Thermo Scientific, Fremont, CA).

### iCasp9-ΔCD19 retrovirus production and transduction of MSCs

The SFG.iCasp9.2A.ΔCD19 (iCasp-ΔCD19) retrovirus consists of iCasp9 linked, via a cleavable 2A-like sequence, to truncated human CD19 (ΔCD19). As noted above, iCasp9 is a human FK506-binding protein (FKBP12) with an F36V mutation, which increases the binding affinity of the protein to a synthetic homodimerizer (AP20187 or AP1903), connected via a Ser-Gly-Gly-Gly-Ser-Gly linker to human Caspase-9, whose recruitment domain (CARD) has been deleted, its function replaced by FKBP12.

The 2A-like sequence encodes a 20 amino acid peptide from Thosea Asigna insect virus, which mediates more than 99% cleavage between a glycine and terminal proline residue, to ensure separation of iCasp9 and ΔCD19 upon translation. ΔCD19 consists of human CD19 truncated at amino acid 333, which removes all conserved intracytoplasmic tyrosine residues that are potential sites for phosphorylation. A stable PG13 clone producing Gibbon ape leukemia virus (Gal-V) pseudotyped retrovirus was made by transiently transfecting Phoenix Eco cell line (ATCC product #SD3444; ATCC, Manassas, VA) with SFG.iCasp9.2A.ΔCD19, which yielded Eco-pseudotyped retrovirus. The PG13 packaging cell line (ATCC) was transduced 3 times with Eco-pseudotyped retrovirus to generate a producer line that contained multiple SFG.iCasp9.2A.ΔCD19 proviral integrants per cell. Single-cell cloning was performed, and the PG13 clone that produced the highest titer was expanded and used for vector production. Retroviral supernatant was obtained via culture of the producer cell lines in IMDM (Invitrogen) with 10% FBS, 2 mM alanyl-glutamine, 100 units/mL penicillin and 100 µg/mL streptomycin. Supernatant containing the retrovirus was collected 48 and 72 hours after initial culture. For transduction, approximately 2×10⁴ MSCs/cm² were plated in CM in 6-well plates, T75 or T175 flasks. After 24 hours, medium was replaced by viral supernatant diluted 10-fold together with polybrene (final concentration 5 µg/mL) and the cells were incubated at 37°C in 5% CO₂ for 48 hours, after which cells were maintained in complete medium.

### Cell enrichment

For inducible iCasp9-ΔCD19-positive MSC selection for in vitro experiments, retrovirally transduced MSC were enriched for CD19-positive cells using magnetic beads (Miltenyi Biotec) conjugated with anti-CD19 (clone 4G7), per manufacturer instructions. Cell samples were stained with PE- or APC- conjugated CD19 (clone SJ25C1) antibody to assess the purity of the cellular fractions.

### Apoptosis studies in vitro

Undifferentiated MSCs. The chemical inducer of dimerization (CID) (AP20187; ARIAD Pharmaceuticals, Cambridge, MA) was added at 50 nM to iCasp9-transduced MSCs cultures in complete medium. Apoptosis was evaluated 24 hours later by FACS analysis, after cell harvest and staining with annexin V-PE and 7-AAD in annexin V binding buffer (BD Biosciences, San Diego, CA). Control iCasp9-transduced MSCs were maintained in culture without exposure to CID.

Differentiated MSCs. Transduced MSCs were differentiated as presented above. At the end of the differentiation period, CID was added to the differentiation media at 50 nM. Cells were stained appropriately for the tissue being studied, as presented above, and a contrast stain (methylene azur or methylene blue) was used to evaluate the nuclear and cytoplasmic morphology. In parallel, tissues were processed for terminal deoxynucleotidyl-transferase dUTP nick end labeling (TUNEL) assay as per manufacturer instructions (In Situ Cell Death Detection Kit, Roche Diagnostics, Mannheim, Germany). For each time point, four random fields were photographed at a final magnification of 40x and the images were analyzed with ImageJ software version 1.43o (NIH, Bethesda, MD). Cell density was calculated as the number of nuclei (DAPI positivity) per unit of surface area (in mm²). The percentage of apoptotic cells was determined as the ratio of the number of nuclei with positive TUNEL signal (FITC positivity) to the total number of nuclei. Controls were maintained in culture without CID.

### In vivo killing studies in murine model

All mouse experiments were performed in accordance with the Baylor College of Medicine animal husbandry guidelines. To assess the persistence of modified MSCs in vivo, a SCID mouse model was used in conjunction with an in vivo imaging system. MSCs were transduced with retroviruses coding for the enhanced green fluorescent protein-firefly luciferase (eGFP-FFLuc) gene alone or together with the iCasp9-ΔCD19 gene. Cells were sorted for eGFP positivity by fluorescence activated cell sorting using a MoFlo flow cytometer (Beckman Coulter, Fullerton, CA). Doubly transduced cells were also stained with PE-conjugated anti-CD19 and sorted for PE-positivity. SCID mice (8-10 weeks old) were injected subcutaneously with 5×10⁵ MSCs with and without iCasp9-ΔCD19 in opposite flanks. Mice received two intraperitoneal injections of 50 µg of CID 24 hours apart starting a week later. For in vivo imaging of MSCs expressing eGFP-FFLuc, mice were injected intraperitoneally with D-luciferin (150 mg/kg) and analyzed using the Xenogen-IVIS Imaging System.

Total luminescence (a measurement proportional to the total labeled MSCs deposited) at each time point was calculated by automatically defining regions-of-interest (ROIs) over the MSC implantation sites. These ROIs included all areas with luminescence signals at least 5% above background. Total photon counts were integrated for each ROI and an average value calculated. Results were normalized so that time zero would correspond to 100% signal.

In a second set of experiments, a mixture of 2.5×10⁶ eGFP-FFLuc-labeled MSCs and 2.5×10⁶ eGFP-FFLuc-labeled, iCasp9-ΔCD19-transduced MSCs was injected subcutaneously in the right flank, and the mice received two intraperitoneal injections of 50 µg of CID 24 h apart starting 7 days later. At several time points after CID injection, the subcutaneous pellet of MSCs was harvested using tissue luminescence to identify and collect the whole human specimen and to minimize mouse tissue contamination. Genomic DNA was then isolated using QIAmp® DNA Mini (Qiagen, Valencia, CA). Aliquots of 100 ng of DNA were used in a quantitative PCR (qPCR) to determine the number of copies of each transgene using specific primers and probes (for the eGFP-FFLuc construct: forward primer 5'-TCCGCCCTGAGCAAAGAC-3', reverse 5'-ACGAACTCCAGCAGGACCAT-3', probe 5' FAM, 6-carboxyfluorescein-ACGAGAAGCGCGATC-3' MGBNFQ, minor groove binding non-fluorescent quencher; iCasp9-ΔCD19: forward 5'-CTGGAATCTGGCGGTGGAT-3', reverse 5'-CAAACTCTCAAGAGCACCGACAT-3', probe 5' FAM-CGGAGTCGACGGATT-3' MGBNFQ). Known numbers of plasmids containing single copies of each transgene were used to establish standard curves. It was determined that approximately 100 ng of DNA isolated from "pure" populations of singly eGFP-FFLuc- or doubly eGFP-FFLuc- and iCasp9-transduced MSCs had similar numbers of eGFP-FFLuc gene copies (approximately 3.0×10⁴), as well as zero and 1.7×10³ of iCasp9-ΔCD19 gene copies, respectively.

Untransduced human cells and mouse tissues had zero copies of either gene in 100 ng of genomic DNA. Because the copy number of the eGFP gene is the same on identical amounts of DNA isolated from either population of MSCs (iCasp9-negative or positive), the copy number of this gene in DNA isolated from any mixture of cells will be proportional to the total number of eGFP-FFLuc-positive cells (iCasp9-positive plus negative MSCs). Moreover, because iCasp9-negative tissues do not contribute to the iCasp9 copy number, the copy number of the iCasp9 gene in any DNA sample will be proportional to the total number of iCasp9-positive cells. Therefore, if G is the total number of GFP-positive and iCasp9-negative cells and C the total number of GFP-positive and iCasp9-positive cells, for any DNA sample then N_{eGFP} = g·(C+G) and N_{iCasp9} = k·C, where N represents gene copy number and g and k are constants relating copy number and cell number for the eGFP and iCasp9 genes, respectively. Thus N_{iCasp9}/N_{eGFP} = (k/g)·[C/(C+G)], i.e., the ratio between iCasp9 copy number and eGFP copy number is proportional to the fraction of doubly transduced (iCasp9-positive) cells among all eGFP positive cells. Although the absolute values of N_{iCasp9} and N_{eGFP} will decrease with increasing contamination by murine cells in each MSC explant, for each time point the ratio will be constant regardless of the amount of murine tissue included, since both types of human cells are physically mixed. Assuming similar rates of spontaneous apoptosis in both populations (as documented by in vitro culture) the quotient between N_{iCasp9}/N_{eGFP} at any time point and that at time zero will represent the percentage of surviving iCasp9-positive cells after exposure to CID. All copy number determinations were done in triplicate.

### Statistical Analysis

Paired 2-tailed Student's t-test was used to determine the statistical significance of differences between samples. All numerical data are represented as mean ± 1 standard deviation.

### Results

### MSCs are readily transduced with iCasp9-ΔCD19 and maintain their basic phenotype

Flow cytometric analysis of MSCs from 3 healthy donors showed they were uniformly positive for CD73, CD90 and CD105 and negative for the hematopoietic markers CD45, CD14, CD133 and CD34. The mononuclear adherent fraction isolated from bone marrow was homogenously positive for CD73, CD90 and CD105 and negative for hematopoietic markers. The differentiation potential, of isolated MSCs, into adipocytes, osteoblasts and chondroblasts was confirmed in specific assays, demonstrating that these cells are *bona fide* MSCs.
Early passage MSCs were transduced with an iCasp9-ΔCD19 retroviral vector, encoding an inducible form of Caspase-9. Under optimal single transduction conditions, 47 ± 6% of the cells expressed CD19, a truncated form of which is transcribed in cis with iCasp9, serving as a surrogate for successful transduction and allowing selection of transduced cells. The percentage of cells positive for CD19 was stable for more than two weeks in culture, suggesting no deleterious or growth advantageous effects of the construct on MSCs. The percentage of CD19-positive cells, a surrogate for successful transduction with iCasp9, remains constant for more than 2 weeks. To further address the stability of the construct, a population of iCasp9-positive cells purified by a fluorescence activated cell sorter (FACS) was maintained in culture: no significant difference in the percentage of CD19-positive cells was observed over six weeks (96.5 ± 1.1% at baseline versus 97.4 ± 0.8% after 43 days, P = 0.46). The phenotype of the iCasp9-CD19-positive cells was otherwise substantially identical to that of untransduced cells, with virtually all cells positive for CD73, CD90 and CD105 and negative for hematopoietic markers, confirming that the genetic manipulation of MSCs did not modify their basic characteristics.

### iCasp9-ΔCD19 transduced MSCs undergo selective apoptosis after exposure to CID in vitro

The proapoptotic gene product iCasp9 can activated by a small chemical inducer of dimerization (CID), AP20187, an analogue of tacrolimus that binds the FK506-binding domain present in the iCasp9 product. Non-transduced MSCs have a spontaneous rate of apoptosis in culture of approximately 18% (± 7%) as do iCasp9-positive cells at baseline (15 ± 6%, P = 0.47). Addition of CID (50 nM) to MSC cultures after transduction with iCasp9-ΔCD19 results in the apoptotic death of more than 90% of iCasp9-positive cells within 24 hrs (93 ± 1%, P < 0.0001), while iCasp9-negative cells retain an apoptosis index similar to that of non-transduced controls (20 ± 7%, P = 0.99 and P = 0.69 vs. non-transduced controls with or without CID respectively) (see FIGS. 17A and 70B). After transduction of MSCs with iCasp9, the chemical inducer of dimerization (CID) was added at 50 nM to cultures in complete medium. Apoptosis was evaluated 24 hours later by FACS analysis, after cell harvest and staining with annexin V-PE and 7-AAD. Ninety-three percent of the iCasp9-CD19-positive cells (iCasp pos/CID) became annexin positive versus only 19% of the negative population (iCasp neg/CID), a proportion comparable to non-transduced control MSC exposed to the same compound (Control/CID, 15%) and to iCasp9-CD19-positive cells unexposed to CID (iCasp pos/no CID, 13%), and similar to the baseline apoptotic rate of non-transduced MSCs (Control/no CID, 16%). Magnetic immunoselection of iCap9-CD19-positive cells can be achieved to high degree of purity. More than 95% of the selected cells become apoptotic after exposure to CID.

Analysis of a highly purified iCasp9-positive population at later time points after a single exposure to CID shows that the small fraction of iCasp9-negative cells expands and that a population of iCasp9-positive cells remains, but that the latter can be killed by re-exposure to CID. Thus, no iCasp9-positive population resistant to further killing by CID was detected. A population of iCasp9-CD19-negative MSCs emerges as early as 24 hours after CID introduction. A population of iCasp9-CD19-negative MSCs is expected since achieving a population with 100% purity is unrealistic and because the MSCs are being cultured in conditions that favor their rapid expansion in vitro. A fraction of iCasp9-CD19-positive population persists, as predicted by the fact that killing is not 100% efficient (assuming, for example, 99% killing of a 99% pure population, the resulting population would have 49.7% iCasp9-positive and 50.3% iCasp9-negative cells). The surviving cells, however, can be killed at later time points by re-exposure to CID.

### iCasp9-ΔCD19 transduced MSCs maintain the differentiation potential of unmodified MSCs and their progeny is killed by exposure to CID

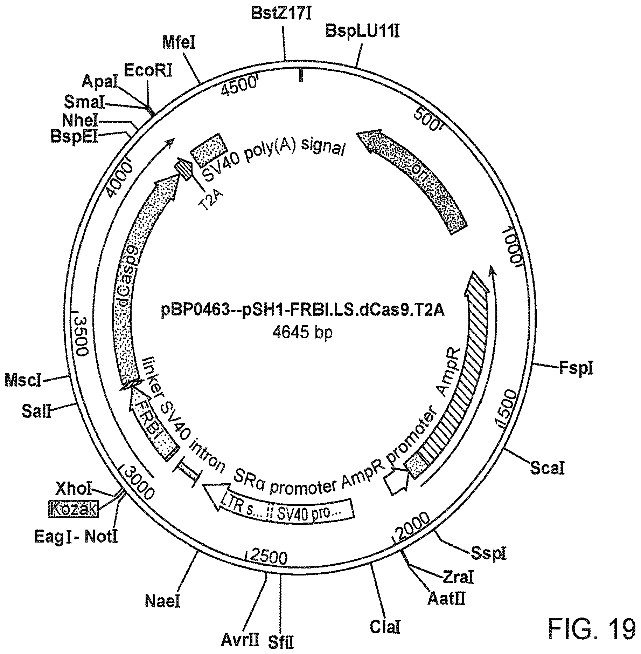

To determine if the CID can selectively kill the differentiated progeny of iCasp9-positive MSCs, immunomagnetic selection for CD19 was used to increase the purity of the modified population (>90% after one round of selection. The iCasp9-positive cells thus selected were able to differentiate in vivo into all connective tissue lineages studied (see FIGS. 19A-19Q). Human MSCs were immunomagnetically selected for CD19 (thus iCasp9) expression, with a purity greater than 91%. After culture in specific differentiation media, iCasp9-positive cells were able to give rise to adipocytic (A, oil red and methylene azur), osteoblastic (B, alkaline phosphatase-BCIP/NBT and methylene blue) and chondroblastic lineages (C, alcian blue and nuclear red) lineages. These differentiated tissues are driven to apoptosis by exposure to 50 nM CID (D-N). Note numerous apoptotic bodies (arrows), cytoplasmic membrane blebbing (inset) and loss of cellular architecture (D and E); widespread TUNEL positivity in chondrocytic nodules (F-H), and adipogenic (I-K) and osteogenic (L-N) cultures, in contrast to that seen in untreated iCasp9-transduced controls (adipogenic condition shown, O-Q) (F, I, L, O, DAPI; G, J, M, P,TUNEL-FITC; H, K, N, Q, overlay).
After 24 hours of exposure to 50 nM of CID, microscopic evidence of apoptosis was observed with membrane blebbing, cell shrinkage and detachment, and presence of apoptotic bodies throughout the adipogenic and osteogenic cultures. A TUNEL assay showed widespread positivity in adipogenic and osteogenic cultures and the chondrocytic nodules (see FIGS. 19A-19Q), which increased over time. After culture in adipocytic differentiation media, iCasp9-positive cells gave rise to adipocytes. After exposure to 50 nM CID, progressive apoptosis was observed as evidenced by an increasing proportion of TUNEL-positive cells. After 24 hours, there was a significant decrease in cell density (from 584 cells/mm2 to <14 cells/mm2), with almost all apoptotic cells having detached from the slides, precluding further reliable calculation of the proportion of apoptotic cells. Thus, iCasp9 remained functional even after MSC differentiation, and its activation results in the death of the differentiated progeny.

### iCasp9-ΔCD19 transduced MSCs undergo selective apoptosis after in vivo exposure to CID

Although intravenously injected MSC already appear to have a short in vivo survival time, cells injected locally may survive longer and produce correspondingly more profound adverse effects. To assess the in vivo functionality of the iCasp9 suicide system in such a setting, SCID mice were subcutaneously injected with MSCs. MSCs were doubly transduced with the eGFP-FFLuc (previously presented) and iCasp9-ΔCD19 genes. MSCs were also singly transduced with eGFP-FFLuc. The eGFP-positive (and CD19-positive, where applicable) fractions were isolated by fluorescence activated cell sorting, with a purity > 95%. Each animal was injected subcutaneously with iCasp9-positive and control MSCs (both eGFP-FFLuc-positive) in opposite flanks. Localization of the MSCs was evaluated using the Xenogen-IVIS Imaging System. In another set of experiments, a 1:1 mixture of singly and doubly transduced MSCs was injected subcutaneously in the right flank and the mice received CID as above. The subcutaneous pellet of MSCs was harvested at different time points, genomic DNA was isolated and qPCR was used to determine copy numbers of the eGFP-FFLuc and iCasp9-ΔCD19 genes. Under these conditions, the ratio of the iCasp9 to eGFP gene copy numbers is proportional to the fraction of iCasp9-positive cells among total human cells (see Methods above for details). The ratios were normalized so that time zero corresponds to 100% of iCasp9-positive cells. Serial examination of animals after subcutaneous inoculation of MSCs (prior to CID injection) shows evidence of spontaneous apoptosis in both cell populations (as demonstrated by a fall in the overall luminescence signal to ∼20% of the baseline). This has been previously observed after systemic and local delivery of MSCs in xenogeneic models.

The luminescence data showed a substantial loss of human MSCs over the first 96 h after local delivery of MSCs, even before administration of CID, with only approximately 20% cells surviving after one week. From that time point onward, however, there were significant differences between the survival of icasp9-positive MSCs with and without dimerizer drug. Seven days after MSC implantation, animals were given two injections of 50 µg of CID, 24 hours apart. MSCs transduced with iCasp9 were quickly killed by the drug, as demonstrated by the disappearance of their luminescence signal. Cells negative for iCasp9 were not affected by the drug. Animals not injected with the drug showed persistence of signal in both populations up to a month after MSC implantation. To further quantify cell killing, qPCR assays were developed to measure copy numbers of the eGFP-FFLuc and iCasp9-ΔCD19 genes. Mice were injected subcutaneously with a 1:1 mixture of doubly and singly transduced MSCs and administered CID as above, one week after MSC implantation. MSCs explants were collected at several time points, genomic DNA isolated from the samples and qPCR assays performed on substantially identical amounts of DNA. Under these conditions (see Methods), at any time point, the ratio of iCasp9-ΔCD19 to eGFP-FFLuc copy numbers is proportional to the fraction of viable iCasp9-positive cells. Progressive killing of iCasp9-positive cells was observed (>99%) so that the proportion of surviving iCasp9-positive cells was reduced to 0.7% of the original population after one week. Therefore, MSCs transduced with iCasp9 can be selectively killed in vivo after exposure to CID, but otherwise persist.

### Discussion

The feasibility of engineering human MSCs to express a safety mechanism using an inducible suicide protein is demonstrated herein. The date presented herein show that MSC can be readily transduced with the suicide gene iCasp9 coupled to the selectable surface maker CD19. Expression of the co-transduced genes is stable both in MSCs and their differentiated progeny, and does not evidently alter their phenotype or potential for differentiation. These transduced cells can be killed in vitro and in vivo when exposed to the appropriate small molecule chemical inducer of dimerization that binds to the iCasp9.

For a cell based therapy to be successful, transplanted cells must survive the period between their harvest and their ultimate in vivo clinical application. Additionally, a safe cell based therapy also should include the ability to control the unwanted growth and activity of successfully transplanted cells. Although MSCs have been administered to many patients without notable side effects, recent reports indicate additional protections, such as the safety switch presented herein, may offer additional methods of control over cell based therapies as the potential of transplanted MSC to be genetically and epigenetically modified to enhance their functionality, and to differentiate into lineages including bone and cartilage is further investigated and exploited. Subjects receiving MSCs that have been genetically modified to release biologically active proteins might particularly benefit from the added safety provided by a suicide gene.

The suicide system presented herein offers several potential advantages over other known suicide systems. Strategies involving nucleoside analogues, such as those combining Herpes Simplex Virus thymidine kinase (HSV-tk) with gancyclovir (GCV) and bacterial or yeast cytosine deaminase (CD) with 5-fluoro-cytosine (5-FC), are cell-cycle dependent and are unlikely to be effective in the post-mitotic tissues that may be formed during the application of MSCs to regenerative medicine. Moreover, even in proliferating tissues the mitotic fraction does not comprise all cells, and a significant portion of the graft may survive and remain dysfunctional. In some instance, the prodrugs required for suicide may themselves have therapeutic uses that are therefore excluded (e.g., GCV), or may be toxic (e.g., 5-FC), either as a result of their metabolism by non-target organs (e.g., many cytochrome P450 substrates), or due to diffusion to neighboring tissues after activation by target cells (e.g., CB1954, a substrate for bacterial nitroreductase).

In contrast, the small molecule chemical inducers of dimerization presented herein have shown no evidence of toxicities even at doses ten fold higher than those required to activate the iCasp9. Additionally, nonhuman enzymatic systems, such as HSV-tk and DC, carry a high risk of destructive immune responses against transduced cells. Both the iCasp9 suicide gene and the selection marker CD19, are of human origin, and thus should be less likely to induce unwanted immune responses. Although linkage of expression of the selectable marker to the suicide gene by a 2A-like cleavable peptide of nonhuman origin could pose problems, the 2A-like linker is 20 amino acids long, and is likely less immunogenic than a nonhuman protein. Finally, the effectiveness of suicide gene activation in iCasp9-positive cells compares favorably to killing of cells expressing other suicide systems, with 90% or more of iCasp9-modified T cells eliminated after a single dose of dimerizer, a level that is likely to be clinically efficacious.

The iCasp9 system presented herein also may avoid additional limitations seen with other cell based and/or suicide switch based therapies. Loss of expression due to silencing of the transduced construct is frequently observed after retroviral transduction of mammalian cells. The expression constructs presented herein showed no evidence of such an effect. No decrease in expression or induced death was evident, even after one month in culture.

Another potential problem sometimes observed in other cell based and/or suicide switch based therapies, is the development of resistance in cells that have upregulated anti-apoptotic genes. This effect has been observed in other suicide systems involving different elements of the programmed cell death pathways such as Fas. iCasp9 was chosen as the suicide gene for the expression constructs presented herein because it was less likely to have this limitation. Compared to other members of the apoptotic cascade, activation of Caspase-9 occurs late in the apoptotic pathway and therefore should bypass the effects of many if not all anti-apoptotic regulators, such as c-FLIP and bcl-2 family members.

A potential limitation specific to the system presented herein may be spontaneous dimerization of iCasp9, which in turn could cause unwanted cell death and poor persistence. This effect has been observed in certain other inducible systems that utilize Fas. The observation of low spontaneous death rate in transduced cells and long term persistence of transgenic cells in vivo indicate this possibility is not a significant consideration when using iCasp9 based expression constructs.

Integration events deriving from retroviral transduction of MSCs may potentially drive deleterious mutagenesis, especially when there are multiple insertions of the retroviral vector, causing unwanted copy number effects and/or other undesirable effects. These unwanted effects could offset the benefit of a retrovirally transduced suicide system. These effects often can be minimized using clinical grade retroviral supernatant obtained from stable producer cell lines and similar culture conditions to transduce T lymphocytes. The T cells transduced and evaluated herein contain in the range of about 1 to 3 integrants (the supernatant containing in the range of about 1×10⁶ viral particles/mL). The substitution of lentiviral for retroviral vectors could further reduce the risk of genotoxicity, especially in cells with high self-renewal and differentiation potential.

While a small proportion of iCasp9-positive MSCs persists after a single exposure to CID, these surviving cells can subsequently be killed following re-exposure to CID. In vivo, there is >99% depletion with two doses, but it is likely that repeated doses of CID will be needed for maximal depletion in the clinical setting. Additional nonlimiting methods of providing extra safety when using an inducible suicide switch system include additional rounds of cell sorting to further increase the purity of the cell populations administered and the use of more than one suicide gene system to enhance the efficiency of killing.

The CD19 molecule, which is physiologically expressed by B lymphocytes, was chosen as the selectable marker for transduced cells, because of its potential advantages over other available selection systems, such as neomycin phosphotransferase (neo) and truncated low affinity nerve growth factor receptor (ΔLNGFR). "neo" encodes a potentially immunogenic foreign protein and requires a 7-day culture in selection medium, increasing the complexity of the system and potentially damaging the selected cells. ΔLNGFR expression should allow for isolation strategies similar to other surface markers, but these are not widely available for clinical use and a lingering concern remains about the oncogenic potential of ΔLNGFR. In contrast, magnetic selection of iCasp9-positive cells by CD19 expression using a clinical grade device is readily available and has shown no notable effects on subsequent cell growth or differentiation.

The procedure used for preparation and administration of mesenchymal stromal cells comprising the Caspase-9 safety switch may also be used for the preparation of embryonic stem cells and inducible pluripotent stem cells. Thus for the procedures outlined in the present example, either embryonic stem cells or inducible pluripotent stem cells may be substituted for the mesenchymal stromal cells provided in the example. In these cells, retroviral and lentiviral vectors may be used, with, for example, CMV promoters, or the ronin promoter.

### Example 6: Modified Caspase-9 Polypeptides with Lower Basal Activity and Minimal Loss of Ligand IC₅₀

Basal signaling, signaling in the absence of agonist or activating agent, is prevalent in a multitude of biomolecules. For example, it has been observed in more than 60 wild-type G protein coupled receptors (GPCRs) from multiple subfamilies [1], kinases, such as ERK and abl [2], surface immunoglobulins [3], and proteases. Basal signaling has been hypothesized to contribute to a vast variety of biological events, from maintenance of embryonic stem cell pluripotency, B cell development and differentiation [4-6], T cell differentiation [2, 7], thymocyte development [8], endocytosis and drug tolerance [9], autoimmunity [10], to plant growth and development [11]. While its biological significance is not always fully understood or apparent, defective basal signaling can lead to serious consequences. Defective basal Gₛ protein signaling has led to diseases, such as retinitis pigmentosa, color blindness, nephrogenic diabetes insipidus, familial ACTH resistance, and familial hypocalciuric hypercalcemia [12, 13].

Even though homo-dimerization of wild-type initiator Caspase-9 is energetically unfavorable, making them mostly monomers in solution [14-16], the low-level inherent basal activity of unprocessed Caspase-9 [15, 17] is enhanced in the presence of the Apaf-1-based "apoptosome", its natural allosteric regulator [6]. Moreover, supra-physiological expression levels and/or co-localization could lead to proximity-driven dimerization, further enhancing basal activation.
In the chimeric unmodified Caspase-9 polypeptide, innate Caspase-9 basal activity was significantly diminished by removal of the CAspase-Recruitment pro-Domain (CARD) [18], replacing it with the cognate high affinity AP1903-binding domain, FKBP12-F36V. Its usefulness as a pro-apoptotic "safety switch" for cell therapy has been well demonstrated in multiple studies [18-20]. While its high specific and low basal activity has made it a powerful tool in cell therapy, in contrast to G protein coupled receptors, there are currently no "inverse agonists" [21] to eliminate basal signaling, which may be desirable for manufacturing, and in some applications. Preparation of Master Cell Banks has proven challenging due to high amplification of the low-level basal activity of the chimeric polypeptide. In addition, some cells are more sensitive than others to low-level basal activity of Caspase-9, leading to unintended apoptosis of transduced cells [18].

To modify the basal activity of the chimeric Caspase-9 polypeptide, "rational design"-based methods were used to engineer 75i Casp9 mutants based on residues known to play crucial roles in homo-dimerization, XIAP-mediated inhibition, or phosphorylation (Table below) rather than "directed evolution" [22] that use multiple cycles of screening as selective pressure on randomly generated mutants. Dimerization-driven activation of Caspase-9 has been considered a dominant model of initiator Caspase activation [15, 23, 24]. To reduce spontaneous dimerization, site-directed mutagenesis was conducted of residues crucial for homo-dimerization and thus basal Caspase-9 signaling. Replacement of five key residues in the β6 strand (G402-C-F-N-F406), the key dimerization interface of Caspase-9, with those of constitutively dimeric effector Caspase-3 (C264-I-V-S-M268) converted it to a constitutively dimeric protein unresponsive to Apaf-1 activation without significant structural rearrangements [25]. To modify spontaneous homo-dimerization, systemic mutagenesis of the five residues was made, based on amino acid chemistry, and on corresponding residues of initiator Caspases-2, -8, -9, and -10 that exist predominately as a monomer in solution [14, 15]. After making and testing twenty-eight iCasp9 mutants by a secreted alkaline phosphatase (SEAP)-based surrogate killing assay (Table, below), the N405Q mutation was found to lower basal signaling with a moderate (< 10-fold) cost of higher IC₅₀ to AP1903.
Since proteolysis, typically required for Caspase activation, is not absolutely required for Caspase-9 activation [26], the thermodynamic "hurdle" was increased to inhibit auto-proteolysis. In addition, since XIAP-mediated Caspase-9 binding traps Caspase-9 in a monomeric state to attenuate its catalytic and basal activity [14], there was an effort to strengthen the interaction between XIAP and Caspase-9 by mutagenizing the tetrapeptide critical for interaction with XIAP (A316-T-P-F319, D330-A-I-S-S334). From 17 of these iCasp-9 mutants, it was determined that the D330A mutation lowered basal signaling with a minimum (< 5-fold) AP1903 IC₅₀ cost.

The third approach was based on previously reported findings that Caspase-9 is inhibited by kinases upon phosphorylation of S144 by PKC-ζ [27], S183 by protein kinase A [28], S196 by Akt1 [29], and activated upon phosphorylation of Y153 by c-abl [30]. These "brakes" might improve the IC₅₀, or substitutions with phosphorylation mimic ("phosphomimetic") residues could augment these "brakes" to lower basal activity. However, none of the 15 single residue mutants based on these residues successfully lowered the IC₅₀ to AP1903.

Methods such as those discussed, for example, in Examples 1-5, and throughout the present application may be applied, with appropriate modifications, if necessary to the chimeric modified Caspase-9 polypeptides, as well as to various therapeutic cells.

### Example 7: Materials and Methods

### PCR site-directed mutagenesis of Caspase-9:

To modify basal signaling of Caspase-9, PCR-based site directed mutagenesis [31] was done with mutation-containing oligos and Kapa (Kapa Biosystems, Woburn, MA). After 18 cycles of amplification, parental plasmid was removed with methylation-dependent Dpnl restriction enzyme that leaves the PCR products intact. 2 µl of resulting reaction was used to chemically transform XL1-blue or DH5α. Positive mutants were subsequently identified via sequencing (SeqWright, Houston, TX).

### Cell line maintenance and transfection:

Early passage HEK293T/16 cells (ATCC, Manassas, VA) were maintained in IMDM, GlutaMAX™ (Life Technologies, Carlsbad, CA) supplemented with 10% FBS, 100 U/mL penicillin, and 100 U/mL streptomycin until transfection in a humidified, 37°C, 5% CO₂/95% air atmosphere. Cells in logarithmic-phase growth were transiently transfected with 800 ng to 2 µg of expression plasmid encoding iCasp9 mutants and 500 ng of an expression plasmid encoding SRα promoter driven SEAP per million cells in 15-mL conical tubes. Catalytically inactive Caspase-9 (C285A) (without the FKBP domain) or "empty" expression plasmid ("pSH1-null") were used to keep the total plasmid levels constant between transfections. GeneJammer® Transfection Reagent at a ratio of 3 µl per ug of plasmid DNA was used to transiently transfect HEK293T/16 cells in the absence of antibiotics. 100 µl or 2 mL of the transfection mixture was added to each well in 96-well or 6-well plate, respectively. For SEAP assays, log dilutions of AP1903 were added after a minimum 3-hour incubation post-transfection. For western blots, cells were incubated for 20 minutes with AP1903 (10 nM) before harvesting.

### Secreted alkaline phosphatase (SEAP) assay:

Twenty-four to forty-eight hours after AP1903 treatment, ∼100 µl of supernatants were harvested into a 96-well plate and assayed for SEAP activity as discussed [19, 32]. Briefly, after 65°C heat denaturation for 45 minutes to reduce background caused by endogenous (and serum-derived) alkaline phosphatases that are sensitive to heat, 5 µl of supernatants was added to 95 µl of PBS and added to 100 µl of substrate buffer, containing 1 µl of 100 mM 4-methylumbelliferyl phosphate (4-MUP; Sigma, St. Louis, MO) re-suspended in 2 M diethanolamine. Hydrolysis of 4-MUP by SEAP produces a fluorescent substrate with excitation/emission (355/460 nm), which can be easily measured. Assays were performed in black opaque 96-well plates to minimize fluorescence leakage between wells. To examine both basal signaling and AP1903 induced activity, 106 early-passage HEK293T/16 cells were co-transfected with various amount of wild type Caspase and 500 ng of an expression plasmid that uses an SRα promoter to drive SEAP, a marker for cell viability. Following manufacturer's suggestions, 1 mL of IMDM+10% FBS without antibiotics was added to each mixture. 1000-ul of the mixture was seeded onto each well of a 96-well plate. 100-ul of AP1903 was added at least three hours post-transfection. After addition of AP1903 for at least 24 hours, 100-ul of supernatant was transferred to a 96-well plate and heat denatured at 68□C for 30 minutes to inactivate endogenous alkaline phosphatases. For the assay, 4-methylumbelliferyl phosphate substrate was hydrolyzed by SEAP to 4-methylumbelliferon, a metabolite that can be excited with 364 nm and detected with an emission filter of 448 nm. Since SEAP is used as a marker for cell viability, reduced SEAP reading corresponds with increased iCaspase-9 activities. Thus, a higher SEAP reading in the absence of AP1903 would indicate lower basal activity. Desired caspase mutants would have diminished basal signaling with increased sensitivity (i.e., lower IC₅₀) to AP1903. The goal of the study is to reduce basal signaling without significantly impairing IC₅₀.

### Western blot analysis:

HEK293T/16 cells transiently transfected with 2 µg of plasmid for 48-72 hours were treated with AP1903 for 7.5 to 20 minutes (as indicated) at 37°C and subsequently lysed in 500 µl of RIPA buffer (0.01 M Tris·HCl, pH 8.0/140 mM NaCl/1% Triton X-100/1 mM phenylmethylsulfonyl fluoride/1% sodium deoxycholate/0.1% SDS) with Halt™ Protease Inhibitor Cocktail. The lysates were collected and lysed on ice for 30 min. After pelleting cell debris, protein concentrations from overlying supernatants were measured in 96-well plates with BCA™ Protein Assay as recommended by the manufacturer. 30 µg of proteins were boiled in Laemmli sample buffer (Bio-Rad, Hercules, CA) with 2.5% 2-mercaptoethanol for 5 min at 95°C before being separated by Criterion TGX 10% Tris/glylcine protein gel. Membranes were probed with 1/1000 rabbit anti-human Caspase-9 polyclonal antibody followed by 1/10,000 HRP-conjugated goat anti-rabbit IgG F(ab')2 secondary antibody (Bio-Rad). Protein bands were detected using Supersignal West Femto chemiluminescent substrate. To ensure equivalent sample loading, blots were stripped at 65°C for 1 hour with Restore PLUS Western Blot Stripping Buffer before labeling with 1/10,000 rabbit anti-actin polyclonal antibody. Unless otherwise stated, all the reagents were purchased from Thermo Scientific.

Methods and constructs discussed in Examples 1-5, and throughout the present specification may also be used to assay and use the modified Caspase-9 polypeptides.

### Example 8: Evaluation and Activity of Chimeric Modified Caspase-9 Polypeptides

### Comparison of basal activity and AP1903 induced activity:

To examine both basal activity and AP1903 induced activity of the chimeric modified Caspase-9 polypeptides, SEAP activities of HEK293T/16 cells co-transfected with SEAP and different amounts of iCasp9 mutants were examined. iCasp9 D330A, N405Q, and D330A-N405Q showed significantly less basal activity than unmodified iCasp9 for cells transfected with either 1 µg iCasp9 per million cells (relative SEAP activity Units of 148928, 179081, 205772 vs. 114518) or 2 µg iCasp9 per million cells (136863, 175529, 174366 vs. 98889). The basal signaling of all three chimeric modified Caspase-9 polypeptides when transfected at 2 µg per million cells was significantly higher (p value < 0.05). iCasp9 D330A, N405Q, and D330A-N405Q also showed increased estimated IC₅₀s for AP1903, but they are all still less than 6 pM (based on the SEAP assay), compared to 1 pM for WT, making them potentially useful apoptosis switches.

### Evaluation of protein expression levels and proteolysis:

To exclude the possibility that the observed reduction in basal activity of the chimeric modified Caspase-9 polypeptides was attributable to decreased protein stability or variation in transfection efficiency, and to examine auto-proteolysis of iCasp9, the protein expression levels of Caspase-9 variants in transfected HEK293T/16 cells was assayed. Protein levels of chimeric unmodified Caspase-9 polypeptide, iCasp9 D330A, and iCasp9 D330A-N405Q all showed similar protein levels under the transfection conditions used in this study. In contrast, the iCasp9 N405Q band appeared darker than the others, particularly when 2 µg of expression plasmids was used. Auto-proteolysis was not easily detectable at the transfection conditions used, likely because only viable cells were collected. Anti-actin protein reblotting confirmed that comparable lysate amounts were loaded into each lane. These results support the observed lower basal signaling in the iCasp9 D330A, N405Q, and D330A-N405Q mutants, observed by SEAP assays.

### Discussion:

Based on the SEAP screening assay, these three chimeric modified Caspase-9 polypeptides showed higher AP1903-independent SEAP activity, compared to iCasp9 WT transfectants, and hence lower basal signaling. However, the double mutation (D330-N405Q) failed to further decrease either basal activity or IC₅₀ (0.05 nM) vs. the single amino acid mutants. The differences observed did not appear to be due to protein instability or differential amount of plasmids used during transfection.

### Example 9: Evaluation and Activity of Chimeric Modified Caspase-9 Polypeptides

Inducible Caspase-9 provides for rapid, cell-cycle-independent, cell autonomous killing in an AP1903-dependent fashion. Improving the characteristics of this inducible Caspase-9 polypeptide would allow for even broader applicability. It is desirable to decrease the protein's ligand-independent cytotoxicity, and increase its killing at low levels of expression. Although ligand-independent cytotoxicity is not a concern at relatively low levels of expression, it can have a material impact where levels of expression can reach one or more orders of magnitude higher than in primary target cells, such as during vector production. Also, cells can be differentially sensitive to low levels of caspase expression due to the level of apoptosis inhibitors, like XIAP and Bcl-2, which cells express. Therefore, to re-engineer the caspase polypeptide to have a lower basal activity and possibly higher sensitivity to AP1903 ligand, four mutagenesis strategies were devised.

Dimerization Domain: Although Caspase-9 is a monomer in solution at physiological levels, at high levels of expression, such as occurs in the pro-apoptotic, Apaf-driven "apoptosome", Caspase-9 can dimerize, leading to auto-proteolysis at D315 and a large increase in catalytic activity. Since C285 is part of the active site, mutation C285A is catalytically inactive and is used as a negative control construct. Dimerization involves very close interaction of five residues in particular, namely G402, C403, F404, N405, and F406. For each residue, a variety of amino acid substitutions, representing different classes of amino acids (e.g., hydrophobic, polar, etc.) were constructed. Interestingly, all mutants at G402 (i.e., G402A, G402I, G402Q, G402Y) and C403P led to a catalytically inactive caspase polypeptide. Additional C403 mutations (i.e., C403A, C403S, and C403T) were similar to the wild type caspase and were not pursued further. Mutations at F404 all lowered basal activity, but also reflected reduced sensitivity to IC₅₀, from ∼ 1 log to unmeasurable. In order of efficacy, they are: F404Y > F404T, F404W >> F404A, F404S. Mutations at N405 either had no effect, as with N405A, increased basal activity, as in N405T, or lowered basal activity concomitant with either a small (∼ 5-fold) or larger deleterious effect on IC₅₀, as with N405Q and N405F, respectively. Finally, like F404, mutations at F406 all lowered basal activity, and reflected reduced sensitivity to IC₅₀, from ∼ 1 log to unmeasurable. In order of efficacy, they are: F406A F406W, F406Y > F406T » F406L.

Some polypeptides were constructed and tested that had compound mutations within the dimerization domain, but substituting the analogous 5 residues from other caspases, known to be monomers (e.g., Caspase-2,-8, -10) or dimers (e.g., Caspase-3) in solution. Caspase-9 polypeptides, containing the 5-residue change from Caspase-2, -3, and -8, along with an AAAAA alanine substitution were all catalytically inactive, while the equivalent residues from Caspase-10 (ISAQT), led to reduced basal activity but higher IC₅₀.

Overall, based on the combination of consistently lower basal activity, combined with only a mild effect on IC₅₀, N405Q was selected for further experiments. To improve on efficacy, a codon-optimized version of the modified Caspase-9 polypeptide, having the N405Q substitution, called N405Qco, was tested. This polypeptide appeared marginally more sensitive to AP1903 than the wild type N405Q-substituted Caspase-9 polypeptide.

Cleavage site mutants: Following aggregation of Caspase-9 within the apoptosome or via AP1903-enforced homodimerization, auto-proteolysis at D315 occurs. This creates a new amino-terminus at A316, at least transiently. Interestingly, the newly revealed tetra-peptide, ³¹⁶ATPF³¹⁹, binds to the Caspase-9 inhibitor, XIAP, which competes for dimerization with Caspase-9 itself at the dimerization motif, GCFNF, discussed above. Therefore, the initial outcome of D315 cleavage is XIAP binding, attenuating further Caspase-9 activation. However, a second caspase cleavage site exists at D330, which is the target of downstream effector caspase, caspase-3. As the pro-apoptotic pressure builds, D330 becomes increasingly cleaved, releasing the XIAP-binding small peptide within residue 316 to 330, and hence, removing this mitigating Caspase-9 inhibitor. A D330A mutant was constructed, which lowered basal activity, but not as low as in N405Q. By SEAP assay at high copy number, it also revealed a slight increase in IC₅₀, but at low copy number in primary T cells, there was actually a slight increase in IC₅₀ with improved killing of target cells. Mutation at auto-proteolysis site, D315, also reduced basal activity, but this led to a large increase in IC₅₀, likely as D330 cleavage was then necessary for caspase activation. A double mutation at D315A and D330A, led to an inactive "locked" Caspase-9 that could not be processed properly.

Other D330 mutants were created, including D330E, D330G, D330N, D330S, and D330V. Mutation at D327 also prevented cleavage at D330, as the consensus Caspase-3 cleavage site is DxxD, but several D327 mutations (i.e., D327G, D327K, and D327R) along with F326K, Q328K, Q328R, L329K, L329G, and A331K, unlike D330 mutations, did not lower basal activity and were not pursued further.

XIAP-binding mutants: As discussed above, autoproteolysis at D315 reveals an XIAP-binding tetrapeptide, ³¹⁶ATPF³¹⁹, which "lures" XIAP into the Caspase-9 complex. Substitution of ATPF with the analogous XIAP-binding tetrapeptide, AVPI, from mitochondria-derived anti-XIAP inhibitor, SMAC/DIABLO, might bind more tightly to XIAP and lower basal activity. However, this 4-residue substitution had no effect. Other substitutions within the ATPF motif ranged from no effect, (i.e., T317C, P318A, F319A) to lower basal activity with either a very mild (i.e., T317S, mild (i.e., T317A) to large (i.e., A316G, F319W) increase in IC₅₀. Overall, the effects of changing the XIAP-binding tetrapeptide were mild; nonetheless, T317S was selected for testing in double mutations (discussed below), since the effects on IC₅₀ were the most mild of the group.

Phosphorylation mutants: A small number of Caspase-9 residues were reported to be the targets of either inhibitory (e.g., S144, S183, S195, S196, S307, T317) or activating (i.e., Y153) phosphorylations. Therefore, mutations that either mimic the phosphorylation ("phosphomimetics") by substitution with an acidic residue (e.g., Asp) or eliminate phosphorylation were tested. In general, most mutations, regardless of whether a phosphomimetic or not was tried, lowered basal activity. Among the mutants with lower basal activity, mutations at S144 (i.e., S144A and S144D) and S1496D had no discernable effect on IC₅₀, mutants S183A, S195A, and S196A increased the IC₅₀ mildly, and mutants Y153A, Y153A, and S307A had a big deleterious effect on IC₅₀. Due to the combination of lower basal activity and minimal, if any effect on IC₅₀, S144A was chosen for double mutations (discussed below).

Double mutants: In order to combine the slightly improved efficacy of D330A variant with possible residues that could further lower basal activity, numerous D330A double mutants were constructed and tested. Typically, they maintained lower basal activity with only a slight increase in IC₅₀, including 2nd mutations at N405Q, S144A, S144D, S183A, and S196A. Double mutant D330A-N405T had higher basal activity and double mutants at D330A with Y153A, Y153F, and T317E were catalytically inactive. A series of double mutants with low basal activity N405Q, intended to improve efficacy or decrease the IC₅₀ was tested. These all appeared similar to N405Q in terms of low basal activity and slightly increased IC₅₀ relative to CaspaCIDe-1.0, and included N405Q with S144A, S144D, S196D, and T317S.

SEAP assays were conducted to study the basal activity and CID sensitivity of some of the dimerization domain mutants. N405Q was the most AP1903-sensitive of the mutants tested with lower basal activity than the WT Caspase-9, as determined by a shift upwards of AP1903-independent signaling. F406T was the least CID-sensitive from this group..
The dimer-independent SEAP activity of mutant caspase polypeptides D330A and N405Q was assayed, along with double mutant D330A-N405Q. The results of multiple transfections (N = 7 to 13) found that N405Q has lower basal activity than D330A and the double mutant is intermediate.

Obtaining the average (+ stdev, n =5) IC₅₀ of mutant caspase polypeptides D330A and N405Q, along with double mutant D330A-N405Qshows that D330A is somewhat more sensitive to AP1903 than N405Q mutants but about 2-fold less sensitive than WT Caspase-9 in a transient transfection assay.

SEAP assays were conducted using wild type (WT) Caspase-9, N405Q, inactive C285A, and several T317 mutants within the XIAP-binding domain. The results show that T317S and T317A can reduce basal activity without a large shift in the IC₅₀ to APf1903. Therefore, T317S was chosen to make double mutants with N405Q.
IC₅₀s from the SEAP assays above showed that T317A and T317S have similar IC₅₀s to wild type Caspase-9 polypeptide despite having lower basal activity.

The dimer-independent SEAP activity from several D330 mutants showed that all members of this class tested, including D330A, D330E, D330N, D330V, D330G, and D330S, have less basal activity than wild type Caspase-9. Basal and AP1903-induced activation of D330A variants was assayed. SEAP assay of transiently transfected HEK293/16 cells with 1 or 2 ug of mutant caspase polypeptides and 0.5 ug of pSH1-kSEAP per million HEK293 cells, 72 hours post-transfection. Normalized data based on 2 ug of each expression plasmid (including WT) were mixed with normalized data from 1 ug-based transfections. iCasp9-D330A, -D330E, and - D330S showed statistically lower basal signaling than wildtype Caspase-9.

The result of a western blot shoed that the D330 mutations block cleavage at D330, leading to a slightly largely (slower migrating) small band (< 20 kDa marker). Other blots show that D327 mutation also blocks cleavage.

The mean fluorescence intensities of multiple clones of PG13 transduced 5X with retroviruses encoding the indicated Caspase-9 polypeptides was measured. Lower basal activity typically translates to higher levels of expression of the Caspase-9 gene along with the genetically linked reporter, CD19. The results show that on the average, clones expressing the N405Q mutant express higher levels of CD19, reflecting the lower basal activity of N405Q over D330 mutants or WT Caspase-9. The effects of various caspase mutations on viral titers derived from PG13 packaging cells cross-transduced with VSV-G envelope-based retroviral supernatants was assayed. To examine the effect of CaspaCIDe-derived basal signaling on retrovirus master cell line production, retrovirus packaging cell line, PG13, was cross-transduced five times with VSV-G-based retroviral supernatants in the presence of 4 µg/ml transfection-enhancer, polybrene. CaspaCIDe-transduced PG13 cells were subsequently stained with PE-conjugated anti-human CD19 antibody, as an indication of transduction. CaspaCIDe-D330A, -D330E, and -N405Q-transduced PG13 cells showed enhanced CD19 mean fluorescence intensity (MFI), indicating higher retroviral copy numbers, implying lower basal activity. To more directly examine the viral titer of the PG13 transductants, HT1080 cells were treated with viral supernatant and 8 ug/ml polybrene. The enhanced CD19 MFIs of iCasp9-D330A, -N405Q, and -D330E transductants *vs* WT iCasp9 in PG13 cells are positively correlated with higher viral titers, as observed in HT1080 cells. Due to the initially low viral titers (approximately 1E5 transduction units (TU)/ml), no differences in viral titers were observed in the absence of HAT treatment to increase virus yields. Upon HAT media treatment, PG13 cells transduced with CaspaCIDe-D330A, - N405Q, or -D330E demonstrated higher viral titers. Viral titer (transducing units) is calculated with the formula: Viral titer = (# cells on the day of transduction) * (% CD19⁺)/Volume of supernatant (ml). In order to further investigate the effect of CaspaCIDe mutants with lower basal activity, individual clones (colonies) of CaspaCIDe-transduced PG13 cells were selected and expanded. CaspaCIDe-N405Q clones with higher CD19 MFIs than the other cohorts were observed.

The effects of various caspase polypeptides at mostly single copy in primary T cells was assayed. This may reflect more accurately how these suicide genes will be used therapeutically. Surprisingly, the data show that the D330A mutant is actually more sensitive to AP1903 at low titers and kills at least as well as WT Caspase-9 when tested in a 24-hour assay. The N405Q mutant is less sensitive to AP1903 and cannot kill target cells as efficiently within 24 hours.

Results of transducing 6 independent T cell samples from separate healthy donors showed that the D330A mutant (mut) is more sensitive to AP1903 than the wild type Caspase-9 polypeptide.

Figure 57 shows the average IC₅₀, range and standard deviation from the 6 healthy donors shown in Fig 56. This data shows that the improvement is statistically significant. The iCasp9-D330A mutant demonstrated improved AP1903-dependent cytotoxicity in transduced T cells. Primary T cells from healthy donors (n=6) were transduced with retrovirus encoding mutant or wild-type iCasp9 or iCasp9-D330A, and the ΔCD19 cell surface marker. Following transduction, iCasp9-transduced T cells were purified using CD19-microbeads and a magnetic column. T cells were then exposed to AP1903 (0-100 nM) and measured for CD3+CD19+ T cells by flow cytometry after 24 hours. The IC₅₀ of iCasp9-D330A was significantly lower (p=0.002) than wild-type iCasp9.

Results of several D330 mutants, revealed that all six D330 mutants tested (D330A, E, N, V, G, and S) are more sensitive to AP1903 than wild type Caspase-9 polypeptide.

The N405Q mutant along with other dimerization domain mutants, including N404Y and N406Y, can kill target T cells indistinguishable from wild type Caspase-9 polypeptide or D330A within 10 days. Cells that received AP1903 at Day 0 received a second dose of AP1903 at day 4. This data supports the use of reduced sensitivity Caspase-9 mutants, like N405Q as part of a regulated efficacy switch.
The results of codon optimization of N405Q caspase polypeptide, called "N405Qco", revealed that codon optimization, likely leading to an increase in expression only has a very subtle effect on inducible caspase function. This likely reflects the use of common codons in the original Caspase-9 gene.

The Caspase-9 polypeptide has a dose-response curve in vivo, which could be used to eliminate a variable fraction of T cells expressing the Caspase-9 polypeptide. The data also shows that a dose of 0.5 mg/kg AP1903 is sufficient to eliminate most modified T cells in vivo.
AP1903 dose-dependent elimination in vivo of T cells transduced with D330E iCasp9 was assayed. T cells were transduced with SFG-iCasp9-D330E-2A-ΔCD19 retrovirus and injected i.v. into immune deficient mice (NSG). After 24 hours, mice were injected i.p. with AP1903 (0-5 mg/kg). After an additional 24 hours, mice were sacrificed and lymphocytes from the spleen (A) were isolated and analyzed by flow cytometry for the frequency of human CD3+CD19+ T cells. This shows that iCasp9-D330E demonstrates a similar in vivo cytotoxicity profile in response to AP1903 as wild-type iCasp9.

Conclusions: As discussed, from this analysis of 78 mutants so far, out of the single mutant mutations, the D330 mutations combine somewhat improved efficacy with slightly reduced basal activity. N405Q mutants are also attractive since they have very low basal activity with only slightly decreased efficacy, reflected by a 4-5-fold increase in IC₅₀. Experiments in primary T cells have shown that N405Q mutants can effectively kill target cells, but with somewhat slower kinetics than D330 mutants, making this potentially very useful for a graduated suicide switch that kills partially after an initial dose of AP1903, and up to full killing can be achieved upon a second dose of AP1903.

The following table provides a summary of basal activity and IC₅₀ for various chimeric modified Caspase-9 polypeptides prepared and assayed according to the methods discussed herein. The results are based on a minimum of two independent SEAP assays, except for a subset (i.e., A316G, T317E, F326K, D327G, D327K, D327R, Q328K, Q328R, L329G, L329K, A331K, S196A, S196D, and the following double mutants: D330A with S144A, S144D, or S183A; and N405Q with S144A, S144D, S196D, or T317S) that were tested once. Four multi-pronged approaches were taken to generate the tested chimeric modified Caspase-9 polypeptides. "Dead" modified Caspase-9 polypeptides were no longer responsive to AP1903. Double mutants are indicated by a hyphen, for example, D330A-N405Q denotes a modified Caspase-9 polypeptide having a substitution at position 330 and a substitution at position 405.

**Table 5 Caspase Mutant Classes**

| **Basal Activity** | **Homodimerization domain** | **Cleavage sites & XIAP Interaction** | **Phosph orylatio n** | **Double mutants, Misc.** | **Total mutants** |
|---|---|---|---|---|---|
| **Decreased basal and similar IC₅₀** | | | S144A | | 80 |
| | | | S144D | | *, **predicted** |
| | | T317S | S196D | | |
| **Decreased basal but higher IC₅₀** | **N405Q** | **D330A** | S183A | D330A-N405Q | **Bold**, Tested in T cells |
| | ⁴⁰²GCFNF⁴⁰⁶ISAQT (Casp-10) | **D330E** | S195A | D330A-S144A | |
| | **F404Y** | **D330G** | S196A | D330A-S144D | |
| | F406A | **D330N** | | D330A-S183A | |
| | **F406W** | **D330S** | | D330A-S196A | |
| | **F406Y** | **D330V** | | N405Q-S144A | |
| | **N405Qco** | L329E | | N405Q-S144D | |
| | | T317A | | N405Q-S196D | |
| | | | | N405Q-T317S | |
| | | | | **N405Q-S144Aco* | |
| | | | | **N405Q-T317Sco* | |
| **Decreased basal but much higher IC₅₀** | F404T | D315A | Y153A | | |
| | F404W | A316G | Y153F | | |
| | N405F | F319W | S307A | | |
| | **F406T** | | | | |
| **Similar basal and IC₅₀** | C403A | ³¹⁶ATPF³¹⁹AVPI (SMAC/Diablo) | | | |
| | C403S | T317C | | | |
| | C403T | P318A | | | |
| | N405A | F319A | | | |
| **Increased basal** | N405T | T317E | | D330A-N405T | |
| | | F326K | | | |
| | | D327G | | | |
| | | D327K | | | |
| | | D327R | | | |
| | | Q328K | | | |
| | | Q328R | | | |
| | | L329G | | | |
| | | L329K | | | |
| | | A331K | | | |
| **Catalytically dead** | ⁴⁰²GCFNF⁴⁰⁶AAAAA | | | C285A | |
| | ⁴⁰²GCFNF⁴⁰⁶YCSTL (Casp-2) | | | D315A-D330A | |
| | ⁴⁰²GCFNF⁴⁰⁶CIVSM (Casp-3) | | | D330A-Y153A | |
| | ⁴⁰²GCFNF⁴⁰⁶QPTFT (Casp-8) | | | D330A-Y153F | |
| | G402A | | | D330A-T317E | |
| | G402I | | | | |
| | G402Q | | | | |
| | G402Y | | | | |
| | C403P | | | | |
| | F404A | | | | |
| | F404S | | | | |
| | F406L | | | | |

### Literature References Cited in Examples 6-9

1. Seifert, R. and K. Wenzel-Seifert, Constitutive activity of G-protein-coupled receptors: cause of disease and common property of wild-type receptors. Naunyn Schmiedebergs Arch Pharmacol, 2002. 366(5): p. 381-416.
2. Roose, J.P., et al., T cell receptor-independent basal signaling via Erk and Abl kinases suppresses RAG gene expression. PLoS Biol, 2003. 1(2): p. E53.
3. Tze, L.E., et al., Basal immunoglobulin signaling actively maintains developmental stage in immature B cells. PLoS Biol, 2005. 3(3): p. e82.
4. Schram, B.R., et al., B cell receptor basal signaling regulates antigen-induced Ig light chain rearrangements. J Immunol, 2008. 180(7): p. 4728-41.
5. Randall, K.L., et al., Dock8 mutations cripple B cell immunological synapses, germinal centers and long-lived antibody production. Nat Immunol, 2009. 10(12): p. 1283-91.
6. Kouskoff, V., et al., B cell receptor expression level determines the fate of developing B lymphocytes: receptor editing versus selection. Proc Natl Acad Sci U S A, 2000. 97(13): p. 7435-9.
7. Hong, T., et al., A simple theoretical framework for understanding heterogeneous differentiation of CD4+ T cells. BMC Syst Biol, 2012. 6: p. 66.
8. Rudd, M.L., A. Tua-Smith, and D.B. Straus, Lck SH3 domain function is required for T-cell receptor signals regulating thymocyte development. Mol Cell Biol, 2006. 26(21): p. 7892-900.
9. Sorkin, A. and M. von Zastrow, Endocytosis and signalling: intertwining molecular networks. Nat Rev Mol Cell Biol, 2009. 10(9): p. 609-22.
10. Luning Prak, E.T., M. Monestier, and R.A. Eisenberg, B cell receptor editing in tolerance and autoimmunity. Ann N Y Acad Sci, 2011. 1217: p. 96-121.
11. Boss, W.F., et al., Basal signaling regulates plant growth and development. Plant Physiol, 2010. 154(2): p. 439-43.
12. Tao, Y.X., Constitutive activation of G protein-coupled receptors and diseases: insights into mechanisms of activation and therapeutics. Pharmacol Ther, 2008. 120(2): p. 129-48.
13. Spiegel, A.M., Defects in G protein-coupled signal transduction in human disease. Annu Rev Physiol, 1996. 58: p. 143-70.
14. Shiozaki, E.N., et al., Mechanism of XIAP-mediated inhibition of Caspase-9. Mol Cell, 2003. 11(2): p. 519-27.
15. Renatus, M., et al., Dimer formation drives the activation of the cell death protease Caspase-9. Proc Natl Acad Sci U S A, 2001. 98(25): p. 14250-5.
16. Shi, Y., Mechanisms of Caspase activation and inhibition during apoptosis. Mol Cell, 2002. 9(3): p. 459-70.
17. Shiozaki, E.N., J. Chai, and Y. Shi, Oligomerization and activation of Caspase-9, induced by Apaf-1 CARD. Proc Natl Acad Sci USA, 2002. 99(7): p. 4197-202.
18. Straathof, K.C., et al., An inducible Caspase-9 safety switch for T-cell therapy. Blood, 2005. 105(11): p. 4247-54.
19. MacCorkle, R.A., K.W. Freeman, and D.M. Spencer, Synthetic activation of Caspases: artificial death switches. Proc Natl Acad Sci USA, 1998. 95(7): p. 3655-60.
20. Di Stasi, A., et al., Inducible apoptosis as a safety switch for adoptive cell therapy. N Engl J Med, 2011. 365(18): p. 1673-83.
21. Chang, W.C., et al., Modifying ligand-induced and constitutive signaling of the human 5-HT4 receptor. PLoS One, 2007. 2(12): p. e1317.
22. Bloom, J.D. and F.H. Arnold, In the light of directed evolution: pathways of adaptive protein evolution. Proc Natl Acad Sci U S A, 2009. 106 Suppl 1: p. 9995-10000.
23. Boatright, K.M. and G.S. Salvesen, Mechanisms of Caspase activation. Curr Opin Cell Biol, 2003. 15(6): p. 725-31.
24. Boatright, K.M., et al., A unified model for apical Caspase activation. Mol Cell, 2003. 11(2): p. 529-41.
25. Chao, Y., et al., Engineering a dimeric Caspase-9: a re-evaluation of the induced proximity model for Caspase activation. PLoS Biol, 2005. 3(6): p. e183.
26. Stennicke, H.R., et al., Caspase-9 can be activated without proteolytic processing. J Biol Chem, 1999. 274(13): p. 8359-62.
27. Brady, S.C., L.A. Allan, and P.R. Clarke, Regulation of Caspase-9 through phosphorylation by protein kinase C zeta in response to hyperosmotic stress. Mol Cell Biol, 2005. 25(23): p. 10543-55.
28. Martin, M.C., et al., Protein kinase A regulates Caspase-9 activation by Apaf-1 downstream of cytochrome c. J Biol Chem, 2005. 280(15): p. 15449-55.
29. Cardone, M.H., et al., Regulation of cell death protease Caspase-9 by phosphorylation. Science, 1998. 282(5392): p. 1318-21.
30. Raina, D., et al., c-Abl tyrosine kinase regulates Caspase-9 autocleavage in the apoptotic response to DNA damage. J Biol Chem, 2005. 280(12): p. 11147-51.
31. Papworth, C., Bauer, J. C., Braman, J. and Wright, D. A., Site-directed mutagenesis in one day with >80% efficiency. Strategies, 1996. 9(3): p. 3-4.
32. Spencer, D.M., et al., Functional analysis of Fas signaling in vivo using synthetic inducers of dimerization. Curr Biol, 1996. 6(7): p. 839-47.
33. Hsiao, E.C., et al., Constitutive Gs activation using a single-construct tetracycline-inducible expression system in embryonic stem cells and mice. Stem Cell Res Ther, 2011. 2(2): p. 11.
34. Waldner, C., et al., Double conditional human embryonic kidney cell line based on FLP and PhiC31 mediated transgene integration. BMC Res Notes, 2011. 4: p. 420.

The chimeric caspase polypeptides may include amino acid substitutions, including amino acid substitutions that result in a caspase polypeptide with lower basal activity. These may include, for example, iCasp9 D330A, iCasp9 N405Q, and iCasp9 D330A N405Q, demonstrated low to undetectable basal activity, respectively, with a minimum deleterious effect on their AP1903 IC₅₀ in a SEAP reporter-based, surrogate killing assay.

### Example 10: Examples of Particular Nucleic Acid and Amino Acid Sequences

The following is nucleotide sequences provide an example of a construct that may be used for expression of the chimeric protein and CD19 marker. The figure presents the SFG.iC9.2A.²CD19.gcs construct
SEQ ID NO: 1, nucleotide sequence of 5'LTR sequence 
TGAAAGACCCCACCTGTAGGTTTGGCAAGCTAGCTTAAGTAACGCCATTTTGCA
AGGCATGGAAAAATACATAACTGAGAATAGAAAAGTTCAGATCAAGGTCAGGAA
CAGATGGAACAGCTGAATATGGGCCAAACAGGATATCTGTGGTAAGCAGTTCCT
GCCCCGGCTCAGGGCCAAGAACAGATGGAACAGCTGAATATGGGCCAAACAGG
ATATCTGTGGTAAGCAGTTCCTGCCCCGGCTCAGGGCCAAGAACAGATGGTCC
CCAGATGCGGTCCAGCCCTCAGCAGTTTCTAGAGAACCATCAGATGTTTCCAGG
GTGCCCCAAGGACCTGAAATGACCCTGTGCCTTATTTGAACTAACCAATCAGTT
CGCTTCTCGCTTCTGTTCGCGCGCTTATGCTCCCCGAGCTCAATAAAAGAGCCC
ACAACCCCTCACTCGGGGCGCCAGTCCTCCGATTGACTGAGTCGCCCGGGTAC
CCGTGTATCCAATAAACCCTCTTGCAGTTGCATCCGACTTGTGGTCTCGCTGTT
CCTTGGGAGGGTCTCCTCTGAGTGATTGACTACCCGTCAGCGGGGGTCTTTCA
SEQ ID NO: 2, nucleotide sequence of Fᵥ (human FKBP12v36) 
GGAGTGCAGGTGGAAACCATCTCCCCAGGAGACGGGCGCACCTTCCCCAAGC
GCGGCCAGACCTGCGTGGTGCACTACACCGGGATGCTTGAAGATGGAAAGAAA
GTTGATTCCTCCCGGGACAGAAACAAGCCCTTTAAGTTTATGCTAGGCAAGCAG
GAGGTGATCCGAGGCTGGGAAGAAGGGGTTGCCCAGATGAGTGTGGGTCAGA
GAGCCAAACTGACTATATCTCCAGATTATGCCTATGGTGCCACTGGGCACCCAG
GCATCATCCCACCACATGCCACTCTCGTCTTCGATGTGGAGCTTCTAAAACTGG
AA
SEQ ID NO: 3 amino acid sequence of Fv (human FKBP12v36) 
G V Q V E T I S P G D G R T F P K R G Q T C V V H Y T G M L
E D G K K V D S S R D R N K P F K F M L G K Q E V I R G W E
E G V A Q M S V G Q R A K L T I S P D Y A Y G A T G H P G I I
P P H A T L V F D V E L L K L E
SEQ ID NO: 4, GS linker nucleotide sequence
   TCTGGCGGTGGATCCGGA
SEQ ID NO: 5, GS linker amino acid sequence
   S G G G S G
SEQ ID NO: 6, linker nucleotide sequence (between GS linker and Casp 9)
   GTCGAC
SEQ ID NO: 7, linker amino acid sequence (between GS linker and Casp 9)
   VD
SEQ ID NO: 8, Casp 9 (truncated) nucleotide sequence 
GGATTTGGTGATGTCGGTGCTCTTGAGAGTTTGAGGGAAATGCAGATTTGGCT
TACATCCTGAGCATGGAGCCCTGTGGCCACTGCCTCATTATCAACAATGTGAAC
TTCTGCCGTGAGTCCGGGCTCCGCACCCGCACTGGCTCCAACATCGACTGTGA
 
GAAGTTGCGGCGTCGCTTCTCCTCGCTGCATTTCATGGTGGAGGTGAAGGGCG
ACCTGACTGCCAAGAAAATGGTGCTGGCTTTGCTGGAGCTGGCGCAGCAGGAC
CACGGTGCTCTGGACTGCTGCGTGGTGGTCATTCTCTCACGGCTGTCAGGC
CAGCCACCTGCAGTTCCCAGGGGCTGTCTACGGCACAGATGGATGCCCTGTGT
CGGTCGAGAAGATTGTGAACATCTTCAATGGGACCAGCTGCCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
GACGCCATATCTAGTTTGCCCACACCCAGTGACATCTTTGTGTCCTACTCTACTT
TCCCAGGTTTTGTTTCCTGGAGGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTAATTTCCTCCGGAAAAACTTTTCTTTAAAACATCA
SEQ ID NO: 9, Caspase-9 (truncated) amino acid sequence-CARD domain deleted 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A Q Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L D A I S S L P T P S D I F V S Y S T F P G F
V S W R D P K S G S W Y V E T L D D I F E Q W A H S E D L Q
S L L L R V A N A V S V K G I Y K Q M P G C F N F L R K K L F
F K T S
SEQ ID NO: 10, linker nucleotide sequence (between Caspase-9 and 2A)
   GCTAGCAGA
SEQ ID NO: 11, linker amino acid sequence (between Caspase-9 and 2A)
   ASR
SEQ ID NO: 12, Thosea asigna virus-2A from capsid protein precursor nucleotide sequence 
GCCGAGGGCAGGGGAAGTCTTCTAACATGCGGGGACGTGGAGGAAAATCCCG
GGCCC
SEQ ID NO: 13, Thosea asigna virus-2A from capsid protein precursor amino acid sequence
   A E G R G S L L T C G D V E E N P G P
SEQ ID NO: 14, human CD19 (Δ cytoplasmic domain) nucleotide sequence (transmembrane domain in bold) 
ATGCCACCTCCTCGCCTCCTCTTCTTCCTCCTCTTCCTCACCCCCATGGAAGTC
AGGCCCGAGGAACCTCTAGTGGTGAAGGTGGAAGAGGGAGATAACGCTGTGCT
GCAGTGCCTCAAGGGGACCTCAGATGGCCCCACTCAGCAGCTGACCTGGTCTC
GGGAGTCCCCGCTTAAACCCTTCTTAAAACTCAGCCTGGGGCTGCCAGGCCTG
GGAATCCACATGAGGCCCCTGGCCATCTGGCTTTTCATCTTCAACGTCTCTCAA
CAGATGGGGGGCTTCTACCTGTGCCAGCCGGGGCCCCCCTCTGAGAAGGCCT
GGCAGCCTGGCTGGACAGTCAATGTGGAGGGCAGCGGGGAGCTGTTCCGGTG
GAATGTTTCGGACCTAGGTGGCCTGGGCTGTGGCCTGAAGAACAGGTCCTCAG
AGGGCCCCAGCTCCCCTTCCGGGAAGCTCATGAGCCCCAAGCTGTATGTGTGG
GCCAAAGACCGCCCTGAGATCTGGGAGGGAGAGCCTCCGTGTCTCCCACCGA
GGGACAGCCTGAACCAGAGCCTCAGCCAGGACCTCACCATGGCCCCTGGCTC
CACACTCTGGCTGTCCTGTGGGGTACCCCCTGACTCTGTGTCCAGGGGCCCCC
TCTCCTGGACCCATGTGCACCCCAAGGGGCCTAAGTCATTGCTGAGCCTAGAG
CTGAAGGACGATCGCCCGGCCAGAGATATGTGGGTAATGGAGACGGGTCTGTT
GTTGCCCCGGGCCACAGCTCAAGACGCTGGAAAGTATTATTGTCACCGTGGCA
ACCTGACCATGTCATTCCACCTGGAGATCACTGCTCGGCCAGTACTATGGCACT
GGCTGCTGAGGACTGGTGGCTGGAAGGTCTCAGCTGTGACTTTGGCTTATCTG
ATCTTCTGCCTGTGTTCCCTTGTGGGCATTCTTCATCTTCAAAGAGCCCTGGTC
CTGAGGAGGAAAAGAAAGCGAATGACTGACCCCACCAGGAGATTC
SEQ ID NO: 15, human CD19 (Δ cytoplasmic domain) amino acid sequence 
M P P P R L L F F L L F L T P M E V R P E E P L V V K V E G
D N A V L Q C L K G T S D G P T Q Q L T W S R E S P L K P F L
K L S L G L P G L G I H M R P L A I W L F I F N V S Q Q M G G
 
F Y L C Q P G P P S E K A W Q P G W T V N V E G S G E L F R
W N V S D L G G L G C G L K N R S S E G P S S P S G K L M S
P K L Y V W A K D R P E I W E G E P P C L P P R D S L N Q S L
S Q D L T M A P G S T L W L S C G V P P D S V S R G P L S W
T H V H P K G P K S L L S L E L K D D R P A R D M W V M E T
G L L L P R A T A Q D A G K Y Y C H R G N L T M S F H L E I T
A R P V L W H W L L R T G G W K V S A V T L A Y L I F C L C S
L V G I L H L Q R A L V L R R K R K R M T D P T R R F
SEQ ID NO: 16, 3'LTR nucleotide sequence 
TGAAAGACCCCACCTGTAGGTTTGGCAAGCTAGCTTAAGTAACGCCATTTTGCA
AGGCATGGAAAAATACATAACTGAGAATAGAGAAGTTCAGATCAAGGTCAGGAA
CAGATGGAACAGCTGAATATGGGCCAAACAGGATATCTGTGGTAAGCAGTTCCT
GCCCCGGCTCAGGGCCAAGAACAGATGGAACAGCTGAATATGGGCCAAACAGG
ATATCTGTGGTAAGCAGTTCCTGCCCCGGCTCAGGGCCAAGAACAGATGGTCC
CCAGATGCGGTCCAGCCCTCAGCAGTTTCTAGAGAACCATCAGATGTTTCCAGG
GTGCCCCAAGGACCTGAAATGACCCTGTGCCTTATTTGAACTAACCAATCAGTT
CGCTTCTCGCTTCTGTTCGCGCGCTTCTGCTCCCCGAGCTCAATAAAAGAGCCC
ACAACCCCTCACTCGGGGCGCCAGTCCTCCGATTGACTGAGTCGCCCGGGTAC
CCGTGTATCCAATAAACCCTCTTGCAGTTGCATCCGACTTGTGGTCTCGCTGTT
CCTTGGGAGGGTCTCCTCTGAGTGATTGACTACCCGTCAGCGGGGGTCTTTCA
SEQ ID NO: 17, Expression vector construct nucleotide sequence-nucleotide sequence coding for the chimeric protein and 5' and 3' LTR sequences, and additional vector sequence. 
TGAAAGACCCCACCTGTAGGTTTGGCAAGCTAGCTTAAGTAACGCCATTTTGCA
AGGCATGGAAAAATACATAACTGAGAATAGAAAAGTTCAGATCAAGGTCAGGAA
CAGATGGAACAGCTGAATATGGGCCAAACAGGATATCTGTGGTAAGCAGTTCCT
GCCCCGGCTCAGGGCCAAGAACAGATGGAACAGCTGAATATGGGCCAAACAGG
ATATCTGTGGTAAGCAGTTCCTGCCCCGGCTCAGGGCCAAGAACAGATGGTCC
CCAGATGCGGTCCAGCCCTCAGCAGTTTCTAGAGAACCATCAGATGTTTCCAGG
GTGCCCCAAGGACCTGAAATGACCCTGTGCCTTATTTGAACTAACCAATCAGTT
CGCTTCTCGCTTCTGTTCGCGCGCTTATGCTCCCCGAGCTCAATAAAAGAGCCC
ACAACCCCTCACTCGGGGCGCCAGTCCTCCGATTGACTGAGTCGCCCGGGTAC
CCGTGTATCCAATAAACCCTCTTGCAGTTGCATCCGACTTGTGGTCTCGCTGTT
 
CCTTGGGAGGGTCTCCTCTGAGTGATTGACTACCCGTCAGCGGGGGTCTTTCA
TTTGGGGGCTCGTCCGGGATCGGGAGACCCCTGCCCAGGGACCACCGACCCA
CCACCGGGAGGTAAGCTGGCCAGCAACTTATCTGTGTCTGTCCGATTGTCTAGT
GTCTATGACTGATTTTATGCGCCTGCGTCGGTACTAGTTAGCTAACTAGCTCTGT
ATCTGGCGGACCCGTGGTGGAACTGACGAGTTCGGAACACCCGGCCGCAACC
CTGGGAGACGTCCCAGGGACTTCGGGGGCCGTTTTTGTGGCCCGACCTGAGTC
CTAAAATCCCGATCGTTTAGGACTCTTTGGTGCACCCCCCTTAGAGGAGGGATA
TGTGGTTCTGGTAGGAGACGAGAACCTAAAACAGTTCCCGCCTCCGTCTGAATT
TTTGCTTTCGGTTTGGGACCGAAGCCGCGCCGCGCGTCTTGTCTGCTGCAGCA
TCGTTCTGTGTTGTCTCTGTCTGACTGTGTTTCTGTATTTGTCTGAAAATATGGG
CCCGGGCTAGCCTGTTACCACTCCCTTAAGTTTGACCTTAGGTCACTGGAAAGA
TGTCGAGCGGATCGCTCACAACCAGTCGGTAGATGTCAAGAAGAGACGTTGGG
TTACCTTCTGCTCTGCAGAATGGCCAACCTTTAACGTCGGATGGCCGCGAGACG
GCACCTTTAACCGAGACCTCATCACCCAGGTTAAGATCAAGGTCTTTTCACCTG
GCCCGCATGGACACCCAGACCAGGTGGGGTACATCGTGACCTGGGAAGCCTT
GGCTTTTGACCCCCCTCCCTGGGTCAAGCCCTTTGTACACCCTAAGCCTCCGCC
TCCTCTTCCTCCATCCGCCCCGTCTCTCCCCCTTGAACCTCCTCGTTCGACCCC
GCCTCGATCCTCCCTTTATCCAGCCCTCACTCCTTCTCTAGGCGCCCCCATATG
GCCATATGAGATCTTATATGGGGCACCCCCGCCCCTTGTAAACTTCCCTGACCC
TGACATGACAAGAGTTACTAACAGCCCCTCTCTCCAAGCTCACTTACAGGCTCT
CTACTTAGTCCAGCACGAAGTCTGGAGACCTCTGGCGGCAGCCTACCAAGAAC
AACTGGACCGACCGGTGGTACCTCACCCTTACCGAGTCGGCGACACAGTGTGG
GTCCGCCGACACCAGACTAAGAACCTAGAACCTCGCTGGAAAGGACCTTACAC
AGTCCTGCTGACCACCCCCACCGCCCTCAAAGTAGACGGCATCGCAGCTTGGA
TACACGCCGCCCACGTGAAGGCTGCCGACCCCGGGGGTGGACCATCCTCTAG
ACTGCCATGCTCGAGGGAGTGCAGGTGGAAACCATCTCCCCAGGAGACGGGC
GCACCTTCCCCAAGCGCGGCCAGACCTGCGTGGTGCACTACACCGGGATGCTT
GAAGATGGAAAGAAAGTTGATTCCTCCCGGGACAGAAACAAGCCCTTTAAGTTT
ATGCTAGGCAAGCAGGAGGTGATCCGAGGCTGGGAAGAAGGGGTTGCCCAGA
TGAGTGTGGGTCAGAGAGCCAAACTGACTATATCTCCAGATTATGCCTATGGTG
CCACTGGGCACCCAGGCATCATCCCACCACATGCCACTCTCGTCTTCGATGTG
GAGCTTCTAAAACTGGAATCTGGCGGTGGATCCGGAGTCGACGGATTTGGTGA
TGTCGGTGCTCTTGAGAGTTTGAGGGGAAATGCAGATTTGGCTTACATCCTGAG
CATGGAGCCCTGTGGCCACTGCCTCATTATCAACAATGTGAACTTCTGCCGTGA
GTCCGGGCTCCGCACCCGCACTGGCTCCAACATCGACTGTGAGAAGTTGCGGC
GTCGCTTCTCCTCGCTGCATTTCATGGTGGAGGTGAAGGGCGACCTGACTGCC
AAGAAAATGGTGCTGGCTTTGCTGGAGCTGGCGCAGCAGGACCACGGTGCTCT
 
GGACTGCTGCGTGGTGGTCATTCTCTCTCACGGCTGTCAGGCCAGCCACCTGC
AGTTCCAGGGGCTGTCTACGGCACAGATGGATGCCCTGTGTCGGTCGAGAAG
ATTGTGAACATCTTCAATGGACCAGCTGCCCCAGCCTGGGAGGGAAGCCCAA
GCTCTTTTTCATCCAGGCCTGTGGTGGGGAGCAGAAAGACCATGGGTTTGAGG
TGGCCTCCACTTCCCCTGAAGACGAGTCCCCTGGCAGTAACCCCGAGCCAGAT
GCCACCCCGTTCCAGGAAGGTTTGAGGACCTTCGACCAGCTGGACGCCATATC
TAGTTTGCCCACACCCAGTGACATCTTTGTGTCCTACTCTACTTTCCCAGGTTTT
GTTTCCTGGAGGGACCCCAAGAGTGGCTCCTGGTACGTTGAGACCCTGGACGA
CATCTTTGAGCAGTGGGCTCACTCTGAAGACCTGCAGTCCCTCCTGCTTAGGGT
CGCTAATGCTGTTTCGGTGAAAGGGATTTATAAACAGATGCCTGGTTGCTTTAAT
TTCCTCCGGAAAAAACTTTTCTTTAAAACATCAGCTAGCAGAGCCGAGGGCAGG
GGAAGTCTTCTAACATGCGGGGACGTGGAGGAAAATCCCGGCCCATGCCACC
TCCTCGCCTCCTCTTCTTCCTCCTCTTCCTCACCCCCATGGAAGTCAGGCCCGA
GGAACCTCTAGTGGTGAAGGTGGAAGAGGGAGATAACGCTGTGCTGCAGTGCC
TCAAGGGGACCTCAGATGGCCCCACTCAGCAGCTGACCTGGTCTCGGGAGTCC
CCGCTTAAACCCTTCTTAAAACTCAGCCTGGGGCTGCCAGGCCTGGGAATCCA
CATGAGGCCCCTGGCCATCTGGCTTTTCATCTTCAACGTCTCTCAACAGATGGG
GGGCTTCTACCTGTGCCAGCCGGGGCCCCCCTCTGAGAAGGCCTGGCAGCCT
GGCTGGACAGTCAATGTGGAGGGCAGCGGGGAGCTGTTCCGGTGGAATGTTTC
GGACCTAGGTGGCCTGGGCTGTGGCCTGAAGAACAGGTCCTCAGAGGGCCCC
AGCTCCCCTTCCGGGAAGCTCATGAGCCCCAAGCTGTATGTGTGGGCCAAAGA
CCGCCCTGAGATCTGGGAGGGAGAGCCTCCGTGTCTCCCACCGAGGGACAGC
CTGAACCAGAGCCTCAGCCAGGACCTCACCATGGCCCCTGGCTCCACACTCTG
GCTGTCCTGTGGGGTACCCCCTGACTCTGTGTCCAGGGGCCCCCTCTCCTGGA
CCCATGTGCACCCCAAGGGGCCTAAGTCATTGCTGAGCCTAGAGCTGAAGGAC
GATCGCCCGGCCAGAGATATGTGGGTAATGGAGACGGGTCTGTTGTTGCCCCG
GGCCACAGCTCAAGACGCTGGAAAGTATTATTGTCACCGTGGCAACCTGACCAT
GTCATTCCACCTGGAGATCACTGCTCGGCCAGTACTATGGCACTGGCTGCTGA
GGACTGGTGGCTGGAAGGTCTCAGCTGTGACTTTGGCTTATCTGATCTTCTGCC
TGTGTTCCCTTGTGGGCATTCTTCATCTTCAAAGAGCCCTGGTCCTGAGGAGGA
AAAGAAAGCGAATGACTGACCCCACCAGGAGATTCTAACGCGTCATCATCGATC
CGGATTAGTCCAATTTGTTAAAGACAGGATATCAGTGGTCCAGGCTCTAGTTTTG
ACTCAACAATATCACCAGCTGAAGCCTATAGAGTACGAGCCATAGATAAAATAAA
AGATTTTATTTAGTCTCCAGAAAAAGGGGGGAATGAAAGACCCCACCTGTAGGT
TTGGCAAGCTAGCTTAAGTAACGCCATTTTGCAAGGCATGGAAAAATACATAACT
GAGAATAGAGAAGTTCAGATCAAGGTCAGGAACAGATGGAACAGCTGAATATGG
GCCAAACAGGATATCTGTGGTAAGCAGTTCCTGCCCCGGCTCAGGGCCAAGAA
 
CAGATGGAACAGCTGAATATGGGCCAAACAGGATATCTGTGGTAAGCAGTTCCT
GCCCCGGCTCAGGGCCAAGAACAGATGGTCCCCAGATGCGGTCCAGCCCTCA
GCAGTTTCTAGAGAACCATCAGATGTTTCCAGGGTGCCCCAAGGACCTGAAATG
ACCCTGTGCCTTATTTGAACTAACCAATCAGTTCGCTTCTCGCTTCTGTTCGCGC
GCTTCTGCTCCCCGAGCTCAATAAAAGAGCCCACAACCCCTCACTCGGGGCGC
CAGTCCTCCGATTGACTGAGTCGCCCGGGTACCCGTGTATCCAATAAACCCTCT
TGCAGTTGCATCCGACTTGTGGTCTCGCTGTTCCTTGGGAGGGTCTCCTCTGAG
TGATTGACTACCCGTCAGCGGGGGTCTTTCACACATGCAGCATGTATCAAAATT
AATTTGGTTTTTTTTCTTAAGTATTTACATTAAATGGCCATAGTACTTAAAGTTACA
TTGGCTTCCTTGAAATAAACATGGAGTATTCAGAATGTGTCATAAATATTTCTAAT
TTTAAGATAGTATCTCCATTGGCTTTCTACTTTTTCTTTTATTTTTTTTTGTCCTCT
GTCTTCCATTTGTTGTTGTTGTTTGTTTGTTTGTTTGTTGGTTGGTTGGTTAA
TTTTTTTTTAAAGATCCTACACTATAGTTCAAGCTAGACTATTAGCTACTCTGTAA
CCCAGGGTGACCTTGAAGTCATGGGTAGCCTGCTGTTTTAGCCTTCCCACATCT
AAGATTACAGGTATGAGCTATCATTTTTGGTATATTGATTGATTGATTGATTGATG
TGTGTGTGTGTGATTGTGTTTGTGTGTGTGACTGTGAAAATGTGTGTATGGGTG
TGTGTGAATGTGTGTATGTATGTGTGTGTGTGAGTGTGTGTGTGTGTGTGTGCA
TGTGTGTGTGTGTGACTGTGTCTATGTGTATGACTGTGTGTGTGTGTGTGTGTG
TGTGTGTGTGTGTGTGTGTGTGTGTGTTGTGAAAAAATATTCTATGGTAGTGAGA
GCCAACGCTCCGGCTCAGGTGTCAGGTTGGTTTTTGAGACAGAGTCTTTCACTT
AGCTTGGAATTCACTGGCCGTCGTTTTACAACGTCGTGACTGGGAAAACCCTGG
CGTTACCCAACTTAATCGCCTTGCAGCACATCCCCCTTTCGCCAGCTGGCGTAA
TAGCGAAGAGGCCCGCACCGATCGCCCTTCCCAACAGTTGCGCAGCCTGAATG
GCGAATGGCGCCTGATGCGGTATTTTCTCCTTACGCATCTGTGCGGTATTTCAC
ACCGCATATGGTGCACTCTCAGTACAATCTGCTCTGATGCCGCATAGTTAAGCC
AGCCCCGACACCCGCCAACACCCGCTGACGCGCCCTGACGGGCTTGTCTGCT
CCCGGCATCCGCTTACAGACAAGCTGTGACCGTCTCCGGGAGCTGCATGTGTC
AGAGGTTTTCACCGTCATCACCGAAACGCGCGATGACGAAAGGGCCTCGTGAT
ACGCCTATTTTTATAGGTTAATGTCATGATAATAATGGTTTCTTAGACGTCAGGT
GGCACTTTTCGGGGAAATGTGCGCGGAACCCCTATTTGTTTATTTTTCTAAATAC
ATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTT
TTGCGGCATTTTGCCTTCCTGTTTTTGCTCACCCAGAAACGCTGGTGAAAGTAAA
AGATGCTGAAGATCAGTTGGGTGCACGAGTGGGTTACATCGAACTGGATCTCAA
CAGCGGTAAGATCCTTGAGAGTTTTCGCCCCGAAGAACGTTTTCCAATGATGAG
CACTTTTAAAGTTCTGCTATGTGGCGCGGTATTATCCCGTATTGACGCCGGGCA
AGAGCAACTCGGTCGCCGCATACACTATTCTCAGAATGACTTGGTTGAGTACTC
 
ACCAGTCACAGAAAAGCATCTTACGGATGGCATGACAGTAAGAGAATTATGCAG
TGCTGCCATAACCATGAGTGATAACACTGCGGCCAACTTACTTCTGACAACGAT
CGGAGGACCGAAGGAGCTAACCGCTTTTTTGCACAACATGGGGGATCATGTAA
CTCGCCTTGATCGTTGGGAACCGGAGCTGAATGAAGCCATACCAAACGACGAG
CGTGACACCACGATGCCTGTAGCAATGGCAACAACGTTGCGCAAACTATTAACT
GGCGAACTACTTACTCTAGCTTCCCGGCAACAATTAATAGACTGGATGGAGGCG
GATAAAGTTGCAGGACCACTTCTGCGCTCGGCCCTTCCGGCTGGCTGGTTTATT
GCTGATAAATCTGGAGCCGGTGAGCGTGGGTCTCGCGGTATCATTGCAGCACT
GGGGCCAGATGGTAAGCCCTCCCGTATCGTAGTTATCTACACGACGGGGAGTC
AGGCAACTATGGATGAACGAAATAGACAGATCGCTGAGATAGGTGCCTCACTGA
TTAAGCATTGGTAACTGTCAGACCAAGTTTACTCATATATACTTTAGATTGATTTA
AAACTTCATTTTTAATTTAAAAGGATCTAGGTGAAGATCCTTTTTGATAATCTCAT
GACCAAAATCCCTTAACGTGAGTTTTCGTTCCACTGAGCGTCAGACCCCGTAGA
AAAGATCAAAGGATCTTCTTGAGATCCTTTTTTTCTGCGCGTAATCTGCTGCTTG
CAAACAAAAAAACCACCGCTACCAGCGGTGGTTTGTTTGCCGGATCAAGAGCTA
CCAACTCTTTTTCCGAAGGTAACTGGCTTCAGCAGAGCGCAGATACCAAATACT
GTCCTTCTAGTGTAGCCGTAGTTAGGCCACCACTTCAAGAACTCTGTAGCACCG
CCTACATACCTCGCTCTGCTAATCCTGTTACCAGTGGCTGCTGCCAGTGGCGAT
AAGTCGTGTCTTACCGGGTTGGACTCAAGACGATAGTTACCGGATAAGGCGCA
GCGGTCGGGCTGAACGGGGGGTTCGTGCACACAGCCCAGCTTGGAGCGAACG
ACCTACACCGAACTGAGATACCTACAGCGTGAGCATTGAGAAAGCGCCACGCTT
CCCGAAGGGAGAAAGGCGGACAGGTATCCGGTAAGCGGCAGGGTCGGAACAG
GAGAGCGCACGAGGGAGCTTCCAGGGGGAAACGCCTGGTATCTTTATAGTCCT
GTCGGGTTTCGCCACCTCTGACTTGAGCGTCGATTTTTGTGATGCTCGTCAGGG
GGGCGGAGCCTATGGAAAAACGCCAGCAACGCGGCCTTTTTACGGTTCCTGGC
CTTTTGCTGGCCTTTTGCTCACATGTTCTTTCCTGCGTTATCCCCTGATTCTGTG
GATAACCGTATTACCGCCTTTGAGTGAGCTGATACCGCTCGCCGCAGCCGAAC
GACCGAGCGCAGCGAGTCAGTGAGCGAGGAAGCGGAAGAGCGCCCAATACGC
AAACCGCCTCTCCCCGCGCGTTGGCCGATTCATTAATGCAGCTGGCACGACAG
GTTTCCCGACTGGAAAGCGGGCAGTGAGCGCAACGCAATTAATGTGAGTTAGC
TCACTCATTAGGCACCCCAGGCTTTACACTTTATGCTTCCGGCTCGTATGTTGTG
TGGAATTGTGAGCGGATAACAATTTCACACAGGAAACAGCTATGACCATGATTA
CGCCAAGCTTTGCTCTTAGGAGTTTCCTAATACATCCCAAACTCAAATATATAAA
GCATTTGACTTGTTCTATGCCCTAGGGGGCGGGGGGAAGCTAAGCCAGCTTTTT
TTAACATTTAAAATGTTAATTCCATTTTAAATGCACAGATGTTTTTATTTCATAAGG
GTTTCAATGTGCATGAATGCTGCAATATTCCTGTTACCAAAGCTAGTATAAATAA
AAATAGATAAACGTGGAAATTACTTAGAGTTTCTGTCATTAACGTTTCCTTCCTCA
 
GTTGACAACATAAATGCGCTGCTGAGCAAGCCAGTTTGCATCTGTCAGGATCAA
TTTCCCATTATGCCAGTCATATTAATTACTAGTCAATTAGTTGATTTTTATTTTTGA
CATATACATGTGAA
SEQ ID NO: 18, (nucleotide sequence of F_{v'}Fᵥₗₛ with Xhol/Sall linkers, (wobbled codons lowercase in F_{v'})) 
ctcgagGGcGTcCAaGTcGAaACcATtagtCCcGGcGAtGGcaGaACaTTtCCtAAaaGgGG
aCAaACaTGtGTcGTcCAtTAtACaGGcATGtTgGAgGAcGGcAAaAAgGTgGAcagtagta
GaGAtcGcAAtAAaCCtTTcAAaTTcATGtTgGGaAAaCAaGAaGTcATtaGgGGaTGGG
AgGAgGGcGTgGCtCAaATGtccGTcGGcCAacGcGCtAAgCTcACcATcagcCCcGAcT
AcGCaTAcGGcGCtACcGGaCAtCCcGGaATtATtCCcCCtCAcGCtACctTgGTgTTtGAc
GTcGAaCTgtTgAAgCTcGAagtcgagggagtgcaggtggaaaccatctccccaggagacggcgcacct
tccccaagcgcggccagacctgcgtggtgcactacaccgggatgcttgaagatggaaagaaagttgattcctcccgg
gacagaaacaagccctttaagtttatgctaggcaagcaggaggtgatccgaggctgggaagaaggggttgcccag
atgagtgtgggtcagagagccaaactgactatatctccagattatgcctatggtgccactgggcacccaggcatcatcc
caccacatgccactctcgtcttcgatgtggagcttctaaaactggaatctggcggtggatccggagtcgag
SEQ ID NO: 19, (F_{V'}F_{VLS} amino acid sequence) 
GlyValGlnValGluThrIleSerProGlyAspGlyArgThrPheProLysArgGlyGlnThrCysValValHi
sTyrThrGlyMetLeuGluAspGlyLysLysValAspSerSerArgAspArgAsnLysProPheLysPhe
MetLeuGlyLysGlnGluValIleArgGlyTrpGluGluGlyValAlaGlnMetSerValGlyGlnArgAlaLy
sLeuThrIleSerProAspTyrAlaTyrGlyAlaThrGlyHisProGlyIleIleProProHisAlaThrLeuValP
heAspValGluLeuLeuLysLeuGlu (ValGlu)

GlyValGlnValGluThrIleSerProGlyAspGlyArgThrPheProLysArgGlyGlnThrCysValValHi
sTyrThrGlyMetLeuGluAspGlyLysLysValAspSerSerArgAspArgAsnLysProPheLysPhe
MetLeuGlyLysGlnGluValIleArgGlyTrpGluGluGlyValAlaGlnMetSerValGlyGlnArgAlaLy
sLeuThrIleSerProAspTyrAlaTyrGlyAlaThrGlyHisProGlyIleIleProProHisAlaThrLeuValP
heAspValGluLeuLeuLysLeuGlu-SerGlyGlyGlySerGly
SEQ ID NO: 20, FKBP12v36 (res. 2-108)
   SGGGSG Linker (6 aa)
   ΔCasp9 (res. 135-416) 
ATGCTCGAGGGAGTGCAGGTGGAgACtATCTCCCCAGGAGACGGGCGCACCTT
CCCCAAGCGCGGCCAGACCTGCGTGGTGCACTACACCGGGATGCTTGAAGATG
 
GAAAGAAAGTTGATTCCTCCCGGGACAGAAACAAGCCCTTTAAGTTTATGCTAG
GCAAGCAGGAGGTGATCCGAGGCTGGGAAGAAGGGGTTGCCCAGATGAGTGT
GGGTCAGAGAGCCAAACTGACTATATCTCCAGATTATGCCTATGGTGCCACTGG
GCACCCAGGCATCATCCCACCACATGCCACTCTCGTCTTCGATGTGGAGCTTCT
AAAACTGGAATCTGGCGGTGGATCCGGAGTCGACGGATTTGGTGATGTCGGTG
CTCTTGAGAGTTTGAGGGGAAATGCAGATTTGGCTTACATCCTGAGCATGGAGC
CCTGTGGCCACTGCCTCATTATCAACAATGTGAACTTCTGCCGTGAGTCCGGGC
TCCGCACCCGCACTGGCTCCAACATCGACTGTGAGAAGTTGCGGCGTCGCTTC
TCCTCGCTGCATTTCATGGTGGAGGTGAAGGGCGACCTGACTGCCAAGAAAT
GGTGCTGGCTTTGCTGGAGCTGGCGCgGCAGGACCACGGTGCTCTGGACTGCT
GCGTGGTGGTCATTCTCTCTCACGGCTGTCAGGCCAGCCACCTGCAGTTCCCA
GGGGCTGTCTACGGCACAGATGGATGCCCTGTGTCGGTCGAGAAGATTGTGAA
CATCTTCAATGGGACCAGCTGCCCCAGCCTGGGAGGGAAGCCCAAGCTCTTTT
TCATCCAGGCCTGTGGTGGGGAGCAGAAAGACCATGGGTTTGAGGTGGCCTCC
ACTTCCCCTGAAGACGAGTCCCCTGGCAGTAACCCCGAGCCAGATGCCACCCC
GTTCCAGGAAGGTTTGAGGACCTTCGACCAGCTGGACGCCATATCTAGTTTGCC
CACACCCAGTGACATCTTTGTGTCCTACTCTACTTTCCCAGGTTTTGTTTCCTGG
AGGGACCCCAAGAGTGGCTCCTGGTACGTTGAGACCCTGGACGACATCTTTGA
GCAGTGGGCTCACTCTGAAGACCTGCAGTCCCTCCTGCTTAGGGTCGCTAATG
CTGTTTCGGTGAAAGGGATTTATAAACAGATGCCTGGTTGCTTTAATTTCCTCCG
GAAAAAACTTTTCTTTAAAACATCA
SEQ ID NO: 21, FKBP12v36 (res. 2-108) 
G V Q V E T I S P G D G R T F P K R G Q T C V V H Y T G M L
E D G K K V D S S R D R N K P F K F M L G K Q E V I R G W E
E G V A Q M S V G Q R A K L T I S P D Y A Y G A T G H P G I I
P P H A T L V F D V E L L K L E
SEQ ID NO: 22, ΔCasp9 (res. 135-416) 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L D A I S S L P T P S D I F V S Y S T F P G F
V S W R D P K S G S W Y V E T L D D I F E Q W A H S E D L Q
S L L L R V A N A V S V K G I Y K Q M P G C F N F L R K K L F
F K T S
SEQ ID NO: 23, ΔCasp9 (res. 135-416) D330A, nucleotide sequence 
GGATTTGGTGATGTCGGTGCTCTTGAGAGTTTGAGGGGAAATGCAGATTTGGCT
TACATCCTGAGCATGGAGCCCTGTGGCCACTGCCTCATTATCAACAATGTGAAC
TTCTGCCGTGAGTCCGGGCTCCGCACCCGCACTGGCTCCAACATCGACTGTGA
GAAGTTGCGGCGTCGCTTCTCCTCGCTGCATTTCATGGTGGAGGTGAAGGGCG
ACCTGACTGCCAAGAAAATGGTGCTGGCTTTGCTGGAGCTGGCGCgGCAGGAC
CACGGTGCTCTGGACTGCTGCGTGGTGGTCATTCTCTCTCACGGCTGTCAGGC
CAGCCACCTGCAGTTCCCAGGGGCTGTCTACGGCACAGATGGATGCCCTGTGT
CGGTCGAGAAGATTGTGAACATCTTCAATGGGACCAGCTGCCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
 
GCCGCCATATCTAGTTTGCCCACACCCAGTGACATCTTTGTGTCCTACTCTACTT
TCCCAGGTTTTGTTTCCTGGAGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTAATTTCCTCCGGAAAAACTTTTCTTTAAAACATCA
SEQ ID NO: 24, ΔCasp9 (res. 135-416) D330A, amino acid sequence 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L A A I S S L P T P S D I F V S Y S T F P G F
V S W R D P K S G S W Y V E T L D D I F E Q W A H S E D L Q
S L L L R V A N A V S V K G I Y K Q M P G C F N F L R K K L F
F K T S
SEQ ID NO: 25, ΔCasp9 (res. 135-416) N405Q nucleotide sequence 
GGATTTGGTGATGTCGGTGCTCTTGAGAGTTTGAGGGGAAATGCAGATTTGGCT
TACATCCTGAGCATGGAGCCCTGTGGCCACTGCCTCATTATCAACAATGTGAAC
TTCTGCCGTGAGTCCGGGCTCCGCACCCGCACTGGCTCCAACATCGACTGTGA
GAAGTTGCGGCGTCGCTTCTCCTCGCTGCATTTCATGGTGGAGGTGAAGGGCG
ACCTGACTGCCAAGAAAATGGTGCTGGCTTTGCTGGAGCTGGCGcGGCAGGAC
CACGGTGCTCTGGACTGCTGCGTGGTGGTCATTCTCTCTCACGGCTGTCAGGC
CAGCCACCTGCAGTTCCCAGGGGCTGTCTACGGCACAGATGGATGCCCTGTGT
CGGTCGAGAAGATTGTGAACATCTTCAATGGGACCAGCTGCCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
GACGCCATATCTAGTTTGCCCACACCCAGTGACATCTTTGTGTCCTACTCTACTT
TCCCAGGTTTTGTTTCCTGGAGGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTCAGTTCCTCCGGAAAAAACTTTTCTTTAAAACATCA
SEQ ID NO: 26, ΔCasp9 (res. 135-416) N405Q amino acid sequence 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L D A I S S L P T P S D I F V S Y S T F P G F
V S W R D P K S G S W Y V E T L D D I F E Q W A H S E D L Q
S L L L R V A N A V S V K G I Y K Q M P G C F Q F L R K K L F
F K T S
SEQ ID NO: 27, ΔCasp9 (res. 135-416) D330A N405Q nucleotide sequence 
GGATTTGGTGATGTCGGTGCTCTTGAGAGTTTGAGGGAAATGCAGATTTGGCT
TACATCCTGAGCATGGAGCCCTGTGGCCACTGCCTCATTATCAACAATGTGAAC
 
TTCTGCCGTGAGTCCGGGCTCCGCACCCGCACTGGCTCCAACATCGACTGTGA
GAAGTTGCGGCGTCGCTTCTCCTCGCTGCATTTCATGGTGGAGGTGAAGGGCG
ACCTGACTGCCAAGAAAATGGTGCTGGCTTTGCTGGAGCTGGCGcGGCAGGAC
CACGGTGCTCTGGACTGCTGCGTGGTGGTCATTCTCTCTCACGGCTGTCAGGC
CAGCCACCTGCAGTTCCCAGGGGCTGTCTACGGCACAGATGGATGCCCTGTGT
CGGTCGAGAAGATTGTGAACATCTTCAATGGGACCAGCTGCCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
GCCGCCATATCTAGTTTGCCCACACCCAGTGACATCTTTGTGTCCTACTCTACTT
TCCCAGGTTTTGTTTCCTGGAGGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTCAGTTCCTCCGGAAAAAACTTTTCTTTAAAACATCA
SEQ ID NO: 28, ΔCasp9 (res. 135-416) D330A N405Q amino acid sequence 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L A A I S S L P T P S D I F V S Y S T F P G F
V S W R D P K S G S W Y V E T L D D I F E Q W A H S E D L Q
S L L L R V A N A V S V K G I Y K Q M P G C F Q F L R K K L F
F K T S
SEQ ID NO: 29, FKBPv36 (Fv1) nucleotide sequence 
GGCGTTCAAGTAGAAACAATCAGCCCAGGAGACGGAAGGACTTTCCCCAAACG
AGGCCAAACATGCGTAGTTCATTATACTGGGATGCTCGAAGATGGAAAAAAAGT
AGATAGTAGTAGAGACCGAAACAAACCATTTAAATTTATGTTGGGAAAACAAGAA
GTAATAAGGGGCTGGGAAGAAGGTGTAGCACAAATGTCTGTTGGCCAGCGCGC
AAAACTCACAATTTCTCCTGATTATGCTTACGGAGCTACCGGCCACCCCGGCAT
CATACCCCCTCATGCCACACTGGTGTTTGACGTCGAATTGCTCAAACTGGAA
SEQ ID NO: 30, FKBPv36 (Fv1) amino acid sequence 
GVQVETISPGDGRTFPKRGQTCVVHYTGMLEDGKKVDSSRDRNKPFKFMLGKQEV
IRGWEEGVAQMSVGQRAKLTISPDYAYGATGHPGIIPPHATLVFDVELLKLE
SEQ ID NO: 31, FKBPv36 (Fv2) nucleotide sequence 
GGaGTgCAgGTgGAgACgATtAGtCCtGGgGAtGGgAGaACcTTtCCaAAgCGcGGtCA
gACcTGtGTtGTcCAcTAcACcGGtATGCTgGAgGAcGGgAAgAAgGTgGActcTtcacGc
GAtCGcAAtAAgCCtTTcAAgTTcATGcTcGGcAAgCAgGAgGTgATccGGGGgTGGGA
gGAgGGcGTgGCtCAgATGTCgGTcGGgCAaCGaGCgAAgCTtACcATcTCaCCcGAc
TAcGCgTAtGGgGCaACgGGgCAtCCgGGaATtATcCCtCCcCAcGCtACgCTcGTaTTc
GAtGTgGAgcTcttgAAgCTtGag
SEQ ID NO: 32, FKBPv36 (Fv2) amino acid sequence GVQVETISPGDGRTFPKRGQTCVVHYTGMLEDGKKVDSSRDRNKPFKFMLGKQEV IRGWEEGVAQMSVGQRAKLTISPDYAYGATGHPGIIPPHATLVFDVELLKLE
SEQ ID NO: 33, ΔCD19 nucleotide sequence 
ATGCCCCCTCCTAGACTGCTGTTTTCCTGCTCTTTCTCACCCCAATGGAAGTTA
GACCTGAGGAACCACTGGTCGTTAAAGTGGAAGAAGGTGATAATGCTGTCCTCC
AATGCCTTAAAGGACCAGCGACGGACCAACGCAGCAACTGACTTGGAGCCGG
GAGTCCCCTCTCAAGCCGTTTCTCAAGCTGTCACTTGGCCTGCCAGGTCTTGGT
ATTCACATGCGCCCCCTTGCCATTTGGCTCTTCATATTCAATGTGTCTCAACAAA
TGGGTGGATTCTACCTTTGCCAGCCCGGCCCCCCTTCTGAGAAAGCTTGGCAG
CCTGGATGGACCGTCAATGTTGAAGGCTCCGGTGAGCTGTTTAGATGGAATGT
GAGCGACCTTGGCGGACTCGGTTGCGGACTGAAAAATAGGAGCTCTGAAGGAC
CCTCTTCTCCCTCCGGTAAGTTGATGTCACCTAAGCTGTACGTGTGGGCCAAGG
ACCGCCCCGAAATCTGGAGGGCGAGCCTCCATGCCTGCCGCCTCGCGATTCA
CTGAACCAGTCTCTGTCCCAGGATCTCACTATGGCGCCCGGATCTACTCTTTGG
CTGTCTTGCGGCGTTCCCCCAGATAGCGTGTCAAGAGGACCTCTGAGCTGGAC
CCACGTACACCCTAAGGGCCCTAAGAGCTTGTTGAGCCTGGAACTGAAGGACG
ACAGACCCGCACGCGATATGTGGGTAATGGAGACCGGCCTTCTGCTCCCTCGC
GCTACCGCACAGGATGCAGGGAAATACTACTGTCATAGAGGGAATCTGACTATG
AGCTTTCATCTCGAAATTACAGCACGGCCCGTTCTTTGGCATTGGCTCCTCCGG
ACTGGAGGCTGGAAGGTGTCTGCCGTAACACTCGCTTACTTGATTTTTTGCCTG
TGTAGCCTGGTTGGGATCCTGCATCTTCAGCGAGCCCTTGTATTGCGCCGAAAA
AGAAAACGAATGACTGACCCTACACGACGATTCTGA
SEQ ID NO: 34, ΔCD19 amino acid sequence 
MPPPRLLFFLLFLTPMEVRPEEPLVVKVEEGDNAVLQCLKGTSDGPTQQLTWSRES
PLKPFLKLSLGLPGLGIHMRPLAIWLFIFNVSQQMGGFYLCQPGPPSEKAWQPGWT
VNVEGSGELFRWNVSDLGGLGCGLKNRSSEGPSSPSGKLMSPKLYVWAKDRPEI
WEGEPPCLPPRDSLNQSLSQDLTMAPGSTLWLSCGVPPDSVSRGPLSWTHVHPK
GPKSLLSLELKDDRPARDMWVMETGLLLPRATAQDAGKYYCHRGNLTMSFHLEITA
RPVLWHWLLRTGGWKVSAVTLAYLIFCLCSLVGILHLQRALVLRRKRKRMTDPTRR
F*
Codon optimized iCasp9-N405Q-2A-ΔCD19 sequence: (the .co following the name of a nucleotide sequence indicates that it is codon optimized (or the amino acid sequence coded by the codon-optimized nucleotide sequence). 
SEQ-ID NO: 35, FKBPv36.co (Fv3) nucleotide sequence
ATGCTGGAGGGAGTGCAGGTGGAGACTATTAGCCCCGGAGATGGCAGAACATT
CCCCAAAAGAGGACAGACTTGCGTCGTGCATTATACTGGAATGCTGGAAGACG
GCAAGAAGGTGGACAGCAGCCGGGACCGAAACAAGCCCTTCAAGTTCATGCTG
GGGAAGCAGGAAGTGATCCGGGGCTGGGAGGAAGGAGTCGCACAGATGTCAG
TGGGACAGAGGGCCAAACTGACTATTAGCCCAGACTACGCTTATGGAGCAACC
GGCCACCCCGGGATCATTCCCCCTCATGCTACACTGGTCTTCGATGTGGAGCT
GCTGAAGCTGGAA
SEQ ID NO: 36, FKBPv36.co (Fv3) amino acid sequence 
MLEGVQVETISPGDGRTFPKRGQTCVVHYTGMLEDGKKVDSSRDRNKPFKFMLGK
QEVIRGWEEGVAQMSVGQRAKLTISPDYAYGATGHPGIIPPHATLVFDVELLKLE
SEQ ID NO: 37, Linker.co nucleotide sequence
   AGCGGAGGAGGATCCGGA
SEQ ID NO: 38, Linker.co amino acid sequence
   SGGGSG
SEQ IDNO: 39, Caspase-9.co nucleotide sequence 
GTGGACGGGTTTGGAGATGTGGGAGCCCTGGAATCCCTGCGGGCAATGCCG
ATCTGGCTTACATCCTGTCTATGGAGCCTTGCGGCCACTGTCTGATCATTAACA
ATGTGAACTTCTGCAGAGAGAGCGGGCTGCGGACCAGAACAGGATCCAATATT
GACTGTGAAAAGCTGCGGAGAAGGTTCTCTAGTCTGCACTTTATGGTCGAGGTG
AAAGGCGATCTGACCGCTAAGAAAATGGTGCTGGCCCTGCTGGAACTGGCTCG
GCAGGACCATGGGGCACTGGATTGCTGCGTGGTCGTGATCCTGAGTCACGGCT
GCCAGGCTTCACATCTGCAGTTCCCTGGGGCAGTCTATGGAACTGACGGCTGT
CCAGTCAGCGTGGAGAAGATCGTGAACATCTTCAACGGCACCTCTTGCCCAAGT
CTGGGCGGGAAGCCCAAACTGTTCTTTATTCAGGCCTGTGGAGGCGAGCAGAA
AGATCACGGCTTCGAAGTGGCTAGCACCTCCCCCGAGGACGAATCACCTGGAA
GCAACCCTGAGCCAGATGCAACCCCCTTCCAGGAAGGCCTGAGGACATTTGAC
CAGCTGGATGCCATCTCAAGCCTGCCCACACCTTCTGACATTTTCGTCTCTTAC
AGTACTTTCCCTGGATTTGTGAGCTGGCGCGATCCAAAGTCAGGCAGCTGGTAC
GTGGAGACACTGGACGATATCTTTGAGCAGTGGGCCCATTCTGAAGACCTGCA
GAGTCTGCTGCTGCGAGTGGCCAATGCTGTCTCTGTGAAGGGGATCTACAAAC
AGATGCCAGGATGCTTCCAGTTTCTGAGAAAGAAACTGTTCTTTAAGACCTCCG
CATCTAGGGCC
SEQ ID NO: 40, Caspase-9.co amino acid sequence 
VDGFGDVGALESLRGNADLAYILSMEPCGHCLIINNVNFCRESGLRTRTGSNIDCEK
LRRRFSSLHFMVEVKGDLTAKKMVLALLELARQDHGALDCCVVVILSHGCQASHLQ
FPGAVYGTDGCPVSVEKIVNIFNGTSCPSLGGKPKLFFIQACGGEQKDHGFEVAST
SPEDESPGSNPEPDATPFQEGLRTFDQLDAISSLPTPSDIFVSYSTFPGFVSWRDPK
SGSWYVETLDDIFEQWAHSEDLQSLLLRVANAVSVKGIYKQMPGCFQFLRKKLFFK
TSASRA
SEQ ID NO: 41, Linker.co nucleotide sequence
   CCGCGG
SEQ ID NO: 42, Linker.co amino acid sequence
   PR
SEQ ID NO: 42: T2A.co nucleotide sequence 
GAAGGCCGAGGGAGCCTGCTGACATGTGGCGATGTGGAGGAAAACCCAGGAC
CA
SEQ ID NO: 43: T2A.co amino acid sequence
   EGRGSLLTCGDVEENPGP
SEQ ID NO: 43: Δ CD19.co nucleotide sequence 
ATGCCACCACCTCGCCTGCTGTTCTTTCTGCTGTTCCTGACACCTATGGAGGTG
CGACCTGAGGAACCACTGGTCGTGAAGGTCGAGGAAGGCGACAATGCCGTGCT
 
GCAGTGCCTGAAAGGCACTTCTGATGGGCCAACTCAGCAGCTGACCTGGTCCA
GGGAGTCTCCCCTGAAGCCTTTTCTGAAACTGAGCCTGGACTGCCAGGACTG
GGAATCCACATGCGCCCTCTGGCTATCTGGCTGTTCATCTTCAACGTGAGCCAG
CAGATGGGAGGATTCTACCTGTGCCAGCCAGGACCACCATCCGAGAAGGCCTG
GCAGCCTGGATGGACCGTCAACGTGGAGGGGTCTGGAGAACTGTTTAGGTGGA
ATGTGAGTGACCTGGGAGGACTGGGATGTGGGCTGAAGAACCGCTCCTCTGAA
GGCCCAAGTTCACCCTCAGGGAAGCTGATGAGCCCAAAACTGTACGTGTGGGC
CAAAGATCGGCCCGAGATCTGGGAGGGAGAACCTCCATGCCTGCCACCTAGAG
ACAGCCTGAATCAGAGTCTGTCACAGGATCTGACAATGGCCCCCGGGTCCACT
CTGTGGCTGTCTTGTGGAGTCCCACCCGACAGCGTGTCCAGAGGCCCTCTGTC
CTGGACCCACGTGCATCCTAAGGGGCCAAAAAGTCTGCTGTCACTGGAACTGA
AGGACGATCGGCCTGCCAGAGACATGTGGGTCATGGAGACTGGACTGCTGCTG
CCACGAGCAACCGCACAGGATGCTGGAAAATACTATTGCCACCGGGGCAATCT
GACAATGTCCTTCCATCTGGAGATCACTGCAAGGCCCGTGCTGTGGCACTGGC
TGCTGCGAACCGGAGGATGGAAGGTCAGTGCTGTGACACTGGCATATCTGATC
TTTTGCCTGTGCTCCCTGGTGGGCATTCTGCATCTGCAGAGAGCCCTGGTGCT
GCGGAGAAAGAGAAAGAGAATGACTGACCCAACAAGAAGGTTTTGA
SEQ ID NO: 43: Δ CD19.co amino acid sequence

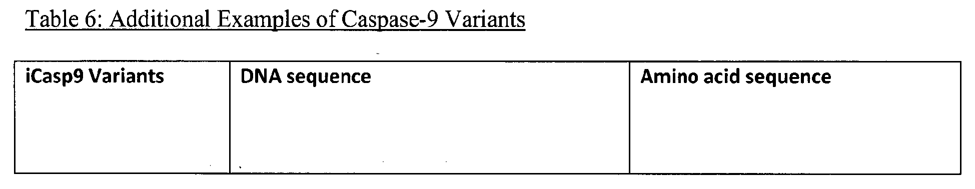

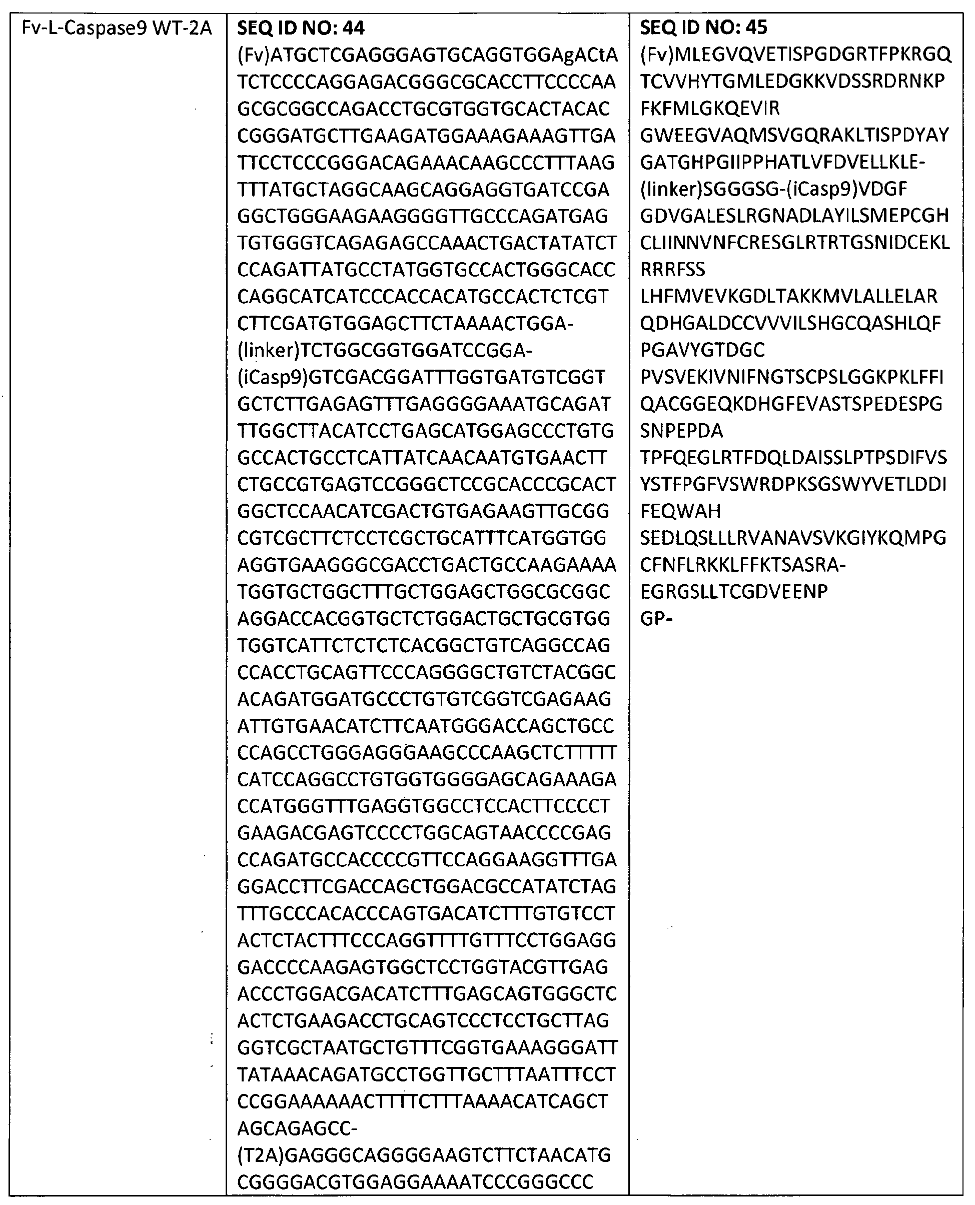

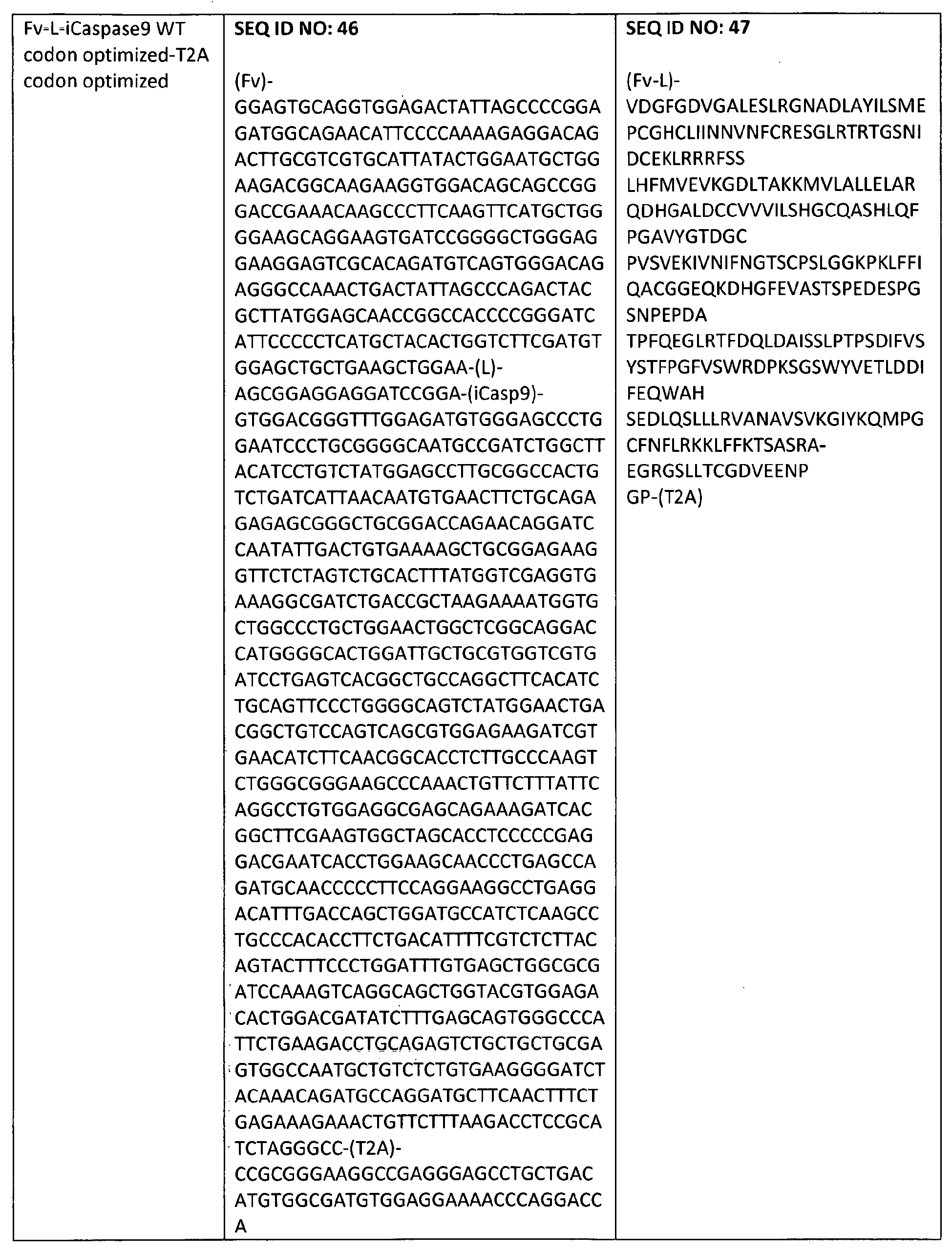

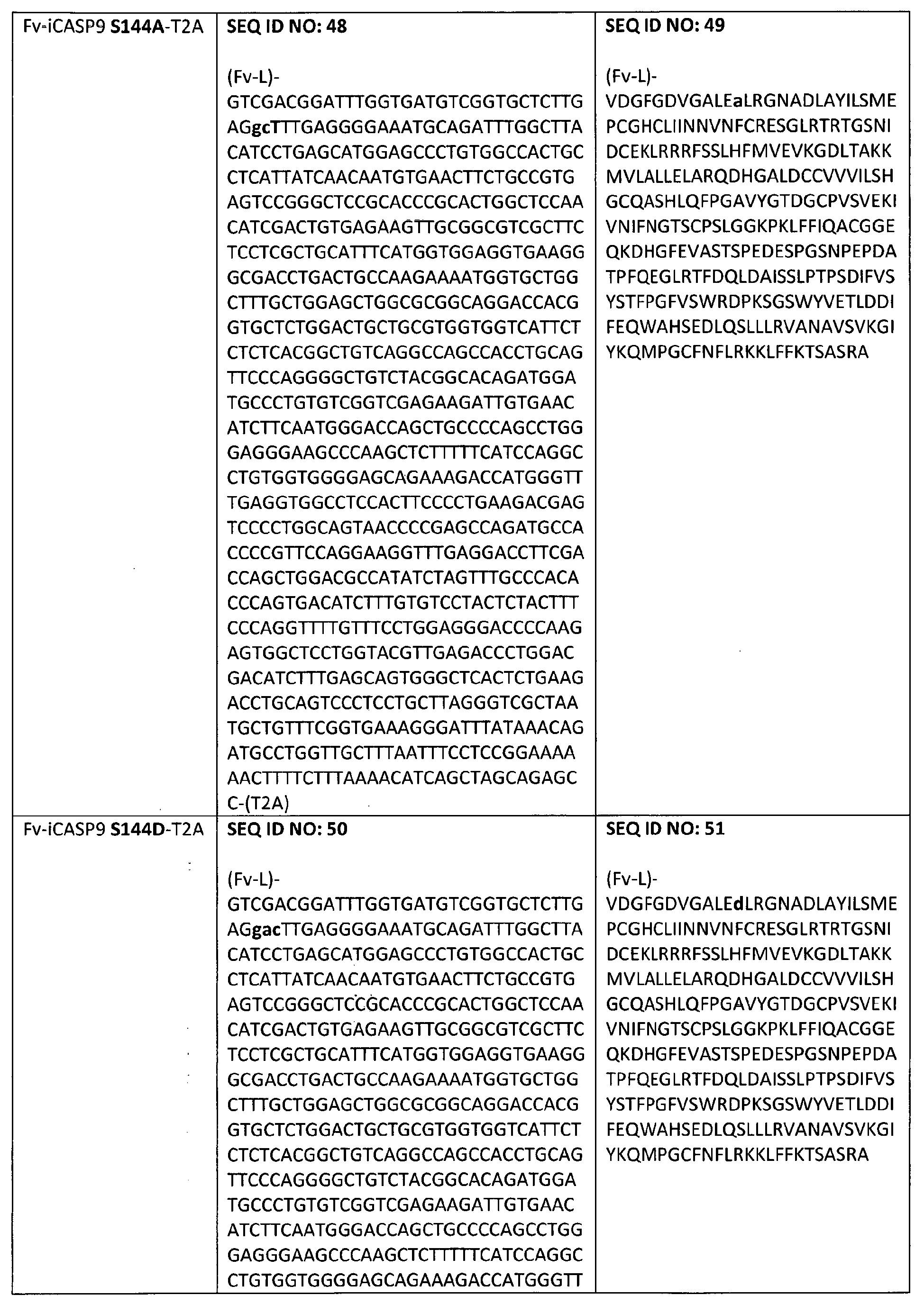

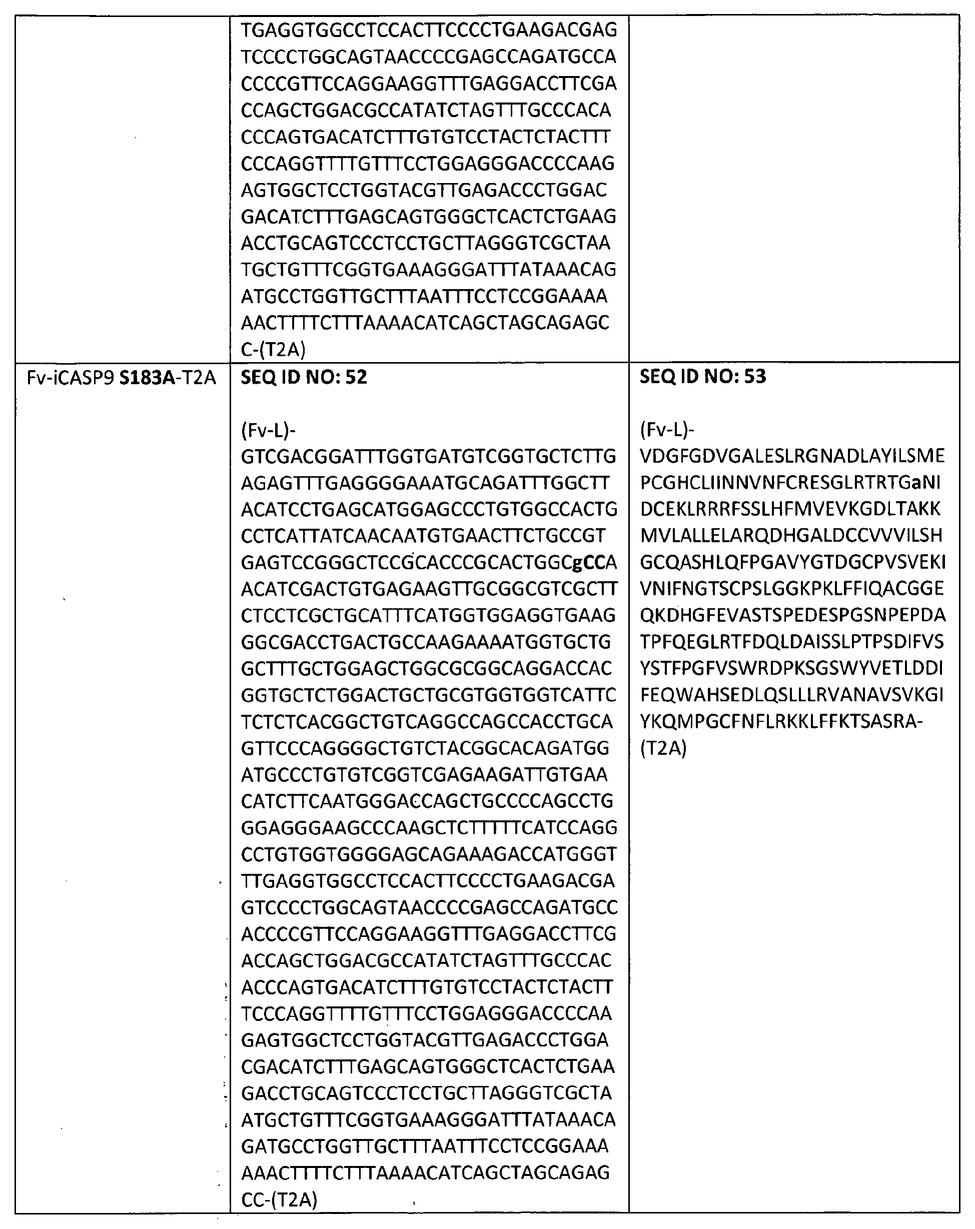

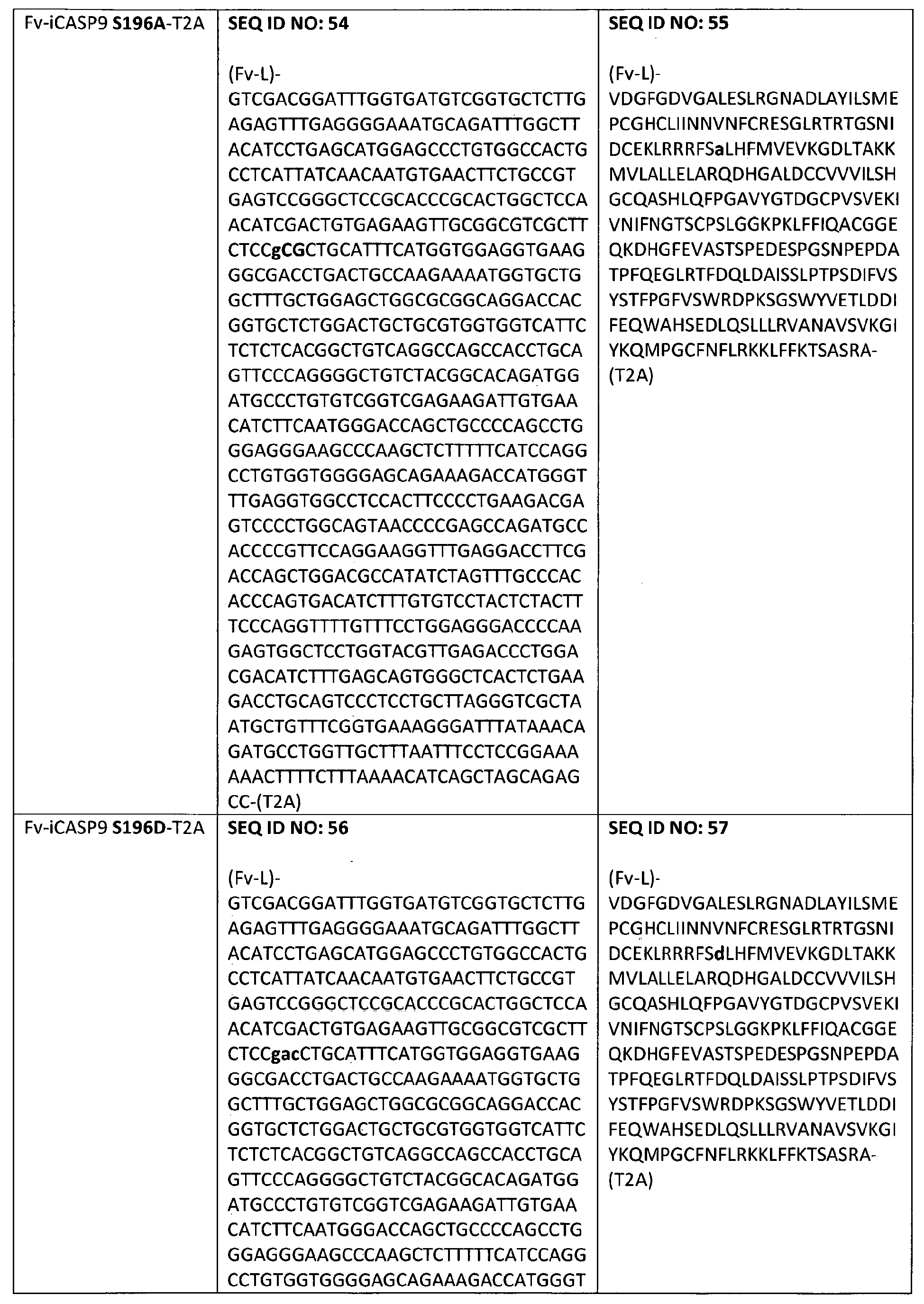

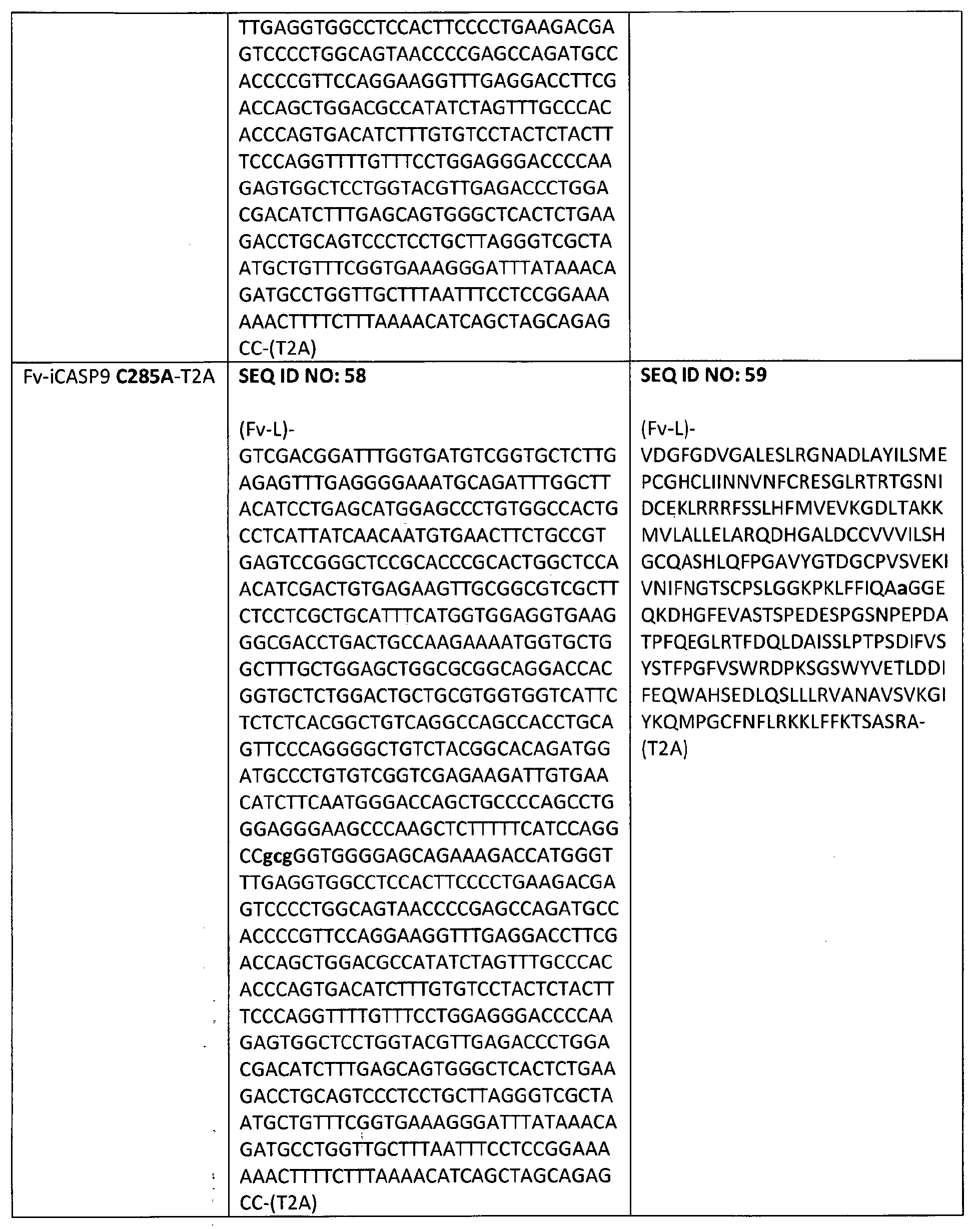

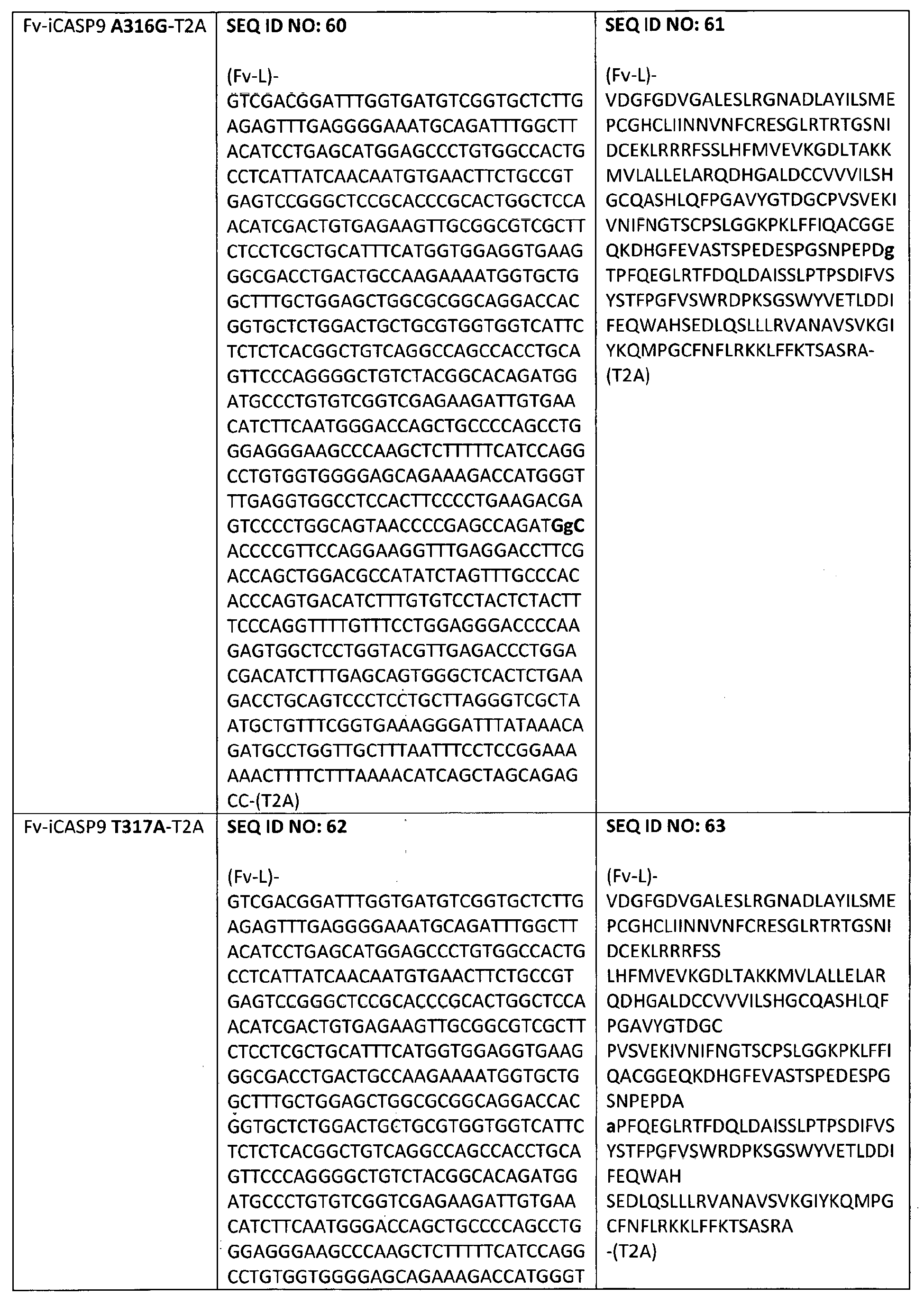

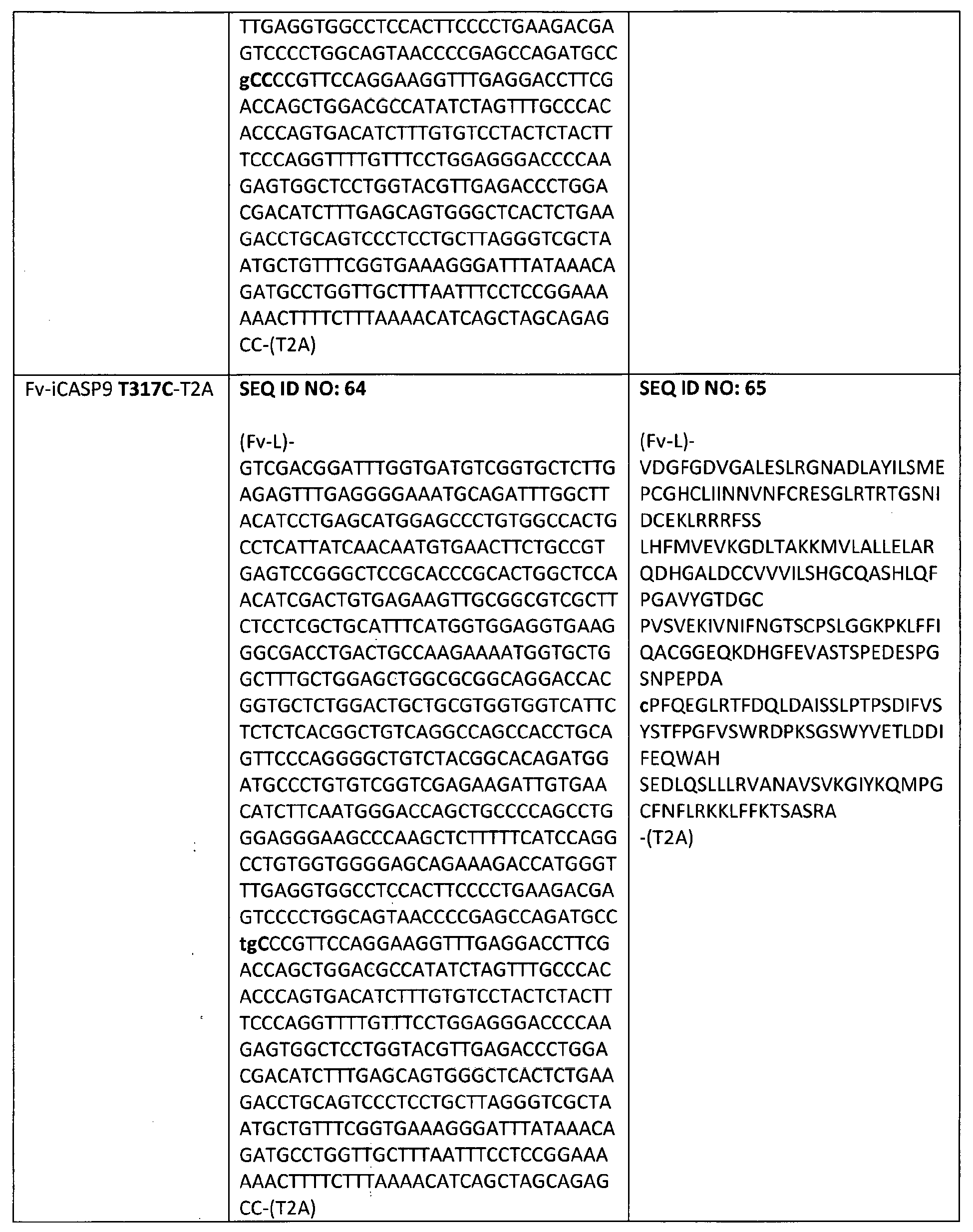

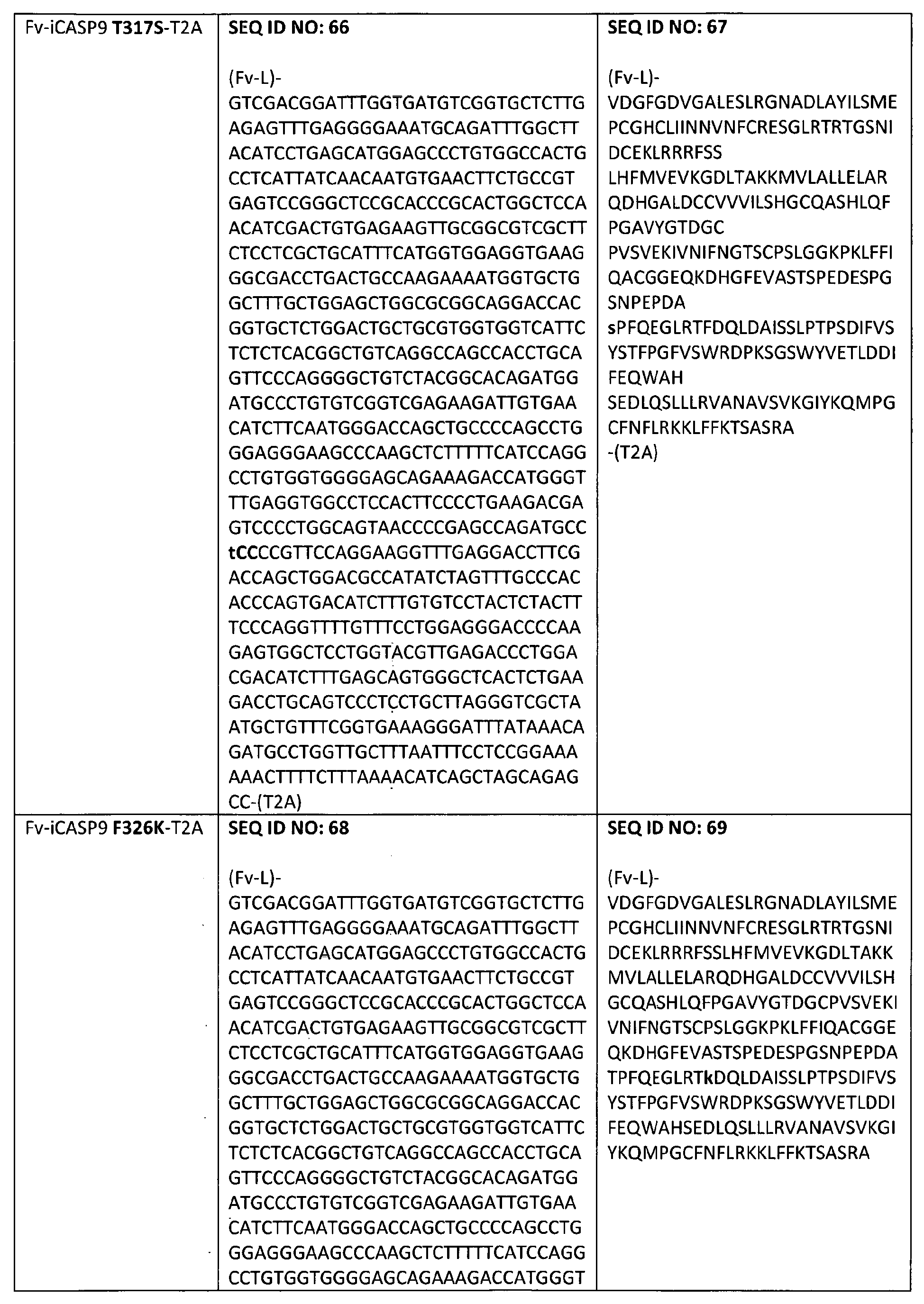

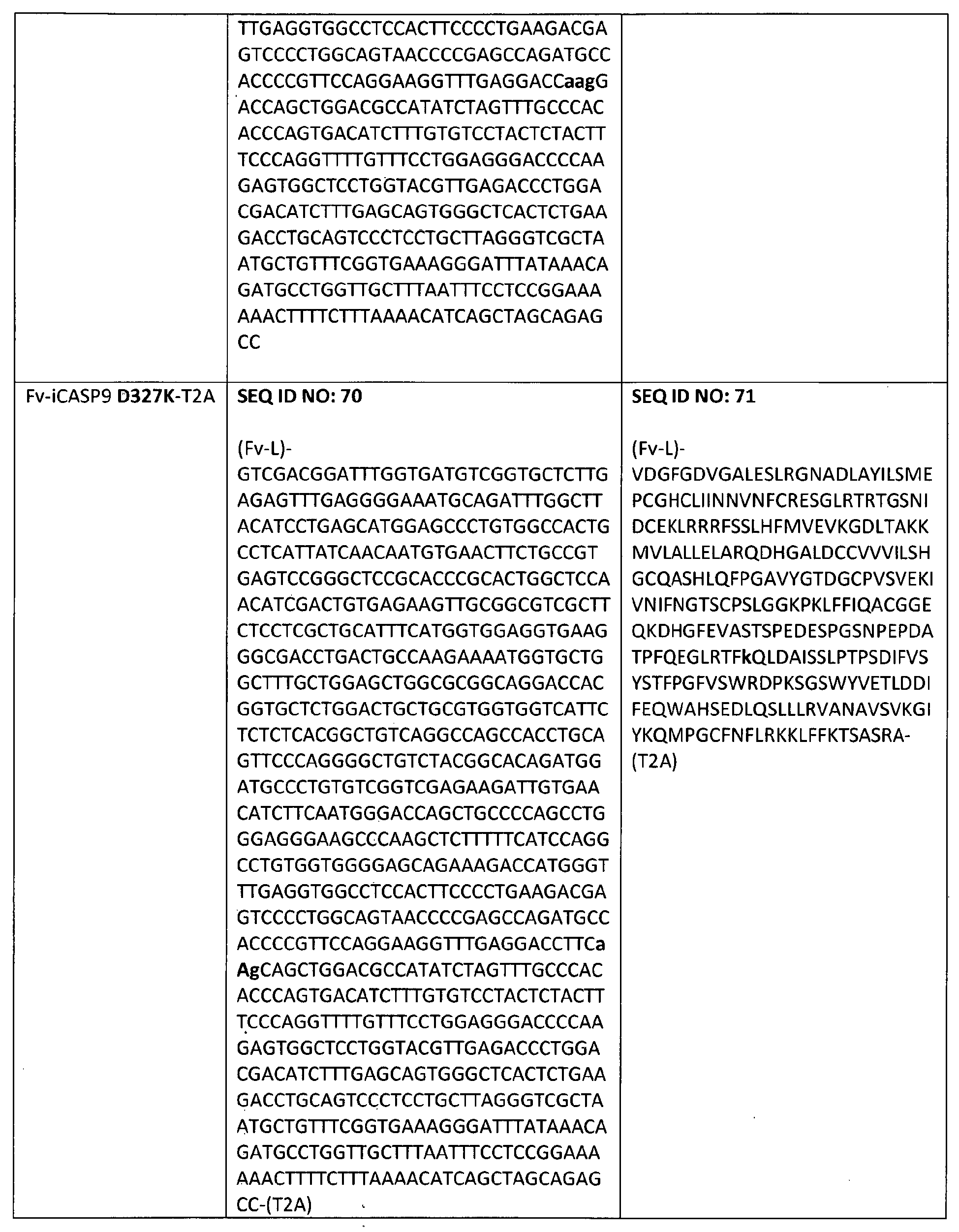

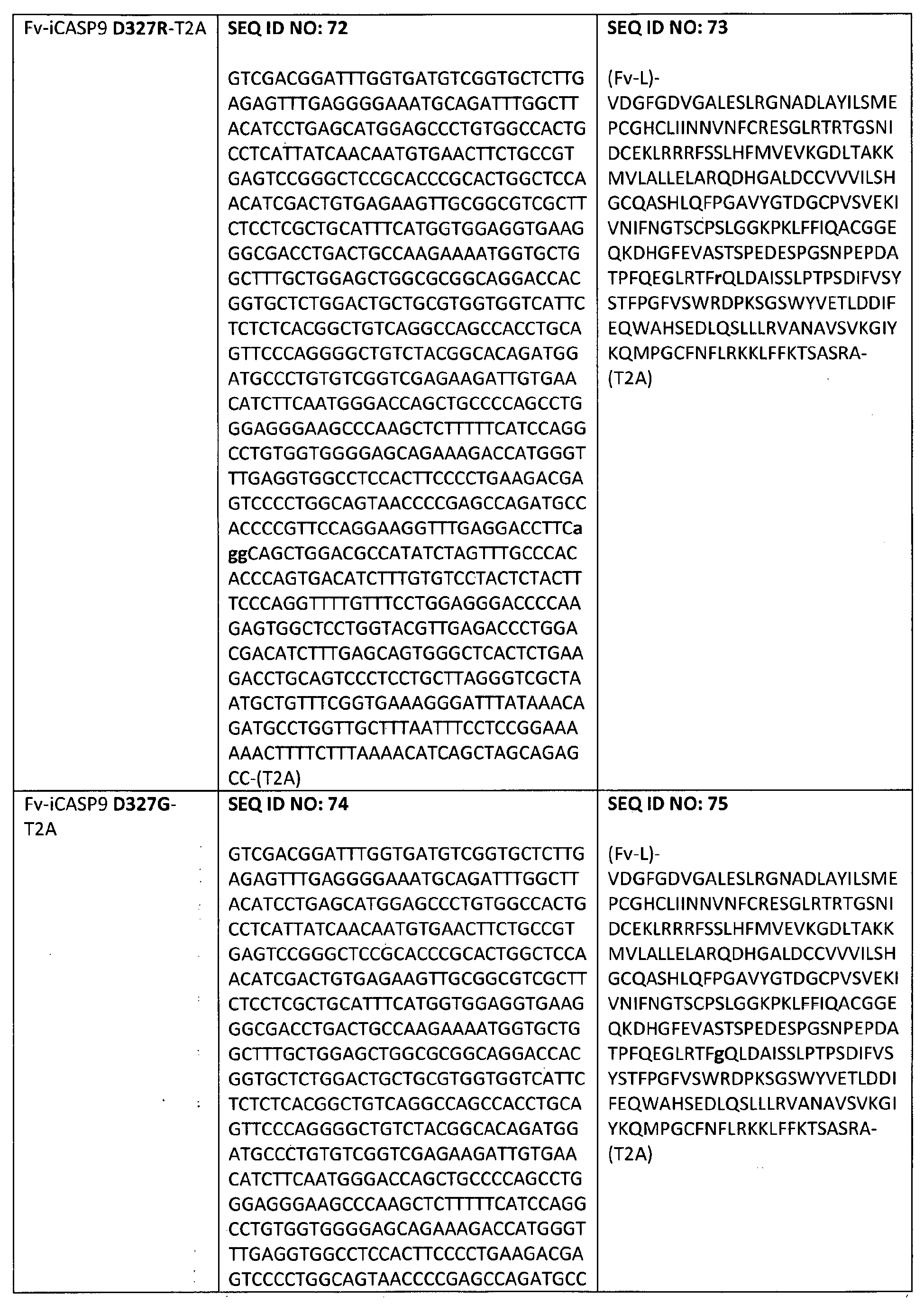

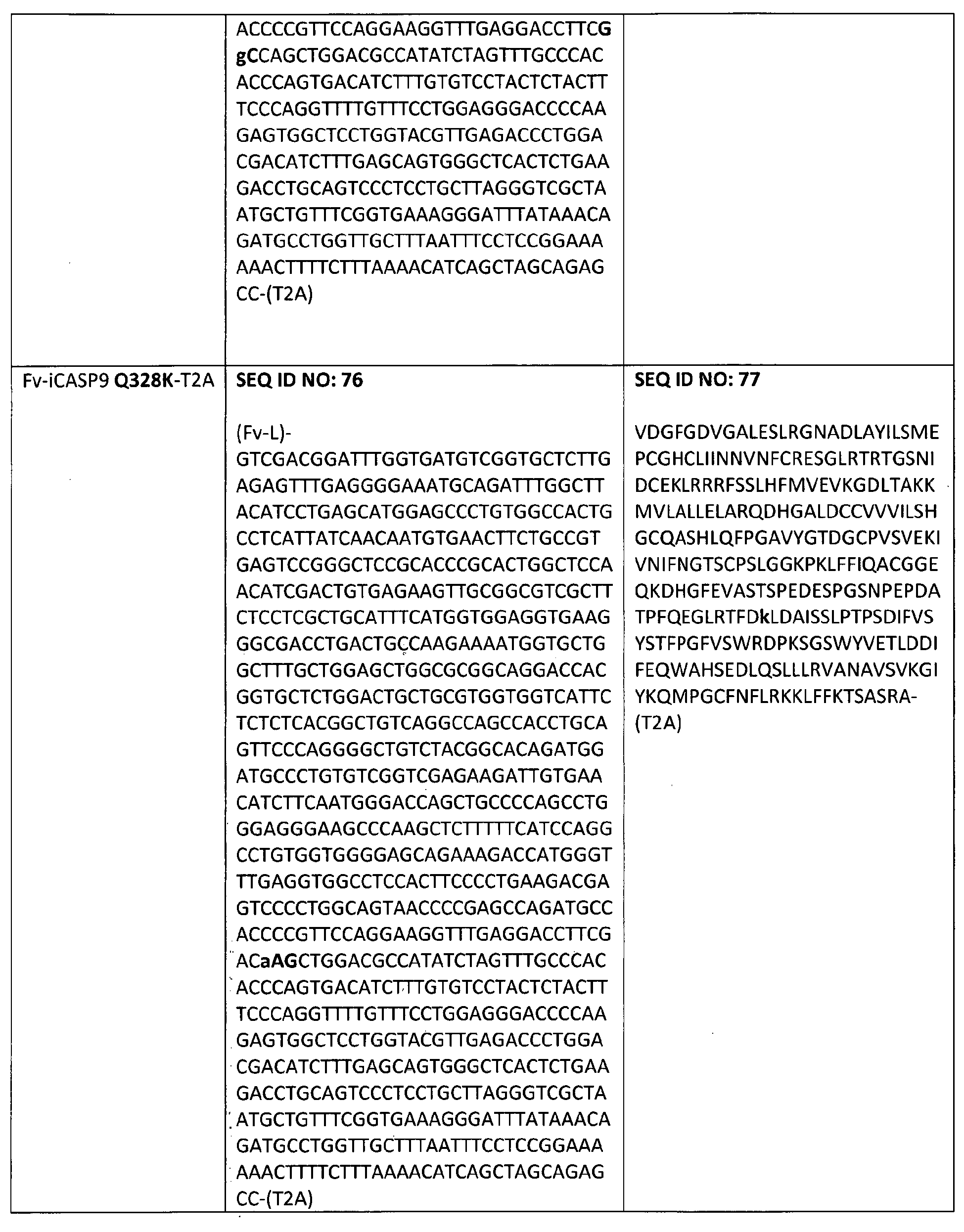

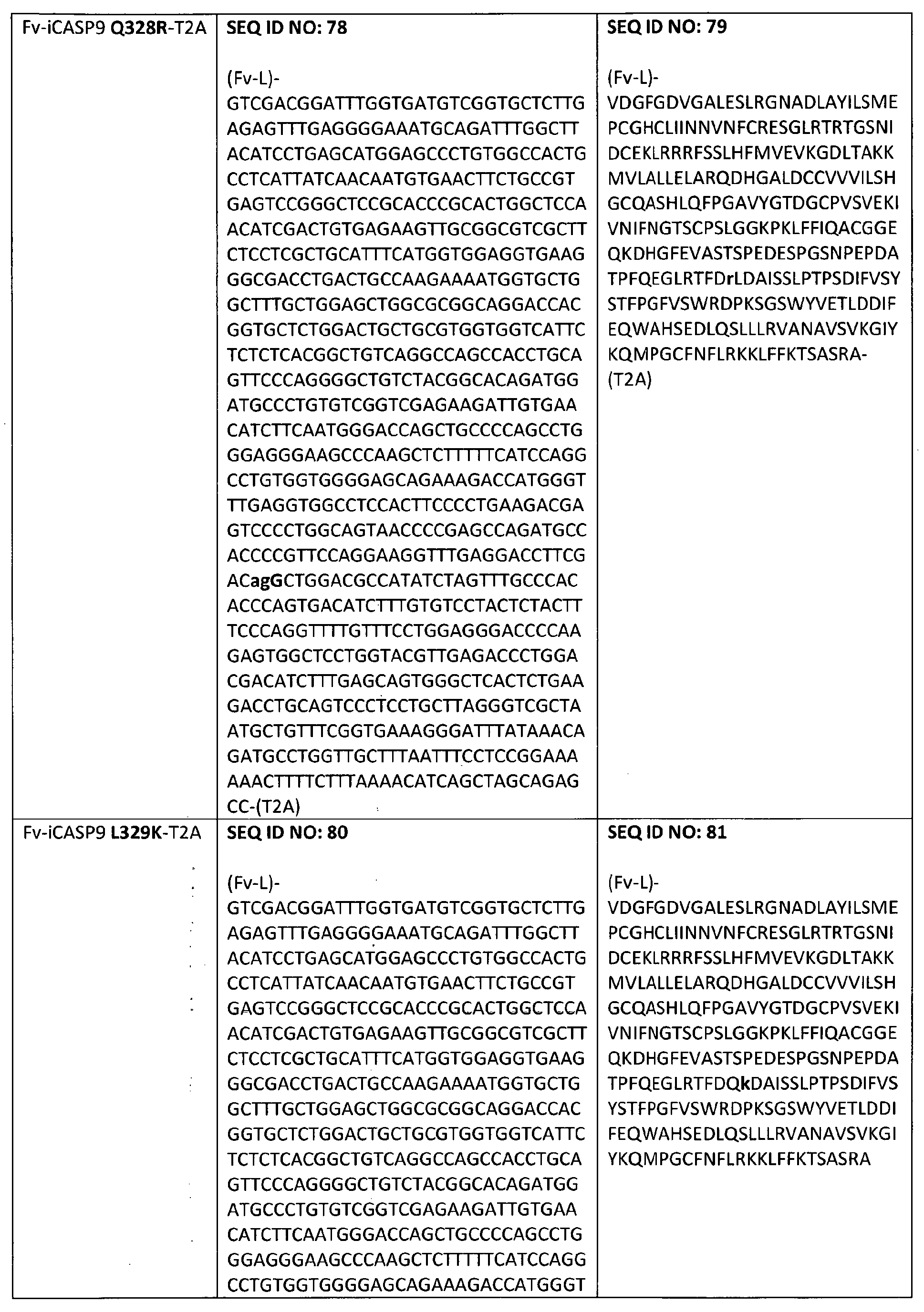

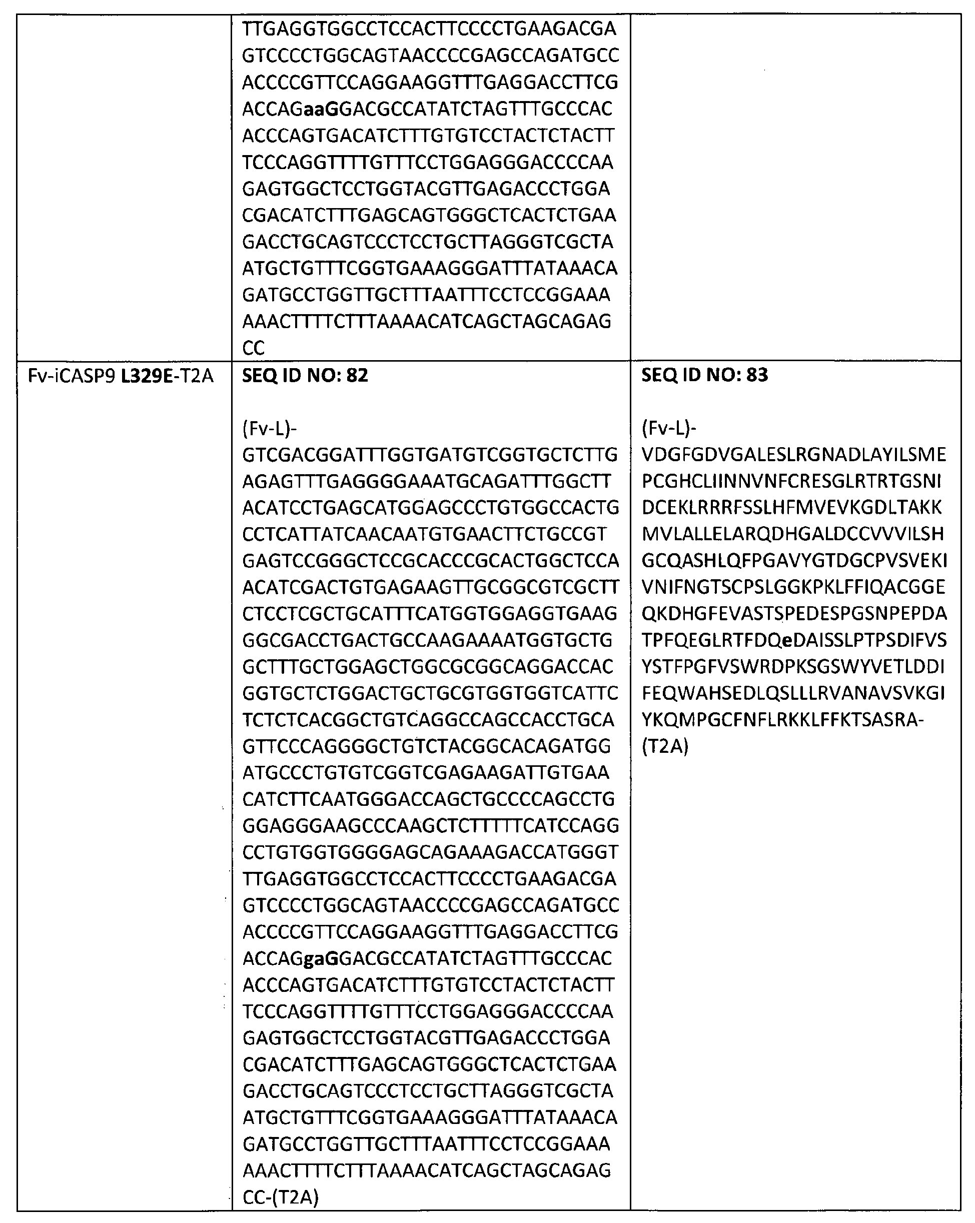

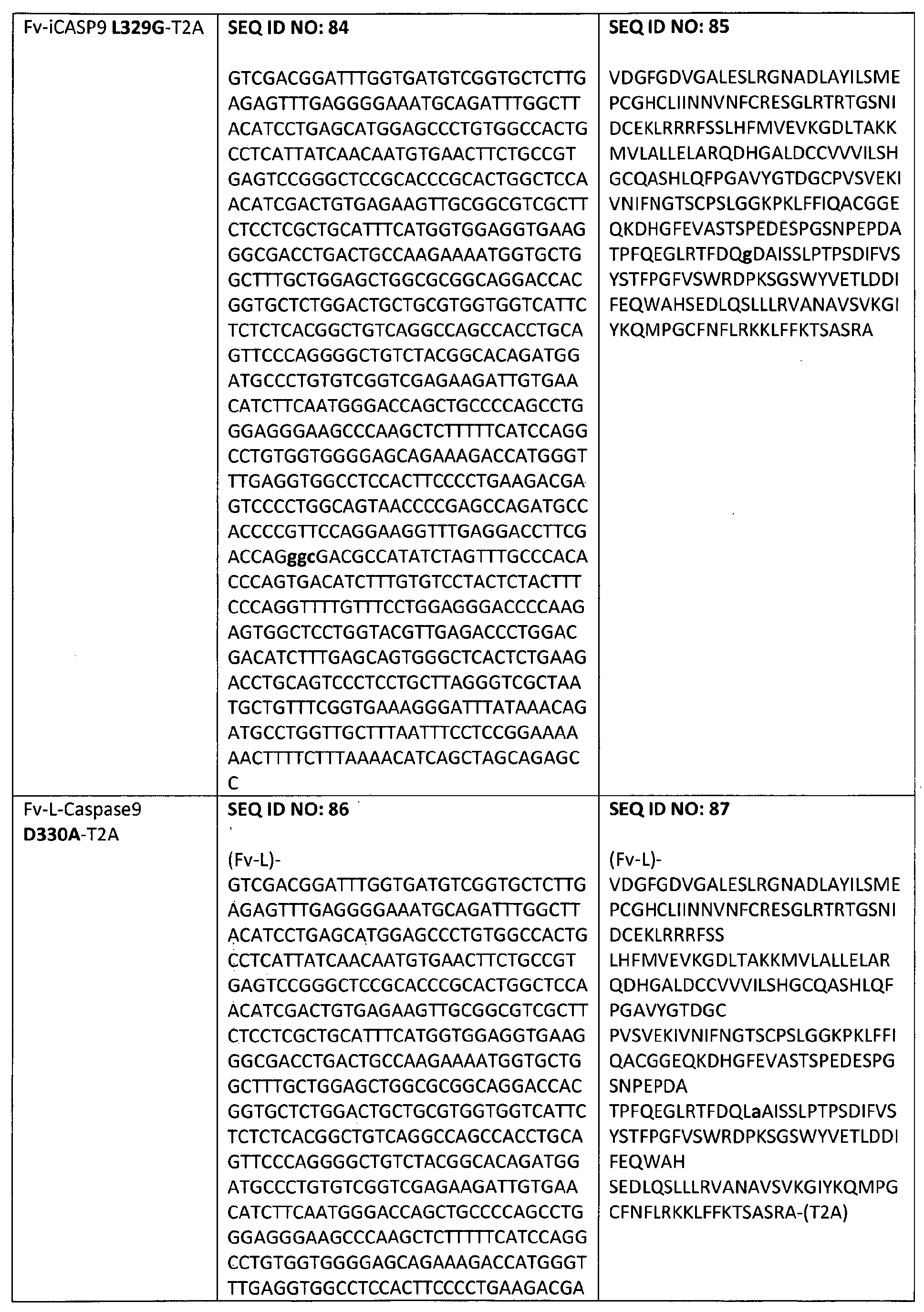

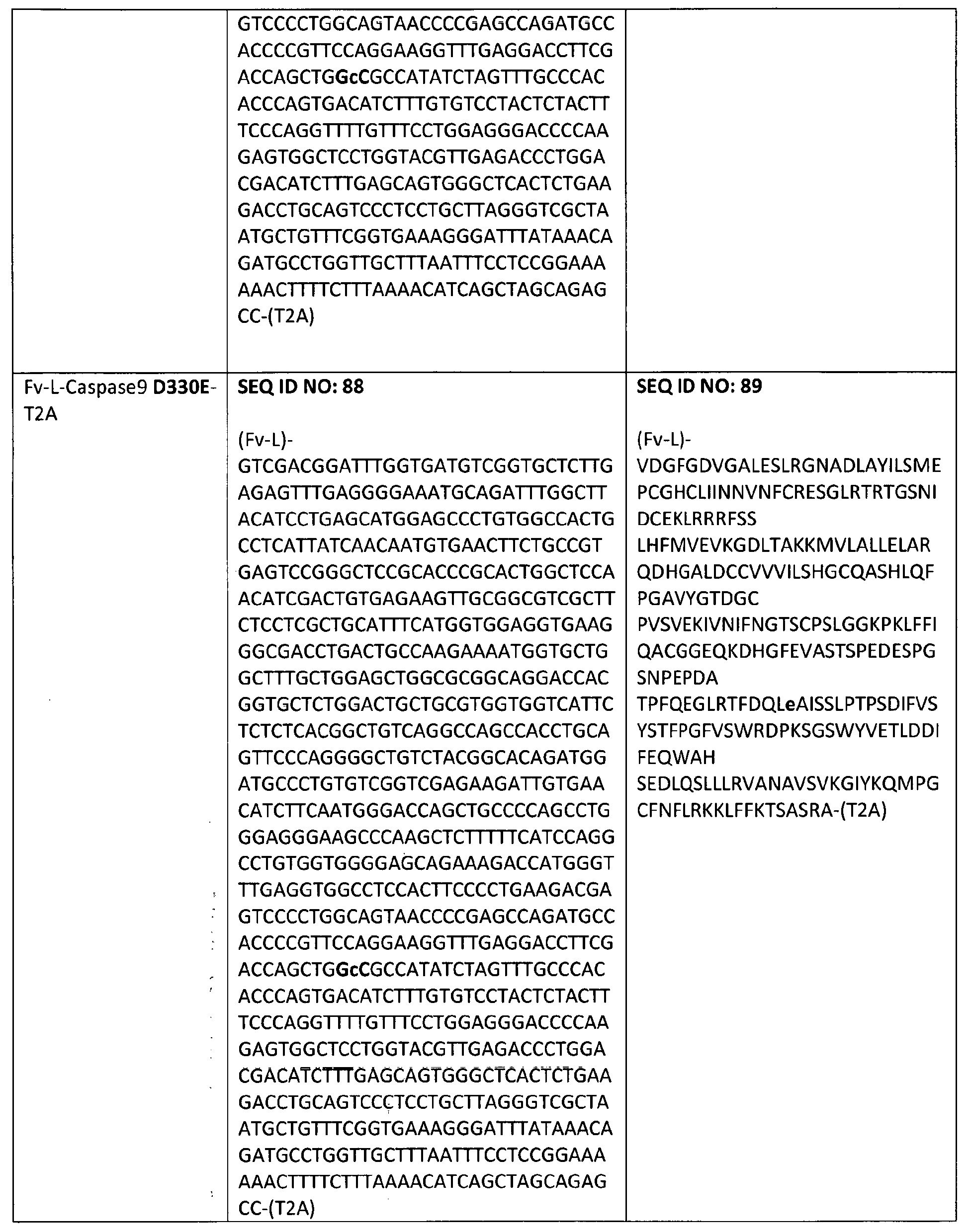

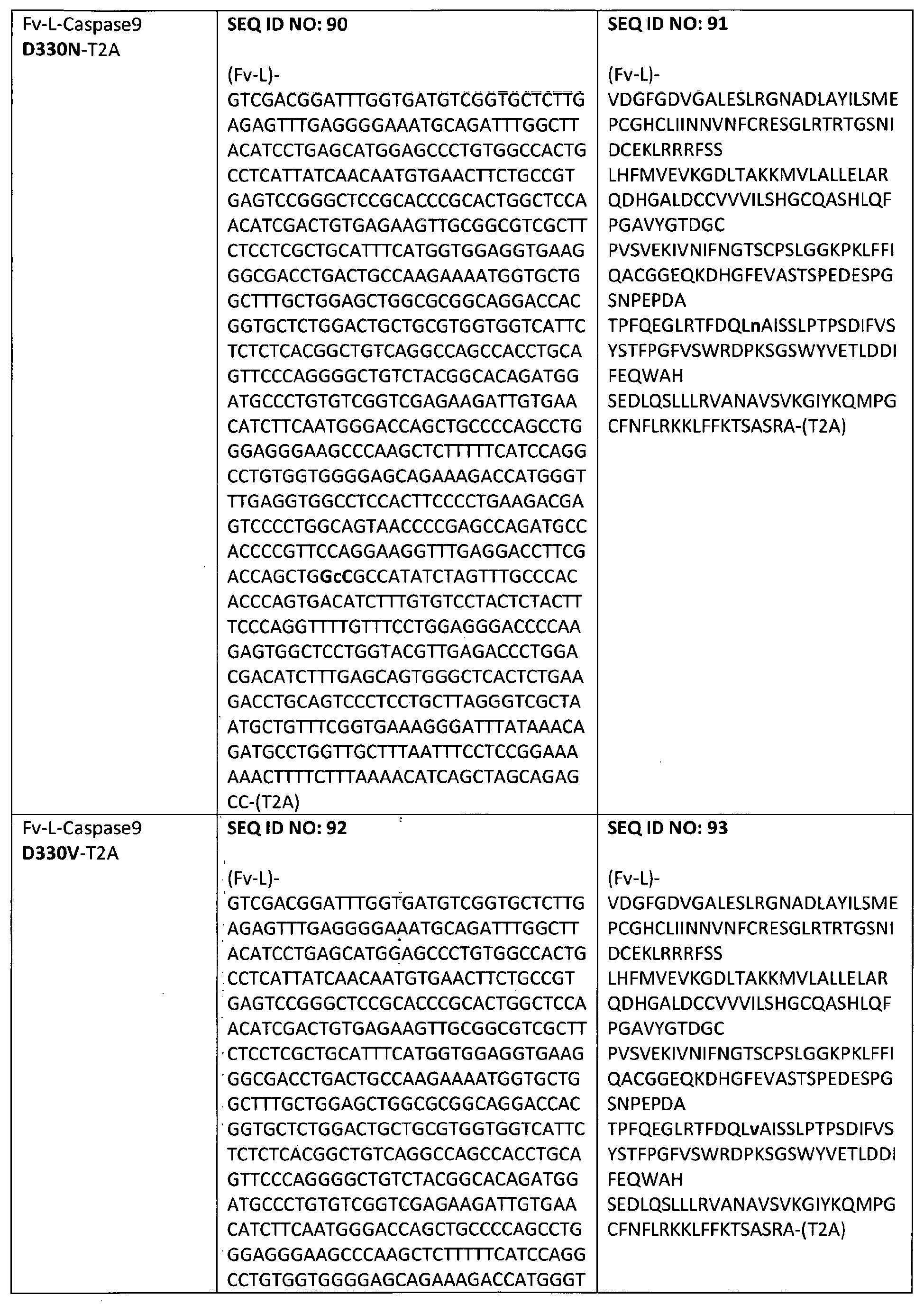

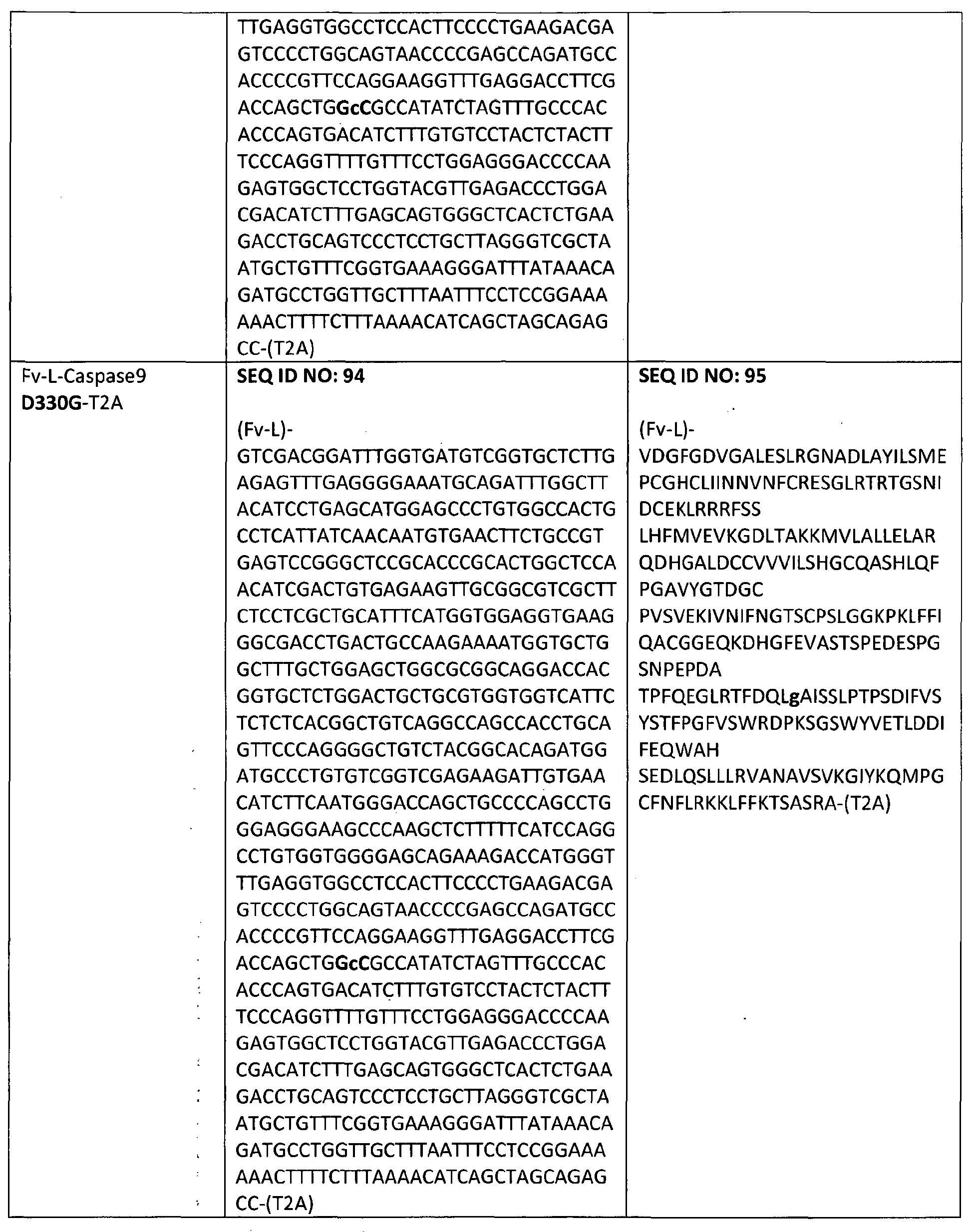

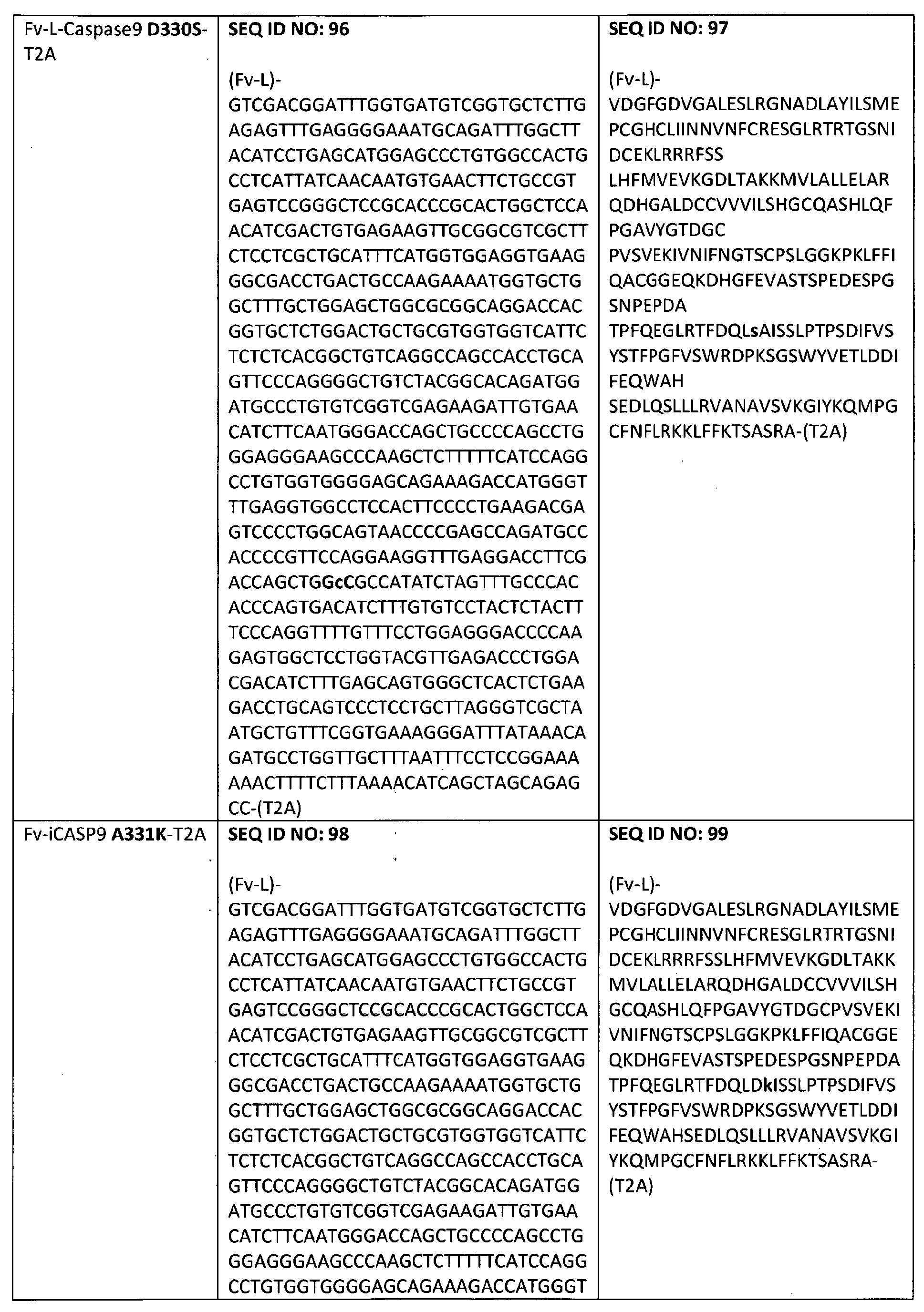

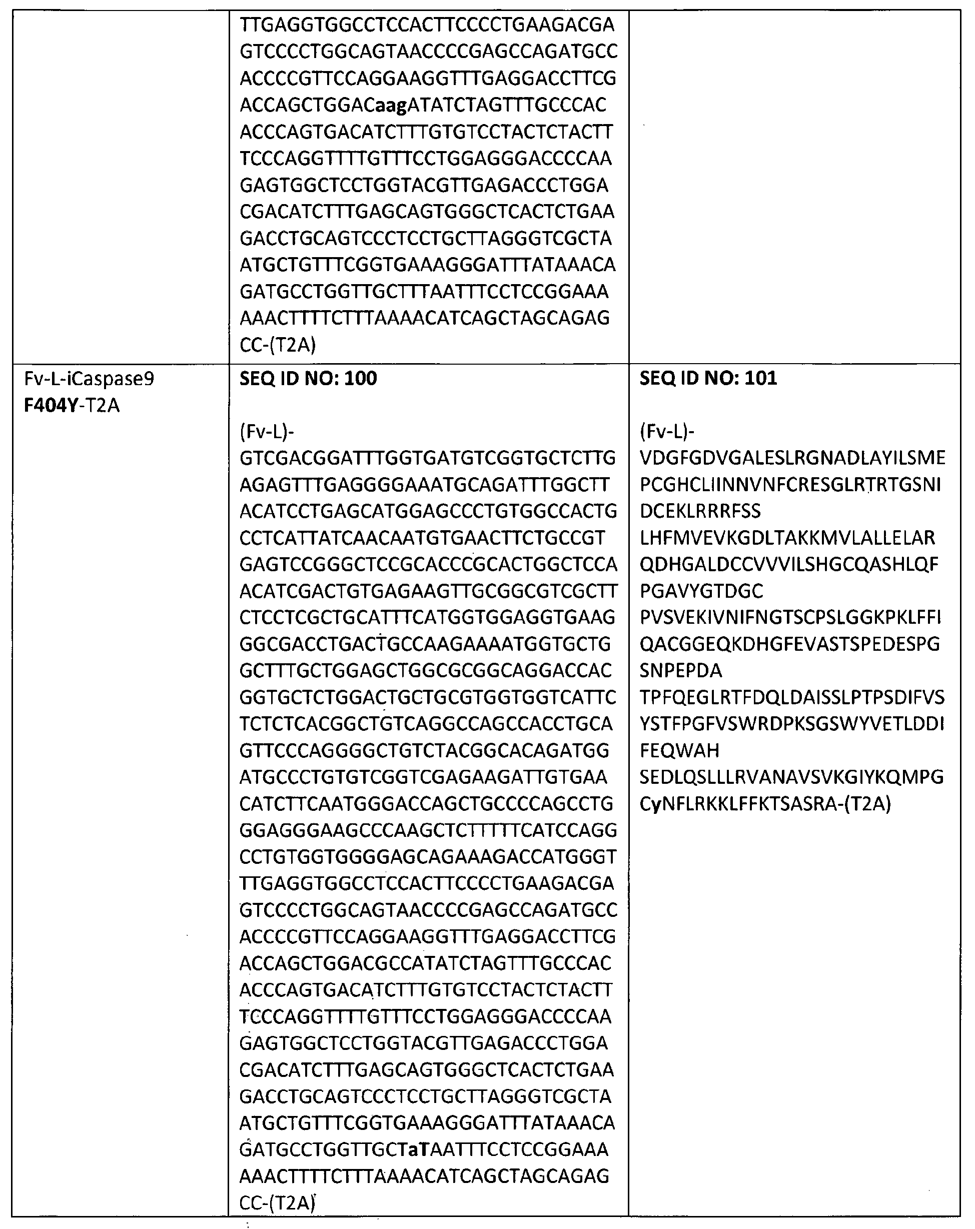

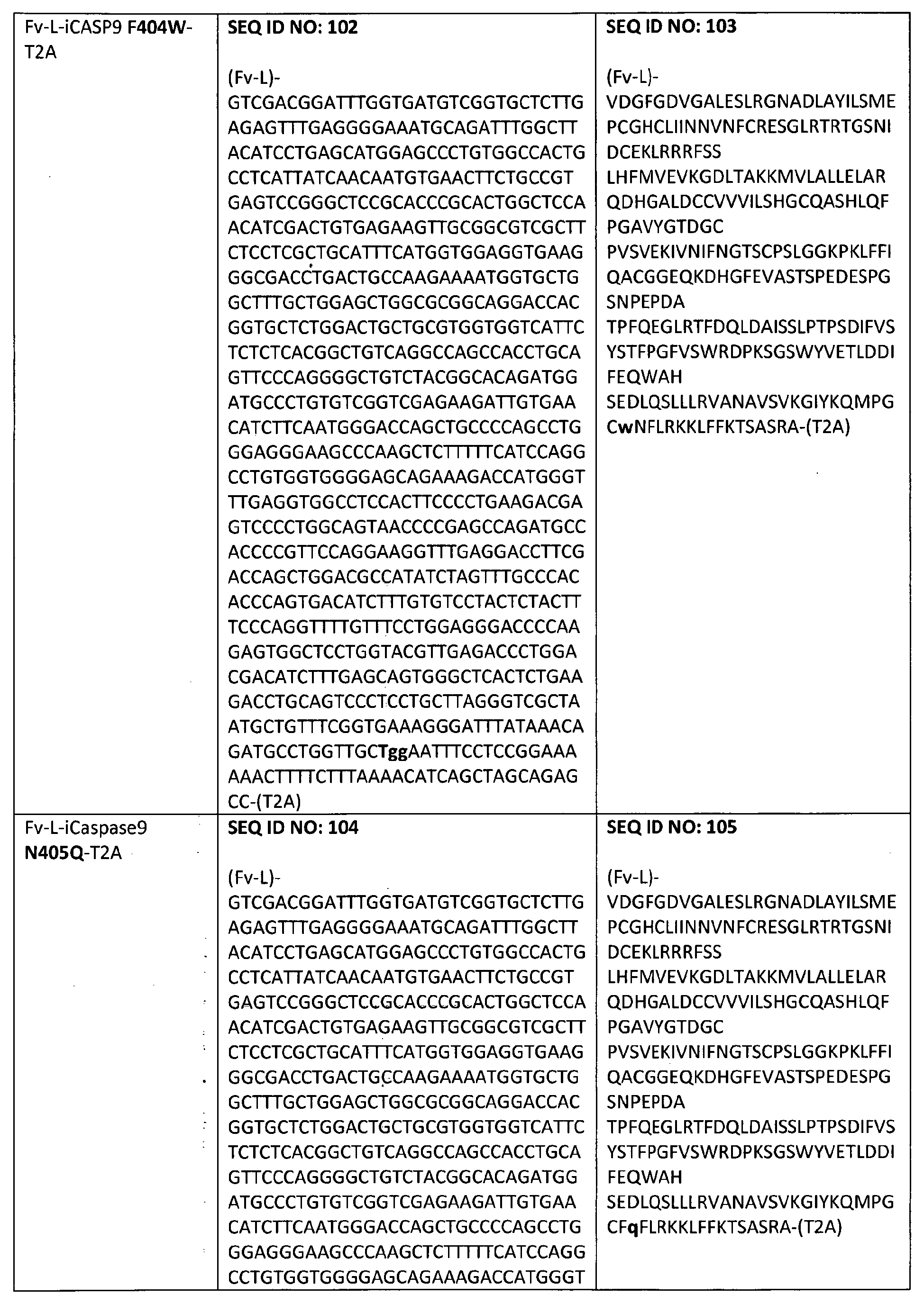

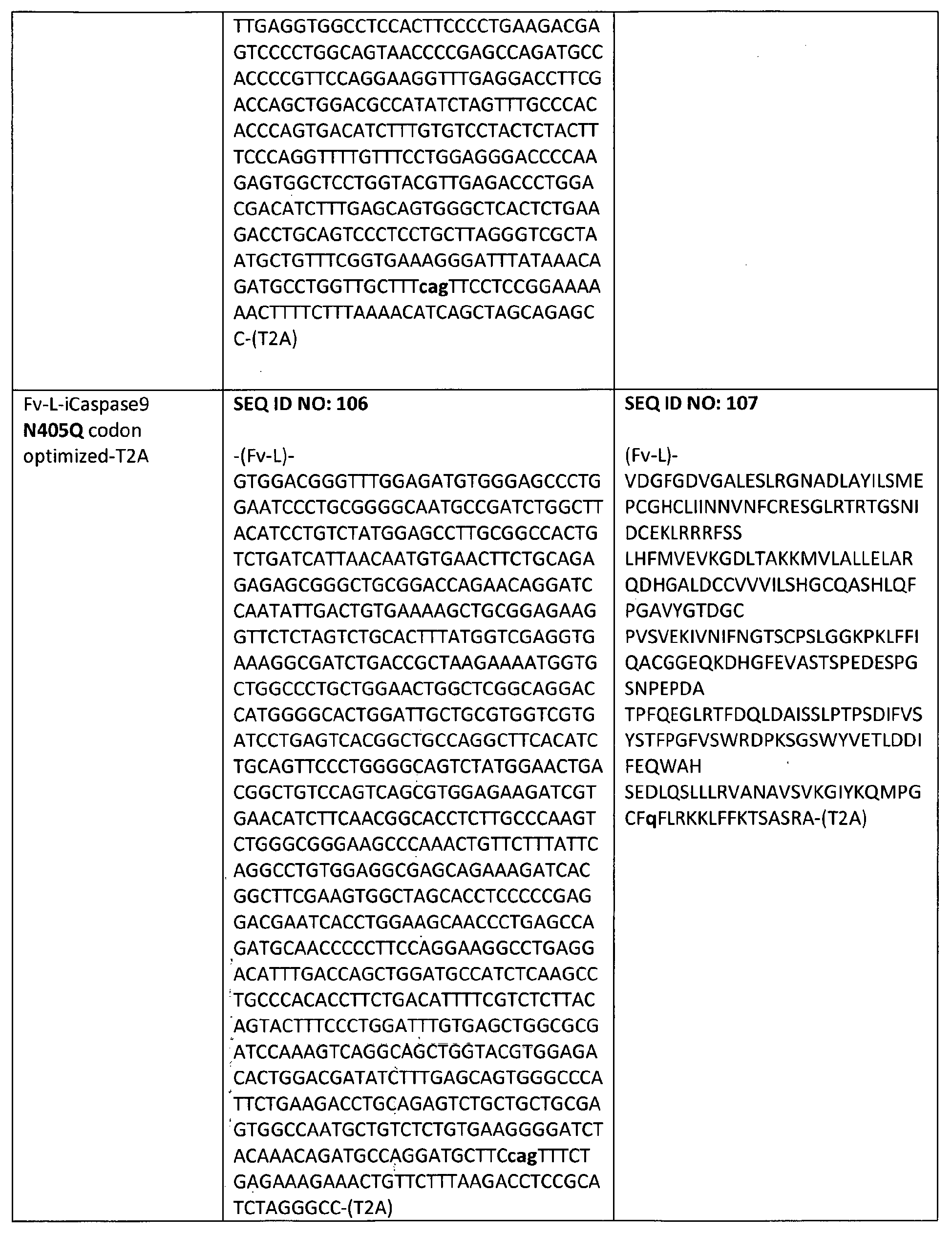

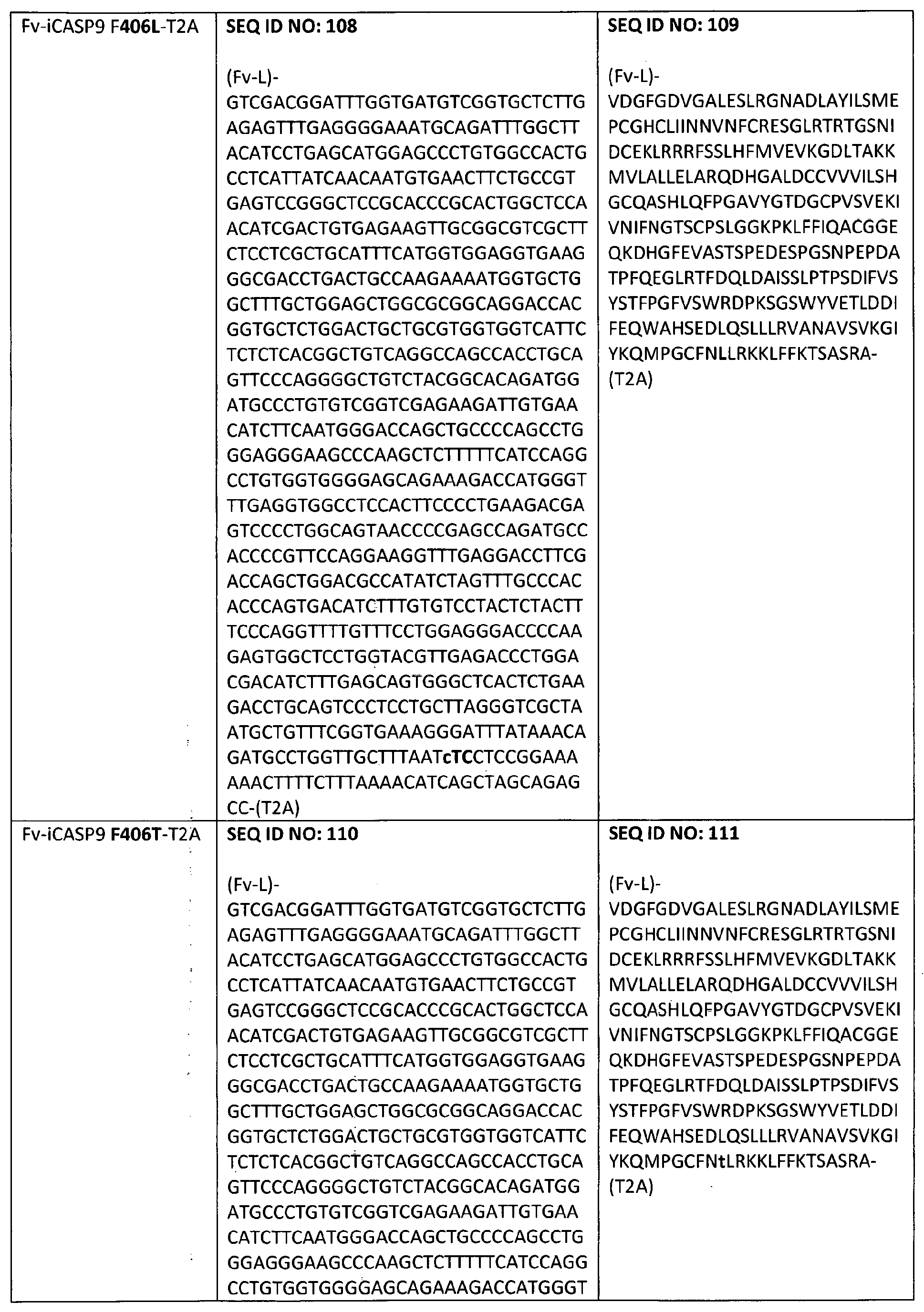

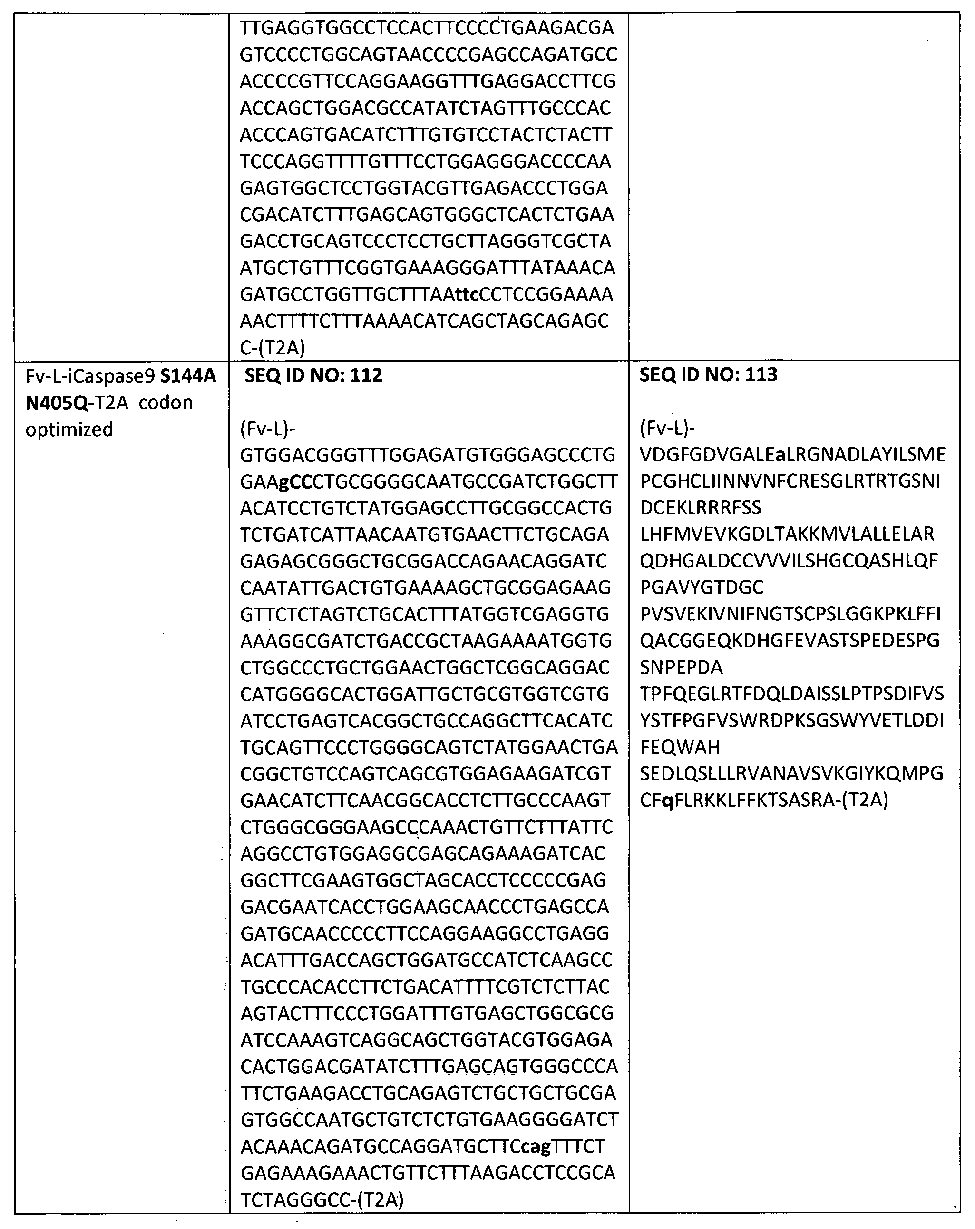

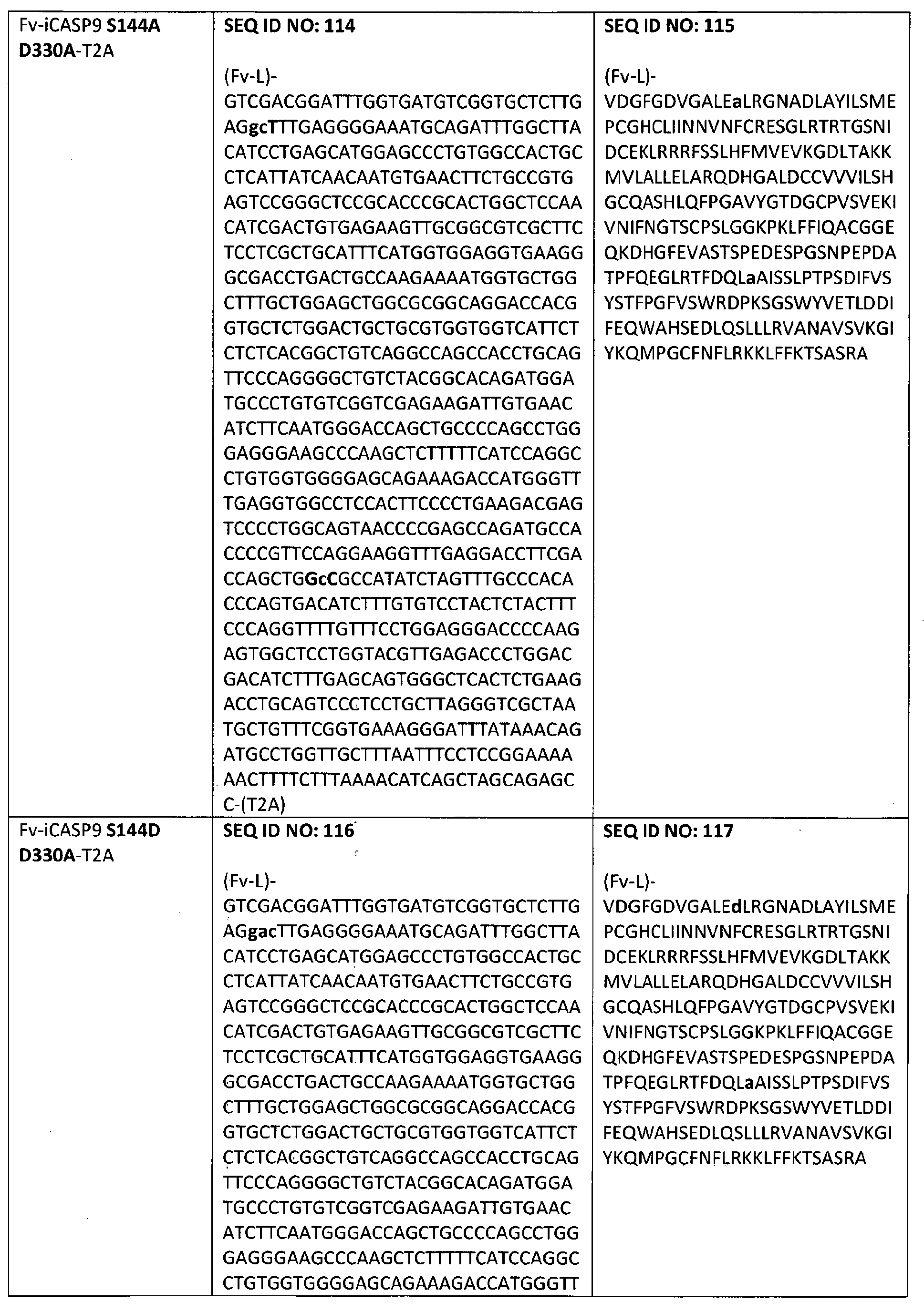

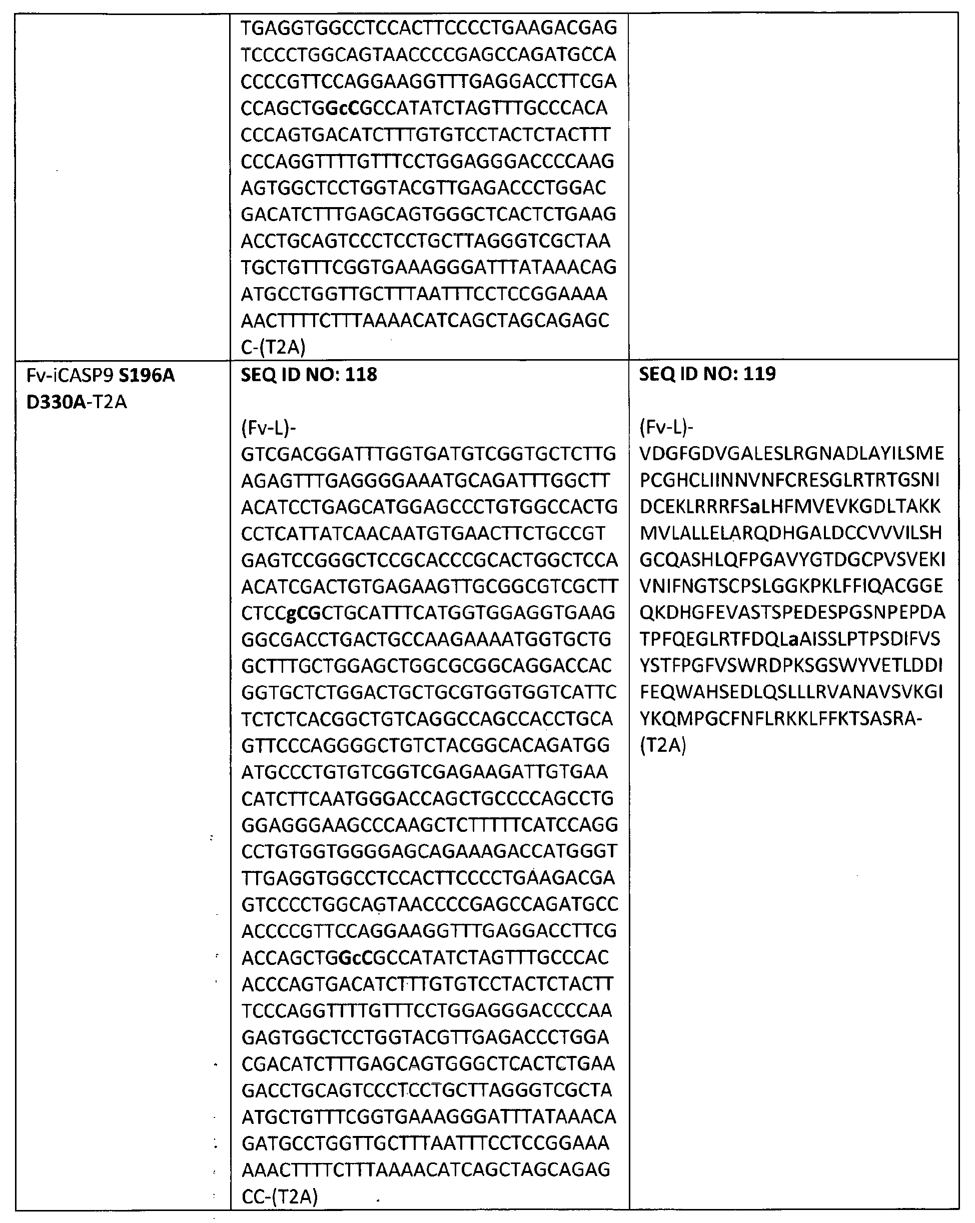

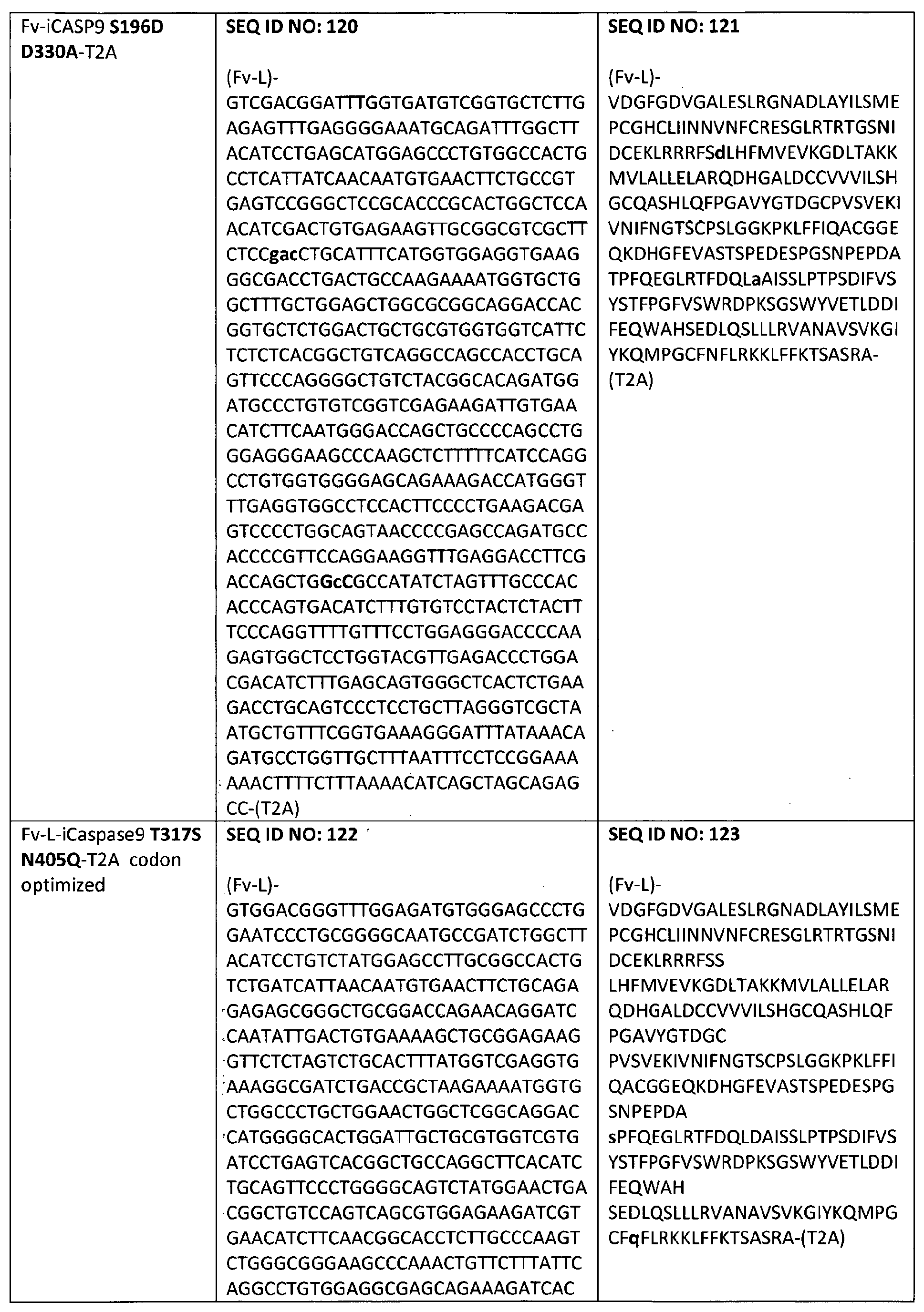

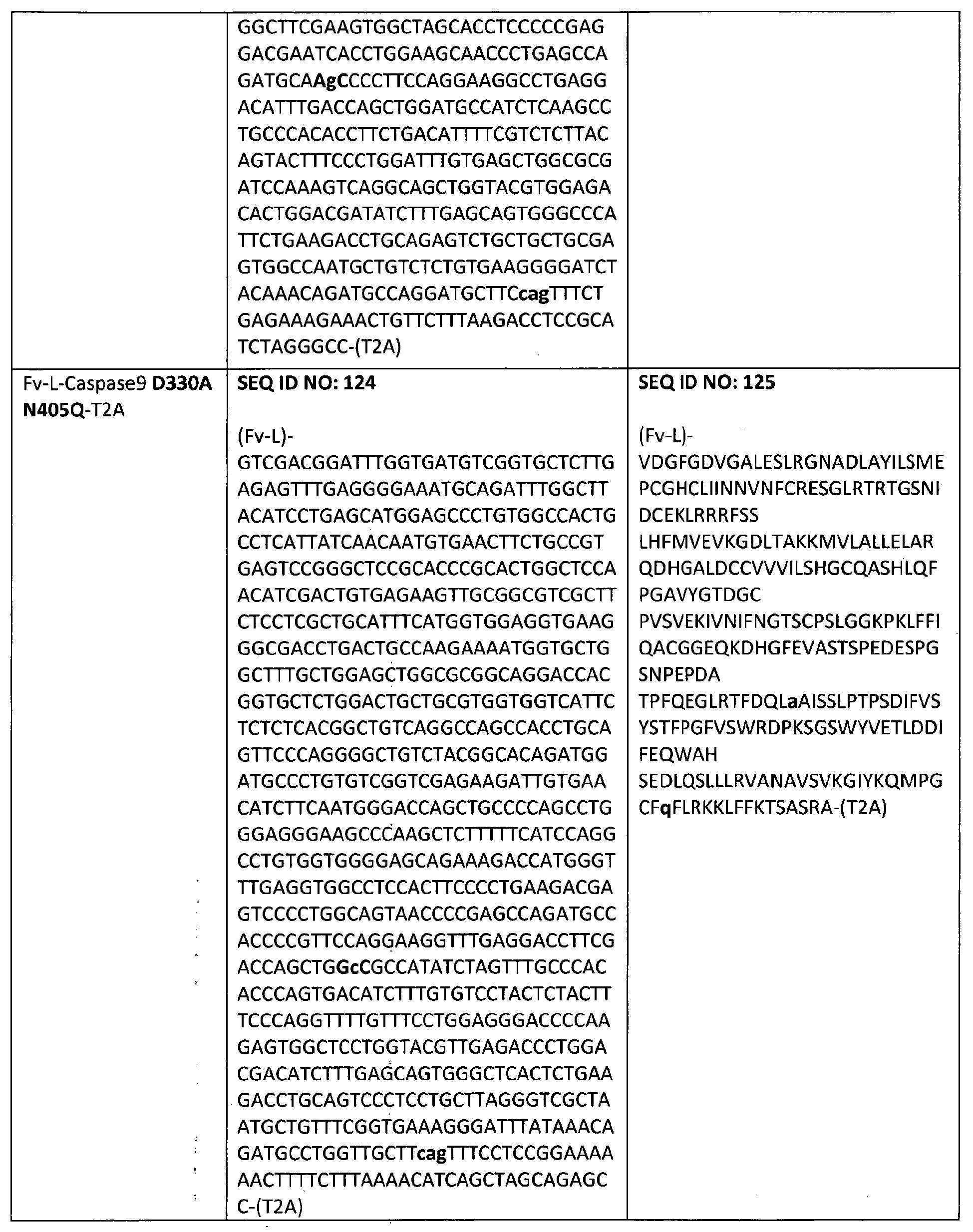

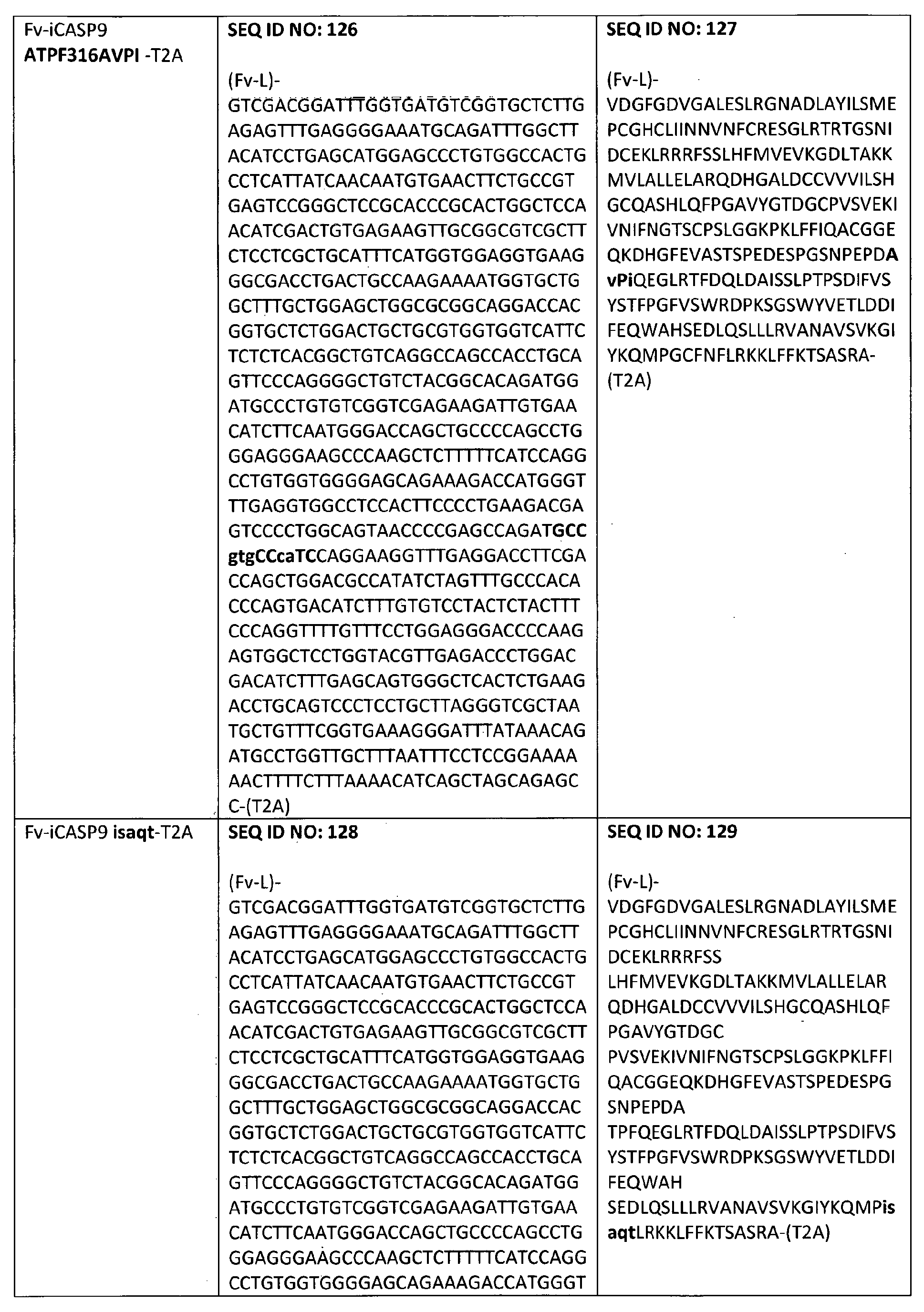

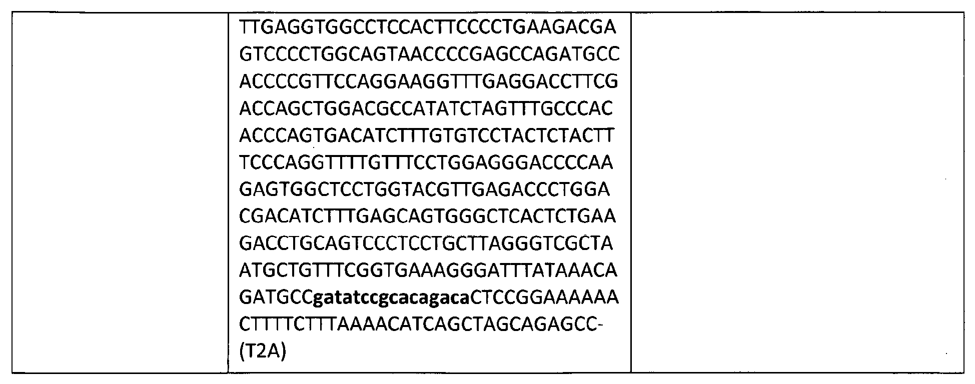

MPPPRLLFFLLFLTPMEVRPEEPLVVKVEEGDNAVLQCLKGTSDGPTQQLTWSRES
PLKPFLKLSLGLPGLGIHMRPLAIWLFIFNVSQQMGGFYLCQPGPPSEKAWQPGWT
VNVEGSGELFRWNVSDLGGLGCGLKNRSSEGPSSPSGKLMSPKLYVWAKDRPEI
WEGEPPCLPPRDSLNQSLSQDLTMAPGSTLWLSCGVPPDSVSRGPLSWTHVHPK
GPKSLLSLELKDDRPARDMWVMETGLLLPRATAQDAGKYYCHRGNLTMSFHLEITA
RPVLWHWLLRTGGWKVSAVTLAYLIFCLCSLVGILHLQRALVLRRKRKRMTDPTRR
F*

Partial sequence of a plasmid insert coding for a polypeptide that encodes an inducible Caspase-9 polypeptide and a chimeric antigen receptor that binds to CD19, separated by a 2A linker, wherein the two Caspase-9 polypeptide and the chimeric antigen receptor are separated during translation. The example of a chimeric antigen receptor provided herein may be further modified by including costimulatory polypeptides such as, for example, but not limited to, CD28, 4-1BB and OX40. The inducible Caspase-9 polypeptide provided herein may be substituted by an inducible modified Caspase-9 polypeptide, such as, for example, those provided herein.
SEQ ID NO: 130 FKBPv36 
ATGCTGGAGGGAGTGCAGGTGGAGACTATTAGCCCCGGAGATGGCAGAACATT
CCCCAAAAGAGGACAGACTTGCGTCGTGCATTATACTGGAATGCTGGAAGACG
GCAAGAAGGTGGACAGCAGCCGGGACCGAAACAAGCCCTTCAAGTTCATGCTG
GGGAAGCAGGAAGTGATCCGGGGCTGGGAGGAAGGAGTCGCACAGATGTCAG
TGGGACAGAGGGCCAAACTGACTATTAGCCCAGACTACGCTTATGGAGCAACC
GGCCACCCCGGGATCATTCCCCCTCATGCTACACTGGTCTTCGATGTGGAGCT
GCTGAAGCTGGAA
SEQ ID NO: 131 FKBPv36 
MLEGVQVETISPGDGRTFPKRGQTCVVHYTGMLEDGKKVDSSRDRNKPFKFMLGK
QEVIRGWEEGVAQMSVGQRAKLTISPDYAYGATGHPGIIPPHATLVFDVELLKLE
SEQ ID NO: 132 Linker
   AGCGGAGGAGGATCCGGA
SEQ ID NO: 133 Linker
   SGGGSG
SEQ ID NO: 134 Caspase-9 
GTGGACGGGTTTGGAGATGTGGGAGCCCTGGAATCCCTGCGGGGCAATGCCG
ATCTGGCTTACATCCTGTCTATGGAGCCTTGCGGCCACTGTCTGATCATTAACA
ATGTGAACTTCTGCAGAGAGCGGGCTGCGGACCAGAACAGGATCCAATATT
GACTGTGAAAAGCTGCGGAGAAGGTTCTCTAGTCTGCACTTTATGGTCGAGGTG
AAAGGCGATCTGACCGCTAAGAAAATGGTGCTGGCCCTGCTGGAACTGGCTCG
GCAGGACCATGGGGCACTGGATTGCTGCGTGGTCGTGATCCTGAGTCACGGCT
GCCAGGCTTCACATCTGCAGTTCCCTGGGGCAGTCTATGGAACTGACGGCTGT
CCAGTCAGCGTGGAGAAGATCGTGAACATCTTCAACGGCACCTCTTGCCCAAGT
CTGGGCGGGAAGCCCAAACTGTTCTTTATTCAGGCCTGTGGAGGCGAGCAGAA
AGATCACGGCTTCGAAGTGGCTAGCACCTCCCCCGAGGACGAATCACCTGGAA
GCAACCCTGAGCCAGATGCAACCCCCTTCCAGGAAGGCCTGAGGACATTTGAC
CAGCTGGATGCCATCTCAAGCCTGCCCACACCTTCTGACATTTTCGTCTCTTAC
AGTACTTTCCCTGGATTTGTGAGCTGGCGCGATCCAAAGTCAGGCAGCTGGTAC
GTGGAGACACTGGACGATATCTTTGAGCAGTGGGCCCATTCTGAAGACCTGCA
GAGTCTGCTGCTGCGAGTGGCCAATGCTGTCTCTGTGAAGGGGATCTACAAAC
AGATGCCAGGATGCTTCAACTTTCTGAGAAAGAAACTGTTCTTTAAGACCTCCG
CATCTAGGGCC
SEQ ID NO: 135 Caspase-9 
VDGFGDVGALESLRGNADLAYILSMEPCGHCLIINNVNFCRESGLRTRTGSNIDCEK
LRRRFSSLHFMVEVKGDLTAKKMVLALLELARQDHGALDCCVVVILSHGCQASHLQ
FPGAVYGTDGCPVSVEKIVNIFNGTSCPSLGGKPKLFFIQACGGEQKDHGFEVAST
SPEDESPGSNPEPDATPFQEGLRTFDQLDAISSLPTPSDIFVSYSTFPGFVSWRDPK
SGSWYVETLDDIFEQWAHSEDLQSLLLRVANAVSVKGIYKQMPGCFNFLRKKLFFK
TSASRA
SEQ ID NO: 136 Linker
   CCGCGG
SEQ ID NO: 137 Linker
   PR
SEQ ID NO: 138 T2A 
GAAGGCCGAGGGAGCCTGCTGACATGTGGCGATGTGGAGGAAAACCCAGGAC
CA
SEQ ID NO: 139 T2A
   EGRGSLLTCGDVEENPGP
SEQ ID NO: 140 Linker
   CCATGG
SEQ ID NO: 141 Linker
   PW
SEQ ID NO: 142 Signal peptide 
ATGGAGTTTGGACTTTCTTGGTTGTTTTTGGTGGCAATTCTGAAGGGTGTCCAGT
GTAGCAGG
SEQ ID NO: 143 Signal peptide
   MEFGLSWLFLVAILKGVQCSR
SEQ ID NO: 144 FMC63 variable light chain (anti-CD19) 
GACATCCAGATGACACAGACTACATCCTCCCTGTCTGCCTCTCTGGGAGACAGA
GTCACCATCAGTTGCAGGGCAAGTCAGGACATTAGTAAATATTTAAATTGGTATC
AGCAGAAACCAGATGGAACTGTTAAACTCCTGATCTACCATACATCAAGATTACA
CTCAGGAGTCCCATCAAGGTTCAGTGGCAGTGGGTCTGGAACAGATTATTCTCT
CACCATTAGCAACCTGGAGCAAGAAGATATTGCCACTTACTTTTGCCAACAGGG
TAATACGCTTCCGTACACGTTCGGAGGGGGGACTAAGTTGGAAATAACA
SEQ ID NO: 145 FMC63 variable light chain (anti CD19) 
DIQMTQTTSSLSASLGDRVTISCRASQDISKYLNWYQQKPDGTVKLLIYHTSRLHSG
VPSRFSGSGSGTDYSLTISNLEQEDIATYFCQQGNTLPYTFGGGTKLEIT
SEQ ID NO: 146 Flexible linker
   GGCGGAGGAAGCGGAGGTGGGGGC
SEQ ID NO: 147 Flexible linker
   GGGSGGGG
SEQ ID NO: 148 FMC63 variable heavy chain (anti-CD19) 
GAGGTGAAACTGCAGGAGTCAGGACCTGGCCTGGTGGCGCCCTCACAGAGCC
TGTCCGTCACATGCACTGTCTCAGGGGTCTCATTACCCGACTATGGTGTAAGCT
GGATTCGCCAGCCTCCACGAAAGGGTCTGGAGTGGCTGGGAGTAATATGGGGT
AGTGAAACCACATACTATAATTCAGCTCTCAAATCCAGACTGACCATCATCAAGG
ACAACTCCAAGAGCCAAGTTTTCTTAAAAATGAACAGTCTGCAAACTGATGACAC
AGCCATTTACTACTGTGCCAAACATTATTACTACGGTGGTAGCTATGCTATGGAC
TACTGGGGTCAAGGAACCTCAGTCACCGTCTCCTCA
SEQ ID NO: 149 FMC63 variable heavy chain (anti CD19) 
EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWG
QGTSVTVSS
SEQ ID NO: 150 Linker
   GGATCC
SEQ ID NO: 151 Linker
   GS
SEQ ID NO: 152 CD34 minimal epitope
   GAACTTCCTACTCAGGGGACTTTCTCAAACGTTAGCACAAACGTAAGT
SEQ ID NO: 153 CD34 minimal epitope
   ELPTQGTFSNVSTNVS
SEQ ID NO: 154 CD8 α stalk domain 
CCCGCCCCAAGACCCCCCACACCTGCGCCGACCATTGCTTCTCAACCCCTGAG
TTTGAGACCCGAGGCCTGCCGGCCAGCTGCCGGCGGGGCCGTGCATACAAGA
GGACTCGATTTCGCTTGCGAC
SEQ ID NO: 155 CD8 α stalk domain
   PAPRPPTPAPTIASQPLSLRPEACRPAAGGAVHTRGLDFACD
SEQ ID NO: 156 CD8 α transmembrane domain 
ATCTATATCTGGGCACCTCTCGCTGGCACCTGTGGAGTCCTTCTGCTCAGCCTG
GTTATTACTCTGTACTGTAATCACCGGAATCGCCGCCGCGTTTGTAAGTGTCCC
AGG
SEQ ID NO: 157 CD8 α transmembrane domain
   IYIWAPLAGTCGVLLLSLVITLYCNHRNRRRVCKCPR
SEQ ID NO: 158 Linker
   GTCGAC
SEQ ID NO: 159 Linker
   VD
SEQ ID NO: 160 CD3 zeta 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
CCTTTACCAGGGTCTCAGTACAGCCACCAAGGACACCTACGACGCCCTTCACAT
GCAGGCCCTGCCCCCTCGC
SEQ ID NO: 161 CD3 zeta

RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR

Provided below is an example of a plasmid insert coding for a chimeric antigen receptor that binds to Her2/Neu. The chimeric antigen receptor may be further modified by including costimulatory polypeptides such as, for example, but not limited to, CD28, OX40, and 4-1BB.
SEQ ID NO: 162 Signal peptide 
ATGGAGTTTGGACTTTCTTGGTTGTTTTGGTGGCAATTCTGAAGGGTGTCCAGT
GTAGCAGG
SEQ ID NO: 163 Signal peptide
   MEFGLSWLFLVAILKGVQCSR
SEQ ID NO: 164 FRP5 variable light chain (anti-Her2) 
GACATCCAATTGACACAATCACACAAATTTCTCTCAACTTCTGTAGGAGACAGAG
TGAGCATAACCTGCAAAGCATCCCAGGACGTGTACAATGCTGTGGCTTGGTACC
AACAGAAGCCTGGACAATCCCCAAAATTGCTGATTTATTCTGCCTCTAGTAGGTA
CACTGGGGTACCTTCTCGGTTTACGGCTCTGGGTCCGGACCAGATTTCACGTT
CACAATCAGTTCCGTTCAAGCTGAAGACCTCGCTGTTTATTTTTGCCAGCAGCA
CTTCCGAACCCCTTTTACTTTTGGCTCAGGCACTAAGTTGGAAATCAAGGCTTTG
SEQ ID NO: 165 FRP5 variable light chain (anti-Her2) 
DIQLTQSHKFLSTSVGDRVSITCKASQDVYNAVAWYQQKPGQSPKLLIYSASSRYT
GVPSRFTGSGSGPDFTFTISSVQAEDLAVYFCQQHFRTPFTFGSGTKLEIKAL
SEQ ID NO: 166 Flexible linker
   GGCGGAGGAAGCGGAGGTGGGGGC
SEQ ID NO: 167 Flexible linker
   GGGSGGGG
SEQ ID NO: 168 FRP5 variable heavy chain (anti-Her2/Neu) 
GAAGTCCAATTGCAACAGTCAGGCCCCGAATTGAAAAAGCCCGGCGAAACAGT
GAAGATATCTTGTAAAGCCTCCGGTTACCCTTTTACGAACTATGGAATGAACTGG
GTCAAACAAGCCCCTGGACAGGGATTGAAGTGGATGGGATGGATCAATACATC
AACAGGCGAGTCTACCTTCGCAGATGATTTCAAAGGTCGCTTTGACTTCTCACT
GGAGACCAGTGCAAATACCGCCTACCTTCAGATTAACAATCTTAAAGCGAGGA
TATGGCAACCTACTTTTGCGCAAGATGGGAAGTTTATCACGGGTACGTGCCATA
CTGGGGACAAGGAACGACAGTGACAGTTAGTAGC
SEQ ID NO: 169 FRP5 variable heavy chain (anti-Her2/Neu) 
EVQLQQSGPELKKPGETVKISCKASGYPFTNYGMNWVKQAPGQGLKWMGWINTS
TGESTFADDFKGRFDFSLETSANTAYLQINNLKSEDMATYFCARWEVYHGYVPYW
GQGTTVTVSS
SEQ ID NO: 170 Linker
   GGATCC
SEQ ID NO: 171 Linker
   GS
SEQ ID NO: 172 CD34 minimal epitope
   GAACTTCCTACTCAGGGGACTTTCTCAAACGTTAGCACAAACGTAAGT
SEQ ID NO: 173 CD34 minimal epitope
   ELPTQGTFSNVSTNVS
SEQ ID NO: 174 CD8 alpha stalk 
CCCGCCCCAAGACCCCCCACACCTGCGCCGACCATTGCTTCTCAACCCCTGAG
TTTGAGACCCGAGGCCTGCCGGCCAGCTGCCGGCGGGGCCGTGCATACAAGA
GGACTCGATTTCGCTTGCGAC
SEQ ID NO: 175 CD8 alpha stalk
   PAPRPPTPAPTIASQPLSLRPEACRPAAGGAVHTRGLDFACD
SEQ ID NO: 176 CD8 alpha transmembrane region 
ATCTATATCTGGGCACCTCTCGCTGGCACCTGTGGAGTCCTTCTGCTCAGCCTG
GTTATTACTCTGTACTGTAATCACCGGAATCGCCGCCGCGTTTGTAAGTGTCCC
AGG
SEQ ID NO: 177 CD8 alpha transmembrane region
   IYIWAPLAGTCGVLLLSLVITLYCNHRNRRRVCKCPR
SEQ ID NO: 178 Linker
   Ctcgag
SEQ ID NO: 179 Linker
   LE
SEQ ID NO: 180 CD3 zeta cytoplasmic domain 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
 
CCTTTACCAGGGTCTCAGTACAGCCACCAAGGACACCTACGACGCCCTTCACAT
GCAGGCCCTGCCCCCTCGC
SEQ ID NO: 181 CD3 zeta cytoplasmic domain

RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR

### Additional sequences

SEQ ID NO: 182, CD28 nt 
TTCTGGGTACTGGTTGTAGTCGGTGGCGTACTTGCTTGTTATTCTCTTCTTGTTA
CCGTAGCCTTCATTATATTCTGGGTCCGATCAAAGCGCTCAAGACTCCTCCATT
CCGATTATATGAACATGACACCTCGCCGACCTGGTCCTACACGCAAACATTATC
AACCCTACGCACCCCCCCGAGACTTCGCTGCTTATCGATCC
SEQ ID NO: 183, CD28 aa 
FWVLVVVGGVLACYSLLVTVAFIIFWVRSKRSRLLHSDYMNMTPRRPGPTRKHYQP
YAPPRDFAAYRS
SEQ ID NO: 184, OX40 nt 
GTTGCCGCCATCCTGGGCCTGGGCCTGGTGCTGGGGCTGCTGGGCCCCCTGG
CCATCCTGCTGGCCCTGTACCTGCTCCGGGACCAGAGGCTGCCCCCCGATGCC
CACAAGCCCCCTGGGGGAGGCAGTTTCCGGACCCCCATCCAAGAGGAGCAGG
CCGACGCCCACTCCACCCTGGCCAAGATC
SEQ ID NO: 185, OX40 aa 
VAAILGLGLVLGLLGPLAILLALYLLRRDQRLPPDAHKPPGGGSFRTPIQEEQADAHS
TLAKI
SEQ ID NO: 186, 4-1BB nt 
AGTGTAGTTAAAAGAGGAAGAAAAAAGTTGCTGTATATATTTAAACAACCATTTAT
GAGACCAGTGCAAACCACCCAAGAAGAAGACGGATGTTCATGCAGATTCCCAG
AAGAAGAAGAAGGAGGATGTGAATTG
SEQ ID NO: 187, 4-1BB aa
   SVVKRGRKKLLYIFKQPFMRPVQTTQEEDGCSCRFPEEEEGGCEL

### Expression of MyD88/CD40 Chimeric Antigen Receptors and Chimeric Stimulating Molecules

The following examples discuss the compositions and methods relating to MyD88/CD40 chimeric antigen receptors and chimeric stimulating molecules, as provided in this application. Also included are compositions and methods related to a Caspase-9-based safety switch, and its use in cells that express the MyD88/CD40 chimeric antigen receptors or chimeric stimulating molecules.

### Example 11: Design and Activity of MyD88/CD40 Chimeric Antigen Receptors

### Design of MC-CAR constructs

Based on the activation data from inducible MyD88/CD40 experiments, the potential of MC signaling in a CAR molecule in place of conventional endodomains (e.g., CD28 and 4-1BB) was examined. MC (without AP1903-binding FKBPv36 regions) was subcloned into the PSCA.ζ to emulate the position of the CD28 endodomain. Retrovirus was generated for each of the three constructs, transduced human T cells and subsequently measured transduction efficiency demonstrating that PSCA.MC.ζ could be expressed. To confirm that T cells bearing each of these CAR constructs retained their ability to recognize PSCA⁺ tumor cells, 6-hour cytotoxicity assays were performed, which showed lysis of Capan-1 target cells. Therefore, the addition of MC into the cytoplasmic region of a CAR molecule does not affect CAR expression or the recognition of antigen on target cells.

### MC costimulation enhances T cell killing, proliferation and survival in CAR-modified T cells

As demonstrated in short-term cytotoxicity assays, each of the three CAR designs showed the capacity to recognize and lyse Capan-1 tumor cells. Cytolytic effector function in effector T cells is mediated by the release of pre-formed granzymes and perforin following tumor recognition, and activation through CD3ζ is sufficient to induce this process without the need for costimulation. First generation CAR T cells (e.g., CARs constructed with only the CD3ζ cytoplasmic region) can lyse tumor cells; however, survival and proliferation is impaired due to lack of costimulation. Hence, the addition of CD28 or 4-1BB co-stimulating domains constructs has significantly improved the survival and proliferative capacity of CAR T cells.

To examine whether MC can similarly provide costimulating signals affecting survival and proliferation, coculture assays were performed with PSCA⁺Capan-1 tumor cells under high tumor:T cell ratios (1:1, 1:5, 1:10 T cell to tumor cell). When T cell and tumor cell numbers were equal (1:1), there was efficient killing of Capan-1-GFP cells from all three constructs compared to non-transduced control T cells. However, when the CAR T cells were challenged with high numbers of tumor cells (1:10), there was a significant reduction of Capan-1-GFP tumor cells only when the CAR molecule contained either MC or CD28.
To further examine the mechanism of costimulation by these two CARs cell viability and proliferation was assayed. PSCA CARs containing MC or CD28 showed improved survival compared to non-transduced T cells and the CD3ζ only CAR, and T cell proliferation by PSCA.MC.ζ and PSCA.28.ζ was significantly enhanced. As other groups have shown that CARs that contain co-stimulating signaling regions produce IL-2, a key survival and growth molecule for T cells (4), ELISAs were performed on supernatants from CAR T cells challenged with Capan-1 tumor cells. Although PSCA.28.ζ produced high levels of IL-2, PSCA.MC.ζ signaling also produced significant levels of IL-2, which likely contributes to the observed T cell survival and expansion in these assays. Additionally, IL-6 production by CAR-modified T cells was examined, as IL-6 has been implicated as a key cytokine in the potency and efficacy of CAR-modified T cells (15). In contrast to IL-2, PSCA.MC.ζ produced higher levels of IL-6 compared to PSCA.28.ζ, consistent with the observations that iMC activation in primary T cells induces IL-6. Together, these data suggest that co-stimulation through MC produces similar effects to that of CD28, whereby following tumor cell recognition, CAR-modified T cells produce IL-2 and IL-6, which enhance T cell survival

Immunotherapy using CAR-modified T cells holds great promise for the treatment of a variety of malignancies. While CARs were first designed with a single signaling domain (e.g., CD3ζ,(16-19) clinical trials evaluating the feasibility of CAR immunotherapy showed limited clinical benefit.(1,2,20,21) This has been primarily attributed to the incomplete activation of T cells following tumor recognition, which leads to limited persistence and expansion in vivo.(22) To address this deficiency, CARs have been engineered to include another stimulating domain, often derived from the cytoplasmic portion of T cell costimulating molecules including CD28, 4-1BB, OX40, ICOS and DAP10, (4,23-30) which allow CAR T cells to receive appropriate costimulation upon engagement of the target antigen. Indeed, clinical trials conducted with anti-CD19 CARs bearing CD28 or 4-1BB signaling domains for the treatment of refractory acute lymphoblastic leukemia (ALL) have demonstrated impressive T cell persistence, expansion and serial tumor killing following adoptive transfer. (6-8)

CD28 costimulation provides a clear clinical advantage for the treatment of CD19⁺ lymphomas. Savoldo and colleagues conducted a CAR-T cell clinical trial comparing first (CD19.ζ) and second generation CARs (CD19.28.ζ) and found that CD28 enhanced T cell persistence and expansion following adoptive transfer.31 One of the principal functions of second generation CARs is the ability to produce IL-2 that supports T cell survival and growth through activation of the NFAT transcription factor by CD3ζ (signal 1), and NF-κB (signal 2) by CD28 or 4-1 BB.32 This suggested other molecules that similarly activated NF-κB might be paired with the CD3ζ chain within a CAR molecule. Our approach has employed a T cell costimulating molecule that was originally developed as an adjuvant for a dendritic cell (DC) vaccine.(12,33) For full activation or licensing of DCs, TLR signaling is usually involved in the upregulation of the TNF family member, CD40, which interacts with CD40L on antigen-primed CD4⁺ T cells. Because iMC was a potent activator of NF-κB in DCs, transduction of T cells with CARs that incorporated MyD88 and CD40 might provide the required costimulation (signal 2) to T cells, and enhance their survival and proliferation.

A set of experiments was performed to examine whether MyD88, CD40 or both components were required for optimum T cell stimulation using the iMC molecule. Remarkably, it was found that neither MyD88 nor CD40 could sufficiently induce T cell activation, as measured by cytokine production (IL-2 and IL-6), but when combined as a single fusion protein, could induce potent T cell activation. A PSCA CAR incorporating MC was constructed and subsequently compared its function against a first (PSCA.ζ) and second generation (PSCA.28.ζ) CAR. Here it was found that MC enhanced survival and proliferation of CAR T cells to a comparable level as the CD28 endodomain, suggesting that costimulation was sufficient. While PSCA.MC.ζ CAR-transduced T cells produced lower levels of IL-2 than PSCA.28.ζ, the secreted levels were significantly higher than non-transduced T cells and T cells transduced with the PSCA.ζ CAR. On the other hand, PSCA.MC.ζ CAR-transduced T cells secreted significantly higher levels of IL-6, an important cytokine associated with T cell activation, than PSCA.28.ζ transduced T cells, indicating that MC conferred unique properties to CAR function that may translate to improved tumor cell killing in vivo. These experiments indicate that MC can activate NF-κB (signal 2) following antigen recognition by the extracellular CAR domain.

Design and functional validation of MC-CAR. Three PSCA CAR constructs were designed incorporating only CD3ζ, or with CD28 or MC endodomains. Transduction efficiency (percentage) was measured by anti-CAR-APC (recognizing the IgG1 CH₂CH₃ domain). C) Flow cytometry analysis demonstrating high transduction efficiency of T cells with PSCA.MC.ζ CAR. D) Analysis of specific lysis of PSCA⁺ Capan-1 tumor cells by CAR-modified T cells in a 6 hour LDH release assay at a ratio of 1:1 T cells to tumor cells.

MC-CAR modified T cells kill Capan-1 tumor cells in long-term coculture assays. Flow cytometric analysis of CAR-modified and non-transduced T cells cultured with Capan-1-GFP tumor cells after 7 days in culture at a 1:1 ratio. Quantitation of viable GFP⁺ cells by flow cytometry in coculture assays at a 1:1 and 1:10 T cell to tumor cell ratio.

MC and CD28 costimulation enhance T cell survival, proliferation and cytokine production. T cells isolated from 1:10 T cell to tumor cell coculture assays were assayed for cell viability and cell number to assess survival and proliferation in response to tumor cell exposure. Supernatants from coculture assays were subsequently measured for IL-2 and IL-6 production by ELISA.

Design of inducible costimulating molecules and effect on T cell activation. Four vectors were designed incorporating FKBPv36 AP1903-binding domains (Fv'.Fv) alone, or with MyD88, CD40 or the MyD88/CD40 fusion protein. Transduction efficiency of primary activated T cells using CD3⁺CD19⁺ flow cytometric analysis. Analysis of IFN-γ production of modified T cells following activation with and without 10 nM AP1903. Analysis of IL-6 production of modified T cells following activation with and without 10 nM AP1903.

Apart from survival and growth advantages, MC-induced costimulation may also provide additional functions to CAR-modified T cells. Medzhitov and colleagues recently demonstrated that MyD88 signaling was critical for both Th1 and Th17 responses and that it acted via IL-1 to render CD4⁺ T cells refractory to regulatory T cell (Treg)-driven inhibition.(34) Experiments with iMC show that IL-1α and β are secreted following AP1903 activation. In addition, Martin et al demonstrated that CD40 signaling in CD8⁺ T cells via Ras, PI3K and protein kinase C, result in NF-κB-dependent induction of cytotoxic mediators granzyme and perforin that lyse CD4⁺CD25⁺ Treg cells (35). Thus, MyD88 and CD40 co-activation may render CAR-T cells resistant to the immunosuppressive effects of Treg cells, a function that could be critically important in the treatment of solid tumors and other types of cancers.

In summary, MC can be incorporated into a CAR molecule and primary T cells transduced with retrovirus can express PSCA.MC.ζ without overt toxicity or CAR stability issues. Further, MC appears to provide similar costimulation to that of CD28, where transduced T cells show improved survival, proliferation and tumor killing compared to T cells transduced with a first generation CAR.

### Example 12: References

The following references are cited in, or provide additional information that may be relevant, including, for example, in Example 11.
1. Till BG, Jensen MC, Wang J, et al: CD20-specific adoptive immunotherapy for lymphoma using a chimeric antigen receptor with both CD28 and 4-1BB domains: pilot clinical trial results. Blood 119:3940-50, 2012.
2. Pule MA, Savoldo B, Myers GD, et al: Virus-specific T cells engineered to coexpress tumor-specific receptors: persistence and antitumor activity in individuals with neuroblastoma. Nat Med 14:1264-70, 2008.
3. Kershaw MH, Westwood JA, Parker LL, et al: A phase 1 study on adoptive immunotherapy using gene-modified T cells for ovarian cancer. Clin Cancer Res 12:6106-15, 2006.
4. Carpenito C, Milone MC, Hassan R, et al: Control of large, established tumor xenografts with genetically retargeted human T cells containing CD28 and CD137 domains. Proc Natl Acad Sci U S A 106:3360-5, 2009.
5. Song DG, Ye Q, Poussin M, et al: CD27 costimulation augments the survival and antitumor activity of redirected human T cells in vivo. Blood 119:696-706, 2012.
6. Kalos M, Levine BL, Porter DL, et al: T cells with chimeric antigen receptors have potent antitumor effects and can establish memory in patients with advanced leukemia. Sci Transl Med 3:95ra73, 2011.
7. Porter DL, Levine BL, Kalos M, et al: Chimeric antigen receptor-modified T cells in chronic lymphoid leukemia. N Engl J Med 365:725-33, 2011.
8. Brentjens RJ, Davila ML, Riviere I, et al: CD19-targeted T cells rapidly induce molecular remissions in adults with chemotherapy-refractory acute lymphoblastic leukemia. Sci Transl Med 5:177ra38, 2013.
9. Pule MA, Straathof KC, Dotti G, et al: A chimeric T cell antigen receptor that augments cytokine release and supports clonal expansion of primary human T cells. Mol Ther 12:933-41, 2005.
10. Finney HM, Akbar AN, Lawson AD: Activation of resting human primary T cells with chimeric receptors: costimulation from CD28, inducible costimulator, CD134, and CD137 in series with signals from the TCR zeta chain. J Immunol 172:104-13,2004.
11. Guedan S, Chen X, Madar A, et al: ICOS-based chimeric antigen receptors program bipolar TH17/TH1 cells. Blood, 2014.
12. Narayanan P, Lapteva N, Seethammagari M, et al: A composite MyD88/CD40 switch synergistically activates mouse and human dendritic cells for enhanced antitumor efficacy. J Clin Invest 121:1524-34, 2011.
13. Anurathapan U, Chan RC, Hindi HF, et al: Kinetics of tumor destruction by chimeric antigen receptor-modified T cells. Mol Ther 22:623-33, 2014.
14. Craddock JA, Lu A, Bear A, et al: Enhanced tumor trafficking of GD2 chimeric antigen receptor T cells by expression of the chemokine receptor CCR2b. J Immunother 33:780-8, 2010.
15. Lee DW, Gardner R, Porter DL, et al: Current concepts in the diagnosis and management of cytokine release syndrome. Blood 124:188-95, 2014.
16. Becker ML, Near R, Mudgett-Hunter M, et al: Expression of a hybrid immunoglobulin-T cell receptor protein in transgenic mice. Cell 58:911-21, 1989.
17. Goverman J, Gomez SM, Segesman KD, et al: Chimeric immunoglobulin-T cell receptor proteins form functional receptors: implications for T cell receptor complex formation and activation. Cell 60:929-39, 1990.
18. Gross G, Waks T, Eshhar Z: Expression of immunoglobulin-T-cell receptor chimeric molecules as functional receptors with antibody-type specificity. Proc Natl Acad Sci U S A 86:10024-8, 1989.
19. Kuwana Y, Asakura Y, Utsunomiya N, et al: Expression of chimeric receptor composed of immunoglobulin-derived V regions and T-cell receptor-derived C regions. Biochem Biophys Res Commun 149:960-8, 1987.
20. Jensen MC, Popplewell L, Cooper LJ, et al: Antitransgene rejection responses contribute to attenuated persistence of adoptively transferred CD20/CD19-specific chimeric antigen receptor redirected T cells in humans. Biol Blood Marrow Transplant 16:1245-56, 2010.
21. Park JR, Digiusto DL, Slovak M, et al: Adoptive transfer of chimeric antigen receptor re-directed cytolytic T lymphocyte clones in patients with neuroblastoma. Mol Ther 15:825-33, 2007.
22. Ramos CA, Dotti G: Chimeric antigen receptor (CAR)-engineered lymphocytes for cancer therapy. Expert Opin Biol Ther 11:855-73, 2011.
23. Finney HM, Lawson AD, Bebbington CR, et al: Chimeric receptors providing both primary and costimulatory signaling in T cells from a single gene product. J Immunol 161:2791-7, 1998.
24. Hombach A, Wieczarkowiecz A, Marquardt T, et al: Tumor-specific T cell activation by recombinant immunoreceptors: CD3 zeta signaling and CD28 costimulation are simultaneously required for efficient IL-2 secretion and can be integrated into one combined CD28/CD3 zeta signaling receptor molecule. J Immunol 167:6123-31, 2001.
25. Maher J, Brentjens RJ, Gunset G, et al: Human T-lymphocyte cytotoxicity and proliferation directed by a single chimeric TCRzeta /CD28 receptor. Nat Biotechnol 20:70-5, 2002.
26. Imai C, Mihara K, Andreansky M, et al: Chimeric receptors with 4-1BB signaling capacity provoke potent cytotoxicity against acute lymphoblastic leukemia. Leukemia 18:676-84, 2004.
27. Wang J, Jensen M, Lin Y, et al: Optimizing adoptive polyclonal T cell immunotherapy of lymphomas, using a chimeric T cell receptor possessing CD28 and CD137 costimulatory domains. Hum Gene Ther 18:712-25, 2007.
28. Zhao Y, Wang QJ, Yang S, et al: A herceptin-based chimeric antigen receptor with modified signaling domains leads to enhanced survival of transduced T lymphocytes and antitumor activity. J Immunol 183:5563-74, 2009.
29. Milone MC, Fish JD, Carpenito C, et al: Chimeric receptors containing CD137 signal transduction domains mediate enhanced survival of T cells and increased antileukemic efficacy in vivo. Mol Ther 17:1453-64, 2009.
30. Yvon E, Del Vecchio M, Savoldo B, et al: Immunotherapy of metastatic melanoma using genetically engineered GD2-specific T cells. Clin Cancer Res 15:5852-60, 2009.
31. Savoldo B, Ramos CA, Liu E, et al: CD28 costimulation improves expansion and persistence of chimeric antigen receptor-modified T cells in lymphoma patients. J Clin Invest 121:1822-6, 2011.
32. Kalinski P, Hilkens CM, Wierenga EA, et al: T-cell priming by type-1 and type-2 polarized dendritic cells: the concept of a third signal. Immunol Today 20:561-7, 1999.
33. Kemnade JO, Seethammagari M, Narayanan P, et al: Off-the-shelf Adenoviral-mediated Immunotherapy via Bicistronic Expression of Tumor Antigen and iMyD88/CD40 Adjuvant. Mol Ther, 2012.
34. Schenten D, Nish SA, Yu S, et al: Signaling through the adaptor molecule MyD88 in CD4+ T cells is required to overcome suppression by regulatory T cells. Immunity 40:78-90, 2014.
35. Martin S, Pahari S, Sudan R, et al: CD40 signaling in CD8+CD40+ T cells turns on contra-T regulatory cell functions. J Immunol 184:5510-8, 2010

### Example 13: MC costimulation enhances function and proliferation of CD19 CARs

Experiments similar to those discussed herein, are provided, using an antigen recognition moiety that recognizes the CD19 antigen. It is understood that the vectors provided herein may be modified to construct a MyD88/CD40 CAR construct that targets CD19⁺ tumor cells, which also incorporates an inducible Caspase-9 safety switch.
To examine whether MC costimulation functioned in CARs targeting other antigens, T cells were modified with either CD19.ζ or with CD19.MC.ζ. The cytotoxicity, activation and survival against CD19+ Burkitt's lymphoma cell lines (Raji and Daudi) of the modified cells were assayed. In coculture assays, T cells transduced with either CAR showed killing of CD19+ Raji cells at an effector to target ratio as low as 1:1. However, analysis of cytokine production from co-culture assays showed that CD19.MC.ζ transduced T cells produced higher levels of IL-2 and IL-6 compared to CD19.ζ, which is consistent with the costimulatory effects observed with iMC and PSCA CARs containing the MC signaling domain. Further, T cells transduced with CD19.MC.ζ showed enhanced proliferation following activation by Raji tumor cells. These data support earlier experiments demonstrating that MC signaling in CAR molecules improves T cell activation, survival and proliferation following ligation to a target antigen expressed on tumor cells.

### pBP0526-SFG.iCasp9wt.2A.CD19scFv.CD34e.CD8stm.MC.zeta

SEQ ID NO: 116 FKBPv36 
ATGCTGGAGGGAGTGCAGGTGGAGACTATTAGCCCCGGAGATGGCAGAACATT
CCCCAAAAGAGGACAGACTTGCGTCGTGCATTATACTGGAATGCTGGAAGACG
GCAAGAAGGTGGACAGCAGCCGGGACCGAAACAAGCCCTTCAAGTTCATGCTG
GGGAAGCAGGAAGTGATCCGGGGCTGGGAGGAAGGAGTCGCACAGATGTCAG
TGGGACAGAGGGCCAAACTGACTATTAGCCCAGACTACGCTTATGGAGCAACC
 
GGCCACCCCGGGATCATTCCCCCTCATGCTACACTGGTCTTCGATGTGGAGCT
GCTGAAGCTGGAA
SEQ ID NO: 117 FKBPv36 
MLEGVQVETISPGDGRTFPKRGQTCVVHYTGMLEDGKKVDSSRDRNKPFKFMLGK
QEVIRGWEEGVAQMSVGQRAKLTISPDYAYGATGHPGIIPPHATLVFDVELLKLE
SEQ ID NO: 118 Linker
   AGCGGAGGAGGATCCGGA
SEQ ID NO: 119 Linker
   SGGGSG
SEQ ID NO: 120 Caspase-9 
GTGGACGGGTTTGGAGATGTGGAGCCCTGGAATCCCTGCGGGGCAATGCCG
ATCTGGCTTACATCCTGTCTATGGAGCCTTGCGGCCACTGTCTGATCATTAACA
ATGTGAACTTCTGCAGAGAGAGCGGGCTGCGGACCAGAACAGGATCCAATATT
GACTGTGAAAAGCTGCGGAGAAGGTTCTCTAGTCTGCACTTTATGGTCGAGGTG
AAAGGCGATCTGACCGCTAAGAAAATGGTGCTGGCCCTGCTGGAACTGGCTCG
GCAGGACCATGGGGCACTGGATTGCTGCGTGGTCGTGATCCTGAGTCACGGCT
GCCAGGCTTCACATCTGCAGTTCCCTGGGGCAGTCTATGGAACTGACGGCTGT
CCAGTCAGCGTGGAGAAGATCGTGAACATCTTCAACGGCACCTCTTGCCCAAGT
CTGGGCGGGAAGCCCAAACTGTTCTTTATTCAGGCCTGTGGAGGCGAGCAGAA
AGATCACGGCTTCGAAGTGGCTAGCACCTCCCCCGAGGACGAATCACCTGGAA
GCAACCCTGAGCCAGATGCAACCCCCTTCCAGGAAGGCCTGAGGACATTTGAC
CAGCTGGATGCCATCTCAAGCCTGCCCACACCTTCTGACATTTTCGTCTCTTAC
AGTACTTTCCCTGGATTTGTGAGCTGGCGCGATCCAAAGTCAGGCAGCTGGTAC
GTGGAGACACTGGACGATATCTTTGAGCAGTGGGCCCATTCTGAAGACCTGCA
GAGTCTGCTGCTGCGAGTGGCCAATGCTGTCTCTGTGAAGGGGATCTACAAAC
AGATGCCAGGATGCTTCAACTTTCTGAGAAAGAAACTGTTCTTTAAGACCTCCG
CATCTAGGGCC
SEQ ID NO: 121 Caspase-9 
VDGFGDVGALESLRGNADLAYILSMEPCGHCLIINNVNFCRESGLRTRTGSNIDCEK
LRRRFSSLHFMVEVKGDLTAKKMVLALLELARQDHGALDCCVVVILSHGCQASHLQ
FPGAVYGTDGCPVSVEKIVNIFNGTSCPSLGGKPKLFFIQACGGEQKDHGFEVAST
SPEDESPGSNPEPDATPFQEGLRTFDQLDAISSLPTPSDIFVSYSTFPGFVSWRDPK
 
SGSWYVETLDDIFEQWAHSEDLQSLLLRVANAVSVKGIYKQMPGCFNFLRKKLFFK
TSASRA
SEQ ID NO: 122 Linker
   CCGCGG
SEQ ID NO: 123 Linker
   PR
SEQ ID NO: 124 T2A 
GAAGGCCGAGGGAGCCTGCTGACATGTGGCGATGTGGAGGAAAACCCAGGAC
CA
SEQ ID NO: 125 T2A
   EGRGSLLTCGDVEENPGP
SEQ ID NO: 126 Linker
   CCATGG
SEQ ID NO: 127 Linker
   PW
SEQ ID NO: 128 Signal peptide 
ATGGAGTTTGGACTTTCTTGGTTGTTTTTGGTGGCAATTCTGAAGGGTGTCCAGT
GTAGCAGG
SEQ ID NO: 129 Signal peptide
   MEFGLSWLFLVAILKGVQCSR
SEQ ID NO: 130 FMC63 variable light chain (anti-CD19) 
GACATCCAGATGACACAGACTACATCCTCCCTGTCTGCCTCTCTGGGAGACAGA
GTCACCATCAGTTGCAGGGCAAGTCAGGACATTAGTAAATATTTAAATTGGTATC
AGCAGAAACCAGATGGAACTGTTAAACTCCTGATCTACCATACATCAAGATTACA
CTCAGGAGTCCCATCAAGGTTCAGTGGCAGTGGGTCTGGAACAGATTATTCTCT
CACCATTAGCAACCTGGAGCAAGAAGATATTGCCACTTACTTTTGCCAACAGGG
TAATACGCTTCCGTACACGTTCGGAGGGGGGACTAAGTTGGAAATAACA
SEQ ID NO: 131 FMC63 variable light chain (anti CD19) 
DIQMTQTTSSLSASLGDRVTISCRASQDISKYLNWYQQKPDGTVKLLIYHTSRLHSG
VPSRFSGSGSGTDYSLTISNLEQEDIATYFCQQGNTLPYTFGGGTKLEIT
SEQ ID NO: 132 Flexible linker
   GGCGGAGGAAGCGGAGGTGGGGGC
SEQ ID NO: 133 Flexible linker
   GGGSGGGG
SEQ ID NO: 134 FMC63 variable heavy chain (anti-CD19) 
GAGGTGAAACTGCAGGAGTCAGGACCTGGCCTGGTGGCGCCCTCACAGAGCC
TGTCCGTCACATGCACTGTCTCAGGGGTCTCATTACCCGACTATGGTGTAAGCT
GGATTCGCCAGCCTCCACGAAAGGGTCTGGAGTGGCTGGGAGTAATATGGGGT
AGTGAAACCACATACTATAATTCAGCTCTCAAATCCAGACTGACCATCATCAAGG
ACAACTCCAAGAGCCAAGTTTTCTTAAAAATGAACAGTCTGCAAACTGATGACAC
AGCCATTTACTACTGTGCCAAACATTATTACTACGGTGGTAGCTATGCTATGGAC
TACTGGGGTCAAGGAACCTCAGTCACCGTCTCCTCA
SEQ ID NO: 135 FMC63 variable heavy chain (anti CD19) 
EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWG
QGTSVTVSS
SEQ ID NO: 136 Linker
   GGATCC
SEQ ID NO: 137 Linker
   GS
SEQ ID NO: 138 CD34 minimal epitope
   GAACTTCCTACTCAGGGGACTTTCTCAAACGTTAGCACAAACGTAAGT
SEQ ID NO: 139 CD34 minimal epitope
   ELPTQGTFSNVSTNVS
SEQ ID NO: 140 CD8 α stalk domain 
CCCGCCCCAAGACCCCCCACACCTGCGCCGACCATTGCTTCTCAACCCCTGAG
TTTGAGACCCGAGGCCTGCCGGCCAGCTGCCGGCGGGGCCGTGCATACAAGA
GGACTCGATTTCGCTTGCGAC
SEQ ID NO: 141 CD8 α stalk domain
   PAPRPPTPAPTIASQPLSLRPEACRPAAGGAVHTRGLDFACD
SEQ ID NO: 142 CD8 α transmembrane domain 
ATCTATATCTGGGCACCTCTCGCTGGCACCTGTGGAGTCCTTCTGCTCAGCCTG
GTTATTACTCTGTACTGTAATCACCGGAATCGCCGCCGCGTTTGTAAGTGTCCC
AGG
SEQ ID NO: 143 CD8 α transmembrane domain
   IYIWAPLAGTCGVLLLSLVITLYCNHRNRRRVCKCPR
SEQ ID NO: 144 Linker
   GTCGAC
SEQ ID NO: 145 Linker
   VD
SEQ ID NO: 146 Truncated MyD88 lacking the TIR domain 
ATGGCCGCTGGGGGCCCAGGCGCCGGATCAGCTGCTCCCGTATCTTCTACTTC
TTCTTTGCCGCTGGCTGCTCTGAACATGCGCGTGAGAAGACGCCTCTCCCTGTT
CCTTAACGTTCGCACACAAGTCGCTGCCGATTGGACCGCCCTTGCCGAAGAAAT
GGACTTTGAATACCTGGAAATTAGACAACTTGAAACACAGGCCGACCCCACTGG
CAGACTCCTGGACGCATGGCAGGGAAGACCTGGTGCAAGCGTTGGACGGCTC
CTGGATCTCCTGACAAAACTGGGACGCGACGACGTACTGCTTGAACTCGGACC
TAGCATTGAAGAAGACTGCCAAAAATATATCCTGAAACAACAACAAGAAGAAGC
CGAAAAACCTCTCCAAGTCGCAGCAGTGGACTCATCAGTACCCCGAACAGCTG
AGCTTGCTGGGATTACTACACTCGACGACCCACTCGGACATATGCCTGAAAGAT
TCGACGCTTTCATTTGCTATTGCCCCTCTGACATA
SEQ ID NO: 147 Truncated MyD88 lacking the TIR domain 
MAAGGPGAGSAAPVSSTSSLPLAALNMRVRRRLSLFLNVRTQVAADWTALAEEMD
FEYLEIRQLETQADPTGRLLDAWQGRPGASVGRLLDLLTKLGRDDVLLELGPSIEED
 
CQKYILKQQQEEAEKPLQVAAVDSSVPRTAELAGITTLDDPLGHMPERFDAFICYCP
SDI
SEQ ID NO: 148 CD40 without the extracellular domain 
AAGAAAGTTGCAAAGAAACCCACAAATAAAGCCCCACACCCTAAACAGGAACCC
CAAGAAATCAATTTCCCAGATGATCTCCCTGGATCTAATACTGCCGCCCCGGTC
CAAGAAACCCTGCATGGTTGCCAGCCTGTCACCCAAGAGGACGGAAAAGAATC
ACGGATTAGCGTACAAGAGAGACAA
SEQ ID NO: 149 CD40 without the extracellular domain 
KKVAKKPTNKAPHPKQEPQEINFPDDLPGSNTAAPVQETLHGCQPVTQEDGKESR[?]
SVQERQ
SEQ ID NO: 150 CD3 zeta 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
CCTTTACCAGGGTCTCAGTACAGCCACCAAGGACACCTACGACGCCCTTCACAT
GCAGGCCCTGCCCCCTCGC
SEQ ID NO: 151 CD3 zeta

RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR

### Example 14: Cytokine Production of T Cells Co-expressing a MyD88/CD40 Chimeric Antigen Receptor and Inducible Caspase-9 Polypeptide

Various chimeric antigen receptor constructs were created to compare cytokine production of transduced T cells after exposure to antigen. The chimeric antigen receptor constructs all had an antigen recognition region that bound to CD19. It is understood that the vectors provided herein may be modified to construct a CAR construct that also incorporates an inducible Caspase-9 safety switch. It is further understood that the CAR construct may further comprise an FRB domain.

### Example 15: An example of a MyD88/CD40 CAR construct for targeting Her2⁺ tumor cells,

It is understood that the vectors provided herein may be modified to construct a MyD88/CD40 CAR construct that targets Her2⁺ tumor cells, which also incorporates an inducible Caspase-9 safety switch. It is further understood that the CAR construct may further comprise an FRB domain.

### SFG-Her2scFv.CD34e.CD8stm.MC.zeta sequence

SEQ ID NO: 152 Signal peptide 
ATGGAGTTTGGACTTTCTTGGTTGTTTTGGTGGCAATTCTGAAGGGTGTCCAGT
GTAGCAGG
SEQ ID NO: 153 Signal peptide
   MEFGLSWLFLVAILKGVQCSR
SEQ ID NO: 154 FRP5 variable light chain (anti-Her2) 
GACATCCAATTGACACAATCACACAAATTTCTCTCAACTTCTGTAGGAGACAGAG
TGAGCATAACCTGCAAAGCATCCCAGGACGTGTACAATGCTGTGGCTTGGTACC
AACAGAAGCCTGGACAATCCCCAAAATTGCTGATTTATTCTGCCTCTAGTAGGTA
CACTGGGGTACCTTCTCGGTTTACGGCTCTGGGTCCGGACCAGATTTCACGTT
CACAATCAGTTCCGTTCAAGCTGAAGACCTCGCTGTTTATTTTTGCCAGCAGCA
CTTCCGAACCCCTTTTACTTTTGGCTCAGGCACTAAGTTGGAAATCAAGGCTTTG
SEQ ID NO: 155 FRP5 variable light chain (anti-Her2) 
DIQLTQSHKFLSTSVGDRVSITCKASQDVYNAVAWYQQKPGQSPKLLIYSASSRYT
GVPSRFTGSGSGPDFTFTISSVQAEDLAVYFCQQHFRTPFTFGSGTKLEIKAL
SEQ ID NO: 156 Flexible linker
   GGCGGAGGAAGCGGAGGTGGGGGC
SEQ ID NO: 157 Flexible linker
   GGGSGGGG
SEQ ID NO: 158 FRP5 variable heavy chain (anti-Her2/Neu) 
GAAGTCCAATTGCAACAGTCAGGCCCCGAATTGAAAAAGCCCGGCGAAACAGT
GAAGATATCTTGTAAAGCCTCCGGTTACCCTTTTACGAACTATGGAATGAACTGG
 
GTCAAACAAGCCCCTGGACAGGGATTGAAGTGGATGGATGGATCAATACATC
AACAGGCGAGTCTACCTTCGCAGATGATTTCAAAGGTCGCTTTGACTTCTCACT
GGAGACCAGTGCAAATACCGCCTACCTTCAGATTAACAATCTTAAAGCGAGGA
TATGGCAACCTACTTTTGCGCAAGATGGGAAGTTTATCACGGGTACGTGCCATA
CTGGGGACAAGGAACGACAGTGACAGTTAGTAGC
SEQ ID NO: 159 FRP5 variable heavy chain (anti-Her2/Neu) 
EVQLQQSGPELKKPGETVKISCKASGYPFTNYGMNWVKQAPGQGLKWMGWINTS
TGESTFADDFKGRFDFSLETSANTAYLQINNLKSEDMATYFCARWEVYHGYVPYW
GQGTTVTVSS
SEQ ID NO: 160 Linker
   GGATCC
SEQ ID NO: 161 Linker
   GS
SEQ ID NO: 162 CD34 minimal epitope
   GAACTTCCTACTCAGGGGACTTTCTCAAACGTTAGCACAAACGTAAGT
SEQ ID NO: 163 CD34 minimal epitope
   ELPTQGTFSNVSTNVS
SEQ ID NO: 164 CD8 alpha stalk 
CCCGCCCCAAGACCCCCCACACCTGCGCCGACCATTGCTTCTCAACCCCTGAG
TTTGAGACCCGAGGCCTGCCGGCCAGCTGCCGGCGGGGCCGTGCATACAAGA
GGACTCGATTTCGCTTGCGAC
SEQ ID NO: 165 CD8 alpha stalk
   PAPRPPTPAPTIASQPLSLRPEACRPAAGGAVHTRGLDFACD
SEQ ID NO: 166 CD8 alpha transmembrane region 
ATCTATATCTGGGCACCTCTCGCTGGCACCTGTGGAGTCCTTCTGCTCAGCCTG
GTTATTACTCTGTACTGTAATCACCGGAATCGCCGCCGCGTTTGTAAGTGTCCC
AGG
SEQ ID NO: 167 CD8 alpha transmembrane region
   IYIWAPLAGTCGVLLLSLVITLYCNHRNRRRVCKCPR
SEQ ID NO: 168 Linker
   Ctcgag
SEQ ID NO: 169 Linker
   LE
SEQ ID NO: 170 Truncated MyD88 
ATGGCCGCTGGGGGCCCAGGCGCCGGATCAGCTGCTCCCGTATCTTCTACTTC
TTCTTTGCCGCTGGCTGCTCTGAACATGCGCGTGAGAAGACGCCTCTCCCTGTT
CCTTAACGTTCGCACACAAGTCGCTGCCGATTGGACCGCCCTTGCCGAAGAAAT
GGACTTTGAATACCTGGAAATTAGACAACTTGAAACACAGGCCGACCCCACTGG
CAGACTCCTGGACGCATGGCAGGGAAGACCTGGTGCAAGCGTTGGACGGCTC
CTGGATCTCCTGACAAAACTGGGACGCGACGACGTACTGCTTGAACTCGGACC
TAGCATTGAAGAAGACTGCCAAAAATATATCCTGAAACAACAACAAGAAGAAGC
CGAAAAACCTCTCCAAGTCGCAGCAGTGGACTCATCAGTACCCCGAACAGCTG
AGCTTGCTGGGATTACTACACTCGACGACCCACTCGGACATATGCCTGAAAGAT
TCGACGCTTTCATTTGCTATTGCCCCTCTGACATA
SEQ ID NO: 171 Truncated MyD88 
MAAGGPGAGSAAPVSSTSSLPLAALNMRVRRRLSLFLNVRTQVAADWTALAEEMD
FEYLEIRQLETQADPTGRLLDAWQGRPGASVGRLLDLLTKLGRDDVLLELGPSIEED
CQKYILKQQQEEAEKPLQVAAVDSSVPRTAELAGITTLDDPLGHMPERFDAFICYCP
SDI
SEQ ID NO: 172 CD40 cytoplasmic domain 
AAGAAAGTTGCAAAGAAACCCACAAATAAAGCCCCACACCCTAAACAGGAACCC
CAAGAAATCAATTTCCCAGATGATCTCCCTGGATCTAATACTGCCGCCCCGGTC
CAAGAAACCCTGCATGGTTGCCAGCCTGTCACCCAAGAGGACGGAAAAGAATC
ACGGATTAGCGTACAAGAGAGACAA
SEQ ID NO: 173 CD40 cytoplasmic domain 
KKVAKKPTNKAPHPKQEPQEINFPDDLPGSNTAAPVQETLHGCQPVTQEDGKESR[?]
SVQERQ
SEQ ID NO: 174 Linker
   gcggccgcagtcgag
SEQ ID NO: 175 Linker
   AAAVE
SEQ ID NO: 176 CD3 zeta cytoplasmic domain 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
CCTTTACCAGGGTCTCAGTACAGCCACCAAGGACACCTACGACGCCCTTCACAT
GCAGGCCCTGCCCCCTCGC
SEQ ID NO: 177 CD3 zeta cytoplasmic domain

RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR

### Example 16: Additional Sequences

SEQ ID NO: 178, ΔCasp9 (res. 135-416) 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L D A I S S L P T P S D I F V S Y S T F P G F
V S W R D P K S G S W Y V E T L D D I F E Q W A H S E D L Q
S L L L R V A N A V S V K G I Y K Q M P G C F N F L R K K L F
F K T S
SEQ ID NO: 179, ΔCasp9 (res. 135-416) D330A, nucleotide sequence 
GGATTTGGTGATGTCGGTGCTCTTGAGAGTTTGAGGGGAAATGCAGATTTGGCT
TACATCCTGAGCATGGAGCCCTGTGGCCACTGCCTCATTATCAACAATGTGAAC
TTCTGCCGTGAGTCCGGGCTCCGCACCCGCACTGGCTCCAACATCGACTGTGA
GAAGTTGCGGCGTCGCTTCTCCTCGCTGCATTTCATGGTGGAGGTGAAGGGCG
ACCTGACTGCCAAGAAAATGGTGCTGGCTTTGCTGGAGCTGGCGcgGCAGGAC
CACGGTGCTCTGGACTGCTGCGTGGTGGTCATTCTCTCTCACGGCTGTCAGGC
CAGCCACCTGCAGTTCCCAGGGGCTGTCTACGGCACAGATGGATGCCCTGTGT
CGGTCGAGAAGATTGTGAACATCTTCAATGGGACCAGCTGCCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
GCCGCCATATCTAGTTTGCCCACACCCAGTGACATCTTTGTGTCCTACTCTACTT
TCCCAGGTTTTGTTTCCTGGAGGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTAATTTCCTCCGGAAAAACTTTTCTTTAAAACATCA
SEQ ID NO: 180, ΔCasp9 (res. 135-416) D330A, amino acid sequence 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L A A I S S L P T P S D I F V S Y S T F P G F
V S W R D P K S G S W Y V E T L D D I F E Q W A H S E D L Q
S L L L R V A N A V S V K G I Y K Q M P G C F N F L R K K L F
F K T S
SEQ ID NO: 181, ΔCasp9 (res. 135-416) N405Q nucleotide sequence 
GGATTTGGTGATGTCGGTGCTCTTGAGAGTTTGAGGGGAAATGCAGATTTGGCT
TACATCCTGAGCATGGAGCCCTGTGGCCACTGCCTCATTATCAACAATGTGAAC
TTCTGCCGTGAGTCCGGGCTCCGCACCCGCACTGGCTCCAACATCGACTGTGA
GAAGTTGCGGCGTCGCTTCTCCTCGCTGCATTTCATGGTGGAGGTGAAGGCG
ACCTGACTGCCAAGAAAATGGTGCTGGCTTTGCTGGAGCTGGCGCgGCAGGAC
CACGGTGCTCTGGACTGCTGCGTGGTGGTCATTCTCTCTCACGGCTGTCAGGC
 
CAGCCACCTGCAGTTCCCAGGGGCTGTCTACGGCACAGATGGATGCCCTGTGT
CGGTCGAGAAGATTGTGAACATCTTCAATGGACCAGCTGCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
GACGCCATATCTAGTTTGCCCACACCCAGTGACATCTTTGTGTCCTACTCTACTT
TCCCAGGTTTTGTTTCCTGGAGGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTCAGTTCCTCCGGAAAAACTTTTCTTTAAAACATCA
SEQ ID NO: 182, ΔCasp9 (res. 135-416) N405Q amino acid sequence 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L D A I S S L P T P S D I F V S Y S T F P G F
V S W R D P K S G S W Y V E T L D D I F E Q W A H S E D L Q
S L L L R V A N A V S V K G I Y K Q M P G C F Q F L R K K L F
F K T S
SEQ ID NO: 183, ΔCasp9 (res. 135-416) D330A N405Q nucleotide sequence 
GGATTTGGTGATGTCGGTGCTCTTGAGAGTTTGAGGGGAAATGCAGATTTGGCT
TACATCCTGAGCATGGAGCCCTGTGGCCACTGCCTCATTATCAACAATGTGAAC
TTCTGCCGTGAGTCCGGGCTCCGCACCCGCACTGGCTCCAACATCGACTGTGA
GAAGTTGCGGCGTCGCTTCTCCTCGCTGCATTTCATGGTGGAGGTGAAGGGCG
ACCTGACTGCCAAGAAAATGGTGCTGGCTTTGCTGGAGCTGGCGcGGCAGGAC
CACGGTGCTCTGGACTGCTGCGTGGTGGTCATTCTCTCTCACGGCTGTCAGGC
CAGCCACCTGCAGTTCCCAGGGGCTGTCTACGGCACAGATGGATGCCCTGTGT
CGGTCGAGAAGATTGTGAACATCTTCAATGGGACCAGCTGCCCCAGCCTGGGA
GGGAAGCCCAAGCTCTTTTTCATCCAGGCCTGTGGTGGGGAGCAGAAAGACCA
TGGGTTTGAGGTGGCCTCCACTTCCCCTGAAGACGAGTCCCCTGGCAGTAACC
CCGAGCCAGATGCCACCCCGTTCCAGGAAGGTTTGAGGACCTTCGACCAGCTG
 
GCCGCCATATCTAGTTTGCCCACACCCAGTGACATCTTTGTGTCCTACTCTACTT
TCCCAGGTTTTGTTTCCTGGAGGGACCCCAAGAGTGGCTCCTGGTACGTTGAG
ACCCTGGACGACATCTTTGAGCAGTGGGCTCACTCTGAAGACCTGCAGTCCCT
CCTGCTTAGGGTCGCTAATGCTGTTTCGGTGAAAGGGATTTATAAACAGATGCC
TGGTTGCTTTCAGTTCCTCCGGAAAAAACTTTTCTTTAAAACATCA
SEQ ID NO: 184, ΔCasp9 (res. 135-416) D330A N405Q amino acid sequence 
G F G D V G A L E S L R G N A D L A Y I L S M E P C G H C L I
I N N V N F C R E S G L R T R T G S N I D C E K L R R R F S S
L H F M V E V K G D L T A K K M V L A L L E L A R Q D H G A L
D C C V V V I L S H G C Q A S H L Q F P G A V Y G T D G C P V
S V E K I V N I F N G T S C P S L G G K P K L F F I Q A C G G
E Q K D H G F E V A S T S P E D E S P G S N P E P D A T P F
Q E G L R T F D Q L A A I S S L P T P S D I F V S Y S T F P G F
V S W R D P K S G S W Y V E T L D D I F E Q W A H S E D L Q
S L L L R V A N A V S V K G I Y K Q M P G C F Q F L R K K L F
F K T S
SEQ IDNO: 185, Caspase-9.co nucleotide sequence 
GTGGACGGGTTTGGAGATGTGGAGCCCTGGAATCCCTGCGGGGCAATGCCG
ATCTGGCTTACATCCTGTCTATGGAGCCTTGCGGCCACTGTCTGATCATTAACA
ATGTGAACTTCTGCAGAGAGAGCGGGCTGCGGACCAGAACAGGATCCAATATT
GACTGTGAAAAGCTGCGGAGAAGGTTCTCTAGTCTGCACTTTATGGTCGAGGTG
AAAGGCGATCTGACCGCTAAGAAAATGGTGCTGGCCCTGCTGGAACTGGCTCG
GCAGGACCATGGGGCACTGGATTGCTGCGTGGTCGTGATCCTGAGTCACGGCT
GCCAGGCTTCACATCTGCAGTTCCCTGGGGCAGTCTATGGAACTGACGGCTGT
CCAGTCAGCGTGGAGAAGATCGTGAACATCTTCAACGGCACCTCTTGCCCAAGT
CTGGGCGGGAAGCCCAAACTGTTCTTTATTCAGGCCTGTGGAGGCGAGCAGAA
AGATCACGGCTTCGAAGTGGCTAGCACCTCCCCCGAGGACGAATCACCTGGAA
GCAACCCTGAGCCAGATGCAACCCCCTTCCAGGAAGGCCTGAGGACATTTGAC
CAGCTGGATGCCATCTCAAGCCTGCCCACACCTTCTGACATTTTCGTCTCTTAC
AGTACTTTCCCTGGATTTGTGAGCTGGCGCGATCCAAAGTCAGGCAGCTGGTAC
GTGGAGACACTGGACGATATCTTTGAGCAGTGGGCCCATTCTGAAGACCTGCA
GAGTCTGCTGCTGCGAGTGGCCAATGCTGTCTCTGTGAAGGGGATCTACAAAC
 
AGATGCCAGGATGCTTCCAGTTTCTGAGAAAGAAACTGTTCTTTAAGACCTCCG
CATCTAGGGCC
SEQ ID NO: 186, Caspase-9.co amino acid sequence 
VDGFGDVGALESLRGNADLAYILSMEPCGHCLIINNVNFCRESGLRTRTGSNIDCEK
LRRRFSSLHFMVEVKGDLTAKKMVLALLELARQDHGALDCCVVVILSHGCQASHLQ
FPGAVYGTDGCPVSVEKIVNIFNGTSCPSLGGKPKLFFIQACGGEQKDHGFEVAST
SPEDESPGSNPEPDATPFQEGLRTFDQLDAISSLPTPSDIFVSYSTFPGFVSWRDPK
SGSWYVETLDDIFEQWAHSEDLQSLLLRVANAVSVKGIYKQMPGCFQFLRKKLFFK
TSASRA
SEQ ID NO: 187: Caspase9 D330E nucleotide sequence 
GTCGACGGATTTGGTGATGTCGGTGCTCTTGAGAGTTTGAGGGGAAATGCAGA
TTTGGCTTACATCCTGAGCATGGAGCCCTGTGGCCACTGCCTCATTATCAACAA
TGTGAACTTCTGCCGTGAGTCCGGGCTCCGCACCCGCACTGGCTCCAACATCG
ACTGTGAGAAGTTGCGGCGTCGCTTCTCCTCGCTGCATTTCATGGTGGAGGTG
AAGGGCGACCTGACTGCCAAGAAAATGGTGCTGGCTTTGCTGGAGCTGGCGCG
GCAGGACCACGGTGCTCTGGACTGCTGCGTGGTGGTCATTCTCTCTCACGGCT
GTCAGGCCAGCCACCTGCAGTTCCCAGGGGCTGTCTACGGCACAGATGGATGC
CCTGTGTCGGTCGAGAAGATTGTGAACATCTTCAATGGGACCAGCTGCCCCAG
CCTGGGAGGGAAGCCCAAGCTCTTTTTCATCCAGGCCTGTGGTGGGGAGCAGA
AAGACCATGGGTTTGAGGTGGCCTCCACTTCCCCTGAAGACGAGTCCCCTGGC
AGTAACCCCGAGCCAGATGCCACCCCGTTCCAGGAAGGTTTGAGGACCTTCGA
CCAGCTGGcCGCCATATCTAGTTTGCCCACACCCAGTGACATCTTTGTGTCCTA
CTCTACTTTCCCAGGTTTTGTTTCCTGGAGGGACCCCAAGAGTGGCTCCTGGTA
CGTTGAGACCCTGGACGACATCTTTGAGCAGTGGGCTCACTCTGAAGACCTGC
AGTCCCTCCTGCTTAGGGTCGCTAATGCTGTTTCGGTGAAAGGGATTTATAAAC
AGATGCCTGGTTGCTTTAATTTCCTCCGGAAAAAACTTTTCTTTAAAACATCAGC
TAGCAGAGCC
SEQ ID NO: 188: Caspase9 D330E amino acid sequence 
VDGFGDVGALESLRGNADLAYILSMEPCGHCLIINNVNFCRESGLRTRTGSNIDCEK
LRRRFSS
LHFMVEVKGDLTAKKMVLALLELARQDHGALDCCVVVILSHGCQASHLQFPGAVYG
TDGC
 
PVSVEKIVNIFNGTSCPSLGGKPKLFFIQACGGEQKDHGFEVASTSPEDESPGSNPE
PDA
TPFQEGLRTFDQLeAISSLPTPSDIFVSYSTFPGFVSWRDPKSGSWYVETLDDIFEQ
WAH
SEDLQSLLLRVANAVSVKGIYKQMPGCFNFLRKKLFFKTSASRA
*Sequences for pBPO509*
   pBP0509-SFG-PSCAscFv.CH2CH3.CD28tm.zeta.MyD88/CD40 sequence
SEQ ID NO: 189 Signal peptide 
ATGGAGTTTGGACTTTCTTGGTTGTTTTGGTGGCAATTCTGAAGGGTGTCCAGT
GTAGCAGG
SEQ ID NO: 190 Signal peptide
   MEFGLSWLFLVAILKGVQCSR
SEQ ID NO: 191 bm2B3 variable light chain 
GACATCCAGCTGACACAAAGTCCCAGTAGCCTGTCAGCCAGTGTCGGCGATAG
GGTGACAATTACATGCTCCGCAAGTAGTAGCGTCAGATTCATACACTGGTACCA
GCAGAAGCCTGGGAAGGCCCCAAAGAGGCTTATCTACGATACCAGTAAACTCG
CCTCTGGAGTTCCTAGCCGGTTTTCTGGATCTGGCAGCGGAACTAGCTACACCC
TCACAATCTCCAGTCTGCAACCAGAGGACTTTGCAACCTACTACTGCCAGCAAT
GGAGCAGCTCCCCTTTCACCTTTGGGCAGGGTACTAAGGTGGAGATCAAG
SEQ ID NO: 192 bm2B3 variable light chain 
DIQLTQSPSSLSASVGDRVTITCSASSSVRFIHWYQQKPGKAPKRLIYDTSKLASGV
PSRFSGSGSGTSYTLTISSLQPEDFATYYCQQWSSSPFTFGQGTKVEIK
SEQ ID NO: 193 Flexible linker
   GGCGGAGGAAGCGGAGGTGGGGGC
SEQ ID NO: 194 Flexible linker
   GGGSGGGG
SEQ ID NO: 195 bm2B3 variable heavy chain 
GAGGTGCAGCTTGTAGAGAGCGGGGGAGGCCTCGTACAGCCAGGGGGCTCTC
TGCGCCTGTCATGTGCAGCTTCAGGATTCAATATAAAGGACTATTACATTCACTG
GGTACGGCAAGCTCCCGGTAAGGGCCTGGAATGGATCGGTTGGATCGACCCTG
 
AAAACGGAGATACAGAATTTGTGCCCAAGTTCCAGGGAAAGGCTACCATGTCTG
CCGATACTTCTAAGAATACAGCATACCTTCAGATGAATTCTCTCCGCGCCGAGG
ACACAGCCGTGTATTATTGTAAAACGGAGGGTTCTGGGGTCAGGGTACCCTT
GTGACTGTGTCTTCC
SEQ ID NO: 196 bm2B3 variable heavy chain 
EVQLVESGGGLVQPGGSLRLSCAASGFNIKDYYIHWVRQAPGKGLEWIGWIDPEN
GDTEFVPKFQGKATMSADTSKNTAYLQMNSLRAEDTAVYYCKTGGFWGQGTLVTV
SS
SEQ ID NO: 197 Linker
   GGGGATCCCGCC
SEQ ID NO: 198 Linker
   GDPA
SEQ ID NO: 199 IgG1 hinge region
   GAGCCCAAATCTCCTGACAAAACTCACACATGCCCA
SEQ ID NO: 200 IgG1 hinge region
   EPKSPDKTHTCP
SEQ ID NO: 201 IgG1 CH2 region 
CCGTGCCCAGCACCTGAACTCCTGGGGGGACCGTCAGTCTTCCTCTTCCCCCC
AAAACCCAAGGACACCCTCATGATCTCCCGGACCCCTGAGGTCACATGCGTGG
TGGTGGACGTGAGCCACGAAGACCCTGAGGTCAAGTTCAACTGGTATGTGGAC
GGCGTGGAGGTGCATAATGCAAAGACAAAGCCGCGGGAGGAGCAGTACAACA
GCACGTACCGTGTGGTCAGCGTCCTCACCGTCCTGCACCAGGACTGGCTGAAT
GGCAAGGAGTACAAGTGCAAGGTCTCCAACAAAGCCCTCCCAGCCCCCATCGA
GAAAACCATCTCCAAAGCCAAA
SEQ ID NO: 202 IgG1 CH2 region 
PCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAK
SEQ ID NO: 203 IgG1 CH3 region 
GGGCAGCCCCGAGAACCACAGGTGTACACCCTGCCCCCATCCCGGGATGAGC
TGACCAAGAACCAGGTCAGCCTGACCTGCCTGGTCAAAGGCTTCTATCCCAGC
 
GACATCGCCGTGGAGTGGGAGAGCAATGGGCAACCGGAGAACAACTACAAGAC
CACGCCTCCCGTGCTGGACTCCGACGGCTCCTTCTTCCTCTACAGCAAGCTCA
CCGTGGACAAGAGCAGGTGGCAGCAGGGGAACGTCTTCTCATGCTCCGTGATG
CATGAGGCTCTGCACAACCACTACACGCAGAAGAGCCTCTCCCTGTCTCCGGG
TAAA
SEQ ID NO: 204 IgG1 CH3 region 
GQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK
SEQ ID NO: 205 Linker
   AAAGATCCCAAA
SEQ ID NO: 206 Linker
   KDPK
SEQ ID NO: 207 CD28 transmembrane region 
TTTTGGGTGCTGGTGGTGGTTGGTGGAGTCCTGGCTTGCTATAGCTTGCTAGTA
ACAGTGGCCTTTATTATT
SEQ ID NO: 208 CD28 transmembrane region
   FWVLVVVGGVLACYSLLVTVAFII
SEQ ID NO: 209 Linker
   gccggc
SEQ ID NO: 210 Linker
   AG
SEQ ID NO: 211 CD3 zeta 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
CCTTTACCAGGGTCTCAGTACAGCCACCAAGGACACCTACGACGCCCTTCACAT
GCAGGCCCTGCCCCCTCGC
SEQ ID NO: 212 CD3 zeta 
RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR
SEQ ID NO: 213 MyD88 
GCCGCTGGGGGCCCAGGCGCCGGATCAGCTGCTCCCGTATCTTCTACTTCTTC
TTTGCCGCTGGCTGCTCTGAACATGCGCGTGAGAAGACGCCTCTCCCTGTTCCT
TAACGTTCGCACACAAGTCGCTGCCGATTGGACCGCCCTTGCCGAAGAAATGG
ACTTTGAATACCTGGAAATTAGACAACTTGAAACACAGGCCGACCCCACTGGCA
GACTCCTGGACGCATGGCAGGGAAGACCTGGTGCAAGCGTTGGACGGCTCCT
GGATCTCCTGACAAAACTGGGACGCGACGACGTACTGCTTGAACTCGGACCTA
GCATTGAAGAAGACTGCCAAAAATATATCCTGAAACAACAACAAGAAGAAGCCG
AAAAACCTCTCCAAGTCGCAGCAGTGGACTCATCAGTACCCCGAACAGCTGAG
CTTGCTGGGATTACTACACTCGACGACCCACTCGGACATATGCCTGAAAGATTC
GACGCTTTCATTTGCTATTGCCCCTCTGACATA
SEQ ID NO: 214 MyD88 
AAGGPGAGSAAPVSSTSSLPLAALNMRVRRRLSLFLNVRTQVAADWTALAEEMDF
EYLEIRQLETQADPTGRLLDAWQGRPGASVGRLLDLLTKLGRDDVLLELGPSIEEDC
QKYILKQQQEEAEKPLQVAAVDSSVPRTAELAGITTLDDPLGHMPERFDAFICYCPS
DI
SEQ ID NO: 215 CD40 
AAGAAAGTTGCAAAGAAACCCACAAATAAAGCCCACACCCTAAACAGGAACCC
CAAGAAATCAATTTCCCAGATGATCTCCCTGGATCTAATACTGCCGCCCCGGTC
CAAGAAACCCTGCATGGTTGCCAGCCTGTCACCCAAGAGGACGGAAAAGAATC
ACGGATTAGCGTACAAGAGAGACAATAG
SEQ ID NO: 216 CD40

KKVAKKPTNKAPHPKQEPQEINFPDDLPGSNTAAPVQETLHGCQPVTQEDGKESRI
SVQERQ*

### Sequences for pBPO425

### pBP0521-SFG-CD19scFv.CH2CH3.CD28tm.MyD88/CD40.zeta sequence

SEQ ID NO: 217 Signal peptide 
ATGGAGTTTGGACTTTCTTGGTTGTTTTGGTGGCAATTCTGAAGGGTGTCCAGT
GTAGCAGG
SEQID NO: 218 Signal peptide
   MEFGLSWLFLVAILKGVQCSR
SEQ ID NO: 219 FMC63 variable light chain 
GACATCCAGAT
GACACAGACTACATCCTCCCTGTCTGCCTCTCTGGGAGACAGAGTCACCATCAG
TTGCAGGGCAAGTCAGGACATTAGTAAATATTTAAATTGGTATCAGCAGAAACCA
GATGGAACTGTTAAACTCCTGATCTACCATACATCAAGATTACACTCAGGAGTCC
CATCAAGGTTCAGTGGCAGTGGGTCTGGAACAGATTATTCTCTCACCATTAGCA
ACCTGGAGCAAGAAGATATTGCCACTTACTTTTGCCAACAGGGTAATACGCTTC
CGTACACGTTCGGAGGGGGGACTAAGTTGGAAATAACA
SEQ ID NO: 220 FMC63 variable light chain 
DIQMTQTTSSLSASLGDRVTISCRASQDISKYLNWYQQKPDGTVKLLIYHTSRLHSG
VPSRFSGSGSGTDYSLTISNLEQEDIATYFCQQGNTLPYTFGGGTKLEIT
SEQ ID NO: 221 Flexible linker
   GGCGGAGGAAGCGGAGGTGGGGGC
SEQ ID NO: 222 Flexible linker
   GGGSGGGG
SEQ ID NO: 223 FMC63 variable heavy chain 
GAGGTGAAACTGCAGGAGTCAGGACCTGGCCTGGTGGCGCCCTCACAGAGCC
TGTCCGTCACATGCACTGTCTCAGGGGTCTCATTACCCGACTATGGTGTAAGCT
GGATTCGCCAGCCTCCACGAAAGGGTCTGGAGTGGCTGGGAGTAATATGGGGT
AGTGAAACCACATACTATAATTCAGCTCTCAAATCCAGACTGACCATCATCAAGG
ACAACTCCAAGAGCCAAGTTTTCTTAAAAATGAACAGTCTGCAAACTGATGACAC
AGCCATTTACTACTGTGCCAAACATTATTACTACGGTGGTAGCTATGCTATGGAC
TACTGGGGTCAAGGAACCTCAGTCACCGTCTCCTCA
SEQ ID NO: 224 FMC63 variable heavy chain 
EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWG
QGTSVTVSS
SEQ ID NO: 225 Linker
   GGGGATCCCGCC
SEQ ID NO: 226 Linker
   GDPA
SEQ ID NO: 227 IgG1 hinge
   GAGCCCAAATCTCCTGACAAAACTCACACATGCCCA
SEQ ID NO: 228 IgG1 hinge
   EPKSPDKTHTCP
SEQ ID NO: 229 IgG1 CH2 region 
CCGTGCCCAGCACCTGAACTCCTGGGGGGACCGTCAGTCTTCCTCTTCCCCCC
AAAACCCAAGACACCCTCATGATCTCCCGGACCCCTGAGGTCACATGCGTGG
TGGTGGACGTGAGCCACGAAGACCCTGAGGTCAAGTTCAACTGGTATGTGGAC
GGCGTGGAGGTGCATAATGCAAAGACAAAGCCGCGGGAGGAGCAGTACAACA
GCACGTACCGTGTGGTCAGCGTCCTCACCGTCCTGCACCAGGACTGGCTGAAT
GGCAAGGAGTACAAGTGCAAGGTCTCCAACAAAGCCCTCCCAGCCCCCATCGA
GAAAACCATCTCCAAAGCCAAA
SEQ ID NO: 230 IgG1 CH2 region 
PCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAK
SEQ ID NO: 231 IgG1 CH3 region 
GGGCAGCCCCGAGAACCACAGGTGTACACCCTGCCCCCATCCCGGGATGAGC
TGACCAAGAACCAGGTCAGCCTGACCTGCCTGGTCAAAGGCTTCTATCCCAGC
GACATCGCCGTGGAGTGGGAGAGCAATGGGCAACCGGAGAACAACTACAAGAC
CACGCCTCCCGTGCTGGACTCCGACGGCTCCTTCTTCCTCTACAGCAAGCTCA
CCGTGGACAAGAGCAGGTGGCAGCAGGGGAACGTCTTCTCATGCTCCGTGATG
CATGAGGCTCTGCACAACCACTACACGCAGAAGAGCCTCTCCCTGTCTCCGGG
TAAA
SEQ ID NO: 232 IgG1 CH3 region 
GQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK
SEQ ID NO: 233 Linker
   AAAGATCCCAAA
SEQ ID NO: 234 Linker
   KDPK
SEQ ID NO: 235 CD28 transmembrane region 
TTTTGGGTGCTGGTGGTGGTTGGTGGAGTCCTGGCTTGCTATAGCTTGCTAGTA
ACAGTGGCCTTTATTATT
SEQ ID NO: 236 CD28 transmembrane region
   FWVLVVVGGVLACYSLLVTVAFII
SEQ ID NO: 237 Linker
   Ctcgag
SEQ ID NO: 238 Linker
   LE
SEQ ID NO: 239 MyD88 
ATGGCCGCTGGGGGCCCAGGCGCCGGATCAGCTGCTCCCGTATCTTCTACTTC
TTCTTTGCCGCTGGCTGCTCTGAACATGCGCGTGAGAAGACGCCTCTCCCTGTT
CCTTAACGTTCGCACACAAGTCGCTGCCGATTGGACCGCCCTTGCCGAAGAAAT
GGACTTTGAATACCTGGAAATTAGACAACTTGAAACACAGGCCGACCCCACTGG
CAGACTCCTGGACGCATGGCAGGGAAGACCTGGTGCAAGCGTTGGACGGCTC
CTGGATCTCCTGACAAAACTGGGACGCGACGACGTACTGCTTGAACTCGGACC
TAGCATTGAAGAAGACTGCCAAAAATATATCCTGAAACAACAACAAGAAGAAGC
CGAAAAACCTCTCCAAGTCGCAGCAGTGGACTCATCAGTACCCCGAACAGCTG
AGCTTGCTGGGATTACTACACTCGACGACCCACTCGGACATATGCCTGAAAGAT
TCGACGCTTTCATTTGCTATTGCCCCTCTGACATA
SEQ ID NO: 240 MyD88 
MAAGGPGAGSAAPVSSTSSLPLAALNMRVRRRLSLFLNVRTQVAADWTALAEEMD
FEYLEIRQLETQADPTGRLLDAWQGRPGASVGRLLDLLTKLGRDDVLLELGPSIEED
CQKYILKQQQEEAEKPLQVAAVDSSVPRTAELAGITTLDDPLGHMPERFDAFICYCP
SDI
SEQ ID NO: 241 CD40 
AAGAAAGTTGCAAAGAAACCCACAAATAAAGCCCCACACCCTAAACAGGAACCC
CAAGAAATCAATTTCCCAGATGATCTCCCTGGATCTAATACTGCCGCCCCGGTC
CAAGAAACCCTGCATGGTTGCCAGCCTGTCACCCAAGAGGACGGAAAAGAATC
ACGGATTAGCGTACAAGAGAGACAA
SEQ ID NO: 242 CD40 
KKVAKKPTNKAPHPKQEPQEINFPDDLPGSNTAAPVQETLHGCQPVTQEDGKESRI
SVQERQ
SEQ ID NO: 243 Linker
   gcggccgcagTCGAG
SEQ ID NO: 244 Linker
   AAAVE
SEQ ID NO: 245 CD3 zeta chain 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
CCTTTACCAGGGTCTCAGTACAGCCACCAAGGACACCTACGACGCCCTTCACAT
GCAGGCCCTGCCCCCTCGCTAA
SEQ ID NO: 246 CD3 zeta chain

RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR*

### Sequences for SFG-Myr. MC-2A-CD19. scfv. CD34e. CD8stm.zeta

### SFG-Myr.MC.2A.CD19scFv.CD34e.CD8stm.zeta sequence

SEQ ID NO: 247 Myristolation
   atggggagtagcaagagcaagcctaaggaccccagccagcgc
SEQ ID NO: 248 Myristolation
   MGSSKSKPKDPSQR
SEQ ID NO: 249 Linker
   ctcgac
SEQ ID NO: 250 Linker
   LD
SEQ ID NO: 251 MyD88 
atggctgcaggaggtcccggcgcggggtctgcggccccggtctcctccacatcctcccttcccctggctgctctcaaca
tgcgagtgcggcgccgcctgtctctgttcttgaacgtgcggacacaggtggcggccgactggaccgcgctggcggag
gagatggactttgagtacttggagatccggcaactggagacacaagcggaccccactggcaggctgctggacgcct
ggcagggacgccctggcgcctctgtaggccgactgctcgatctgcttaccaagctgggccgcgacgacgtgctgctg
gagctgggacccagcattgaggaggattgccaaaagtatatcttgaagcagcagcaggaggaggctgagaagcct
ttacaggtggccgctgtagacagcagtgtcccacggacagcagagctggcgggcatcaccacacttgatgaccccc
tggggcatatgcctgagcgtttcgatgccttcatctgctattgccccagcgacatc
SEQ ID NO:252 MyD88 
MAAGGPGAGSAAPVSSTSSLPLAALNMRVRRRLSLFLNVRTQVAADWTALAEEMD
FEYLEIRQLETQADPTGRLLDAWQGRPGASVGRLLDLLTKLGRDDVLLELGPSIEED
CQKYILKQQQEEAEKPLQVAAVDSSVPRTAELAGITTLDDPLGHMPERFDAFICYCP
SDI
SEQ ID NO: 253 Linker
   gtcgag
SEQ ID NO: 254 Linker
   VE
SEQ ID NO: 255 CD40 
aaaaaggtggccaagaagccaaccaataaggccccccaccccaagcaggagccccaggagatcaattttcccg
acgatcttcctggctccaacactgctgctccagtgcaggagactttacatggatgccaaccggtcacccaggaggatg
gcaaagagagtcgcatctcagtgcaggagagacag
SEQ ID NO: 256 CD40 
KKVAKKPTNKAPHPKQEPQEINFPDDLPGSNTAAPVQETLHGCQPVTQEDGKESRI
SVQERQ
SEQ ID NO: 257 Linker
   CCGCGG
SEQ ID NO: 258 Linker
   PR
SEQ ID NO: 259 T2A sequence 
GAAGGCCGAGGGAGCCTGCTGACATGTGGCGATGTGGAGGAAAACCCAGGAC
CA
SEQ ID NO: 260 T2A sequence
   EGRGSLLTCGDVEENPGP
SEQ ID NO: 261 Signal peptide 
ATGGAGTTTGGACTTTCTTGGTTGTTTTGGTGGCAATTCTGAAGGGTGTCCAGT
GTAGCAGG
SEQ ID NO: 262 *Signal peptide*
   MEFGLSWLFLVAILKGVQCSR
SEQ ID NO: 263 FMC63 variable light chain 
GACATCCAGATGACACAGACTACATCCTCCCTGTCTGCCTCTCTGGGAGACAGA
GTCACCATCAGTTGCAGGGCAAGTCAGGACATTAGTAAATATTTAAATTGGTATC
AGCAGAAACCAGATGGAACTGTTAAACTCCTGATCTACCATACATCAAGATTACA
CTCAGGAGTCCCATCAAGGTTCAGTGGCAGTGGGTCTGGAACAGATTATTCTCT
CACCATTAGCAACCTGGAGCAAGAAGATATTGCCACTTACTTTTGCCAACAGGG
TAATACGCTTCCGTACACGTTCGGAGGGGGGACTAAGTTGGAAATAACA
SEQ ID NO: 264 FMC63 variable light chain 
DIQMTQTTSSLSASLGDRVTISCRASQDISKYLNWYQQKPDGTVKLLIYHTSRLHSG
VPSRFSGSGSGTDYSLTISNLEQEDIATYFCQQGNTLPYTFGGGTKLEIT
SEQ ID NO: 265 Flexible linker
   GGCGGAGGAAGCGGAGGTGGGGGC
SEQ ID NO: 266 Flexible linker
   GGGSGGGG
SEQ ID NO: 267 FMC63 variable heavy chain 
GAGGTGAAACTGCAGGAGTCAGGACCTGGCCTGGTGGCGCCCTCACAGAGCC
TGTCCGTCACATGCACTGTCTCAGGGGTCTCATTACCCGACTATGGTGTAAGCT
GGATTCGCCAGCCTCCACGAAAGGGTCTGGAGTGGCTGGGAGTAATATGGGGT
AGTGAAACCACATACTATAATTCAGCTCTCAAATCCAGACTGACCATCATCAAGG
ACAACTCCAAGAGCCAAGTTTTCTTAAAAATGAACAGTCTGCAAACTGATGACAC
AGCCATTTACTACTGTGCCAAACATTATTACTACGGTGGTAGCTATGCTATGGAC
TACTGGGGTCAAGGAACCTCAGTCACCGTCTCCTCA
SEQ ID NO: 268 FMC63 variable heavy chain 
EVKLQESGPGLVAPSQSLSVTCTVSGVSLPDYGVSWIRQPPRKGLEWLGVIWGSE
TTYYNSALKSRLTIIKDNSKSQVFLKMNSLQTDDTAIYYCAKHYYYGGSYAMDYWG
QGTSVTVSS
SEQ ID NO: 269 Linker
   GGATCC
SEQ ID NO: 270 Linker
   GS
SEQ ID NO: 271 CD34 minimal epitope
   GAACTTCCTACTCAGGGGACTTTCTCAAACGTTAGCACAAACGTAAGT
SEQ ID NO: 272 CD34 minimal epitope
   ELPTQGTFSNVSTNVS
SEQ ID NO: 273 CD8 alpha stalk domain 
CCCGCCCCAAGACCCCCCACACCTGCGCCGACCATTGCTTCTCAACCCCTGAG
TTTGAGACCCGAGGCCTGCCGGCCAGCTGCCGGCGGGGCCGTGCATACAAGA
GGACTCGATTTCGCTTGCGAC
SEQ ID NO: 274 CD8 alpha stalk domain
   PAPRPPTPAPTIASQPLSLRPEACRPAAGGAVHTRGLDFACD
SEQ ID NO: 275 CD8 alpha transmembrane domain 
ATCTATATCTGGGCACCTCTCGCTGGCACCTGTGGAGTCCTTCTGCTCAGCCTG
GTTATTACTCTGTACTGTAATCACCGGAATCGCCGCCGCGTTTGTAAGTGTCCC
AGG
SEQ ID NO: 276 CD8 alpha transmembrane domain
   IYIWAPLAGTCGVLLLSLVITLYCNHRNRRRVCKCPR
SEQ ID NO: 277 Linker
   GTCGAC
SEQ ID NO: 278 Linker
   VD
SEQ ID NO: 279 CD3 zeta 
AGAGTGAAGTTCAGCAGGAGCGCAGACGCCCCCGCGTACCAGCAGGGCCAGA
ACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGG
CCTTTACCAGGGTCTCAGTACAGCCACCAAGGACACCTACGACGCCCTTCACAT
GCAGGCCCTGCCCCCTCGC
SEQ ID NO: 280 CD3 zeta 
RVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQAL
PPR
SEQ ID NO: 281 (MyD88 nucleotide sequence) 
atggctgcaggaggtcccggcgcggggtctgcggccccggtctcctccacatcctcccttcccctggctgctctcaaca
tgcgagtgcggcgccgcctgtctctgttcttgaacgtgcggacacaggtggcggccgactggaccgcgctggcggag
gagatggactttgagtacttggagatccggcaactggagacacaagcggaccccactggcaggctgctggacgcct
ggcagggacgccctggcgcctctgtaggccgactgctcgagctgcttaccaagctgggccgcgacgacgtgctgctg
gagctgggacccagcattgaggaggattgccaaaagtatatcttgaagcagcagcaggaggaggctgagaagcct
ttacaggtggccgctgtagacagcagtgtcccacggacagcagagctggcgggcatcaccacacttgatgaccccc
tggggcatatgcctgagcgtttcgatgccttcatctgctattgccccagcgacatccagtttgtgcaggagatgatccggc
aactggaacagacaaactatcgactgaagttgtgtgtgtctgaccgcgatgtcctgcctggcacctgtgtctggtctattg
ctagtgagctcatcgaaaagaggtgccgccggatggtggtggttgtctctgatgattacctgcagagcaaggaatgtg
acttccagaccaaatttgcactcagcctctctccaggtgcccatcagaagcgactgatccccatcaagtacaaggcaa
tgaagaaagagttccccagcatcctgaggttcatcactgtctgcgactacaccaacccctgcaccaaatcttggttctg
gactcgccttgccaaggccttgtccctgccc
SEQ ID NO: 282 (MyD88 amino acid sequence)

M A A G G P G A G S A A P V S S T S S L P L A A L N M R V R R R L S L F L N V
R T Q V A A D W T A L A E E M D F E Y L E I R Q L E T Q A D P T G R L L D A W
Q G R P G A S V G R L L E L L T K L G R D D V L L E L G P S I E E D C Q K Y I L
K Q Q Q E E A E K P L Q V A A V D S S V P R T A E L A G I T T L D D P L G H M P
E R F D A F I C Y C P S D I Q F V Q E M I R Q L E Q T N Y R L K L C V S D R D V
L P G T C V W S I A S E L I E K R C R R M V V V V S D D Y L Q S K E C D F Q T K
F A L S L S P G A H Q K R L I P I K Y K A M K K E F P S I L R F I T V C D Y T N P
C T K S W F W T R L A K A L S L P

### Example 17: Development of Improved Therapeutic Cell Dimmer Switch

Therapy using autologous T cells expressing chimeric antigen receptors (CARs) directed toward tumor-associated antigens (TAAs) has had a transformational effect on the treatment of certain types of leukemias ("liquid tumors") and lymphomas with objective response (OR) rates approaching 90%. Despite their great clinical promise and the predictable accompanying enthusiasm, this success is tempered by the observed high level of on-target, off-tumor adverse events, typical of a cytokine release syndrome (CRS). To maintain the benefit of these revolutionary treatments while minimizing the risk, a chimeric caspase polypeptide-based suicide gene system has been developed, which is based on synthetic ligand-mediated dimerization of a modified Caspase-9 protein, fused to a ligand binding domain, called FKBP12v36. In the presence of the FKBP12v36-binding to the small molecule dimerizer, rimiducid (AP1903), Caspase-9 is activated, leading to rapid apoptosis of target cells. Addition of reduced levels of rimiducid can lead to a tempered rate of killing, allowing the amount of T cell elimination to be regulated from almost nothing to almost full elimination of chimeric caspase-modified T cells. To maximize the utility of this "dimmer" switch, the slope of the dose-response curve should be as gradual as possible; otherwise, administration of the correct dose is challenging. With the current, first generation, clinical iCaspase-9 construct, a dose response curve covering about 1.5 to 2 logs has been observed.

To improve on the therapeutic cell dimmer function, a second level of control may be added to Caspase-9 aggregation, separating rapamycin-driven low levels of aggregation from rimiducid-driven high levels of dimerization. In the first level of control, chimeric caspase polypeptides are recruited by rapamycin/sirolimus (or non-immunosuppressant analog) to a chimeric antigen receptor (CAR), which is modified to contain one or more copies of the 89-amino acid FKBP12-Rapamycin-Binding (FRB) domain (encoded within mTOR) on its carboxy terminus (Fig. 3, left panel). Relative to rimiducid-driven homodimerization of iCaspase-9, it is predicted that the level of Caspase-9 oligomerization would be reduced, both due to the relative affinities of rapamycin-bound FKBP12v36 to FRB (K_{d} ∼ 4 nM) vs rimiducid-bound FKBP12v36 (∼ 0.1 nM) and due to the "staggered" geometry of the crosslinked proteins. An additional level of "fine-tuning" can be provided at the CAR docking site by changing the number of FRB domains fused to each CAR. Meanwhile, target-dependent specificity will be provided by normal target-driven CAR clustering, which should, in turn, be translated to chimeric caspase polypeptide clustering in the presence of rapamycin. When a maximum level of cell elimination is required, rimiducid can also be administered under the current protocol (i.e., currently 0.4 mg/kg in a 2-hour infusion (Figure 3, right panel).

### Methods:

Vectors for rapalog-regulated chimeric caspase polypeptide: The Schreiber lab initially identified the minimal FKBP12-rapamycin binding (FRB) domain from mTOR/FRAP (residues 2025-2114), determining it to have a rapamycin dissociation constant (Kd) about 4 nM (Chen J et al (95) PNAS 92, 4947-51). Subsequent studies identified orthogonal mutants of FRB, such as FRBI (L2098) that bind with relatively high affinity to non-immunosuppressant "bumped" rapamycin analogs ("rapalogs") (Liberles SD (97) PNAS 94, 7825-30; Bayle JH (06) Chem & Biol 13, 99-107). In order to develop modified MC-CARs that can recruit CaspaCIDe, the carboxy terminal CD3 zeta domain (from pBP0526) and pBP0545, Figure 7) are fused to 1 or 2 tandem FRB_{L} domains using a commercially synthesized Sall-Mlul fragment that contains MyD88, CD40, and CD3ζ domains to produce vectors pBP0612 and pBP0611, respectively (Figures 4 and 5) and Tables 7 and 8. The approach should also be applicable to any CAR construct, including standard, "non-MyD88/CD40" constructs, such as those that include CD28, OX40, and/or 4-1BB, and CD3zeta.

### Results:

As a proof of principal, two tandem FRB_{I} domains were fused to either a 1^{st} generation Her2-CAR or to a 1^{st} generation CD19-CAR co-expressing inducible Caspase-9. 293 cells were transiently transfected with a constitutive reporter plasmid, SRα-SEAP, along with normalized levels of expression plasmids encoding Her2-CAR-FRB_{I}2, iCaspase-9, Her2-CAR-FRB_{I}2 + iCasp9, iC9-CAR(19).FRB_{I}2 (coexpressing both CD19-CAR-FRB_{I}2 and iCaspase9), or control vector. After 24 hours, cells were washed and distributed into duplicate wells with half-log dilutions of rapamycin or rimiducid. After overnight incubation with drugs, SEAP activity was determined. Interestingly, rapamycin addition led to a broad decrement of SEAP activity up to about a 50% decrease (Figure 6). This dose-dependent decrease required the presence of both the FRB-tagged CAR and the FKBP-tagged Caspase-9. In contrast, AP1903 decreased SEAP activity to about 20% normal levels at much lower levels of drug, comparable to previous experience. It is likely possible to reduce cell viability with rapamycin and switch to rimiducid for more efficient killing in vivo if necessary. Moreover, on- or off-target-mediated CAR clustering should increase the sensitivity of killing primarily at the site of scFv engagement.

### Additional permutations of the hetero-switch:

Although inducible Caspase-9 has been found to be the fastest and most CID-sensitive suicide gene tested among a large cohort of inducible signaling molecules, many other proteins or protein domains that lead to apoptosis (or related necroptosis, triggering inflammation and necrosis as the means of cell death) could be adapted to homo- or heterodimer-based killing using this approach.

A partial list of proteins that could be activated by rapamycin (or rapalog)-mediated membrane recruitment includes:
Other Caspases (i.e., Caspases 1 to 14, which have been identified in mammals) Other Caspase-associated adapter molecules, such as FADD (DED), APAF1 (CARD), CRADD/RAIDD (CARD), and ASC (CARD) that function as natural caspase dimerizers (dimerization domains in parentheses).
Pro-apoptotic Bcl-2 Family members, such as Bax and Bak, which can cause mitochondrial depolarization (or mislocalization of anti-apoptotic family members, like Bcl-xL or Bcl-2).
RIPK3 or the RIPK1-RHIM domain that can trigger a related form of pro-inflammatory cell death, called necroptosis, due to MLKL-mediated membrane lysis.
Due to its target-dependent level of aggregation, CAR receptors should provide ideal docking sites for rapamycin-mediated recruitment of pro-apoptotic molecules. Nevertheless, many examples exist of multivalent docking site containing FRB domains that could potentially provide rapalog-mediated cell death in the presence of co-expressed chimeric inducible caspase-like molecules.

**TABLE 7: iCasp9-2A-ΔCD19-Q-CD28stm-MCz-FRBI2**

| Fragment | Nucleotide | Polypeptide |
|---|---|---|
| **FKBP12v36** | ATGGGAGTGCAGGTGGAGACTATTAGCCCCGGAGATGGCAGAACATTC CCCAAAAGAGGACAGACTTGCGTCGTGCATTATACTGGAATGCTGGAA GACGGCAAGAAGGTGGACAGCAGCCGGGACCGAAACAAGCCCTTCAA GTTCATGCTGGGGAAGCAGGAAGTGATCCGGGGCTGGGAGGAAGGAG TCGCACAGATGTCAGTGGGACAGAGGGCCAAACTGACTATTAGCCCAG ACTACGCTTATGGAGCAACCGGCCACCCCGGGATCATTCCCCCTCATG CTACACTGGTCTTCGATGTGGAGCTGCTGAAGCTGGAA | MGVQVETISPGDGRTFPKRGQTC VVHYTGMLEDGKKVDSSRDRNKP FKFMLGKQEVIRGWEEGVAQMSV GQRAKLTISPDYAYGATGHPGIIPP HATLVFDVELLKLE |
| **Linker** | AGCGGAGGAGGATCCGGA | SGGGSG |
| **ΔCaspase-9** | GTGGACGGGTTTGGAGATGTGGAGCCCTGGAATCCCTGCGGGGCAA TGCCGATCTGGCTTACATCCTGTCTATGGAGCCTTGCGGCCACTGTCT GATCATTAACAATGTGAACTTCTGCAGAGAGAGCGGGCTGCGGACCAG AACAGGATCCAATATTGACTGTGAAAAGCTGCGGAGAAGGTTCTCTAGT CTGCACTTTATGGTCGAGGTGAAAGGCGATCTGACCGCTAAGAAAATG GTGCTGGCCCTGCTGGAACTGGCTCGGCAGGACCATGGGGCACTGGA TTGCTGCGTGGTCGTGATCCTGAGTCACGGCTGCCAGGCTTCACATCT GCAGTTCCCTGGGGCAGTCTATGGAACTGACGGCTGTCCAGTCAGCGT GGAGAAGATCGTGAACATCTTCAACGGCACCTCTTGCCCAAGTCTGGG CGGGAAGCCCAAACTGTTCTTTATTCAGGCCTGTGGAGGCGAGCAGAA AGATCACGGCTTCGAAGTGGCTAGCACCTCCCCCGAGGACGAATCACC TGGAAGCAACCCTGAGCCAGATGCAACCCCCTTCCAGGAAGGCCTGAG GACATTTGACCAGCTGGATGCCATCTCAAGCCTGCCCACACCTTCTGAC ATTTTCGTCTCTTACAGTACTTTCCCTGGATTTGTGAGCTGGCGCGATC CAAAGTCAGGCAGCTGGTACGTGGAGACACTGGACGATATCTTTGAGC AGTGGGCCCATTCTGAAGACCTGCAGAGTCTGCTGCTGCGAGTGGCCA ATGCTGTCTCTGTGAAGGGGATCTACAAACAGATGCCAGGATGCTTCAA CTTTCTGAGAAAGAAACTGTTCTTTAAGACCTCCGCATCTAGGGCC | VDGFGDVGALESLRGNADLAYILS MEPCGHCLIINNVNFCRESGLRTR TGSNIDCEKLRRRFSSLHFMVEVK GDLTAKKMVLALLELARQDHGALD CCVVVILSHGCQASHLQFPGAVY GTDGCPVSVEKIVNIFNGTSCPSL GGKPKLFFIQACGGEQKDHGFEV ASTSPEDESPGSNPEPDATPFQE GLRTFDQLDAISSLPTPSDIFVSYS TFPGFVSWRDPKSGSWYVETLDD IFEQWAHSEDLQSLLLRVANAVSV KGIYKQMPGCFNFLRKKLFFKTSA SRA **SEQ ID NO: 300** |
| **Linker** | CCGCGG | PR |
| **T2A** | GAAGGCCGAGGGAGCCTGCTGACATGTGGCGATGTGGAGGAAAACCC AGGACCA | EGRGSLLTCGDVEENPGP |
| **Linker (NcoI)** | Ccatgg | PW |
| **Sig Peptide** | ATGGAGTTTGGACTTTCTTGGTTGTTTTGGTGGCAATTCTGAAGGGTG TCCAGTGTAGCAGG | MEFGLSWLFLVAILKGVQCSR |
| **FMC63-VL** | GACATCCAGATGACACAGACTACATCCTCCCTGTCTGCCTCTCTGGGA GACAGAGTCACCATCAGTTGCAGGGCAAGTCAGGACATTAGTAAATATT TAAATTGGTATCAGCAGAAACCAGATGGAACTGTTAAACTCCTGATCTA CCATACATCAAGATTACACTCAGGAGTCCCATCAAGGTTCAGTGGCAGT GGGTCTGGAACAGATTATTCTCTCACCATTAGCAACCTGGAGCAAGAAG ATATTGCCACTTACTTTTGCCAACAGGGTAATACGCTTCCGTACACGTT CGGAGGGGGGACTAAGTTGGAAATAACA | DIQMTQTTSSLSASLGDRVTISCR ASQDISKYLNWYQQKPDGTVKLLI YHTSRLHSGVPSRFSGSGSGTDY SLTISNLEQEDIATYFCQQGNTLPY TFGGGTKLEIT |
| **Flex-linker** | GGCGGAGGAAGCGGAGGTGGGGGC | GGGSGGGG |
| **FMC63-VH** | GAGGTGAAACTGCAGGAGTCAGGACCTGGCCTGGTGGCGCCCTCACA GAGCCTGTCCGTCACATGCACTGTCTCAGGGGTCTCATTACCCGACTAT GGTGTAAGCTGGATTCGCCAGCCTCCACGAAAGGGTCTGGAGTGGCTG GGAGTAATATGGGGTAGTGAAACCACATACTATAATTCAGCTCTCAAAT CCAGACTGACCATCATCAAGGACAACTCCAAGAGCCAAGTTTTCTTAAA AATGAACAGTCTGCAAACTGATGACACAGCCATTTACTACTGTGCCAAA CATTATTACTACGGTGGTAGCTATGCTATGGACTACTGGGGTCAAGGAA CCTCAGTCACCGTCTCCTCA | EVKLQESGPGLVAPSQSLSVTCTV SGVSLPDYGVSWIRQPPRKGLEW LGVIWGSETTYYNSALKSRLTIIKD NSKSQVFLKMNSLQTDDTAIYYCA KHYYYGGSYAMDYWGQGTSVTV SS |
| **Linker(BamHI)** | GGATCC | GS |
| **CD34 epitope** | GAACTTCCTACTCAGGGGACTTTCTCAAACGTTAGCACAAACGTAAGT | ELPTQGTFSNVSTNVS |
| **CD8a stalk** | CCCGCCCCAAGACCCCCCACACCTGCGCCGACCATTGCTTCTCAACCC CTGAGTTTGAGACCCGAGGCCTGCCGGCCAGCTGCCGGCGGGGCCGT GCATACAAGAGGACTCGATTTCGCTTGCGAC | PAPRPPTPAPTIASQPLSLRPEAC RPAAGGAVHTRGLDFACD |
| **CD8tm + stop tf** | ATCTATATCTGGGCACCTCTCGCTGGCACCTGTGGAGTCCTTCTGCTCA GCCTGGTTATTACTCTGTACTGTAATCACCGGAATCGCCGCCGCGTTTG TAAGTGTCCCAGG | IYIWAPLAGTCGVLLLSLVITLYCN HRNRRRVCKCPR |
| **Linker (SalI)** | gtcgac | VD |
| **MyD88** | ATGGCCGCTGGGGGCCCAGGCGCCGGATCAGCTGCTCCCGTATCTTC TACTTCTTCTTTGCCGCTGGCTGCTCTGAACATGCGCGTGAGAAGACG CCTCTCCCTGTTCCTTAACGTTCGCACACAAGTCGCTGCCGATTGGACC GCCCTTGCCGAAGAAATGGACTTTGAATACCTGGAAATTAGACAACTTG AAACACAGGCCGACCCCACTGGCAGACTCCTGGACGCATGGCAGGGA AGACCTGGTGCAAGCGTTGGACGGCTCCTGGATCTCCTGACAAAACTG GGACGCGACGACGTACTGCTTGAACTCGGACCTAGCATTGAAGAAGAC TGCCAAAAATATATCCTGAAACAACAACAAGAAGAAGCCGAAAAACCTC TCCAAGTCGCAGCAGTGGACTCATCAGTACCCCGAACAGCTGAGCTTG CTGGGATTACTACACTCGACGACCCACTCGGACATATGCCTGAAAGATT CGACGCTTTCATTTGCTATTGCCCCTCTGACATA | MAAGGPGAGSAAPVSSTSSLPLA ALNMRVRRRLSLFLNVRTQVAAD WTALAEEMDFEYLEIRQLETQADP TGRLLDAWQGRPGASVGRLLDLL TKLGRDDVLLELGPSIEEDCQKYIL KQQQEEAEKPLQVAAVDSSVPRT AELAGITTLDDPLGHMPERFDAFIC YCPSDI |
| **dCD40** | AAGAAAGTTGCAAAGAAACCCACAAATAAAGCCCACACCCTAAACAGG AACCCCAAGAAATCAATTTCCCAGATGATCTCCCTGGATCTAATACTGC CGCCCCGGTCCAAGAAACCCTGCATGGTTGCCAGCCTGTCACCCAAGA GGACGGAAAAGAATCACGGATTAGCGTACAAGAGAGACAA | KKVAKKPTNKAPHPKQEPQEINFP DDLPGSNTAAPVQETLHGCQPVT QEDGKESRISVQERQ |
| **CD3z** | AGAGTGAAGTTCAGCAGGAGCGCAGACGCCCCCGCGTACCAGCAGGG CCAGAACCAGCTCTATAACGAGCTCAATCTAGGACGAAGAGAGGAGTA CGATGTTTTGGACAAGAGACGTGGCCGGGACCCTGAGATGGGGGGAA AGCCGAGAAGGAAGAACCCTCAGGAAGGCCTGTACAATGAACTGCAGA AAGATAAGATGGCGGAGGCCTACAGTGAGATTGGGATGAAAGGCGAGC GCCGGAGGGGCAAGGGGCACGATGGCCTTTACCAGGGTCTCAGTACA GCCACCAAGGACACCTACGACGCCCTTCACATGCAAGCTCTTCCACCT CGt | RVKFSRSADAPAYQQGQNQLYNE LNLGRREEYDVLDKRRGRDPEMG GKPRRKNPQEGLYNELQKDKMAE AYSEIGMKGERRRGKGHDGLYQG LSTATKDTYDALHMQALPPR |
| **Linker** | Acg | T |
| **FRBI^^** | TGGCACGAAGGCCTGGAAGAGGCCTCAAGACTTTACTTTGGTGAACGC AACGTTAAAGGCATGTTCGAGGTGCTGGAACCCTTGCATGCAATGATG GAGCGAGGTCCTCAGACACTCAAAGAGACATCTTTTAACCAGGCGTAT GGACGGGACCTCATGGAGGCTCAGGAATGGTGCCGCAAGTACATGAAA AGTGGGAATGTGAAGGATCTGCTGCAAGCATGGGATCTGTATTACCAC GTGTTTAGACGGATCAGCAAA | WHEGLEEASRLYFGERNVKGMFE VLEPLHAMMERGPQTLKETSFNQ AYGRDLMEAQEWCRKYMKSGNV KDLLQAWDLYYHVFRRISK |
| **Linker (BsiWI)** | Cgtacg | RT |
| **FRBI** | TGGCATGAAGGGTTGGAAGAAGCTTCAAGGCTGTACTTCGGAGAGAGG AACGTGAAGGGCATGTTTGAGGTTCTTGAACCTCTGCACGCCATGATG GAACGGGGACCGCAGACACTGAAAGAAACCTCTTTTAATCAGGCCTAC GGCAGAGACCTGATGGAGGCCCAAGAATGGTGTAGAAAGTATATGAAA TCCGGTAACGTGAAAGACCTGCTCCAGGCCTGGGACCTTTATTACCAT GTGTTCAGGCGGATCAGTAAGTAA | WHEGLEEASRLYFGERNVKGMFE VLEPLHAMMERGPQTLKETSFNQ AYGRDLMEAQEWCRKYMKSGNV KDLLQAWDLYYHVFRRISK* |

**TABLE 8**

| **Fragment** | **Nucleotide** | **Polypeptide** |
|---|---|---|
| FKBP12v36 | ATGGGAGTGCAGGTGGAGACTATTAGCCCCGGAGATGGCAGAACATTC CCCAAAAGAGGACAGACTTGCGTCGTGCATTATACTGGAATGCTGGAA GACGGCAAGAAGGTGGACAGCAGCCGGGACCGAAACAAGCCCTTCAA GTTCATGCTGGGGAAGCAGGAAGTGATCCGGGGCTGGGAGGAAGGAG TCGCACAGATGTCAGTGGGACAGAGGGCCAAACTGACTATTAGCCCAG ACTACGCTTATGGAGCAACCGGCCACCCCGGGATCATTCCCCCTCATG CTACACTGGTCTTCGATGTGGAGCTGCTGAAGCTGGAA | MGVQVETISPGDGRTFPKRGQTC VVHYTGMLEDGKKVDSSRDRNKP FKFMLGKQEVIRGWEEGVAQMSV GQRAKLTISPDYAYGATGHPGIIPP HATLVFDVELLKLE |
| Linker | AGCGGAGGAGGATCCGGA | SGGGSG |
| dCaspase9 | GTGGACGGGTTTGGAGATGTGGGAGCCCTGGAATCCCTGCGGGGCAA TGCCGATCTGGCTTACATCCTGTCTATGGAGCCTTGCGGCCACTGTCT GATCATTAACAATGTGAACTTCTGCAGAGAGAGCGGGCTGCGGACCAG AACAGGATCCAATATTGACTGTGAAAAGCTGCGGAGAAGGTTCTCTAGT CTGCACTTTATGGTCGAGGTGAAAGGCGATCTGACCGCTAAGAAAATG GTGCTGGCCCTGCTGGAACTGGCTCGGCAGGACCATGGGGCACTGGA TTGCTGCGTGGTCGTGATCCTGAGTCACGGCTGCCAGGCTTCACATCT GCAGTTCCCTGGGGCAGTCTATGGAACTGACGGCTGTCCAGTCAGCGT GGAGAAGATCGTGAACATCTTCAACGGCACCTCTTGCCCAAGTCTGGG CGGGAAGCCCAAACTGTTCTTTATTCAGGCCTGTGGAGGCGAGCAGAA AGATCACGGCTTCGAAGTGGCTAGCACCTCCCCCGAGGACGAATCACC TGGAAGCAACCCTGAGCCAGATGCAACCCCCTTCCAGGAAGGCCTGAG GACATTTGACCAGCTGGATGCCATCTCAAGCCTGCCCACACCTTCTGAC ATTTTCGTCTCTTACAGTACTTTCCCTGGATTTGTGAGCTGGCGCGATC CAAAGTCAGGCAGCTGGTACGTGGAGACACTGGACGATATCTTTGAGC AGTGGGCCCATTCTGAAGACCTGCAGAGTCTGCTGCTGCGAGTGGCCA ATGCTGTCTCTGTGAAGGGGATCTACAAACAGATGCCAGGATGCTTCAA CTTTCTGAGAAAGAAACTGTTCTTTAAGACCTCCGCATCTAGGGCC | VDGFGDVGALESLRGNADLAYILS MEPCGHCLIINNVNFCRESGLRTR TGSNIDCEKLRRRFSSLHFMVEVK GDLTAKKMVLALLELARQDHGALD CCVVVILSHGCQASHLQFPGAVY GTDGCPVSVEKIVNIFNGTSCPSL GGKPKLFFIQACGGEQKDHGFEV ASTSPEDESPGSNPEPDATPFQE GLRTFDQLDAISSLPTPSDIFVSYS TFPGFVSWRDPKSGSWYVETLDD IFEQWAHSEDLQSLLLRVANAVSV KGIYKQMPGCFNFLRKKLFFKTSA SRA |
| Linker (SacII) | CCGCGG | PR |
| T2A | GAGGGCAGGGGAAGTCTTCTAACATGCGGGGACGTGGAGGAAAATCC CGGGCCC | EGRGSLLTCGDVEENPGP |
| Linker (NcoI) | GCATGCGCCACC | ACAT |
| Sig Peptide | ATGGAGTTTGGGTTGTCATGGTTGTTTCTCGTCGCTATTCTCAAAGGTG TACAATGCTCCCGC | MEFGLSWLFLVAILKGVQCSR |
| FRP5-VH | GAAGTCCAATTGCAACAGTCAGGCCCCGAATTGAAAAAGCCCGGCGAA ACAGTGAAGATATCTTGTAAAGCCTCCGGTTACCCTTTTACGAACTATG GAATGAACTGGGTCAAACAAGCCCCTGGACAGGGATTGAAGTGGATGG GATGGATCAATACATCAACAGGCGAGTCTACCTTCGCAGATGATTTCAA AGGTCGCTTTGACTTCTCACTGGAGACCAGTGCAAATACCGCCTACCTT CAGATTAACAATCTTAAAAGCGAGGATATGGCAACCTACTTTTGCGCAA GATGGGAAGTTTATCACGGGTACGTGCCATACTGGGGACAAGGAACGA CAGTGACAGTTAGTAGC | EVQLQQSGPELKKPGETVKISCKA SGYPFTNYGMNWVKQAPGQGLK WMGWINTSTGESTFADDFKGRFD FSLETSANTAYLQINNLKSEDMAT YFCARWEVYHGYVPYWGQGTTV TVSS |
| Flex-linker | GGCGGTGGAGGCTCCGGTGGAGGCGGCTCTGGAGGAGGAGGTTCA | GGGGSGGGGSGGGGS |
| FRP5VL | GACATCCAATTGACACAATCACACAAATTTCTCTCAACTTCTGTAGGAGA CAGAGTGAGCATAACCTGCAAAGCATCCCAGGACGTGTACAATGCTGT GGCTTGGTACCAACAGAAGCCTGGACAATCCCCAAAATTGCTGATTTAT TCTGCCTCTAGTAGGTACACTGGGGTACCTTCTCGGTTTACGGCTCTG GGTCCGGACCAGATTTCACGTTCACAATCAGTTCCGTTCAAGCTGAAGA CCTCGCTGTTTATTTTTGCCAGCAGCACTTCCGAACCCCTTTTACTTTTG | DIQLTQSHKFLSTSVGDRVSITCKA SQDVYNAVAWYQQKPGQSPKLLI YSASSRYTGVPSRFTGSGSGPDF TFTISSVQAEDLAVYFCQQHFRTP FTFGSGTKLEIKAL |
| | GCTCAGGCACTAAGTTGGAAATCAAGGCTTTG | |
| Linker(NsiI) | Atgcat | MH |
| CD34 epitope | GAACTTCCTACTCAGGGGACTTTCTCAAACGTTAGCACAAACGTAAGT | ELPTQGTFSNVSTNVS |
| CD8a stalk | CCCGCCCCAAGACCCCCACACCTGCGCCGACCATTGCTTCTCAACCC CTGAGTTTGAGACCCGAGGCCTGCCGGCCAGCTGCCGGCGGGGCCGT GCATACAAGAGGACTCGATTTCGCTTGCGAC | PAPRPPTPAPTIASQPLSLRPEAC RPAAGGAVHTRGLDFACD |
| CD8tm + stop tf | ATCTATATCTGGGCACCTCTCGCTGGCACCTGTGGAGTCCTTCTGCTCA GCCTGGTTATTACTCTGTACTGTAATCACCGGAATCGCCGCCGCGTTTG TAAGTGTCCCAGG | IYIWAPLAGTCGVLLLSLVITLYCN HRNRRRVCKCPR |
| Linker (SalI) | gtcgac | VD |
| MyD88 | ATGGCCGCTGGGGGCCCAGGCGCCGGATCAGCTGCTCCCGTATCTTC TACTTCTTCTTTGCCGCTGGCTGCTCTGAACATGCGCGTGAGAAGACG CCTCTCCCTGTTCCTTAACGTTCGCACACAAGTCGCTGCCGATTGGACC GCCCTTGCCGAAGAAATGGACTTTGAATACCTGGAAATTAGACAACTTG AAACACAGGCCGACCCCACTGGCAGACTCCTGGACGCATGGCAGGGA AGACCTGGTGCAAGCGTTGGACGGCTCCTGGATCTCCTGACAAAACTG GGACGCGACGACGTACTGCTTGAACTCGGACCTAGCATTGAAGAAGAC TGCCAAAAATATATCCTGAAACAACAACAAGAAGAAGCCGAAAAACCTC TCCAAGTCGCAGCAGTGGACTCATCAGTACCCCGAACAGCTGAGCTTG CTGGGATTACTACACTCGACGACCCACTCGGACATATGCCTGAAAGATT CGACGCTTTCATTTGCTATTGCCCCTCTGACATA | MAAGGPGAGSAAPVSSTSSLPLA ALNMRVRRRLSLFLNVRTQVAAD WTALAEEMDFEYLEIRQLETQADP TGRLLDAWQGRPGASVGRLLDLL TKLGRDDVLLELGPSIEEDCQKYIL KQQQEEAEKPLQVAAVDSSVPRT AELAGITTLDDPLGHMPERFDAFIC YCPSDI |
| dCD40 | AAGAAAGTTGCAAAGAAACCCACAAATAAAGCCCCACACCCTAAACAGG AACCCCAAGAAATCAATTTCCCAGATGATCTCCCTGGATCTAATACTGC CGCCCCGGTCCAAGAAACCCTGCATGGTTGCCAGCCTGTCACCCAAGA GGACGGAAAAGAATCACGGATTAGCGTACAAGAGAGACAA | KKVAKKPTNKAPHPKQEPQEINFP DDLPGSNTAAPVQETLHGCQPVT QEDGKESRISVQERQ |
| CD3z | AGAGTGAAGTTCAGCAGGAGCGCAGACGCCCCCGCGTACCAGCAGGG CCAGAACCAGCTCTATAACGAGCTCAATCTAGGACGAAGAGAGGAGTA CGATGTTTTGGACAAGAGACGTGGCCGGGACCCTGAGATGGGGGGAA AGCCGAGAAGGAAGAACCCTCAGGAAGGCCTGTACAATGAACTGCAGA AAGATAAGATGGCGGAGGCCTACAGTGAGATTGGGATGAAAGGCGAGC GCCGGAGGGGCAAGGGGCACGATGGCCTTTACCAGGGTCTCAGTACA GCCACCAAGGACACCTACGACGCCCTTCACATGCAAGCTCTTCCACCT CGt | RVKFSRSADAPAYQQGQNQLYNE LNLGRREEYDVLDKRRGRDPEMG GKPRRKNPQEGLYNELQKDKMAE AYSEIGMKGERRRGKGHDGLYQG LSTATKDTYDALHMQALPPR |
| Linker | Acg | T |
| FRBI^^ | TGGCACGAAGGCCTGGAAGAGGCCTCAAGACTTTACTTTGGTGAACGC AACGTTAAAGGCATGTTCGAGGTGCTGGAACCCTTGCATGCAATGATG GAGCGAGGTCCTCAGACACTCAAAGAGACATCTTTTAACCAGGCGTAT GGACGGGACCTCATGGAGGCTCAGGAATGGTGCCGCAAGTACATGAAA AGTGGGAATGTGAAGGATCTGCTGCAAGCATGGGATCTGTATTACCAC GTGTTTAGACGGATCAGCAAA | WHEGLEEASRLYFGERNVKGMFE VLEPLHAMMERGPQTLKETSFNQ AYGRDLMEAQEWCRKYMKSGNV KDLLQAWDLYYHVFRRISK |
| Linker (BsiWI) | Cgtacg | RT |
| FRBI | TGGCATGAAGGGTTGGAAGAAGCTTCAAGGCTGTACTTCGGAGAGAGG AACGTGAAGGGCATGTTTGAGGTTCTTGAACCTCTGCACGCCATGATG GAACGGGGACCGCAGACACTGAAAGAAACCTCTTTTAATCAGGCCTAC GGCAGAGACCTGATGGAGGCCCAAGAATGGTGTAGAAAGTATATGAAA TCCGGTAACGTGAAAGACCTGCTCCAGGCCTGGGACCTTTATTACCAT GTGTTCAGGCGGATCAGTAAGTAA | WHEGLEEASRLYFGERNVKGMFE VLEPLHAMMERGPQTLKETSFNQ AYGRDLMEAQEWCRKYMKSGNV KDLLQAWDLYYHVFRRISK* |

**Table 9 pBP0545.pSFG.iCasp9.2A.Her2scFv.Q.CD8stm.MC-zeta**

| Fragment | Nucleotide | Polypeptide |
|---|---|---|
| **Kozak (ribosome- binding seq.)** | GCCACC | N/A |
| **FKBP12v36** | ATGGGAGTGCAGGTGGAGACTATTAGCCCCGGAGATGGCAGAACATTC CCCAAAAGAGGACAGACTTGCGTCGTGCATTATACTGGAATGCTGGAA GACGGCAAGAAGGTGGACAGCAGCCGGGACCGAAACAAGCCCTTCAA GTTCATGCTGGGGAAGCAGGAAGTGATCCGGGGCTGGGAGGAAGGAG TCGCACAGATGTCAGTGGGACAGAGGGCCAAACTGACTATTAGCCCAG ACTACGCTTATGGAGCAACCGGCCACCCCGGGATCATTCCCCCTCATG CTACACTGGTCTTCGATGTGGAGCTGCTGAAGCTGGAA | MGVQVETISPGDGRTFPKRGQTC VVHYTGMLEDGKKVDSSRDRNKP FKFMLGKQEVIRGWEEGVAQMSV GQRAKLTISPDYAYGATGHPGIIPP HATLVFDVELLKLE |
| **Linker** | AGCGGAGGAGGATCCGGA | SGGGSG |
| **ΔCaspase9** | GTGGACGGGTTTGGAGATGTGGAGCCCTGGAATCCCTGCGGGGCAA TGCCGATCTGGCTTACATCCTGTCTATGGAGCCTTGCGGCCACTGTCT GATCATTAACAATGTGAACTTCTGCAGAGAGAGCGGGCTGCGGACCAG AACAGGATCCAATATTGACTGTGAAAAGCTGCGGAGAAGGTTCTCTAGT CTGCACTTTATGGTCGAGGTGAAAGGCGATCTGACCGCTAAGAAAATG GTGCTGGCCCTGCTGGAACTGGCTCGGCAGGACCATGGGGCACTGGA TTGCTGCGTGGTCGTGATCCTGAGTCACGGCTGCCAGGCTTCACATCT GCAGTTCCCTGGGGCAGTCTATGGAACTGACGGCTGTCCAGTCAGCGT GGAGAAGATCGTGAACATCTTCAACGGCACCTCTTGCCCAAGTCTGGG CGGGAAGCCCAAACTGTTCTTTATTCAGGCCTGTGGAGGCGAGCAGAA AGATCACGGCTTCGAAGTGGCTAGCACCTCCCCCGAGGACGAATCACC TGGAAGCAACCCTGAGCCAGATGCAACCCCCTTCCAGGAAGGCCTGAG GACATTTGACCAGCTGGATGCCATCTCAAGCCTGCCCACACCTTCTGAC ATTTTCGTCTCTTACAGTACTTTCCCTGGATTTGTGAGCTGGCGCGATC CAAAGTCAGGCAGCTGGTACGTGGAGACACTGGACGATATCTTTGAGC AGTGGGCCCATTCTGAAGACCTGCAGAGTCTGCTGCTGCGAGTGGCCA ATGCTGTCTCTGTGAAGGGGATCTACAAACAGATGCCAGGATGCTTCAA CTTTCTGAGAAAGAAACTGTTCTTTAAGACCTCCGCATCTAGGGCC | VDGFGDVGALESLRGNADLAYILS MEPCGHCLIINNVNFCRESGLRTR TGSNIDCEKLRRRFSSLHFMVEVK GDLTAKKMVLALLELARQDHGALD CCVVVILSHGCQASHLQFPGAVY GTDGCPVSVEKIVNIFNGTSCPSL GGKPKLFFIQACGGEQKDHGFEV ASTSPEDESPGSNPEPDATPFQE GLRTFDQLDAISSLPTPSDIFVSYS TFPGFVSWRDPKSGSWYVETLDD IFEQWAHSEDLQSLLLRVANAVSV KGIYKQMPGCFNFLRKKLFFKTSA SRA |
| **Linker (SacII)** | CCGCGG | PR |
| **T2A** | GAGGGCAGGGGAAGTCTTCTAACATGCGGGGACGTGGAGGAAAATCC CGGGCCC | EGRGSLLTCGDVEENPGP |
| **Linker (NcoI)** | GCATGCGCCACC | ACAT |
| **Sig Peptide** | ATGGAGTTTGGGTTGTCATGGTTGTTTCTCGTCGCTATTCTCAAAGGTG TACAATGCTCCCGC | MEFGLSWLFLVAILKGVQCSR |
| **FRP5-VH** (**anti-Her2)** | GAAGTCCAATTGCAACAGTCAGGCCCCGAATTGAAAAAGCCCGGCGAA ACAGTGAAGATATCTTGTAAAGCCTCCGGTTACCCTTTTACGAACTATG GAATGAACTGGGTCAAACAAGCCCCTGGACAGGGATTGAAGTGGATGG GATGGATCAATACATCAACAGGCGAGTCTACCTTCGCAGATGATTTCAA AGGTCGCTTTGACTTCTCACTGGAGACCAGTGCAAATACCGCCTACCTT CAGATTAACAATCTTAAAAGCGAGGATATGGCAACCTACTTTTGCGCAA GATGGGAAGTTTATCACGGGTACGTGCCATACTGGGGACAAGGAACGA CAGTGACAGTTAGTAGC | EVQLQQSGPELKKPGETVKISCKA SGYPFTNYGMNWVKQAPGQGLK WMGWINTSTGESTFADDFKGRFD FSLETSANTAYLQINNLKSEDMAT YFCARWEVYHGYVPYWGQGTTV TVSS |
| **Flex-linker** | GGCGGTGGAGGCTCCGGTGGAGGCGGCTCTGGAGGAGGAGGTTCA | GGGGSGGGGSGGGGS |
| **FRP5VL (anti-Her2)** | GACATCCAATTGACACAATCACACAAATTTCTCTCAACTTCTGTAGGAGA CAGAGTGAGCATAACCTGCAAAGCATCCCAGGACGTGTACAATGCTGT GGCTTGGTACCAACAGAAGCCTGGACAATCCCCAAAATTGCTGATTTAT TCTGCCTCTAGTAGGTACACTGGGGTACCTTCTCGGTTTACGGGCTCTG GGTCCGGACCAGATTTCACGTTCACAATCAGTTCCGTTCAAGCTGAAGA CCTCGCTGTTTATTTTTGCCAGCAGCACTTCCGAACCCCTTTTACTTTTG GCTCAGGCACTAAGTTGGAAATCAAGGCTTTG | DIQLTQSHKFLSTSVGDRVSITCKA SQDVYNAVAWYQQKPGQSPKLLI YSASSRYTGVPSRFTGSGSGPDF TFTISSVQAEDLAVYFCQQHFRTP FTFGSGTKLEIKAL |
| **Linker(NsiI)** | Atgcat | MH |
| **CD34 epitope** | GAACTTCCTACTCAGGGGACTTTCTCAAACGTTAGCACAAACGTAAGT | ELPTQGTFSNVSTNVS |
| **CD8a stalk** | CCCGCCCCAAGACCCCCACACCTGCGCCGACCATTGCTTCTCAACCC CTGAGTTTGAGACCCGAGGCCTGCCGGCCAGCTGCCGGCGGGGCCGT GCATACAAGAGGACTCGATTTCGCTTGCGAC | PAPRPPTPAPTIASQPLSLRPEAC RPAAGGAVHTRGLDFACD |
| **CD8tm + stop tf** | ATCTATATCTGGGCACCTCTCGCTGGCACCTGTGGAGTCCTTCTGCTCA GCCTGGTTATTACTCTGTACTGTAATCACCGGAATCGCCGCCGCGTTTG TAAGTGTCCCAGG | IYIWAPLAGTCGVLLLSLVITLYCN HRNRRRVCKCPR |
| **Linker (SalI)** | gtcgac | VD |
| **MyD88** | ATGGCCGCTGGGGGCCCAGGCGCCGGATCAGCTGCTCCCGTATCTTC TACTTCTTCTTTGCCGCTGGCTGCTCTGAACATGCGCGTGAGAAGACG CCTCTCCCTGTTCCTTAACGTTCGCACACAAGTCGCTGCCGATTGGACC GCCCTTGCCGAAGAAATGGACTTTGAATACCTGGAAATTAGACAACTTG AAACACAGGCCGACCCCACTGGCAGACTCCTGGACGCATGGCAGGGA AGACCTGGTGCAAGCGTTGGACGGCTCCTGGATCTCCTGACAAAACTG GGACGCGACGACGTACTGCTTGAACTCGGACCTAGCATTGAAGAAGAC TGCCAAAAATATATCCTGAAACAACAACAAGAAGAAGCCGAAAAACCTC TCCAAGTCGCAGCAGTGGACTCATCAGTACCCCGAACAGCTGAGCTTG CTGGGATTACTACACTCGACGACCCACTCGGACATATGCCTGAAAGATT CGACGCTTTCATTTGCTATTGCCCCTCTGACATA | MAAGGPGAGSAAPVSSTSSLPLA ALNMRVRRRLSLFLNVRTQVAAD WTALAEEMDFEYLEIRQLETQADP TGRLLDAWQGRPGASVGRLLDLL TKLGRDDVLLELGPSIEEDCQKYIL KQQQEEAEKPLQVAAVDSSVPRT AELAGITTLDDPLGHMPERFDAFIC YCPSDI |
| **dCD40** | AAGAAAGTTGCAAAGAAACCCACAAATAAAGCCCCACACCCTAAACAGG AACCCCAAGAAATCAATTTCCCAGATGATCTCCCTGGATCTAATACTGC CGCCCCGGTCCAAGAAACCCTGCATGGTTGCCAGCCTGTCACCCAAGA GGACGGAAAAGAATCACGGATTAGCGTACAAGAGAGACAA | KKVAKKPTNKAPHPKQEPQEINFP DDLPGSNTAAPVQETLHGCQPVT QEDGKESRISVQERQ |
| **CD3z** | AGAGTGAAGTTCAGCAGGAGCGCAGACGCCCCCGCGTACCAGCAGGG CCAGAACCAGCTCTATAACGAGCTCAATCTAGGACGAAGAGAGGAGTA CGATGTTTTGGACAAGAGACGTGGCCGGGACCCTGAGATGGGGGGAA AGCCGAGAAGGAAGAACCCTCAGGAAGGCCTGTACAATGAACTGCAGA AAGATAAGATGGCGGAGGCCTACAGTGAGATTGGGATGAAAGGCGAGC GCCGGAGGGGCAAGGGGCACGATGGCCTTTACCAGGGTCTCAGTACA GCCACCAAGGACACCTACGACGCCCTTCACATGCAAGCTCTTCCACCT CGTtga | RVKFSRSADAPAYQQGQNQLYNE LNLGRREEYDVLDKRRGRDPEMG GKPRRKNPQEGLYNELQKDKMAE AYSEIGMKGERRRGKGHDGLYQG LSTATKDTYDALHMQALPPR* |

Methods discussed herein, including, but not limited to, methods for constructing vectors, assays for activity or function, administration to patients, transfecting or transforming cells, assay, and methods for monitoring patients may also be found in the following patents and patent applications.

U.S. Patent Application Serial Number 14/210,034, titled METHODS FOR CONTROLLING T CELL PROLIFERATION, filed March 13, 2014; U.S. Patent Application Serial Number 13/112,739, filed May 20, 2011, , issued as U.S. Patent 9,089,520, July 28, 2015, and entitled METHODS FOR INDUCING SELECTIVE APOPTOSIS; U.S. Patent Application Serial Number 14/622,018, filed February 13, 2014, titled METHODS FOR ACTIVATING T CELLS USING AN INDUCIBLE CHIMERIC POLYPEPTIDE; ;U.S. Patent application Serial Number 13/112,739, filed May 20, 2011, titled METHODS FOR INDUCING SELECTIVE APOPTOSIS; U.S. Patent Application Serial Number 13/792,135, filed March 10, 2013, titled MODIFIED CASPASE POLYPEPTIDES AND USES THEREOF; U.S. Patent Application Serial Number 14/296,404, filed June 4, 2014, titled METHODS FOR INDUCING PARTIAL APOPTOSIS USING CASPASE POLYPEPTIDES; U.S. Provisional Patent Application Serial Number 62/044,885, filed September 2, 2014, and U.S. Patent Application 14/842,710, filed September 1, 2015, each titled COSTIMULATION OF CHIMERIC ANTIGEN RECEPTORS BY MyD88 AND CD40 POLYPEPTIDES; U.S. Patent Application Serial Number 14/640,554, filed 6 March, 2015, titled CASPASE POLYPEPTIDES HAVING MODIFIED ACTIVITY AND USES THEREOF; U.S. Patent Number 7,404,950, issued June 29, 2008, to Spencer, D. et al., U.S. Patent applications 12/445,939 by Spencer, D., et al., filed October 26, 2010; U.S. Patent application 12/563,991 by Spencer, D., et al., filed September 21, 2009; 13/087,329 by Slawin, K., et al., filed April 14, 2011; 13/763,591 by Spencer, D., et al., filed February 8, 2013; and International Patent Application Number PCT/US2014/022004, filed 7 March 2014, published as PCT/US2014/022004 on 9 October 2014, titled MODIFIED CASPASE POLYPEPTIDES AND USES THEREOF.

### Example 18: FRB-based scaffold assembly and activation of iCaspase-9.

To determine if iCaspase-9 could be aggregated by tandem multimers of FRB_{L}, one to four tandem copies of FRB_{L} were subcloned into an expression vector, pSH1, driving transgene expression from an SRa promoter. A subset of constructs also contained the myristoylation-targeting domain from v-Src for membrane localization of the FRB-scaffold (Fig. 12A). 293 cells were transfected with the SRα-SEAP reporter plasmid along with FKBP12-ΔCaspase-9 (iCaspase-9/CaspaCIDe), plus 1 of several FRB-based, non-myristoylated scaffold proteins containing 0, 1, or 4 tandem copies of FRB_{L}. Addition of either rapamycin or analog, C7-isopropoxy-rapamycin, created by the method of Luengo et al., (Luengo JI (95) Chem & Biol 2, 471-81. Luengo JI (94) J. Org Chem 59: 6512-13), led to a diminution of reporter activity when the 4x FRB construct was present, consistent with cell death, as predicted (Fig. 8B, 10D, 10E) with a IC₅₀ ∼ 3 nM (Fig. 12B). Addition of rapamycin had no effect on reporter activity when only 1 (or 0) FRB domain was present, which would preclude oligomerization of iCasp9 (Fig. 10C). Similar results were obtained when the FRB-scaffold was myristoylated (Fig. 12C) to localize the scaffold to the plasma membrane. Thus, the Caspase-9 polypeptide can be activated with rapamycin or analogs when oligomerized on a FRB-based scaffold.

### Example 19: FKBP12-based scaffolds assemble and activate FRB-ΔCaspase-9.

To determine if the polarity of heterodimerization and Caspase-9 assembly could be reversed, one to four 1 to 4 tandem copies of FKBP12 were subcloned into expression vector, pSH1, as above. (Fig. 13A). As above, 293 cells were transfected with the SRα-SEAP reporter plasmid along with FRBL-ΔCaspase-9, plus a non-myristoylated scaffold protein containing 1 or 4 tandem copies of FKBP12. Addition of either rapamycin or analog, C7-isopropoxy-rapamycin, led to a diminution of reporter activity when the 4x FRB_{L} construct was present, consistent with cell death with a IC₅₀ ∼ 3 nM (Fig. 13B). Addition of rapamycin had no effect on reporter activity when only 1 (or 0) FKBP domain was present, similar to the results in Fig. 12. Thus, Caspase-9 can be activated with rapamycin or analogs when oligomerized on a FRB or FKBP12-based scaffold.

### Example 20: FRB-based scaffold assembly and activation of iCaspase-9 in primary T cells.

To determine if iCaspase-9 could be aggregated by tandem multimers of FRB_{L} in primary, non-transformed T cells, zero to three 3 tandem copies of FRBL were subcloned into a retroviral expression vector, pBP0220--pSFG-iC9.T2A-ΔCD19, encoding Caspase-9 (CaspaCIDe) along with a non-signaling truncated version of CD19 that served as a surface marker. The resulting unified plasmid vectors, named pBP0756-iC9.T2A-ΔCD19.P2A-FRB_{L}, pBP0755-iC9.T2A-ΔCD19.P2A-FRB_{L}2, and pBP0757-iC9.T2A-ΔCD19.P2A-FRB_{L}3, were subsequently used to make infectious γ-retroviruses (γ-RVs) encoding scaffolds of 1, 2 or 3 tandem FRB_{L} domains, respectively.

T cells from 3 different donors were transduced with the vectors and plated with varying rapamycin dilutions. After 24 and 48 hours, cell aliquots were harvested, stained with anti-CD19 APC and analyzed by flow cytometry. Cells were initially gated on live lymphocytes by FSC vs SSC and then plotted as a CD19 histogram and subgated for high, medium and low expression within the CD19⁺ gate. Line graphs were prepared to represent the relative percentage of the total cell population that express high levels of CD19, normalized to the no "0" drug control (Fig. 14). Similar to the surrogate SEAP reporter assay performed in transformed epithelial cells, as rapamycin concentration increased, the percentage of CD19hi cells decreased in cells expressing Caspase-9 and FRB_{L}2 or FRB_{L}3, but not in cells expressing Caspase-9 along with 0 or 1 FRB_{L} domains, indicating that rapamycin induces heterodimerization between the FRB-based scaffolds and iCaspase9, leading to Caspase-9 dimerization and cell death. Similar results were seen when rapamycin was replaced with C7-isopropoxyrapamycin.

### Example 21: FRB-based scaffolds attached to signaling molecules can dimerize and activate iCaspase-9.

To determine if multimers of FRB would still act as a recruitment scaffold to enable rapalog-mediated Caspase-9 dimerization when attached to another signaling domain, 1 or 2 FRB_{L} domains were fused to the potent chimeric stimulatory molecule, MyD88/CD40, to derive iMC.FRB_{L} (pBP0655) and iMC.FRB_{L}2 (pBP0498), respectively (Fig. 9B). As an initial test, 293 cells were transiently transfected with reporter plasmid SRα-SEAP, Caspase-9, a 1^{st} generation anti-HER2 CAR (pBP0488) and (pBP0655 or pBP0498) (Fig. 15). Control transfections contained Caspase-9 (pBP0044) alone or eGFP expression vector (pBP0047). In the presence of rimiducid, Caspase-9-containing cells, but not control eGFP-cells, were killed by Caspase-9 homodimerization as usual, reflected by diminution of SEAP activity (Fig. 15, left); however, rapamycin only triggered SEAP reduction in cells expressing iMC.FRB_{L}2 and Caspase-9, but not cells expressing iMC.FRB_{L} and Caspase-9, or control cells. Thus, heterodimerizer-mediated activation of Caspase-9 is possible in cells containing multimers of FRB_{L} fused to distinct proteins, such as MyD88/CD40.

In a second test for rapalog-mediated scaffold-based activation of Caspase-9, 293 cells were transiently transfected with SRα-SEAP reporter plasmid, plus myristoylated or non-myristoylated inducible iMC co-expressed with 1^{st} generation anti-CD19 CAR, plus FRB_{L}2-fused Caspase-9 (plasmid pBP0467) (Fig. 16). After 24 hours, cells were treated with log dilutions of rimiducid, rapamycin, or C7-isopropoxy (IsoP)- rapamycin. Unlike FKBP12-linked Caspase-9 (CaspaCIDe), FRB_{L}2-Caspase-9 is not activated by rimiducid; however, it is activated by rapamycin or C7-isopropoxy-rapamycin when tandem FKBPs are present. Thus, rapamcyin and analogs can activate Caspase-9 via a molecular scaffold comprised of FRB or FKBP12 domains.

### Example 22: The iMC "switch", FKBPx2.MyD88.CD40, creates a scaffold for FRB_{L}2. Caspase9 in the presence of rapamycin to induce cell death.

The use of iMC as an FKBP12-based scaffold for activating FRB_{L}2-Caspase-9 was tested in primary T cells (Fig. 17). Primary T cells (2 donors) were transduced with γ-RVs derived from SFG-Δmyr.iMC.2A-CD19 (pBP0606) and SFG-FRB_{L}2.Caspase9.2A-Q.8stm.zeta (pBP0668). Transduced T cells were then plated with 5-fold dilutions of rapamycin. After 24 hours, cells were harvested and analyzed by flow cytometry for expression of iMC (via anti-CD19-APC), Caspase-9 (via anti-CD34-PE), and T cell identity (via anti-CD3-PerCPCy5.5). Cells were initially gated for lymphocyte morphology by FSC vs SSC, followed by CD3 expression (∼ 99% of lymphocytes).
To focus on doubly transduced cells, CD3⁺ lymphocytes were gated on CD19⁺ (ΔMyr.iMC.2A-CD19) and CD34⁺ (FRB_{I}2.Caspase9.2A-Q.8stm.zeta) expression. To normalize gated populations, percentages of CD34⁺CD19⁺ cells were divided by percent CD19⁺CD34⁻ cells within each sample as an internal control. Those values were then normalized to drug-free wells for each transduction, which were set at 100%. The results show rapid and efficient elimination of doubly transduced cells in the presence of relatively low (2 nM) levels of rapamycin (Fig. 17A, C). Similar analysis was applied to the Hi-, Med-, and Lo-expressing cells within the CD34⁺CD19⁺ gate (Fig. 17B). As rapamycin concentrations increase, percentage of CD34⁺CD19⁺ cells decrease, indicating elimination of cells. Finally, T cells from a single donor were transduced with ΔMyr.iMC.2A-CD19 (pBP0606) and FRB_{L}2.Caspase9.2A-Q.8stm.zeta (pBP0668) and plated in IL-2-containing media along with varying concentrations of rapamycin for 24 or 48 hrs. After 24 or 48 hrs, cells were harvested and analyzed by flow, as above. Interestingly, although elimination of cells expressing high levels of both transgenes was nearly complete at 24 hours, by 48 hours even cells expressing low levels of both transgenes are killed by rapamycin, showing the efficiency of the process in primary T cells (Fig. 17D).

### Example 23: Examples of plasmids and sequences discussed in Examples 17-21

### pBP0044: pSH1-iCaspase9wt

| Fragment | Nucleotide | Peptide |
|---|---|---|
| **Linker** | ATG-CTCGAG | MLE |
| **FKBPv36** | GGAGTGCAGGTGGAgACtATCTCCCCAGGAGACGG GCGCACCTTCCCCAAGCGCGGCCAGACCTGCGTG | GVQVETISPGDGRTFPK RGQTCVVHYTGMLEDG |
| | GTGCACTACACCGGGATGCTTGAAGATGGAAAGAA AGTTGATTCCTCCCGGGACAGAAACAAGCCCTTTA AGTTTATGCTAGGCAAGCAGGAGGTGATCCGAGGC TGGGAAGAAGGGGTTGCCCAGATGAGTGTGGGTC AGAGAGCCAAACTGACTATATCTCCAGATTATGCCT ATGGTGCCACTGGGCACCCAGGCATCATCCCACCA CATGCCACTCTCGTCTTCGATGTGGAGCTTCTAAAA CTGGA | KKVDSSRDRNKPFKFML GKQEVIRGWEEGVAQM SVGQRAKLTISPDYAYG ATGHPGIIPPHATLVFDV ELLKL |
| **Linker** | ATCTGGCGGTGGATCCGGA | SGGGSG |
| **Δ Caspase9** | GTCGACGGATTTGGTGATGTCGGTGCTCTTGAGAG TTTGAGGGGAAATGCAGATTTGGCTTACATCCTGA GCATGGAGCCCTGTGGCCACTGCCTCATTATCAAC AATGTGAACTTCTGCCGTGAGTCCGGGCTCCGCAC CCGCACTGGCTCCAACATCGACTGTGAGAAGTTGC GGCGTCGCTTCTCCTCGCTGCATTTCATGGTGGAG GTGAAGGGCGACCTGACTGCCAAGAAAATGGTGCT GGCTTTGCTGGAGCTGGCGCgGCAGGACCACGGT GCTCTGGACTGCTGCGTGGTGGTCATTCTCTCTCA CGGCTGTCAGGCCAGCCACCTGCAGTTCCCAGGG GCTGTCTACGGCACAGATGGATGCCCTGTGTCGGT CGAGAAGATTGTGAACATCTTCAATGGGACCAGCT GCCCCAGCCTGGGAGGGAAGCCCAAGCTCTTTTC ATCCAGGCCTGTGGTGGGGAGCAGAAAGACCATG GGTTTGAGGTGGCCTCCACTTCCCCTGAAGACGAG TCCCCTGGCAGTAACCCCGAGCCAGATGCCACCC CGTTCCAGGAAGGTTTGAGGACCTTCGACCAGCTG GACGCCATATCTAGTTTGCCCACACCCAGTGACAT CTTTGTGTCCTACTCTACTTTCCCAGGTTTTGTTTC CTGGAGGGACCCCAAGAGTGGCTCCTGGTACGTT GAGACCCTGGACGACATCTTTGAGCAGTGGGCTCA CTCTGAAGACCTGCAGTCCCTCCTGCTTAGGGTCG CTAATGCTGTTTCGGTGAAAGGGATTTATAAACAGA TGCCTGGTTGCTTTAATTTCCTCCGGAAAAAACTTT TCTTTAAAACATCAGCTAGCAGAGCCGAGGGCAGG GGAAGTCTTCTAACATGCGGGGACGTGGAGGAAAA TCCCGGGCCC-tga | VDGFGDVGALESLRGN ADLAYILSMEPCGHCLII NNVNFCRESGLRTRTGS NIDCEKLRRRFSSLHFM VEVKGDLTAKKMVLALL ELARQDHGALDCCVVVI LSHGCQASHLQFPGAVY GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTS |
| **Linker** | GCTAGCAGAGCC | ASRA |
| **T2A** | GAGGGCAGGGGAAGTCTTCTAACATGCGGGGACG TGGAGGAAAATCCCGGGCCC-tga | EGRGSLLTCGDVEENP GP* |

### pBP0463--pSH1-Fpk-Fpk'.LS.Fpk".Fpk"'.LS.HA

| Fragment | Nucleotide | Peptide |
|---|---|---|
| **Linker** | ATGCTCGAG | MLE |
| **FRBI** | TGGCATGAAGGGTTGGAAGAAGCTTCAAGGCTGT ACTTCGGAGAGAGGAACGTGAAGGGCATGTTTGA GGTTCTTGAACCTCTGCACGCCATGATGGAACGG GGACCGCAGACACTGAAAGAAACCTCTTTTAATC AGGCCTACGGCAGAGACCTGATGGAGGCCCAAG AATGGTGTAGAAAGTATATGAAATCCGGTAACGT GAAAGACCTGCTCCAGGCCTGGGACCTTTATTAC CATGTGTTCAGGCGGATCAGTAAG | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| **Linker** | TCAGGCGGTGGCTCAGGTGTCGAG | SGGGSGVD |
| **Δ-Caspase9** | GTCGACGGATTTGGTGATGTCGGTGCTCTTGAGA GTTTGAGGGGAAATGCAGATTTGGCTTACATCCT GAGCATGGAGCCCTGTGGCCACTGCCTCATTATC AACAATGTGAACTTCTGCCGTGAGTCCGGGCTCC GCACCCGCACTGGCTCCAACATCGACTGTGAGAA GTTGCGGCGTCGCTTCTCCTCGCTGCATTTCATG GTGGAGGTGAAGGGCGACCTGACTGCCAAGAAA ATGGTGCTGGCTTTGCTGGAGCTGGCGCgGCAG GACCACGGTGCTCTGGACTGCTGCGTGGTGGTC ATTCTCTCTCACGGCTGTCAGGCCAGCCACCTGC AGTTCCCAGGGGCTGTCTACGGCACAGATGGAT GCCCTGTGTCGGTCGAGAAGATTGTGAACATCTT CAATGGGACCAGCTGCCCCAGCCTGGGAGGGAA GCCCAAGCTCTTTTTCATCCAGGCCTGTGGTGGG GAGCAGAAAGACCATGGGTTTGAGGTGGCCTCC ACTTCCCCTGAAGACGAGTCCCCTGGCAGTAACC CCGAGCCAGATGCCACCCCGTTCCAGGAAGGTT TGAGGACCTTCGACCAGCTGGACGCCATATCTAG | DGFGDVGALESLRGNA DLAYILSMEPCGHCLIIN NVNFCRESGLRTRTGSN IDCEKLRRRFSSLHFMV EVKGDLTAKKMVLALLE LARQDHGALDCCVVVIL SHGCQASHLQFPGAVY GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| | TTTGCCCACACCCAGTGACATCTTTGTGTCCTACT CTACTTTCCCAGGTTTTGTTTCCTGGAGGGACCC CAAGAGTGGCTCCTGGTACGTTGAGACCCTGGA CGACATCTTTGAGCAGTGGGCTCACTCTGAAGAC CTGCAGTCCCTCCTGCTTAGGGTCGCTAATGCTG TTTCGGTGAAAGGGATTTATAAACAGATGCCTGG TTGCTTTAATTTCCTCCGGAAAAAACTTTTCTTTAA AACATCAGCTAGCAGAGCC | |
| **T2A** | GAGGGCAGGGGAAGTCTTCTAACATGCGGGGAC GTGGAGGAAAATCCCGGGCCCtga | EGRGSLLTCGDVEENP GP |

### pBP0725--pSH1-FRBI.FRBI'.LS.FRBI".FRBI'"

| Fragment | Nucleotide | Peptide |
|---|---|---|
| **FRBI** | ATGctcgagTGGCATGAAGGCCTGGAAGAGGCATC TCGTTTGTACTTTGGGGAAAGGAACGTGAAAGGC ATGTTTGAGGTGCTGGAGCCCTTGCACGCTATGA TGGAACGGGGCCCCCAGACTCTGAAGGAAACAT CCTTTAATCAGGCCTATGGTCGAGATTTAATGGA GGCCCAAGAGTGGTGCAGGAAGTACATGAAATCA GGGAATGTCAAGGACCTCCTCCAAGCCTGGGAC CTCTATTATCATGTGTTCCGACGAATCTCAAAG | MLEWHEGLEEASRLYF GERNVKGMFEVLEPLHA MMERGPQTLKETSFNQ AYGRDLMEAQEWCRKY MKSGNVKDLLQAWDLY YHVFRRISK |
| **Linker** | gtcgag | VD |
| **FRBI'** | TGGCATGAAGGGTTGGAAGAAGCTTCAAGGCTGT ACTTCGGAGAGAGGAACGTGAAGGGCATGTTTGA GGTTCTTGAACCTCTGCACGCCATGATGGAACGG GGACCGCAGACACTGAAAGAAACCTCTTTTAATC AGGCCTACGGCAGAGACCTGATGGAGGCCCAAG AATGGTGTAGAAAGTATATGAAATCCGGTAACGT GAAAGACCTGCTCCAGGCCTGGGACCTTTATTAC CATGTGTTCAGGCGGATCAGTAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | | SGGGSGVD |
| **FRBI"** | TGGCATGAAGGCCTGGAAGAGGCATCTCGTTTGT ACTTTGGGGAAAGGAACGTGAAAGGCATGTTTGA | WHEGLEEASRLYFGER NVKGMFEVLEPLHAMM |
| | GGTGCTGGAGCCCTTGCACGCTATGATGGAACG GGGCCCCCAGACTCTGAAGGAAACATCCTTTAAT CAGgCCTATGGTCGAGATTTAATGGAGGCCCAAG AGTGGtGCAGGAAGTACATGAAATCAGGGAATGT CAAGGACCTCCTCCAAGCCTGGGACCTCTATTAT CATGTGTTCCGACGAATCTCAAAG | ERGPQTLKETSFNQAYG RDLMEAQEWCRKYMKS GNVKDLLQAWDLYYHVF RRISK |
| **Linker** | GTCGAC | VD |
| **FRBI"'** | TGGCATGAAGGGTTGGAAGAAGCTTCAAGGCTGT ACTTCGGAGAGAGGAACGTGAAGGGCATGTTTGA GGTTCTTGAACCTCTGCACGCCATGATGGAACGG GGACCGCAGACACTGAAAGAAACCTCTTTTAATC AGGCCTACGGCAGAGACCTGATGGAGGCCCAAG AATGGTGTaGAAAGTATATGAAATCCGGTAACGT GAAAGACCTGCTCCAGGCCTGGGACCTTTATTAC CATGTGTTCAGGCGGATCAGTAAGTCAGGCGGT GGCTCAGGTGTCGAC | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | GTCGAC | VE |
| **HA tag** | TATCCGTACGACGTACCAGACTACGCACTCGACT AA | YPYDVPDYALD* |

### pBP0465--pSH1-M-FRBI.FRBI'.LS.HA

| Fragment | Nucleotide | Peptide |
|---|---|---|
| **Myr** | atgggctgtgtgcaatgtaaggataaagaagcaacaaaactgacgga ggag | MGCVQCKDKEATKLTEE |
| **Linker** | CTCGAG | LG |
| **FRBI** | TGGCATGAAGGCCTGGAAGAGGCATCTCGTTTGT ACTTTGGGGAAAGGAACGTGAAAGGCATGTTTGA GGTGCTGGAGCCCTTGCACGCTATGATGGAACG GGGCCCCCAGACTCTGAAGGAAACATCCTTTAAT CAGGCCTATGGTCGAGATTTAATGGAGGCCCAAG AGTGGTGCAGGAAGTACATGAAATCAGGGAATGT CAAGGACCTCCTCCAAGCCTGGGACCTCTATTAT CATGTGTTCCGACGAATCTCAAAG | MLEWHEGLEEASRLYF GERNVKGMFEVLEPLHA MMERGPQTLKETSFNQ AYGRDLMEAQEWCRKY MKSGNVKDLLQAWDLY YHVFRRISK |
| **Linker** | gtcgag | VD |
| **FRBI'** | TGGCATGAAGGGTTGGAAGAAGCTTCAAGGCTGT ACTTCGGAGAGAGGAACGTGAAGGGCATGTTTGA GGTTCTTGAACCTCTGCACGCCATGATGGAACGG GGACCGCAGACACTGAAAGAAACCTCTTTTAATC AGGCCTACGGCAGAGACCTGATGGAGGCCCAAG AATGGTGTAGAAAGTATATGAAATCCGGTAACGT GAAAGACCTGCTCCAGGCCTGGGACCTTTATTAC CATGTGTTCAGGCGGATCAGTAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | TCAGGCGGTGGCTCAGGTG | SGGGSGVD |
| **HA tag** | tatccgtacgacgtaccagactacgcactcgactaa | YPYDVPDYALD* |

### pBP0722--pSH1-Fpk-Fpk'.LS.Fpk".Fpk"'.LS.HA

| Fragment | Nucleotide | Peptide |
|---|---|---|
| **Linker** | ATGCTCGAG | MLE |
| **FKBPpk** | GGcGTcCAaGTcGAaACcATtagtCCcGGcGAtGGcaG aACaTTtCCtAAaaGgGGaCAaACaTGtGTcGTcCAtTA tACaGGcATGtTgGAgGAcGGcAAaAAgttcGAcagtagta GaGAtcGcAAtAAaCCtTTcAAaTTcATGtTgGGaAAaC AaGAaGTcATtaGgGGaTGGGAgGAgGGcGTgGCtC AaATGtccGTcGGcCAacGcGCtAAgCTcACcATcagcC CcGAcTAcGCaTAcGGcGCtACcGGaCAtCCccctaagA TtCCcCCtCAcGCtACctTgGTgTTtGAcGTcGAaCTgtT gAAgCTcGAa | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| **Linker** | gtcgag | VD |
| **FKBPpk'** | ggagtgcaggtggagactatctccccaggagacgggcgcaccttcccc aagcgcggccagacctgcgtggtgcactacaccgggatgcttgaagat ggaaagaaattcgattcctctcgggacagaaacaagccctttaagtttat gctaggcaagcaggaggtgatccgaggctgggaagaaggggttgccc agatgagtgtgggtcagagagccaaactgactatatctccagattatgcc tatggtgccactgggcacccacctaagatcccaccacatgccactctcgt cttcgatgtggagcttctaaaactggaa | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| **Linker** | | SGGGSGVD |
| **FKBPpk"** | GGcGTcCAaGTcGAaACcATtagtCCcGGcGAtGGcaG aACaTTtCCtAAaaGgGGaCAaACaTGtGTcGTcCAtTA | GVQVETISPGDGRTFPK RGQTCVVHYTGMLEDG |
| | tACaGGcATGtTgGAgGAcGGcAAaAAgttcGAcagtagta GaGAtcGcAAtAAaCCtTTcAAaTTcATGtTgGGaAAaC AaGAaGTcATtaGgGGaTGGGAgGAgGGcGTgGCtC AaATGtccGTcGGcCAacGcGCtAAgCTcACcATcagcC CcGAcTAcGCaTAcGGcGCtACcGGaCAtCCccctaagA TtCCcCCtCAcGCtACctTgGTgTTtGAcGTcGAaCTgtT gAAgCTcGAa | KKFDSSRDRNKPFKFML GKQEVIRGWEEGVAQM SVGQRAKLTISPDYAYG ATGHPPKIPPHATLVFDV ELLKLE |
| **Linker** | GTCGAC | VD |
| **FKBPpk'"** | ggagtgcaggtggagactatctccccaggagacgggcgcaccttcccc aagcgcggccagacctgcgtggtgcactacaccgggatgcttgaagat ggaaagaaattcgattcctctcgggacagaaacaagccctttaagtttat gctaggcaagcaggaggtgatccgaggctgggaagaaggggttgccc agatgagtgtgggtcagagagccaaactgactatatctccagattatgcc tatggtgccactgggcacccacctaagatcccaccacatgccactctcgt cttcgatgtggagcttctaaaactggaa | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| **Linker** | | SGGGSGVD |
| **HA tag** | TATCCGTACGACGTACCAGACTACGCACTCGACT AA | YPYDVPDYALD* |

### pBP0220--pSFG-iC9.T2A-ΔCD19

| Fragment | Nucleotide | Peptide |
|---|---|---|
| **FKBP12v36** | ATGCTCGAGGGAGTGCAGGTGGAGACTATCTCCCC AGGAGACGGGCGCACCTTCCCCAAGCGCGGCCAG ACCTGCGTGGTGCACTACACCGGGATGCTTGAAGA TGGAAAGAAAGTTGATTCCTCCCGGGACAGAAACA AGCCCTTTAAGTTTATGCTAGGCAAGCAGGAGGTG ATCCGAGGCTGGGAAGAAGGGGTTGCCCAGATGA GTGTGGGTCAGAGAGCCAAACTGACTATATCTCCA GATTATGCCTATGGTGCCACTGGGCACCCAGGCAT CATCCCACCACATGCCACTCTCGTCTTCGATGTGG AGCTTCTAAAACTGGAA | MLEGVQVETISPGDGRT FPKRGQTCVVHYTGML EDGKKVDSSRDRNKPF KFMLGKQEVIRGWEEG VAQMSVGQRAKLTISPD YAYGATGHPGIIPPHATL VFDVELLKLE |
| **Linker** | TCTGGCGGTGGATCCGGA | SGGGSG |
| **ΔCaspase9** | GTCGACGGATTTGGTGATGTCGGTGCTCTTGAGAG TTTGAGGGGAAATGCAGATTTGGCTTACATCCTGA GCATGGAGCCCTGTGGCCACTGCCTCATTATCAAC AATGTGAACTTCTGCCGTGAGTCCGGGCTCCGCAC CCGCACTGGCTCCAACATCGACTGTGAGAAGTTGC GGCGTCGCTTCTCCTCGCTGCATTTCATGGTGGAG GTGAAGGGCGACCTGACTGCCAAGAAAATGGTGCT GGCTTTGCTGGAGCTGGCGCGGCAGGACCACGGT GCTCTGGACTGCTGCGTGGTGGTCATTCTCTCTCA CGGCTGTCAGGCCAGCCACCTGCAGTTCCCAGGG GCTGTCTACGGCACAGATGGATGCCCTGTGTCGGT CGAGAAGATTGTGAACATCTTCAATGGGACCAGCT GCCCCAGCCTGGGAGGGAAGCCCAAGCTCTTTTTC ATCCAGGCCTGTGGTGGGGAGCAGAAAGACCATG GGTTTGAGGTGGCCTCCACTTCCCCTGAAGACGAG TCCCCTGGCAGTAACCCCGAGCCAGATGCCACCC CGTTCCAGGAAGGTTTGAGGACCTTCGACCAGCTG GACGCCATATCTAGTTTGCCCACACCCAGTGACAT CTTTGTGTCCTACTCTACTTTCCCAGGTTTTGTTTC CTGGAGGGACCCCAAGAGTGGCTCCTGGTACGTT GAGACCCTGGACGACATCTTTGAGCAGTGGGCTCA | VDGFGDVGALESLRGN ADLAYILSMEPCGHCLII NNVNFCRESGLRTRTGS NIDCEKLRRRFSSLHFM VEVKGDLTAKKMVLALL ELARQDHGALDCCVVVI LSHGCQASHLQFPGAVY GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| | CTCTGAAGACCTGCAGTCCCTCCTGCTTAGGGTCG CTAATGCTGTTTCGGTGAAAGGATTTATAAACAGA TGCCTGGTTGCTTTAATTTCCTCCGGAAAAAACTTT TCTTTAAAACATCAGCTAGCAGAGCC | |
| **T2A** | GAGGGCAGGGGAAGTCTTCTAACATGCGGGGACG TGGAGGAAAATCCCGGGCCC | EGRGSLLTCGDVEENP GP |
| **ΔCD19** | ATGCCACCTCCTCGCCTCCTCTTCTTCCTCCTCTTC CTCACCCCCATGGAAGTCAGGCCCGAGGAACCTCT AGTGGTGAAGGTGGAAGAGGGAGATAACGCTGTG CTGCAGTGCCTCAAGGGGACCTCAGATGGCCCCA CTCAGCAGCTGACCTGGTCTCGGGAGTCCCCGCTT AAACCCTTCTTAAAACTCAGCCTGGGGCTGCCAGG CCTGGGAATCCACATGAGGCCCCTGGCCATCTGG CTTTTCATCTTCAACGTCTCTCAACAGATGGGGG CTTCTACCTGTGCCAGCCGGGGCCCCCCTCTGAGA AGGCCTGGCAGCCTGGCTGGACAGTCAATGTGGA GGGCAGCGGGGAGCTGTTCCGGTGGAATGTTTCG GACCTAGGTGGCCTGGGCTGTGGCCTGAAGAACA GGTCCTCAGAGGGCCCCAGCTCCCCTTCCGGGAA GCTCATGAGCCCCAAGCTGTATGTGTGGGCCAAAG ACCGCCCTGAGATCTGGGAGGGAGAGCCTCCGTG TCTCCCACCGAGGGACAGCCTGAACCAGAGCCTCA GCCAGGACCTCACCATGGCCCCTGGCTCCACACTC TGGCTGTCCTGTGGGGTACCCCCTGACTCTGTGTC CAGGGGCCCCCTCTCCTGGACCCATGTGCACCCC AAGGGGCCTAAGTCATTGCTGAGCCTAGAGCTGAA GGACGATCGCCCGGCCAGAGATATGTGGGTAATG GAGACGGGTCTGTTGTTGCCCCGGGCCACAGCTC AAGACGCTGGAAAGTATTATTGTCACCGTGGCAAC CTGACCATGTCATTCCACCTGGAGATCACTGCTCG GCCAGTACTATGGCACTGGCTGCTGAGGACTGGTG GCTGGAAGGTCTCAGCTGTGACTTTGGCTTATCTG ATCTTCTGCCTGTGTTCCCTTGTGGGCATTCTTCAT CTTCAAAGAGCCCTGGTCCTGAGGAGGAAAAGAAA GCGAATGACTGACCCCACCAGGAGATTCTAA | MPPPRLLFFLLFLTPMEV RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF* |

### pBP0756--pSFG-iC9.T2A-dCD19.P2A-FRB_{I}

| Fragmen t | Nucleotide | Peptide |
|---|---|---|
| **FKBP12 v36** | ATGCTCGAGGGAGTGCAGGTGGAGACTATCTCCCC AGGAGACGGGCGCACCTTCCCCAAGCGCGGCCAG ACCTGCGTGGTGCACTACACCGGGATGCTTGAAGA TGGAAAGAAAGTTGATTCCTCCCGGGACAGAAACA AGCCCTTTAAGTTTATGCTAGGCAAGCAGGAGGTG ATCCGAGGCTGGGAAGAAGGGGTTGCCCAGATGA GTGTGGGTCAGAGAGCCAAACTGACTATATCTCCA GATTATGCCTATGGTGCCACTGGGCACCCAGGCAT CATCCCACCACATGCCACTCTCGTCTTCGATGTGG AGCTTCTAAAACTGGAA | MLEGVQVETISPGDGRT FPKRGQTCVVHYTGML EDGKKVDSSRDRNKPF KFMLGKQEVIRGWEEG VAQMSVGQRAKLTISPD YAYGATGHPGIIPPHATL VFDVELLKLE |
| **Linker** | TCTGGCGGTGGATCCGGA | SGGGSG |
| **dCaspas e9** | GTCGACGGATTTGGTGATGTCGGTGCTCTTGAGAG TTTGAGGGGAAATGCAGATTTGGCTTACATCCTGA GCATGGAGCCCTGTGGCCACTGCCTCATTATCAAC AATGTGAACTTCTGCCGTGAGTCCGGGCTCCGCAC CCGCACTGGCTCCAACATCGACTGTGAGAAGTTGC GGCGTCGCTTCTCCTCGCTGCATTTCATGGTGGAG GTGAAGGGCGACCTGACTGCCAAGAAAATGGTGCT GGCTTTGCTGGAGCTGGCGCGGCAGGACCACGGT GCTCTGGACTGCTGCGTGGTGGTCATTCTCTCTCA CGGCTGTCAGGCCAGCCACCTGCAGTTCCCAGGG GCTGTCTACGGCACAGATGGATGCCCTGTGTCGGT CGAGAAGATTGTGAACATCTTCAATGGGACCAGCT GCCCCAGCCTGGGAGGGAAGCCCAAGCTCTTTTTC ATCCAGGCCTGTGGTGGGGAGCAGAAAGACCATG GGTTTGAGGTGGCCTCCACTTCCCCTGAAGACGAG TCCCCTGGCAGTAACCCCGAGCCAGATGCCACCC CGTTCCAGGAAGGTTTGAGGACCTTCGACCAGCTG GACGCCATATCTAGTTTGCCCACACCCAGTGACAT CTTTGTGTCCTACTCTACTTTCCCAGGTTTTGTTTC CTGGAGGGACCCCAAGAGTGGCTCCTGGTACGTT GAGACCCTGGACGACATCTTTGAGCAGTGGGCTCA | VDGFGDVGALESLRGN ADLAYILSMEPCGHCLII NNVNFCRESGLRTRTGS NIDCEKLRRRFSSLHFM VEVKGDLTAKKMVLALL ELARQDHGALDCCVVVI LSHGCQASHLQFPGAVY GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| | CTCTGAAGACCTGCAGTCCCTCCTGCTTAGGGTCG CTAATGCTGTTTCGGTGAAAGGGATTTATAAACAGA TGCCTGGTTGCTTTAATTTCCTCCGGAAAAAACTTT TCTTTAAAACATCAGCTAGCAGAGCC | |
| **T2A** | GAGGGCAGGGGAAGTCTTCTAACATGCGGGGACG TGGAGGAAAATCCCGGGCCC | EGRGSLLTCGDVEENP GP |
| **dCD19** | ATGCCACCTCCTCGCCTCCTCTTCTTCCTCCTCTTC CTCACCCCCATGGAAGTCAGGCCCGAGGAACCTCT AGTGGTGAAGGTGGAAGAGGGAGATAACGCTGTG CTGCAGTGCCTCAAGGGGACCTCAGATGGCCCCA CTCAGCAGCTGACCTGGTCTCGGGAGTCCCCGCTT AAACCCTTCTTAAAACTCAGCCTGGGGCTGCCAGG CCTGGGAATCCACATGAGGCCCCTGGCCATCTGG CTTTTCATCTTCAACGTCTCTCAACAGATGGGGGG CTTCTACCTGTGCCAGCCGGGGCCCCCCTCTGAGA AGGCCTGGCAGCCTGGCTGGACAGTCAATGTGGA GGGCAGCGGGGAGCTGTTCCGGTGGAATGTTTCG GACCTAGGTGGCCTGGGCTGTGGCCTGAAGAACA GGTCCTCAGAGGGCCCCAGCTCCCCTTCCGGGAA GCTCATGAGCCCCAAGCTGTATGTGTGGGCCAAAG ACCGCCCTGAGATCTGGGAGGGAGAGCCTCCGTG TCTCCCACCGAGGGACAGCCTGAACCAGAGCCTCA GCCAGGACCTCACCATGGCCCCTGGCTCCACACTC TGGCTGTCCTGTGGGGTACCCCCTGACTCTGTGTC CAGGGGCCCCCTCTCCTGGACCCATGTGCACCCC AAGGGGCCTAAGTCATTGCTGAGCCTAGAGCTGAA GGACGATCGCCCGGCCAGAGATATGTGGGTAATG GAGACGGGTCTGTTGTTGCCCCGGGCCACAGCTC AAGACGCTGGAAAGTATTATTGTCACCGTGGCAAC CTGACCATGTCATTCCACCTGGAGATCACTGCTCG GCCAGTACTATGGCACTGGCTGCTGAGGACTGGTG GCTGGAAGGTCTCAGCTGTGACTTTGGCTTATCTG ATCTTCTGCCTGTGTTCCCTTGTGGGCATTCTTCAT CTTCAAAGAGCCCTGGTCCTGAGGAGGAAAAGAAA GCGAATGACTGACCCCACCAGGAGATTC | MPPPRLLFFLLFLTPMEV RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF |
| **gsg** | GGGAGTGGG | GSG |
| **P2A** | GCTACGAATTTTAGCTTGCTGAAGCAGGCCGGTGA TGTGGAAGAGAACCCCGGGCCT | ATNFSLLKQAGDVEENP GP |
| **FRBI** | TGGCACGAAGGTTTGGAAGAGGCCTCCCGCCTGTA TTTCGGTGAGAGAAATGTCAAAGGTATGTTTGAAGT GCTTGAGCCCCTGCACGCCATGATGGAACGGGGG CCGCAGACTCTGAAAGAAACCTCATTCAACCAGGC ATACGGGCGAGACCTGATGGAAGCGCAGGAATGG TGTAGGAAGTACATGAAGTCCGGAAATGTGAAGGA CTTGCTCCAGGCTTGGGACCTGTACTATCACGTATT TCGGAGAATAAGCAAG-TAA | WHEGLEEASRLYFGER NVKGMFEVLEPLHAMM ERGPQTLKETSFNQAYG RDLMEAQEWCRKYMKS GNVKDLLQAWDLYYHVF RRISK* |

### pBP0755--pSFG-iC9.T2A-dCD19.P2A-FRB_{I}2

| Fragmen t | Nucleotide | Peptide |
|---|---|---|
| **FKBP12 v36** | ATGCTCGAGGGAGTGCAGGTGGAGACTATCTCCCC AGGAGACGGGCGCACCTTCCCCAAGCGCGGCCAG ACCTGCGTGGTGCACTACACCGGGATGCTTGAAGA TGGAAAGAAAGTTGATTCCTCCCGGGACAGAAACA AGCCCTTTAAGTTTATGCTAGGCAAGCAGGAGGTG ATCCGAGGCTGGGAAGAAGGGGTTGCCCAGATGA GTGTGGGTCAGAGAGCCAAACTGACTATATCTCCA GATTATGCCTATGGTGCCACTGGGCACCCAGGCAT CATCCCACCACATGCCACTCTCGTCTTCGATGTGG AGCTTCTAAAACTGGAA | MLEGVQVETISPGDGRT FPKRGQTCVVHYTGML EDGKKVDSSRDRNKPF KFMLGKQEVIRGWEEG VAQMSVGQRAKLTISPD YAYGATGHPGIIPPHATL VFDVELLKLE |
| **Linker** | TCTGGCGGTGGATCCGGA | SGGGSG |
| **ΔCaspas e9** | GTCGACGGATTTGGTGATGTCGGTGCTCTTGAGAG TTTGAGGGAAATGCAGATTTGGCTTACATCCTGA GCATGGAGCCCTGTGGCCACTGCCTCATTATCAAC AATGTGAACTTCTGCCGTGAGTCCGGGCTCCGCAC CCGCACTGGCTCCAACATCGACTGTGAGAAGTTGC GGCGTCGCTTCTCCTCGCTGCATTTCATGGTGGAG GTGAAGGGCGACCTGACTGCCAAGAAAATGGTGCT GGCTTTGCTGGAGCTGGCGCGGCAGGACCACGGT GCTCTGGACTGCTGCGTGGTGGTCATTCTCTCTCA CGGCTGTCAGGCCAGCCACCTGCAGTTCCCAGGG GCTGTCTACGGCACAGATGGATGCCCTGTGTCGGT | VDGFGDVGALESLRGN ADLAYILSMEPCGHCLII NNVNFCRESGLRTRTGS NIDCEKLRRRFSSLHFM VEVKGDLTAKKMVLALL ELARQDHGALDCCVVVI LSHGCQASHLQFPGAVY GTDGCPVSVEKIVNIFNG TSCPSLGGKPKLFFIQAC GGEQKDHGFEVASTSP EDESPGSNPEPDATPFQ |
| | CGAGAAGATTGTGAACATCTTCAATGGGACCAGCT GCCCCAGCCTGGGAGGGAAGCCCAAGCTCTTTTTC ATCCAGGCCTGTGGTGGGGAGCAGAAAGACCATG GGTTTGAGGTGGCCTCCACTTCCCCTGAAGACGAG TCCCCTGGCAGTAACCCCGAGCCAGATGCCACCC CGTTCCAGGAAGGTTTGAGGACCTTCGACCAGCTG GACGCCATATCTAGTTTGCCCACACCCAGTGACAT CTTTGTGTCCTACTCTACTTTCCCAGGTTTTGTTTC CTGGAGGGACCCCAAGAGTGGCTCCTGGTACGTT GAGACCCTGGACGACATCTTTGAGCAGTGGGCTCA CTCTGAAGACCTGCAGTCCCTCCTGCTTAGGGTCG CTAATGCTGTTTCGGTGAAAGGGATTTATAAACAGA TGCCTGGTTGCTTTAATTTCCTCCGGAAAAACTTT TCTTTAAAACATCAGCTAGCAGAGCC | EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| **T2A** | GAGGGCAGGGGAAGTCTTCTAACATGCGGGGACG TGGAGGAAAATCCCGGGCCC | EGRGSLLTCGDVEENP GP |
| **ΔCD19** | ATGCCACCTCCTCGCCTCCTCTTCTTCCTCCTCTTC CTCACCCCCATGGAAGTCAGGCCCGAGGAACCTCT AGTGGTGAAGGTGGAAGAGGGAGATAACGCTGTG CTGCAGTGCCTCAAGGGGACCTCAGATGGCCCCA CTCAGCAGCTGACCTGGTCTCGGGAGTCCCCGCTT AAACCCTTCTTAAAACTCAGCCTGGGGCTGCCAGG CCTGGGAATCCACATGAGGCCCCTGGCCATCTGG CTTTTCATCTTCAACGTCTCTCAACAGATGGGGGG CTTCTACCTGTGCCAGCCGGGGCCCCCCTCTGAGA AGGCCTGGCAGCCTGGCTGGACAGTCAATGTGGA GGGCAGCGGGGAGCTGTTCCGGTGGAATGTTTCG GACCTAGGTGGCCTGGGCTGTGGCCTGAAGAACA GGTCCTCAGAGGGCCCCAGCTCCCCTTCCGGGAA GCTCATGAGCCCCAAGCTGTATGTGTGGGCCAAAG ACCGCCCTGAGATCTGGGAGGGAGAGCCTCCGTG TCTCCCACCGAGGGACAGCCTGAACCAGAGCCTCA GCCAGGACCTCACCATGGCCCCTGGCTCCACACTC TGGCTGTCCTGTGGGGTACCCCCTGACTCTGTGTC CAGGGGCCCCCTCTCCTGGACCCATGTGCACCCC AAGGGGCCTAAGTCATTGCTGAGCCTAGAGCTGAA | MPPPRLLFFLLFLTPMEV RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF |
| | GGACGATCGCCCGGCCAGAGATATGTGGGTAATG GAGACGGGTCTGTTGTTGCCCCGGGCCACAGCTC AAGACGCTGGAAAGTATTATTGTCACCGTGGCAAC CTGACCATGTCATTCCACCTGGAGATCACTGCTCG GCCAGTACTATGGCACTGGCTGCTGAGGACTGGTG GCTGGAAGGTCTCAGCTGTGACTTTGGCTTATCTG ATCTTCTGCCTGTGTTCCCTTGTGGGCATTCTTCAT CTTCAAAGAGCCCTGGTCCTGAGGAGGAAAAGAAA GCGAATGACTGACCCCACCAGGAGATTC | |
| **GSG-linker** | GGGAGTGGG | GSG |
| **P2A** | GCTACGAATTTTAGCTTGCTGAAGCAGGCCGGTGA TGTGGAAGAGAACCCCGGGCCT | ATNFSLLKQAGDVEENP GP |
| **FRBI** | TGGCATGAAGGTCTGGAAGAAGCTTCTCGCCTTTA TTTTGGCGAACGGAACGTAAAAGGTATGTTTGAAGT CCTGGAGCCATTGCACGCCATGATGGAGCGCGGG CCTCAGACCCTCAAGGAAACCAGTTTTAATCAGGC CTATGGGCGAGACCTCATGGAGGCACAGGAATGG TGTCGGAAGTATATGAAGTCCGGCAACGTTAAGGA TCTCTTGCAGGCCTGGGACTTGTATTATCACGTGTT CCGGCGAATCAGCAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | Cgtacg | RT |
| **FRBI"** | TGGCACGAAGGTTTGGAAGAGGCCTCCCGCCTGTA TTTCGGTGAGAGAAATGTCAAAGGTATGTTTGAAGT GCTTGAGCCCCTGCACGCCATGATGGAACGGGGG CCGCAGACTCTGAAAGAAACCTCATTCAACCAGGC ATACGGGCGAGACCTGATGGAAGCGCAGGAATGG TGTAGGAAGTACATGAAGTCCGGAAATGTGAAGGA CTTGCTCCAGGCTTGGGACCTGTACTATCACGTATT TCGGAGAATAAGCAAG-TAA | WHEGLEEASRLYFGER NVKGMFEVLEPLHAMM ERGPQTLKETSFNQAYG RDLMEAQEWCRKYMKS GNVKDLLQAWDLYYHVF RRISK* |

### pBP0757--pSFG-iC9T2A-dCD19.P2A-FRB_{I}3

| Fragment | Nucleotide | Peptide |
|---|---|---|
| **FKBP12v3 6** | ATGCTCGAGGGAGTGCAGGTGGAGACTATCTCC CCAGGAGACGGGCGCACCTTCCCCAAGCGCGGC CAGACCTGCGTGGTGCACTACACCGGGATGCTT GAAGATGGAAAGAAAGTTGATTCCTCCCGGGACA GAAACAAGCCCTTTAAGTTTATGCTAGGCAAGCA GGAGGTGATCCGAGGCTGGGAAGAAGGGGTTGC CCAGATGAGTGTGGGTCAGAGAGCCAAACTGACT ATATCTCCAGATTATGCCTATGGTGCCACTGGGC ACCCAGGCATCATCCCACCACATGCCACTCTCGT CTTCGATGTGGAGCTTCTAAAACTGGAA | MLEGVQVETISPGDGRT FPKRGQTCVVHYTGML EDGKKVDSSRDRNKPF KFMLGKQEVIRGWEEG VAQMSVGQRAKLTISPD YAYGATGHPGIIPPHATL VFDVELLKLE |
| **Linker** | TCTGGCGGTGGATCCGGA | SGGGSG |
| **ΔCaspase 9** | GTCGACGGATTTGGTGATGTCGGTGCTCTTGAGA GTTTGAGGGGAAATGCAGATTTGGCTTACATCCT GAGCATGGAGCCCTGTGGCCACTGCCTCATTATC AACAATGTGAACTTCTGCCGTGAGTCCGGGCTCC GCACCCGCACTGGCTCCAACATCGACTGTGAGAA GTTGCGGCGTCGCTTCTCCTCGCTGCATTTCATG GTGGAGGTGAAGGGCGACCTGACTGCCAAGAAA ATGGTGCTGGCTTTGCTGGAGCTGGCGCGGCAG GACCACGGTGCTCTGGACTGCTGCGTGGTGGTC ATTCTCTCTCACGGCTGTCAGGCCAGCCACCTGC AGTTCCCAGGGGCTGTCTACGGCACAGATGGAT GCCCTGTGTCGGTCGAGAAGATTGTGAACATCTT CAATGGGACCAGCTGCCCCAGCCTGGGAGGGAA GCCCAAGCTCTTTTTCATCCAGGCCTGTGGTGGG GAGCAGAAAGACCATGGGTTTGAGGTGGCCTCC ACTTCCCCTGAAGACGAGTCCCCTGGCAGTAACC CCGAGCCAGATGCCACCCCGTTCCAGGAAGGTT TGAGGACCTTCGACCAGCTGGACGCCATATCTAG TTTGCCCACACCCAGTGACATCTTTGTGTCCTACT CTACTTTCCCAGGTTTTGTTTCCTGGAGGGACCC CAAGAGTGGCTCCTGGTACGTTGAGACCCTGGA CGACATCTTTGAGCAGTGGGCTCACTCTGAAGAC CTGCAGTCCCTCCTGCTTAGGGTCGCTAATGCTG TTTCGGTGAAAGGGATTTATAAACAGATGCCTGG TTGCTTTAATTTCCTCCGGAAAAACTTTTCTTTAA | VDGFGDVGALESLRGN ADLAYILSMEPCGHCLII NNVNFCRESGLRTRTGS NIDCEKLRRRFSSLHFM VEVKGDLTAKKMVLALL ELARQDHGALDCCVVVI LSHGCQASHLQFPGAVY GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| | AACATCAGCTAGCAGAGCC | |
| **T2A** | GAGGGCAGGGGAAGTCTTCTAACATGCGGGGAC GTGGAGGAAAATCCCGGGCCC | EGRGSLLTCGDVEENP GP |
| **ΔCD19** | ATGCCACCTCCTCGCCTCCTCTTCTTCCTCCTCTT CCTCACCCCCATGGAAGTCAGGCCCGAGGAACC TCTAGTGGTGAAGGTGGAAGAGGGAGATAACGC TGTGCTGCAGTGCCTCAAGGGGACCTCAGATGG CCCCACTCAGCAGCTGACCTGGTCTCGGGAGTC CCCGCTTAAACCCTTCTTAAAACTCAGCCTGGGG CTGCCAGGCCTGGGAATCCACATGAGGCCCCTG GCCATCTGGCTTTTCATCTTCAACGTCTCTCAACA GATGGGGGGCTTCTACCTGTGCCAGCCGGGGCC CCCCTCTGAGAAGGCCTGGCAGCCTGGCTGGAC AGTCAATGTGGAGGGCAGCGGGGAGCTGTTCCG GTGGAATGTTTCGGACCTAGGTGGCCTGGGCTG TGGCCTGAAGAACAGGTCCTCAGAGGGCCCCAG CTCCCCTTCCGGGAAGCTCATGAGCCCCAAGCT GTATGTGTGGGCCAAAGACCGCCCTGAGATCTG GGAGGGAGAGCCTCCGTGTCTCCCACCGAGGGA CAGCCTGAACCAGAGCCTCAGCCAGGACCTCAC CATGGCCCCTGGCTCCACACTCTGGCTGTCCTGT GGGGTACCCCCTGACTCTGTGTCCAGGGGCCCC CTCTCCTGGACCCATGTGCACCCCAAGGGGCCT AAGTCATTGCTGAGCCTAGAGCTGAAGGACGATC GCCCGGCCAGAGATATGTGGGTAATGGAGACGG GTCTGTTGTTGCCCCGGGCCACAGCTCAAGACG CTGGAAAGTATTATTGTCACCGTGGCAACCTGAC CATGTCATTCCACCTGGAGATCACTGCTCGGCCA GTACTATGGCACTGGCTGCTGAGGACTGGTGGC TGGAAGGTCTCAGCTGTGACTTTGGCTTATCTGA TCTTCTGCCTGTGTTCCCTTGTGGGCATTCTTCAT CTTCAAAGAGCCCTGGTCCTGAGGAGGAAAAGAA AGCGAATGACTGACCCCACCAGGAGATTC | MPPPRLLFFLLFLTPMEV RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF |
| **GSG (linker)** | GGGAGTGGG | GSG |
| **P2A** | GCTACGAATTTTAGCTTGCTGAAGCAGGCCGGTG ATGTGGAAGAGAACCCCGGGCCT | ATNFSLLKQAGDVEENP GP |
| **FRBI** | TGGCATGAAGGTCTGGAAGAAGCTTCTCGCCTTT ATTTTGGCGAACGGAACGTAAAAGGTATGTTTGA AGTCCTGGAGCCATTGCACGCCATGATGGAGCG CGGGCCTCAGACCCTCAAGGAAACCAGTTTTAAT CAGGCCTATGGGCGAGACCTCATGGAGGCACAG GAATGGTGTCGGAAGTATATGAAGTCCGGCAACG TTAAGGATCTCTTGCAGGCCTGGGACTTGTATTAT CACGTGTTCCGGCGAATCAGCAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | Cgtacg | RT |
| **FRBI'** | TGGCAcGAAGGTCTgGAcGAGGCTAGTAGACTGT ATTTCGGCGAGAGAAATGTAAAGGGAATGTTCGA GGTACTGGAGCCTCTGCACGCCATGATGGAACG CGGCCCTCAGACACTCAAGGAGACTAGTTTTAAC CAGGCCTATGGCAGGGATCTGATGGAGGCTCAG GAATGGTGCCGGAAGTAtATGAAAAGCGGTAACG TGAAGGACCTGCTGCAGGCCTGGGATCTGTATTA TCACGTGTTTAGAAGAATCTCTAAA | WHEGLDEASRLYFGER NVKGMFEVLEPLHAMM ERGPQTLKETSFNQAYG RDLMEAQEWCRKYMKS GNVKDLLQAWDLYYHVF RRISK |
| **Linker** | Cgtacg | RT |
| **FRBI"** | TGGCACGAAGGTTTGGAAGAGGCCTCCCGCCTG TATTTCGGTGAGAGAAATGTCAAAGGTATGTTTGA AGTGCTTGAGCCCCTGCACGCCATGATGGAACG GGGGCCGCAGACTCTGAAAGAAACCTCATTCAAC CAGGCATACGGGCGAGACCTGATGGAAGCGCAG GAATGGTGTAGGAAGTACATGAAGTCCGGAAATG TGAAGGACTTGCTCCAGGCTTGGGACCTGTACTA TCACGTATTTCGGAGAATAAGCAAG-TAA | WHEGLEEASRLYFGER NVKGMFEVLEPLHAMM ERGPQTLKETSFNQAYG RDLMEAQEWCRKYMKS GNVKDLLQAWDLYYHVF RRISK* |

### pBP0655--pSFG-ΔMyr.FRB_{I}.MC.2A-ΔCD19

| Fragmen t | Nucleotide | Peptide |
|---|---|---|
| **FRB_{I}'** | TGGCACGAGGGGCTGGAGGAGGCAAGTCGACTGT ATTTTGGAGAACGCAACGTAAAGGGAATGTTTGAG GTGCTCGAACCACTCCATGCTATGATGGAAAGGGG | WHEGLEEASRLYFGER NVKGMFEVLEPLHAMM ERGPQTLKETSFNQAYG |
| | GCCTCAGACTCTTAAGGAAACAAGTTTTAATCAAGC CTACGGACGAGACCTCATGGAGGCGCAGGAGTGG TGCAGAAAATACATGAAATCAGGTAATGTTAAGGAC CTGCTGCAGGCATGGGACCTGTACTACCATGTCTT CAGGCGCATCTCAAAG | RDLMEAQEWCRKYMKS GNVKDLLQAWDLYYHVF RRISK |
| **Linker** | ATGCATTCTGGTGGAGGATCAGGCGTTGAA | MHSGGGSGVE |
| **MyD88L** | GCAGCTGGAGGCCCTGGCGCAGGCTCTGCAGCCC CTGTATCTAGCACCTCTTCTCTTCCTCTGGCTGCGC TGAACATGAGAGTGCGGAGACGGTTGTCTTTGTTC TTGAATGTCAGAACACAGGTTGCAGCGGACTGGAC CGCTCTGGCCGAGGAAATGGACTTCGAGTACCTGG AGATCAGGCAACTCGAAACGCAGGCAGATCCTACA GGCAGACTGTTGGATGCGTGGCAGGGACGGCCCG GAGCCAGCGTTGGACGGCTCCTTGATCTTCTCACC AAGCTGGGCAGAGATGACGTGCTGCTGGAATTGG GCCCCAGTATTGAGGAGGACTGCCAAAAATACATC TTGAAGCAGCAACAGGAGGAGGCGGAGAAGCCC TCCAGGTCGCAGCCGTCGATTCATCCGTGCCTAGA ACAGCCGAACTTGCAGGCATCACTACCCTGGATGA TCCCCTGGGCCATATGCCAGAGAGGTTTGATGCGT TTATCTGCTATTGCCCAAGCGATATC | AAGGPGAGSAAPVSST SSLPLAALNMRVRRRLS LFLNVRTQVAADWTALA EEMDFEYLEIRQLETQA DPTGRLLDAWQGRPGA SVGRLLDLLTKLGRDDV LLELGPSIEEDCQKYILK QQQEEAEKPLQVAAVD SSVPRTAELAGITTLDDP LGHMPERFDAFICYCPS DI |
| **Linker** | GTTGAG | VE |
| **hCD40** | AAGAAGGTGGCCAAGAAGCCAACCAATAAAGCTCC ACATCCTAAACAGGAGCCACAAGAAATCAACTTTCC AGATGATCTCCCTGGCTCTAATACTGCAGCCCCCG TGCAGGAAACCCTGCACGGCTGTCAACCTGTGACA CAGGAAGACGGGAAGGAAAGCAGGATATCCGTGC AGGAACGGCAA | KKVAKKPTNKAPHPKQE PQEINFPDDLPGSNTAA PVQETLHGCQPVTQED GKESRISVQERQ |
| **Linker** | GTCGAC | VD |
| **HA epitope** | TACCCATACGACGTGCCAGATTATGCT | YPYDVPDYA |
| **Linker** | CCGCGG; | PR |
| **T2A** | GAAGGCCGAGGGAGCCTGCTGACATGTGGCGATG TGGAGGAAAACCCAGGACCA | EGRGSLLTCGDVEENP GP |
| **ΔCD19** | ATGCCACCACCTCGCCTGCTGTTCTTTCTGCTGTTC CTGACACCTATGGAGGTGCGACCTGAGGAACCACT | MPPPRLLFFLLFLTPMEV RPEEPLVVKVEEGDNAV |
| | GGTCGTGAAGGTCGAGGAAGGCGACAATGCCGTG CTGCAGTGCCTGAAAGGCACTTCTGATGGCCAAC TCAGCAGCTGACCTGGTCCAGGGAGTCTCCCCTGA AGCCTTTTCTGAAACTGAGCCTGGGACTGCCAGGA CTGGGAATCCACATGCGCCCTCTGGCTATCTGGCT GTTCATCTTCAACGTGAGCCAGCAGATGGGAGGAT TCTACCTGTGCCAGCCAGGACCACCATCCGAGAAG GCCTGGCAGCCTGGATGGACCGTCAACGTGGAGG GGTCTGGAGAACTGTTTAGGTGGAATGTGAGTGAC CTGGGAGGACTGGGATGTGGCTGAAGAACCGCT CCTCTGAAGGCCCAAGTTCACCCTCAGGGAAGCTG ATGAGCCCAAAACTGTACGTGTGGGCCAAAGATCG GCCCGAGATCTGGGAGGGAGAACCTCCATGCCTG CCACCTAGAGACAGCCTGAATCAGAGTCTGTCACA GGATCTGACAATGGCCCCCGGGTCCACTCTGTGG CTGTCTTGTGGAGTCCCACCCGACAGCGTGTCCAG AGGCCCTCTGTCCTGGACCCACGTGCATCCTAAGG GGCCAAAAAGTCTGCTGTCACTGGAACTGAAGGAC GATCGGCCTGCCAGAGACATGTGGGTCATGGAGA CTGGACTGCTGCTGCCACGAGCAACCGCACAGGA TGCTGGAAAATACTATTGCCACCGGGGCAATCTGA CAATGTCCTTCCATCTGGAGATCACTGCAAGGCCC GTGCTGTGGCACTGGCTGCTGCGAACCGGAGGAT GGAAGGTCAGTGCTGTGACACTGGCATATCTGATC TTTTGCCTGTGCTCCCTGGTGGGCATTCTGCATCT GCAGAGAGCCCTGGTGCTGCGGAGAAAGAGAAAG AGAATGACTGACCCAACAAGAAGGTTTTGA | LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF* |

### pBP0498--pSFG-ΔMyr.iMC.FRB_{I}2.P2A-ΔCD19

| Fragmen t | Nucleotide | Peptide |
|---|---|---|
| **Start** | ATGCTCGAG | MLE |
| **FRB_{I}^** | TGGCACGAGGGGCTGGAGGAGGCAAGTCGACTGT ATTTTGGAGAACGCAACGTAAAGGGAATGTTTGAG GTGCTCGAACCACTCCATGCTATGATGGAAAGGGG | WHEGLEEASRLYFGER NVKGMFEVLEPLHAMM ERGPQTLKETSFNQAYG |
| | GCCTCAGACTCTTAAGGAAACAAGTTTTAATCAAGC CTACGGACGAGACCTCATGGAGGCGCAGGAGTGG TGCAGAAAATACATGAAATCAGGTAATGTTAAGGAC CTGCTGCAGGCATGGGACCTGTACTACCATGTCTT CAGGCGCATCTCAAAG | RDLMEAQEWCRKYMKS GNVKDLLQAWDLYYHVF RRISK |
| **Linker** | ATGCAT | MH |
| **FRB_{I}^^** | TGGCACGAAGGCCTGGAAGAGGCCTCAAGACTTTA CTTTGGTGAACGCAACGTTAAAGGCATGTTCGAGG TGCTGGAACCCTTGCATGCAATGATGGAGCGAGGT CCTCAGACACTCAAAGAGACATCTTTTAACCAGGC GTATGGACGGACCTCATGGAGGCTCAGGAATGG TGCCGCAAGTACATGAAAAGTGGGAATGTGAAGGA TCTGCTGCAAGCATGGGATCTGTATTACCACGTGTT TAGACGGATCAGCAAA | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | ATGCATTCTGGTGGAGGATCAGGCGTTGAA | MHSGGGSGVE |
| **MyD88L** | GCAGCTGGAGGCCCTGGCGCAGGCTCTGCAGCCC CTGTATCTAGCACCTCTTCTCTTCCTCTGGCTGCGC TGAACATGAGAGTGCGGAGACGGTTGTCTTTGTTC TTGAATGTCAGAACACAGGTTGCAGCGGACTGGAC CGCTCTGGCCGAGGAAATGGACTTCGAGTACCTGG AGATCAGGCAACTCGAAACGCAGGCAGATCCTACA GGCAGACTGTTGGATGCGTGGCAGGGACGGCCCG GAGCCAGCGTTGGACGGCTCCTTGATCTTCTCACC AAGCTGGGCAGAGATGACGTGCTGCTGGAATTGG GCCCCAGTATTGAGGAGGACTGCCAAAAATACATC TTGAAGCAGCAACAGGAGGAGGCGGAGAAGCCCC TCCAGGTCGCAGCCGTCGATTCATCCGTGCCTAGA ACAGCCGAACTTGCAGGCATCACTACCCTGGATGA TCCCCTGGGCCATATGCCAGAGAGGTTTGATGCGT TTATCTGCTATTGCCCAAGCGATATC | AAGGPGAGSAAPVSST SSLPLAALNMRVRRRLS LFLNVRTQVAADWTALA EEMDFEYLEIRQLETQA DPTGRLLDAWQGRPGA SVGRLLDLLTKLGRDDV LLELGPSIEEDCQKYILK QQQEEAEKPLQVAAVD SSVPRTAELAGITTLDDP LGHMPERFDAFICYCPS DI |
| **Linker** | GTTGAG | VE |
| **hCD40** | AAGAAGGTGGCCAAGAAGCCAACCAATAAAGCTCC ACATCCTAAACAGGAGCCACAAGAAATCAACTTTCC AGATGATCTCCCTGGCTCTAATACTGCAGCCCCCG TGCAGGAAACCCTGCACGGCTGTCAACCTGTGACA CAGGAAGACGGGAAGGAAAGCAGGATATCCGTGC | KKVAKKPTNKAPHPKQE PQEINFPDDLPGSNTAA PVQETLHGCQPVTQED GKESRISVQERQ |
| | AGGAACGGCAA | |
| **Linker** | GTCGAC | VD |
| **HA** | TACCCATACGACGTGCCAGATTATGCT | YPYDVPDYA |
| **Linker** | CCGCGG | PR |
| **T2A** | GAAGGCCGAGGGAGCCTGCTGACATGTGGCGATG TGGAGGAAAACCCAGGACCA | EGRGSLLTCGDVEENP GP |
| **ΔCD19** | ATGCCACCACCTCGCCTGCTGTTCTTTCTGCTGTTC CTGACACCTATGGAGGTGCGACCTGAGGAACCACT GGTCGTGAAGGTCGAGGAAGGCGACAATGCCGTG CTGCAGTGCCTGAAAGGCACTTCTGATGGCCAAC TCAGCAGCTGACCTGGTCCAGGGAGTCTCCCCTGA AGCCTTTTCTGAAACTGAGCCTGGGACTGCCAGGA CTGGGAATCCACATGCGCCCTCTGGCTATCTGGCT GTTCATCTTCAACGTGAGCCAGCAGATGGGAGGAT TCTACCTGTGCCAGCCAGGACCACCATCCGAGAAG GCCTGGCAGCCTGGATGGACCGTCAACGTGGAGG GGTCTGGAGAACTGTTTAGGTGGAATGTGAGTGAC CTGGGAGGACTGGGATGTGGGCTGAAGAACCGCT CCTCTGAAGGCCCAAGTTCACCCTCAGGGAAGCTG ATGAGCCCAAAACTGTACGTGTGGGCCAAAGATCG GCCCGAGATCTGGGAGGGAGAACCTCCATGCCTG CCACCTAGAGACAGCCTGAATCAGAGTCTGTCACA GGATCTGACAATGGCCCCCGGGTCCACTCTGTGG CTGTCTTGTGGAGTCCCACCCGACAGCGTGTCCAG AGGCCCTCTGTCCTGGACCCACGTGCATCCTAAGG GGCCAAAAAGTCTGCTGTCACTGGAACTGAAGGAC GATCGGCCTGCCAGAGACATGTGGGTCATGGAGA CTGGACTGCTGCTGCCACGAGCAACCGCACAGGA TGCTGGAAAATACTATTGCCACCGGGGCAATCTGA CAATGTCCTTCCATCTGGAGATCACTGCAAGGCCC GTGCTGTGGCACTGGCTGCTGCGAACCGGAGGAT GGAAGGTCAGTGCTGTGACACTGGCATATCTGATC TTTTGCCTGTGCTCCCTGGTGGGCATTCTGCATCT GCAGAGAGCCCTGGTGCTGCGGAGAAAGAGAAAG AGAATGACTGACCCAACAAGAAGGTTTTGA | MPPPRLLFFLLFLTPMEV RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF* |

### pBP0488--pSFG-aHER2.Q.8stm.CD3zeta.Fpk2

| **Fragmen t** | **Nucleotide** | **Peptide** |
|---|---|---|
| **Signal Peptide** | ATGGAGTTTGGACTTTCTTGGTTGTTTTTGGTGGCA ATTCTGAAGGGTGTCCAGTGTAGCAGG | MEFGLSWLFLVAILKGV QCSR |
| **FRP5-VL** | GACATCCAATTGACACAATCACACAAATTTCTCTCA ACTTCTGTAGGAGACAGAGTGAGCATAACCTGCAA AGCATCCCAGGACGTGTACAATGCTGTGGCTTGGT ACCAACAGAAGCCTGGACAATCCCCAAAATTGCTG ATTTATTCTGCCTCTAGTAGGTACACTGGGGTACCT TCTCGGTTTACGGGCTCTGGGTCCGGACCAGATTT CACGTTCACAATCAGTTCCGTTCAAGCTGAAGACCT CGCTGTTTATTTTTGCCAGCAGCACTTCCGAACCCC TTTTACTTTTGGCTCAGGCACTAAGTTGGAAATCAA GGCTTTG | DIQLTQSHKFLSTSVGD RVSITCKASQDVYNAVA WYQQKPGQSPKLLIYSA SSRYTGVPSRFTGSGS GPDFTFTISSVQAEDLAV YFCQQHFRTPFTFGSGT KLEIKAL |
| **Linker** | GGCGGAGGAAGCGGAGGTGGGGGC | GGGSGGGG |
| **FRP5-VH** | GAAGTCCAATTGCAACAGTCAGGCCCCGAATTGAA AAAGCCCGGCGAAACAGTGAAGATATCTTGTAAAG CCTCCGGTTACCCTTTTACGAACTATGGAATGAACT GGGTCAAACAAGCCCCTGGACAGGGATTGAAGTG GATGGGATGGATCAATACATCAACAGGCGAGTCTA CCTTCGCAGATGATTTCAAAGGTCGCTTTGACTTCT CACTGGAGACCAGTGCAAATACCGCCTACCTTCAG ATTAACAATCTTAAAAGCGAGGATATGGCAACCTAC TTTTGCGCAAGATGGGAAGTTTATCACGGGTACGT GCCATACTGGGGACAAGGAACGACAGTGACAGTTA GTAGC | EVQLQQSGPELKKPGET VKISCKASGYPFTNYGM NWVKQAPGQGLKWMG WINTSTGESTFADDFKG RFDFSLETSANTAYLQIN NLKSEDMATYFCARWE VYHGYVPYWGQGTTVT VSS |
| **Linker** | GGATCC | GS |
| **Q-Bend-10 (CD34 Epitope)** | GAACTTCCTACTCAGGGGACTTTCTCAAACGTTAGC ACAAACGTAAGT | ELPTQGTFSNVSTNVS |
| **CD8 Stalk** | CCCGCCCCAAGACCCCCCACACCTGCGCCGACCA TTGCTTCTCAACCCCTGAGTTTGAGACCCGAGGCC TGCCGGCCAGCTGCCGGCGGGGCCGTGCATACAA GAGGACTCGATTTCGCTTGCGAC | PAPRPPTPAPTIASQPLS LRPEACRPAAGGAVHTR GLDFACD |
| **CD8a tm** | ATCTATATCTGGGCACCTCTCGCTGGCACCTGTGG AGTCCTTCTGCTCAGCCTGGTTATTACTCTGTACTG TAATCACCGGAATCGCCGCCGCGTTTGTAAGTGTC CCAGG | IYIWAPLAGTCGVLLLSL VITLYCNHRNRRRVCKC PR |
| **Linker** | CTCGAG | LE |
| **CD3 zeta** | AGAGTGAAGTTCAGCAGGAGCGCAGACGCCCCCG CGTACCAGCAGGGCCAGAACCAGCTCTATAACGAG CTCAATCTAGGACGAAGAGAGGAGTACGATGTTTT GGACAAGAGACGTGGCCGGGACCCTGAGATGGGG GGAAAGCCGAGAAGGAAGAACCCTCAGGAAGGCC TGTACAATGAACTGCAGAAAGATAAGATGGCGGAG GCCTACAGTGAGATTGGGATGAAAGGCGAGCGCC GGAGGGGCAAGGGGCACGATGGCCTTTACCAGGG TCTCAGTACAGCCACCAAGGACACCTACGACGCCC TTCACATGCAAGCTCTTCCACCTCG | RVKFSRSADAPAYQQG QNQLYNELNLGRREEYD VLDKRRGRDPEMGGKP RRKNPQEGLYNELQKD KMAEAYSEIGMKGERRR GKGHDGLYQGLSTATK DTYDALHMQALPP |
| **Linker** | TCAGGCGGTGGCTCAGGTGTTAAC | SGGGSGVN |
| **Fpk'** | GGCGTCCAAGTCGAAACCATTAGTCCCGGCGATGG CAGAACATTTCCTAAAGGGGACAAACATGTGTCG TCCATTATACAGGCATGTTGGAGGACGGCAAAAG TTCGACAGTAGTAGAGATCGCAATAAACCTTTCAAA TTCATGTTGGGAAAACAAGAAGTCATTAGGGGATG GGAGGAGGGCGTGGCTCAAATGTCCGTCGGCCAA CGCGCTAAGCTCACCATCAGCCCCGACTACGCATA CGGCGCTACCGGACATCCCCCTAAGATTCCCCCTC ACGCTACCTTGGTGTTTGACGTCGAACTGTTGAAG CTCGAA | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| **Linker** | GTTAAC | VN |
| **Fpk** | GGAGTGCAGGTGGAGACTATCTCCCCAGGAGACG GGCGCACCTTCCCCAAGCGCGGCCAGACCTGCGT GGTGCACTACACCGGGATGCTTGAAGATGGAAAGA AATTCGATTCCTCTCGGGACAGAAACAAGCCCTTTA AGTTTATGCTAGGCAAGCAGGAGGTGATCCGAGGC TGGGAAGAAGGGGTTGCCCAGATGAGTGTGGGTC AGAGAGCCAAACTGACTATATCTCCAGATTATGCCT ATGGTGCCACTGGGCACCCACCTAAGATCCCACCA CATGCCACTCTCGTCTTCGATGTGGAGCTTCTAAAA | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| | CTGGAA | |
| **GSG Linker** | GGATCGGGA | GSG |
| **P2A** | GCTACTAACTTCAGCCTGCTGAAGCAGGCTGGAGA CGTGGAGGAGAACCCCGGGCCT | ATNFSLLKQAGDVEENP GP |

### pBP0467--pSH1-FRBI'.FRBI.LS.ΔCaspase9

| Fragmen t | Nucleotide | Peptide |
|---|---|---|
| **FRB_{I}'** | TGGCATGAAGGCCTGGAAGAGGCATCTCGTTTGTA CTTTGGGGAAAGGAACGTGAAAGGCATGTTTGAGG TGCTGGAGCCCTTGCACGCTATGATGGAACGGGG CCCCCAGACTCTGAAGGAAACATCCTTTAATCAGG CCTATGGTCGAGATTTAATGGAGGCCCAAGAGTGG TGCAGGAAGTACATGAAATCAGGGAATGTCAAGGA CCTCCTCCAAGCCTGGGACCTCTATTATCATGTGTT CCGACGAATCTCAAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | GTCGAG | VE |
| **FRB_{I}** | TGGCATGAAGGGTTGGAAGAAGCTTCAAGGCTGTA CTTCGGAGAGAGGAACGTGAAGGGCATGTTTGAG GTTCTTGAACCTCTGCACGCCATGATGGAACGGGG ACCGCAGACACTGAAAGAAACCTCTTTTAATCAGG CCTACGGCAGAGACCTGATGGAGGCCCAAGAATG GTGTAGAAAGTATATGAAATCCGGTAACGTGAAAG ACCTGCTCCAGGCCTGGGACCTTTATTACCATGTG TTCAGGCGGATCAGTAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | TCAGGCGGTGGCTCAGGT | SGGGSG |
| **ΔCaspas e9** | GTCGACGGATTTGGTGATGTCGGTGCTCTTGAGAG TTTGAGGGGAAATGCAGATTTGGCTTACATCCTGA GCATGGAGCCCTGTGGCCACTGCCTCATTATCAAC AATGTGAACTTCTGCCGTGAGTCCGGGCTCCGCAC CCGCACTGGCTCCAACATCGACTGTGAGAAGTTGC GGCGTCGCTTCTCCTCGCTGCATTTCATGGTGGAG GTGAAGGGCGACCTGACTGCCAAGAAAATGGTGCT GGCTTTGCTGGAGCTGGCGCgGCAGGACCACGGT | VDGFGDVGALESLRGN ADLAYILSMEPCGHCLII NNVNFCRESGLRTRTGS NIDCEKLRRRFSSLHFM VEVKGDLTAKKMVLALL ELARQDHGALDCCVVVI LSHGCQASHLQFPGAVY GTDGCPVSVEKIVNIFNG |
| | GCTCTGGACTGCTGCGTGGTGGTCATTCTCTCTCA CGGCTGTCAGGCCAGCCACCTGCAGTTCCCAGGG GCTGTCTACGGCACAGATGGATGCCCTGTGTCGGT CGAGAAGATTGTGAACATCTTCAATGGGACCAGCT GCCCCAGCCTGGGAGGGAAGCCCAAGCTCTTTTTC ATCCAGGCCTGTGGTGGGGAGCAGAAAGACCATG GGTTTGAGGTGGCCTCCACTTCCCCTGAAGACGAG TCCCCTGGCAGTAACCCCGAGCCAGATGCCACCC CGTTCCAGGAAGGTTTGAGGACCTTCGACCAGCTG GACGCCATATCTAGTTTGCCCACACCCAGTGACAT CTTTGTGTCCTACTCTACTTTCCCAGGTTTTGTTTC CTGGAGGGACCCCAAGAGTGGCTCCTGGTACGTT GAGACCCTGGACGACATCTTTGAGCAGTGGGCTCA CTCTGAAGACCTGCAGTCCCTCCTGCTTAGGGTCG CTAATGCTGTTTCGGTGAAAGGGATTTATAAACAGA TGCCTGGTTGCTTTAATTTCCTCCGGAAAAACTTT TCTTTAAAACATCAGCTAGCAGAGCCGAGGGCAGG GGAAGTCTTCTAACATGCGGGGACGTGGAGGAAAA TCCCGGGCCCTGA | TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRAEGR GSLLTCGDVEENPGP* |

### pBP0606--pSFG-k-ΔMyr.iMC.2A-ΔCD19

| **Fragmen t** | **Nucleotide** | **Peptide** |
|---|---|---|
| **MyD88** | ATGGCTGCAGGAGGTCCCGGCGCGGGGTCTGCGG CCCCGGTCTCCTCCACATCCTCCCTTCCCCTGGCT GCTCTCAACATGCGAGTGCGGCGCCGCCTGTCTCT GTTCTTGAACGTGCGGACACAGGTGGCGGCCGAC TGGACCGCGCTGGCGGAGGAGATGGACTTTGAGT ACTTGGAGATCCGGCAACTGGAGACACAAGCGGA CCCCACTGGCAGGCTGCTGGACGCCTGGCAGGGA CGCCCTGGCGCCTCTGTAGGCCGACTGCTCGATCT GCTTACCAAGCTGGGCCGCGACGACGTGCTGCTG GAGCTGGGACCCAGCATTGAGGAGGATTGCCAAAA GTATATCTTGAAGCAGCAGCAGGAGGAGGCTGAGA AGCCTTTACAGGTGGCCGCTGTAGACAGCAGTGTC | MAAGGPGAGSAAPVSS TSSLPLAALNMRVRRRL SLFLNVRTQVAADWTAL AEEMDFEYLEIRQLETQ ADPTGRLLDAWQGRPG ASVGRLLDLLTKLGRDD VLLELGPSIEEDCQKYIL KQQQEEAEKPLQVAAV DSSVPRTAELAGITTLDD PLGHMPERFDAFICYCP SDI |
| | CCACGGACAGCAGAGCTGGCGGCATCACCACAC TTGATGACCCCCTGGGGCATATGCCTGAGCGTTTC GATGCCTTCATCTGCTATTGCCCCAGCGACATC | |
| **Linker** | GTCGAG | VG |
| **hCD40** | AAAAAGGTGGCCAAGAAGCCAACCAATAAGGCCCC CCACCCCAAGCAGGAGCCCCAGGAGATCAATTTTC CCGACGATCTTCCTGGCTCCAACACTGCTGCTCCA GTGCAGGAGACTTTACATGGATGCCAACCGGTCAC CCAGGAGGATGGCAAAGAGAGTCGCATCTCAGTG CAGGAGAGACAG | KKVAKKPTNKAPHPKQE PQEINFPDDLPGSNTAA PVQETLHGCQPVTQED GKESRISVQERQ |
| **Linker** | GTCGAG | VG |
| **Fv'** | GGCGTCCAAGTCGAAACCATTAGTCCCGGCGATGG CAGAACATTTCCTAAAGGGACAAACATGTGTCG TCCATTATACAGGCATGTTGGAGGACGGCAAAAG GTGGACAGTAGTAGAGATCGCAATAAACCTTTCAAA TTCATGTTGGGAAAACAAGAAGTCATTAGGGGATG GGAGGAGGGCGTGGCTCAAATGTCCGTCGGCCAA CGCGCTAAGCTCACCATCAGCCCCGACTACGCATA CGGCGCTACCGGACATCCCGGAATTATTCCCCCTC ACGCTACCTTGGTGTTTGACGTCGAACTGTTGAAG CTCGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKLE |
| **Linker** | GTCGAG | VG |
| **Fv** | GGAGTGCAGGTGGAGACTATCTCCCCAGGAGACG GGCGCACCTTCCCCAAGCGCGGCCAGACCTGCGT GGTGCACTACACCGGGATGCTTGAAGATGGAAAGA AAGTTGATTCCTCCCGGGACAGAAACAAGCCCTTT AAGTTTATGCTAGGCAAGCAGGAGGTGATCCGAGG CTGGGAAGAAGGGGTTGCCCAGATGAGTGTGGGT CAGAGAGCCAAACTGACTATATCTCCAGATTATGCC TATGGTGCCACTGGGCACCCAGGCATCATCCCACC ACATGCCACTCTCGTCTTCGATGTGGAGCTTCTAAA ACTGGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKLE |
| **Linker** | CCGCGG | PR |
| **T2A** | GAAGGCCGAGGGAGCCTGCTGACATGTGGCGATG TGGAGGAAAACCCAGGACCA | EGRGSLLTCGDVEENP GP |
| **ΔCD19** | ATGCCACCACCTCGCCTGCTGTTCTTTCTGCTGTTC | MPPPRLLFFLLFLTPMEV |
| | CTGACACCTATGGAGGTGCGACCTGAGGAACCACT GGTCGTGAAGGTCGAGGAAGGCGACAATGCCGTG CTGCAGTGCCTGAAAGGCACTTCTGATGGCCAAC TCAGCAGCTGACCTGGTCCAGGGAGTCTCCCCTGA AGCCTTTTCTGAAACTGAGCCTGGGACTGCCAGGA CTGGGAATCCACATGCGCCCTCTGGCTATCTGGCT GTTCATCTTCAACGTGAGCCAGCAGATGGGAGGAT TCTACCTGTGCCAGCCAGGACCACCATCCGAGAAG GCCTGGCAGCCTGGATGGACCGTCAACGTGGAGG GGTCTGGAGAACTGTTTAGGTGGAATGTGAGTGAC CTGGGAGGACTGGGATGTGGCTGAAGAACCGCT CCTCTGAAGGCCCAAGTTCACCCTCAGGGAAGCTG ATGAGCCCAAAACTGTACGTGTGGGCCAAAGATCG GCCCGAGATCTGGGAGGGAGAACCTCCATGCCTG CCACCTAGAGACAGCCTGAATCAGAGTCTGTCACA GGATCTGACAATGGCCCCCGGGTCCACTCTGTGG CTGTCTTGTGGAGTCCCACCCGACAGCGTGTCCAG AGGCCCTCTGTCCTGGACCCACGTGCATCCTAAGG GGCCAAAAAGTCTGCTGTCACTGGAACTGAAGGAC GATCGGCCTGCCAGAGACATGTGGGTCATGGAGA CTGGACTGCTGCTGCCACGAGCAACCGCACAGGA TGCTGGAAAATACTATTGCCACCGGGGCAATCTGA CAATGTCCTTCCATCTGGAGATCACTGCAAGGCCC GTGCTGTGGCACTGGCTGCTGCGAACCGGAGGAT GGAAGGTCAGTGCTGTGACACTGGCATATCTGATC TTTTGCCTGTGCTCCCTGGTGGGCATTCTGCATCT GCAGAGAGCCCTGGTGCTGCGGAGAAAGAGAAAG AGAATGACTGACCCAACAAGAAGGTTTTGA | RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF* |

### pBP0607--pSFG-k-iMC.2A-ΔCD19

| Fragment | Nucleotide | Peptide |
|---|---|---|
| **Myr** | ATGGGGAGTAGCAAGAGCAAGCCTAAGGACCCCA GCCAGCGC | MGSSKSKPKDPSQR |
| **Linker** | CTCGAC | LN |
| **MyD88** | ATGGCTGCAGGAGGTCCCGGCGCGGGGTCTGCG | MAAGGPGAGSAAPVSS |
| | GCCCCGGTCTCCTCCACATCCTCCCTTCCCCTGG CTGCTCTCAACATGCGAGTGCGGCGCCGCCTGTC TCTGTTCTTGAACGTGCGGACACAGGTGGCGGCC GACTGGACCGCGCTGGCGGAGGAGATGGACTTT GAGTACTTGGAGATCCGGCAACTGGAGACACAAG CGGACCCCACTGGCAGGCTGCTGGACGCCTGGC AGGGACGCCCTGGCGCCTCTGTAGGCCGACTGCT CGATCTGCTTACCAAGCTGGGCCGCGACGACGTG CTGCTGGAGCTGGGACCCAGCATTGAGGAGGATT GCCAAAAGTATATCTTGAAGCAGCAGCAGGAGGA GGCTGAGAAGCCTTTACAGGTGGCCGCTGTAGAC AGCAGTGTCCCACGGACAGCAGAGCTGGCGGGC ATCACCACACTTGATGACCCCCTGGGGCATATGC CTGAGCGTTTCGATGCCTTCATCTGCTATTGCCCC AGCGACATC | TSSLPLAALNMRVRRRL SLFLNVRTQVAADWTAL AEEMDFEYLEIRQLETQ ADPTGRLLDAWQGRPG ASVGRLLDLLTKLGRDD VLLELGPSIEEDCQKYIL KQQQEEAEKPLQVAAV DSSVPRTAELAGITTLDD PLGHMPERFDAFICYCP SDI |
| **Linker** | GTCGAG | VG |
| **hCD40** | AAAAAGGTGGCCAAGAAGCCAACCAATAAGGCCC CCCACCCCAAGCAGGAGCCCCAGGAGATCAATTT TCCCGACGATCTTCCTGGCTCCAACACTGCTGCTC CAGTGCAGGAGACTTTACATGGATGCCAACCGGT CACCCAGGAGGATGGCAAAGAGAGTCGCATCTCA GTGCAGGAGAGACAG | KKVAKKPTNKAPHPKQE PQEINFPDDLPGSNTAA PVQETLHGCQPVTQED GKESRISVQERQ |
| **Linker** | GTCGAG | VG |
| **Fv'** | GGCGTCCAAGTCGAAACCATTAGTCCCGGCGATG GCAGAACATTTCCTAAAAGGGGACAAACATGTGTC GTCCATTATACAGGCATGTTGGAGGACGGCAAAA AGGTGGACAGTAGTAGAGATCGCAATAAACCTTTC AAATTCATGTTGGGAAAACAAGAAGTCATTAGGGG ATGGGAGGAGGGCGTGGCTCAAATGTCCGTCGG CCAACGCGCTAAGCTCACCATCAGCCCCGACTAC GCATACGGCGCTACCGGACATCCCGGAATTATTC CCCCTCACGCTACCTTGGTGTTTGACGTCGAACTG TTGAAGCTCGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKLE |
| **Linker** | GTCGAG | VG |
| **Fv** | GGAGTGCAGGTGGAGACTATCTCCCCAGGAGACG GGCGCACCTTCCCCAAGCGCGGCCAGACCTGCG | GVQVETISPGDGRTFPK RGQTCVVHYTGMLEDG |
| | TGGTGCACTACACCGGGATGCTTGAAGATGGAAA GAAAGTTGATTCCTCCCGGGACAGAAACAAGCCC TTTAAGTTTATGCTAGGCAAGCAGGAGGTGATCCG AGGCTGGGAAGAAGGGGTTGCCCAGATGAGTGTG GGTCAGAGAGCCAAACTGACTATATCTCCAGATTA TGCCTATGGTGCCACTGGGCACCCAGGCATCATC CCACCACATGCCACTCTCGTCTTCGATGTGGAGCT TCTAAAACTGGAA | KKVDSSRDRNKPFKFML GKQEVIRGWEEGVAQM SVGQRAKLTISPDYAYG ATGHPGIIPPHATLVFDV ELLKLE |
| **Linker** | CCGCGG | PR |
| **T2A** | GAAGGCCGAGGGAGCCTGCTGACATGTGGCGAT GTGGAGGAAAACCCAGGACCA | EGRGSLLTCGDVEENP GP |
| **ΔCD19** | ATGCCACCACCTCGCCTGCTGTTCTTTCTGCTGTT CCTGACACCTATGGAGGTGCGACCTGAGGAACCA CTGGTCGTGAAGGTCGAGGAAGGCGACAATGCCG TGCTGCAGTGCCTGAAAGGCACTTCTGATGGGCC AACTCAGCAGCTGACCTGGTCCAGGGAGTCTCCC CTGAAGCCTTTTCTGAAACTGAGCCTGGGACTGC CAGGACTGGGAATCCACATGCGCCCTCTGGCTAT CTGGCTGTTCATCTTCAACGTGAGCCAGCAGATG GGAGGATTCTACCTGTGCCAGCCAGGACCACCAT CCGAGAAGGCCTGGCAGCCTGGATGGACCGTCAA CGTGGAGGGGTCTGGAGAACTGTTTAGGTGGAAT GTGAGTGACCTGGGAGGACTGGGATGTGGGCTG AAGAACCGCTCCTCTGAAGGCCCAAGTTCACCCT CAGGGAAGCTGATGAGCCCAAAACTGTACGTGTG GGCCAAAGATCGGCCCGAGATCTGGAGGGAGA ACCTCCATGCCTGCCACCTAGAGACAGCCTGAAT CAGAGTCTGTCACAGGATCTGACAATGGCCCCCG GGTCCACTCTGTGGCTGTCTTGTGGAGTCCCACC CGACAGCGTGTCCAGAGGCCCTCTGTCCTGGACC CACGTGCATCCTAAGGGGCCAAAAAGTCTGCTGT CACTGGAACTGAAGGACGATCGGCCTGCCAGAGA CATGTGGGTCATGGAGACTGGACTGCTGCTGCCA CGAGCAACCGCACAGGATGCTGGAAAATACTATT GCCACCGGGGCAATCTGACAATGTCCTTCCATCT GGAGATCACTGCAAGGCCCGTGCTGTGGCACTGG | MPPPRLLFFLLFLTPMEV RPEEPLVVKVEEGDNAV LQCLKGTSDGPTQQLT WSRESPLKPFLKLSLGL PGLGIHMRPLAIWLFIFN VSQQMGGFYLCQPGPP SEKAWQPGWTVNVEGS GELFRWNVSDLGGLGC GLKNRSSEGPSSPSGKL MSPKLYVWAKDRPEIWE GEPPCLPPRDSLNQSLS QDLTMAPGSTLWLSCG VPPDSVSRGPLSWTHV HPKGPKSLLSLELKDDR PARDMWVMETGLLLPR ATAQDAGKYYCHRGNL TMSFHLEITARPVLWHW LLRTGGWKVSAVTLAYLI FCLCSLVGILHLQRALVL RRKRKRMTDPTRRF* |
| | CTGCTGCGAACCGGAGGATGGAAGGTCAGTGCTG TGACACTGGCATATCTGATCTTTTGCCTGTGCTCC CTGGTGGGCATTCTGCATCTGCAGAGAGCCCTGG TGCTGCGGAGAAAGAGAAAGAGAATGACTGACCC AACAAGAAGGTTTTGA | |

### pBP0668--pSFG-FRB_{I}x2.Caspase9.2A-Q.8stm.CD3zeta

| Fragment | Nucleotide | Peptide |
|---|---|---|
| **FRB_{I}'** | TGGCATGAAGGCCTGGAAGAGGCATCTCGTTTGTA CTTTGGGGAAAGGAACGTGAAAGGCATGTTTGAGG TGCTGGAGCCCTTGCACGCTATGATGGAACGGGG CCCCCAGACTCTGAAGGAAACATCCTTTAATCAGG CCTATGGTCGAGATTTAATGGAGGCCCAAGAGTGG TGCAGGAAGTACATGAAATCAGGGAATGTCAAGGA CCTCCTCCAAGCCTGGGACCTCTATTATCATGTGTT CCGACGAATCTCAAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | GTCGAG | VG |
| **FRB_{I}** | TGGCATGAAGGGTTGGAAGAAGCTTCAAGGCTGTA CTTCGGAGAGAGGAACGTGAAGGGCATGTTTGAG GTTCTTGAACCTCTGCACGCCATGATGGAACGGGG ACCGCAGACACTGAAAGAAACCTCTTTTAATCAGG CCTACGGCAGAGACCTGATGGAGGCCCAAGAATG GTGTAGAAAGTATATGAAATCCGGTAACGTGAAAG ACCTGCTCCAGGCCTGGGACCTTTATTACCATGTG TTCAGGCGGATCAGTAAG | WHEGLEEASRLYFGER NVKGMFEVLEPLHAMM ERGPQTLKETSFNQAYG RDLMEAQEWCRKYMKS GNVKDLLQAWDLYYHVF RRISK |
| **Linker** | TCAGGCGGTGGCTCAGGT | SGGGSG |
| **ΔCaspas e9** | TCGACGGATTTGGTGATGTCGGTGCTCTTGAGAGT TTGAGGGGAAATGCAGATTTGGCTTACATCCTGAG CATGGAGCCCTGTGGCCACTGCCTCATTATCAACA ATGTGAACTTCTGCCGTGAGTCCGGGCTCCGCACC CGCACTGGCTCCAACATCGACTGTGAGAAGTTGCG GCGTCGCTTCTCCTCGCTGCATTTCATGGTGGAGG TGAAGGGCGACCTGACTGCCAAGAAAATGGTGCTG GCTTTGCTGGAGCTGGCGCGGCAGGACCACGGTG | DGFGDVGALESLRGNA DLAYILSMEPCGHCLIIN NVNFCRESGLRTRTGSN IDCEKLRRRFSSLHFMV EVKGDLTAKKMVLALLE LARQDHGALDCCVVVIL SHGCQASHLQFPGAVY GTDGCPVSVEKIVNIFNG |
| | CTCTGGACTGCTGCGTGGTGGTCATTCTCTCTCAC GGCTGTCAGGCCAGCCACCTGCAGTTCCCAGGGG CTGTCTACGGCACAGATGGATGCCCTGTGTCGGTC GAGAAGATTGTGAACATCTTCAATGGGACCAGCTG CCCCAGCCTGGGAGGGAAGCCCAAGCTCTTTTCA TCCAGGCCTGTGGTGGGGAGCAGAAAGACCATGG GTTTGAGGTGGCCTCCACTTCCCCTGAAGACGAGT CCCCTGGCAGTAACCCCGAGCCAGATGCCACCCC GTTCCAGGAAGGTTTGAGGACCTTCGACCAGCTGG ACGCCATATCTAGTTTGCCCACACCCAGTGACATCT TTGTGTCCTACTCTACTTTCCCAGGTTTTGTTTCCT GGAGGGACCCCAAGAGTGGCTCCTGGTACGTTGA GACCCTGGACGACATCTTTGAGCAGTGGGCTCACT CTGAAGACCTGCAGTCCCTCCTGCTTAGGGTCGCT AATGCTGTTTCGGTGAAAGGGATTTATAAACAGATG CCTGGTTGCTTTAATTTCCTCCGGAAAAAACTTTTC TTTAAAACATCAGCTAGCAGAGCC | TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| **Linker** | CCGCGG | PR |
| **T2A** | GAAGGCCGAGGGAGCCTGCTGACATGTGGCGATG TGGAGGAAAACCCAGGACCA | EGRGSLLTCGDVEENP GP |
| **Signal Peptide** | ATGGAATTTGGCCTCTCCTGGTTGTTTCTCGTGGC CATTCTTAAGGGTGTGCAGTGCTCCAGA | MEFGLSWLFLVAILKGV QCSR |
| **Linker** | ATGCAT | MH |
| **Q-Bend (CD34 Epitope)** | GAACTTCCTACTCAGGGGACTTTCTCAAACGTTAGC ACAAACGTAAGT | ELPTQGTFSNVSTNVS |
| **CD8 Stalk** | CCCGCCCCAAGACCCCCCACACCTGCGCCGACCA TTGCTTCTCAACCCCTGAGTTTGAGACCCGAGGCC TGCCGGCCAGCTGCCGGCGGGGCCGTGCATACAA GAGGACTCGATTTCGCTTGCGAC | PAPRPPTPAPTIASQPLS LRPEACRPAAGGAVHTR GLDFACD |
| **CD8a tm** | ATCTATATCTGGGCACCTCTCGCTGGCACCTGTGG AGTCCTTCTGCTCAGCCTGGTTATTACTCTGTACTG TAATCACCGGAATCGCCGCCGCGTTTGTAAGTGTC CCAGGGTCGAC | IYIWAPLAGTCGVLLLSL VITLYCNHRNRRRVCKC PRVD |
| **CD3 zeta** | AGAGTGAAGTTCAGCAGGAGCGCAGACGCCCCCG CGTACCAGCAGGGCCAGAACCAGCTCTATAACGAG | RVKFSRSADAPAYQQG QNQLYNELNLGRREEYD |
| | CTCAATCTAGGACGAAGAGAGGAGTACGATGTTTT GGACAAGAGACGTGGCCGGGACCCTGAGATGGGG GGAAAGCCGAGAAGGAAGAACCCTCAGGAAGGCC TGTACAATGAACTGCAGAAAGATAAGATGGCGGAG GCCTACAGTGAGATTGGGATGAAAGGCGAGCGCC GGAGGGGCAAGGGGCACGATGGCCTTTACCAGGG TCTCAGTACAGCCACCAAGGACACCTACGACGCCC TTCACATGCAAGCTCTTCCACCTCG | VLDKRRGRDPEMGGKP RRKNPQEGLYNELQKD KMAEAYSEIGMKGERRR GKGHDGLYQGLSTATK DTYDALHMQALPP |

### pBP0608--pSFG-ΔMyr.iMC.2A-ΔCD19.Q.8stm.CD3zeta

| **Fragmen t** | **Nucleotide** | **Peptide** |
|---|---|---|
| **MyD88** | ATGGCTGCAGGAGGTCCCGGCGCGGGGTCTGCGG CCCCGGTCTCCTCCACATCCTCCCTTCCCCTGGCT GCTCTCAACATGCGAGTGCGGCGCCGCCTGTCTCT GTTCTTGAACGTGCGGACACAGGTGGCGGCCGAC TGGACCGCGCTGGCGGAGGAGATGGACTTTGAGT ACTTGGAGATCCGGCAACTGGAGACACAAGCGGA CCCCACTGGCAGGCTGCTGGACGCCTGGCAGGGA CGCCCTGGCGCCTCTGTAGGCCGACTGCTCGATCT GCTTACCAAGCTGGGCCGCGACGACGTGCTGCTG GAGCTGGGACCCAGCATTGAGGAGGATTGCCAAAA GTATATCTTGAAGCAGCAGCAGGAGGAGGCTGAGA AGCCTTTACAGGTGGCCGCTGTAGACAGCAGTGTC CCACGGACAGCAGAGCTGGCGGGCATCACCACAC TTGATGACCCCCTGGGGCATATGCCTGAGCGTTTC GATGCCTTCATCTGCTATTGCCCCAGCGACATC | MAAGGPGAGSAAPVSS TSSLPLAALNMRVRRRL SLFLNVRTQVAADWTAL AEEMDFEYLEIRQLETQ ADPTGRLLDAWQGRPG ASVGRLLDLLTKLGRDD VLLELGPSIEEDCQKYIL KQQQEEAEKPLQVAAV DSSVPRTAELAGITTLDD PLGHMPERFDAFICYCP SDI |
| **Linker** | GTCGAG | VE |
| **hCD40** | AAAAAGGTGGCCAAGAAGCCAACCAATAAGGCCCC CCACCCCAAGCAGGAGCCCCAGGAGATCAATTTTC CCGACGATCTTCCTGGCTCCAACACTGCTGCTCCA GTGCAGGAGACTTTACATGGATGCCAACCGGTCAC CCAGGAGGATGGCAAAGAGAGTCGCATCTCAGTG CAGGAGAGACAG | KKVAKKPTNKAPHPKQE PQEINFPDDLPGSNTAA PVQETLHGCQPVTQED GKESRISVQERQ |
| **Linker** | GTCGAG | VE |
| **Fv'** | GGCGTCCAAGTCGAAACCATTAGTCCCGGCGATGG CAGAACATTTCCTAAAAGGGACAAACATGTGTCG TCCATTATACAGGCATGTTGGAGGACGGCAAAAG GTGGACAGTAGTAGAGATCGCAATAAACCTTTCAAA TTCATGTTGGGAAAACAAGAAGTCATTAGGGGATG GGAGGAGGGCGTGGCTCAAATGTCCGTCGGCCAA CGCGCTAAGCTCACCATCAGCCCCGACTACGCATA CGGCGCTACCGGACATCCCGGAATTATTCCCCCTC ACGCTACCTTGGTGTTTGACGTCGAACTGTTGAAG CTCGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKLE |
| **Linker** | GTCGAG | VE |
| **Fv** | GGAGTGCAGGTGGAGACTATCTCCCCAGGAGACG GGCGCACCTTCCCCAAGCGCGGCCAGACCTGCGT GGTGCACTACACCGGGATGCTTGAAGATGGAAAGA AAGTTGATTCCTCCCGGGACAGAAACAAGCCCTTT AAGTTTATGCTAGGCAAGCAGGAGGTGATCCGAGG CTGGGAAGAAGGGGTTGCCCAGATGAGTGTGGGT CAGAGAGCCAAACTGACTATATCTCCAGATTATGCC TATGGTGCCACTGGGCACCCAGGCATCATCCCACC ACATGCCACTCTCGTCTTCGATGTGGAGCTTCTAAA ACTGGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKLE |
| **Linker** | CCGCGG | PR |
| **T2A** | GAAGGCCGAGGGAGCCTGCTGACATGTGGCGATG TGGAGGAAAACCCAGGACCA | EGRGSLLTCGDVEENP GP |
| **Linker** | CCATGG | PW |
| **Signal Peptide** | ATGGAGTTTGGACTTTCTTGGTTGTTTTTGGTGGCA ATTCTGAAGGGTGTCCAGTGTAGCAGG | MEFGLSWLFLVAILKGV QCSR |
| **FMC63- VL** | GACATCCAGATGACACAGACTACATCCTCCCTGTC TGCCTCTCTGGGAGACAGAGTCACCATCAGTTGCA GGGCAAGTCAGGACATTAGTAAATATTTAAATTGGT ATCAGCAGAAACCAGATGGAACTGTTAAACTCCTG ATCTACCATACATCAAGATTACACTCAGGAGTCCCA TCAAGGTTCAGTGGCAGTGGGTCTGGAACAGATTA TTCTCTCACCATTAGCAACCTGGAGCAAGAAGATAT TGCCACTTACTTTTGCCAACAGGGTAATACGCTTCC GTACACGTTCGGAGGGGGGACTAAGTTGGAAATAA | DIQMTQTTSSLSASLGD RVTISCRASQDISKYLN WYQQKPDGTVKLLIYHT SRLHSGVPSRFSGSGS GTDYSLTISNLEQEDIAT YFCQQGNTLPYTFGGG TKLEIT |
| | CA | |
| **Flex Linker** | GGCGGAGGAAGCGGAGGTGGGGGC | GGGSGGGG |
| **FMC63- VH** | GAGGTGAAACTGCAGGAGTCAGGACCTGGCCTGG TGGCGCCCTCACAGAGCCTGTCCGTCACATGCACT GTCTCAGGGGTCTCATTACCCGACTATGGTGTAAG CTGGATTCGCCAGCCTCCACGAAAGGGTCTGGAGT GGCTGGGAGTAATATGGGGTAGTGAAACCACATAC TATAATTCAGCTCTCAAATCCAGACTGACCATCATC AAGGACAACTCCAAGAGCCAAGTTTTCTTAAAAATG AACAGTCTGCAAACTGATGACACAGCCATTTACTAC TGTGCCAAACATTATTACTACGGTGGTAGCTATGCT ATGGACTACTGGGGTCAAGGAACCTCAGTCACCGT CTCCTCA | EVKLQESGPGLVAPSQS LSVTCTVSGVSLPDYGV SWIRQPPRKGLEWLGVI WGSETTYYNSALKSRLT IIKDNSKSQVFLKMNSLQ TDDTAIYYCAKHYYYGG SYAMDYWGQGTSVTVS S |
| **Linker** | GGATCC | GS |
| **Q-Bend (CD34 Epitope)** | GAACTTCCTACTCAGGGGACTTTCTCAAACGTTAGC ACAAACGTAAGT | ELPTQGTFSNVSTNVS |
| **CD8 Stalk** | CCCGCCCCAAGACCCCCCACACCTGCGCCGACCA TTGCTTCTCAACCCCTGAGTTTGAGACCCGAGGCC TGCCGGCCAGCTGCCGGCGGGGCCGTGCATACAA GAGGACTCGATTTCGCTTGCGAC | PAPRPPTPAPTIASQPLS LRPEACRPAAGGAVHTR GLDFACD |
| **CD8a tm** | ATCTATATCTGGGCACCTCTCGCTGGCACCTGTGG AGTCCTTCTGCTCAGCCTGGTTATTACTCTGTACTG TAATCACCGGAATCGCCGCCGCGTTTGTAAGTGTC CCAGG | IYIWAPLAGTCGVLLLSL VITLYCNHRNRRRVCKC PR |
| **Linker** | GTCGAC | VD |
| **CD3 zeta** | AGAGTGAAGTTCAGCAGGAGCGCAGACGCCCCCG CGTACCAGCAGGGCCAGAACCAGCTCTATAACGAG CTCAATCTAGGACGAAGAGAGGAGTACGATGTTTT GGACAAGAGACGTGGCCGGGACCCTGAGATGGGG GGAAAGCCGAGAAGGAAGAACCCTCAGGAAGGCC TGTACAATGAACTGCAGAAAGATAAGATGGCGGAG GCCTACAGTGAGATTGGGATGAAAGGCGAGCGCC GGAGGGGCAAGGGGCACGATGGCCTTTACCAGGG TCTCAGTACAGCCACCAAGGACACCTACGACGCCC | RVKFSRSADAPAYQQG QNQLYNELNLGRREEYD VLDKRRGRDPEMGGKP RRKNPQEGLYNELQKD KMAEAYSEIGMKGERRR GKGHDGLYQGLSTATK DTYDALHMQALPP |
| | TTCACATGCAAGCTCTTCCACCTCG | |

### pBP0609: pSFG-iMC.2A-ΔCD19.Q.8stm.CD3zeta

| **Fragmen t** | **Nucleotide** | **Peptide** |
|---|---|---|
| **Myr** | ATGGGGAGTAGCAAGAGCAAGCCTAAGGACCCCA GCCAGCGC | MGSSKSKPKDPSQR |
| **Linker** | CTCGAC | LD |
| **MyD88** | ATGGCTGCAGGAGGTCCCGGCGCGGGGTCTGCGG CCCCGGTCTCCTCCACATCCTCCCTTCCCCTGGCT GCTCTCAACATGCGAGTGCGGCGCCGCCTGTCTCT GTTCTTGAACGTGCGGACACAGGTGGCGGCCGAC TGGACCGCGCTGGCGGAGGAGATGGACTTTGAGT ACTTGGAGATCCGGCAACTGGAGACACAAGCGGA CCCCACTGGCAGGCTGCTGGACGCCTGGCAGGGA CGCCCTGGCGCCTCTGTAGGCCGACTGCTCGATCT GCTTACCAAGCTGGGCCGCGACGACGTGCTGCTG GAGCTGGGACCCAGCATTGAGGAGGATTGCCAAAA GTATATCTTGAAGCAGCAGCAGGAGGAGGCTGAGA AGCCTTTACAGGTGGCCGCTGTAGACAGCAGTGTC CCACGGACAGCAGAGCTGGCGGGCATCACCACAC TTGATGACCCCCTGGGGCATATGCCTGAGCGTTTC GATGCCTTCATCTGCTATTGCCCCAGCGACATC | MAAGGPGAGSAAPVSS TSSLPLAALNMRVRRRL SLFLNVRTQVAADWTAL AEEMDFEYLEIRQLETQ ADPTGRLLDAWQGRPG ASVGRLLDLLTKLGRDD VLLELGPSIEEDCQKYIL KQQQEEAEKPLQVAAV DSSVPRTAELAGITTLDD PLGHMPERFDAFICYCP SDI |
| **Linker** | GTCGAG | VE |
| **hCD40** | AAAAAGGTGGCCAAGAAGCCAACCAATAAGGCCCC CCACCCCAAGCAGGAGCCCCAGGAGATCAATTTTC CCGACGATCTTCCTGGCTCCAACACTGCTGCTCCA GTGCAGGAGACTTTACATGGATGCCAACCGGTCAC CCAGGAGGATGGCAAAGAGAGTCGCATCTCAGTG CAGGAGAGACAG | KKVAKKPTNKAPHPKQE PQEINFPDDLPGSNTAA PVQETLHGCQPVTQED GKESRISVQERQ |
| **Linker** | GTCGAG | VE |
| **Fv'** | GGCGTCCAAGTCGAAACCATTAGTCCCGGCGATGG CAGAACATTTCCTAAAAGGGGACAAACATGTGTCG TCCATTATACAGGCATGTTGGAGGACGGCAAAAAG GTGGACAGTAGTAGAGATCGCAATAAACCTTTCAAA | GVQVETISPGDGRTFPK RGQTCVVHYTGMLEDG KKVDSSRDRNKPFKFML GKQEVIRGWEEGVAQM |
| | TTCATGTTGGGAAAACAAGAAGTCATTAGGGGATG GGAGGAGGGCGTGGCTCAAATGTCCGTCGGCCAA CGCGCTAAGCTCACCATCAGCCCCGACTACGCATA CGGCGCTACCGGACATCCCGGAATTATTCCCCCTC ACGCTACCTTGGTGTTTGACGTCGAACTGTTGAAG CTCGAA | SVGQRAKLTISPDYAYG ATGHPGIIPPHATLVFDV ELLKLE |
| **Linker** | GTCGAG | VE |
| **Fv** | GGAGTGCAGGTGGAGACTATCTCCCCAGGAGACG GGCGCACCTTCCCCAAGCGCGGCCAGACCTGCGT GGTGCACTACACCGGGATGCTTGAAGATGGAAAGA AAGTTGATTCCTCCCGGGACAGAAACAAGCCCTTT AAGTTTATGCTAGGCAAGCAGGAGGTGATCCGAGG CTGGGAAGAAGGGGTTGCCCAGATGAGTGTGGGT CAGAGAGCCAAACTGACTATATCTCCAGATTATGCC TATGGTGCCACTGGGCACCCAGGCATCATCCCACC ACATGCCACTCTCGTCTTCGATGTGGAGCTTCTAAA ACTGGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKLE |
| **Linker** | CCGCGG | PR |
| **T2A** | GAAGGCCGAGGGAGCCTGCTGACATGTGGCGATG TGGAGGAAAACCCAGGACCA | EGRGSLLTCGDVEENP GP |
| **Linker** | CCATGG | PW |
| **Signal Peptide** | ATGGAGTTTGGACTTTCTTGGTTGTTTTTGGTGGCA ATTCTGAAGGGTGTCCAGTGTAGCAGG | MEFGLSWLFLVAILKGV QCSR |
| **FMC63-VL** | GACATCCAGATGACACAGACTACATCCTCCCTGTC TGCCTCTCTGGGAGACAGAGTCACCATCAGTTGCA GGGCAAGTCAGGACATTAGTAAATATTTAAATTGGT ATCAGCAGAAACCAGATGGAACTGTTAAACTCCTG ATCTACCATACATCAAGATTACACTCAGGAGTCCCA TCAAGGTTCAGTGGCAGTGGGTCTGGAACAGATTA TTCTCTCACCATTAGCAACCTGGAGCAAGAAGATAT TGCCACTTACTTTTGCCAACAGGGTAATACGCTTCC GTACACGTTCGGAGGGGGGACTAAGTTGGAAATAA CA | DIQMTQTTSSLSASLGD RVTISCRASQDISKYLN WYQQKPDGTVKLLIYHT SRLHSGVPSRFSGSGS GTDYSLTISNLEQEDIAT YFCQQGNTLPYTFGGG TKLEIT |
| **Flex Linker** | GGCGGAGGAAGCGGAGGTGGGGGC | GGGSGGGG |
| **FMC63-VH** | GAGGTGAAACTGCAGGAGTCAGGACCTGGCCTGG | EVKLQESGPGLVAPSQS |
| | TGGCGCCCTCACAGAGCCTGTCCGTCACATGCACT GTCTCAGGGGTCTCATTACCCGACTATGGTGTAAG CTGGATTCGCCAGCCTCCACGAAAGGGTCTGGAGT GGCTGGGAGTAATATGGGGTAGTGAAACCACATAC TATAATTCAGCTCTCAAATCCAGACTGACCATCATC AAGGACAACTCCAAGAGCCAAGTTTTCTTAAAAATG AACAGTCTGCAAACTGATGACACAGCCATTTACTAC TGTGCCAAACATTATTACTACGGTGGTAGCTATGCT ATGGACTACTGGGGTCAAGGAACCTCAGTCACCGT CTCCTCA | LSVTCTVSGVSLPDYGV SWIRQPPRKGLEWLGVI WGSETTYYNSALKSRLT IIKDNSKSQVFLKMNSLQ TDDTAIYYCAKHYYYGG SYAMDYWGQGTSVTVS S |
| **Linker** | GGATCC | GS |
| **Q-Bend (CD34 Epitope)** | GAACTTCCTACTCAGGGGACTTTCTCAAACGTTAGC ACAAACGTAAGT | ELPTQGTFSNVSTNVS |
| **CD8 Stalk** | CCCGCCCCAAGACCCCCCACACCTGCGCCGACCA TTGCTTCTCAACCCCTGAGTTTGAGACCCGAGGCC TGCCGGCCAGCTGCCGGCGGGGCCGTGCATACAA GAGGACTCGATTTCGCTTGCGAC | PAPRPPTPAPTIASQPLS LRPEACRPAAGGAVHTR GLDFACD |
| **CD8a tm** | ATCTATATCTGGGCACCTCTCGCTGGCACCTGTGG AGTCCTTCTGCTCAGCCTGGTTATTACTCTGTACTG TAATCACCGGAATCGCCGCCGCGTTTGTAAGTGTC CCAGG | IYIWAPLAGTCGVLLLSL VITLYCNHRNRRRVCKC PR |
| **Linker** | GTCGAC | VD |
| **CD3 zeta** | AGAGTGAAGTTCAGCAGGAGCGCAGACGCCCCCG CGTACCAGCAGGGCCAGAACCAGCTCTATAACGAG CTCAATCTAGGACGAAGAGAGGAGTACGATGTTTT GGACAAGAGACGTGGCCGGGACCCTGAGATGGGG GGAAAGCCGAGAAGGAAGAACCCTCAGGAAGGCC TGTACAATGAACTGCAGAAAGATAAGATGGCGGAG GCCTACAGTGAGATTGGGATGAAAGGCGAGCGCC GGAGGGGCAAGGGGCACGATGGCCTTTACCAGGG TCTCAGTACAGCCACCAAGGACACCTACGACGCCC TTCACATGCAAGCTCTTCCACCTCG | RVKFSRSADAPAYQQG QNQLYNELNLGRREEYD VLDKRRGRDPEMGGKP RRKNPQEGLYNELQKD KMAEAYSEIGMKGERRR GKGHDGLYQGLSTATK DTYDALHMQALPP |

### Example 24: An Inducible Cell Death Switch Directed by Heterodimerizing Ligands

### Materials and Equipment

| Product | Specifications | Company | Catalog |
|---|---|---|---|
| D-PBS | US Certified | Sigma-Aldrich | D8537 |
| DMEM 4500 | NA | Sigma-Aldrich | D5796 |
| FBS | Clinical grade | GIBCO | 26140-079 |
| 96-well plate | NA | Greiner | 655180 |
| DMEM | NA | Sigma-Aldrich | D5796 |
| Rapamycin | Res. Grade | LC Labs | R5000 |
| Penicillin-Streptomycin | NA | Sigma | P0781 |
| 96-well plate black | NA | Greiner | 655076 |
| L-Glutamine | NA | Sigma-Aldrich | G7513 |
| 293-T cells | Sterile | ATCC | CRL-3216 |
| 4-Methylumbelliferylphosphate (4-MUP) | NA | Sigma | M8168 |
| Diethanolamine | NA | Sigma | D8885 |
| GeneJuice | NA | Novagen | 70967 |
| Trypsin-EDTA | NA | Sigma-Aldrich | T4049 |
| AP1903/Rimiducid | NA | Bellicum | NA |
| Isopropoxylrapamycin | NA | Bellicum | NA |
| OPTIMEM | NA | GIBCO | 31985-070 |
| Silica gel | NA | Sigma | 288624 |
| Ethyl acetate | NA | Sigma | 650528 |
| Hexanes | NA | Sigma | 227064 |
| p-toluene sulfonic acid | NA | Sigma | T35920 |

| Item | Specifications | Company | Model | Serial Number |
|---|---|---|---|---|
| Centrifuge | NA | Thermo Scientific | Sorval Legend XFR | 75004538 |
| Microcentrifuge | NA | Thermo Scientific | Sorval Micro 21 | 75002435 |
| Spectrophotometer | NA | Thermo | Nanodrop 2000 | G900 |
| Fluorescence plate reader | NA | BMG Labtek | Polaris Omega | 415 1361 |

### METHODS

### Transfection of cells

HEK 293T cells (5 x 10⁵) were seeded on a 100-mm tissue culture dish in 10 mL DMEM4500, supplemented with glutamine, penicillin/streptomycin and 10% fetal calf serum. After 16-30 hours incubation, cells were transfected using Novagen's GeneJuice® protocol. Briefly, for each transfection, 0.5 mL OptiMEM was pipeted into a 1.5-mL microcentrifuge tube and 15 µL GeneJuice reagent added followed by 5 sec. vortexing. Samples were rested 5 minutes to settle the GeneJuice suspension. DNA (5 µg total) was added to each tube and mixed by pipetting up and down four times. Samples were allowed to rest for 5 minutes for GeneJuice-DNA complex formation and the suspension added dropwise to one dish of 293T cells. A typical transfection contains 1 µg SRα-SEAP (pBP0046) (3), 2 µg FRB-Caspase-9 (pBP0463) and 2 µg FKBPv12-Caspase-9 (pBP0044) (7).

### Stimulation of cells with dimerizing drugs

24 hours following transfection (4.1), 293T cells were split to 96-well plates and incubated with dilutions of dimerizing drugs. Briefly, 100 µL media was added to each well of a 96-well flat-bottom plate. Drugs were diluted in tubes to a concentration 4X the top concentration in the gradient to be place on the plate. 100 µL of dimerizing ligand (rimiducid, rapamycin, isopropoxylrapamycin) was added to each of three wells on the far right of the plate (assays are thereby performed in triplicate). 100 µL from each drug-containing well was then transferred to the adjacent well and the cycle repeated 10 times to produce a serial two-fold step gradient. The last wells were untreated and serve as a control for basal reporter activity. Transfected 293 cells were then trypsinized, washed with complete media, suspended in media and 100 µL aliquoted to each well containing drug (or no drug). Cells were incubated 24 hours.

### Assay of reporter activity

The SRa promoter is a hybrid transcriptional element comprising the SV40 early region (which drives T antigen transcription) and parts (R and U5) of the Long Terminal Repeat (LTR) of Human T Cell Lymphotropic Virus (HTLV-1). This promoter drives high, constitutive levels of the Secreted Alkaline Phosphate (SeAP) reporter gene. Activation of caspase-9 by dimerization rapidly leads to cell death and the proportion of cells dying increases with increasing drug amounts. When cells die, transcription and translation of reporter stops but already secreted reporter proteins persists in the media. Loss of constitutive SeAP activity is thereby an effective proxy for drug-dependent activation of cell death.

24 hours after drug stimulation, 96-well plates were wrapped to prevent evaporation and incubated at 65®C for 2 hours to inactivate endogenous and serum phosphatases while the heat-stable SeAP reporter remains (1, 4, 12). 100 µL samples from each well were loaded into individual wells of a 96-well assay plate with black sides. Samples were incubated with 0.5 mM 4-methylumbelliferyl phosphate (4-MUP) in 0.5 M diethanolamine at pH 10.0 for 4 to 16 hours. Phosphatase activity was measured by fluorescence with excitation at 355 nm and emission at 460 nm. Data was transferred to a Microsoft Excel spreadsheet for tabulation and graphed with GraphPad Prism.

### Production of isopropyloxyrapamycin

The method of Luengo et al. ((J. Org. Chem 59:6512, (1994)), (16, 17)) was employed. Briefly, 20 mg of rapamycin was dissolved in in 3 mL isopropanol and 22.1 mg of p-toluene sulfonic acid was added and incubated at room temperature with stirring for 4-12 hours. At completion, 5 mL ethyl acetate was added and products were extracted five times with saturated sodium bicarbonate and 3 times with brine (saturated sodium chloride). The organic phase was dried and redissolved in ethyl acetate : hexane (3:1). Stereoisomers and minor products were resolved by FLASH chromatography on a 10 to 15-mL silica gel column with 3:1 ethyl acetate : hexane under 3-4 KPa pressure and fractions dried. Fractions were assayed by spectrophotometry at 237nM, 267 nM, 278 nM and 290 nM and tested for binding specificity in a FRB allele-specific transcriptional switch.

### Direct dimerization of FRB-Caspase with FKBP-Caspase with rapamycin directs apoptosis.

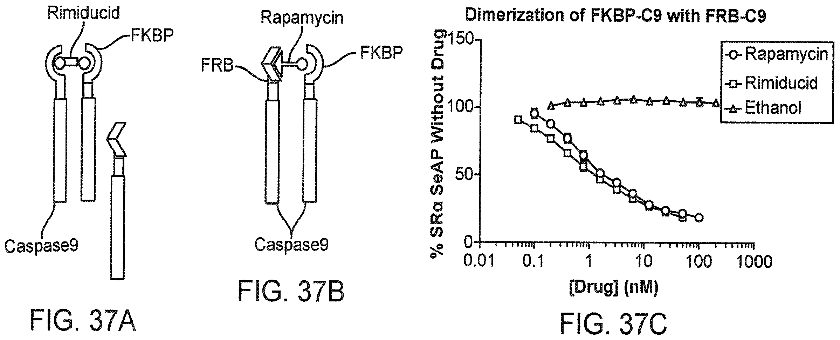

Dimerization of FKBP-fused caspases can be dimerized by homodimerizer molecules, such as AP1510, AP20187 or AP1903. A similar pro-apototic switch can be directed via heterodimerization of a binary switch using rapamycin by coexpression of a FRB-Caspase-9 fusion protein along with FKBP-Caspase-9, leading to homodimerization of the caspase domains. In figure 37, a constitutively active SeAP reporter plasmid was cotransfected into 293T cells along with the caspase constructs. Transfected cells abundantly produced SeAP that was readily measured without drug and which served as the 100% normalization standard in the experiment. Incubation of the two fusion proteins with rimiducid produces a dose-dependent homodimerization of only FKBP12-Caspase9, leading to dimerization and activation of apoptosis, while FRB-Caspase9 remains excluded from the rimiducid-driven complex (left). In contrast, incubation with rapamycin associates FRB and FKBP directly and linked Caspase-9 moieties associate and activate. Cell death was measured indirectly by the loss of SeAP reporter production as cells die. This experiment demonstrated that heterodimerization with rapamycin produces dose-dependent cell death, revealing a novel safety switch with nanomolar drug sensitivity.

Figure 37 - Drug induced programmed cell death by homodimerization or heterodimerization of tagged caspase 9. 293T cells were transfected with SRα-SeAP (pBP0046), pSH1-FKBPv12-Caspase9 (pBP0044) and pSH1-FRB_{L}-Caspase9 (pBP0463). After 24 hours incubation, cells were split and incubated with increasing concentrations of rapamycin (blue), rimiducid (red) or ethanol (the solvent containing stock rapamycin). Loss of reporter activity is a proxy for the loss of cell viability. Reporter activity is expressed as a percentage of the average of 8 control wells containing no drug. Assays with drugs were performed in triplicate.

### Cell death can be directed by rapamycin or rapamycin analogs.

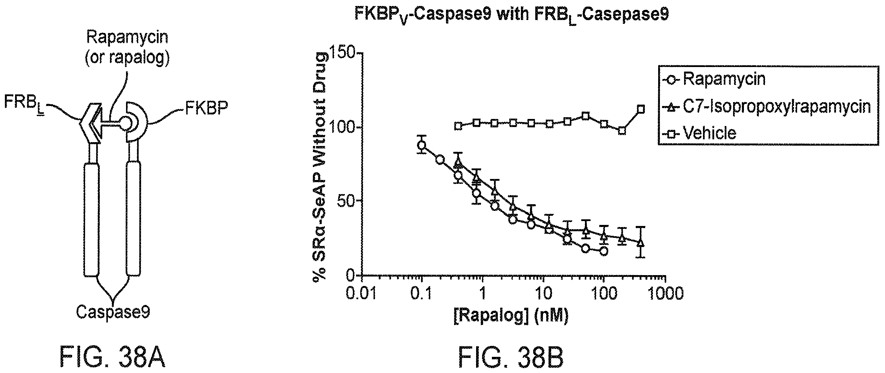

Rapamycin is an effective heterodimerizing agent, but as a result of causing the docking of FKBP12 with the protein kinase mTOR, rapamycin is also a potent inhibitor of signal transduction, resulting in reduced protein translation and reduced cell growth. Derivatives of rapamycin at C3 or C7 ring positions have reduced affinity for mTOR but retain high affinity for mutants in "helix 4" of the FRB domain. Plasmid pBP0463 contains a mutation that substitutes leucine for the wild-type threonine at position 2098 in the FRB domain (using the mTOR numbering) and accommodates derivatives at C7. Incubation of 293T cells transfected with FRB_{L}-Caspase 9, FKBP_{V}12-Caspase 9 and the constitutive SeAP reporter produced a dose-dependent high efficacy cell death switch with rapamycin or the rapamycin analog (rapalog) C7-isopropyloxlrapamycin (Figure 38).

Figure 38 - Rapalog-induced cell death switch. 293T cells were transfected with SRa-SeAP (pBP0046), pSH1-FKBPv12-Caspase9 (pBP0044) and pSH1-FRB_{L}-Caspase9 (pBP0463). After 24 hours incubation, cells were split and incubated with increasing concentrations of rapamycin (blue), C7-isopropyloxlrapamcin (green) or ethanol (the solvent containing drug stocks). Loss of reporter activity is a proxy for loss of cell viability. Reporter activity is expressed as a percentage of the average of 8 wells containing no drug. Drug-containing assays were performed in triplicate.

### Rapamycin-induced cell death requires the presence of FRB-Caspase-9.

To demonstrate that rapamycin-induced cell death results from dimerization of Caspase-9 molecules linked separately with FRB and FKBP12, two control experiments were performed

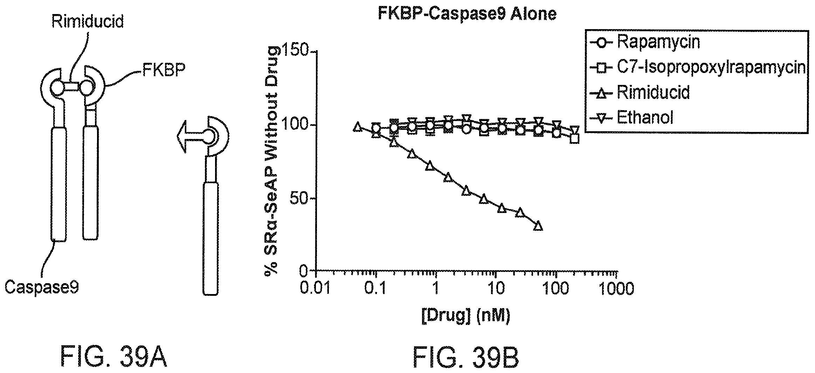

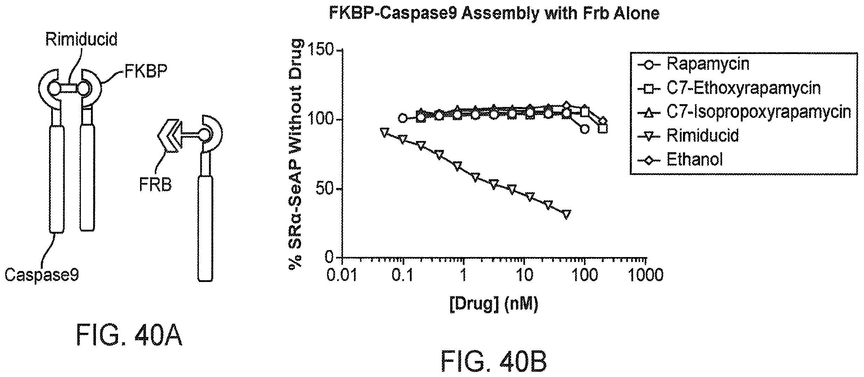

(Figures 39 and 40). iC9 (FKBPv12-Caspase-9) was cotransfected with a control vector expressing only an epitope tag (Figure 39) or a vector containing FRB without caspase fusion, but instead with a short, irrelevant tag (Figure 40). In each case, incubation with rimiducid effectively permitted homodimerization and induction of Caspase-9, but rapamycin incubation did not promote cell death. These findings support the conclusion that the mechanism of rapamycin/rapalog-mediated cell death is activation of dimerized C9 molecules rather than recruitment of mTOR to Caspase-9 or due to an indirect mechanism involving endogenous mTOR inhibition.

Figure 39 - FRB-Caspase-9 is required for a rapamycin-induced cell death switch. 293T cells were transfected with SRa-SeAP (pBP0046), pS-NLS-E and pSH1-FKBPv12-Caspase9 (pBP0044).

Figure 40 - Caspase-9 fusion with FRB is required for a rapamycin-induced cell death switch. 293T cells were transfected with SRa-SeAP (pBP0046), pSH1-FRB_{L}-VP16 (pBP0731) (4) and pSH1-FKBPv12-Caspase9 (pBP0044). After 24 hours incubation, cells were split and incubated with increasing concentrations of rapamycin (blue), C7-isopropyloxlrapamcin (red), rimiducid (green) or ethanol (the solvent containing drug stocks). Loss of reporter activity is a proxy for the loss of cell viability. Reporter activity is expressed as a percentage of the average of 8 wells containing no drug. Drug-containing wells were assayed in triplicate wells.

The following references are referred to in this Example:
1. Spencer DM, Wandless TJ, Schreiber SL, and Crabtree GR. Controlling signal transduction with synthetic ligands. Science. 1993;262(5136):1019-24.
2. Acevedo VD, Gangula RD, Freeman KW, Li R, Zhang Y, Wang F, Ayala GE, Peterson LE, Ittmann M, and Spencer DM. Inducible FGFR-1 activation leads to irreversible prostate adenocarcinoma and an epithelial-to-mesenchymal transition. Cancer Cell. 2007;12(6):559-71.
3. Spencer DM, Belshaw PJ, Chen L, Ho SN, Randazzo F, Crabtree GR, and Schreiber SL. Functional analysis of Fas signaling in vivo using synthetic inducers of dimerization. Curr Biol. 1996;6(7):839-47.
4. Bayle JH, Grimley JS, Stankunas K, Gestwicki JE, Wandless TJ, and Crabtree GR. Rapamycin analogs with differential binding specificity permit orthogonal control of protein activity. Chem Biol. 2006;13(1):99-107.
5. Strasser A, Cory S, and Adams JM. Deciphering the rules of programmed cell death to improve therapy of cancer and other diseases. EMBO J. 2011;30(18):3667-83.
6. Fan L, Freeman KW, Khan T, Pham E, and Spencer DM. Improved artificial death switches based on caspases and FADD. Hum Gene Ther. 1999;10(14):2273-85.
7. Straathof KC, Pule MA, Yotnda P, Dotti G, Vanin EF, Brenner MK, Heslop HE, Spencer DM, and Rooney CM. An inducible caspase 9 safety switch for T-cell therapy. Blood. 2005;105(11):4247-54.
8. Sabatini DM, Erdjument-Bromage H, Lui M, Tempst P, and Snyder SH. RAFT1: a mammalian protein that binds to FKBP12 in a rapamycin-dependent fashion and is homologous to yeast TORs. Cell. 1994;78(1):35-43.
9. Brown EJ, Albers MW, Shin TB, Ichikawa K, Keith CT, Lane WS, and Schreiber SL. A mammalian protein targeted by G1-arresting rapamycin-receptor complex. Nature. 1994;369(6483):756-8.
10. Chen J, Zheng XF, Brown EJ, and Schreiber SL. Identification of an 11-kDa FKBP12-rapamycin-binding domain within the 289-kDa FKBP12-rapamycin-associated protein and characterization of a critical serine residue. Proc Natl Acad Sci U S A. 1995;92(11):4947-51.
11. Choi J, Chen J, Schreiber SL, and Clardy J. Structure of the FKBP12-rapamycin complex interacting with the binding domain of human FRAP. Science. 1996;273(5272):239-42.
12. Ho SN, Biggar SR, Spencer DM, Schreiber SL, and Crabtree GR. Dimeric ligands define a role for transcriptional activation domains in reinitiation. Nature. 1996;382(6594):822-6.
13. Klemm JD, Beals CR, and Crabtree GR. Rapid targeting of nuclear proteins to the cytoplasm. Curr Biol. 1997;7(9):638-44.
14. Stankunas K, Bayle JH, Gestwicki JE, Lin YM, Wandless TJ, and Crabtree GR. Conditional protein alleles using knockin mice and a chemical inducer of dimerization. Mol Cell. 2003;12(6):1615-24.
15. Stankunas K, Bayle JH, Havranek JJ, Wandless TJ, Baker D, Crabtree GR, and Gestwicki JE. Rescue of Degradation-Prone Mutants of the FK506-Rapamycin Binding (FRB) Protein with Chemical Ligands. Chembiochem. 2007.
16. Liberles SD, Diver ST, Austin DJ, and Schreiber SL. Inducible gene expression and protein translocation using nontoxic ligands identified by a mammalian three-hybrid screen. Proc Natl Acad Sci U S A. 1997;94(15):7825-30.
17. Luengo JI, Yamashita DS, Dunnington D, Beck AK, Rozamus LW, Yen HK, Bossard MJ, Levy MA, Hand A, Newman-Tarr T, et al. Structure-activity studies of rapamycin analogs: evidence that the C-7 methoxy group is part of the effector domain and positioned at the FKBP12-FRAP interface. Chem Biol. 1995;2(7):471-81.

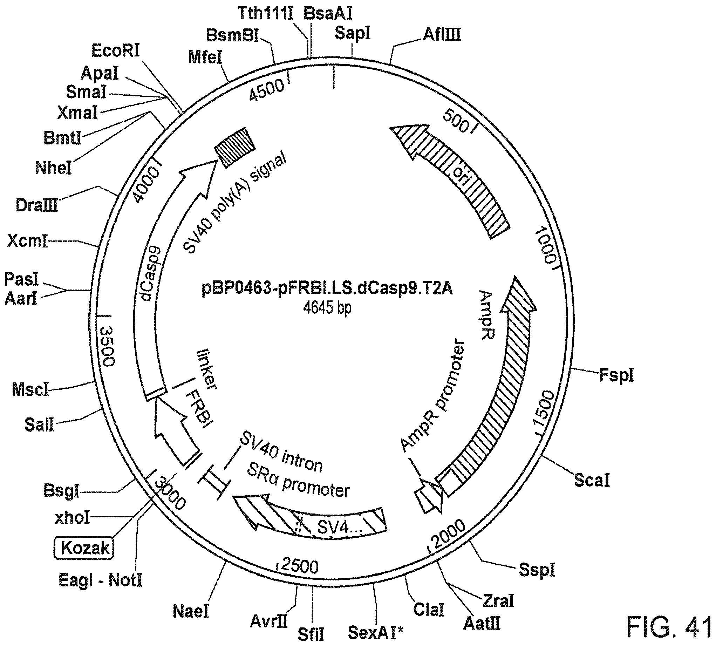

### pBP0463--pSH1-FRB_{L}.dCaspase9..T2A (From Figure 41)

| ***Fragment*** | ***Nucleotide*** | ***Peptide*** |
|---|---|---|
| ***Linker*** | *ATGCTCGAG* | *MLE* |
| ***FRB_{L}*** | TGGCATGAAGGGTTGGAAGAAGCTTCAAGGCTGT ACTTCGGAGAGAGGAACGTGAAGGGCATGTTTGA GGTTCTTGAACCTCTGCACGCCATGATGGAACGG GGACCGCAGACACTGAAAGAAACCTCTTTTAATC AGGCCTACGGCAGAGACCTGATGGAGGCCCAAG AATGGTGTAGAAAGTATATGAAATCCGGTAACGT GAAAGACCTGCTCCAGGCCTGGGACCTTTATTAC CATGTGTTCAGGCGGATCAGTAAG | GVQVETISPGDGRTFPK RGQTCVVHYTGMLEDG KKFDSSRDRNKPFKFML GKQEVIRGWEEGVAQM SVGQRAKLTISPDYAYG ATGHPPKIPPHATLVFDV ELLKLE |
| *Linker* | *TCAGGCGGTGGCTCAGGTGTCGAG* | *SGGGSGVD* |
| ***Δ-Caspase9*** | GTCGACGGATTTGGTGATGTCGGTGCTCTTGAGA GTTTGAGGGGAAATGCAGATTTGGCTTACATCCT GAGCATGGAGCCCTGTGGCCACTGCCTCATTATC AACAATGTGAACTTCTGCCGTGAGTCCGGGCTCC GCACCCGCACTGGCTCCAACATCGACTGTGAGAA GTTGCGGCGTCGCTTCTCCTCGCTGCATTTCATG GTGGAGGTGAAGGGCGACCTGACTGCCAAGAAA | DGFGDVGALESLRGNA DLAYILSMEPCGHCLIIN NVNFCRESGLRTRTGSN IDCEKLRRRFSSLHFMV EVKGDLTAKKMVLALLE LARQDHGALDCCVVVIL SHGCQASHLQFPGAVY |
| | ATGGTGCTGGCTTTGCTGGAGCTGGCGgGCAG GACCACGGTGCTCTGGACTGCTGCGTGGTGGTC ATTCTCTCTCACGGCTGTCAGGCCAGCCACCTGC AGTTCCCAGGGGCTGTCTACGGCACAGATGGAT GCCCTGTGTCGGTCGAGAAGATTGTGAACATCTT CAATGGGACCAGCTGCCCCAGCCTGGGAGGGAA GCCCAAGCTCTTTTTCATCCAGGCCTGTGGTGGG GAGCAGAAAGACCATGGGTTTGAGGTGGCCTCC ACTTCCCCTGAAGACGAGTCCCCTGGCAGTAACC CCGAGCCAGATGCCACCCCGTTCCAGGAAGGTT TGAGGACCTTCGACCAGCTGGACGCCATATCTAG TTTGCCCACACCCAGTGACATCTTTGTGTCCTACT CTACTTTCCCAGGTTTTGTTTCCTGGAGGGACCC CAAGAGTGGCTCCTGGTACGTTGAGACCCTGGA CGACATCTTTGAGCAGTGGGCTCACTCTGAAGAC CTGCAGTCCCTCCTGCTTAGGGTCGCTAATGCTG TTTCGGTGAAAGGGATTTATAAACAGATGCCTGG TTGCTTTAATTTCCTCCGGAAAAAACTTTTCTTTAA AACATCAGCTAGCAGAGCC | GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| ***T2A*** | GAGGGCAGGGGAAGTCTTCTAACATGCGGGGAC GTGGAGGAAAATCCCGGGCCCtga | EGRGSLLTCGDVEENP GP |

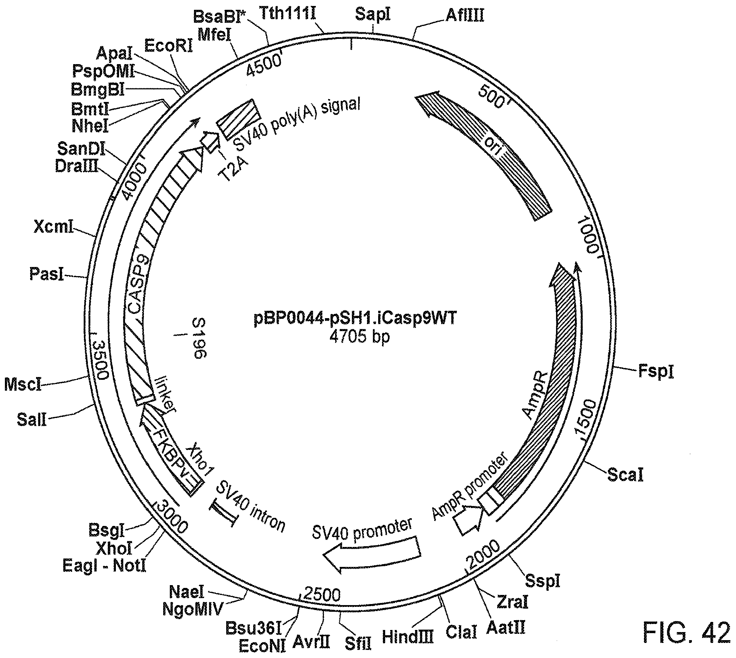

### pBP0044--pSH1-FKBP_{V36}.dCaspase9.T2A (from Figure 42

| ***Fragment*** | ***Nucleotide*** | ***Peptide*** |
|---|---|---|
| ***Linker*** | *ATGCTCGAG* | *MLE* |
| ***FKBP_{V36}*** | GGAGTGCAGGTGGAgACtATCTCCCCAGGAGACG GGCGCACCTTCCCCAAGCGCGGCCAGACCTGCG TGGTGCACTACACCGGGATGCTTGAAGATGGAAA GAAAGTTGATTCCTCCCGGGACAGAAACAAGCCC TTTAAGTTTATGCTAGGCAAGCAGGAGGTGATCC GAGGCTGGGAAGAAGGGGTTGCCCAGATGAGTG TGGGTCAGAGAGCCAAACTGACTATATCTCCAGA TTATGCCTATGGTGCCACTGGGCACCCAGGCATC ATCCCACCACATGCCACTCTCGTCTTCGATGTGG AGCTTCTAAAACTGGAA | GVQVETISPGDGRTFPK RGQTCVVHYTGMLEDG KKVDSSRDRNKPFKFML GKQEVIRGWEEGVAQM SVGQRAKLTISPDYAYG ATGHPGIIPPHATLVFDV ELLKL |
| ***Linker*** | *TCAGGCGGTGGCTCAGGTGTCGAG* | *SGGGSGVD* |
| ***Δ-Caspase9*** | GTCGACGGATTTGGTGATGTCGGTGCTCTTGAGA GTTTGAGGGGAAATGCAGATTTGGCTTACATCCT GAGCATGGAGCCCTGTGGCCACTGCCTCATTATC AACAATGTGAACTTCTGCCGTGAGTCCGGGCTCC GCACCCGCACTGGCTCCAACATCGACTGTGAGAA GTTGCGGCGTCGCTTCTCCTCGCTGCATTTCATG GTGGAGGTGAAGGGCGACCTGACTGCCAAGAAA ATGGTGCTGGCTTTGCTGGAGCTGGCGcGGCAG GACCACGGTGCTCTGGACTGCTGCGTGGTGGTC ATTCTCTCTCACGGCTGTCAGGCCAGCCACCTGC AGTTCCCAGGGGCTGTCTACGGCACAGATGGAT GCCCTGTGTCGGTCGAGAAGATTGTGAACATCTT CAATGGGACCAGCTGCCCCAGCCTGGGAGGGAA GCCCAAGCTCTTTTTCATCCAGGCCTGTGGTGGG GAGCAGAAAGACCATGGGTTTGAGGTGGCCTCC ACTTCCCCTGAAGACGAGTCCCCTGGCAGTAACC CCGAGCCAGATGCCACCCCGTTCCAGGAAGGTT TGAGGACCTTCGACCAGCTGGACGCCATATCTAG TTTGCCCACACCCAGTGACATCTTTGTGTCCTACT CTACTTTCCCAGGTTTTGTTTCCTGGAGGGACCC CAAGAGTGGCTCCTGGTACGTTGAGACCCTGGA CGACATCTTTGAGCAGTGGGCTCACTCTGAAGAC CTGCAGTCCCTCCTGCTTAGGGTCGCTAATGCTG TTTCGGTGAAAGGGATTTATAAACAGATGCCTGG TTGCTTTAATTTCCTCCGGAAAAAACTTTTCTTTAA AACATCAGCTAGCAGAGCC | DGFGDVGALESLRGNA DLAYILSMEPCGHCLIIN NVNFCRESGLRTRTGSN IDCEKLRRRFSSLHFMV EVKGDLTAKKMVLALLE LARQDHGALDCCVVVIL SHGCQASHLQFPGAVY GTDGCPVSVEKIVNIFNG TSCPSLGGKPKLFFIQAC GGEQKDHGFEVASTSP EDESPGSNPEPDATPFQ EGLRTFDQLDAISSLPTP SDIFVSYSTFPGFVSWR DPKSGSWYVETLDDIFE QWAHSEDLQSLLLRVAN AVSVKGIYKQMPGCFNF LRKKLFFKTSASRA |
| ***T2A*** | GAGGGCAGGGGAAGTCTTCTAACATGCGGGGAC GTGGAGGAAAATCCCGGGCCCtga | EGRGSLLTCGDVEENP GP |

### Materials and Methods

The following set of materials and methods may be consulted for preparing or assaying certain embodiments of the present application.

### Production of Retroviruses and transduction of Peripheral Blood Mononuclear Cells (PBMCs)

HEK 293T cells (1.5 x 10⁵) are seeded on a 100-mm tissue culture dish in 10 mL DMEM4500, supplemented with glutamine, penicillin/streptomycin and 10% fetal calf serum. After 16-30 hours incubation, cells are transfected using Novagen's GeneJuice® protocol. Briefly, for each transfection, 0.5 mL OptiMEM (LifeTechnologies) is pipeted into a 1.5-mL microcentrifuge tube and 30 µL GeneJuice reagent added followed by 5 sec. vortexing. Samples are rested 5 minutes to settle the GeneJuice suspension. DNA (15 µg total) is added to each tube and mixed by pipetting up and down four times. Samples are allowed to rest for 5 minutes for GeneJuice-DNA complex formation and the suspension added dropwise to one dish of 293T cells. A typical transfection included these plasmids to produce replication incompetent retrovirus: 3.75 µg plasmid containing gag-pol (pEQ-PAM3(-E)), 2.5 µg plasmid containing viral envelope (e.g., RD114), Retrovirus containing gene of interest = 3 = 3.75 µg.

PBMCs are stimulated with anti-CD3 and anti-CD28 antibodies precoated to wells of tissue culture plates. 24 hours after plating, 100 U/ml IL-2 is added to the culture. On day 2 or three supernatant containing retrovirus from transfected 293T cells is filtered at 0.45µm and centrifuged on non-TC treated plates precoated with Retronectin (10 µl per well in 1 ml of PBS per 1 cm² of surface). Plates are centrifuged at 2000 g for 2 hours at room temperature. CD3/CD28 blasts are resuspended at 2.5x10⁵ cells/ml in complete media, supplemented with 100 U/ml IL-2 and centrifuged on the plate at 1000 x g for 10 minutes at room temperature. After 3-4 days incubation cells are counted and transduction efficiency measured by flow cytometry using the appropriate marker antibodies (typically CD34 or CD19). Cells are maintained in complete media supplemented with 100 U/ml IL-2, refed cells every 2-3 days with fresh media and IL-2 and split as needed to expand the cells.

### T cell caspase assay in cultured cells

After transduction with the appropriate retrovirus, 50,000 T are seeded per well of 96-well plates in the presence or absence of suicide drugs (rimiducid or rapamycin) in CTL medium without IL-2. To enable detection of apoptosis using the IncuCyte instrument, 2 µM of IncuCyte™ Kinetic Caspase-3/7 Apoptosis reagent (Essen Bioscience, 4440) are add to each well to reach a total volume of 200 ul. The plates are centrifuged for 5 min at 400 x g and placed inside the IncuCyte (Dual Color Model 4459) to monitor green fluorescence every 2-3 hours for a total of 48 hours at 10x objective. Image analysis is performed using the
"Tcells_caspreagent_phase_green_10x_MLD" processing definition. The "Total Green Object Integrated Intensity" metric is used to quantify caspase activation. Each condition is performed in duplicates and each well is imaged at 4 different locations.

### T cell anti-tumor assay

The HPAC PSCA⁺ tumor cells are stably labeled with nuclear-localized RFP protein using the NucLight™ Red Lentivirus Reagent (Essen Bioscience, 4625). To set up the coculture, 4000 HPAC-RFP cells are seeded per well of 96-well plates in 100 ul of CTL medium without IL-2 for at least 4 hours to allow tumor cells to adhere. After transduction with the appropriate retrovirus and allowed to rest for at least 7 days in culture, T are seeded according to various E:T ratios to the HPAC-RFP-containing 96-well plates. Rimiducid is also added to the culture to reach 300 ul total volume per well. Each plate is set up in duplicates, one plate to monitor with the IncuCyte and one plate for supernatant collection for ELISA assay on day 2. The plates are centrifuged for 5 min at 400 x g and placed inside the IncuCyte (Dual Color Model 4459) to monitor red fluorescence (and green fluorescence if T cells are labeled with GFP-Ffluc) every 2-3 hours for a total of 7 days at 10x objective. Image analysis is performed using the "HPAC-RFP_TcellsGFP_10x_MLD" processing definition. On day 7, HPAC-RFP cells are analyzed using the "Red Object Count (1/well)" metric. Also on day 7, 0 or 10 nM of suicide drug are added to each well of the coculture and placed back in the IncuCyte to monitor T cell elimination. On day 8, Tcell-GFP cells are analyzed using the "Total Green Object Integrated Intensity" metric. Each condition is performed at least in duplicates and each well is imaged at 4 different locations.

To measure Raji cell anti-tumor activity populations of cells are determined by flow cytometry rather than incucyte as the cells do not adhere to a plate. Raji cells (ATCC) labeled by stable expression of Green Fluorescent Protein (Raji-GFP) are a Burkitt's lymphoma cell line that express CD19 on the cell surface and are a target for an anti-CD19 CAR. 50000 Raji-GFP cells are seeded on a 24 well plate with 10000 CAR-T cells, a 1:5 E:T ratio. Media supernatant is taken at 48 hours for determination of cytokine release by activated CAR-T cells. The degree of tumor killing is determined at 7 days an 14 days by flow cytometry (Galeos, Beckman-Coulter) by the proportion of GFP labeled tumor cells and CD3 labeled T cells.

### IVIS imaging

NSG mice with labeled T cells anesthetized with isofluorane and injected with 100 µl D-luciferin (15 mg/ml stock solution in PBS) by an intraperitoneal (i.p.) route in the lower abdomen. After 10 minutes the animals are transferred from the anesthesia chamber to the IVIS platform. Images are acquired from the dorsal and ventral sides with an IVIS imager (Perkin-Elmer), and BLI quantitated and documented with Living Image software (IVIS Imaging Systems).

### Western blot

After transduction with the appropriate retrovirus, 6,000,000 T cells are seeded per well of 6-well plates in 3 ml CTL medium. Twenty four hours later, cells are collected, washed in cold PBS, and lysed in RIPA Lysis and Extraction Buffer (Thermo, 89901) containing 1x Halt Protease Inhibitor Cocktail (Thermo, 87786) on ice for 30 min. in the plated. The lysates are centrifuged at 16,000 x g for 20 min at 4°C and the supernatants are transferred to new Eppendorf tubes. Protein assay is performed using the Pierce BCA Protein Assay Kit (Thermo, 23227) per manufacturer's recommendation. To prepare samples for SDS-PAGE, 50 ug of lysates are mixed with 4x Laemmli Sample Buffer (Bio Rad, 1610747) and heat at 95°C for 10 min. Meanwhile, 10% SDS gels are prepared using Bio Rad casting apparatus and 30% Acrylamide/bis Solution (Bio Rad, 160158). The samples are loaded along with Precision Plus Protein Dual Color Standards (Bio Rad, 1610374) and ran in 1x Tris/glycine Running Buffer (Bio Rad, 1610771) at 140 V for 90 min. After protein separation, the gels are transferred onto PVDF membranes using the program 0 (7 min total) in the iBlot 2 device (Thermo, IB21001). The membranes are probed with primary and secondary antibodies using the iBind Flex Western Device (Thermo, SLF2000) according to manufacturer's recommendation. Anti-MyD88 antibody (Sigma, SAB1406154) is used at 1:200 dilution and the secondary HRP-conjugated goat anti-mouse IgG antibody (Thermo, A16072) is used at 1:500 dilution. The caspase-9 antibody (Thermo, PA1-12506) is used at 1:200 dilution and the secondary HRP-conjugated goat anti-rabbit IgG antibody (Thermo, A16104) is used at 1:500 dilution. The β-actin antibody (Thermo, PA1-16889) is used at 1:1000 dilution and the secondary HRP-conjugated goat anti-rabbit IgG antibody (Thermo, A16104) is used at 1:1000 dilution. The membranes are developed using the SuperSignal West Femto Maximum Sensitivity Substrate Kit (Thermo, 34096) and imaged using the GelLogic 6000 Pro camera and the CareStream MI software (v.5.3.1.16369).

### Transfection of cells for reporter assay

HEK 293T cells (1.5 x 10⁵) are seeded on a 100-mm tissue culture dish in 10 mL DMEM4500, supplemented with glutamine, penicillin/streptomycin and 10% fetal calf serum. After 16-30 hours incubation, cells are transfected using Novagen's GeneJuice® protocol. Briefly, for each transfection, 0.5 mL OptiMEM is pipeted into a 1.5-mL microcentrifuge tube and 15 µL GeneJuice reagent added followed by 5 sec. vortexing. Samples are rested 5 minutes to settle the GeneJuice suspension. DNA (5 µg total) is added to each tube and mixed by pipetting up and down four times. Samples are allowed to rest for 5 minutes for GeneJuice-DNA complex formation and the suspension added dropwise to one dish of 293T cells. A typical transfection contains 1 µg NFkB-SEAP (5), 4 µg Go-CAR (pBP0774) or 4 µg MC-Rap-CAR (pBP1440) (1).

### Stimulation of cells with dimerizing drugs

24 hours following transfection (4.1), 293T cells are split to 96-well plates and incubated with dilutions of dimerizing drugs. Briefly, 100 µL media is added to each well of a 96-well flat-bottom plate. Drugs are diluted in tubes to a concentration 4X the top concentration in the gradient to be place on the plate. 100 µL of dimerizing ligand (rimiducid, rapamycin, isopropoxylrapamycin) is added to each of three wells on the far right of the plate (assays are thereby performed in triplicate). 100 µL from each drug-containing well is then transferred to the adjacent well and the cycle repeated 10 times to produce a serial two-fold step gradient. The last wells are untreated and serve as a control for basal reporter activity. Transfected 293 cells are then trypsinized, washed with complete media, suspended in media and 100 µL aliquoted to each well containing drug (or no drug). Cells are incubated 24 hours.

### Assay of reporter activity

The SRα promoter is a hybrid transcriptional element comprising the SV40 early region (which drives T antigen transcription) and parts (R and U5) of the Long Terminal Repeat (LTR) of Human T Cell Lymphotropic Virus (HTLV-1). This promoter drives high, constitutive levels of the Secreted Alkaline Phosphate (SeAP) reporter gene. Activation of caspase-9 by dimerization rapidly leads to cell death and the proportion of cells dying increases with increasing drug amounts. When cells die, transcription and translation of reporter stops but already secreted reporter proteins persists in the media. Loss of constitutive SeAP activity is thereby an effective proxy for drug-dependent activation of cell death.

24 hours after drug stimulation, 96-well plates are wrapped to prevent evaporation and incubated at 65®C for 2 hours to inactivate endogenous and serum phosphatases while the heat-stable SeAP reporter remains (3, 12, 14). 100 µL samples from each well are loaded into individual wells of a 96-well assay plate with black sides. Samples are incubated with 0.5 mM 4-methylumbelliferyl phosphate (4-MUP) in 0.5 M diethanolamine at pH 10.0 for 4 to 16 hours. Phosphatase activity is measured by fluorescence with excitation at 355 nm and emission at 460 nm. Data is transferred to a Microsoft Excel spreadsheet for tabulation and graphed with GraphPad Prism.

### Production of isopropyloxyrapamycin

The method of Luengo et al. ((J. Org. Chem 59:6512, (1994)), (17, 18)) is employed. Briefly, 20 mg of rapamycin is dissolved in in 3 mL isopropanol and 22.1 mg of p-toluene sulfonic acid is added and incubated at room temperature with stirring for 4-12 hours. At completion, 5 mL ethyl acetate is added and products are extracted five times with saturated sodium bicarbonate and 3 times with brine (saturated sodium chloride). The organic phase is dried and redissolved in ethyl acetate : hexane (3:1). Stereoisomers and minor products are resolved by FLASH chromatography on a 10 to 15-mL silica gel column with 3:1 ethyl acetate : hexane under 3-4 KPa pressure and fractions dried. Fractions are assayed by spectrophotometry at 237nM, 267 nM, 278 nM and 290 nM and tested for binding specificity in a FRB allele-specific transcriptional switch.

The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%).

## Claims

1. A nucleic acid comprising a promoter, operably linked to
a) a first polynucleotide encoding a first chimeric polypeptide, wherein the first chimeric polypeptide comprises (i) a first multimerizing region or a second multimerizing region; and (ii) a MyD88 polypeptide region or a truncated MyD88 polypeptide region lacking the TIR domain; and
b) a second polynucleotide encoding a second chimeric polypeptide, wherein the second chimeric polypeptide comprises (i) a pro-apoptotic polypeptide region and (ii) the first multimerizing region or the second multimerizing region, wherein
the second multimerizing region has a different amino acid sequence than the first multimerizing region;
the first chimeric polypeptide comprises the first multimerizing region and the second chimeric polypeptide comprises the second multimerizing region, or the first chimeric polypeptide comprises the second multimerizing region and the second chimeric polypeptide comprises the first multimerizing region;
the first multimerizing region and the second multimerizing region bind to a first ligand; the first multimerizing region binds to a second ligand; and
the second ligand does not significantly bind to the second multimerizing region.

2. A modified cell, transfected or transduced with the nucleic acid of claim 1.

3. A modified cell comprising
a) a first polynucleotide encoding a first chimeric polypeptide, wherein the first chimeric polypeptide comprises (i) a first multimerizing region or a second multimerizing region; and (ii) a MyD88 polypeptide region or a truncated MyD88 polypeptide region lacking the TIR domain; and
b) a second polynucleotide encoding a second chimeric polypeptide, wherein the second chimeric polypeptide comprises (i) a pro-apoptotic polypeptide region and (ii) the first multimerizing region or the second multimerizing region, wherein
the second multimerizing region has a different amino acid sequence than the first multimerizing region;
the first chimeric polypeptide comprises the first multimerizing region and the second chimeric polypeptide comprises the second multimerizing region, or the first chimeric polypeptide comprises the second multimerizing region and the second chimeric polypeptide comprises the first multimerizing region;
the first multimerizing region and the second multimerizing region bind to a first ligand; the first multimerizing region binds to a second ligand; and
the second ligand does not significantly bind to the second multimerizing region.

4. The nucleic acid of any preceding claim or the modified cell of any preceding claim, wherein the first chimeric polypeptide further comprises a CD40 cytoplasmic polypeptide region lacking the CD40 extracellular domain.

5. The nucleic acid of any preceding claim, or the modified cell of any preceding claim, wherein the nucleic acid or modified cell further comprises a polynucleotide that encodes a chimeric antigen receptor, a T cell receptor, or a T cell receptor-based chimeric antigen receptor.

6. The nucleic acid of any preceding claim or the modified cell of any preceding claim, wherein the first multimerizing region is a FKBP12 variant region and the second multimerizing region is a FKBP12-Rapamycin Binding (FRB) or FRB variant region.

7. The nucleic acid of any preceding claim or the modified cell of any preceding claim, wherein the first ligand is rapamycin or a rapalog, and the second ligand is selected from the group consisting of AP1903, AP20187, and AP1510.

8. The nucleic acid of any preceding claim, or the modified cell of any preceding claim, wherein the FKBP12 variant region has an amino acid substitution at position 36 selected from the group consisting of valine, leucine, isoleucine and alanine.

9. The nucleic acid of any preceding claim, or the modified cell of any preceding claim, wherein
the first chimeric polypeptide comprises the first multimerizing region;
the first multimerizing region comprises two FKBP12 variant regions;
the second chimeric polypeptide comprises the second multimerizing region; and
the second multimerizing region comprises a FRB region or a FRB variant region.

10. The nucleic acid or modified cell of claim 9, wherein
the first chimeric polypeptide comprises two FKBP12v36 regions and a truncated MyD88 polypeptide region lacking the TIR domain; and
the second chimeric polypeptide comprises a Caspase-9 region and a FRB region or a FRB variant region.

11. The nucleic acid or modified cell of claim 10, wherein the first chimeric polypeptide comprises a CD40 cytoplasmic polypeptide region lacking the CD40 extracellular domain.

12. The nucleic acid of any preceding claim or the modified cell of any preceding claim, wherein
the first chimeric polypeptide comprises (i) two FKBP12v36 regions; (ii) a truncated MyD88 polypeptide region lacking the TIR domain; and (iii) a CD40 cytoplasmic polypeptide region lacking the CD40 extracellular domain;
the second chimeric polypeptide comprises a Caspase-9 region and a FRB region or a FRB_{L} region; and
the nucleic acid or modified cell comprises a third polynucleotide encoding a chimeric antigen receptor comprising a transmembrane region, a T cell activation molecule, and an antigen recognition moiety, wherein the antigen recognition moiety binds to an antigen selected from the group consisting of CD19, PSCA, Her2/Neu, PSMA, Muc1, ROR1, Mesothelin, GD2, CD123, Muc16, CD33, CD38, and CD44v6.

13. The nucleic acid of any one of claims 1 or 4 to 8, or the modified cell of any one of claims 2 to 8, wherein
the first chimeric polypeptide comprises a FRB region or a FRB variant region and a truncated MyD88 polypeptide region lacking the TIR domain; and
the second chimeric polypeptide comprises a FKBP12v36 region and a Caspase-9 polypeptide region.

14. The nucleic acid or modified cell of claim 13, wherein the first chimeric polypeptide comprises a CD40 cytoplasmic polypeptide region lacking the CD40 extracellular domain.

15. The nucleic acid or the modified cell of any one of claims 1 to 8 or 13 to 14, wherein
the first chimeric polypeptide comprises (i) an FRB or FRB variant region; (ii) a truncated MyD88 polypeptide region lacking the TIR domain; and (iii) a CD40 cytoplasmic polypeptide region lacking the CD40 extracellular domain;
the second chimeric polypeptide comprises a Caspase-9 region and a FKBP12v36 region;
the nucleic acid or modified cell comprises a third polynucleotide encoding a chimeric antigen receptor comprising a transmembrane region, a T cell activation molecule, and an antigen recognition moiety selected from the group consisting of PSMA, PSCA, Muc1, CD19, ROR1, Mesothelin, GD2, CD123, Muc16, CD33, CD38, CD44v6, and Her2/Neu; and
the FRB variant region is an FRB_{L} region.

16. The nucleic acid of any preceding claim or the modified cell of any preceding claim, wherein the second polynucleotide encodes a Caspase-9 polypeptide having the amino acid sequence of SEQ ID NO: 300; or
the second polynucleotide encodes a Caspase-9 polypeptide having an amino acid sequence having at least 95% identity to SEQ ID NO: 300.

17. The nucleic acid of any preceding claim or the modified cell of any preceding claim, wherein the first polynucleotide encodes a truncated MyD88 polypeptide having the amino acid sequence of SEQ ID NO: 252; or
the first polynucleotide encodes a MyD88 polypeptide having an amino acid sequence having at least 95% identity to SEQ ID NO: 252.

18. The nucleic acid of any preceding claim or the modified cell of any preceding claim, wherein the nucleic acid or modified cell comprises a FRB variant region, and
the FRB variant region is an FRB_{L} region having the FRBI amino acid sequence provided in Table 7; or
the FRB variant region is an FRB_{L} region having an amino acid sequence having at least 95% identity to the FRBI amino acid sequence provided in Table 7.

19. The nucleic acid of any preceding claim or the modified cell of any preceding claim, wherein the nucleic acid or modified cell comprises a FKBP12 variant region, and
the FKBP12 variant region is an FKBP12v36 region having the amino acid sequence of SEQ ID NO: 21; or
the FKBP12 variant region is an FKBP12v36 region having at least 95% identity to the amino acid sequence of SEQ ID NO: 21.

20. The modified cell of any preceding claim, wherein the cell is a T cell, tumor infiltrating lymphocyte, NK-T cell, or NK cell.

## Patentansprüche

1. Nukleinsäure, umfassend einen Promoter, der funktionell verknüpft ist mit
a) einem ersten Polynukleotid, das ein erstes chimäres Polypeptid kodiert, wobei das erste chimäre Polypeptid (i) eine erste Multimerisierungsregion oder eine zweite Multimerisierungsregion und (ii) eine MyD88 Polypeptid-Region oder eine verkürzte MyD88-Polypeptid-Region, der die TIR-Domäne fehlt umfasst; und
b) einem zweiten Polynukleotid, das ein zweites chimäres Polypeptid kodiert, wobei das zweite chimäre Polypeptid (i) eine pro-apoptotische Polypeptid-Region und (ii) die erste Multimerisierungsregion oder die zweite Multimerisierungsregion umfasst, wobei
die zweite Multimerisierungsregion eine andere Aminosäuresequenz als die erste Multimerisierungsregion hat;
das erste chimäre Polypeptid die erste Multimerisierungsregion umfasst und das zweite chimäre Polypeptid die zweite Multimerisierungsregion umfasst, oder das erste chimäre Polypeptid die zweite Multimerisierungsregion umfasst und das zweite chimäre Polypeptid die erste Multimerisierungsregion umfasst;
die erste Multimerisierungsregion und die zweite Multimerisierungsregion an einen ersten Liganden binden;
die erste Multimerisierungsregion an einen zweiten Liganden bindet; und
der zweite Ligand nicht signifikant an die zweite Multimerisierungsregion bindet.

2. Modifizierte Zelle, transfiziert oder transduziert mit der Nukleinsäure nach Anspruch 1.

3. Modifizierte Zelle, umfassend
a) ein erstes Polynukleotid, das ein erstes chimäres Polypeptid kodiert, wobei das erste chimäre Polypeptid (i) eine erste Multimerisierungsregion oder eine zweite Multimerisierungsregion und (ii) eine MyD88 Polypeptid-Region oder eine verkürzte MyD88 Polypeptid-Region, der die TIR-Domäne fehlt umfasst; und
b) ein zweites Polynukleotid, das ein zweites chimäres Polypeptid kodiert, wobei das zweite chimäre Polypeptid (i) eine pro-apoptotische Polypeptid-Region und (ii) die erste Multimerisierungsregion oder die zweite Multimerisierungsregion umfasst, wobei
die zweite Multimerisierungsregion eine andere Aminosäuresequenz als die erste Multimerisierungsregion hat;
das erste chimäre Polypeptid die erste Multimerisierungsregion umfasst und das zweite chimäre Polypeptid die zweite Multimerisierungsregion umfasst, oder das erste chimäre Polypeptid die zweite Multimerisierungsregion umfasst und das zweite chimäre Polypeptid die erste Multimerisierungsregion umfasst;
die erste Multimerisierungsregion und die zweite Multimerisierungsregion an einen ersten Liganden binden;
die erste Multimerisierungsregion an einen zweiten Liganden bindet; und
der zweite Ligand nicht signifikant an die zweite Multimerisierungsregion bindet.

4. Nukleinsäure nach einem der vorherigen Ansprüche oder modifizierte Zelle nach einem der vorherigen Ansprüche, wobei das erste chimäre Polypeptid weiterhin eine cytoplasmatische CD40-Polypeptid-Region umfasst, der die extrazelluläre CD40-Domäne fehlt.

5. Nukleinsäure nach einem der vorherigen Ansprüche oder modifizierte Zelle nach einem der vorherigen Ansprüche, wobei die Nukleinsäure oder die modifizierte Zelle weiterhin ein Polynukleotid umfasst, das einen chimären Antigenrezeptor, einen T-Zell-Rezeptor oder einen T-Zell-Rezeptor basierten chimären Antigenrezeptor kodiert.

6. Nukleinsäure nach einem der vorherigen Ansprüche oder modifizierte Zelle nach einem der vorherigen Ansprüche, wobei die erste Multimerisierungsregion eine FKBP12-Variante-Region ist und die zweite Multimerisierungsregion eine FKBP12-Rapamycin-Bindungs(FRB)- oder eine FRB Variante-Region ist.

7. Nukleinsäure nach einem der vorherigen Ansprüche oder modifizierte Zelle nach einem der vorherigen Ansprüche, wobei der erste Ligand Rapamycin oder ein Rapalog ist und der zweite Ligand ausgewählt ist aus der Gruppe bestehend aus AP1903, AP20187 und AP1510.

8. Nukleinsäure nach einem der vorherigen Ansprüche oder modifizierte Zelle nach einem der vorherigen Ansprüche, wobei die FKBP12-Variante-Region eine Aminosäuresubstitution an Position 36 hat, ausgewählt aus Valin, Leucin, Isoleucin und Alanin.

9. Nukleinsäure nach einem der vorherigen Ansprüche oder modifizierte Zelle nach einem der vorherigen Ansprüche, wobei
das erste chimäre Polypeptid die erste Multimerisierungsregion umfasst;
die erste Multimerisierungsregion zwei FKBP12-Variante-Regionen umfasst; das zweite chimäre Polypeptid die zweite Multimerisierungsregion umfasst; und die zweite Multimerisierungsregion eine FRB-Region oder eine FRB-Variante-Region umfasst.

10. Nukleinsäure oder modifizierte Zelle nach Anspruch 9, wobei
das erste chimäre Polypeptid zwei FKBP12v36-Regionen und eine verkürzte MyD88 Polypeptid-Region, der die TIR-Domäne fehlt umfasst; und
das zweite chimäre Polypeptid eine Caspase-9-Region und eine FRB-Region oder eine FRB-Variante-Region umfasst.

11. Nukleinsäure oder modifizierte Zelle nach Anspruch 10, wobei das erste chimäre Polypeptid eine cytoplasmatische CD40-Polypeptid-Region umfasst, der die extrazelluläre CD40-Domäne fehlt.

12. Nukleinsäure nach einem der vorherigen Ansprüche oder modifizierte Zelle nach einem der vorherigen Ansprüche, wobei
das erste chimäre Polypeptid (i) zwei FKBP12v36-Regionen, (ii) eine verkürzte MyD88 Polypeptid-Region, der die TIR-Domäne fehlt und (iii) eine cytoplasmatische CD40-Polypeptid-Region, der die extrazelluläre CD40-Domäne fehlt, umfasst;
das zweite chimäre Polypeptid eine Caspase-9-Region und eine FRB-Region oder eine FRB_{L}-Region umfasst, und
die Nukleinsäure oder die modifizierte Zelle ein drittes Polynukleotid umfasst, das einen chimären Antigenrezeptor kodiert, umfassend eine Transmembranregion, ein T-Zell-Aktivierungs-Molekül und eine Antigenerkennungseinheit kodiert, wobei die Antigenerkennungseinheit ein Antigen bindet ausgewählt aus der Gruppe bestehend aus CD19, PSCA, Her2/Neu, PSMA, Muc1, ROR1, Mesothelin, GD2, CD123, Muc16, CD33, CD38 und CD44v6.

13. Nukleinsäure nach einem der Ansprüche 1 oder 4 bis 8 oder modifizierte Zelle nach einem der Ansprüche 2 bis 8, wobei
das erste chimäre Polypeptid eine FRB-Region oder eine FRB-Variante-Region und eine verkürzte MyD88 Polypeptid-Region, der die TIR-Domäne fehlt, umfasst; und
das zweite chimäre Polypeptid eine FKBP12v36-Region und eine Caspase-9-Polypeptid-Region umfasst.

14. Nukleinsäure oder modifizierte Zelle nach Anspruch 13, wobei das erste chimäre Polypeptid eine cytoplasmatische CD40-Polypeptid-Region umfasst, der die extrazelluläre CD40-Domäne fehlt.

15. Nukleinsäure oder modifizierte Zelle nach einem der Ansprüche 1 bis 8 oder 13 bis 14, wobei
das erste chimäre Polypeptid (i) eine FRB-Region oder eine FRB-Variante-Region, (ii) eine verkürzte MyD88 Polypeptid-Region, der die TIR-Domäne fehlt, und (iii) eine cytoplasmatische CD40-Polypeptid-Region, der die extrazelluläre CD40-Domäne fehlt, umfasst;
das zweite chimäre Polypeptid eine Caspase-9-Polypeptid-Region und eine FKBP12v36-Region umfasst;
die Nukleinsäure oder die modifizierte Zelle ein drittes Polynukleotid umfasst, das einen chimären Antigenrezeptor kodiert, umfassend eine Transmembranregion, ein T-Zell-Aktivierungs-Molekül und eine Antigenerkennungseinheit, ausgewählt aus der Gruppe bestehend aus PSMA, PSCA, Muc1, CD19, ROR1, Mesothelin, GD2, CD123, Muc16, CD33, CD38, CD44v6 und Her2/Neu; und
die FRB Variante-Region eine FRB_{L}-Region ist.

16. Nukleinsäure nach einem der vorherigen Ansprüche oder modifizierte Zelle nach einem der vorherigen Ansprüche, wobei
das zweite Polynukleotid ein Caspase-9-Polypeptid kodiert, das die Aminosäuresequenz der SEQ ID Nr: 300 hat; oder
das zweite Polynukleotid ein Caspase-9-Polypeptid kodiert, das eine Aminosäuresequenz hat, die mindestens 95% Identität mit der SEQ ID Nr: 300 hat.

17. Nukleinsäure nach einem der vorherigen Ansprüche oder modifizierte Zelle nach einem der vorherigen Ansprüche, wobei
das erste Polynukleotid ein verkürztes MyD88-Polypeptid kodiert, das die Aminosäuresequenz der SEQ ID Nr: 252 hat; oder
das erste Polynukleotid ein MyD88-Polypeptid kodiert, das eine Aminosäuresequenz hat, die mindestens 95% Identität mit der SEQ ID Nr: 252 hat.

18. Nukleinsäure nach einem der vorherigen Ansprüche oder modifizierte Zelle nach einem der vorherigen Ansprüche, wobei die Nukleinsäure oder die modifizierte Zelle eine FRB-Variante-Region umfasst, und
die FRB-Variante-Region eine FRB_{L}-Region ist, die die in der Tabelle 7 bereitgestellte FRBI-Aminosäuresequenz hat; oder
die FRB-Variante-Region eine FRB_{L}-Region ist, die eine Aminosäuresequenz hat, die mindestens 95% Identität mit der in der Tabelle 7 bereitgestellten FRBI-Aminosäuresequenz hat.

19. Nukleinsäure nach einem der vorherigen Ansprüche oder modifizierte Zelle nach einem der vorherigen Ansprüche, wobei
die Nukleinsäure oder die modifizierte Zelle eine FKBP12-Variante-Region umfasst und die FKBP12-Variante-Region eine FKBP12v36-Variante-Region ist, die die Aminosäuresequenz der SEQ ID Nr: 21 hat; oder
die FKBP12-Variante-Region eine FKBP12v36-Region ist, die mindestens 95% Identität mit der Aminosäuresequenz der SEQ ID Nr: 21 hat.

20. Modifizierte Zelle nach einem der vorherigen Ansprüche, wobei die Zelle eine T-Zelle, ein Tumor infiltrierender Lymphocyt, eine NK-T-Zelle oder eine NK-Zelle ist.

## Revendications

1. Acide nucléique comprenant un promoteur, lié de manière fonctionnelle à
a) un premier polynucléotide codant un premier polypeptide chimère, dans lequel le premier polypeptide chimère comprend (i) une première région de multimérisation ou une deuxième région de multimérisation ; et (ii) une région de polypeptide MyD88 ou une région de polypeptide MyD88 tronqué dépourvue du domaine TIR ; et
b) un deuxième polynucléotide codant un deuxième polypeptide chimère, dans lequel le deuxième polypeptide chimère comprend (i) une région de polypeptide pro-apoptotique et (ii) la première région de multimérisation ou la deuxième région de multimérisation, dans lequel
la deuxième région de multimérisation a une séquence d'acides aminés différente de celle de la première région de multimérisation ;
le premier polypeptide chimère comprend la première région de multimérisation et le deuxième polypeptide chimère comprend la deuxième région de multimérisation, ou le premier polypeptide chimère comprend la deuxième région de multimérisation et le deuxième polypeptide chimère comprend la première région de multimérisation ;
la première région de multimérisation et la deuxième région de multimérisation se lient à un premier ligand ;
la première région de multimérisation se lie à un deuxième ligand ; et
le deuxième ligand ne se lie pas significativement à la deuxième région de multimérisation.

2. Cellule modifiée, transfectée ou transduite avec l'acide nucléique de la revendication 1.

3. Cellule modifiée comprenant
a) un premier polynucléotide codant un premier polypeptide chimère, dans lequel le premier polypeptide chimère comprend (i) une première région de multimérisation ou une deuxième région de multimérisation ; et (ii) une région de polypeptide MyD88 ou une région de polypeptide MyD88 tronqué dépourvue du domaine TIR ; et
b) un deuxième polynucléotide codant un deuxième polypeptide chimère, dans lequel le deuxième polypeptide chimère comprend (i) une région de polypeptide pro-apoptotique et (ii) la première région de multimérisation ou la deuxième région de multimérisation, où
la deuxième région de multimérisation a une séquence d'acides aminés différente de celle de la première région de multimérisation ;
le premier polypeptide chimère comprend la première région de multimérisation et le deuxième polypeptide chimère comprend la deuxième région de multimérisation, ou le premier polypeptide chimère comprend la deuxième région de multimérisation et le deuxième polypeptide chimère comprend la première région de multimérisation ;
la première région de multimérisation et la deuxième région de multimérisation se lient à un premier ligand ;
la première région de multimérisation se lie à un deuxième ligand ; et
le deuxième ligand ne se lie pas significativement à la deuxième région de multimérisation.

4. Acide nucléique selon l'une quelconque des revendications précédentes ou cellule modifiée selon l'une quelconque des revendications précédentes, où le premier polypeptide chimère comprend en outre une région de polypeptide cytoplasmique CD40 dépourvue du domaine extracellulaire de CD40.

5. Acide nucléique selon l'une quelconque des revendications précédentes, ou cellule modifiée selon l'une quelconque des revendications précédentes, lequel acide nucléique ou laquelle cellule modifiée comprend en outre un polynucléotide qui code un récepteur d'antigène chimère, un récepteur de cellules T, ou un récepteur d'antigène chimère basé sur un récepteur de cellules T.

6. Acide nucléique selon l'une quelconque des revendications précédentes ou cellule modifiée selon l'une quelconque des revendications précédentes, où la première région de multimérisation est une région variante de FKBP12 et la deuxième région de multimérisation est une région de liaison à la rapamycine-FKBP12 (FRB) ou une région variante de FRB.

7. Acide nucléique selon l'une quelconque des revendications précédentes ou cellule modifiée selon l'une quelconque des revendications précédentes, où le premier ligand est la rapamycine ou un rapalogue, et le deuxième ligand est choisi dans le groupe constitué par AP1903, AP20187, et AP1510.

8. Acide nucléique selon l'une quelconque des revendications précédentes, ou cellule modifiée selon l'une quelconque des revendications précédentes, où la région variante de FKBP12 a une substitution d'acide aminé à la position 36 choisie dans le groupe constitué par la valine, la leucine, l'isoleucine et l'alanine.

9. Acide nucléique selon l'une quelconque des revendications précédentes ou cellule modifiée selon l'une quelconque des revendications précédentes, où
le premier polypeptide chimère comprend la première région de multimérisation ;
la première région de multimérisation comprend deux régions variantes de FKBP12 ;
le deuxième polypeptide chimère comprend la deuxième région de multimérisation ; et
la deuxième région de multimérisation comprend une région FRB ou une région variante de FRB.

10. Acide nucléique ou cellule modifiée selon la revendication 9, où le premier polypeptide chimère comprend deux régions FKBP12v36 et une région de polypeptide MyD88 tronqué dépourvue du domaine TIR ; et le deuxième polypeptide chimère comprend une région de caspase-9 et une région FRB ou une région variante de FRB.

11. Acide nucléique ou cellule modifiée selon la revendication 10, où le premier polypeptide chimère comprend une région de polypeptide cytoplasmique CD40 dépourvue du domaine extracellulaire de CD40.

12. Acide nucléique selon l'une quelconque des revendications précédentes ou cellule modifiée selon l'une quelconque des revendications précédentes, où
le premier polypeptide chimère comprend (i) deux régions FKBP12v36 ; (ii) une région de polypeptide MyD88 tronqué dépourvue du domaine TIR ;
et (iii) une région de polypeptide cytoplasmique CD40 dépourvue du domaine extracellulaire de CD40 ;
le deuxième polypeptide chimère comprend une région de caspase-9 et une région FRB ou une région FRB_{L} ; et
l'acide nucléique ou la cellule modifiée comprend un troisième polynucléotide codant un récepteur d'antigène chimère comprenant une région transmembranaire, une molécule d'activation des cellules T, et un fragment de reconnaissance d'antigène, lequel fragment de reconnaissance d'antigène se lie à un antigène choisi dans le groupe constitué par CD19, PSCA, Her2/Neu, PSMA, Muc1, ROR1, la mésothéline, GD2, CD123, Muc16, CD33, CD38 et CD44v6.

13. Acide nucléique selon l'une quelconque des revendications 1 ou 4 à 8, ou cellule modifiée selon l'une quelconque des revendications 2 à 8, où le premier polypeptide chimère comprend une région FRB ou une région variante de FRB et une région de polypeptide MyD88 tronqué dépourvue du domaine TIR ; et
le deuxième polypeptide chimère comprend une région FKBP12v36 et une région de polypeptide caspase-9.

14. Acide nucléique ou cellule modifiée selon la revendication 13, où le premier polypeptide chimère comprend une région de polypeptide cytoplasmique CD40 dépourvue du domaine extracellulaire de CD40.

15. Acide nucléique ou cellule modifiée selon l'une quelconque des revendications 1 à 8, ou 13 à 14, où
le premier polypeptide chimère comprend (i) une région FRB ou une région variante de FRB ; (ii) une région de polypeptide MyD88 tronqué dépourvue du domaine TIR ; et (iii) une région de polypeptide cytoplasmique CD40 dépourvue du domaine extracellulaire de CD40 ;
le deuxième polypeptide chimère comprend une région de caspase-9 et une région FKBP12v36 ;
l'acide nucléique ou la cellule modifiée comprend un troisième polynucléotide codant un récepteur d'antigène chimère comprenant une région transmembranaire, une molécule d'activation des cellules T, et un fragment de reconnaissance d'antigène choisie dans le groupe constitué par PSMA, PSCA, Muc1, CD19, ROR1, la mésothéline, GD2, CD123, Muc16, CD33, CD38, CD44v6, et Her2/Neu ; et
la région variante de FRB est FRB_{L}.

16. Acide nucléique selon l'une quelconque des revendications précédentes ou cellule modifiée selon l'une quelconque des revendications précédentes, où le deuxième polynucléotide code un polypeptide de caspase-9 ayant la séquence d'acides aminés de la SEQ ID NO : 300 ; ou le deuxième polynucléotide code un polypeptide de caspase-9 ayant une séquence d'acides aminés ayant au moins 95 % d'identité avec la SEQ ID NO : 300.

17. Acide nucléique selon l'une quelconque des revendications précédentes ou cellule modifiée selon l'une quelconque des revendications précédentes, où le premier polypeptide code un polypeptide MyD88 tronqué ayant la séquence d'acides aminés de la SEQ ID NO : 252 ; ou
le premier polynucléotide code un polypeptide MyD88 ayant une séquence d'acides aminés ayant au moins 95 % d'identité avec la SEQ ID NO : 252.

18. Acide nucléique selon l'une quelconque des revendications précédentes ou cellule modifiée selon l'une quelconque des revendications précédentes, lequel acide nucléique ou laquelle cellule modifiée comprend une région variante de FRB, et
la région variante de FRB est une région FRB_{L} ayant la séquence d'acides aminés de FRBI présentée dans le Tableau 7 ; ou
la région variante de FRB est une région FRB_{L} ayant une séquence d'acides aminés ayant au moins 95 % d'identité avec la séquence d'acides aminés de FRBI présentée dans le Tableau 7.

19. Acide nucléique selon l'une quelconque des revendications précédentes ou cellule modifiée selon l'une quelconque des revendications précédentes, lequel acide nucléique ou laquelle cellule modifiée comprend une région variante de FKBP12, et
la région variante de FKBP12 est une région FKBP12v36 ayant la séquence d'acides aminés de la SEQ ID NO : 21 ; ou
la région variante de FKBP12 est une région FKBP12v36 ayant au moins 95 % d'identité avec la séquence d'acides aminés de la SEQ ID NO : 21.

20. Cellule modifiée selon l'une quelconque des revendications précédentes, laquelle cellule est une cellule T, un lymphocyte infiltrant les tumeurs, une cellule NK-T, ou une cellule NK.
